(19) Europäisches Patentamt
European Patent Office
Office européen des brevets

(11) EP 1 381 685 B1

(12) **EUROPEAN PATENT SPECIFICATION**

(45) Date of publication and mention of the grant of the patent:
**14.09.2005 Bulletin 2005/37**

(21) Application number: **02724065.4**

(22) Date of filing: **26.04.2002**

(51) Int Cl.[7]: **C12N 15/52**, C07K 14/36, C07K 16/12, C12P 17/00

(86) International application number:
**PCT/CA2002/000591**

(87) International publication number:
**WO 2002/088176 (07.11.2002 Gazette 2002/45)**

(54) **GENES AND PROTEINS FOR THE BIOSYNTHESIS OF POLYKETIDES**
GENE UND PROTEINE FÜR POLYKETIDE BIOSYNTHESE
GENES ET PROTEINES DESTINES A LA BIOSYNTHESE DE POLYKETIDES

(84) Designated Contracting States:
**AT BE CH CY DE DK ES FI FR GB GR IE IT LI LU MC NL PT SE TR**

(30) Priority: **26.04.2001 US 286346 P**

(43) Date of publication of application:
**21.01.2004 Bulletin 2004/04**

(60) Divisional application:
**04029857.2 / 1 524 318**

(73) Proprietor: **Ecopia Biosciences Inc.**
**Québec H4S 2A1 (CA)**

(72) Inventors:
- **FARNET, Chris, M.**
  **Outremont, Québec H2V 3S3 (CA)**
- **ZAZOPOULOS, Emmanuel**
  **Montreal, Québec H2L 2Y8 (CA)**
- **STAFFA, Alfredo**
  **Saint-Laurent, Québec H4L 5M5 (CA)**
- **YANG, Xianshu**
  **Belmont, MA 02478 (US)**

(74) Representative: **Vossius & Partner**
**Siebertstrasse 4**
**81675 München (DE)**

(56) References cited:
**WO-A-00/40704**
**WO-A-01/09155**
**US-A- 5 484 799**

- **APARICIO J F ET AL: "A COMPLEX MULTIENZYME SYSTEM ENCODED BY FIVE POLYKETIDE SYNTHASE GENES IS INVOLVED IN THE BIOSYNTHESIS OF THE 26-MEMBERED POLYENE MACROLIDE PIMARICIN IN STREPTOMYCES NATALENSIS" CHEMISTRY AND BIOLOGY, CURRENT BIOLOGY, LONDON, GB, vol. 7, no. 11, November 2000 (2000-11), pages 895-905, XP001014263 ISSN: 1074-5521 -& DATABASE EMBL [Online] 6 January 2001 (2001-01-06) APARICIO J.F.: "Streptomyces natalensis pimaricin biosynthetic gene cluster" Database accession no. AJ278573 XP002222207**
- **DATABASE SWALL [Online] Entry O31783, 1 January 1998 (1998-01-01) KUNST, F. ET AL.: "Polyketide synthase of type I" Database accession no. O31783 XP002234042**
- **DATABASE SWALL [Online] Entry Q9F0Q6, 1 March 2001 (2001-03-01) SANCHEZ, C. ET AL.: "Phosphopantetheinyl transferase PptA" Database accession no. Q9F0Q6 XP002234043**
- **LIANGCHENG DU ET AL.: "The biosynthetic gene cluster for the antitumor drug bleomycin from Streptomyces verticillus ATCC15003 supporting functional interactions between nonribosomal peptide synthetases and a polyketide synthase" CHEMISTRY & BIOLOGY , vol. 7, no. 8, 1 August 2000 (2000-08-01), pages 623-642, XP001148345 & DATABASE EMBL [Online] Entry AF210249, 30 August 2000 (2000-08-30) DU L. ET AL.: "Streptomyces verticillus bleomycin biosynthetic gene cluster"**

EP 1 381 685 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- SUGAWARA K ET AL: "LACTIMIDOMYCIN, A NEW GLUTARIMIDE GROUP ANTIBIOTIC PRODUCTION, ISOLATION, STRUCTURE AND BIOLOGICAL ACTIVITY" JOURNAL OF ANTIBIOTICS, JAPAN ANTIBIOTICS RESEARCH ASSOCIATION. TOKYO, JP, vol. 45, no. 9, September 1992 (1992-09), pages 1433-1441, XP002938948 ISSN: 0021-8820

## Description

### FIELD OF INVENTION:

The present invention relates to nucleic acids molecules which encode proteins that direct the synthesis of polyketides, particularly dorrigocin polyketides. The present invention also is directed to use of DNA to produce compounds exhibiting antibiotic activity based on the dorrigocin structures.

### BACKGROUND:

Dorrigocins, migrastatins and lactimidomycins are polyketides. Polyketides occur in many types of organisms including fungi and bacteria, in particular, the actinomycetes. The structure of two dorrigocins, designated as dorrigocin A and dorrigocin B, is described in Hochlowski et al, *J. Antibiotics* 47:870 (1994) and US Patent No. 5,484,799. Dorrigocins have been reported to have antifungal and antitumor activity (Karwowski et al., *J. Antibiotics* 47:862 (1994); US Patent No. 5,589,485). Biological properties of dorrigocins are also discussed in Kadam and McAlpine, *J. Antibiotics* 47:875 (1994). The structure of migrastatin is described in Nakae et al, *J. of Antibiotics* 53: 1228 (2000). Migrastatin has been reported to inhibit tumor cell migration (Nakae et al, *J. of Antibiotics* 53:1130 (2000). A related compound, referred to as isomigrastin was described in Woo *et al*., *J Antibiotics,* Vol 55, pp.141-146 (2002). Polyketides are a class of compounds formed of 2-carbon units through a series of condensations and subsequent modifications. Polyketides are synthesized in nature by polyketide synthase (PKS) enzymes. Given the difficulty in producing polyketide compounds by traditional chemical methodology, and the typically low production of polyketides in wild-type cells, there has been considerable interest in finding improved or alternate means to produce polyketide compounds.

Aparicio et al. (2001), database EMBL accession number AJ278573.1 describes sequences of a gene cluster which encodes a multienzyme system for the biosynthesis of pimaricin in Streptomyces natalensis.
Polyketides are a class of compounds formed of 2-carbon units through a series of condensations and subsequent modifications. Polyketides are synthesized in nature by 30 polyketide synthase (PKS) enzymes. Given the difficulty in producing polyketide compounds by traditional chemical methodology, and the typically low production of polyketides in wild-type cells, there has been considerable interest in finding improved or alternate means to produce polyketide compounds.

Polyketide synthase (PKS) enzymes are complexes of multiple large proteins. PKSs catalyse the biosynthesis of polyketides through repeated, decarboxylative Claisen condensations between acylthioester building blocks. PKS enzymes are generally classified into Type I or "modular" PKSs and Type II or "iterative" PKSs according to the type of polyketide synthetized and the mode by which the polyketide is synthesized. Type I PKSs are responsible for producing a large number of 12-, 14- and 16- membered macrolide antibiotics.

Type I or modular PKS enzymes are formed by a set of separate catalytic active sites for each cycle of carbon chain elongation and modification in the polyketide synthesis pathway. Each active site is termed a domain. A set of active sites is termed a module. The typical modular PKS multienzyme system is composed of several large polypeptides, which can be segregated from amino to carboxy termini into a loading module, multiple extender modules, and a releasing module that frequently contains a thioesterase domain.

Generally, the loading module is responsible for binding the first building block used to synthesize the polyketide and transferring it to the first extender module. The loading molecule recognizes a particular acyl-CoA (usually acetyl or propionyl but sometimes butyryl or other acyl-CoA) and transfers it as a thiol ester to the ACP of the loading module.

The AT on each of the extender modules recognizes a particular extender-CoA and transfers it to the ACP of that extender module to form a thioester. Each extender module is responsible for accepting a compound from a prior module, binding a building block, attaching the building block to the compound from the prior module, optionally performing one or more additional functions, and transferring the resulting compound to the next module.

Each extender module of all modular PKS reported to date contains a KS, AT, ACP, and zero, one, two or three domains that modify the beta-carbon of the growing polyketide chain. A typical (non-loading) minimal Type I PKS extender may contain a KS domain, an AT domain, and an ACP domain. Such domains are sufficient to activate a 2-carbon extender unit and attach it to the growing polyketide molecule. The next extender module, in turn, is responsible for attaching the next building block and transferring the growing compound to the next extender module until synthesis is complete.

Once the PKS is primed with acyl-ACPs, the acyl group of the loading module is transferred to form a thiol ester (trans-esterification) at the KS of the first extender module; at this stage, extender module one possesses an acyl-KS and a malonyl- (or substituted malonyl-) ACP. The acyl group derived from the loading module is then covalently attached to the alpha-carbon of the malonyl group to form a carbon-carbon bond, driven by concomitant decarboxylation, and generating a new acyl-ACP that has a backbone two carbons longer than the loading building block (elongation or extension).

The polyketide chain, growing by two carbons with each extender module, is sequentially passed as covalently bound thiol esters from extender module to extender module, in an assembly line-like process. The carbon chain produced by this process alone would possess a ketone at every other carbon atom, producing a polyketone, from which the name polyketide arises. Most commonly, however, additional enzymatic activities modify the beta keto group of each two carbon unit just after it has been added to the growing polyketide chain but before it is transferred to the next module.

In addition to the typical KS, AT, and ACP domains necessary to form the carbon-carbon bond, a module may contain other domains that modify the beta-carbonyl moiety. For example, modules may contain a ketoreductase (KR) domain that reduces the keto group to an alcohol. Modules may also contain a KR domain plus a dehydratase (DH) domain that dehydrates the alcohol to a double bond. Modules may also contain a KR domain, a DH domain, and an enoylreductase (ER) domain that converts the double bond product to a saturated single bond. An extender module can also contain other enzymatic activities, such as, for example, a methylase or dimethylase activity.

After traversing the final extender module, the polyketide encounters a releasing domain that cleaves the polyketide from the PKS and typically cyclizes the polyketide. The polyketide can be further modified by tailoring enzymes; these enzymes add carbohydrate groups or methyl groups, or make other modifications, i.e. oxidation or reduction, on the polyketide core molecule.

In type I PKS polypeptides, the order of catalytic domains has been conserved in all type I PKSs reported to date. Thus, when all beta-keto processing domains are present in a module, the order of domains in that module from N-to-C-terminus has always been found to be KS, AT, DH, ER, KR, and ACP. Some or all of the beta-keto processing domains may be missing in particular modules, but the order of the domains present in a module has remained the same in all reported cases.

Engineering of these enzymes is achieved by modifying, adding, or deleting domains, or replacing them with those taken from other type I PKS enzymes. It is also achieved by deleting, replacing, or adding entire modules with those taken from other sources. A genetically engineered PKS complex should of course have the ability to catalyze the synthesis of the product predicted from the genetic alterations made. Between the catalytic domains and at the N- and C-termini of individual polypeptides there are linker regions. The sequences of these linker regions are less well conserved than are those for the catalytic domains. Linker regions can be important for proper association between domains and between the individual polypeptides that comprise the PKS complex. One can thus view the linkers and domains together as creating a scaffold on which the domains and modules are positioned in the correct orientation to be active. This organization and positioning, if retained, permits PKS domains of different or identical substrate specificities to be substituted (usually at the DNA level) between PKS enzymes by various available methodologies. In selecting the boundaries of, for example, an AT replacement, one can thus make the replacement so as to retain the linkers of the recipient PKS or to replace them with the linkers of the donor PKS AT domain, or, preferably, make both constructs to ensure that the correct linker regions between the KS and AT domains have been included in at least one of the engineering enzymes. Thus, there is considerable flexibility in the design of new PKS enzymes with the result that known polyketides can be produced more effectively, and novel polyketides can be made.

Although large numbers of therapeutically important polyketides have been identified, there remains a need to obtain novel polyketides that have enhanced properties such as better pharmacokinetic profile and metabolism and fewer side effects. In addition there is a need to obtain novel polyketides that possess completely novel bioactivities. The complex polyketides produced by modular type I PKSs are particularly valuable, in that they include compounds with known utility as antihelminthics, insecticides, immunosuppressants, antifungal or antibacterial agents. Because of their structural complexity, such novel polyketides are not readily obtainable by total chemical synthesis, or by chemical modifications of known polyketides.

## SUMMARY OF THE INVENTION:

The present invention advantageously provides genes and proteins involved in the production of polyketides, in particular dorrigocin polyketides. Specific embodiments of the genes and proteins are provided in the accompanying sequence listing. SEQ ID NOS: 1, 3, 5, 7, 9, 11, 13, 15, 17, 19, 21, 22 and 24 provide nucleic acids responsible for biosynthesis of the polyketide dorrigocin. SEQ ID NOS: 2, 4, 6, 8, 10, 12, 14, 16, 18, 20 and 23 provide amino acid sequences for proteins responsible for biosynthesis of the polyketide dorrigocin. The genes and proteins of the invention provide the machinery for producing novel polyketide-related compounds based on dorrigocin compounds.

The invention discloses polyketide synthase (PKS) genes (SEQ ID NOS: 11, 13 and 15) and proteins (SEQ ID NOS: 10, 12 and 14) that can be used to produce a variety of polyketides, in particular dorrigocin polyketides, some of which are now produced only by fermentation, others of which are now produced by fermentation and chemical modification, and still others of which are novel polyketides which are now not produced either by fermentation or chemical modification. The invention allows direct manipulation of dorrigocin and related chemical structures via chemical engineering of the enzymes involved in the biosynthesis of dorrigocin modifications which are presently not possible by chemical methodology because of complexity of the structures.

The invention can also be used to introduce "chemical handles" into normally inert positions that permit subsequence chemical modifications. Several general approaches to achieve the development of novel polyketides are facilitated by the methods and reagents of the present invention. For example, molecular modeling can be used to predict optimal structures. Various polyketide structures can be generated by genetic manipulation of the dorrigocin gene cluster in accordance with the methods of the invention. The invention can be used to generate a focused library of analogs around a polyketide lead candidate to fine-tune the compound for optimal properties. Genetic engineering methods of the invention can be directed to modify positions of the molecule previously inert to chemical modifications. Known techniques allow one to manipulate a known PKS gene cluster either to produce the polyketide synthesized by that PKS at higher levels than occur in nature or in hosts that otherwise do not produce the polyketide. Known techniques allow one to produce molecules that are structurally related to, but distinct from the polyketides produces from known PKS gene clusters. See, for example, PCT publications WO 93/3663; 95/08548; 96/40968; 97/02358; 98/49315; US Patent Nos. 4,874,748; 5,063,155; 5,098,837; 5,149,639; 5,672,491; 5,712,146; 5,830,750; and 5,843,718.

Thus, in a first aspect the invention provides an isolated, purified nucleic acid or enriched comprising a sequence selected from the group consisting of SEQ ID NOS: 1 and 22; the sequences complementary to SEQ ID NOS: 1 and 22; fragments comprising at least 10, 15, 20, 25, 30, 35, 40, 50, 75, 100, 200 or 500 consecutive nucleotides of SEQ ID NO: 1 and 22; and fragments comprising at least 10, 15, 20, 25, 30, 35, 40, 50, 75, 100, 200 or 500 consecutive nucleotides of the sequences complementary to SEQ ID NOS: 1 and 22. Preferred embodiments of this aspect include isolated, purified or enriched nucleic acids capable of hybridizing to the above sequences under conditions of moderate or high stringency and which encode a polypeptide useful to direct biosynthesis or dorrigocin; isolated, purified or enriched nucleic acid comprising at least 10, 15, 20, 25, 30, 35, 40, 50, 75, 100, 200 or 500 consecutive bases of the above sequences; and isolated, purified or enriched nucleic acid having at least 70%, 75%, 80%, 85%, 90%, 95%, 97% or 99% homology to the above sequences as determined by analysis with BLASTN version 2.0 with the default parameters which encodes a polypeptide useful to direct biosynthesis of dorrigocin.

More preferred embodiments of this aspect of the invention include an isolated, purified or enriched nucleic acid comprising a sequence selected from the group consisting of SEO ID NOS: 3, 5, 7, 9, 11, 13, 15, 17, 19, 21 and 24 and the sequences complementary thereto; an isolated, purified or enriched nucleic acid comprising at least 10, 15, 20, 25, 30, 35, 40, 50, 75, 100, 200 or 500 consecutive bases of a sequence selected from the group consisting of SEQ ID NOS: 3, 5, 7, 9, 11, 13, 15, 17, 19, 21 and 24 and the sequences complementary thereto; and an isolated, purified or enriched nucleic acid capable of hybridizing to the above listed nucleic acids under conditions of moderate or high stringency which encodes a polypetide useful to direct biosynthesis of dorrigocin, and isolated, purified or enriched nucleic acid having at least 70%, 75%, 80%, 85%, 90%, 95%, 97% or 99% homology to the herein described nucleic acids as determined by analysis with BLASTN version 2.0 with the default parameters and which encode a polypeptide useful to direct biosythesis of dorrogicin.

Still more preferred embodiments of this aspect of the invention include an isolated nucleic acid that encodes a domain of the PKSs of SEQ ID NOS: 10, 12 and 14; isolated nucleic acid that encodes all or part of one or more modules of the PKSs of SEQ ID NOS: 10, 12 and 14. These nucleic acids can be readily used, alone or in combination with nucleic acids encoding other PKS domains or modules as intermediates in the construction of recombinant vectors. In another aspect, the invention provides an isolated nucleic acid that encodes all or a part of a PKS that contains at least one module in which at least one of the domains in the module is a domain from a non-dorrigocin PKS and at least one domain is from a dorrigocin PKS.

In a second embodiment, the invention provides an isolated or purified polypeptide comprising a sequence selected from the group consisting of SEQ ID NOS: 2, 4, 6, 8, 10, 12, 14, 16, 18, 20 and 23; an isolated or purified polypeptide comprising at least 10, 15, 20, 25, 30, 35, 40, 50, 75, 100, 200 or 500 consecutive amino acids of the polypeptides of SEQ ID NOS: 2, 4, 6, 8, 10, 12, 14, 16, 18, 20 and 23; and an isolated or purified polypeptide having at least 70%, 75%, 80%, 85%, 90%, 95%, 97%, or 99% homology to the polypeptide of SEQ ID NOS: 2, 4, 6, 8, 10, 12, 14, 16, 18, 20 and 23 as determined by analysis with BLASTP version 2.2.2 with the default parameters. In a further aspect, the invention provides a polypeptide comprising one or two or three or five or more or the above polypeptide sequences.

The invention also provides recombinant DNA expression vectors containing the above nucleic acids. These genes and the methods of the invention enable one skilled in the art to create recombinant host cells with the ability to produce polyketides. Thus, the invention provides a method of preparing a polyketide, said method comprising transforming a heterologous host cell with a recombinant DNA vector that encodes at least one of the above nucleic acids, and culturing said host cell under conditions such that a PKS is produced, which PKS catalyzes synthesis of a polyketide. In one aspect, the method is practiced with a *Streptomyces* host cell. In another aspect, the polyketide produced is dorrigocin. In another aspect, the polyketide produced is a polyketide related in structure to dorrigocin.

One embodiment of this aspect of the invention is a method of expressing a dorrigocin biosynthetic gene product comprising culturing a host cell under conditions that permit expression of the dorrigocin biosynthetic gene product.

The invention also encompasses a reagent comprising a probe of the invention for detecting and/or isolating putative polyketide-producing microorganisms; and a method for detecting and/or isolating putative polyketide-producing microorganisms using a probe of the invention such that hybridization is detected. Cloning, analysis, and manipulation by recombinant DNA technology of genes that encode PKS enzymes can be performed according to known techniques.

## BRIEF DESCRIPTION OF THE DRAWINGS

The present invention will be further understood from the following description with reference to the following figures:

Figure 1 shows a diagram of the dorrigocin biosynthetic gene cluster of *S. platensis* highlighting the deduced domain architecture of the unusual PKS components.

Figure 2 shows one proposed biosynthetic pathway for dorrigocins and migrastatin.

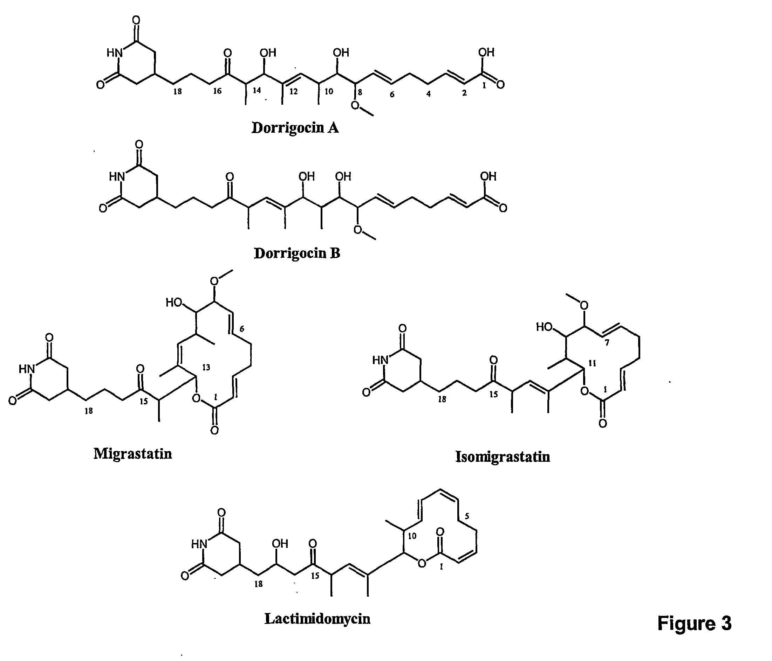

Figure 3 illustrates the structures of the dorrigocins, migrastatin, and isomigrastatin.

Figure 4 shows a diagram comparing the lactimidomycin biosynthetic gene cluster of *S. amphibiosporus* and the dorrigocin biosynthetic gene cluster of *S. platensis*. The deduced domain architecture of the unusual PKS components is highlighted.

Figure 5 shows one proposed biosynthetic pathway for lactimidomycin.

Figure 6 shows an amino acid alignment comparing DORR ORF 2 (SEQ ID NO: 4) to its lactimidomycin homologue, LACT ORF 1 (SEQ ID NO: 26), both of which are fusions of an acyltransferase and a thioesterase designated as AYTT.

Figure 7 shows an amino acid alignment comparing DORR ORF 3 (SEQ ID NO: 6) to its lactimidomycin homologue, LACT ORF 2 (SEQ ID NO: 28), both of which are acyl carrier proteins designated as ACPI.

Figure 8 shows an amino acid alignment comparing DORR ORF 4 (SEQ ID NO: 8) to its lactimidomycin homologue, LACT ORF 3 (SEQ ID NO: 30), both of which are amidotransferases similar to bacterial asparagine synthetases designated as AOTF.

Figures 9A to 9D shows an amino acid alignment comparing DORR ORF 5 (SEQ ID NO: 10) to its lactimidomycin homologue, LACT ORF 4 (SEQ ID NO: 32), both of which are unusual modular PKSs devoid of AT domains designated as PKUN.

Figures 10A to 10J show an amino acid alignment comparing DORR ORF 6 (SEQ ID NO: 12) to its lactimidomycin homologue, LACT ORF 5 (SEQ ID NO: 34), both of which are unusual modular PKSs devoid of AT domains designated as PKUN.

Figures 11A to 11C show an amino acid alignment comparing DORR ORF 7 (SEQ ID NO: 14) to its lactimidomycin homologue, LACT ORF 6 (SEQ ID NO: 12), both of which are unusual modular PKSs devoid of AT domains designated as PKUN.

Figure 12 shows an amino acid alignment comparing DORR ORF 8 (SEQ ID NO: 16) to its lactimidomycin homologue, LACT ORF 7 (SEQ ID NO: 38), both of which are fusions of an acyltransferase and an oxidoreductase designated as AYOA.

Figure 13 shows an amino acid alignment comparing DORR ORF 11 (SEQ ID NO: 23) to its lactimidomycin homologue, LACT ORF 8 (SEQ ID NO: 40), both of which are cytochrome P450 monooxygenases designated as OXRC.

## DETAILED DESCRIPTION OF THE INVENTION:

Some authors have distinguished between dorrigocin and migrastatin molecules. Throughout the specification reference to dorrigocin is intended to encompass the molecules referred to by some authors as migrastatin and isomigrastatin. Likewise reference to the biosynthetic locus for dorrigocin is intended to encompass the biosynthetic locus that directs the synthesis of the molecules some authors have referred to as migrastatin and isomigrastatin.

Throughout the description and the figures, the biosynthetic locus for dorrigocin from *Streptomyces platensis* subsp. *rosaceus* NRRL 18993 is sometimes referred to as DORR. The ORFs in DORR are assigned a putative function and grouped together in families based on homology to known proteins. To correlate structure and function, the protein families are given a four-letter designation used throughout the description and figures as indicated in Table I.

Table 1

| Families | Function |
|---|---|
| REBP | regulator, multidomain; fusion of a pathway specific activator-type regulator with a protein containing domain homology to LuxR family regulators |
| AYTT | acyltransferase-thioesterase fusion; N-terminus shows strong homology to malonyl CoA:ACP transacylases; C-terminal region shows strong homology to thioesterases |
| ACPI | acyl carrier protein; similar to proteins that may carry aminoacyl groups; similar to undecylprodigiosin RedO and coumermycin ProC PCPs that tether prolyl groups that may serve as substrates for oxidation while tethered |
| AOTF | amidotransferase, ATP-dependent,asparaginase; asparagine synthetases class B (glutamine-hydrolyzing); glutamine amidotransferase/asparagine synthase; asparagine synthetases (glutamine amidotransferases); catalyze the transfer of the carboxamide amino group of glutamine to the carboxylate group of aspartate. |
| PKUN | unusual polyketide synthase; devoid of AT domains; strong homology to B,subtilis Pks K and Pks M proteins found in an unknown polyketide locus acyltransferase-oxidoreductase fusion; strong homology to B. subtilis PksE fusion protein found in unknown polyketide locus; N-terminus shows strong homology to malonyl CoA:ACP transacylases; C-terminal region shows strong homology to 2-nitropopane dioxygenase-like enzymes found in loci required for polyunsaturated fatty acid (eicosapentaenoic acid) or polyketide biosynthesis |
| AYOA | |
| OXRY | oxidoreductase; zinc-binding, NADP-dependent dehydrogenase; similar to quinone oxidoreductases |
| MTFA | methyltransferase, SAM-dependent; includes O-methyltransferases, N,N-dimethyltransferases (e.g. spinosyn SpnS N-dimethyltransferase), C-methyltransferases |
| OXRC | oxidoreductase; cytP450 monooxygenase, hydroxylase; includes PikC, DoxA, FkbD |
| PPTF | phosphopantetheinyl transferases, required for activation of both PKSs and NRPSs from inactive apo forms to active holo forms; homology to B.subtilis Sfp, Anabaena Hetl, E. coli EntD and AcpS |

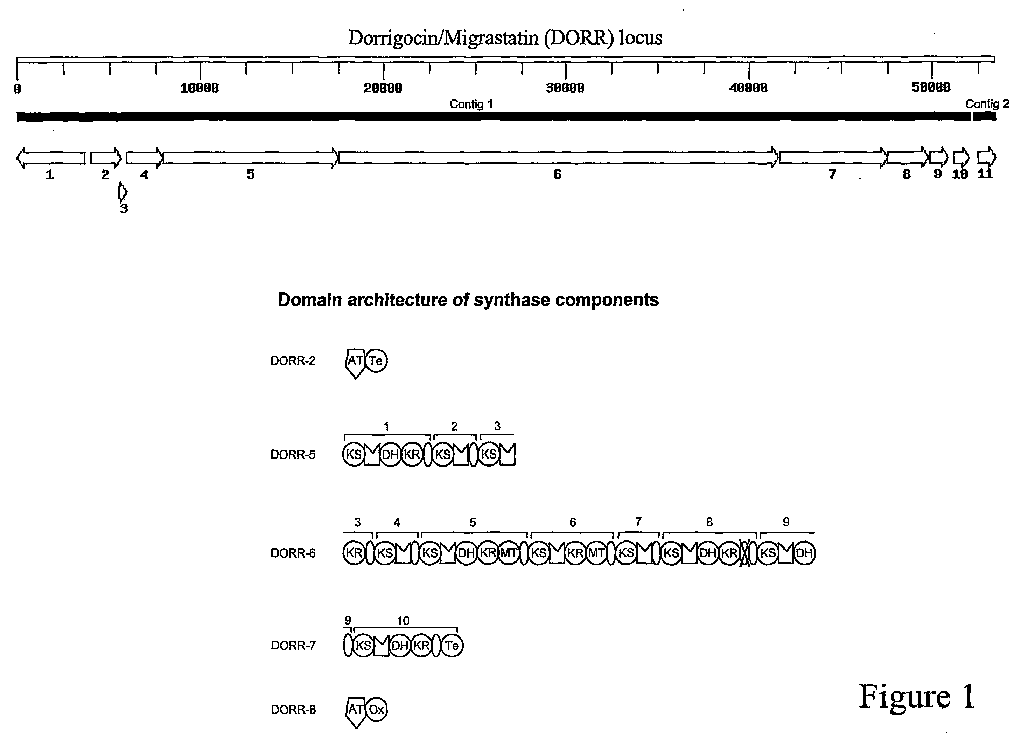

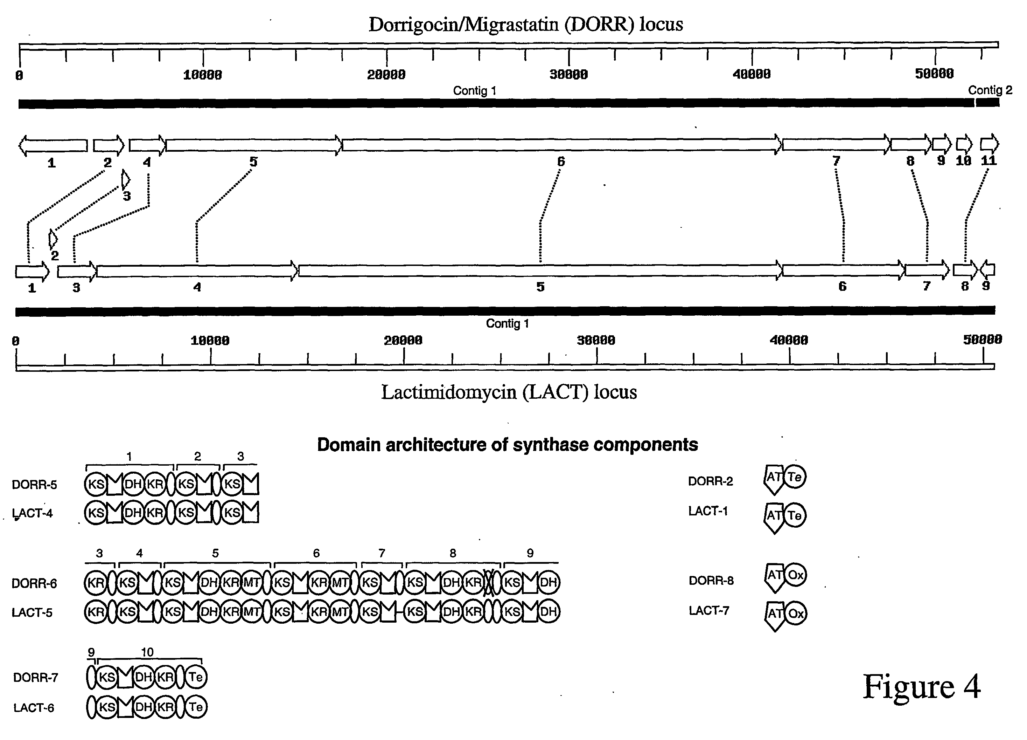

The term dorrigocin biosynthetic gene product refers to any enzyme involved in the biosynthesis of dorrigocin, migrastatin or isomigrastatin. These genes are located in the dorrigocin biosynthetic locus from *Streptomyces platensis* subsp. *rosaceus*. This locus is depicted in Figures 1 and 4. For the sake of particularity the dorrigocin biosynthetic pathway is associated with *Streptomyces platensis* subsp. *rosaceus*. However, it should be understood that this term encompasses dorrigocin biosynthetic enzymes (and genes encoding such enzymes) isolated from any microorganism of the genus *Streptomyces,* and furthermore that these genes may have novel homologues in related actinomycete microorganisms that fall within the scope of the claims here. In specific embodiments, the genes are listed in SEQ ID NOS: 2, 4, 6, 8, 10, 12, 14, 16, 18, 20 and 23.

The term "isolated" means that the material is removed from its original environment, *e.g.* the natural environment if it is naturally occurring. For example, a naturally-occurring polynucleotide or polypeptide present in a living organism is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The term "purified" does not require absolute purity; rather, it is intended as a relative definition. Individual nucleic acids obtained from a library have been conventionally purified to electrophoretic homogeneity. The sequences obtained from these clones could not be obtained directly from a large insert library, such as a cosmid library, or from total organism DNA. The purified nucleic acids of the present invention have been purified from the remainder of the genomic DNA in the organism by at least $10^4$ to $10^6$ fold. However, the term "purified" also includes nucleic acids which have been purified from the remainder of the genomic DNA or from other sequences in a library or other environment by at least one order of magnitude, preferably two or three orders of magnitude, and more preferably four or five orders of magnitude.

"Recombinant" means that the nucleic acid is adjacent to "backbone" nucleic acid to which it is not adjacent in its natural environment. "Enriched" nucleic acids represent 5% or more of the number of nucleic acid inserts in a population of nucleic acid backbone molecules. "Backbone" molecules include nucleic acids such as expression vectors, self-replicating nucleic acids, viruses, integrating nucleic acids, and other vectors or nucleic acids used to maintain or manipulate a nucleic acid of interest. Preferably, the enriched nucleic acids represent 15% or more, more preferably 50% or more, and most preferably 90% or more, of the number of nucleic acid inserts in the population of recombinant backbone molecules.

"Recombinant" polypeptides or proteins refers to polypeptides or proteins produced by recombinant DNA techniques, *i.e.* produced from cells transformed by an exogenous DNA construct encoding the desired polypeptide or protein. "Synthetic" polypeptides or proteins are those prepared by chemical synthesis.

The term "gene" means the segment of DNA involved in producing a polypeptide chain; it includes regions preceding and following the coding region (leader and trailer) as well as, where applicable, intervening regions (introns) between individual coding segments (exons).

A DNA or nucleotide "coding sequence" or "sequence encoding" a particular polypeptide or protein, is a DNA sequence which is transcribed and translated into a polypeptide or protein when placed under the control of appropriate regulatory sequences.

"Oligonucleotide" refers to a nucleic acid, generally of at least 10, preferably 15 and more preferably at least 20 nucleotides, preferably no more than 100 nucleotides, that are hybridizable to a genomic DNA molecule, a cDNA molecule, or an mRNA molecule encoding a gene, mRNA, cDNA or other nucleic acid of interest.

A promoter sequence is "operably linked to" a coding sequence recognized by RNA polymerase which initiates transcription at the promoter and transcribes the coding sequence into mRNA.

"Plasmids" are designated by a lower case p preceded or followed by capital letters and/or numbers. The starting plasmids herein are commercially available, publicly available on an unrestricted basis, or can be constructed from available plasmids in accord with published procedures. In addition, equivalent plasmids to those described herein are known in the art and will be apparent to the skilled artisan.

"Digestion" of DNA refers to enzymatic cleavage of the DNA with a restriction enzyme that acts only at certain sequences in the DNA. The various restriction enzymes used herein are commercially available and their reaction conditions, cofactors and other requirements were used as would be known to the ordinary skilled artisan.

For analytical purposes, typically 1 μg of plasmid or DNA fragment is used with about 2 units of enzyme in about 20 μl of buffer solution. For the purpose of isolating DNA fragments for plasmid construction, typically 5 to 50 μg of DNA are digested with 20 to 250 units of enzyme in a larger volume. Appropriate buffers and substrate amounts for particular enzymes are specified by the manufacturer. Incubation times of about 1 hour at 37°C are ordinarily used, but may vary in accordance with the supplier's instructions. After digestion the gel electrophoresis may be performed to isolate the desired fragment.

We have now discovered the genes and proteins involved in the biosynthesis of the polyketides dorrigocin and lactimidomycin. Nucleic acid sequences encoding proteins involved in the biosynthesis of dorrigocin are provided in the accompanying sequence listing as SEQ ID NOS: 3, 5, 7, 9, 11, 13, 15, 17, 19, 21 and 24. Polypeptides involved in the biosynthesis of dorrigocin are provided in the accompanying sequence listing as SEQ ID NOS: 2, 4, 6, 8, 10, 12, 14, 16, 18, 20 and 23.

One aspect of the present invention is an isolated, purified, or enriched nucleic acid comprising one of the sequences of SEQ ID NOS: 3, 5, 7, 9, 11, 13, 15, 17, 19, 21 and 24, the sequences complementary thereto, or a fragment comprising at least 10, 15, 20, 25, 30, 35, 40, 50, 75, 100, 150, 200, 300, 400 or 500 consecutive bases of one of the sequences of SEQ ID NOS: 3, 5, 7, 9, 11, 13, 15, 17, 19, 21 and 24 or the sequences complementary thereto. The isolated, purified or enriched nucleic acids may comprise DNA, including cDNA, genomic DNA, and synthetic DNA. The DNA may be double stranded or single stranded, and if single stranded may be the coding or non-coding (anti-sense) strand. Alternatively, the isolated, purified or enriched nucleic acids may comprise RNA.

As discussed in more detail below, the isolated, purified or enriched nucleic acids of one of SEQ ID NOS: may be used to prepare one of the polypeptides of SEQ ID NOS: 2, 4, 6, 8, 10, 12, 14, 16, 18, 20 and 23 or fragments comprising at least 5, 10, 15, 20, 25, 30, 35, 40, 50, 75, 100 or 100 consecutive amino acids of one of the polypeptides of SEQ ID NO: 2, 4, 6, 8, 10, 12, 14, 16, 18, 20 and 23.

Accordingly, another aspect of the present invention is an isolated, purified or enriched nucleic acid which encodes one of the polypeptides of SEQ ID NOS: 2, 4, 6, 8, 10, 12, 14, 16, 18, 20 and 23, or fragments comprising at least 5, 10, 15, 20, 25, 30, 35, 40, 50, 75, 100 or 150 consecutive amino acids of one of the polypeptides of SEQ ID NOS: 2, 4, 6, 8, 10, 12, 14, 16, 18, 20 and 23. The coding sequences of these nucleic acids may be identical to one of the coding sequences of one of the nucleic acids of SEQ ID NOS: 3, 5, 7, 9, 11, 13, 15, 17, 19, 21 and 24 or a fragment thereof which encodes a polypeptide useful to direct biosynthesis of dorrigocin or may be different coding sequences which encode one of the polypeptides of SEQ ID NOS: 2, 4, 6, 8, 10, 12, 14, 16, 18, 20 and 23, or fragments comprising at least 5, 10, 15, 20, 25, 30, 35, 40, 50, 75, 100 or 150 consecutive amino acids of one of the polypeptides of SEQ ID NOS: 2, 4, 6, 8, 10, 12, 14, 16, 18, 20 and 23 as a result of the redundancy or degeneracy of the genetic code. The genetic code is well known to those of skill in the art and can be obtained, for example, from Stryer, *Biochemistry*, 3rd edition, W. H. Freeman & Co., New York.

The isolated, purified or enriched nucleic acid which encodes one of the polypeptides of SEQ ID NOS: 2, 4, 6, 8, 10, 12, 14, 16, 18, 20 and 23, may include, but is not limited to: (1) only the coding sequences of one of SEQ ID NOS: 3, 5, 7, 9, 11, 13, 15, 17, 19, 21 and 24; (2) the coding sequences of SEQ ID NOS: 3, 5, 7, 9, 11, 13, 15, 17, 19, 21 and 24 and additional coding sequences, such as leader sequences or proprotein; and (3) the coding sequences of SEQ ID NOS: 3, 5, 7, 9, 11, 13, 15, 17, 19, 21 and 24 and non-coding sequences, such as introns or non-coding sequences 5' and/or 3' of the coding sequence. Thus, as used herein, the term "polynucleotide encoding a polypeptide" encompasses a polynucleotide which includes only coding sequence for the polypeptide as well as a polynucleotide which includes additional coding and/or non-coding sequence.

The invention relates to polynucleotides based on SEQ ID NOS: 3, 5, 7, 9, 11, 13, 15, 17, 19, 21 and 24 but having polynucleotide changes that are "silent", for example changes which do not alter the amino acid sequence encoded by the polynucleotides of SEQ ID NOS: 3, 5, 7, 9, 11, 13, 15, 17, 19, 21 and 24. The invention also relates to polynucleotides which have nucleotide changes which result in amino acid substitutions, additions, deletions, fusions and truncations of the polypeptides of SEQ ID NOS: 2, 4, 6, 8, 10, 12, 14, 16, 18, 20 and 23. Such nucleotide changes may be introduced using techniques such as site directed mutagenesis, random chemical mutagenesis, exonuclease III deletion, and other recombinant DNA techniques.

The isolated, purified or enriched nucleic acids of SEQ ID NOS: 3, 5, 7, 9, 11, 13, 15, 17, 19, 21 and 24, the sequences complementary thereto, or a fragment comprising at least 10, 15, 20, 25, 30, 35, 40, 50, 75, 100, 150, 200, 300, 400 or 500 consecutive bases of one of the sequence of SEQ ID NOS: 3, 5, 7, 9, 11, 13, 15, 17, 19, 21 and 24, or the sequences complementary thereto may be used as probes to identify and isolate DNAs encoding the polypeptides of SEQ ID NOS: 2, 4, 6, 8, 10, 12, 14, 16, 18, 20 and 23. In such procedures, a genomic DNA library is constructed from a sample microorganism or a sample containing a microorganism capable of producing a polyketide. The genomic DNA library is then contacted with a probe comprising a coding sequence or a fragment of the coding sequence, encoding one of the polypeptides of SEQ ID NOS: 2, 4, 6, 8, 10, 12, 14, 16, 18, 20 and 23, or a fragment thereof under conditions which permit the probe to specifically hybridize to sequences complementary thereto. In a preferred embodiment, the probe is an oligonucleotide of about 10 to about 30 nucleotides in length designed based on a nucleic acid of SEQ ID NOS: 3, 5, 7, 9, 11, 13, 15, 17, 19, 21 and 24. Genomic DNA clones which hybridize to the probe are then detected and isolated. Procedures for preparing and identifying DNA clones of interest are disclosed in Ausubel *et al*., Current Protocols in Molecular Biology, John Wiley 503 Sons, Inc. 1997; and Sambrook *et al*., Molecular Cloning: A Laboratory Manual 2d Ed., Cold Spring Harbor Laboratory Press, 1989. In another embodiment, the probe is a restriction fragments or a PCT amplified nucleic acid derived from SEQ ID NOS: 3, 5, 7, 9, 11, 13, 15, 17, 19, 21 and 24. The isolated, purified or enriched nucleic acids of SEQ ID NOS: 3, 5, 7, 9, 11, 13, 15, 17, 19, 21 and 24, the sequences complementary thereto, or a fragment comprising at least 10, 15, 20, 25, 30, 35, 40, 50, 75, 100, 150, 200, 300, 400 or 500 consecutive bases of one of the sequences of SEQ ID NOS: 3, 5, 7, 9, 11, 13, 15, 17, 19, 21 and 24, or the sequences complementary thereto may be used as probes to identify and isolate related nucleic acids. In some embodiments, the related nucleic acids may be genomic DNAs (or cDNAs) from potential polyketide producers. In a preferred embodiment isolated, purified or enriched nucleic acids of SEQ ID NOS: 11, 13 and 15 the sequences complementary thereto, or a fragment comprising at least 10, 15, 20, 25, 30, 35, 40, 50, 75, 100, 150, 200, 300, 400 or 500 consecutive bases of one of the sequences of SEQ ID NOS: 11, 13 and 15, or the sequences complementary thereto may be used as probes to identify and isolate related nucleic acids. In such procedures, a nucleic acid sample containing nucleic acids from a potential polyketide-producer is contacted with the probe under conditions which permit the probe to specifically hybridize to related sequences. The nucleic acid sample may be a genomic DNA (or cDNA) library from the potential polyketide-producer. Hybridization of the probe to nucleic acids is then detected using any of the methods described above.

Hybridization may be carried out under conditions of low stringency, moderate stringency or high stringency. As an example of nucleic acid hybridization, a polymer membrane containing immobilized denatured nucleic acids is first prehybridized for 30 minutes at 45 °C in a solution consisting of 0.9 M NaCl, 50 mM $NaH_2PO_4$, pH 7.0, 5.0 mM $Na_2EDTA$, 0.5% SDS, 10X Denhardt's, and 0.5 mg/ml polyriboadenylic acid. Approximately 2 x $10^7$ cpm (specific activity 4-9 x $10^8$ cpm/ug) of $^{32}P$ end-labeled oligonucleotide probe are then added to the solution. After 12-16 hours of incubation, the membrane is washed for 30 minutes at room temperature in 1 X SET (150 mM NaCl, 20 mM Tris hydrochloride, pH 7.8, 1 mM $Na_2EDTA$) containing 0.5% SDS, followed by a 30 minute wash in fresh 1X SET at Tm-10 C for the oligonucleotide probe where Tm is the melting temperature. The membrane is then exposed to autoradiographic film for detection of hybridization signals.

By varying the stringency of the hybridization conditions used to identify nucleic acids, such as genomic DNAs or cDNAs, which hybridize to the detectable probe, nucleic acids having different levels of homology to the probe can be identified and isolated. Stringency may be varied by conducting the hybridization at varying temperatures below the melting temperatures of the probes. The melting temperature of the probe may be calculated using the following formulas:

For oligonucleotide probes between 14 and 70 nucleotides in length the melting temperature (Tm) in degrees Celcius may be calculated using the formula:

$$Tm=81.5+16.6(\log [Na+]) + 0.41 (\text{fraction } G+C)-(600/N)$$

where N is the length of the oligonucleotide.

If the hybridization is carried out in a solution containing formamide, the melting temperature may be calculated using the equation Tm=81.5+16.6(log [Na +]) + 0.41 (fraction G + C)-(0.63% formamide)-(600/N) where N is the length of the probe. Prehybridization may be carried out in 6X SSC, 5X Denhardt's reagent, 0.5% SDS, 0.1 mg/ml denatured fragmented salmon sperm DNA or 6X SSC, 5X Denhardt's reagent, 0.5% SDS, 0.1 mg/ml denatured fragmented salmon sperm DNA, 50% formamide. The composition of the SSC and Denhardt's solutions are listed in Sambrook et al., *supra.* Hybridization is conducted by adding the detectable probe to the hybridization solutions listed above. Where the probe comprises double stranded DNA, it is denatured by incubating at elevated temperatures and quickly cooling before addition to the hybridization solution. It may also be desirable to similarly denature single stranded probes to eliminate or diminish formation of secondary structures or oligomerization. The filter is contacted with the hybridization solution for a sufficient period of time to allow the probe to hybridize to cDNAs or genomic DNAs containing sequences complementary thereto or homologous thereto. For probes over 200 nucleotides in length, the hybridization may be carried out at 15-25 °C below the Tm. For shorter probes, such as oligonucleotide probes, the hybridization may be conducted at 5-10 °C below the Tm. Preferably, the hybridization is conducted in 6X SSC, for shorter probes. Preferably, the hybridization is conducted in 50% formamide containing solutions, for longer probes. All the foregoing hybridizations would be considered to be examples of hybridization performed under conditions of high stringency.

Following hybridization, the filter is washed for at least 15 minutes in 2X SSC, 0.1 % SDS at room temperature or higher, depending on the desired stringency. The filter is then washed with 0.1X SSC, 0.5% SDS at room temperature (again) for 30 minutes to 1 hour. Nucleic acids which have hybridized to the probe are identified by autoradiography or other conventional techniques.

The above procedure may be modified to identify nucleic acids having decreasing levels of homology to the probe sequence. For example, to obtain nucleic acids of decreasing homology to the detectable probe, less stringent conditions may be used. For example, the hybridization temperature may be decreased in increments of 5 °C from 68 °C to 42 °C in a hybridization buffer having a Na+ concentration of approximately 1 M. Following hybridization, the filter may be washed with 2X SSC, 0.5% SDS at the temperature of hybridization. These conditions are considered to be "moderate stringency" conditions above 50°C and "low stringency" conditions below 50°C. A specific example of "moderate stringency" hybridization conditions is when the above hybridization is conducted at 55°C. A specific example of "low stringency" hybridization conditions is when the above hybridization is conducted at 45°C.

Alternatively, the hybridization may be carried out in buffers, such as 6X SSC, containing formamide at a temperature of 42 °C. In this case, the concentration of formamide in the hybridization buffer may be reduced in 5% increments from 50% to 0% to identify clones having decreasing levels of homology to the probe. Following hybridization, the filter may be washed with 6X SSC, 0.5% SDS at 50 °C. These conditions are considered to be "moderate stringency" conditions above 25% formamide and "low stringency" conditions below 25% formamide. A specific example of "moderate stringency" hybridization conditions is when the above hybridization is conducted at 30% formamide. A specific example of "low stringency" hybridization conditions is when the above hybridization is conducted at 10% formamide. Nucleic acids which have hybridized to the probe are identified by autoradiography or other conventional techniques.

For example, the preceding methods may be used to isolate nucleic acids having a sequence with at least 97%, at least 95%, at least 90%, at least 85%, at least 80%, or at least 70% homology to a nucleic acid sequence selected from the group consisting of the sequences of SEQ ID NOS: 3, 5, 7, 9, 11, 13, 15, 17, 19, 21 and 24 which encode a polypeptide useful to direct biosynthesis of dorrigocin, fragments comprising at least 10, 15, 20, 25, 30, 35, 40, 50, 75, 100, 150, 200, 300, 400, or 500 consecutive bases thereof, and the sequences complementary thereto. Homology may be measured using BLASTN version 2.0 with the default parameters. For example, the homologous polynucleotides may have a coding sequence which is a naturally occurring allelic variant of one of the coding sequences described herein. Such allelic variant may have a substitution, deletion or addition of one or more nucleotides when compared to the nucleic acids of SEQ ID NOS: 3, 5, 7, 9, 11, 13, 15, 17, 19, 21 and 24, or the sequences complementary thereto which encodes a polypeptide useful to direct biosynthesis of dorricogin.

Additionally, the above procedures may be used to isolate nucleic acids which encode polypeptides having at least 99%, 95%, at least 90%, at least 85%, at least 80%, or at least 70% homology to a polypeptide having the sequence of one of SEQ ID NOS: 2, 4, 6, 8, 10, 12, 14, 16, 18, 20 and 23, or fragments comprising at least 5, 10, 15, 20, 25, 30, 35, 40, 50, 75, 100, or 150 consecutive amino acids thereof as determined using the BLASTP version 2.2.2 algorithm with default parameters.

Another aspect of the present invention is an isolated or purified polypeptide comprising the sequence of one of SEQ ID NOS: 2, 4, 6, 8, 10, 12, 14, 16, 18, 20 and 23 or fragments comprising at least 5, 10, 15, 20, 25, 30, 35, 40, 50, 75, 100, or 150 consecutive amino acids thereof. As discussed above, such polypeptides may be obtained by inserting a nucleic acid encoding the polypeptide into a vector such that the coding sequence is operably linked to a sequence capable of driving the expression of the encoded polypeptide in a suitable host cell. For example, the expression vector may comprise a promoter, a ribosome binding site for translation initiation and a transcription terminator. The vector may also include appropriate sequences for modulating expression levels, an origin of replication and a selectable marker.

Promoters suitable for expressing the polypeptide or fragment thereof in bacteria include the *E.coli lac* or *trp* promoters, the lacI promoter, the lacZ promoter, the T3 promoter, the T7 promoter, the gpt promoter, the lambda $P_R$ promoter, the lambda $P_L$ promoter, promoters from operons encoding glycolytic enzymes such as 3-phosphoglycerate kinase (PGK), and the acid phosphatase promoter. Fungal promoters include the $\alpha$ factor promoter. Eukaryotic promoters include the CMV immediate early promoter, the HSV thymidine kinase promoter, heat shock promoters, the early and late SV40 promoter, LTRs from retroviruses, and the mouse metallothionein-I promoter. Other promoters known to control expression of genes in prokaryotic or eukaryotic cells or their viruses may also be used. Mammalian expression vectors may also comprise an origin of replication, any necessary ribosome binding sites, a polyadenylation site, splice donors and acceptor sites, transcriptional termination sequences, and 5' flanking nontranscribed sequences. In some embodiments, DNA sequences derived from the SV40 splice and polyadenylation sites may be used to provide the required nontranscribed genetic elements.

Vectors for expressing the polypeptide or fragment thereof in eukaryotic cells may also contain enhancers to increase expression levels. Enhancers are cis-acting elements of DNA, usually from about 10 to about 300 bp in length that act on a promoter to increase its transcription. Examples include the SV40 enhancer on the late side of the replication origin bp 100 to 270, the cytomegalovirus early promoter enhancer, the polyoma enhancer on the late side of the replication origin, and the adenovirus enhancers.

In addition, the expression vectors preferably contain one or more selectable marker genes to permit selection of host cells containing the vector. Examples of selectable markers that may be used include genes encoding dihydrofolate reductase or genes conferring neomycin resistance for eukaryotic cell culture, genes conferring tetracycline or ampicillin resistance in *E. coli,* and the *S. cerevisiae* TRP1 gene. In some embodiments, the nucleic acid encoding one of the polypeptides of SEQ ID NOS: 2, 4, 6, 8, 10, 12, 14, 16, 18, 20 and 23, or fragments comprising at least 5, 10, 15, 20, 25, 30, 35, 40, 50, 75, 100, or 150 consecutive amino acids thereof is assembled in appropriate phase with a leader sequence capable of directing secretion of the translated polypeptides or fragments thereof. Optionally, the nucleic acid can encode a fusion polypeptide in which one of the polypeptide of SEQ ID NOS: 2, 4, 6, 8, 10, 12, 14, 16, 18, 20 and 23 or fragments comprising at least 5, 10, 15, 20, 25, 30, 35, 40, 50, 75, 100, or 150 consecutive amino acids thereof is fused to heterologous peptides or polypeptides, such as N-terminal identification peptides which impart desired characteristics such as increased stability or simplified purification or detection.

The appropriate DNA sequence may be inserted into the vector by a variety of procedures. In general, the DNA sequence is ligated to the desired position in the vector following digestion of the insert and the vector with appropriate restriction endonucleases. Alternatively, appropriate restriction enzyme sites can be engineered into a DNA sequence by PCR. A variety of cloning techniques are disclosed in Ausbel et al. Current Protocols in Molecular Biology, John Wiley 503 Sons, Inc. 1997 and Sambrook et al., Molecular Cloning: A Laboratory Manual 2d Ed., Cold Spring Harbour Laboratory Press, 1989. Such procedures and others are deemed to be within the scope of those skilled in the art.

The vector may be, for example, in the form of a plasmid, a viral particle, or a phage. Other vectors include derivatives of chromosomal, nonchromosomal and synthetic DNA sequences, viruses, bacterial plasmids, phage DNA, baculovirus, yeast plasmids, vectors derived from combinations of plasmids and phage DNA, viral DNA such as vaccinia, adenovirus, fowl pox virus, and pseudorabies. A variety of cloning and expression vectors for use with prokaryotic and eukaryotic hosts are described by Sambrook, et al., Molecular Cloning: A Laboratory Manual, Second Edition, Cold Spring Harbor, N.Y., (1989).

Particular bacterial vectors which may be used include the commercially available plasmids comprising genetic elements of the well known cloning vector pBR322 (ATCC 37017), pKK223-3 (Pharmacia Fine Chemicals, Uppsala, Sweden), GEM1 (Promega Biotec, Madison, WI, USA) pQE70, pQE60, pQE-9 (Qiagen), pD10, psiX174 pBluescript II KS, pNH8A, pNH16a, pNH18A, pNH46A (Stratagene), ptrc99a, pKK223-3, pKK233-3, pDR540, pRIT5 (Pharmacia), pKK232-8 and pCM7. Particular eukaryotic vectors include pSV2CAT, pOG44, pXT1, pSG (Stratagene) pSVK3, pBPV, pMSG, and pSVL (Pharmacia). However, any other vector may be used as long as it is replicable and stable in the host cell.

The host cell may be any of the host cells familiar to those skilled in the art, including prokaryotic cells or eukaryotic cells. As representative examples of appropriate hosts, there may be mentioned: bacteria cells, such as *E. coli, Streptomyces, Bacillus subtilis, Salmonella typhimurium* and various species within the genera *Pseudomonas, Streptomyces,* and *Staphylococcus,* fungal cells, such as yeast, insect cells such as *Drosophila S2* and *Spodoptera Sf9,* animal cells such as CHO, COS or Bowes melanoma, and adenoviruses. The selection of an appropriate host is within the abilities of those skilled in the art.

The vector may be introduced into the host cells using any of a variety of techniques, including electroporation transformation, transfection, transduction, viral infection, gene guns, or Ti-mediated gene transfer. Where appropriate, the engineered host cells can be cultured in conventional nutrient media modified as appropriate for activating promoters, selecting transformants or amplifying the genes of the present invention. Following transformation of a suitable host strain and growth of the host strain to an appropriate cell density, the selected promoter may be induced by appropriate means (e.g., temperature shift or chemical induction) and the cells may be cultured for an additional period to allow them to produce the desired polypeptide or fragment thereof.

Cells are typically harvested by centrifugation, disrupted by physical or chemical means, and the resulting crude extract is retained for further purification. Microbial cells employed for expression of proteins can be disrupted by any convenient method, including freeze-thaw cycling, sonication, mechanical disruption, or use of cell lysing agents. Such methods are well known to those skilled in the art. The expressed polypeptide or fragment thereof can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the polypeptide. If desired, high performance liquid chromatography (HPLC) can be employed for final purification steps. Various mammalian cell culture systems can also be employed to express recombinant protein. Examples of mammalian expression systems include the COS-7 lines of monkey kidney fibroblasts (described by Gluzman, Cell, 23:175(1981), and other cell lines capable of expressing proteins from a compatible vector, such as the C127, 3T3, CHO, HeLa and BHK cell lines.

The constructs in host cells can be used in a conventional manner to produce the gene product encoded by the recombinant sequence. Depending upon the host employed in a recombinant production procedure, the polypeptide produced by host cells containing the vector may be glycosylated or may be non-glycosylated. Polypeptides of the invention may or may not also include an initial methionine amino acid residue.

Alternatively, the polypeptides of SEQ ID NOs: 2, 4, 6, 8, 10, 12, 14, 16, 18, 20 and 23, or fragments comprising at least 5, 10, 15, 20, 25, 30, 35, 40, 50, 75, 100, or 150 consecutive amino acids thereof can be synthetically produced by conventional peptide synthesizers. In other embodiments, fragments or portions of the polynucleotides may be employed for producing the corresponding full-length polypeptide by peptide synthesis; therefore, the fragments may be employed as intermediates for producing the full-length polypeptides.

Cell-free translation systems can also be employed to produce one of the polypeptides of SEQ ID NOS: 2, 4, 6, 8, 10, 12, 14, 16, 18, 20 and 23, or fragments comprising at least 5, 10, 15, 20, 25, 30, 35, 40, 50, 75, 100, or 150 consecutive amino acids thereof using mRNAs transcribed form a DNA construct comprising a promoter operably linked to a nucleic acid encoding the polypeptide or fragment therof. In some embodiments, the DNA construct may be linearized prior to conducting an *in vitro* transcription reaction. The transcribed mRNA is then incubated with an appropriate cell-free translation extract, such as a rabbit reticulocyte extract, to produce the desired polypeptide or fragment thereof.

The present invention also relates to variants of the polypeptides of SEQ ID NOS: 2, 4, 6, 8, 10, 12, 14, 16, 18, 20 and 23, or fragments comprising at least 5, 10, 15, 20, 25, 30, 35, 40, 50, 75, 100, or 150 consecutive amino acids thereof. The term "variant" includes derivatives or analogs of these polypeptides. In particular, the variants may differ in amino acid sequence from the polypeptides of SEQ ID NOS: 2, 4, 6, 8, 10, 12, 14, 16, 18, 20 and 23, by one or more substitutions, additions, deletions, fusions and truncations, which may be present in any combination and which are useful to direct biosynthesis of dorricogin.

The variants may be naturally occurring or created *in vitro*. In particular, such variants may be created using genetic engineering techniques such as site directed mutagenesis, random chemical mutagenesis, Exonuclease III deletion procedures, and standard cloning techniques. Alternatively, such variants, fragments, analogs, or derivatives may be created using chemical synthesis or modification procedures.

Other methods of making variants are also familiar to those skilled in the art. These include procedures in which nucleic acid sequences obtained from natural isolates are modified to generate nucleic acids which encode polypeptides having characteristics which enhance their value in industrial or laboratory applications. In such procedures, a large number of variant sequences having one or more nucleotide differences with respect to the sequence obtained from the natural isolate are generated and

characterized. Preferably, these nucleotide differences result in amino acid changes with respect to the polypeptides encoded by the nucleic acids from the natural isolates. For example, variants may be created using error prone PCR. In error prone PCR, DNA amplification is performed under conditions where the fidelity of the DNA polymerase is low, such that a high rate of point mutation is obtained along the entire length of the PCR product. Error prone PCR is described in Leung, D.W., *et al*., Technique, 1:11-15 (19 89) and Caldwell, R. C. & Joyce G.F., PCR Methods Applic., 2:28-33 (1992). Variants may also be created using site directed mutagenesis to generate site-specific mutations in any cloned DNA segment of interest. Oligonucleotide mutagenesis is described in Reidhaar-Olson, J.F. & Sauer, R.T., *et al*., Science, 241:53-57 (1988). The variants of the polypeptides of SEQ ID NOS: 2, 4, 6, 8, 10, 12, 14, 16, 18, 20

and 23, may be (i) variants in which one or more of the amino acid residues of the polypeptides of SEQ ID NOS: 2, 4, 6, 8, 10, 12, 14, 16, 18, 20 and 23, 26, 28, 30, 32, 34, 36, 38, 40 and 42, are substituted with a conserved or non-conserved amino acid residue (preferably a conserved amino acid residue) and such substituted amino acid residue may or may not be one encoded by the genetic code, wherein said variants are useful to direct biosynthesis of dorrigocin.

Conservative substitutions are those that substitute a given amino acid in a polypeptide by another amino acid of like characteristics. Typically seen as conservative substitutions are the following replacements: replacements of an aliphatic amino acid such as Ala, Val, Leu and Ile with another aliphatic amino acid; replacement of a Ser with a Thr or vice versa; replacement of an acidic residue such as Asp or Glu with another acidic residue; replacement of a residue bearing an amide group, such as Asn or Gln, with another residue bearing an amide group; exchange of a basic residue such as Lys or Arg with another basic residue; and replacement of an aromatic residue such as Phe or Tyr with another aromatic residue.

Other variants are those in which one or more of the amino acid residues of the polypeptides of SEQ ID Nos: 2, 4, 6, 8, 10, 12, 14, 16, 18, 20 and 23includes a substituent group.

Still other variants are those in which the polypeptide is associated with another compound, such as a compound to increase the half-life of the polypeptide (for example, polyethylene glycol).

Additional variants are those in which additional amino acids are fused to the polypeptide, such as leader sequence, a secretory sequence, a proprotein sequence or a sequence which facilitates purification, enrichment, or stabilization of the polypeptide. In some embodiments, the fragments, derivatives and analogs retain the same biological function or activity as the polypeptides of SEQ ID NOS: 2, 4, 6, 8, 10, 12, 14, 16, 18, 20 and 23. In other embodiments, the fragment, derivative or analogue includes a fused herterologous sequence which facilitates purification, enrichment, detection, stabilization or secretion of the polypeptide that can be enzymatically cleaved, in whole or in part, away from the fragment, derivative or analogue.

Another aspect of the present invention are polypeptides or fragments thereof which have at least 70%, at least 80%, at least 85%, at least 90%, or more than 95% homology to one of the polypeptides of SEQ ID NOS: 2, 4, 6, 8, 10, 12, 14, 16, 18, 20 and 23, or a fragment comprising at least 5, 10, 15, 20, 25, 30, 35, 40, 50, 75, 100, or 150 consecutive amino acids thereof. Homology may be determined using a program, such as BLASTP version 2.2.2 with the default parameters, which aligns the polypeptides or fragments being compared and determines the extent of amino acid identity or similarity between them. It will be appreciated that amino acid "homology" includes conservative substitutions such as those described above.

The polypeptides or fragments having homology to one of the polypeptides of SEQ ID NOS: 2, 4, 6, 8, 10, 12, 14, 16, 18, 20 and 23, or a fragment comprising at least 5, 10, 15, 20, 25, 30, 35, 40, 50, 75, 100, or 150 consecutive amino acids thereof may be obtained by isolating the nucleic acids encoding them using the techniques described above.

Alternatively, the homologous polypeptides or fragments may be obtained through biochemical enrichment or purification procedures. The sequence of potentially homologous polypeptides or fragments may be determined by proteolytic digestion, gel electrophoresis and/or microsequencing. The sequence of the prospective homologous polypeptide or fragment can be compared to one of the polypeptides of SEQ ID NOS: 2, 4, 6, 8, 10, 12, 14, 16, 18, 20 and 23, or a fragment comprising at least 5, 10, 15, 20, 25, 30, 35, 40, 50, 75, 100, or 150 consecutive amino acids thereof using a program such as BLASTP version 2.2.2 with the default parameters.

The polypeptides of SEQ ID NOS: 2, 4, 6, 8, 10, 12, 14, 16, 18, 20 and 23, or fragments, derivatives or analogs thereof comprising at least 5, 10, 15, 20, 25, 30, 35, 40, 50, 75, 100, or 150 consecutive amino acids thereof invention may be used in a variety of application. For example, the polypeptides or fragments thereof may be used to biocatalyze biochemical reactions. In particular, the polypeptides of the AYTT family, namely SEQ ID NO: 4 or fragments thereof; the ACPI family, namely SEQ ID NO: 6 or fragments thereof; the AOTF family, namely SEQ ID NO: 8 or fragments thereof; the PKUN family namely SEQ ID NOS: 10, 12 and 14 or fragments thereof; the AYOA family namely SEQ ID NO: 16 or fragments, derivatives or analogs thereof may be used in any combination, *in vitro* or *in vivo,* to direct the synthesis or modification of a polyketide, in particular dorrigocin or a substructure thereof. Polypeptides of the OXRY family, namely SEQ ID NO: 18 or fragments thereof may be used, *in vitro* or *in vivo*, to catalyze oxidoreduction reactions that modify compounds that are either endogenously produced by the host, supplemented to the growth medium, or are added to a cell-free, purified or enriched preparation of OXRY polypeptide. Polypeptides of the MTFA family, namely SEQ ID NO: 20 or fragments, derivatives or analogs thereof may be used, *in vitro* or *in vivo*, to catalyze methylation reactions that modify compounds that are either endogenously produced by the host, supplemented to the growth medium, or are added to a cell-free, purified or enriched preparation of MTFA polypeptide. Polypeptides of the OXRC family, namely SEQ ID NO: 23 or fragments thereof may be used, *in vitro* or *in vivo,* to catalyze oxidation reactions that modify compounds that are either endogenously produced by the host, supplemented to the growth medium, or are added to a cell-free, purified or enriched preparation of OXRC polypeptide.

The polypeptides of SEQ ID NOS: 2, 4, 6, 8, 10, 12, 14, 16, 18, 20 and 23, or fragments thereof comprising at least 5, 10, 15, 20, 25, 30, 35, 40, 50, 75, 100, or 150 consecutive amino acids thereof, may also be used to generate antibodies which bind specifically to the polypeptides or fragments, derivatives or analogues. The antibodies generated from SEQ ID NOS: 2, 4, 6, 8, 10, 12, 14, 16, 18, 20, and 23, may be used to determine whether a biological sample contains *Streptomyces platensis* subsp. *rosaceus* or a related microorganism. In such procedures, a biological sample is contacted with an antibody capable of specifically binding to one of the polypeptides of SEQ ID NOS: 2, 4, 6, 8, 10, 12, 14, 16, 18, 20 and 23, or fragments comprising at least 5, 10, 15, 20, 25, 30, 35, 40, 50, 75, 100, or 150 consecutive amino acids thereof. The ability of the biological sample to bind to the antibody is then determined. For example, binding may be determined by labeling the antibody with a detectable label such as a fluorescent agent, an enzymatic label, or a radioisotope. Alternatively, binding of the antibody to the sample may be detected using a secondary antibody having such a detectable label thereon. A variety of assay protocols which may be used to detect the presence of *Streptomyces platensis* subsp. *rosaceus* or *Streptomyces amphibiosporus* or of polypeptides related to SEQ ID NOS: 2, 4, 6, 8, 10, 12, 14, 16, 18, 20 and 23, in a sample are familiar to those skilled in the art. Particular assays include ELISA assays, sandwich assays, radioimmunoassays, and Western Blots. Alternatively, antibodies generated from SEQ ID NOS: 2, 4, 6, 8, 10, 12, 14, 16, 18, 20 and 23, may be used to determine whether a biological sample contains related polypeptides that may be involved in the biosynthesis of natural products of the polyketide class or other classes that are characteristically partly polyketide in nature.

Polyclonal antibodies generated against the polypeptides of SEQ ID NOS: 2, 4, 6, 8, 10, 12, 14, 16, 18, 20 and 23, or fragments comprising at least 5, 10, 15, 20, 25, 30, 35, 40, 50, 75, 100, or 150 consecutive amino acids thereof can be obtained by direct injection of the polypeptides into an animal or by administering the polypeptides to an animal, preferably a nonhuman. The antibody so obtained will then bind the polypeptide itself. In this manner, even a sequence encoding only a fragment of the polypeptide can be used to generate antibodies which may bind to the whole native polypeptide. Such antibodies can then be used to isolate the polypeptide from cells expressing that polypeptide.

For preparation of monoclonal antibodies, any technique which provides antibodies produced by continuous cell line cultures can be used. Examples include the hybridoma technique (Kholer and Milstein, 1975, Nature, 256: 495-497), the trioma technique, the human B-cell hybridoma technique (Kozbor et al., 1983, Immunology Today 4:72), and the EBV-hybridoma technique (Cole, et al., 1985, in Monoclonal Antibodies and Cancer Therapy, Alan R. Liss, Inc., pp. 77-96).

Techniques described for the production of single chain antibodies (U.S. Patent 4,946,778) can be adapted to produce single chain antibodies to the polypeptides of SEQ ID NOS: 2, 4, 6, 8, 10, 12, 14, 16, 18, 20 and 23, or fragments comprising at least 5, 10, 15, 20, 25, 30, 35, 40, 50, 75, 100, or 150 consecutive amino acids thereof. Alternatively, transgenic mice may be used to express humanized antibodies to these polypeptides or fragments thereof.

Antibodies generated against the polypeptides of SEQ ID NOS: 2, 4, 6, 8, 10, 12, 14, 16, 18, 20 and 23, or fragments comprising at least 5, 10, 15, 20, 25, 30, 35, 40, 50, 75, 100, or 150 consecutive amino acids thereof may be used in screening for similar polypeptides from a sample containing organisms or cell-free extracts thereof. In such techniques, polypeptides from the sample is contacted with the antibodies and those polypeptides which specifically bind the antibody are detected. Any of the procedures described above may be used to detect antibody binding. One such screening assay is described in "Methods for measuring Cellulase Activities", Methods in Enzymology, Vol 160, pp. 87-116.

As used herein, the term "nucleic acid codes of SEQ ID NOS: 3, 5, 7, 9, 11, 13, 15, 17, 19, 21 and 24" encompass the nucleotide sequences of SEQ ID NOS: 3, 5, 7, 9, 11, 13, 15, 17, 19, 21 and 24, fragments of SEQ ID NOS: 3, 5, 7, 9, 11, 13, 15, 17, 19, 21 and 24, nucleotide sequences homologous to SEQ ID NOS: 3, 5, 7, 9, 11, 13, 15, 17, 19, 21 and 24, or homologous to fragments of SEQ ID NOS: 3, 5, 7, 9, 11, 13, 15, 17, 19, 21 and 24, and sequences complementary to all of the preceding sequences. The fragments include portions of SEQ ID NOS: 3, 5, 7, 9, 11, 13, 15, 17, 19, 21 and 24, comprising at least 10, 15, 20, 25, 30, 35, 40, 50, 75, 100, 150, 200, 300, 400 or 500 consecutive nucleotides of SEQ ID NOS: 3, 5, 7, 9, 11, 13, 15, 17, 19, 21 and 24. Preferably, the fragments are novel fragments. Homologous sequences and fragments of SEQ ID NOS: 3, 5, 7, 9, 11, 13, 15, 17, 19, 21 and 24 refer to a sequence having at least 99%, 98%, 97%, 96%, 95%, 90%, 80%, 75% or 70% homology to these sequences. Homology may be determined using any of the computer programs and parameters described herein, including BLASTN and TBLASTX with the default parameters. Homologous sequences also include RNA sequences in which uridines replace the thymines in the nucleic acid codes of SEQ ID NOS: 3, 5, 7, 9, 11, 13, 15, 17, 19, 21 and 24. The homologous sequences may be obtained using any of the procedures described herein or may result from the correction of a sequencing error. It will be appreciated that the nucleic acid codes of SEQ ID NOS: 3, 5, 7, 9, 11, 13, 15, 17, 19, 21 and 24 can be represented in the traditional single character format in which G, A, T and C denote the guanine, adenine, thymine and cytosine bases of the deoxyribonucleic acid (DNA) sequence respectively, or in which G, A, U and C denote the guanine adenine, uracil and cytosine bases of the ribonucleic acid (RNA) sequence (see the inside back cover of Stryer, *Biochemistry,* 3$^{rd}$ edition, W. H. Freeman & Co., New York) or in any other format which records the identity of the nucleotides in a sequence.

"Polypeptide codes of SEQ ID NOS: 2, 4, 6, 8, 10, 12, 14, 16, 18, 20 and 23" encompass the polypeptide sequences of SEQ ID NOS: 2, 4, 6, 8, 10, 12, 14, 16, 18, 20 and 23 which are encoded by the cDNAs of SEQ ID NOS: 3, 5, 7, 9, 11, 13, 15, 17, 19, 21 and 24, polypeptide sequences homologous to the polypeptides of SEQ ID NOS: 2, 4, 6, 8, 10, 12, 14, 16, 18, 20 and 23, or fragments of any of the preceding sequences. Homologous polypeptide sequences refer to a polypeptide sequence having at least 99%, 98%, 97%, 96%, 95%, 90%, 85%, 80%, 75% or 70% homology to one of the polypeptide sequences of SEQ ID NOS: 2, 4, 6, 8, 10, 12, 14, 16, 18, 20 and 23. Polypeptide sequence homology may be determined using any of the computer programs and parameters described herein, including BLASTP version 2.2.2 with the default parameters or with any user-specified parameters. The homologous sequences may be obtained using any of the procedures described herein or may result from the correction of a sequencing error. The polypeptide fragments comprise at least 5, 10, 15, 20, 25, 30, 35, 40, 50, 75, 100 or 150 consecutive amino acids of the polypeptides of SEQ ID NOS: 2, 4, 6, 8, 10, 12, 14, 16, 18, 20 and 23. Preferably the fragments are novel fragments. It will be appreciated that the polypeptide codes of the SEQ ID NOS: 3, 5, 7, 9, 11, 13, 15, 17, 19, 21 and 24 can be represented in the traditional single character format or three letter format (see the inside back cover of Stryer, *Biochemistry,* 3$^{rd}$ edition, W.H. Freeman & Co., New York) or in any other format which relates the identity of the polypeptides in a sequence.

It will be readily appreciated by those skilled in the art that the nucleic acid codes of SEQ ID NOS: 3, 5, 7, 9, 11, 13, 15, 17, 19, 21 and 24 and polypeptides codes of SEQ ID NOS: 2, 4, 6, 8, 10, 12, 14, 16, 18, 20 and 23 can be stored, recorded and manipulated on any medium which can be read and accessed by a computer. As used herein, the words "recorded" and "stored" refer to a process for storing information on a computer medium. A skilled artisan can readily adopt any of the presently known methods for recording information on a computer readable medium to generate manufactures comprising one or more of the nucleic acid codes of SEQ ID NOS: 3, 5, 7, 9, 11, 13, 15, 17, 19, 21 and 24, one or more of the polypeptide codes of SEQ ID NOS: 2, 4, 6, 8, 10, 12, 14, 16, 18, 20 and 23.

Another embodiment of the present invention is a computer readable medium having stored thereon a sequence selected from the group consisting of a nucleic acid code of SEQ ID NOS: 3, 5, 7, 9, 11, 13, 15, 17, 19, 21 and 24 and a polypeptide code of SEQ ID NOS: 2, 4, 6, 8, 10, 12, 14, 16, 18, 20 and 23. Another aspect of the present invention is a computer readable medium having recorded thereon one or more nucleic acid codes of SEQ ID NOS: 3, 5, 7, 9, 11, 13, 15, 17, 19, 21 and 24, preferably at least 2, 5, 10, 15, or 20 nucleic acid codes of SEQ ID NOS: 3, 5, 7, 9, 11, 13, 15, 17, 19, 21 and 24. Another aspect of the invention is a computer readable medium having recorded thereon one or more of the polypeptide codes of SEQ ID NOS: 2, 4, 6, 8, 10, 12, 14, 16, 18, 20 and 23, preferably at least 2, 5, 10, 15 or 20 polypeptide codes of SEQ ID NOS: 2, 4, 6, 8, 10, 12, 14, 16, 18, 20 and 23.

Another embodiment of the present invention is a computer system comprising a processor and a data storage device wherein said data storage device has stored thereon a reference sequence selected from the group consisting of a nucleic acid code of SEQ ID NOS: 3, 5, 7, 9, 11, 13, 15, 17, 19, 21 and 24 and a polypeptide code of SEQ ID NOS: 2, 4, 6, 8, 10, 12, 14, 16, 18, 20 and 23.

Computer readable media include magnetically readable media, optically readable media, electronically readable media and magnetic/optical media. For example, the computer readable media may be a hard disk, a floppy disk, a magnetic tape, CD-ROM, Digital Versatile Disk (DVD), Random Access Memory (RAM), or Read Only Memory (ROM) as well as other types of media known to those skilled in the art. The present invention will be further described with reference to the following examples; however, it is to be understood that the present invention is not limited to such examples.

EXAMPLE 1: Identification and sequencing of the dorrigocin biosynthetic gene cluster

*Streptomyces platensis* subsp. *rosaceus* strain AB1981 F-75 (NRRL 18993) was obtained from the Agricultural Research Service collection (National Center for Agricultural Utilization Research, 1815 N. University Street, Peoria, Illinois 61604) and cultured using standard microbiological techniques (Kieser et al., supra). This organism was propagated on oatmeal agar medium at 28 degrees Celsius for several days. For isolation of high molecular weight genomic DNA, cell mass from three freshly grown, near confluent 100 mm petri dishes was used. The cell mass was collected by gentle scraping with a plastic spatula. Residual agar medium was removed by repeated washes with STE buffer (75 mM NaCl; 20 mM Tris-HCl, pH 8.0; 25 mM EDTA). High molecular weight DNA was isolated by established protocols (Kieser *et al*. supra) and its integrity was verified by field inversion gel electrophoresis (FIGE) using the preset program number 6 of the FIGE MAPPER™ power supply (BIORAD). This high molecular weight genomic DNA serves for the preparation of a small size fragment genomic sampling library (GSL), *i.e.*, the small insert library, as well as a large size fragment cluster identification library (CIL), *i.e.*, the large insert library. Both libraries contained randomly generated *S. platensis* genomic DNA fragments and, therefore, are representative of the entire genome of this organism.

For the generation of the *S. platensis* GSL library, genomic DNA was randomly sheared by sonication. DNA fragments having a size range between 1.5 and 3 kb were fractionated on a agarose gel and isolated using standard molecular biology techniques (Sambrook et al., *supra*). The ends of the obtained DNA fragments were repaired using T4 DNA polymerase (Roche) as described by the supplier. This enzyme creates DNA fragments with blunt ends that can be subsequently cloned into an appropriate vector. The repaired DNA fragments were subcloned into a derivative of pBluescript SK+ vector (Stratagene) which does not allow transcription of cloned DNA fragments. This vector was selected as it contains a convenient polylinker region surrounded by sequences corresponding to universal sequencing primers such as T3, T7, SK, and KS (Stratagene). The unique *Eco*RV restriction site found in the polylinker region was used as it allows insertion of blunt-end DNA fragments. Ligation of the inserts, use of the ligation products to transform *E. coli* DH10B (Invitrogen) host and selection for recombinant clones were performed as previously described (Sambrook et al., *supra*). Plasmid DNA carrying the *S. platensis* genomic DNA fragments was extracted by the alkaline lysis method (Sambrook et al., *supra*) and the insert size of 1.5 to 3 kb was confirmed by electrophoresis on agarose gels. Using this procedure, a library of small size random genomic DNA fragments is generated that covers the entire genome of the studied microorganism. The number of individual clones that can be generated is infinite but only a small number is further analyzed to sample the microorganism's genome.

A CIL library was constructed from the *S. platensis* high molecular weight genomic DNA using the SuperCos-1 cosmid vector (Stratagene™). The cosmid arms were prepared as specified by the manufacturer. The high molecular weight DNA was subjected to partial digestion at 37 degrees Celsius with approximately one unit of *Sau*3AI restriction enzyme (New England Biolabs) per 100 micrograms of DNA in the buffer supplied by the manufacturer. This enzyme generates random fragments of DNA ranging from the initial undigested size of the DNA to short fragments of which the length is dependent upon the frequency of the enzyme DNA recognition site in the genome and the extent of the DNA digestion. At various timepoints, aliquots of the digestion were transferred to new microfuge tubes and the enzyme was inactivated by adding a final concentration of 10 mM EDTA and 0.1% SDS. Aliquots judged by FIGE analysis to contain a significant fraction of DNA in the desired size range (30-50kb) were pooled, extracted with phenol/chloroform (1:1 vol:vol), and pelletted by ethanol precipitation. The 5' ends of *Sau*3AI DNA fragments were dephosphorylated using alkaline phosphatase (Roche) according to the manufacturer's specifications at 37 degrees Celcius for 30 min. The phosphatase was heat inactivated at 70 degrees Celcius for 10 min and the DNA was extracted with phenol/chloroform (1:1 vol:vol), pelletted by ethanol precipitation, and resuspended in sterile water. The dephosphorylated *Sau*3AI DNA fragments were then ligated overnight at room temperature to the SuperCos-1 cosmid arms in a reaction containing approximately four-fold molar excess SuperCos-1 cosmid arms. The ligation products were packaged using Gigapack® III XL packaging extracts (Stratagene™) according to the manufacturer's specifications. The CIL library consisted of 864 isolated cosmid clones in *E. coli* DH10B (Invitrogen). These clones were picked and inoculated into nine 96-well microtiter plates containing LB broth (per liter of water: 10.0 g NaCl; 10.0 g tryptone; 5.0 g yeast extract) which were grown overnight and then adjusted to contain a final concentration of 25% glycerol. These microtiter plates were stored at -80 degrees Celcius and served as glycerol stocks of the CIL library. Duplicate microtiter plates were arrayed onto nylon membranes as follows. Cultures grown on microtiter plates were concentrated by pelleting and resuspending in a small volume of LB broth. A 3 X 3 96-pin grid was spotted onto nylon membranes. These membranes representing the complete CIL library were then layered onto LB agar and incubated ovenight at 37 degrees Celcius to allow the colonies to grow. The membranes were layered onto filter paper pre-soaked with 0.5 N NaOH/1.5 M NaCl for 10 min to denature the DNA and then neutralized by transferring onto filter paper pre-soaked with 0.5 M Tris (pH 8)/1.5 M NaCl for 10 min. Cell debris was gently scraped off with a plastic spatula and the DNA was crosslinked onto the membranes by UV irradiation using a GS GENE LINKER™ UV Chamber (BIORAD). Considering an average size of 8 Mb for an actinomycete genome and an average size of 35 kb of genomic insert in the CIL library, this library represents roughly a 4-fold coverage of the microorganism's entire genome.

The GSL library was analyzed by sequence determination of the cloned genomic DNA inserts. The universal primers KS or T7, referred to as forward (F) primers, were used to initiate polymerization of labeled DNA. Extension of at least 700 bp from the priming site can be routinely achieved using the TF, BDT v2.0 sequencing kit as specified by the supplier (Applied Biosystems). Sequence analysis of the small genomic DNA fragments (Genomic Sequence Tags, GSTs) was performed using a 3700 ABI capillary electrophoresis DNA sequencer (Applied Biosystems). The average length of the DNA sequence reads was ~700 bp. Further analysis of the obtained GSTs was performed by sequence homology comparison to various protein sequence databases. The DNA sequences of the obtained GSTs were translated into amino acid sequences and compared to the National Center for Biotechnology Information (NCBI) nonredundant protein database and the DECIPHER® database (Ecopia Biosciences Inc., St.-Laurent, QC, canada) using previously described algorithms (Altschul et al., *supra*). Sequence similarity with known proteins of defined function in the database enables one to make predictions on the function of the partial protein that is encoded by the translated GST.

A total of 1536 *S. platensis* GSTs were generated and analyzed by sequence comparison using the Blast algorithm (Altschul et al., *supra*). Sequence alignments displaying an E value of at least e-5 were considered as significantly homologous and retained for further evaluation. GSTs showing similarity to a gene of interest can be at this point selected and used to identify larger segments of genomic DNA from the CIL library that include the gene(s) of interest. As dorrigocins and migrastatin are polyketides, several *S. platensis* GSTs that were clearly portions of type I PKS genes were pursued. Using these type I PKS GSTs, we indeed identified a type I PKS locus in *S. platensis*, however, the PKS domain order and number of modules of this type I PKS was inconsistent with the structures of dorrigocins and migrastatin (data not shown). In addition to the GSTs that were clearly portions of type I PKS genes, we also identified GSTs that were somewhat related to type I PKS genes. When the latter were used as probes to screen the CIL library and the resulting cosmid clones were sequenced, an unusual PKS gene cluster was identified which proved to be the dorrigocin biosynthetic locus.

Hybridization oligonucleotide probes were radiolabeled with $P^{32}$ using T4 polynucleotide kinase (New England Biolabs) in 15 microliter reactions containing 5 picomoles of oligonucleotide and 6.6 picomoles of [$\gamma$-$P^{32}$]ATP in the kinase reaction buffer supplied by the manufacturer. After 1 hour at 37 degrees Celcius, the kinase reaction was terminated by the addition of EDTA to a final concentration of 5 mM. The specific activity of the radiolabeled oligonucleotide probes was estimated using a Model 3 Geiger counter (Ludlum Measurements Inc., Sweetwater, Texas) with a built-in integrator feature. The radiolabeled oligonucleotide probes were heat-denatured by incubation at 85 degrees Celcius for 10 minutes and quick-cooled in an ice bath immediately prior to use.

The *S. platensis* CIL library membranes were pretreated by incubation for at least 2 hours at 42 degrees Celcius in Prehyb Solution (6X SSC; 20mM $NaH_2PO_4$; 5X Denhardt's; 0.4% SDS; 0.1 mg/ml sonicated, denatured salmon sperm DNA) using a hybridization oven with gentle rotation. The membranes were then placed in Hyb Solution (6X SSC; 20mM $NaH_2PO_4$; 0.4% SDS; 0.1 mg/ml sonicated, denatured salmon sperm DNA) containing $1X10^6$ cpm/ml of radiolabeled oligonucleotide probe and incubated overnight at 42 degrees Celcius using a hybridization oven with gentle rotation. The next day, the membranes were washed with Wash Buffer (6X SSC, 0.1% SDS) for 45 minutes each at 46, 48, and 50 degrees Celcius using a hybridization oven with gentle rotation. The *S. platensis* CIL membranes were then exposed to X-ray film to visualize and identify the positive cosmid clones. Positive clones were identified, cosmid DNA was extracted from 30 ml cultures using the alkaline lysis method (Sambrook et al., *supra*) and the inserts were entirely sequenced using a shotgun sequencing approach (Fleischmann et al., *Science*, 269:496-512).

Sequencing reads were assembled using the Phred-Phrap™ algorithm (University of Washington, Seattle, USA) recreating the entire DNA sequence of the cosmid insert. Reiterations of hybridizations of the CIL library with probes derived from the ends of the original cosmid allow indefinite extension of sequence information on both sides of the original cosmid sequence until the complete sought-after gene cluster is obtained. The structure of dorrigocin suggests that it would be synthesized by a modular type I polyketide synthases (PKSs) containing 10 modules. Three overlapping cosmid clones that were detected by the oligonucleotide probe derived from the GSTs remotely related to type I PKSs have been completely sequenced to provide approximately 54 Kb of DNA comprising the dorrigocin biosynthetic locus (Figure 1).

Example 2: Genes and proteins involved in biosynthesis of dorrigocin

The dorrigocin locus encodes 11 proteins and spans approximately 53,800 base pairs of DNA that is contiguous except for one gap beginning after base pair 52,101. More than 15 kilobases of DNA sequence were analyzed on each side of the dorrigocin locus and these regions contain primary metabolic genes. The order and relative position of the 11 open reading frames representing the proteins of the biosynthetic locus for dorrigocin (DORR ORFs) are provided in Figure 1. The top line in Figure 1 provides a scale in kilobase pairs. The black bars depict the two DNA contigs separated by a small gap (<100bp) in the sequencing. The arrows represent the 11 open reading frames of the dorrigocin biosynthetic locus.

Thus, the complete locus of genes regulating the biosynthesis of dorrigocin is formed by two DNA contiguous sequences (SEQ ID NOS: 1 and 22). The contiguous nucleotide sequences are arranged such that, as found within the dorrigocin biosynthetic locus, the 52101 base pairs of DNA contig 1 (SEQ ID NO: 1) is found adjacent to the 5' end of DNA contig 2 (SEQ ID NO: 22). The contiguous nucleotide sequence of SEQ ID NO: 1 contains the 10 open reading frames (ORFs) listed in SEQ ID NOS: 2, 4, 6, 8, 10, 12, 14, 16, 18, 20. DORR ORF 1 (SEQ ID NO: 2) is the 1217 amino acids deduced from the nucleic acid sequence of SEQ ID NO: 3 which is drawn from residues 3720 to 67 (anti sense strand) of contig 1 (SEQ ID NO: 1). DORR ORF 2 (SEQ ID NO: 4) is the 529 amino acids deduced from the nucleic acid sequence of SEQ ID NO: 5 which is drawn from residues 4092 to 5681 (sense strand) of contig 1 (SEQ ID NO: 1). DORR ORF 3 (SEQ ID NO: 6) is the 83 amino acids deduced from the nucleic acid sequence of SEQ ID NO: 7 which is drawn from residues 5767 to 6018 (sense strand) of contig 1 (SEQ ID NO: 1). DORR ORF 4 (SEQ ID NO: 8) is the 656 amino acids deduced from the nucleic acid sequence of SEQ ID NO: 9 which is drawn from residues 6023 to 7993 (sense strand) of contig 1 (SEQ ID NO: 1). DORR ORF 5 (SEQ ID NO: 10) is the 3192 amino acids deduced from the nucleic acid sequence of SEQ ID NO: 11 which is drawn from residues 8009 to 17587 (sense strand) of contig 1 (SEQ ID NO: 1). DORR ORF 6 (SEQ ID NO: 12) is the 8026 amino acids deduced from the nucleic acid sequence of SEQ ID NO: 13 which is drawn from residues 17634 to 41714 (sense strand) of contig 1 (SEQ ID NO: 1). DORR ORF 7 (SEQ ID NO: 14) is the 1953 amino acids deduced from the nucleic acid sequence of SEQ ID NO: 15 which is drawn from residues 41772 to 47633 (sense strand) of contig 1 (SEQ ID NO: 1). DORR ORF 8 (SEQ ID NO:

16) is the 751 amino acids deduced from the nucleic acid sequence of SEQ ID NO: 17 which is drawn from residues 47635 to 49890 (sense strand) of contig 1 (SEQ ID NO: 1). DORR ORF 9 (SEQ ID NO: 18) is the 338 amino acids deduced from the nucleic acid sequence of SEQ ID NO: 19 which is drawn from residues 49922 to 50938 (sense strand) of contig 1 (SEQ ID NO: 1). DORR ORF 10 (SEQ ID NO: 20) is the 281 amino acids deduced from the nucleic acid sequence of SEQ ID NO: 21 which is drawn from residues 51234 to 52079 (sense strand) of contig 1 (SEQ ID NO: 1). The contiguous nucleotide sequence of SEQ ID NO: 22 (1700 base pair) contains DORR ORF 11 (SEQ ID NO: 23). DORR ORF 11 (SEQ ID NO: 23) is the 328 amino acids representing the C-terminus of the expected polypeptide and deduced from the nucleic acid sequence of SEQ ID NO: 24 which is drawn from residues 163 to 1149 (sense strand) of contig 2 (SEQ ID NO: 22).

Two deposits, namely *E. coli* DH10B (088CF) strain and *E. coli* DH10B (088CX) strain each harbouring a cosmid clone of a partial biosynthetic locus for dorrigocin have been deposited with the International Depositary Authority of Canada, Bureau of Microbiology, Health Canada, 1015 Arlington Street, Winnipeg, Manitoba, Canada R3E 3R2 on February 27, 2001 and were assigned deposit accession number IDAC270201-3 and 270101-4 respectively. The *E. coli* strain deposits are referred to herein as "the deposited strains".

The deposited strains comprise the complete biosynthetic locus for dorrigocin. The sequence of the polynucleotides comprised in the deposited strains, as well as the amino acid sequence of any polypeptide encoded thereby are controlling in the event of any conflict with any description of sequences herein.

The deposit of the deposited strains has been made under the terms of the Budapest Treaty on the International Recognition of the Deposit of Micro-organisms for Purposes of Patent Procedure. The deposited strains will be irrevocably and without restriction or condition released to the public upon the issuance of a patent. The deposited strains are provided merely as convenience to those skilled in the art and are not an admission that a deposit is required for enablement, such as that required under 35 U.S.C. §112. A license may be required to make, use or sell the deposited strains, and compounds derived therefrom, and no such license is hereby granted.

In order to identify the function of the genes in the dorrigocin biosynthetic locus, DORR ORFs 1 to 11 (SEQ ID NOS: 2, 4, 6, 8, 10, 12, 14, 16, 18, 20 and 23) were compared, using the BLASTP version 2.2.2 algorithm with the default parameters, to sequences in the National Center for Biotechnology Information (NCBI) nonredundant protein database and the DECIPHER® database of microbial genes, pathways and natural products (available on a subscription basis from Ecopia BioSciences Inc. St.-Laurent, QC, Canada).

The accession numbers of the top GenBank hits of this Blast analysis are presented in Table 2 along with the corresponding E value. The E value relates the expected number of chance alignments with an alignment score at least equal to the observed alignment score. An E value of 0.00 indicates a perfect homolog. The E values are calculated as described in Altschul et al. J. Mol. Biol., October 5; 215(3) 403-10, the teachings of which is incorporated herein by reference. The E value assists in the determination of whether two sequences display sufficient similarity to justify an inference of homology.

Table 2

| ORF | Family | #aa | GenBank homology | probability | % identity | % similarity | proposed function of GenBank match |
|---|---|---|---|---|---|---|---|
| 1 | REBP | 1217 | BAB69312.1,1094aa | 0.0 | 502/784 (64.03%) | 554/784 (70.66%) | putative regulatory protein,Streptomyces avermitilis |
| | | | CAC20917.1,694aa | 0.0 | 456/685 (66.57%) | 502/685 (73.28%) | hypothetical protein,*Streptomyces natalensis* |
| | | | AAF73451.1,272aa | 1e-31 | 89/250 (35.6%) | 123/250 (49.2%) | putative activator AknO,Streptomyces galilaeus |
| 2 | AYTT | 529 | NP_389591.1,288aa | 3e-68 | 143/278 (51.44%) | 187/278 (67.27%) | pksC,Bacillus subtilis |
| | | | NP_405051.1,282aa | 8e-50 | 120/280 (42.86%) | 163/280 (58.21%) | putative acyl transferase,Yersinia pestis |
| | | | NP_484284.1,292aa | 3e-35 | 103/279 (36.92%) | 147/279 (52.69%) | malonyl co-A acyl carrier protein transacylase,Nostoc sp. |
| 3 | ACPI | 83 | NP_437899.1,88aa | 0.002 | 23/76 (30.26%) | 46/76 (60.53%) | hypothetical protein,Sinorhizobium meliloti |
| | | | AAC05776.1,79aa | 0.032 | 21/51 (41.18%) | 32/51 (62.75%) | D-alanyl carrier protein,Streptococcus mutans |
| 4 | AOTF | 656 | NP_437900.1,645aa | 1e-100 | 248/655 (37.86%) | 346/655 (52.82%) | putative asparagine synthetase,Sinorhizobium meliloti |
| | | | NP_107193.1,675aa | 1e-100 | 244/650 (37.54%) | 342/650 (52.62%) | *asparagine synthetase,Mesorhizobium loti* |
| | | | AAF34252.1,643aa | 1e-64 | 206/623 (33.07%) | 297/623 (47.67%) | putative asparagine synthetase,Desulfovibrio gigas |
| 5 | PKUN | 3192 | NP_389600.1,4427aa | 0.0 | 714/2193 (32.56%) | 1044/2193 (47.61%) | polyketide synthase of type I,Bacillus subtilis |
| | | | NP_389603.1,4930aa | 0.0 | 678/2479 (27.35%) | 1064/2479 (42.92%) | polyketide synthase of type I,Bacillus subtilis |
| | | | CAA84505.1,1763aa | 1e-117 | 289/924 (31.28%) | 438/924 (47.4%) | putative polyketide synthase,Bacillus subtilis |
| 6 | PKUN | 8026 | NP_389601.1,4273aa | 0.0 | 904/2663 (33.95%) | 1327/2663 (49.83%) | *polyketide synthase,Bacillus subtilis* |
| | | | NP_389599.1,4447aa | 0.0 | 805/2198 (36.62%) | 1150/2198 (52.32%) | polyketide synthase of type I,Bacillus subtilis |
| | | | AAK15074.1,4801aa | 0.0 | 877/2674 (32.8%) | 1215/2674 (45.44%) | albicidin PKS-NRPS,Xanthomonas albilineans |
| 7 | PKUN | 1953 | NP_389603.1,4930aa | 0.0 | 574/1546 (37.13%) | 837/1546 (54.14%) | polyketide synthase of type I,Bacillus subtilis |
| | | | NP_389600.1,4427aa | 0.0 | 483/1452 (33.26%) | 716/1452 (49.31%) | polyketide synthase of type I,Bacillus subtilis |
| | | | CAA84505.1,1763aa | 1e-154 | 332/1013 (32.77%) | 498/1013 (49.16%) | putative polyketide synthase,Bacillus subtilis |
| 8 | AYOA | 751 | NP_389593.1,650aa | 1e-148 | 285/650 (43.85%) | 391/650 (60.15%) | pksE,Bacillus subtilis |
| | | | T37055,527aa | 6e-97 | 197/450 (43.78%) | 263/450 (58.44%) | probable oxidoreductase,Streptomyces coelicolor |
| | | | T30186,543aa | 9e-93 | 177/434 (40.78%) | 261/434 (60.14%) | hypothetical protein,Shewanella sp |
| 9 | OXRY | 338 | CAB62729.1,364aa | 2e-97 | 180/334 (53.89%) | 222/334 (66.47%) | putative oxidoreductase,Streptomyces coelicolor |
| | | | NP_420823.1,341aa | 5e-77 | 158/341 (46.33%) | 200/341 (58.65%) | alcohol dehydrogenase,Caulobacter crescentus |
| | | | NP_279793.1,380aa | 6e-73 | 153/334 (45.81%) | 198/334 (59.28%) | quinone oxidoreductase,Halobacterium sp. |
| 10 | MTFA | 281 | AAD28459.1,283aa | 2e-73 | 138/262 (52.67%) | 178/262 (67.94%) | MitM,Streptomyces lavendulae |
| | | | AAG42853.1,278aa | 3e-42 | 109/262 (41.6%) | 143/262 (54.58%) | SnogM,Streptomyces nogalater |
| | | | T44579,283aa | 2e-39 | 103/268 (38.43%) | 143/268 (53.36%) | C5-O-methyltransferase,Streptomyces avermitilis |
| 11 | OXRC | 328 | CAB46536.1,410aa | 2e-86 | 165/313 (52.72%) | 218/313 (69.65%) | *NikF protein,Streptomyces tendae* |
| | | | AAL85695.1,410aa | 1e-83 | 162/313 (51.76%) | 213/313 (68.05%) | cytochrome P450,Streptomyces ansochromogenes |
| | | | AAF71771.1,398aa | 2e-79 | 159/310 (51.29%) | 202/310 (65.16%) | NysN,Streptomyces noursei |

Example 5: Unusual two component PKS system involved in biosynthesis ofdorrigocins and lactimidomycin

The dorrigocin locus encodes three PKSs that contain KS, KR, ACP and unusual DH domains in unusual arrangements. The three PKSs in this locus encode a total of 10 ketosynthase (KS) domains, sufficient to produce a polyketide chain the length of dorrigocin. The three PKSs share some features of typical type I PKSs, namely that the synthases contain multiple fused domains. However, the dorrigocin PKSs are distinct from type I PKSs in that they do not contain AT domains that are physically attached to the PKS. Instead, the AT function is provided in *trans* by distinct components.

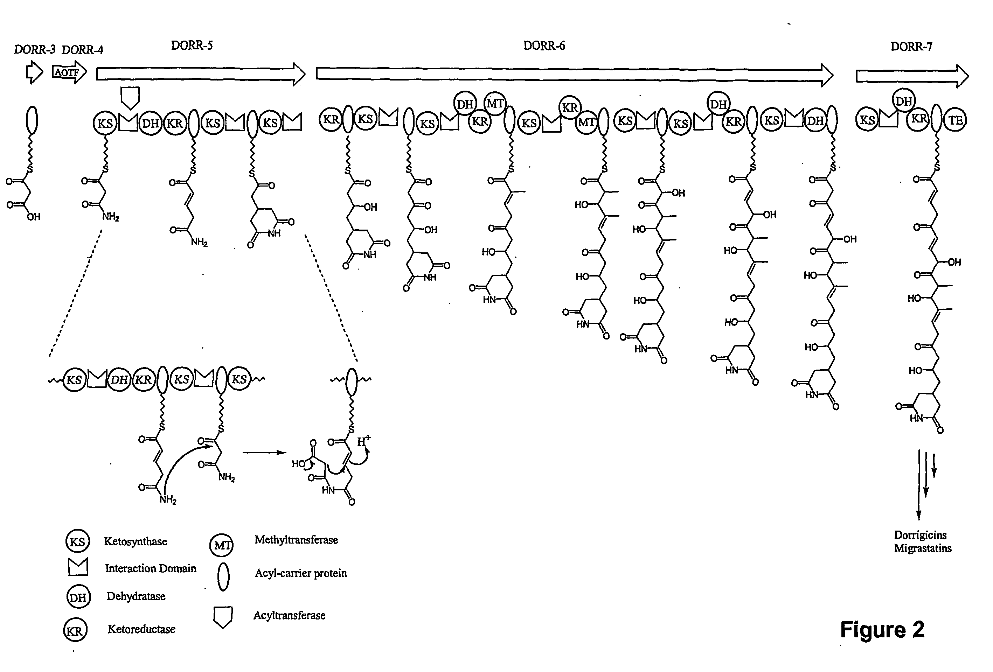

Therefore the dorrigocin PKS system represents a new, two component PKS system. Without intending to be limited to any particular mechanism of action or biosynthetic scheme, the proteins of the invention can explain the formation of dorrigocin. Figure 2 shows disposition of the 10 modules that act in a stepwise fashion to synthesize the polyketide backbone. Referring to Figure 2, DORR acyl carrier protein ACPI (SEQ ID NO: 6) and DORR amidotransferase AOTF (SEQ ID NO: 8) are translationally coupled to the first PKS of the DORR locus (SEQ ID NO: 10). The ACPI (SEQ ID NO: 6) shows most significant similarity to proteins that transfer amino-substituted acyl groups. ACPI (SEQ ID NO: 6) and AOTF (SEQ ID NO: 8) cooperate to generate the starter unit for polyketide chain extension.

Unlike typical type I PKS modules which contain an AT domain downstream of the KS domain, the KS domains in each of the PKS modules in the dorrigocin locus (SEQ ID NOS: 10, 12 and 14) are not followed by an AT domain. Nonetheless, the unusual dorrigocin PKSs contain a small conserved domain downstream of the KS domains. This conserved domain is postulated to act as a docking site for the malonyl-CoA:ACP malonyltransferase activity. The malonyl-CoA:ACP malonyltransferase activity may be provided by the AYTT DORR ORF 2 (SEQ ID NO: 4), AYOA DORR ORF 8 (SEQ ID NO: 16), or by the primary metabolic fatty acid malonyl-CoA:ACP malonyltransferase.

Module 1 carries a bound malonyl extender unit and catalyzes one round of elongation of the starter unit, followed by ketoreductase and dehydration. Module 2 is acylated by the independent AT-thioesterase fusion protein AYTT (SEQ ID NO: 4). This protein consists of a malonyl CoA:ACP malonyltransferase fused to a thioesterase. Module 2 catalyzes the formation of an imide bond between the acyl chains tethered to modules 1 and 2. The formation of an imide bond requires an unusual "backward" step in the elongation cycle, a maneuver that is facilitated by the thioesterase activity associated with the AYTT protein (SEQ ID NO: 4). The KS domain of module 1 is used again, this time to catalyze the Claisen condensation reaction that generates the cyclic glutarimide group.

The nascent polyketide chain now skips from the ACP of module 1 to the KS of module 3 for the next elongation step. Malonyl extender units are used by modules 3 to 6. Beta-ketoreduction occurs at modules 5 and 6. Methyl side chains are added by the MT domains of modules 5 and 6.

Module 7 uses a hydroxymalonyl extender. The hydroxymalonyl extender unit is generated by the independent AT-oxidoreductase fusion protein AYOA (SEQ ID NO: 16) and is transferred to module 7. The hydroxyl side chain is methylated by the MTFA O-methyltransferase (SEQ ID NO: 20).

Modules 8 to 10 use malonyl extender units. Ketoreductation and dehydration occur at modules 8 and 10. Module 9 is notable in that it contains a DH domain, but no KR domain.

The general design rules for the biosynthesis of conventional type I polyketide are applicable to the biosynthesis of the intermediate polyketide backbone structure to dorrigocin A, dorrigocin B and migrastatin molecules, as shown in Figure 2. The intermediate differs from dorrigocin B in the state of beta-carbonyl reduction and the absence of a methyl side chain at C-14. Dorrigocin PKSs (SEQ ID NOS: 10, 12, 14) recruit ketoreductase, dehydratase and enoylreductase from the primary fatty acid synthase as needed to achieve the proper oxidation states at C-5, C-9 and C-17. The two modules that require interaction with enoylreductases correspond to the two modules that span separate PKS peptides. An MT domain was not found in the module that incorporates C-14, suggesting that methylation at C-14 is catalyzed by a primary methyltransferase or the MT domain in the adjacent module.

The oxidoreductases encoded by the OXRC and OXRY proteins (SEQ ID NOS: 23 and 18) provide the necessary activities to catalyze the interconversion of dorrigocin A and dorrigocin B.

The lactimidomycin biosynthetic locus consists of 9 ORFs (SEQ ID NOS: 26, 28, 30, 32, 34, 36, 38, 40 and 42), eight of which are highly homologous to a corresponding ORF of the dorrigocin biosynthetic locus. LACT ORF 1 (SEQ ID NO: 26) is homologous to DORR ORF 2 (SEQ ID NO: 4), both of which are fusions of an acyltransferase and a thioesterase designated as AYTT. LACT ORF 2 (SEQ ID NO: 28) is homologous to DORR ORF 3 (SEQ ID NO: 6), both of which are acyl carrier proteins designated as ACPI. LACT ORF 3 (SEQ ID NO: 30) is homologous to DORR ORF 4 (SEQ ID NO: 8), both of which are amidotransferases similar to bacterial asparagine synthetases designated as AOTF. LACT ORFs 4, 5 and 6 (SEQ ID NOS: 32, 34 and 36) are homologous to DORR ORFs 5, 6, and 7 (SEQ ID NOS: 10, 12 and 14), respectively, all of which are unusual modular PKSs devoid of AT domains designated as PKUN. LACT ORF 7 (SEQ ID NO: 38) is homologous to DORR ORF 8 (SEQ ID NO: 16), both of which are fusions of an acyltransferase and an oxidoreductase designated as AYOA. Finally, LACT ORF 8 (SEQ ID NO: 40) is homologous to DORR ORF 11 (SEQ ID NO: 23), both of which are cytochrome P450 monooxygenases designated as OXRC.

LACT ORF 9 (SEQ ID NO: 42) is a phosphopantetheinyl transferase designated as PPTF for which there is no counterpart in the dorrigocin locus. This phosphopantetheinyl transferase is involved in the covalent attachment of the phosphopantetheinyl prosthetic arm to the acyl carrier proteins of the lactimidomycin synthase complex. In contrast, the acyl carrier proteins of the dorrigocin may be phosphopantetheinylated by a phosphopantetheinyl transferase encoded by a gene outside of the dorrigocin biosynthetic locus.

The dorrigocin biosynthetic locus contains three ORFs that have no counterpart in the lactimidomycin locus. DORR ORF 1 (SEQ ID NO: 2) which is a regulator designated as REBP, DORR ORF 9 (SEQ ID NO: 18) which is an oxidoreductase designated as OXRY, and DORR ORF 10 (SEQ ID NO: 20) which is an O-methyltransferase designated as MTFA. The absence of an OXRY in the lactimidomycin locus is significant as this DORR ORF 9 (SEQ ID NO: 18) is implicated in the interconversion of dorrigocins A and B involving an isomerization of a double bond. No analogous isomerization event is known to occur in the case of lactimidomycin biosynthesis, presumably due to the absence of an OXRY homologue. The absence of an MTFA in the lactimidomycin locus is significant as, unlike dorrigocins and migrastatin, lactimidomycin does not contain any O-methyl groups.

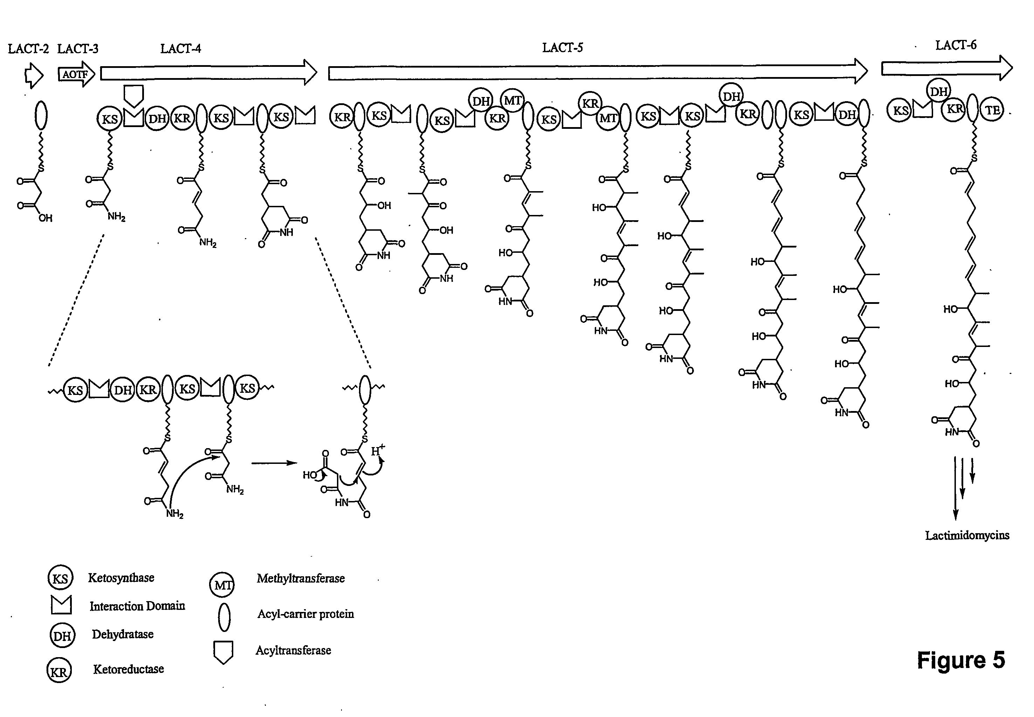

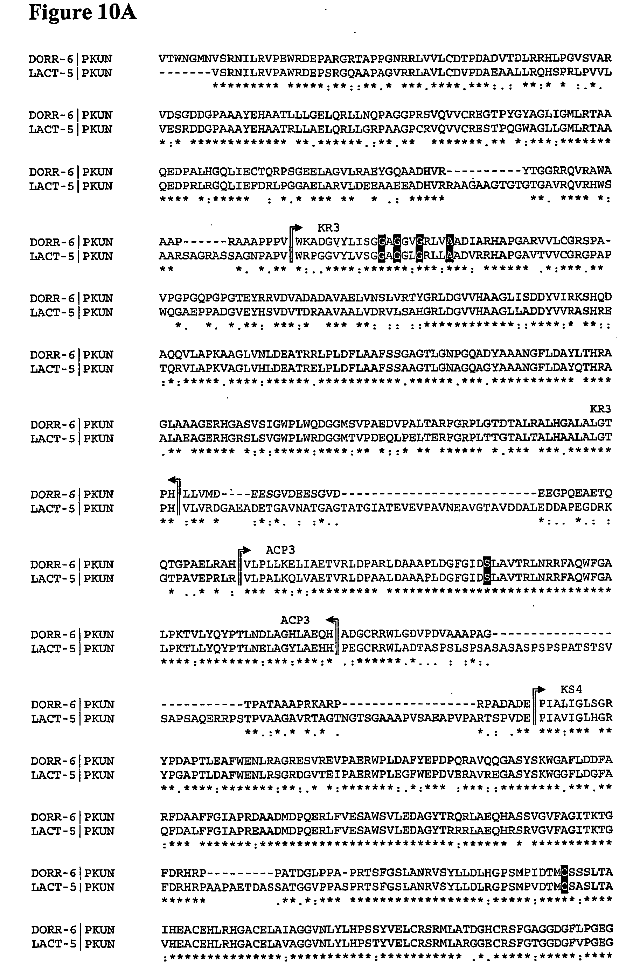

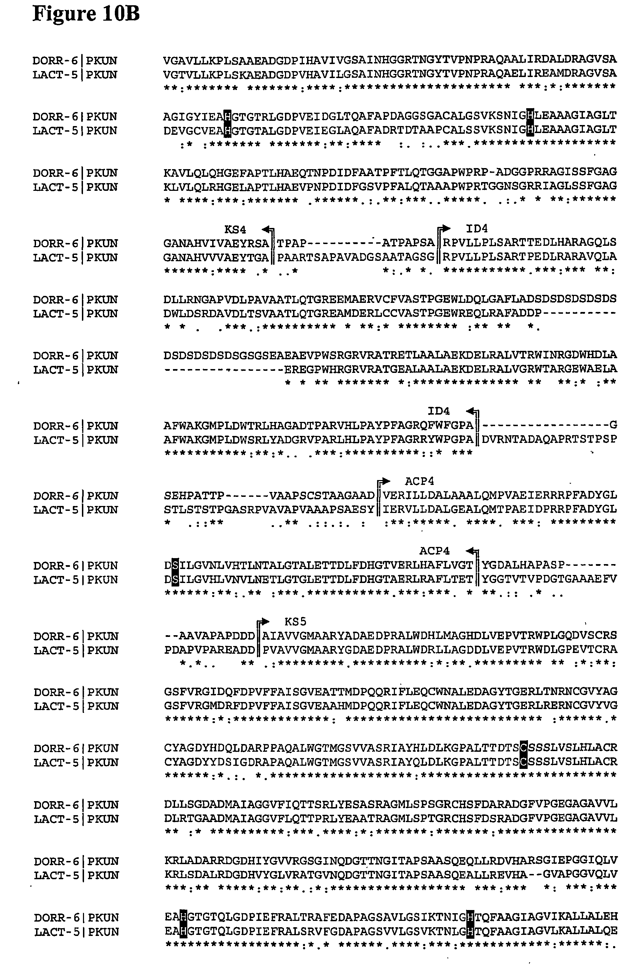

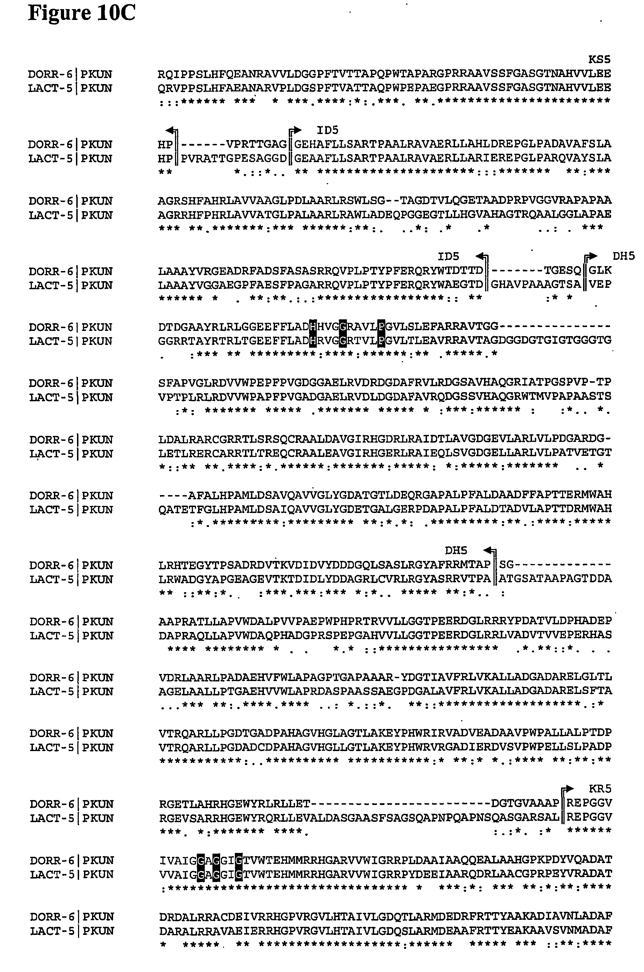

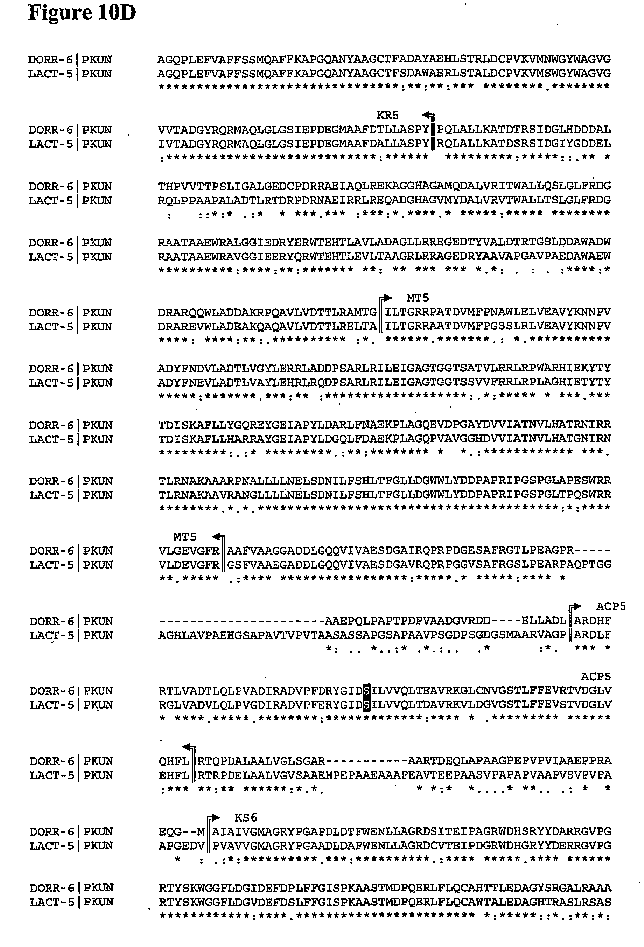

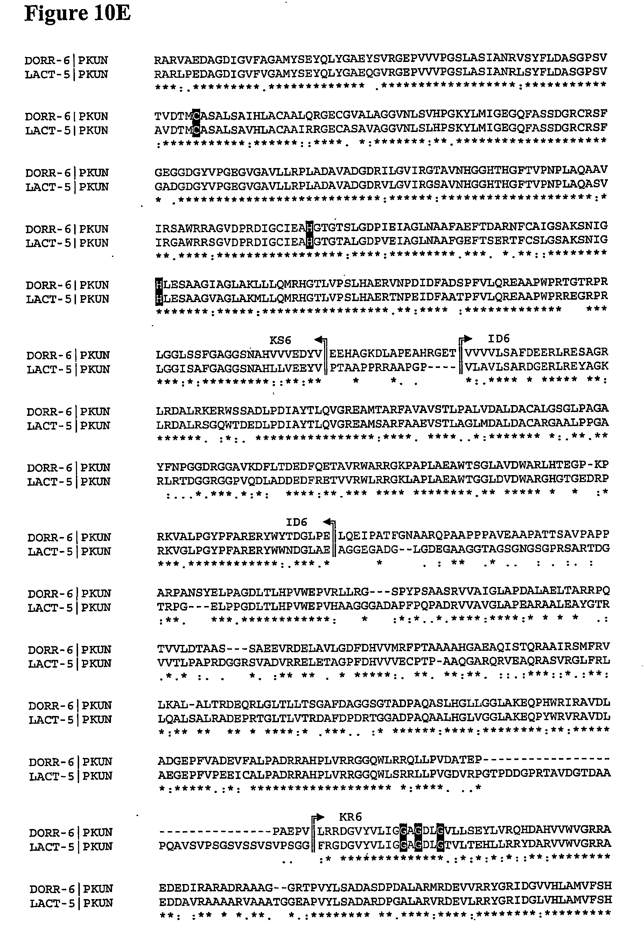

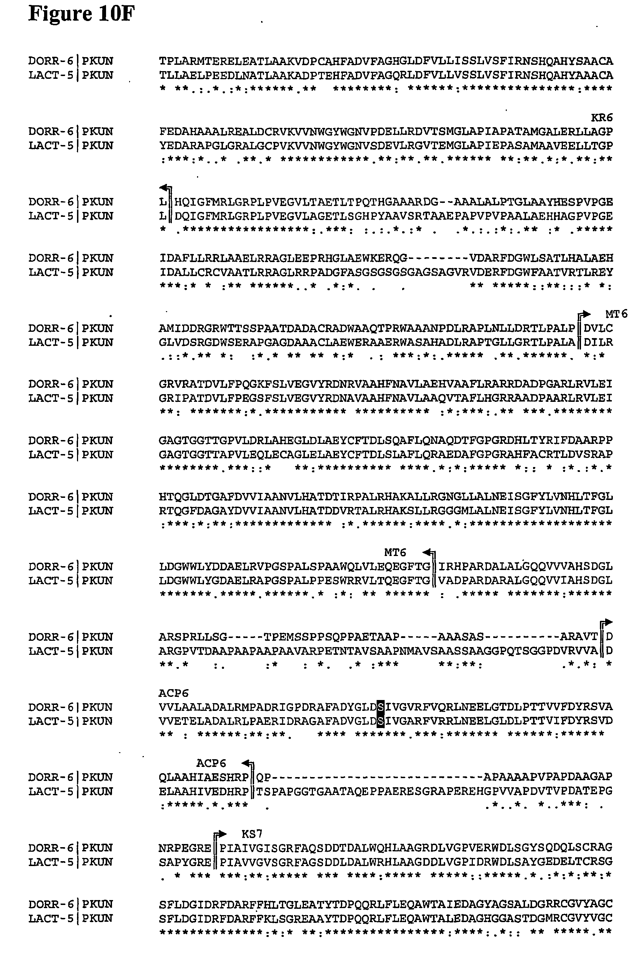

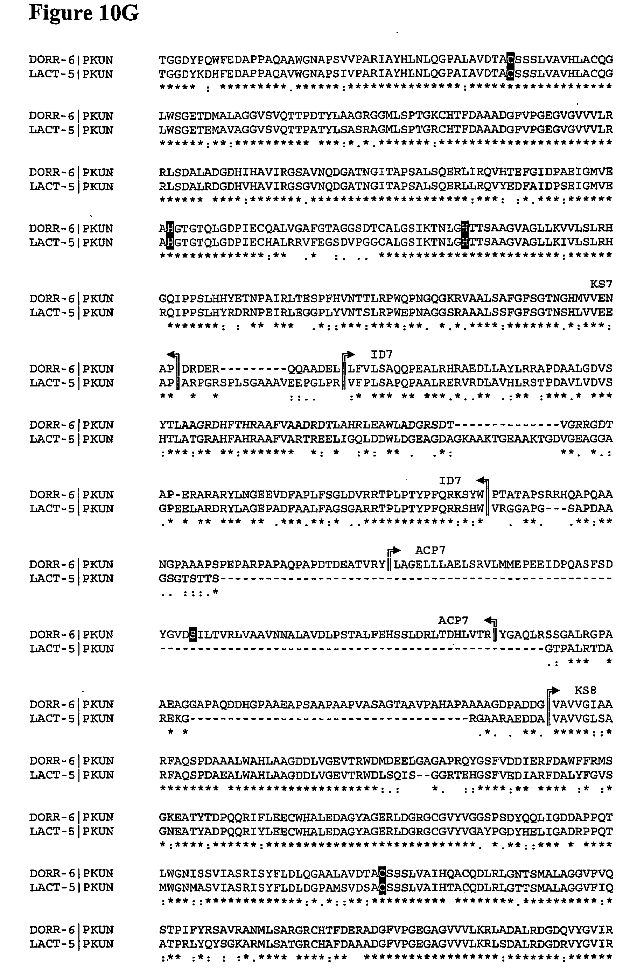

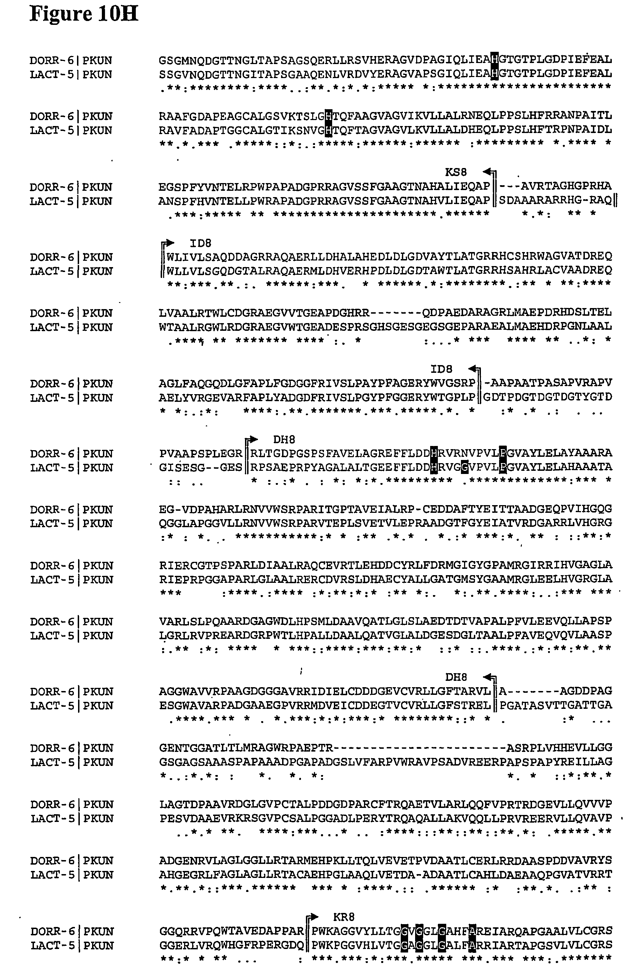

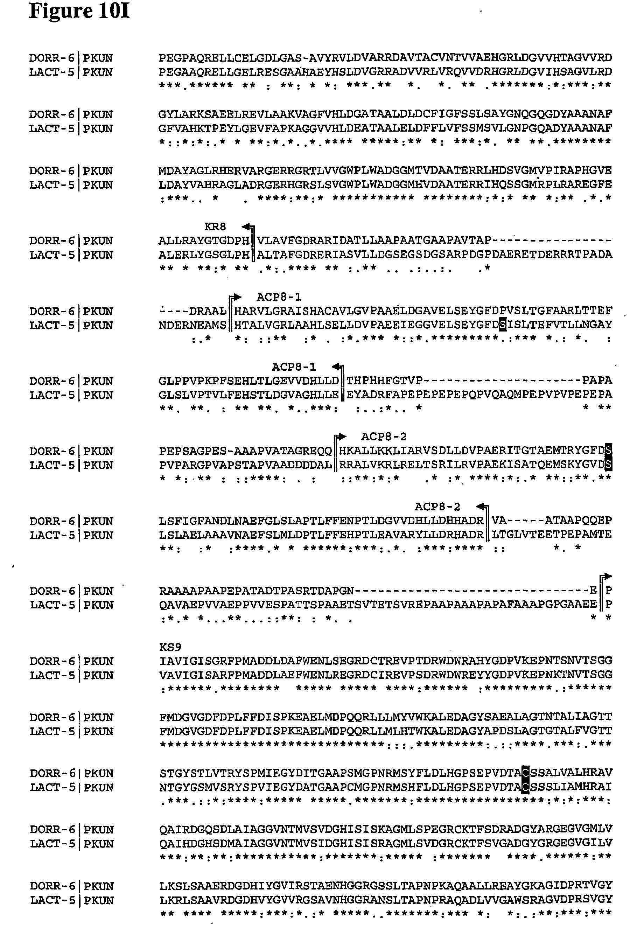

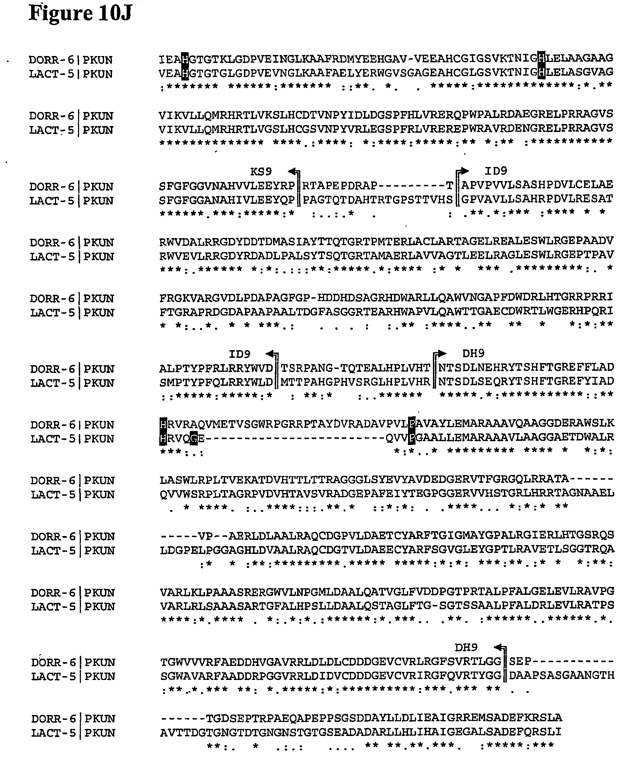

Without intending to be limited to any particular mechanism of action or biosynthetic scheme, the LACT proteins can explain the biosynthesis of lactimidomycin (Figure 5) in a manner analogous to the biosynthetic pathway for dorrigocins and migrastatin (Figure 2). The lactimidomycin and dorrigocin PKS systems differ in modules 7 and 8 of the respective PCK systems (Figure 4, 10). Module 7 in the dorrigocin PKS system comprises a KS domain, an interaction domain, and an ACP domain that, together with a *trans*-acting AT domain, are involved in the incorporation of a methoxymalonyl extender unit (or a hydroxymalonyl extender unit that is subsequently O-methylated). Conversely, module 7 in the lactimidomycin PKS system comprises only a KS domain and an interaction domain; it lacks an ACP domain. As such, it is predicted that this module cannot carry out polyketide chain elongation. Consistent with this prediction, lactimidomycin does not contain a hydroxymethyl substitution on C-8. Module 8 in the dorrigocin PKS system comprises a KS domain, an interaction domain, a DH domain, a KR domain, and two tandem ACP domains. However, the first of these ACP domains is predicted to be inactive (indicated by the 'X' in Figure 4) as the conserved serine residue that normally serves as the phosphopantetheine attachment site has been substituted by a proline residue (Figure 10). Conversely, both ACP domains contain the active site serine residues in module 8 in the lactimidomycin PKS system. Therefore, we propose that both of these ACPs are loaded with malonyl-CoA and either the KS from module 7 or the KS from module 8 catalyzes two rounds of polyketide chain elongation or, alternatively, the KS domains from module 7 and 8 each catalyze one round of polyketide chain elongation.

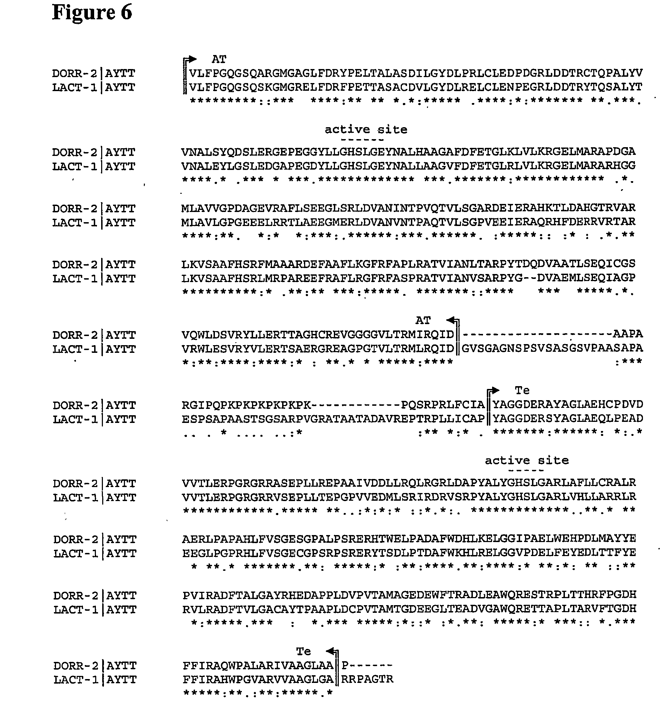

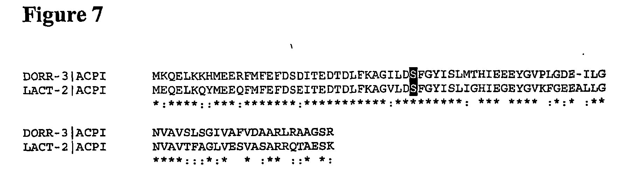

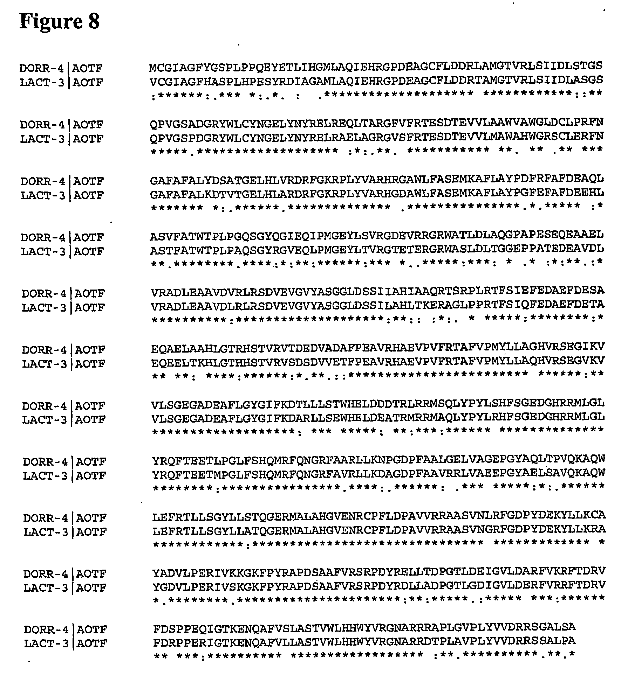

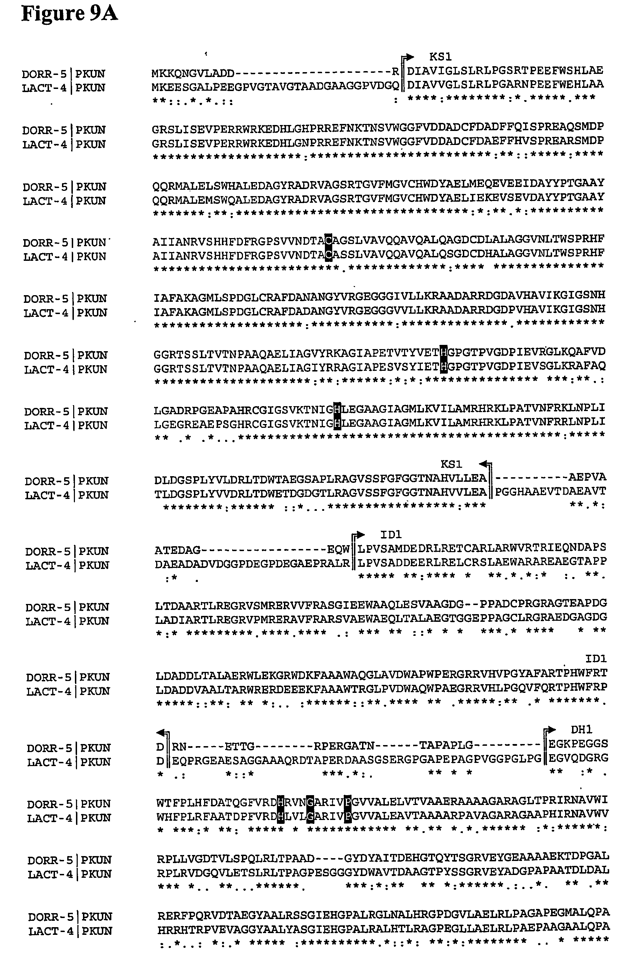

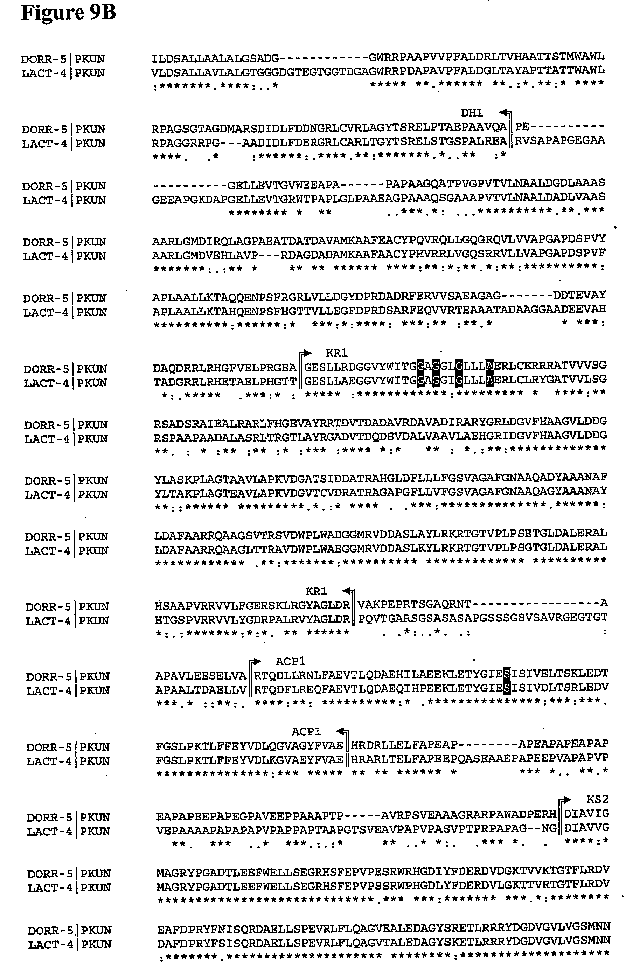

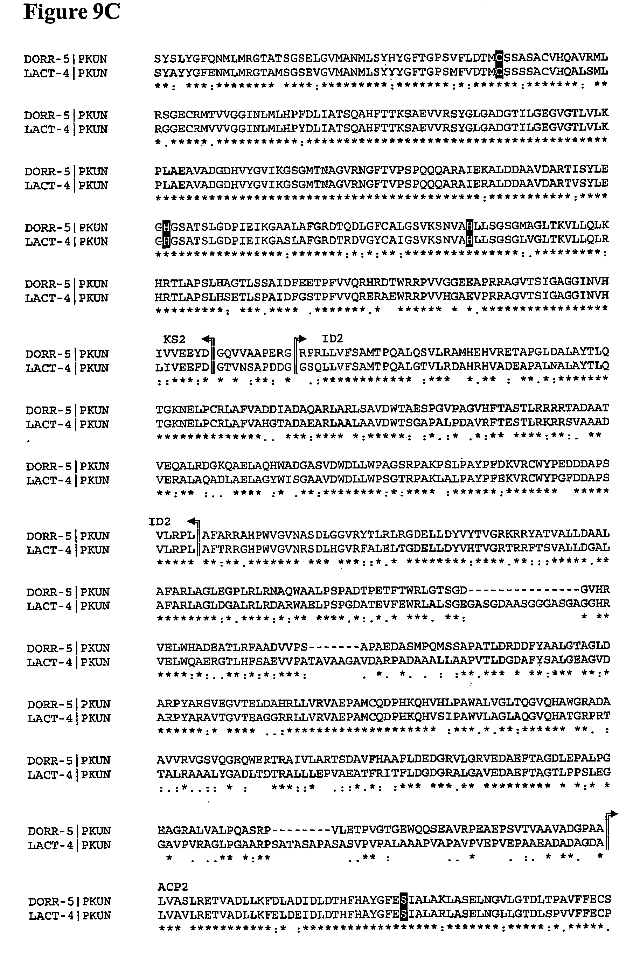

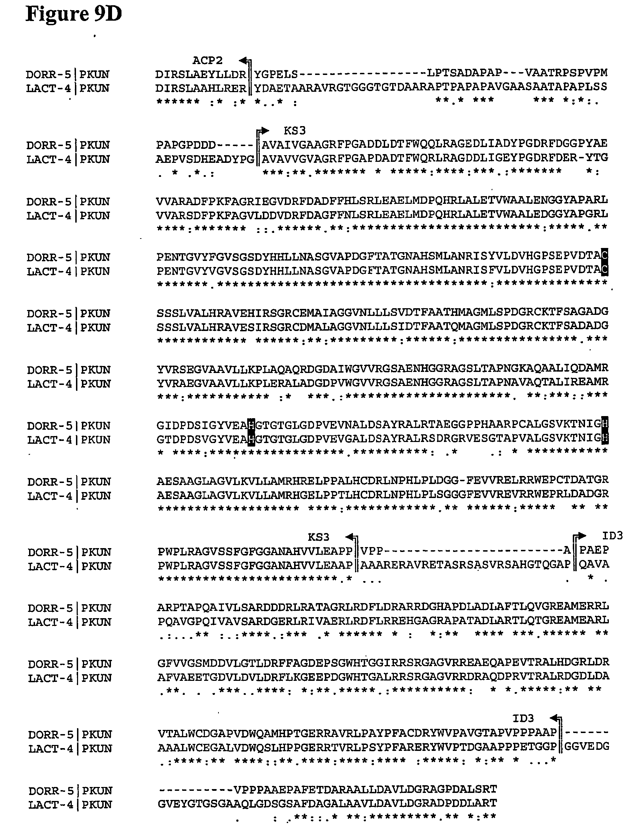

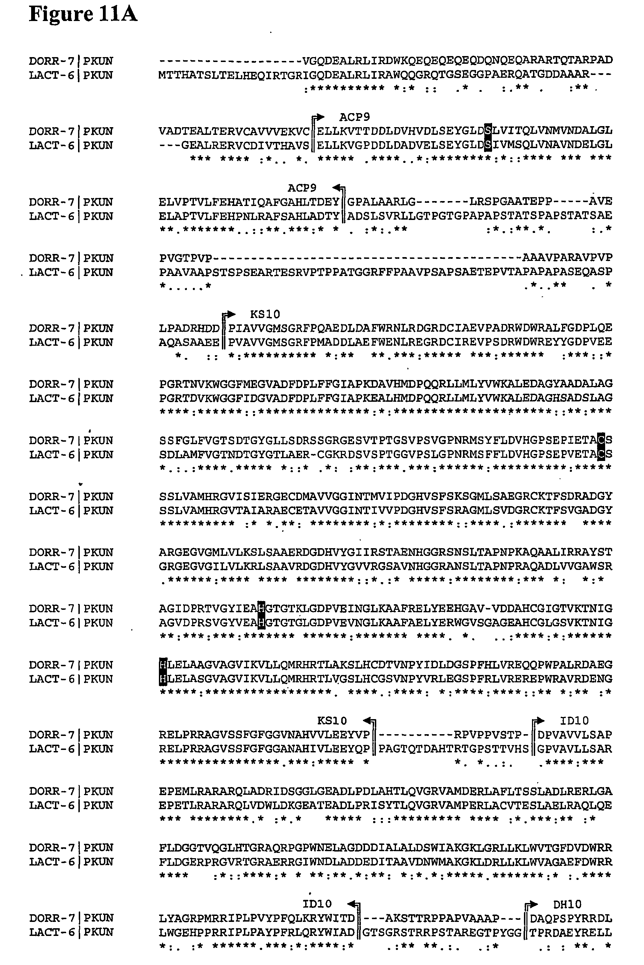

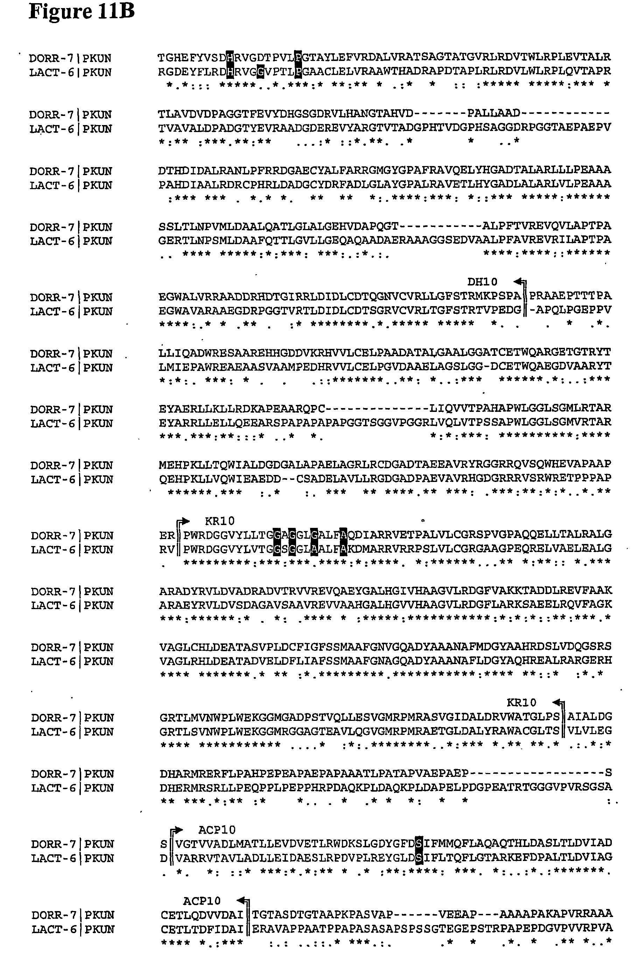

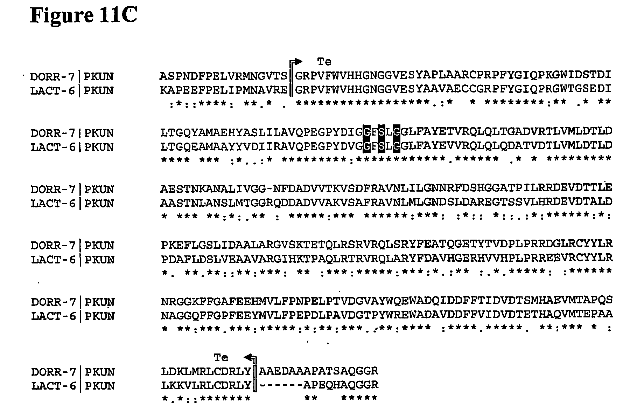

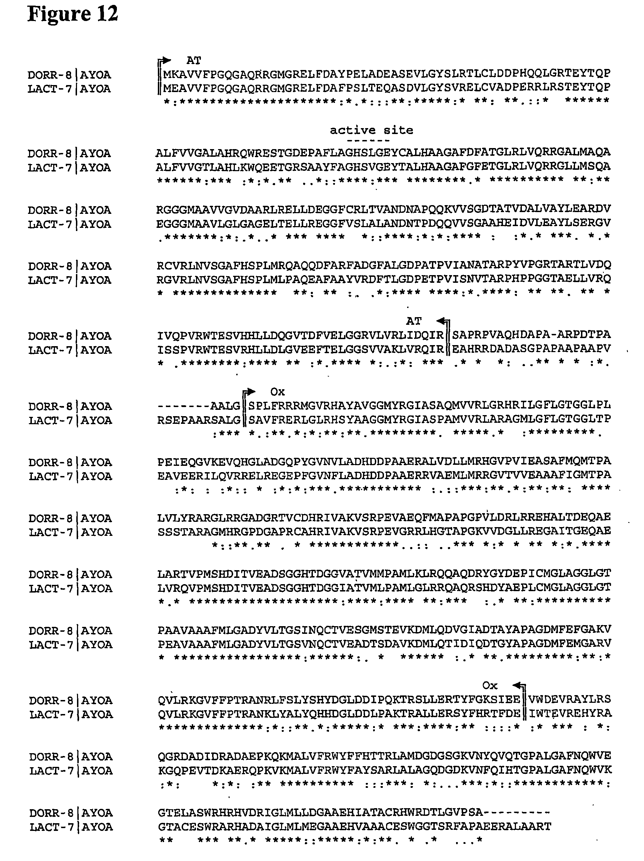

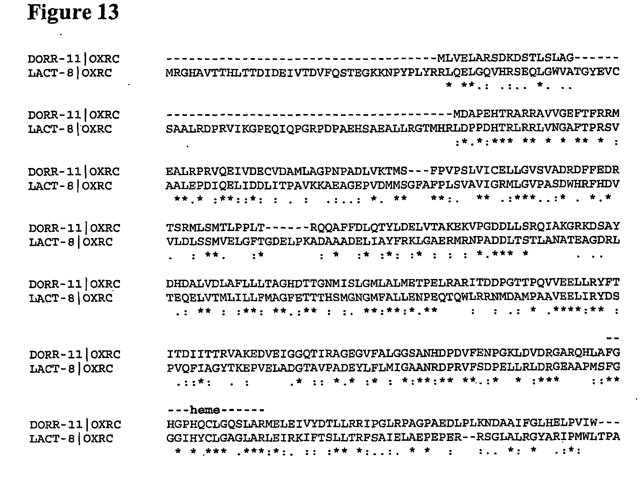

Figures 6 to 13 are amino acid alignments comparing the various ORFs that are common to both the dorrigocin biosynthetic locus and the lactimidomycin biosynthetic locus. Where applicable, key active site residues and motifs for the various polyketide synthase domains as described in Kakavas et al. (1997) J. Bacteriol. Vol 179 pp. 7515-7522 are indicated in Figures 6 to 13.

Identification of domains and assignment of their boundaries is based on the literature pertaining to type I PKSs. Given that the two component PKS systems described in this invention are quite divergent from type I PKS systems, is possible that boundaries may be slightly incorrect or that novel domains that are unique to the two component PKS systems may have been inadvertently missed. Tables 3 and 4 list the approximate amino acid coordinates of the various domains of the polyketide synthase components involved in the biosynthesis of dorrigocins, migrastatin, and isomigrastatin (Table 4) and in the biosynthesis of lactimidomycin (Table 5). The expression of the DORR locus results in the production of both linear polyketides (the dorrigocins) as well as cyclic polyketides (migrastatin and isomigrastatin). Accordingly, it is to be expected that the expression of the LACT locus results in the production of a linear polyketide product in addition to the cyclic polyketide lactimidomycin. To date, a linear of lactimidomycin has not been described either because it is produced at very low levels or it is unstable.

TABLE 4

| ORF no. | Accession no. | Amino acid coordinates | Homology | Module no. |
|---|---|---|---|---|
| 2 | 088CEP_01 | 1 - 276 311 - 529 | acyl transferase domain (AT) thioesterase domain (Te) | NA |
| 3 | 088CEP_02 | NA | acyl carrier protein | NA |
| 4 | 088CEP_03 | NA | amidotransferase | NA |

TABLE 4 (continued)

| ORF no. | Accession no. | Amino acid coordinates | Homology | Module no. |
|---|---|---|---|---|
| 5 | 088CEP_04 | 14 - 444 | ketosynthase domain (KS) | |
| | | 456 - 604 | interaction domain (ID) | |
| | | 631 - 901 | dehydratase domain (DH) | 1 |
| | | 1091 - 1312 | ketoreductase domain (KR) | |
| | | 1361 - 1432 | acyl carrier protein domain (ACP) | |
| | | 1508 - 1939 | ketosynthase domain (KS) | |
| | | 1950 - 2107 | interaction domain (ID) | 2 |
| | | 2439 - 2510 | acyl carrier protein domain (ACP) | |
| | | 2547 - 2976 | ketosynthase domain (KS) | 3 |
| | | 2989 - 3156 | interaction domain (ID) | |
| 6 | 088CEP_05 | 182 - 404 | ketoreductase domain (KR) | 3 |
| | | 446 - 512 | acyl carrier protein domain (ACP) | |
| | | 555 - 984 | ketosynthase domain (KS) | |
| | | 998 - 1190 | interaction domain (ID) | 4 |
| | | 1205 - 1276 | acyl carrier protein domain (ACP) | |
| | | 1299 - 1706 | ketosynthase domain (KS) | |
| | | 1718 - 1852 | interaction domain (ID) | |
| | | 1863 - 2128 | dehydratase domain (DH) | 5 |
| | | 2341 - 2562 | ketoreductase domain (KR) | |
| | | 2733 - 2949 | methyltransferase domain (MT) | |
| | | 3025 - 3093 | acyl carrier protein domain (ACP) | |
| | | 3143 - 3576 | ketosynthase domain (KS) | |
| | | 3592 - 3761 | interaction domain (ID) | |
| | | 4012 - 4228 | ketoreductase domain (KR) | 6 |
| | | 4394 - 4610 | methyltransferase domain (MT) | |
| | | 4677 - 4743 | acyl carrier protein domain (ACP) | |
| | | 4774 - 5186 | ketosynthase domain (KS) | |
| | | 5199 - 5321 | interaction domain (ID) | 7 |
| | | 5368 - 5440 | acyl carrier protein domain (ACP) | |
| | | 5507 - 5918 | ketosynthase domain (KS) | |
| | | 5929 - 6093 | interaction domain (ID) | |
| | | 6113 - 6384 | dehydratase domain (DH) | |
| | | 6567 - 6796 | ketoreductase domain (KR) | 8 |
| | | 6852 - 6907 | inactive acyl carrier protein domain | |
| | | 6943 - 7017 | acyl carrier protein domain (ACP) | |
| 6 | 088CEP_05 | 7061 - 7496 | ketosynthase domain (KS) | |
| | | 7509 - 7666 | interaction domain (ID) | 9 |
| | | 7667 - 7803 | dehydratase domain (DH) | |

TABLE 4 (continued)

| ORF no. | Accession no. | Amino acid coordinates | Homology | Module no. |
|---|---|---|---|---|
| 7 | 088CEP_06 | 62 - 123 | acyl carrier protein domain (ACP) | 9 |
| | | 176 - 611 | ketosynthase domain (KS) | |
| | | 622 - 777 | interaction domain (ID) | |
| | | 790 - 1060 | dehydratase domain (DH) | 10 |
| | | 1242 - 1470 | ketoreductase domain (KR) | |
| | | 1533 - 1589 | acyl carrier protein domain (ACP) | |
| | | 1653 - 1943 | thioesterase domain (Te) | |
| 8 | 088CFP_01 | 1 - 277 | acyl transferase domain (AT) | NA |
| | | 302 - 637 | oxidoreductase domain (Ox) | |
| NA Not Applicable | | | | |

TABLE 5

| ORF no. | Accession no. | Amino acid coordinates | Homology | Module no. |
|---|---|---|---|---|
| 1 | 133CBP_37 | 1 - 274 | acyl transferase domain (AT) | NA |
| | | 341 - 565 | thioesterase domain (Te) | |
| 2 | 133CBP_24 | NA | acyl carrier protein | NA |
| 3 | 133CBP_25 | NA | amidotransferase | NA |
| 4 | 133CBP_26 | 35 - 465 | ketosynthase domain (KS) | |
| | | 501 - 657 | interaction domain (ID) | |
| | | 709 - 992 | dehydratase domain (DH) | 1 |
| | | 1212 - 1433 | ketoreductase domain (KR) | |
| | | 1499 - 1570 | acyl carrier protein domain (ACP) | |
| | | 1657 - 2088 | ketosynthase domain (KS) | |
| | | 2099 - 2253 | interaction domain (ID) | 2 |
| | | 2618 - 2689 | acyl carrier protein domain (ACP) | |
| | | 2751 - 3180 | ketosynthase domain (KS) | 3 |
| | | 3201 - 3436 | interaction domain (ID) | |

TABLE 5 (continued)

| ORF no. | Accession no. | Amino acid coordinates | Homology | Module no. |
|---|---|---|---|---|
| 5 | 133CBP_55 | 191-414 | ketoreductase domain (KR) | 3' |
| | | 482 - 552 | acyl carrier protein domain (ACP) | |
| | | 640 -1080 | ketosynthase domain (KS) | |
| | | 1104 - 1271 | interaction domain (ID) | 4 |
| | | 1308 -1379 | acyl carrier protein domain (ACP) | |
| | | 1411 - 1816 | ketosynthase domain (KS) | |
| | | 1831 - 1984 | interaction domain (ID) | |
| | | 1988 - 2274 | dehydratase domain (DH) | 5 |
| | | 2522 - 2743 | ketoreductase domain (KR) | |
| | | 2917 - 3133 | methyltransferase domain (MT) | |
| | | 3240 - 3308 | acyl carrier protein domain (ACP) | |
| | | 3371 - 3804 | ketosynthase domain (KS) | |
| | | 3816 - 3986 | interaction domain (ID) | |
| | | 4270-4491 | ketoreductase domain (KR) | 6 |
| | | 4664 - 4880 | methyltransferase domain (MT) | |
| | | 4966 - 5033 | acyl carrier protein domain (ACP) | |
| | | 5092 - 5504 | ketosynthase domain (KS) | |
| | | 5523 - 5677 | interaction domain (ID) | 7 |
| | | 5708 - 6117 | ketosynthase domain (KS) | |
| | | 6130 - 6290 | interaction domain (ID) | |
| | | 6318 - 6593 | dehydratase domain (DH) | |
| | | 6805 - 7035 | ketoreductase domain (KR) | 8 |
| | | 7095 - 7166 | acyl carrier protein domain (ACP) | |
| | | 7227 - 7296 | acyl carrier protein domain (ACP) | |
| 5 | 133CBP_55 | 7378 - 7814 | ketosynthase domain (KS) | |
| | | 7836 - 7991 | interaction domain (ID) | 9 |
| | | 8011 - 8294 | dehydratase domain (DH) | |
| 6 | 133CBP_06 | 67 - 136 | acyl carrier protein domain (ACP) | 9 |
| | | 242 - 677 | ketosynthase domain (KS) | |
| | | 699 - 854 | interaction domain (ID) | |
| | | 873 - 1173 | dehydratase domain (DH) | 10 |
| | | 1365 - 1593 | ketoreductase domain (KR) | |
| | | 1661 - 1730 | acyl carrier protein domain (ACP) | |
| | | 1803 - 2094 | thioesterase domain (Te) | |
| 7 | 133CBP_56 | 1 - 277 | acyl transferase domain (AT) | NA |
| | | 310 - 645 | oxidoreductase domain (Ox) | |
| NA Not Applicable | | | | |

It is further to be understood that all sizes and all molecular weight or mass values are approximate, and are provided for description.

Some open reading frames listed herein initiate with non-standard initiation codons (i.e. GTG - Valine) rather than the standard initiation codon ATG, namely DORR ORFs 2, 6, 7 (SEQ ID NOS: 4, 12 and 14) and LACT ORFs 1, 3, 5 and 9 (SEQ ID Nos: 26, 30, 34 and 42. All ORFs are listed with M or V amino acids at the amino-terminal position to indicate the specificity of the first codon of the ORF. It is expected, however, that in all cases the biosynthesized protein will contain a methionine residue, and more specifically a formylmethionine residue, at the amino terminal position, in keeping with the widely accepted principle that protein synthesis in bacteria initiates with methionine (formylmethionine) even when the encoding gene specifies a non-standard initiation codon (*e.g.* Stryer, Biochemistry 3rd edition, 1998, W.H. Freeman and Co., New York, pp. 752-754).

Patents, patent publications, procedures and publications cited throughout this application are incorporated herein in their entirety for all purposes.

## Claims

1. A nucleic acid selected from the group consisting of

(a) a nucleic acid comprising a sequence selected from the group consisting of SEQ ID NOs: 1 and 22; the sequences complementary to SEQ ID NOs: 1 and 22, fragments comprising at least 50 consecutive nucleotides of SEQ ID NOs: 1 and 22; and fragments comprising at least 50 consecutive nucleotides of the sequences complementary to SEQ ID NOs: 1 and 22;
(b) a nucleic acid capable of hybridizing to the nucleic acid of (a) and which encodes a polypeptide useful to direct biosynthesis of dorrigocin;
(c) a nucleic acid having at least 80% or 99 % homology to the nucleic acid of (a) as determined by analysis with BLASTN version 2.0 with the default parameters and which encodes a polypeptide useful to direct biosynthesis of dorrigocin;
(d) a nucleic acid comprising a sequence selected from the group consisting of SEQ ID NOs: 3, 5, 7, 9, 11, 13, 15, 17, 19, 21, 24 and the sequences complementary thereto;
(e) a nucleic acid having at least 90% or 99% homology to a nucleic acid of (d) as determined by analysis with BLASTN version 2.0 with the default parameters and which encodes a polypeptide useful to direct biosynthesis of dorrigocin;
(f) a nucleic acid capable of hybridizing to the nucleic acid of SEQ ID Nos:5, 7, 11, 13, 15, 17, 19, 21 and 24 and which encodes a polypeptide useful to direct biosynthesis of dorrigocin;
(g) a nucleic acid having at least 70% homology to SEQ ID NOs: 5, 7, 9, 11, 13, 15, 17, 19, 21 and sequences complementary thereto as determined by analysis with BLASTN version 2.0 with the default parameters and which encodes a polypeptide useful to direct biosynthesis of dorrigocin;
(h) a nucleic acid comprising at least 50 consecutive bases of a sequence selected from the group consisting of SEQ ID NOs: 3, 5, 7, 9, 11, 13, 15, 17, 19, 21, 24 and the sequences complementary thereto; and
(i) a nucleic acid having at least 80% homology to the nucleic acid of (h) as determined by analysis with BLASTN version 2.0 with the default parameters and which encodes a polypeptide useful to direct biosynthesis of dorrigocin.

**2.** A polypeptide selected from the group consisting of

(a) a polypeptide comprising a sequence selected from the group consisting of SEQ ID NOs: 2, 4, 6, 8, 10, 12, 14, 16, 18, 20 and 23;
(b) a polypeptide comprising at least 20 consecutive amino acids of the polypeptides of (a);
(c) a polypeptide having at least 70% identity to the polypeptide of (a) as determined by analysis with BLASTP version 2.2.2 with the default parameters;
(d) a polypeptide having at least 99% homology to the polypeptide of (a) or (b) as determined with BLASTP version 2.2.2 with the default parameters; and
(e) a polypeptide having at least 75% identity to the polypeptide of (b) as determined by analysis with BLASTP version 2.2.2 with the default parameters.

**3.** An antibody capable of specifically binding to (i) a polypeptide having a sequence selected from the group consisting of SEQ ID NOs: 2, 4, 6, 8, 10, 12, 14, 16, 18, 20 and 23 or to (ii) a polypeptide comprising at least 25 consecutive amino acids of one of the polypeptides of (i).

**4.** A method of making a polypeptide having a sequence selected from the group consisting of SEQ ID NOs: 2, 4, 6, 8, 10, 12, 14, 16, 18, 20 and 23 or a polypeptide thereof having at least 25 consecutive amino acids comprising introducing a nucleic acid encoding said polypeptide, said nucleic acid being operably linked to a promoter, into a host cell.

**5.** A gene cluster comprising open reading frames encoding polypeptides shown in SEQ ID Nos:2, 4, 6, 8, 10, 12, 14, 16, 18, 20 and 23.

**6.** The gene cluster of claim 5 comprising an open reading frame selected from the group consisting of SEQ ID NOs: 2, 4, 6, 8, 10, 12, 14, 16, 18, 20, 23 and the sequences complementary thereto.

**7.** The gene cluster of claim 5 or 6, wherein the gene cluster comprises an open reading frame having at least 10 consecutive bases of a sequence of claim 6.

**8.** The gene cluster of any one of claims 5 to 7, wherein the gene cluster comprises an open reading frame having at least 70% homology to the sequences of claim 6 as determined with BLASTP version 2.2.2 with the default parameters.

**9.** The gene cluster of any one of claims 5 to 8, wherein the gene cluster is present in E. *coli* strains DH10B having accession nos. IDAC 270201-3 or IDAC 270201-4.

**10.** A prokaryotic host cell containing a gene cluster of any one of claims 5 to 9.

**11.** An expression vector comprising the nucleic acid of claim 1.

**12.** A host cell transformed with the expression vector of claim 11.

**13.** A method of expressing a dorrigocin biosynthetic gene product comprising culturing a host cell of claim 10 under conditions that permit expression of the dorrigocin biosynthetic gene product.

**14.** A nucleic acid comprising a nucleic acid sequence which encodes a domain of a dorrigocin polyketide synthetase, wherein said dorrigocin polyketide synthetase comprises an amino acid sequence selected from the group consisting of SEQ ID NO: 4, SEQ ID NO: 10, SEQ ID NO: 12, SEQ ID NO: 14 and SEQ ID NO: 16.

**15.** The nucleic acid of claim 14, wherein said domain is an acyl transferase domain, a thioesterase domain, a ketosynthase domain, an interaction domain, a dehydratase domain, a ketoreductase domain, an acyl carrier protein domain, a methyltransferase domain or an oxidoreductase domain.

**16.** The nucleic acid of claim 14 or 15, wherein said nucleic acid sequence encodes an amino acid sequence selected from the group consisting of

(a) amino acids 1-276 of SEQ ID NO. 4;
(b) amino acids 311-529 of SEQ ID NO. 4;
(c) amino acids 14-444 of SEQ ID NO. 10;
(d) amino acids 456-604 of SEQ ID NO. 10;
(e) amino acids 631-901 of SEQ ID NO: 10;
(f) amino acids 1091-1312 of SEQ ID NO: 10;
(g) amino acids 1361-1432 of SEQ ID NO: 10;
(h) amino acids 1508-1939 of SEQ ID NO: 10;
(i) amino acids 1950-2107 of SEQ ID NO:10;
(j) amino acids 2439-2510 of SEQ ID NO: 10;
(k) amino acids 2547-2976 of SEQ ID NO: 10;
(l) amino acids 2989-3156 of SEQ ID NO. 10;
(m) amino acids 182-404 of SEQ ID NO. 12;
(n) amino acids 446-512 of SEQ ID NO: 12;
(o) amino acids 555-984 of SEQ ID NO: 12;
(p) amino acids 998-1190 of SEQ ID NO: 12;
(q) amino acids 1205-1276 of SEQ ID NO: 12;
(r) amino acids 1299-1706 of SEQ ID NO: 12;
(s) amino acids 1718-1852 of SEQ ID NO: 12;
(t) amino acids 1863-2128 of SEQ ID NO: 12;
(u) amino acids 2341-2562 of SEQ ID NO: 12;
(v) amino acids 2733-2949 of SEQ ID NO: 12;
(w) amino acids 3025-3093 of SEQ ID NO: 12;
(x) amino acids 3143-3576 of SEQ ID NO: 12;
(y) amino acids 3592-3761 of SEQ ID NO: 12;
(z) amino acids 4012-4228 of SEQ ID NO: 12
(aa) amino acids 4394-4610 of SEQ ID NO: 12;
(bb) amino acids 4677-4743 of SEQ ID NO: 12;
(cc) amino acids 4774-5186 of SEQ ID NO: 12;
(dd) amino acids 5199-5321 of SEQ ID NO: 12;
(ee) amino acids 5368-5440 of SEQ ID NO: 12;
(ff) amino acids 5507-5918 of SEQ ID NO: 12;
(gg) amino acids 5929-6093 of SEQ ID NO: 12;
(hh) amino acids 6113-6384 of SEQ ID NO: 12;
(ii) amino acids 6567-6796 of SEQ ID NO: 12;
(jj) amino acids 6852-6907 of SEQ ID NO: 12;
(kk) amino acids 6943-7017 of SEQ ID NO: 12;
(ll) amino acids 7061-7496 of SEQ ID NO: 12;
(mm) amino acids 7509-7666 of SEQ ID NO: 12;
(nn) amino acids 7667-7803 of SEQ ID NO: 12;
(oo) amino acids 62-123 of SEQ ID NO. 14;
(pp) amino acids 176-611 of SEQ ID NO: 14;
(qq) amino acids 622-777 of SEQ ID NO: 14;
(rr) amino acids 790-1060 of SEQ ID NO: 14;
(ss) amino acids 1242-1470 of SEQ ID NO: 14;
(tt) amino acids 1533-1589 of SEQ ID NO: 14;
(uu) amino acids 1653-1943 of SEQ ID NO: 14;
(vv) amino acids 1-277 of SEQ ID NO. 16; and
(ww) amino acids 302-637 of SEQ ID NO: 16.

**17.** An acyl transferase domain useful in the production of dorrigocin, said acyl transferase domain comprises an amino acid sequence selected from the group consisting of:

a) amino acids 1-276 of SEQ ID NO: 4 or a polypeptide having at least 90% identity to amino acids 1-276 of SEQ ID NO: 4 as determined using the BLASTP algorithm with the default parameters; and
b) amino acids 1-277 of SEQ ID NO: 16 or a polypeptide having at least 90% identity to amino acids 1-277 of SEQ ID NO: 16 as determined using the BLASTP algorithm with the default parameters.

**18.** A thioesterase domain useful in the production of dorrigocin, said thioesterase domain comprises an amino acid sequence selected from the group consisting of:

(a) amino acids 311-529 of SEQ ID NO: 4, or a polypeptide having at least 90% identity to amino acids 311-529 of SEQ ID NO: 4 as determined using the BLASTP algorithm with the default parameters; and
(b) amino acids 1653-1943 of SEQ ID NO: 14 or a polypeptide having at least 90% identity to amino acids 1653-1943 of SEQ ID NO: 14 as determined using the BLASTP algorithm with the default parameters.

**19.** A ketosynthase domain useful in the production of dorrigocin, said ketosynthase domain comprises an amino acid sequence selected from the group consisting of:

(a) amino acids 14-444 of SEQ ID NO: 10, or a polypeptide having at least 90% identity to amino acids 14-444 of SEQ ID NO: 10 as determined using the BLASTP algorithm using the default parameters;
(b) amino acids 1508-1939 of SEQ ID NO: 10, or a polypeptide having at least 90% identity to amino acids 1508-1939 of SEQ ID NO: 10 as determined using the BLASTP algorithm with the default parameters;
(c) amino acids 2547-2976 of SEQ ID NO: 10, or a polypeptide having at least 90% identity to amino acids 2547-2976 of SEQ ID NO: 10 as determined using the BLASTP algorithm with the default parameters;
(d) amino acids 555-984 of SEQ ID NO: 12, or a polypeptide having at least 90% identity to amino acids 555-984 of SEQ ID NO: 12 as determined using the BLASTP algorithm with the default parameters;
(e) amino acids 1299-1706 of SEQ ID NO: 12, or a polypeptide having at least 90% identity to amino acids 1299-1706 of SEQ ID NO: 12 as determined using the BLASTP algorithm with the default parameters;
(f) amino acids 3143-3576 of SEQ ID NO: 12, or a polypeptide having at least 90% identity to amino acids 3143-3576 of SEQ ID NO: 12 as determined using the BLASTP algorithm with the default parameters;
(g) amino acids 4774-5186 of SEQ ID NO: 12, or a polypeptide having at least 90% identity to amino acids 4774-5186 of SEQ ID NO: 12 as determined using the BLASTP algorithm with the default parameters;
(h) amino acids 5507-5918 of SEQ ID NO: 12, or a polypeptide having at least 90% identity to amino acids 5507-5918 of SEQ ID NO: 12 as determined using the BLASTP algorithm with the default parameters;
(i) amino acids 7061-7496 of SEQ ID NO: 12, or a polypeptide having at least 90% identity to amino acids 7061-7496 of SEQ ID NO: 12 as determined using the BLASTP algorithm with the default parameters; and
(j) amino acids 176-611 of SEQ ID NO: 14, or a polypeptide having at least 90% identity to amino acids 176-611 of SEQ ID NO: 14 as determined using the BLASTP algorithm with the default parameters.

**20.** An interaction domain useful in the production of dorrigocin, said interaction domain comprises a sequence selected from the group consisting of:

(a) amino acids 456-604 of SEQ ID NO: 10, or a polypeptide having at least 90% identity to amino acids 456-604 of SEQ ID NO: 10 as determined using the BLASTP algorithm using the default parameters;
(b) amino acids 1950-2107 of SEQ ID NO: 10, or a polypeptide having at least 90% identity to amino acids 1950-2107 of SEQ ID NO: 10 as determined using the BLASTP algorithm using the default parameters;
(c) amino acids 2989-3156 of SEQ ID NO: 10, or a polypeptide having at least 90% identity to amino acids 2989-3156 of SEQ ID NO: 10 as determined using the BLASTP algorithm using the default parameters;
(d) amino acids 998-1190 of SEQ ID NO: 12, or a polypeptide having at least 90% identity to amino acids 998-1190 of SEQ ID NO: 12 as determined using the BLASTP algorithm using the default parameters;
(e) amino acids 1718-1852 of SEQ ID NO: 12, or a polypeptide having at least 90% identity to amino acids 1718-1852 of SEQ ID NO: 12 as determined using the BLASTP algorithm using the default parameters;
(f) amino acids 3592-3761 of SEQ ID NO: 12, or a polypeptide having at least 90% identity to amino acids 3592-3761 of SEQ ID NO: 12 as determined using the BLASTP algorithm using the default parameters;
(g) amino acids 5199-5321 of SEQ ID NO: 12, or a polypeptide having at least 90% identity to amino acids 5199-5321 of SEQ ID NO: 12 as determined using the BLASTP algorithm using the default parameters;
(h) amino acids 5929-6093 of SEQ ID NO: 12, or a polypeptide having at least 90% identity to amino acids 5929-6093 of SEQ ID NO: 12 as determined using the BLASTP algorithm using the default parameters;
(i) amino acids 7509-7666 of SEQ ID NO: 12, or a polypeptide having at least 90% identity to amino acids 7509-7666 of SEQ ID NO: 12 as determined using the BLASTP algorithm using the default parameters; and
(j) amino acids 622-777 of SEQ ID NO: 14, or a polypeptide having at least 90% identity to amino acids 622-777 of SEQ ID NO: 14 as determined using the BLASTP algorithm using the default parameters.

**21.** A dehydratase domain useful in the production of dorrigocin, said dehydratase domain comprises a sequence selected from the group consisting of:

(a) amino acids 631-901 of SEQ ID NO: 10, or a polypeptide having at least 90% identity to amino acids 631-901 of SEQ ID NO: 10 as determined using the BLASTP algorithm using the default parameters;
(b) amino acids 1863-2128 of SEQ ID NO: 12, or a polypeptide having at least 90% identity to amino acids 1863-2128 of SEQ ID NO: 12 as determined using the BLASTP algorithm using the default parameters;
(c) amino acids 6113-6384 of SEQ ID NO: 12, or a polypeptide having at least 90% identity to amino acids 6113-6384 of SEQ ID NO: 12 as determined using the BLASTP algorithm using the default parameters;
(d) amino acids 7667-7803 of SEQ ID NO: 12, or a polypeptide having at least 90% identity to amino acids 7667-7803 of SEQ ID NO: 12 as determined using the BLASTP algorithm using the default parameters; and
(e) amino acids 790-1060 of SEQ ID NO: 14, or a polypeptide having at least 90% identity to amino acids 790-1060 of SEQ ID NO: 14 as determined using the BLASTP algorithm using the default parameters.

**22.** A ketoreductase domain useful in the production of dorrigocin, said ketoreductase domain comprises a sequence selected from the group consisting of:

(a) amino acids 1091-1312 of SEQ ID NO: 10, or a polypeptide having at least 90% identity to amino acids 1091-1312 of SEQ ID NO: 10 as determined using the BLASTP algorithm using the default parameters;
(b) amino acids 182-404 of SEQ ID NO: 12, or a polypeptide having at least 90% identity to amino acids 182-404 of SEQ ID NO: 12 as determined using the BLASTP algorithm using the default parameters;
(c) amino acids 2341-2562 of SEQ ID NO: 12, or a polypeptide having at least 90% identity to amino acids 2341-2562 of SEQ ID NO: 12 as determined using the BLASTP algorithm using the default parameters;
(d) amino acids 4012-4228 of SEQ ID NO: 12, or a polypeptide having at least 90% identity to amino acids 4012-4228 of SEQ ID NO: 12 as determined using the BLASTP algorithm using the default parameters;
(e) amino acids 6567-6796 of SEQ ID NO: 12, or a polypeptide having at least 90% identity to amino acids 6567-6796 of SEQ ID NO: 12 as determined using the BLASTP algorithm using the default parameters; and
(f) amino acids 1242-1470 of SEQ ID NO: 14, or a polypeptide having at least 90% identity to amino acids 1242-1470 of SEQ ID NO: 14 as determined using the BLASTP algorithm using the default parameters.

**23.** A acyl carrier protein domain useful in the production of dorrigocin, said acyl carrier-protein domain comprises a sequence selected from the group consisting of:

(a) amino acids 1361-1432 of SEQ ID NO: 10, or a polypeptide having at least 90% identity to amino acids 1361-1432 of SEQ ID NO: 10 as determined using the BLASTP algorithm using the default parameters;
(b) amino acids 2439-2510 of SEQ ID NO: 10, or a polypeptide having at least 90% identity to amino acids 2439-2510 of SEQ ID NO: 10 as determined using the BLASTP algorithm using the default parameters;
(c) amino acids 446-512 of SEQ ID NO: 12, or a polypeptide having at least 90% identity to amino acids 446-512 of SEQ ID NO: 12 as determined using the BLASTP algorithm using the default parameters;
(d) amino acids 1205-1276 of SEQ ID NO: 12, or a polypeptide having at least 90% identity to amino acids 1205-1276 of SEQ ID NO: 12 as determined using the BLASTP algorithm using the default parameters;
(e) amino acids 3025-3093 of SEQ ID NO: 12, or a polypeptide having at least 90% identity to amino acids 3025-3093 of SEQ ID NO: 12 as determined using the BLASTP algorithm using the default parameters;
(f) amino acids 4677-4743 of SEQ ID NO: 12, or a polypeptide having at least 90% identity to amino acids 4677-4743 of SEQ ID NO: 12 as determined using the BLASTP algorithm using the default parameters;
(g) amino acids 5368-5440 of SEQ ID NO: 12, or a polypeptide having at least 90% identity to amino acids 5368-5440 of SEQ ID NO: 12 as determined using the BLASTP algorithm using the default parameters;
(h) amino acids 6852-6907 of SEQ ID NO: 12, or a polypeptide having at least 90% identity to amino acids 6852-6907 of SEQ ID NO: 12 as determined using the BLASTP algorithm using the default parameters;
(i) amino acids 6943-7017 of SEQ ID NO: 12, or a polypeptide having at least 90% identity to amino acids 6943-7017 of SEQ ID NO: 12 as determined using the BLASTP algorithm using the default parameters;
(j) amino acids 62-123 of SEQ ID NO: 14, or a polypeptide having at least 90% identity to amino acids 62-123 of SEQ ID NO: 14 as determined using the BLASTP algorithm using the default parameters; and
(l) amino acids 1533-1589 of SEQ ID NO: 14, or a polypeptide having at least 90% identity to amino acids 1533-1589 of SEQ ID NO: 14 as determined using the BLASTP algorithm using the default parameters.

**24.** A methyltransferase domain useful in the production of dorrigocin, said methyltransferase domain comprises a sequence selected from the group consisting of:

(a) amino acids 2733-2949 of SEQ ID NO: 12, or a polypeptide having at least 90% identity to amino acids 2733-2949 of SEQ ID NO: 12 as determined using the BLASTP algorithm using the default parameters; and
(b) amino acids 4394-4610 of SEQ ID NO: 12, or a polypeptide having at least 90% identity to amino acids 4394-4610 of SEQ ID NO: 12 as determined using the BLASTP algorithm using the default parameters.

**25.** A oxidoreductase domain useful in the production of dorrigocin said oxidoreductase domain comprises a sequence of amino acids 302-637 of SEQ ID NO: 16, or a polypeptide having at least 90% identity to amino acids 302-637 of SEQ ID NO: 16 as determined using the BLASTP algorithm using the default parameters.

**Patentansprüche**

**1.** Nucleinsäure ausgewählt aus der Gruppe bestehend aus:

(a) einer Nucleinsäure, die eine Sequenz umfasst, die ausgewählt ist aus der Gruppe bestehend aus SEQ ID NR: 1 und 22, den komplementären Sequenzen zu SEQ ID NR: 1 und 22, Fragmenten, die mindestens 50 aufeinanderfolgende Nucleotide der SEQ ID NR: 1 und 22 umfassen; und Fragmenten, die mindestens 50 aufeinanderfolgende Nucleotide der zu SEQ ID NR: 1 und 22 komplementären Sequenzen umfassen;
(b) einer Nucleinsäure, die in der Lage ist, an die Nucleinsäure aus (a) zu hybridisieren, und die ein Polypeptid codiert, das zur Regulierung der Biosynthese von Dorrigocin eingesetzt werden kann;
(c) einer Nucleinsäure, die mindestens 80% oder 99% Homologie zu der Nucleinsäure aus (a) aufweist, ermittelt durch Analyse mit BLASTN Version 2.0 mit den Standardparametern, und die ein Polypeptid codiert, das zur Regulierung der Biosynthese von Dorrigocin eingesetzt werden kann;
(d) einer Nucleinsäure, die eine Sequenz umfaßt, die ausgewählt ist aus der Gruppe bestehend aus den SEQ ID NR: 3, 5, 7, 9, 11, 13, 15, 17, 19, 21, 24 und den dazu komplementären Sequenzen;
(e) einer Nucleinsäure, die mindestens 90% oder 99% Homologie zu einer Nucleinsäure aus (d) aufweist, ermittelt durch Analyse mit BLASTN Version 2.0 mit den Standardparametern, und die ein Polypeptid codiert, das zur Regulierung der Biosynthese von Dorrigocin eingesetzt werden kann;
(f) einer Nucleinsäure, die in der Lage ist, mit der Nucleinsäure der SEQ ID NR: 5, 7, 11, 13, 15, 17, 19, 21 und 24 zu hybridisieren, und welche ein Polypeptid codiert, das zur Regulierung der Biosynthese von Dorrigocin eingesetzt werden kann;
(g) einer Nucleinsäure, die mindestens 70% Homologie zu SEC ID NR: 5, 7, 9, 11, 13, 15, 17, 19, 21 und den dazu komplementären Sequenzen aufweist, ermittelt durch Analyse mit BLASTN Version 2.0 mit den Standardparametern, und die ein Polypeptid codiert, das zur Regulierung der Biosynthese von Dorrigocin eingesetzt werden kann;
(h) einer Nucleinsäure, die mindestens 50 aufeinanderfolgende Basen einer Sequenz ausgewählt aus der Gruppe bestehend aus den SEQ ID NR: 3, 5, 7, 9, 11, 13, 15, 17, 19, 21, 24 und den dazu komplementären Sequenzen umfaßt; und
(i) einer Nucleinsäure, die mindestens 80% Homologie zu der Nucleinsäure aus (h) aufweist, ermittelt durch Analyse mit BLASTN Version 2.0 mit den Standardparametern, und die ein Polypeptid codiert, das zur Regulierung der Biosynthese von Dorrigocin eingesetzt werden kann.

**2.** Polypeptid, ausgewählt aus der Gruppe bestehend aus

(a) einem Polypeptid, das eine Sequenz ausgewählt aus der Gruppe bestehend aus SEQ ID NR: 2, 4, 6, 8, 10, 12, 14, 16, 18, 20 und 23 umfaßt;
(b) einem Polypeptid, das mindestens 20 aufeinanderfolgende Aminosäuren der Polypeptide aus (a) umfaßt;
(c) einem Polypeptid, das mindestens 70% Identität mit dem Polypeptid aus (a) aufweist, ermittelt durch Analyse mit BLASTP Version 2.2.2 mit den Standardparametern;
(d) einem Polypeptid, das mindestens 99% Homologie zu dem Polypeptid aus (a) oder (b) aufweist, ermittelt mit BLASTP Version 2.2.2 mit den Standardparametern; und
(e) einem Polypeptid, das mindestens 75% Identität mit dem Polypeptid aus (b) aufweist, ermittelt durch Analyse mit BLASTP Version 2.2.2 mit den Standardparametern.

**3.** Antikörper, der in der Lage ist, spezifisch zu binden an (i) ein Polypeptid, das eine Sequenz ausgewählt aus der Gruppe bestehend aus SEQ ID NR: 2, 4, 6, 8, 10, 12, 14, 16, 18, 20, und 23 hat, oder (ii) ein Polypeptid, das mindestens 25 aufeinanderfolgende Aminosäuren eines der Polypeptide aus (i) umfaßt.

**4.** Verfahren zur Herstellung eines Polypeptids mit einer Sequenz ausgewählt aus der Gruppe bestehend aus SEQ ID NR: 2, 4, 6, 8, 10, 12, 14, 16, 18, 20 und 23 oder eines Polypeptids davon mit mindestens 25 aufeinanderfolgenden Aminosäuren, umfassend die Einführung einer Nucleinsäure, die das Polypeptid codiert, in eine Wirtzelle, wobei die Nucleinsäure funktionell mit einem Promotor verknüpft ist.

**5.** Gencluster, der offene Leserahmen umfaßt, die die Polypeptide codieren, welche in SEQ ID NR: 2, 4, 6, 8, 10, 12 14, 16, 18, 20 und 23 dargestellt sind.

**6.** Gencluster nach Anspruch 5, der einen offenen Leserahmen ausgewählt aus der Gruppe bestehend aus SEQ ID NR: 2, 4, 6, 8, 10, 12, 14, 16, 18, 20, 23 und den dazu komplementären Sequenzen umfaßt.

**7.** Gencluster nach Anspruch 5 oder 6, wobei der Geneluster einen offenen Leserahmen umfaßt, der mindestens 10 aufeinanderfolgende Basen einer Sequenz nach Anspruch 6 enthält.

**8.** Gencluster nach einem der Ansprüche 5 bis 7, wobei der Gencluster einen offenen Leserahmen umfaßt, der mindestens 70% Homologie zu den Sequenzen nach Anspruch 6 aufweist, ermittelt mit BLASTP Version 2.2.2 mit den Standardparametern.

**9.** Gencluster nach einem der Ansprüche 5 bis 8, wobei der Gencluster in *E. coli*-Stämmen DH10B mit den Hinterlegungsnummern IDAC 270201-3 oder IDAC 270201-4 vorkommt.

**10.** Prokaryontische Wirtszelle, die einen Gencluster nach einem der Ansprüche 5 bis 9 enthält.

**11.** Expressionsvektor, der die Nucleinsäure nach Anspruch 1 umfaßt.

**12.** Wirtszelle, die mit dem Expressionsvektor nach Anspruch 11 transformiert ist.

**13.** Verfahren zur Expression eines Genprodukts der Dorrigocin-Biosynthese, das die Züchtung einer Wirtszelle nach Anspruch 10 unter Bedingungen umfasst, die eine Expression des Genprodukts der Dorrigocin-Biosynthese erlauben.

**14.** Nucleinsäure, die eine Nucleinsäuresequenz umfaßt, die eine Domäne einer Dorrigocin-Polyketid-Synthetase codiert, wobei die Dorrigocin-Polyketid-Synthetase eine Aminosäuresequenz umfaßt, ausgewählt aus der Gruppe bestehend aus SEQ ID NR: 4, SEQ ID NR: 10, SEQ ID NR: 12, SEQ ID NR: 14 und SEQ ID NR: 16.

**15.** Nucleinsäure nach Anspruch 14, wobei die Domäne eine Acyltransferase-Domäne, eine Thioesterase-Domäne, eine Ketosynthase-Domäne, eine Interaktionsdomäne, eine Dehydratase-Domäne, eine Ketoreductase-Domäne, eine Acyl-Transportprotein-Domäne, eine Methyltransferase-Domäne oder eine Oxidoreductase-Domäne darstellt.

**16.** Nucleinsäure nach Anspruch 14 oder 15, wobei die Nucleinsäuresequenz eine Aminosäuresequenz codiert, ausgewählt aus der Gruppe bestehend aus

(a) Aminosäuren 1-276 aus SEQ ID NR: 4;
(b) Aminosäuren 311-529 aus SEQ ID NR: 4;
(c) Aminosäuren 14-444 aus SEQ ID NR: 10;
(d) Aminosäuren 456-604 aus SEQ ID NR: 10;
(e) Aminosäuren 631-901 aus SEQ ID NR: 10;
(f) Aminosäuren 1091-1312 aus SEQ ID NR: 10;
(g) Aminosäuren 1361-1432 aus SEQ ID NR: 10;
(h) Aminosäuren 1508-1939 aus SEQ ID NR: 10;
(i) Aminosäuren 1950-2107 aus SEQ ID NR: 10;
(j) Aminosäuren 2439-2510 aus SEQ ID NR: 10;
(k) Aminosäuren 2547-2976 aus SEQ ID NR: 10;
(l) Aminosäuren 2989-3156 aus SEQ ID NR: 10;
(m) Aminosäuren 182-404 aus SEQ ID NR: 12;
(n) Aminosäuren 446-512 aus SEQ ID NR: 12;
(o) Aminosäuren 555-984 aus SEQ ID NR: 12;

(p) Aminosäuren 998-1190 aus SEQ ID NR: 12;
(q) Aminosäuren 1205-1276 aus SEQ ID NR: 12;
(r) Aminosäuren 1299-1706 aus SEQ ID NR: 12;
(s) Aminosäuren 1718-1852 aus SEQ ID NR: 12;
(t) Aminosäuren 1863-2128 aus SEQ ID NR: 12;
(u) Aminosäuren 2341-2562 aus SEQ ID NR: 12;
(v) Aminosäuren 2733-2949 aus SEQ ID NR:12;
(w) Aminosäuren 3025-3093 aus SEQ ID NR: 12;
(x) Aminosäuren 3143-3576 aus SEQ ID NR: 12;
(y) Aminosäuren 3592-3761 aus SEQ ID NR:12;
(z) Aminosäuren 4012-4228 aus SEQ ID NR: 12;
(aa) Aminosäuren 4394-4610 aus SEQ ID NR: 12;
(bb) Aminosäuren 4677-4743 aus SEQ ID NR: 12;
(cc) Aminosäuren 4774-5186 aus SEQ ID NR: 12;
(dd) Aminosäuren 5199-5321 aus SEQ ID NR:12;
(ee) Aminosäuren 5368-5440 aus SEQ ID NR: 12;
(ff) Aminosäuren 5507-5918 aus SEQ ID NR: 12;
(gg) Aminosäuren 5929-6093 aus SEQ ID NR: 12;
(hh) Aminosäuren 6113-6384 aus SEQ ID NR: 12;
(ii) Aminosäuren 6567-6796 aus SEQ ID NR: 12;
(jj) Aminosäuren 6852-6907 aus SEQ ID NR: 12;
(kk) Aminosäuren 6943-7017 aus SEQ ID NR: 12;
(ll) Aminosäuren 7061-7496 aus SEQ ID NR: 12;
(mm) Aminosäuren 7509-7666 aus SEQ ID NR: 12;
(nn) Aminosäuren 7667-7803 aus SEQ ID NR: 12;
(oo) Aminosäuren 62-123 aus SEQ ID NR: 14;
(pp) Aminosäuren 176-611 aus SEQ ID NR: 14;
(qq) Aminosäuren 622-777 aus SEQ ID NR: 14;
(rr) Aminosäuren 790-1060 aus SEQ ID NR: 14;
(ss) Aminosäuren 1242-1470 aus SEQ ID NR: 14;
(tt) Aminosäuren 1533-1589 aus SEC ID NR: 14;
(uu) Aminosäuren 1653-1943 aus SEQ ID NR: 14;
(vv) Aminosäuren 1-277 aus SEQ ID NR: 16; und
(ww) Aminosäuren 302-637 aus SEQ ID NR: 16.

**17.** Acyltransferase-Domäne zur Verwendung bei der Dorrigocinherstellung, wobei die Acyltransferase-Domäne eine Aminosäuresequenz umfasst, ausgewählt aus der Gruppe bestehend aus:

(a) Aminosäuren 1-276 von SEQ ID NR: 4 oder einem Polypeptid, das mindestens 90% Identität mit den Aminosäuren 1-276 der SEQ ID NR: 4 aufweist, wie ermittelt unter Verwendung des BLASTP Algorithmus mit den Standardparametern; und
(b) Aminosäuren 1-277 von SEQ ID NR: 16 oder einem Polypeptid, das mindestens 90% Identität mit den Aminosäuren 1-277 der SEQ ID NR: 16 aufweist, ermittelt unter Verwendung des BLASTP Algorithmus mit den Standardparametern.

**18.** Thioesterase-Domäne zur Verwendung bei der Dorrigocinherstellung, wobei die Thioesterase-Domäne eine Aminosäuresequenz umfasst, ausgewählt aus der Gruppe bestehend aus:

(a) Aminosäuren 311-529 von SEQ ID NR: 4 oder einem Polypeptid, das mindestens 90% Identität mit den Aminosäuren 311-529 der SEQ ID NR: 4 aufweist, wie ermittelt unter Verwendung des BLASTP Algorithmus mit den Standardparametern; und
(b) Aminosäuren 1653-1943 von SEQ ID NR: 14 oder einem Polypeptid, das mindestens 90% Identität mit den Aminosäuren 1653-1943 der SEQ ID NR: 14 aufweist, ermittelt unter Verwendung des BLASTP Algorithmus mit den Standardparametern.

**19.** Ketosynthase-Domäne zur Verwendung bei der Dorrigocinherstellung, wobei die Ketosynthase-Domäne eine Aminosäuresequenz umfasst, ausgewählt aus der Gruppe bestehend aus:

(a) Aminosäuren 14-444 von SEQ ID NR: 10 oder einem Polypeptid, das mindestens 90% Identität mit den Aminosäuren 14-444 der SEQ ID NR: 10 aufweist, wie ermittelt unter Verwendung des BLASTP Algorithmus mit den Standardparametern;
(b) Aminosäuren 1508-1939 von SEQ ID NR: 10 oder einem Polypeptid, das mindestens 90% Identität mit den Aminosäuren 1508-1939 der SEQ ID NR: 10 aufweist, ermittelt unter Verwendung des BLASTP Algorithmus mit den Standardparametern;
(c) Aminosäuren 2547-2976 von SEQ ID NR: 10 oder einem Polypeptid, das mindestens 90% Identität mit den Aminosäuren 2547-2976 der SEQ ID NR: 10 aufweist, ermittelt unter Verwendung des BLASTP Algorithmus mit den Standardparametern;
(d) Aminosäuren 555-984 von SEQ ID NR: 12 oder einem Polypeptid, das mindestens 90% Identität mit den Aminosäuren 555-984 der SEQ ID NR: 12 aufweist, ermittelt unter Verwendung des BLASTP Algorithmus mit den Standardparametern;
(e) Aminosäuren 1299-1706 von SEQ ID NR: 12 oder einem Polypeptid, das mindestens 90% Identität mit den Aminosäuren 1299-1706 der SEQ ID NR: 12 aufweist, ermittelt unter Verwendung des BLASTP Algorithmus mit den Standardparametern;
(f) Aminosäuren 3143-3576 von SEQ ID NR: 12 oder einem Polypeptid, das mindestens 90% Identität mit den Aminosäuren 3143-3576 der SEQ ID NR: 12 aufweist, ermittelt unter Verwendung des BLASTP Algorithmus mit den Standardparametern;
(g) Aminosäuren 4774-5186 von SEQ ID NR: 12 oder einem Polypeptid, das mindestens 90% Identität mit den Aminosäuren 4774-5186 der SEQ ID NR: 12 aufweist, ermittelt unter Verwendung des BLASTP Algorithmus mit den Standardparametern;
(h) Aminosäuren 5507-5918 von SEQ ID NR: 12 oder einem Polypeptid, das mindestens 90% Identität mit den Aminosäuren 5507-5918 der SEQ ID NR: 12 aufweist, ermittelt unter Verwendung des BLASTP Algorithmus mit den Standardparametern;
(i) Aminosäuren 7061-7496 von SEQ ID NR: 12 oder einem Polypeptid, das mindestens 90% Identität mit den Aminosäuren 7061-7496 der SEQ ID NR: 12 aufweist, ermittelt unter Verwendung des BLASTP Algorithmus mit den Standardparametern; und
(j) Aminosäuren 176-611 von SEQ ID NR: 14 oder einem Polypeptid, das mindestens 90% Identität mit den Aminosäuren 176-611 der SEQ ID NR: 14 aufweist, ermittelt unter Verwendung des BLASTP Algorithmus mit den Standardparametern.

**20.** Interaktionsdomäne zur Verwendung bei der Dorrigocinherstellung, wobei die Interaktionsdomäne eine Sequenz umfasst, ausgewählt aus der Gruppe bestehend aus:

(a) Aminosäuren 456-604 von SEQ ID NR: 10 oder einem Polypeptid, das mindestens 90% Identität mit den Aminosäuren 456-604 der SEQ ID NR: 10 aufweist, wie ermittelt unter Verwendung des BLASTP Algorithmus mit den Standardparametern;
(b) Aminosäuren 1950-2107 von SEQ ID NR: 10 oder einem Polypeptid, das mindestens 90% Identität mit den Aminosäuren 1950-2107 der SEQ ID NR: 10 aufweist, ermittelt unter Verwendung des BLASTP Algorithmus mit den Standardparametern;
(c) Aminosäuren 2989-3156 von SEQ ID NR: 10 oder einem Polypeptid, das mindestens 90% Identität mit den Aminosäuren 2989-3156 der SEQ ID NR: 10 aufweist, ermittelt unter Verwendung des BLASTP Algorithmus mit den Standardparametern;
(d) Aminosäuren 998-1190 von SEQ ID NR: 12 oder einem Polypeptid, das mindestens 90% Identität mit den Aminosäuren 998-1190 der SEQ ID NR: 12 aufweist, ermittelt unter Verwendung des BLASTP Algorithmus mit den Standardparametern;
(e) Aminosäuren 1718-1852 von SEQ ID NR: 12 oder einem Polypeptid, das mindestens 90% Identität mit den Aminosäuren 1718-1852 der SEQ ID NR: 12 aufweist, ermittelt unter Verwendung des BLASTP Algorithmus mit den Standardparametern;
(f) Aminosäuren 3592-3761 von SEQ ID NR: 12 oder einem Polypeptid, das mindestens 90% Identität mit den Aminosäuren 3592-3761 der SEQ ID NR: 12 aufweist, ermittelt unter Verwendung des BLASTP Algorithmus mit den Standardparametern;
(g) Aminosäuren 5199-5321 von SEQ ID NR: 12 oder einem Polypeptid, das mindestens 90% Identität mit den Aminosäuren 5199-5321 der SEQ ID NR: 12 aufweist, ermittelt unter Verwendung des BLASTP Algorithmus mit den Standardparametern;
(h) Aminosäuren 5929-6093 von SEQ ID NR: 12 oder einem Polypeptid, das mindestens 90% Identität mit den Aminosäuren 5929-6093 der SEQ ID NR: 12 aufweist, ermittelt unter Verwendung des BLASTP Algorithmus mit den Standardparametern;

(i) Aminosäuren 7509-7666 von SEQ ID NR: 12 oder einem Polypeptid, das mindestens 90% Identität mit den Aminosäuren 7509-7666 der SEQ ID NR: 12 aufweist, ermittelt unter Verwendung des BLASTP Algorithmus mit den Standardparametern; und
(j) Aminosäuren 622-777 von SEQ ID NR: 14 oder einem Polypeptid, das mindestens 90% Identität mit den Aminosäuren 622-777 der SEQ ID NR: 14 aufweist, ermittelt unter Verwendung des BLASTP Algorithmus mit den Standardparametern.

**21.** Dehydratase-Domäne zur Verwendung bei der Dorrigocinherstellung, wobei die Dehydratase-Domäne eine Sequenz umfasst, ausgewählt aus der Gruppe bestehend aus:

(a) Aminosäuren 631-901 von SEQ ID NR: 10 oder einem Polypeptid, das mindestens 90% Identität mit den Aminosäuren 631-901 der SEQ ID NR: 10 aufweist, wie ermittelt unter Verwendung des BLASTP Algorithmus mit den Standardparametern;
(b) Aminosäuren 1863-2128 von SEQ ID NR: 12 oder einem Polypeptid, das mindestens 90% Identität mit den Aminosäuren 1863-2128 der SEQ ID NR: 12 aufweist, wie ermittelt unter Verwendung des BLASTP Algorithmus mit den Standardparametern;
(c) Aminosäuren 6113-6384 von SEQ ID NR: 12 oder einem Polypeptid, das mindestens 90% Identität mit den Aminosäuren 6113-6384 der SEQ ID NR: 12 aufweist, wie ermittelt unter Verwendung des BLASTP Algorithmus mit den Standardparametern;
(d) Aminosäuren 7667-7803 von SEQ ID NR: 12 oder einem Polypeptid, das mindestens 90% Identität mit den Aminosäuren 7667-7803 der SEQ ID NR: 12 aufweist, wie ermittelt unter Verwendung des BLASTP Algorithmus mit den Standardparametern; und
(e) Aminosäuren 790-1060 von SEQ ID NR: 14 oder einem Polypeptid, das mindestens 90% Identität mit den Aminosäuren 790-1060 der SEQ ID NR: 14 aufweist, wie ermittelt unter Verwendung des BLASTP Algorithmus mit den Standardparametern.

**22.** Ketoreduktase-Domäne zur Verwendung bei der Dorrigocinherstellung, wobei die Ketoreduktase-Domäne eine Sequenz umfasst, ausgewählt aus der Gruppe bestehend aus:

(a) Aminosäuren 1091-1312 von SEQ ID NR: 10 oder einem Polypeptid, das mindestens 90% Identität mit den Aminosäuren 1091-1312 der SEQ ID NR: 10 aufweist, wie ermittelt unter Verwendung des BLASTP Algorithmus mit den Standardparametern;
(b) Aminosäuren 182-404 von SEQ ID NR: 12 oder einem Polypeptid, das mindestens 90% Identität mit den Aminosäuren 182-404 der SEQ ID NR: 12 aufweist, wie ermittelt unter Verwendung des BLASTP Algorithmus mit den Standardparametern;
(c) Aminosäuren 2341-2562 von SEQ ID NR: 12 oder einem Polypeptid, das mindestens 90% Identität mit den Aminosäuren 2341-2562 der SEQ ID NR: 12 aufweist, wie ermittelt unter Verwendung des BLASTP Algorithmus mit den Standardparametern;
(d) Aminosäuren 4012-4228 von SEQ ID NR: 12 oder einem Polypeptid, das mindestens 90% Identität mit den Aminosäuren 4012-4228 der SEQ ID NR: 12 aufweist, wie ermittelt unter Verwendung des BLASTP Algorithmus mit den Standardparametern;
(e) Aminosäuren 6567-6796 von SEQ ID NR: 12 oder einem Polypeptid, das mindestens 90% Identität mit den Aminosäuren 6567-6796 der SEQ ID NR: 12 aufweist, wie ermittelt unter Verwendung des BLASTP Algorithmus mit den Standardparametern; und
(f) Aminosäuren 1242-1470 von SEQ ID NR: 14 oder einem Polypeptid, das mindestens 90% Identität mit den Aminosäuren 1242-1470 der SEQ ID NR: 14 aufweist, wie ermittelt unter Verwendung des BLASTP Algorithmus mit den Standardparametern.

**23.** Acyl-Transportprotein-Domäne zur Verwendung bei der Dorrigocinherstellung, wobei die Acyl-Transportprotein-Domäne eine Sequenz umfasst, ausgewählt aus der Gruppe bestehend aus:

(a) Aminosäuren 1361-1432 von SEQ ID NR: 10 oder einem Polypeptid, das mindestens 90% Identität mit den Aminosäuren 1361-1432 der SEQ ID NR: 10 aufweist, wie ermittelt unter Verwendung des BLASTP Algorithmus mit den Standardparametern;
(b) Aminosäuren 2439-2510 von SEQ ID NR: 10 oder einem Polypeptid, das mindestens 90% Identität mit den Aminosäuren 2439-2510 der SEQ ID NR: 10 aufweist, wie ermittelt unter Verwendung des BLASTP Algorithmus mit den Standardparametern;
(c) Aminosäuren 446-512 von SEQ ID NR: 12 oder einem Polypeptid, das mindestens 90% Identität mit den Aminosäuren 446-512 der SEQ ID NR: 12 aufweist, wie ermittelt unter Verwendung des BLASTP Algorithmus mit den Standardparametern;

(d) Aminosäuren 1205-1276 von SEQ ID NR: 12 oder einem Polypeptid, das mindestens 90% Identität mit den Aminosäuren 1205-1276 der SEQ ID NR: 12 aufweist, wie ermittelt unter Verwendung des BLASTP Algorithmus mit den Standardparametern;

(e) Aminosäuren 3025-3093 von SEQ ID NR: 12 oder einem Polypeptid, das mindestens 90% Identität mit den Aminosäuren 3025-3093 der SEQ ID NR: 12 aufweist, wie ermittelt unter Verwendung des BLASTP Algorithmus mit den Standardparametern;

(f) Aminosäuren 4677-4743 von SEQ ID NR: 12 oder einem Polypeptid, das mindestens 90% Identität mit den Aminosäuren 4677-4743 der SEQ ID NR: 12 aufweist, wie ermittelt unter Verwendung des BLASTP Algorithmus mit den Standardparametern;

(g) Aminosäuren 5368-5440 von SEQ ID NR: 12 oder einem Polypeptid, das mindestens 90% Identität mit den Aminosäuren 5368-5440 der SEQ ID NR: 12 aufweist, wie ermittelt unter Verwendung des BLASTP Algorithmus mit den Standardparametern;

(h) Aminosäuren 6852-6907 von SEQ ID NR: 12 oder einem Polypeptid, das mindestens 90% Identität mit den Aminosäuren 6852-6907 der SEQ ID NR: 12 aufweist, wie ermittelt unter Verwendung des BLASTP Algorithmus mit den Standardparametern;

(i) Aminosäuren 6943-7017 von SEQ ID NR: 12 oder einem Polypeptid, das mindestens 90% Identität mit den Aminosäuren 6943-7017 der SEQ ID NR: 12 aufweist, wie ermittelt unter Verwendung des BLASTP Algorithmus mit den Standardparametern;

(j) Aminosäuren 62-123 von SEQ ID NR: 14 oder einem Polypeptid, das mindestens 90% Identität mit den Aminosäuren 62-123 der SEQ ID NR: 14 aufweist, wie ermittelt unter Verwendung des BLASTP Algorithmus mit den Standardparametern; und

(l) Aminosäuren 1533-1589 von SEQ ID NR: 14 oder einem Polypeptid, das mindestens 90% Identität mit den Aminosäuren 1533-1589 der SEQ ID NR: 14 aufweist, wie ermittelt unter Verwendung des BLASTP Algorithmus mit den Standardparametern.

**24.** Methyltransferase-Domäne zur Verwendung bei der Dorrigocinherstellung, wobei die Methyltransferase-Domäne eine Sequenz umfasst, ausgewählt aus der Gruppe bestehend aus:

(a) Aminosäuren 2733-2949 von SEQ ID NR: 12 oder einem Polypeptid, das mindestens 90% Identität mit den Aminosäuren 2733-2949 der SEQ ID NR: 12 aufweist, wie ermittelt unter Verwendung des BLASTP Algorithmus mit den Standardparametern; und

(b) Aminosäuren 4394-4610 von SEQ ID NR: 12 oder einem Polypeptid, das mindestens 90% Identität mit den Aminosäuren 4394-4610 der SEQ ID NR: 12 aufweist, wie ermittelt unter Verwendung des BLASTP Algorithmus mit den Standardparametern.

**25.** Oxidoreduktase-Domäne zur Verwendung bei der Dorrigocinherstellung, wobei die Oxidoreduktase-Domäne eine Sequenz der Aminosäuren 302-637 von SEQ ID NR: 16 umfasst, oder ein Polypeptid, das mindestens 90% Identität mit den Aminosäuren 302-637 der SEC ID NR: 16 aufweist, wie ermittelt unter Verwendung des BLASTP Algorithmus mit den Standardparametern.

## Revendications

**1.** Acide nucléique choisi dans le groupe consistant en

(a) un acide nucléique comprenant une séquence choisie dans le groupe consistant en les SEQ ID NOs : 1 et 22 ; les séquences complémentaires des SEQ ID NOs : 1 et 22, des fragments comprenant au moins 50 nucléotides consécutifs des SEQ ID NOs : 1 et 22 ; et des fragments comprenant au moins 50 nucléotides consécutifs des séquences complémentaires des SEQ ID NOs : 1 et 22 ;

(b) un acide nucléique capable de s'hybrider avec l'acide nucléique de (a) et qui code pour un polypeptide utile pour diriger la biosynthèse de la dorrigocine ;

(c) un acide nucléique ayant au moins 80% ou 99% d'homologie avec l'acide nucléique de (a), comme déterminé par analyse avec BLASTN version 2.0 avec les paramètres par défaut et qui code pour un polypeptide utile pour diriger la biosynthèse de la dorrigocine ;

(d) un acide nucléique comprenant une séquence choisie dans le groupe consistant en les SEQ ID NOs : 3, 5, 7, 9, 11, 13, 15, 17, 19, 21, 24 et les séquences complémentaires de celles-ci ;

(e) un acide nucléique ayant au moins 90% ou 99% d'homologie avec l'acide nucléique de (d), comme déterminé par analyse avec BLASTN version 2.0 avec les paramètres par défaut et qui code pour un polypeptide utile pour diriger la biosynthèse de la dorrigocine ;
(f) un acide nucléique capable de s'hybrider avec l'acide nucléique des SEQ ID NOs : 5, 7, 11,13, 15, 17, 19, 21 et 24 et qui code pour un polypeptide utile pour diriger la biosynthèse de la dorrigocine ;
(g) un acide nucléique ayant au moins 70% d'homologie avec les SEQ ID NOs : 5, 7, 9, 11, 13, 15, 17, 19, 21 et les séquences complémentaires de celles-ci comme déterminé par analyse avec BLASTN version 2.0 avec les paramètres par défaut et qui code pour un polypeptide utile pour diriger la biosynthèse de la dorrigocine ;
(h) un acide nucléique comprenant au moins 50 bases consécutives d'une séquence choisie dans le groupe consistant en les SEQ ID NOs : 3, 5, 7, 9, 11, 13, 15,17, 19, 21, 24 et les séquences complémentaires de celles-ci ; et
(i) un acide nucléique ayant au moins 80% d'homologie avec l'acide nucléique de (h), comme déterminé par analyse avec BLASTN version 2.0 avec les paramètres par défaut et qui code pour un polypeptide utile pour diriger la biosynthèse de la dorrigocine.

**2.** Polypeptide choisi dans le groupe consistant en

(a) un polypeptide comprenant une séquence choisie dans le groupe consistant en les SEQ ID NOs : 2, 4, 6, 8, 10, 12, 14, 16, 18, 20 et 23 ;
(b) un polypeptide comprenant au moins 20 acides aminés consécutifs des polypeptides de (a) ;
(c) un polypeptide ayant au moins 70% d'identité avec le polypeptide de (a), comme déterminé par analyse avec BLASTP version 2.22 avec les paramètres par défaut ;
(d) un polypeptide ayant au moins 99% d'homologie avec le polypeptide de (a) ou (b), comme déterminé avec BLASTP version 2.2.2 avec les paramètres par défaut ; et
(e) un polypeptide ayant au moins 75% d'identité avec le polypeptide de (b), comme déterminé par analyse avec BLASTP version 2.2.2 avec les paramètres par défaut.

**3.** Anticorps capable de se lier spécifiquement à (i) un polypeptide ayant une séquence choisie dans le groupe consistant en les SEQ ID NOs : 2, 4, 6, 8, 10, 12, 14, 16, 18, 20 et 23 ou à (ii) un polypeptide comprenant au moins 25 acides aminés consécutifs de l'un des polypeptides de (i).

**4.** Procédé pour fabriquer un polypeptide ayant une séquence choisie dans le groupe consistant en les SEQ ID NOs : 2, 4, 6, 8, 10, 12, 14, 16, 18, 20 et 23 ou un polypeptide de celles-ci ayant au moins 25 acides aminés consécutifs comprenant d'introduire un acide nucléique codant pour ledit polypeptide, ledit acide nucléique étant opérationnellement lié à un promoteur, dans une cellule hôte.

**5.** Groupe de gènes comprenant des cadres ouverts de lecture codant pour les polypeptides indiqués dans les SEQ ID NOs : 2, 4, 6, 8, 10, 12, 14, 16, 18, 20 et 23.

**6.** Groupe de gènes selon la revendication 5 comprenant un cadre ouvert de lecture choisi dans le groupe consistant en les SEQ ID NOs : 2, 4, 6, 8, 10, 12, 14, 16, 18, 20, 23 et les séquences complémentaires de celles-ci.

**7.** Groupe de gènes selon la revendication 5 ou 6, dans lequel le groupe de gènes comprend un cadre ouvert de lecture ayant au moins 10 bases consécutives d'une séquence de la revendication 6.

**8.** Groupe de gènes selon l'une quelconque des revendications 5 à 7, dans lequel le groupe de gènes comprend un cadre ouvert de lecture ayant au moins 70% d'homologie avec les séquences de la revendication 6, comme déterminé avec BLASTP version 2.2.2 avec les paramètres par défaut.

**9.** Groupe de gènes selon l'une quelconque des revendications 5 à 8, dans lequel le groupe de gènes est présent dans les souches DH10B d'*E. coli* ayant les numéros d'accès IDAC 270201-3 ou mAC 270201-4.

**10.** Cellule hôte procaryote contenant un groupe de gènes selon l'une quelconque des revendications 5 à 9.

**11.** Vecteur d'expression comprenant l'acide nucléique selon la revendication 1.

**12.** Cellule hôte transformée avec le vecteur d'expression selon la revendication 11.

**13.** Procédé pour exprimer un produit d'un gène pour la biosynthèse de la dorrigocine comprenant la culture d'une cellule hôte selon la revendication 10 sous des conditions qui permettent l'expression du produit d'un gène pour la biosynthèse de la dorrigocine.

**14.** Acide nucléique comprenant une séquence d'acides nucléiques qui code pour un domaine d'une dorrigocine polykétide synthétase, dans lequel ladite dorrigocine polykétide synthétase comprend une séquence d'acides aminés choisie dans le groupe consistant en la SEQ m NO : 4, la SEQ ID NO : 10, la SEQ ID NO : 12, la SEQ ID NO: 14 et la SEQ ID NO: 16.

**15.** Acide nucléique selon la revendication 14, dans lequel ledit domaine est un domaine acyle transférase, un domaine thioestérase, un domaine kétosynthétase, un domaine d'interaction, un domaine déshydratase, un domaine kétoréductase, un domaine protéine véhicule acyle, un domaine méthyltransférase ou un domaine oxydoréductase.

**16.** Acide nucléique selon la revendication 14 ou 15, dans lequel ladite séquence d'acides nucléiques code pour une séquence d'acides aminés choisie dans le groupe consistant en

(a) les acides aminés 1 à 276 de la SEQ ID NO : 4 ;
(b) les acides aminés 311 à 529 de la SEQ ID NO : 4 ;
(c) les acides aminés 14 à 444 de la SEQ ID NO : 10 ;
(d) les acides aminés 456 à 604 de la SEQ ID NO : 10 ;
(e) les acides aminés 631 à 901 de la SEQ ID NO : 10 ;
(f) les acides aminés 1091 à 1312 de la SEQ ID NO : 10 ;
(g) les acides aminés 1361 à 1432 de la SEQ ID NO : 10 ;
(h) les acides aminés 1508 à 1939 de la SEQ ID NO : 10 ;
(i) les acides aminés 1950 à 2107 de la SEQ ID NO : 10 ;
(j) les acides aminés 2439 à 2510 de la SEQ ID NO : 10 ;
(k) les acides aminés 2547 à 2976 de la SEQ ID NO : 10 ;
(l) les acides aminés 2989 à 3156 de la SEQ ID NO : 10 ;
(m) les acides aminés 182 à 404 de la SEQ ID NO : 12 ;
(n) les acides aminés 446 à 512 de la SEQ ID NO : 12 ;
(o) les acides aminés 555 à 984 de la SEQ ID NO : 12 ;
(p) les acides aminés 998 à 1190 de la SEQ ID NO : 12 ;
(q) les acides aminés 1205 à 1276 de la SEQ ID NO : 12 ;
(r) les acides aminés 1299 à 1706 de la SEQ ID NO : 12 ;
(s) les acides aminés 1718 à 1852 de la SEQ ID NO : 12 ;
(t) les acides aminés 1863 à 2128 de la SEQ ID NO : 12 ;
(u) les acides aminés 2341 à 2562 de la SEQ ID NO : 12 ;
(v) les acides aminés 2733 à 2949 de la SEQ ID NO : 12 ;
(w) les acides aminés 3025 à 3093 de la SEQ ID NO : 12 ;
(x) les acides aminés 3143 à 3576 de la SEQ ID NO : 12 ;
(y) les acides aminés 3592 à 3761 de la SEQ ID NO : 12 ;
(z) les acides aminés 4012 à 4228 de la SEQ ID NO : 12 ;
(aa) les acides aminés 4394 à 4610 de la SEQ ID NO : 12 ;
(bb) les acides aminés 4677 à 4743 de la SEQ ID NO : 12 ;
(cc) les acides aminés 4774 à 5186 de la SEQ ID NO : 12 ;
(dd) les acides aminés 5199 à 5321 de la SEQ ID NO : 12 ;
(ee) les acides aminés 5368 à 5440 de la SEQ ID NO : 12;
(ff) les acides aminés 5507 à 5918 de la SEQ ID NO : 12 ;
(gg) les acides aminés 5929 à 6093 de la SEQ ID NO : 12 ;
(hh) les acides aminés 6113 à 6384 de la SEQ ID NO : 12 ;
(ii) les acides aminés 6567 à 6796 de la SEQ ID NO : 12 ;
(jj) les acides aminés 6852 à 6907 de la SEQ ID NO : 12 ;
(kk) les acides aminés 6943 à 7017 de la SEQ ID NO : 12 ;
(ll) les acides aminés 7061 à 7496 de la SEQ ID NO : 12 ;
(mm) les acides aminés 7509 à 7666 de la SEQ ID NO : 12 ;
(nn) les acides aminés 7667 à 7803 de la SEQ ID NO : 12 ;
(oo) les acides aminés 62 à 123 de la SEQ ID NO : 14 ;
(pp) les acides aminés 176 à 611 de la SEQ ID NO : 14 ;

(qq) les acides aminés 622 à 777 de la SEQ ID NO : 14 ;
(rr) les acides aminés 790 à 1060 de la SEQ ID NO : 14 ;
(ss) les acides aminés 1242 à 1470 de la SEQ ID NO : 14;
(tt) les acides aminés 1533 à 1589 de la SEQ ID NO : 14 ;
(uu) les acides aminés 1653 à 1943 de la SEQ ID NO : 14 ;
(vv) les acides aminés 1 à 277 de la SEQ ID NO : 16; et
(ww) les acides aminés 302 à 637 de la SEQ ID NO : 16.

**17.** Domaine acyle transférase utile dans la production de dorrigocine, ledit domaine acyle transférase comprenant une séquence d'acides aminés choisie dans le groupe consistant en :

(a) les acides aminés 1 à 276 de la SEQ ID NO : 4 ou un polypeptide ayant au moins 90% d'identité avec les acides aminés 1 à 276 de la SEQ ID NO.: 4, comme déterminé en utilisant l'algorithme BLASTP avec les paramètres par défaut; et
(b) les acides aminés 1 à 277 de la SEQ ID NO : 16 ou un polypeptide ayant au moins 90% d'identité avec les acides aminés 1 à 277 de la SEQ ID NO : 16, comme déterminé en utilisant l'algorithme BLASTP avec les paramètres par défaut.

**18.** Domaine thioestérase utile dans la production de dorrigocine, ledit domaine thioestérase comprenant une séquence d'acides aminés choisie dans le groupe consistant en :

(a) les acides aminés 311 à 529 de la SEQ ID NO : 4 ou un polypeptide ayant au moins 90% d'identité avec les acides aminés 311 à 529 de la SEQ ID NO : 4, comme déterminé en utilisant l'algorithme BLASTP avec les paramètres par défaut ; et
(b) les acides aminés 1653 à 1943 de la SEQ ID NO : 14 ou un polypeptide ayant au moins 90% d'identité avec les acides aminés 1653 à 1943 de la SEQ ID NO : 14, comme déterminé en utilisant l'algorithme BLASTP avec les paramètres par défaut.

**19.** Domaine kétosynthétase utile dans la production de dorrigocine, ledit domaine kétosynthétase comprenant une séquence d'acides aminés choisie dans le groupe consistant en :

(a) les acides aminés 14 à 444 de la SEQ ID NO : 10 ou un polypeptide ayant au moins 90% d'identité avec les acides aminés 14 à 444 de la SEQ ID NO : 10, comme déterminé en utilisant l'algorithme BLASTP utilisant les paramètres par défaut ;
(b) les acides aminés 1508 à 1939 de la SEQ ID NO : 10 ou un polypeptide ayant au moins 90% d'identité avec les acides aminés 1508 à 1939 de la SEQ ID NO : 10, comme déterminé en utilisant l'algorithme BLASTP avec les paramètres par défaut ;
(c) les acides aminés 2547 à 2976 de la SEQ ID NO : 10 ou un polypeptide ayant au moins 90% d'identité avec les acides aminés 2547 à 2976 de la SEQ ID NO : 10, comme déterminé en utilisant l'algorithme BLASTP avec les paramètres par défaut ;
(d) les acides aminés 555 à 984 de la SEQ ID NO : 12 ou un polypeptide ayant au moins 90% d'identité avec les acides aminés 555 à 984 de la SEQ ID NO: 12, comme déterminé en utilisant l'algorithme BLASTP avec les paramètres par défaut ;
(e) les acides aminés 1299 à 1706 de la SEQ ID NO : 12 ou un polypeptide ayant au moins 90% d'identité avec les acides aminés 1299 à 1706 de la SEQ ID NO : 12, comme déterminé en utilisant l'algorithme BLASTP avec les paramètres par défaut ;
(f) les acides aminés 3143 à 3576 de la SEQ ID NO : 12 ou un polypeptide ayant au moins 90% d'identité avec les acides aminés 3143 à 3576 de la SEQ ID NO : 12, comme déterminé en utilisant l'algorithme BLASTP avec les paramètres par défaut ;
(g) les acides aminés 4774 à 5186 de la SEQ ID NO : 12 ou un polypeptide ayant au moins 90% d'identité avec les acides aminés 4774 à 5186 de la SEQ ID NO : 12, comme déterminé en utilisant l'algorithme BLASTP avec les paramètres par défaut ;
(h) les acides aminés 5507 à 5918 de la SEQ ID NO : 12 ou un polypeptide ayant au moins 90% d'identité avec les acides aminés 5507 à 5918 de la SEQ ID NO : 12, comme déterminé en utilisant l'algorithme BLASTP avec les paramètres par défaut ;
(i) les acides aminés 7061 à 7496 de la SEQ ID NO : 12 ou un polypeptide ayant au moins 90% d'identité avec les acides aminés 7061 à 7496 de la SEQ ID NO : 12, comme déterminé en utilisant l'algorithme BLASTP avec les paramètres par défaut ; et

(j) les acides aminés 176 à 611 dé la SEQ ID NO : 14 ou un polypeptide ayant au moins 90% d'identité avec les acides aminés 176 à 611 de la SEQ ID NO : 14, comme déterminé en utilisant l'algorithme BLASTP avec les paramètres par défaut.

**20.** Domaine d'interaction utile dans la production de dorrigocine, ledit domaine d'interaction comprenant une séquence d'acides aminés choisie dans le groupe consistant en :

(a) les acides aminés 456 à 604 de la SEQ ID NO : 10 ou un polypeptide ayant au moins 90% d'identité avec les acides aminés 456 à 604 de la SEQ ID NO : 10, comme déterminé en utilisant l'algorithme BLASTP utilisant les paramètres par défaut ;
(b) les acides aminés 1950 à 2107 de la SEQ ID NO : 10 ou un polypeptide ayant au moins 90% d'identité avec les acides aminés 1950 à 2107 de la SEQ ID NO : 10, comme déterminé en utilisant l'algorithme BLASTP utilisant les paramètres par défaut ;
(c) les acides aminés 2989 à 3156 de la SEQ ID NO : 10 ou un polypeptide ayant au moins 90% d'identité avec les acides aminés 2989 à 3156 de la SEQ ID NO : 10, comme déterminé en utilisant l'algorithme BLASTP utilisant les paramètres par défaut ;
(d) les acides aminés 998 à 1190 de la SEQ ID NO : 12 ou un polypeptide ayant au moins 90% d'identité avec les acides aminés 998 à 1190 de la SEQ ID NO : 12, comme déterminé en utilisant l'algorithme BLASTP utilisant les paramètres par défaut ;
(e) les acides aminés 1718 à 1852 de la SEQ ID NO : 12 ou un polypeptide ayant au moins 90% d'identité avec les acides aminés 1718 à 1852 de la SEQ ID NO : 12, comme déterminé en utilisant l'algorithme BLASTP utilisant les paramètres par défaut ;
(f) les acides aminés 3592 à 3761 de la SEQ ID NO: 12 ou un polypeptide ayant au moins 90% d'identité avec les acides aminés 3592 à 3761 de la SEQ ID NO : 12, comme déterminé en utilisant l'algorithme BLASTP utilisant les paramètres par défaut ;
(g) les acides aminés 5199 à 5321 de la SEQ ID NO : 12 ou un polypeptide ayant au moins 90% d'identité avec les acides aminés 5199 à 5321 de la SEQ ID NO: 12, comme déterminé en utilisant l'algorithme BLASTP utilisant les paramètres par défaut ;
(h) les acides aminés 5929 à 6093 de la SEQ ID NO: 12 ou un polypeptide ayant au moins 90% d'identité avec les acides aminés 5929 à 6093 de la SEQ ID NO : 12, comme déterminé en utilisant l'algorithme BLASTP utilisant les paramètres par défaut ;
(i) les acides aminés 7509 à 7666 de la SEQ ID NO.: 12 ou un polypeptide ayant au moins 90% d'identité avec les acides aminés 7509 à 7666 de la SEQ ID NO : 12, comme déterminé en utilisant l'algorithme BLASTP utilisant les paramètres par défaut ; et
(j) les acides aminés 622 à 777 de la SEQ ID NO : 14 ou un polypeptide ayant au moins 90% d'identité avec les acides aminés 622 à 777 de la SEQ ID NO : 14, comme déterminé en utilisant l'algorithme BLASTP utilisant les paramètres par défaut.

**21.** Domaine déshydratase utile dans la production de dorrigocine, ledit domaine déshydratase comprenant une séquence d'acides aminés choisie dans le groupe consistant en :

(a) les acides aminés 631 à 901 de la SEQ ID NO : 10 ou un polypeptide ayant au moins 90% d'identité avec les acides aminés 631 à 901 de la SEQ ID NO : 10, comme déterminé en utilisant l'algorithme BLASTP utilisant les paramètres par défaut ;
(b) les acides aminés 1863 à 2128 de la SEQ ID NO : 12 ou un polypeptide ayant au moins 90% d'identité avec les acides aminés 1863 à 2128 de la SEQ ID NO : 12, comme déterminé en utilisant l'algorithme BLASTP utilisant les paramètres par défaut ;
(c) les acides aminés 6113 à 6384 de la SEQ ID NO : 12 ou un polypeptide ayant au moins 90% d'identité avec les acides aminés 6113 à 6384 de la SEQ ID NO : 12, comme déterminé en utilisant l'algorithme BLASTP utilisant les paramètres par défaut ;
(d) les acides aminés 7667 à 7803 de la SEQ ID NO : 12 ou un polypeptide ayant au moins 90% d'identité avec les acides aminés 7667 à 7803 de la SEQ ID NO : 12, comme déterminé en utilisant l'algorithme BLASTP utilisant les paramètres par défaut ; et
(e) les acides aminés 790 à 1060 de la SEQ ID NO : 14 ou un polypeptide ayant au moins 90% d'identité avec les acides aminés 790 à 1060 de la SEQ ID NO : 14, comme déterminé en utilisant l'algorithme BLASTP utilisant les paramètres par défaut.

**22.** Domaine kétoréductase utile dans la production de dorrigocine, ledit domaine kétoréductase comprenant une séquence d'acides aminés choisie dans le groupe consistant en :

(a) les acides aminés 1091 à 1312 de la SEQ ID NO : 10 ou un polypeptide ayant au moins 90% d'identité avec les acides aminés 1091 à 1312 de la SEQ ID NO : 10, comme déterminé en utilisant l'algorithme BLASTP utilisant les paramètres par défaut ;
(b) les acides aminés 182 à 404 de la SEQ ID NO : 12 ou un polypeptide ayant au moins 90% d'identité avec les acides aminés 182 à 404 de la SEQ ID NO : 12, comme déterminé en utilisant l'algorithme BLASTP utilisant les paramètres par défaut ;
(c) les acides aminés 2341 à 2562 de la SEQ ID NO : 12 ou un polypeptide ayant au moins 90% d'identité avec les acides aminés 2341 à 2562 de la SEQ ID NO : 12, comme déterminé en utilisant l'algorithme BLASTP utilisant les paramètres par défaut ;
(d) les acides aminés 4012 à 4228 de la SEQ ID NO : 12 ou un polypeptide ayant au moins 90% d'identité avec les acides aminés 4012 à 4228 de la SEQ ID NO : 12, comme déterminé en utilisant l'algorithme BLASTP utilisant les paramètres par défaut ;
(e) les acides aminés 6567 à 6796 de la SEQ ID NO : 12 ou un polypeptide ayant au moins 90% d'identité avec les acides aminés 6567 à 6796 de la SEQ ID NO : 12, comme déterminé en utilisant l'algorithme BLASTP utilisant les paramètres par défaut ; et
(f) les acides aminés 1242 à 1470 de la SEQ ID NO : 14 ou un polypeptide ayant au moins 90% d'identité avec les acides aminés 1242 à 1470 de la SEQ ID NO : 14, comme déterminé en utilisant l'algorithme BLASTP utilisant les paramètres par défaut.

**23.** Domaine protéine véhicule acyle utile dans la production de dorrigocine, ledit domaine de protéine véhicule acyle comprenant une séquence d'acides aminés choisie dans le groupe consistant en :

(a) les acides aminés 1361 à 1432 de la SEQ ID NO : 10 ou un polypeptide ayant au moins 90% d'identité avec les acides aminés 1361 à 1432 de la SEQ ID NO : 10, comme déterminé en utilisant l'algorithme BLASTP utilisant les paramètres par défaut ;
(b) les acides aminés 2439 à 2510 de la SEQ ID NO : 10 ou un polypeptide ayant au moins 90% d'identité avec les acides aminés 2439 à 2510 de la SEQ ID NO : 10, comme déterminé en utilisant l'algorithme BLASTP utilisant les paramètres par défaut ;
(c) les acides aminés 446 à 512 de la SEQ ID NO : 12 ou un polypeptide ayant au moins 90% d'identité avec les acides aminés 446 à 512 de la SEQ ID NO : 12, comme déterminé en utilisant l'algorithme BLASTP utilisant les paramètres par défaut ;
(d) les acides aminés 1205 à 1276 de la SEQ ID NO : 12 ou un polypeptide ayant au moins 90% d'identité avec les acides aminés 1205 à 1276 de la SEQ ID NO : 12, comme déterminé en utilisant l'algorithme BLASTP utilisant les paramètres par défaut ;
(e) les acides aminés 3025 à 3093 de la SEQ ID NO : 12 ou un polypeptide ayant au moins 90% d'identité avec les acides aminés 3025 à 3093 de la SEQ ID NO : 12, comme déterminé en utilisant l'algorithme BLASTP utilisant les paramètres par défaut ;
(f) les acides aminés 4677 à 4743 de la SEQ ID NO : 12 ou un polypeptide ayant au moins 90% d'identité avec les acides aminés 4677 à 4743 de la SEQ ID NO: 12, comme déterminé en utilisant l'algorithme BLASTP utilisant les paramètres par défaut ;
(g) les acides aminés 5368 à 5440 de la SEQ ID NO : 12 ou un polypeptide ayant au moins 90% d'identité avec les acides aminés 5368 à 5440 de la SEQ ID NO: 12, comme déterminé en utilisant l'algorithme BLASTP utilisant les paramètres par défaut ;
(h) les acides aminés 6852 à 6907 de la SEQ ID NO : 12 ou un polypeptide ayant au moins 90% d'identité avec les acides aminés 6852 à 6907 de la SEQ ID NO : 12, comme déterminé en utilisant l'algorithme BLASTP utilisant les paramètres par défaut ;
(i) les acides aminés 6943 à 7017 de la SEQ ID NO : 12 ou un polypeptide ayant au moins 90% d'identité avec les acides aminés 6943 à 7017 de la SEQ ID NO : 12, telle que celle-ci est déterminée en utilisant l'algorithme BLASTP avec les paramètres par défaut ;
(j) les acides aminés 62 à 123 de la SEQ ID NO: 14 ou un polypeptide ayant au moins 90% d'identité avec les acides aminés 62 à 123 de la SEQ ID NO : 14, comme déterminé en utilisant l'algorithme BLASTP utilisant les paramètres par défaut ; et
(l) les acides aminés 1533 à 1589 de la SEQ ID NO : 14 ou un polypeptide ayant au moins 90% d'identité avec les acides aminés 1533 à 1589 de la SEQ ID NO : 14, comme déterminé en utilisant l'algorithme BLASTP utilisant les paramètres par défaut.

**24.** Domaine méthyltransférase utile dans la production de dorrigocine, ledit domaine méthyltransférase comprenant une séquence d'acides aminés choisie dans le groupe consistant en :

(a) les acides aminés 2733 à 2949 de la SEQ ID NO : 12 ou un polypeptide ayant au moins 90% d'identité avec les acides aminés 2733 à 2949 de la SEQ ID NO: 12, comme déterminé en utilisant l'algorithme BLASTP utilisant les paramètres par défaut ; et
(b) les acides aminés 4394 à 4610 de la SEQ ID NO: 12 ou un polypeptide ayant au moins 90% d'identité avec les acides aminés 4394 à 4610 de la SEQ ID NO: 12, comme déterminé en utilisant l'algorithme BLASTP utilisant les paramètres par défaut.

**25.** Domaine oxydoréductase utile dans la production de dorrigocine, ledit domaine oxydoréductase comprenant une séquence d'acides aminés 302 à 637 de la SEQ ID NO : 16 ou un polypeptide ayant au moins 90% d'identité avec les acides aminés 302 à 637 de la SEQ ID NO : 16, comme déterminé en utilisant l'algorithme BLASTP utilisant les paramètres par défaut.

Dorrigocin/Migrastatin (DORR) locus

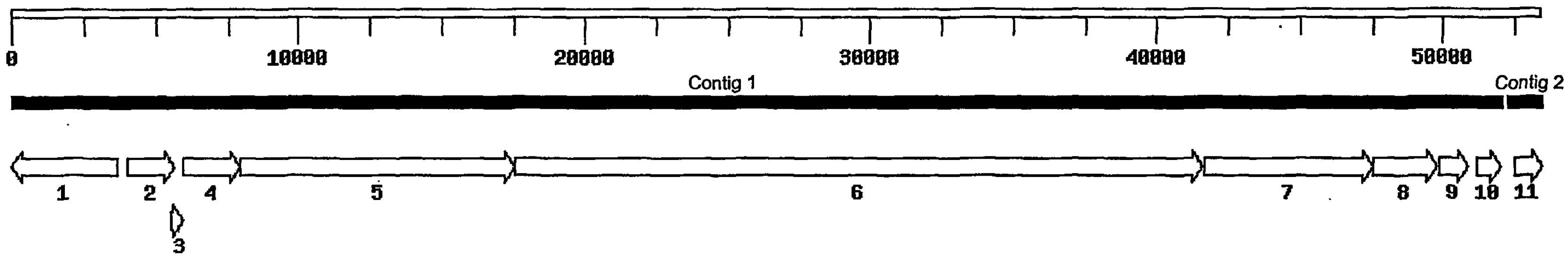


**Domain architecture of synthase components**

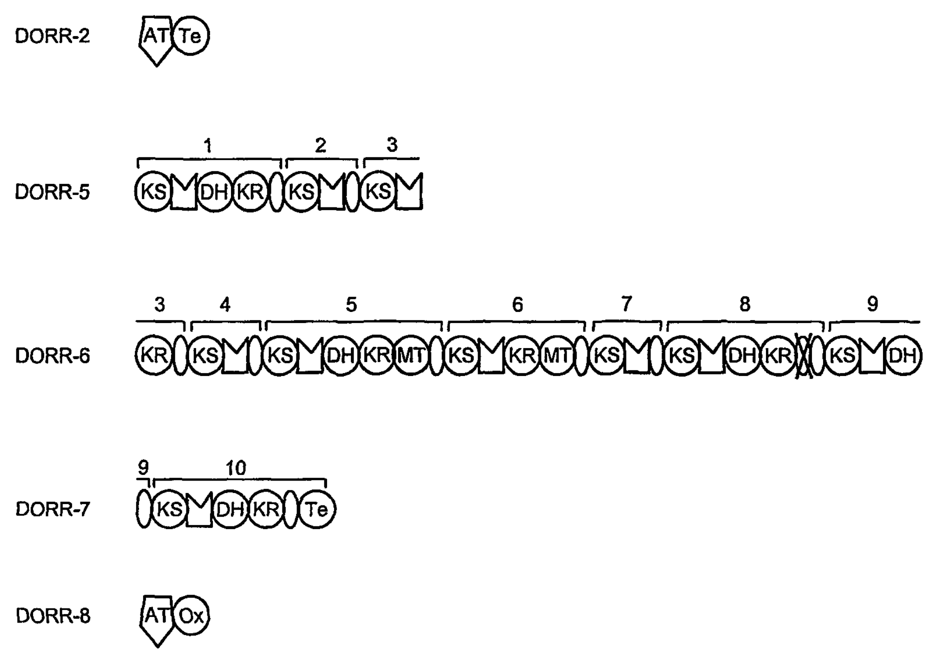


Figure 1

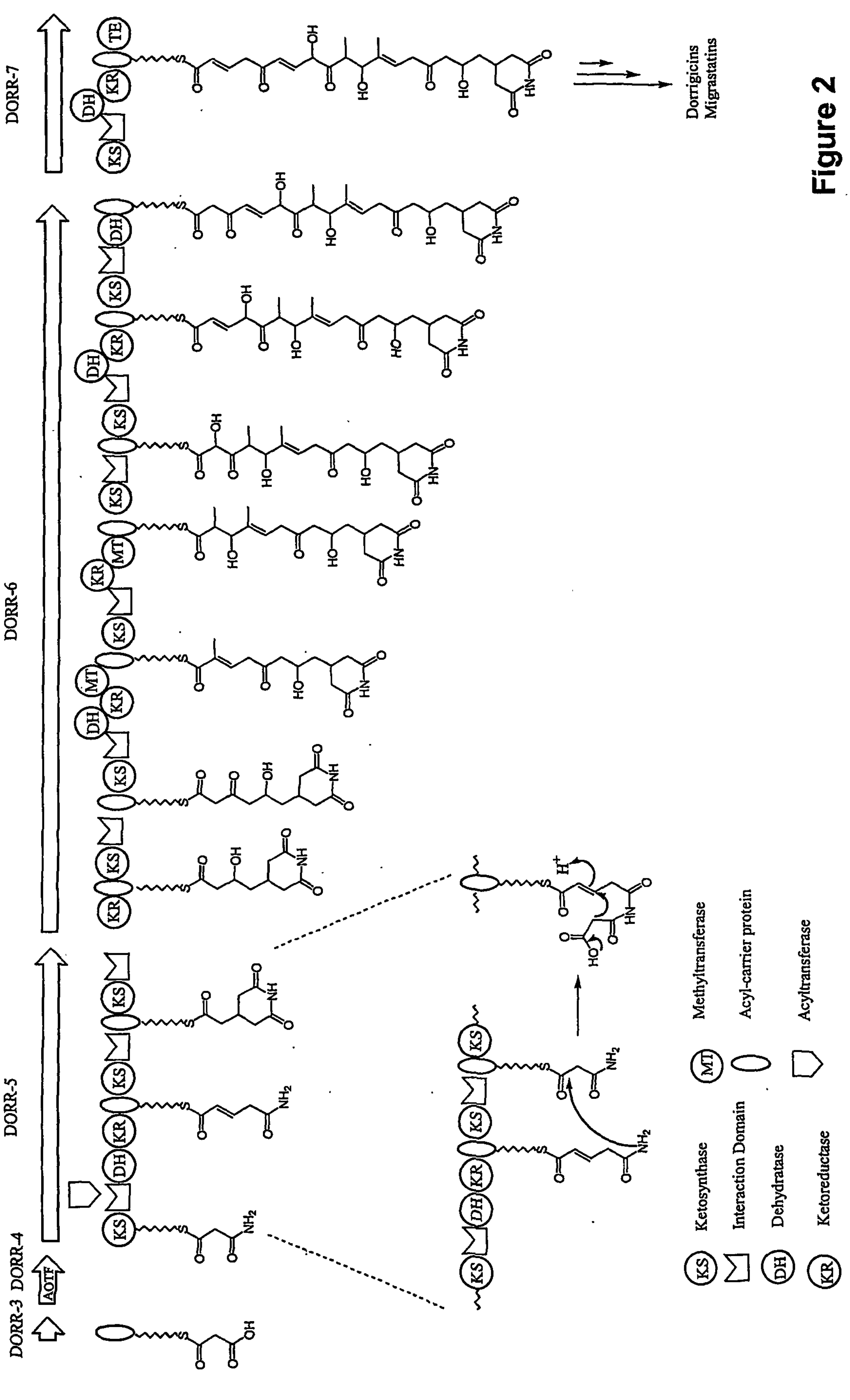
DORR-3
DORR-4
AOTF
DORR-5
DORR-6
DORR-7
Dorrigicins
Migrastatins
KS Ketosynthase
Interaction Domain
DH Dehydratase
KR Ketoreductase
MT Methyltransferase
Acyl-carrier protein
Acyltransferase

Figure 2

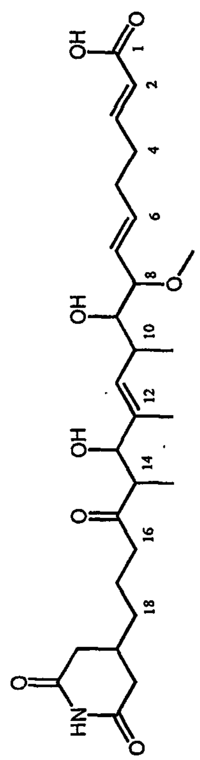

**Dorrigocin A**

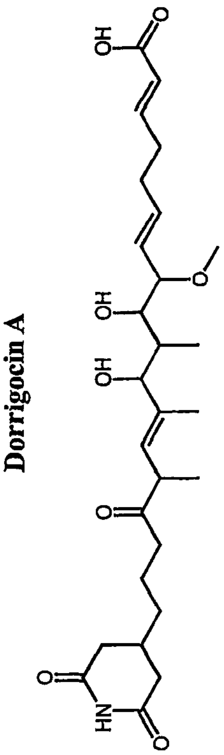

**Dorrigocin B**

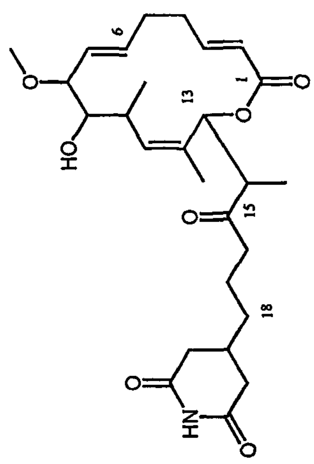

**Migrastatin**

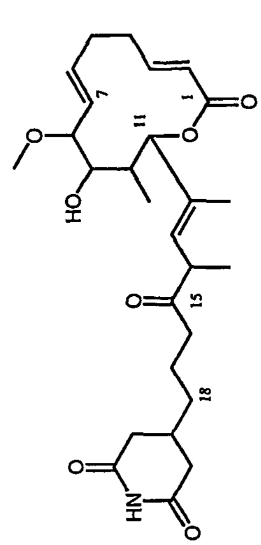

**Isomigrastatin**

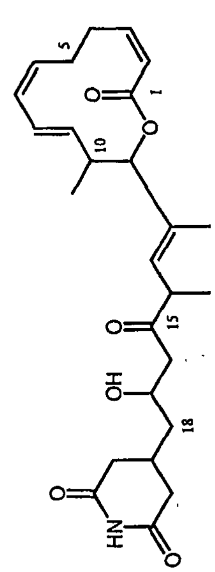

**Lactimidomycin**

**Figure 3**

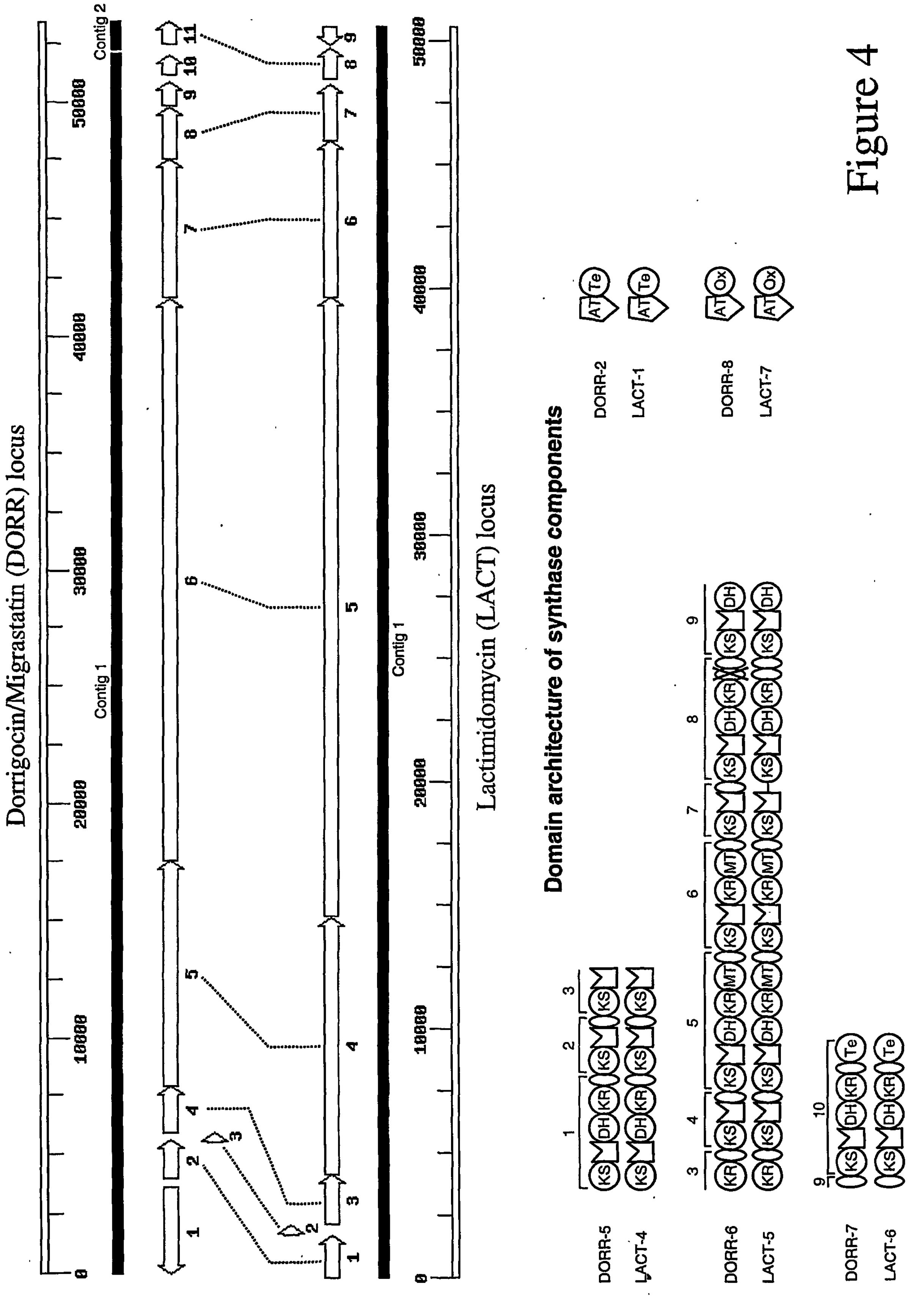

Dorrigocin/Migrastatin (DORR) locus
0
10000
20000
30000
40000
50000
Contig 1
Contig 2
Lactimidomycin (LACT) locus
Domain architecture of synthase components
DORR-5
LACT-4
DORR-6
LACT-5
DORR-7
LACT-6
DORR-2
LACT-1
DORR-8
LACT-7
KS
DH
KR
MT
Te
AT
Ox


Figure 4

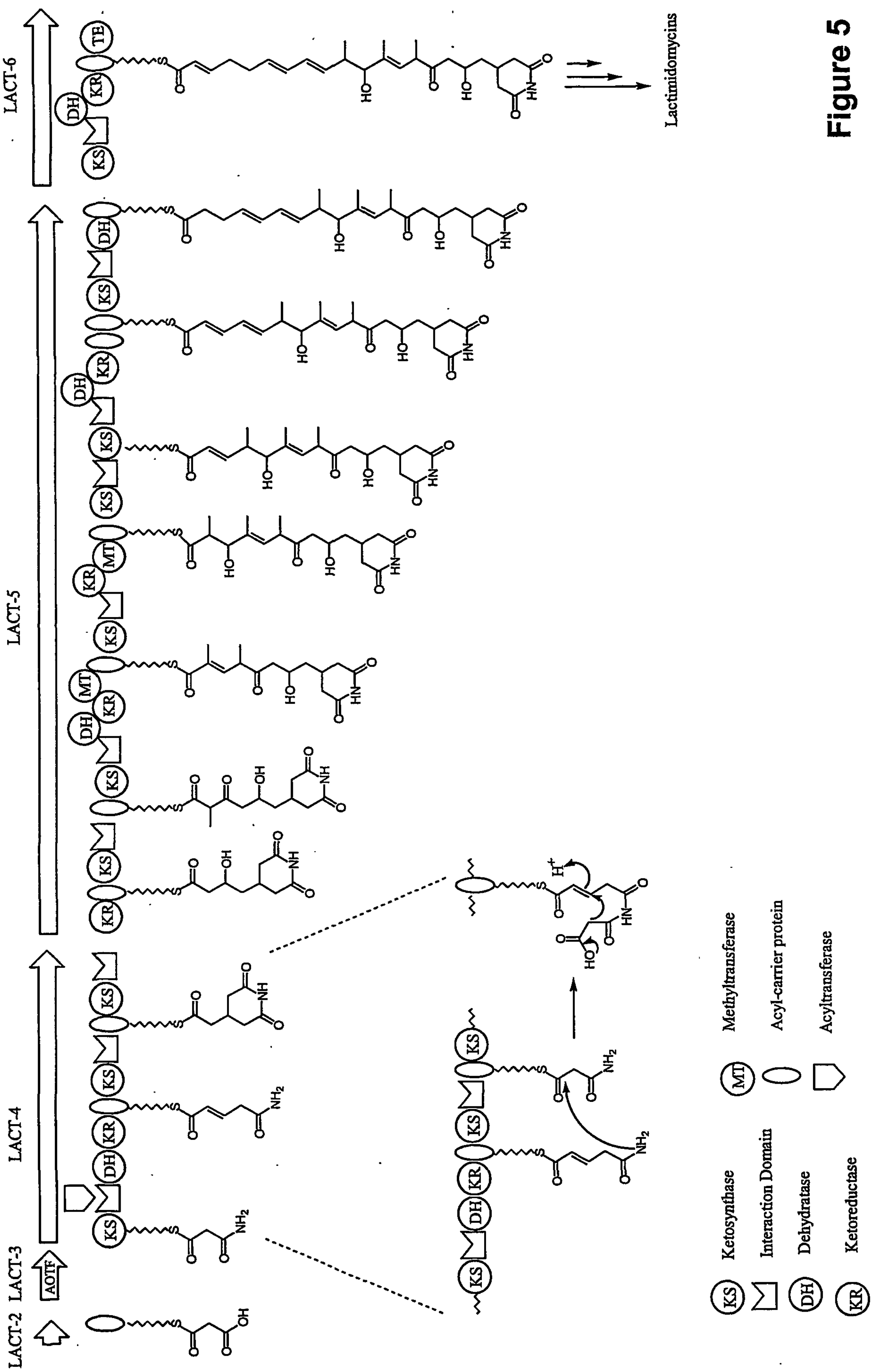
LACT-2
LACT-3
AOTF
LACT-4
LACT-5
LACT-6
KS
DH
KR
MT
TE
Lactimidomycins
Ketosynthase
Interaction Domain
Dehydratase
Ketoreductase
Methyltransferase
Acyl-carrier protein
Acyltransferase

**Figure 5**

# Figure 6

```
                    AT
DORR-2|AYTT      |VLFPGQGSQARGMGAGLFDRYPELTALASDILGYDLPRLCLEDPDGRLDDTRCTQPALYV
LACT-1|AYTT      |VLFPGQGSQSKGMGRELFDRFPETTASACDVLGYDLRELCLENPEGRLDDTRYTQSALYT
                  *********::***   ****:** ** *.*:***** .****:*:******* **.***.

                             active site
                               ------
DORR-2|AYTT       VNALSYQDSLERGEPEGGYLLGHSLGEYNALHAAGAFDFETGLKLVLKRGELMARAPDGA
LACT-1|AYTT       VNALEYLGSLEDGAPEGDYLLGHSLGEYNALLAAGVFDFETGLRLVLKRGELMARARHGG
                  ****.* .*** * ***.************* ***.*******:************ .*.

DORR-2|AYTT       MLAVVGPDAGEVRAFLSEEGLSRLDVANINTPVQTVLSGARDEIERAHKTLDAHGTRVAR
LACT-1|AYTT       MLAVLGPGEEELRRTLAEEGMERLDVANVNTPAQTVLSGPVEEIERAQRHFDERRVRTAR
                  ****:**.   *:*  *:***:.******:***.******. :*****:: :* : .*.**

DORR-2|AYTT       LKVSAAFHSRFMAAARDEFAAFLKGFRFAPLRATVIANLTARPYTDQDVAATLSEQICGS
LACT-1|AYTT       LKVSAAFHSRLMRPAREEFRAFLRGFRFASPRATVIANVSARPYG--DVAEMLSEQIAGP
                  **********:* .**:** ***:*****. *******::****    ***  *****.*.

                                                AT
DORR-2|AYTT       VQWLDSVRYLLERTTAGHCREVGGGGVLTRMIRQID||-------------------AAPA
LACT-1|AYTT       VRWLESVRYVLERTSAERGREAGPGTVLTRMLRQID||GVSGAGNSPSVSASGSVPAASAPA
                  *:**:****:****:* : **.* * *****:****                     :***

                                                           Te
DORR-2|AYTT       RGIPQPKPKPKPKPKPK------------PQSRPRLFCIA||YAGGDERAYAGLAEHCPDVD
LACT-1|AYTT       ESPSAPAASTSGSARPVGRATAATADAVREPTRPLLICAP||YAGGDERSYAGLAEQLPEAD
                  .. . * .... ..:*                :** *:* . *******:******: *:.*

                                                      active site
                                                         -----
DORR-2|AYTT       VVTLERPGRGRRASEPLLREPAAIVDDLLRQLRGRLDAPYALYGHSLGARLAFLLCRALR
LACT-1|AYTT       VVTLERPGRGRRVSEPLLTEPGPVVEDMLSRIRDRVSRPYALYGHSLGARLVHLLARRLR
                  ************.***** **..:*:*:* ::*.*:. *************..**.* **

DORR-2|AYTT       AERLPAPAHLFVSGESGPALPSRERHTWELPADAFWDHLKELGGIPAELWEHPDLMAYYE
LACT-1|AYTT       EEGLPGPRHLFVSGECGPSRPSRERYTSDLPTDAFWKHLRELGGVPDELFEYEDLTTFYE
                   * **.* *******.**: *****:* :**:****.**:****:* **:*: ** ::**

DORR-2|AYTT       PVIRADFTALGAYRHEDAPPLDVPVTAMAGEDEWFTRADLEAWQRESTRPLTTHRFPGDH
LACT-1|AYTT       RVLRADFTVLGACAYTPAAPLDCPVTAMTGDEEGLTEADVGAWQRETTAPLTARVFTGDH
                   *:*****.***   :   *.*** *****:*::* :*.**: *****:* ***:: *.***

                           Te
DORR-2|AYTT       FFIRAQWPALARIVAAGLAA||P------
LACT-1|AYTT       FFIRAHWPGVARVVAAGLGA||RRPAGTR
                  *****:**.:**:*****.*
```

# Figure 7

```
DORR-3|ACPI        MKQELKKHMEERFMFEFDSDITEDTDLFKAGILDSFGYISLMTHIEEEYGVPLGDE-ILG
LACT-2|ACPI        MEQELKQYMEEQFMFEFDSEITEDTDLFKAGVLDSFGYISLIGHIEGEYGVKFGEEALLG
                   *:****::***:*******:***********:*********: *** **** :*:* :**

DORR-3|ACPI        NVAVSLSGIVAFVDAARLRAAGSR
LACT-2|ACPI        NVAVTFAGLVESVASARRQTAESK
                   ****:::*:*  * :** ::* *:
```

# Figure 8

```
DORR-4|AOTF     MCGIAGFYGSPLPPQEYETLIHGMLAQIEHRGPDEAGCFLDDRLAMGTVRLSIIDLSTGS
LACT-3|AOTF     VCGIAGFHASPLHPESYRDIAGAMLAQIEHRGPDEAGCFLDDRTAMGTVRLSIIDLASGS
                :******:.*** *:.*. :  .******************** ************:.**

DORR-4|AOTF     QPVGSADGRYWLCYNGELYNYRELREQLTARGFVFRTESDTEVVLAAWVAWGLDCLPRFN
LACT-3|AOTF     QPVGSPDGRYWLCYNGELYNYRELRAELAGRGVSFRTESDTEVVLMAWAHWGRSCLERFN
                *****.******************* :*:.**. *********** **. ** .** ***

DORR-4|AOTF     GAFAFALYDSATGELHLVRDRFGKRPLYVARHRGAWLFASEMKAFLAYPDFRFAFDEAQL
LACT-3|AOTF     GAFAFALKDTVTGELHLARDRFGKRPLYVARHGDAWLFASEMKAFLAYPGFEFAFDEEHL
                ******* *:.******.************** .***************.*.***** :*

DORR-4|AOTF     ASVFATWTPLPGQSGYQGIEQIPMGEYLSVRGDEVRRGRWATLDLAQGPAPESEQEAAEL
LACT-3|AOTF     ASTFATWTPLPAQSGYRGVEQLPMGEYLTVRGTETERGRWASLDLTGGEPPATEDEAVDL
                **.********.****:*:**:******:*** *..*****:***: * .* :*:**.:*

DORR-4|AOTF     VRADLEAAVDVRLRSDVEVGVYASGGLDSSIIAHIAAQRTSRPLRTFSIEFEDAEFDESA
LACT-3|AOTF     VRADLEAAVDLRLRSDVEVGVYASGGLDSSILAHLTKERAGLPPRTFSIQFEDAEFDETA
                **********:********************:**:: :*:. * *****:********:*

DORR-4|AOTF     EQAELAAHLGTRHSTVRVTDEDVADAFPEAVRHAEVPVFRTAFVPMYLLAGHVRSEGIKV
LACT-3|AOTF     EQEELTKHLGTHHSTVRVSDSDVVETFPEAVRHAEVPVFRTAFVPMYLLAQHVRSEGVKV
                ** **: ****:******:*.**.::************************ ******:**

DORR-4|AOTF     VLSGEGADEAFLGYGIFKDTLLLSTWHELDDDTRLRRMSQLYPYLSHFSGEDGHRRMLGL
LACT-3|AOTF     VLSGEGADEAFLGYGIFKDARLLSEWHELDEATRMRRMAQLYPYLRHFSGEDGHRRMLGL
                *******************: *** *****: **:***:****** **************

DORR-4|AOTF     YRQFTEETLPGLFSHQMRFQNGRFAARLLKNPGDPFAALGELVAGEPGYAQLTPVQKAQW
LACT-3|AOTF     YRQFTEETMPGLFSHQMRFQNGRFAVRLLKDAGDPFAAVRRLVAEEPGYAELSAVQKAQW
                ********:****************.****:.******: .*** *****:*:.******

DORR-4|AOTF     LEFRTLLSGYLLSTQGERMALAHGVENRCPFLDPAVVRRAASVNLRFGDPYDEKYLLKCA
LACT-3|AOTF     LEFRTLLSGYLLATQGERMALAHGVENRCPFLDPAVVRRAASVNGRFGDPYDEKYLLKRA
                ************:******************************* ************* *

DORR-4|AOTF     YADVLPERIVKKGKFPYRAPDSAAFVRSRPDYRELLTDPGTLDEIGVLDARFVKRFTDRV
LACT-3|AOTF     YGDVLPERIVSKGKFPYRAPDSAAFVRSRPDYRDLLADPGTLGDIGVLDERFVRRFTDRV
                *.********.**********************:**:*****.:***** ***:******

DORR-4|AOTF     FDSPPEQIGTKENQAFVSLASTVWLHHWYVRGNARRRAPLGVPLYVVDRRSGALSA
LACT-3|AOTF     FDRPPERIGTKENQAFVLLASTVWLHHWYVRGNARRDTPLAVPLYVVDRRSSALPA
                ** ***:********** ****************** :**.**********.**.*
```

# Figure 9A

```
                                                          KS1
DORR-5|PKUN    MKKQNGVLADD---------------------R||DIAVIGLSLRLPGSRTPEEFWSHLAE
LACT-4|PKUN    MKEESGALPEEGPVGTAVGTAADGAAGGPVDGQ||DIAVVGLSLRLPGARNPEEFWEHLAA
               **::.*.*.::                     :  ****:********:*.*****.***

DORR-5|PKUN    GRSLISEVPERRWRKEDHLGHPRREFNKTNSVWGGFVDDADCFDADFFQISPREAQSMDP
LACT-4|PKUN    GRSLISEVPERRWRKEDHLGNPRREFNKTNSVWGGFVDDADCFDAEFFHVSPREARSMDP
               ********************:************************:**::*****:****

DORR-5|PKUN    QQRMALELSWHALEDAGYRADRVAGSRTGVFMGVCHWDYAELMEQEVEEIDAYYPTGAAY
LACT-4|PKUN    QQRMALEMSWQALEDAGYRADRVAGSRTGVFMGVCHWDYAELIEKEVSEVDAYYPTGAAY
               *******:**:*******************************:*:**.*:**********

DORR-5|PKUN    AIIANRVSHHFDFRGPSVVNDTACAGSLVAVQQAVQALQAGDCDLALAGGVNLTWSPRHF
LACT-4|PKUN    AIIANRVSHHFDFRGPSVVNDTACASSLVAVQQAVQALQSGDCDHALAGGVNLTWSPRHF
               *************************.*************:**** ***************

DORR-5|PKUN    IAFAKAGMLSPDGLCRAFDANANGYVRGEGGGIVLLKRAADARRDGDAVHAVIKGIGSNH
LACT-4|PKUN    IAFAKAGMLSPDGLCRAFDADANGYVRGEGGGVVLLKRAADARRDGDPVHAVIKGIGSNH
               ********************:***********:**************.************

DORR-5|PKUN    GGRTSSLTVTNPAAQAELIAGVYRKAGIAPETVTYVETHGPGTPVGDPIEVRGLKQAFVD
LACT-4|PKUN    GGRTSSLTVTNPAAQAELIAGIYRRAGIAPESVSYIETHGPGTPVGDPIEVSGLKRAFAQ
               *********************:**:******:*:*:*************** ***:**.:

DORR-5|PKUN    LGADRPGEAPAHRCGIGSVKTNIGHLEGAAGIAGMLKVILAMRHRKLPATVNFRKLNPLI
LACT-4|PKUN    LGEGREAEPSGHRCGIGSVKTNIGHLEGAAGIAGMLKVILAMRHRKLPATVNFRRLNPLI
               **  .*  .*...**************************************:*****

                                                     KS1
DORR-5|PKUN    DLDGSPLYVLDRLTDWTAEGSAPLRAGVSSFGFGGTNAHVLLEA||----------AEPVA
LACT-4|PKUN    TLDGSPLYVVDRLTDWETDGDGTLRAGVSSFGFGGTNAHVVLEA||PGGHAAEVTDAEAVT
                ********:******  ::*...******************:***            **.*:

                                            ID1
DORR-5|PKUN    ATEDAG------------------EQW||LPVSAMDEDRLRETCARLARWVRTRIEQNDAPS
LACT-4|PKUN    DAEADADVDGGPDEGPDEGAEPRALR||LPVSADDEERLRELCRSLAEWARAREAEGTAPP
                :*   .                      ***** **:**** *  **.*.*:*  :. **.

DORR-5|PKUN    LTDAARTLREGRVSMRERVVFRASGIEEWAAQLESVAAGDG--PPADCPRGRAGTEAPDG
LACT-4|PKUN    LADIARTLREGRVPMRERAVFRARSVAEWAEQLTALAEGTGGEPPAGCLRGRAEDGAGDG
               *:* *********.****.****  .: *** **  ::* * *   ***.* ****    * **

                                                                          ID1
DORR-5|PKUN    LDADDLTALAERWLEKGRWDKFAAAWAQGLAVDWAPWPERGRRVHVPGYAFARTPHWFRT
LACT-4|PKUN    LDADDVAALTARWRERDEEEKFAAAWTRGLPVDWAQWPAEGRRVHLPGQVFQRTPHWFRP
               *****::**: ** *:.. :******::**.**** ** .*****:** .* *******.

                                                                        DH1
DORR-5|PKUN    D||RN-----ETTG--------RPERGATN------TAPAPLG---------||EGKPEGGS
LACT-4|PKUN    D||EQPRGEAESAGGAAAQRDTAPERDAASGSERGPGAPEPAGPVGGPGLPG||EGVQDGRG
               *  .:       *::*        ***.*:.         ** * *         **  :* .

DORR-5|PKUN    WTFPLHFDATQGFVRDHRVNGARIVPGVVALELVTVAAERAAAAGARAGLTPRIRNAVWI
LACT-4|PKUN    WHFPLRFAATDPFVRDHLVLGARIVPGVVALEAVTAAAARPAVAGARAGAAPHIRNAVWV
               * ***:* **: ***** * ************ **.** *.*.****** :*:******:

DORR-5|PKUN    RPLLVGDTVLSPQLRLTPAAD----GYDYAITDEHGTQYTSGRVEYGEAAAAEKTDPGAL
LACT-4|PKUN    RPLRVDGQVLETSLRLTPAGPESGGGYDWAVTDAAGTPYSSGRVEYADGPAPAATDLDAL
               *** *.. **...******.     ***:*:**  ** *:******.:..*.  ** .**

DORR-5|PKUN    RERFPQRVDTAEGYAALRSSGIEHGPALRGLNALHRGPDGVLAELRLPAGAPEGMALQPA
LACT-4|PKUN    HRRHTRPVEVAGGYAALYASGIEHGPALRALHTLRAGPEGLLAELRLPAEPAAGAALQPA
               :.*..: *:.* ***** :**********.*::*: **:*:********  .. * *****
```

# Figure 9B

```
DORR-5|PKUN    ILDSALLAALALGSADG------------GWRRPAAPVVPFALDRLTVHAATTSTMWAWL
LACT-4|PKUN    VLDSALLAVLALGTGGGDGTEGTGGTDGAGWRRPDAPAVPFALDGLTAYAPTTATTWAWL
               :*******.****:..*            ***** **.****** **.:*.**:* ****

                                                           DH1
DORR-5|PKUN    RPAGSGTAGDMARSDIDLFDDNGRLCVRLAGYTSRELPTAEPAAVQA||PE----------
LACT-4|PKUN    RPAGGRRPG---AADIDLFDERGRLCARLTGYTSRELSTGSPALREA||RVSAPAPGEGAA
               ****.  .*    :******:.****.**:*******.*..**   :*

DORR-5|PKUN    ----------GELLEVTGVWEEAPA------PAPAAGQATPVGPVTVLNAALDGDLAAAS
LACT-4|PKUN    GEEAPGKDAPGELLEVTGRWTPAPLGLPAAEAGPAAAQSGAAAPVTVLNAALDADLVAAS
                         ******** *  **        ..***.*: ...**********.**.***

DORR-5|PKUN    AARLGMDIRQLAGPAEATDATDAVAMKAAFEACYPQVRQLLGQGRQVLVVAPGAPDSPVY
LACT-4|PKUN    AARLGMDVEHLAVP---RDAGDADAMKAAFAACYPHVRRLVGQSRRVLLVAPGAPDSPVF
               *******:.:** *     ** ** ****** ****:**:*:**.*:**:**********:

DORR-5|PKUN    APLAALLKTAQQENPSFRGRLVLLDGYDPRDADRFERVVSAEAGAG-------DDTEVAY
LACT-4|PKUN    APLAALLKTAHQENPSFHGTTVLLEGFDPRDSARFEQVVRTEAAATADAAGGAADEEVAH
               **********:******:*  ***:*:****: ***:** :**.*             * ***:

                                  KR1
DORR-5|PKUN    DAQDRRLRHGFVELPRGEA||GESLLRDGGVYWITGGAGGLGLLLAERLCERRRATVVVSG
LACT-4|PKUN    TADGRRLRHETAELPHGTT||GESLLAEGGVYWITGGAGGIGLLLAERLCLRYGATVVLSG
                *:.*****  .***:* : ***** :************:********* *  ****:**

DORR-5|PKUN    RSADSRAIEALRARLFHGEVAYRRTDVTDADAVRDAVADIRARYGRLDGVFHAAGVLDDG
LACT-4|PKUN    RSPAAPAADALASRLTRGTLAYRGADVTDQDSVDALVAAVLAEHGRIDGVFHAAGVLDDG
               **.  : * :** :** :* :*** :**** *:*   ** : *.:**:*************

DORR-5|PKUN    YLASKPLAGTAAVLAPKVDGATSIDDATRAHGLDFLLLFGSVAGAFGNAAQADYAAANAF
LACT-4|PKUN    YLTAKPLAGTEAVLAPKVDGVTCVDRATRAGAPGFLLVFGSVAGAFGNAAQAGYAAANAY
               **::****** *********.*.:* ****  . .***:**************.******:

DORR-5|PKUN    LDAFAARRQAAGSVTRSVDWPLWADGGMRVDDASLAYLRKRTGTVPLPSETGLDALERAL
LACT-4|PKUN    LDAFAARRQAAGLTTRAVDWPLWAEGGMRVDDASLKYLRKRTGTVPLPSGTGLDALERAL
               ************ .**:*******:********** ************* **********

                                KR1
DORR-5|PKUN    HSAAPVRRVVLFGERSKLRGYAGLDR||VAKPEPRTSGAQRNT-----------------A
LACT-4|PKUN    HTGSPVRRVVLYGDRPALRVYAGLDR||PQVTGARSGSASASAPGSSSGSVSAVRGEGTGT
               *:.:*******:*:*. ** ******   . .*:..*. .:                  :

                              ACP1
DORR-5|PKUN    APAVLEESELVA||RTQDLLRNLFAEVTLQDAEHILAEEKLETYGIESISIVELTSKLEDT
LACT-4|PKUN    APAALTDAELLV||RTQDFLREQFAEVTLQDAEQIHPEEKLETYGIESISIVDLTSRLEDV
               ***.* ::**:. ****:**:  **********:* .**************:***:***.

                                ACP1
DORR-5|PKUN    FGSLPKTLFFEYVDLQGVAGYFVAE||HRDRLLELFAPEAP--------APEAPAPEAPAP
LACT-4|PKUN    FGSLPKTLFFEYVDLKGVAEYFVAE||HRARLTELFAPEEPQASEAAEPAPEEPVAPAPVP
               ***************:*** ***** ** ** ****** *          *** *.. **.*

                                                                      KS2
DORR-5|PKUN    EAPAPEEPAPEGPAVEEPPAAAPTP-----AVRPSVEAAAGRARPAWADPERH||DIAVIG
LACT-4|PKUN    VEPAAAAPAPAPAPVPAPPAPTAAPGTSVEAVPAPVPASVPTPRPAPAG--NG||DIAVVG
                **.  ***  ..*  ***.:.:*      ** ..* *:.  .*** *.  .   ****:*

DORR-5|PKUN    MAGRYPGADTLEEFWELLSEGRHSFEPVPESRWRHGDIYFDERDVDGKTVVKTGTFLRDV
LACT-4|PKUN    MAGRYPGADTLEEFWELLSEGRHSFEPVPSSRWPHGDLYFDERDVLGKTTVRTGTFLRDV
               *****************************.*** ***:******* ***.*:********

DORR-5|PKUN    EAFDPRYFNISQRDAELLSPEVRLFLQAGVEALEDAGYSRETLRRRYDGDVGVLVGSMNN
LACT-4|PKUN    DAFDPRYFSISQRDAELLSPEVRLFLQAGVTALEDAGYSKETLRRRYDGDVGVLVGSMNN
               :*******.********************* ********:********************
```

# Figure 9C

```
DORR-5|PKUN   SYSLYGFQNMLMRGTATSGSELGVMANMLSYHYGFTGPSVFLDTMCSSASACVHQAVRML
LACT-4|PKUN   SYAYYGFENMLMRGTAMSGSEVGVMANMLSYYYGFTGPSMFVDTMCSSSSACVHQALSML
              **: ***:******** ****:*********:*******:*:******:*******: **

DORR-5|PKUN   RSGECRMTVVGGINLMLHPFDLIATSQAHFTTKSAEVVRSYGLGADGTILGEGVGTLVLK
LACT-4|PKUN   RGGECRMVVVGGINLMLHPYDLIATSQAHFTTKSAEVVRSYGLGADGTILGEGVGTLVLK
              *.*****.***********:****************************************

DORR-5|PKUN   PLAEAVADGDHVYGVIKGSGMTNAGVRNGFTVPSPQQQARAIEKALDDAAVDARTISYLE
LACT-4|PKUN   PLAEAVADGDHVYGVIKGSGMTNAGVRNGFTVPSPQQQARAIERALDDAAVDARTVSYLE
              *******************************************:***********:****

DORR-5|PKUN   GHGSATSLGDPIEIKGAALAFGRDTQDLGFCALGSVKSNVAHLLSGSGMAGLTKVLLQLK
LACT-4|PKUN   GHGSATSLGDPIEIKGASLAFGRDTRDVGYCAIGSVKSNVAHLLSGSGLVGLTKVLLQLR
              *****************:*******:*:*:**:***************:.*********:

DORR-5|PKUN   HRTLAPSLHAGTLSSAIDFEETPFVVQRHRDTWRRPVVGGEEAPRRAGVTSIGAGGINVH
LACT-4|PKUN   HRTLAPSLHSETLSPAIDFGSTPFVVQRERAEWRRPVVHGAEVPRRAGVTSIGAGGINVH
              *********: ***.****  .*******.*  ****** * *.*****************

               KS2                    ID2
DORR-5|PKUN   IVVEEYD||GQVVAAPERG||RPRLLVFSAMTPQALQSVLRAMHEHVRETAPGLDALAYTLQ
LACT-4|PKUN   LIVEEFD||GTVNSAPDDG||GSQLLVFSAMTPQALGTVLRDAHRHVADEAPALNALAYTLQ
              ::***:* * * :**: *  .:************ :***  *.** : **.*:*******

DORR-5|PKUN   TGKNELPCRLAFVADDIADAQARLARLSAVDWTAESPGVPAGVHFTASTLRRRRTADAAT
LACT-4|PKUN   TGKNELPCRLAFVAHGTADAEARLAALAAVDWTSGAPALPDAVRFTESTLRKRRSVAAAD
              **************.. ***:**** *:*****.: :*.:* .*:** ****:**:. **

DORR-5|PKUN   VEQALRDGKQAELAQHWADGASVDWDLLWPAGSRPAKPSLPAYPFDKVRCWYPEDDDAPS
LACT-4|PKUN   VERALAQADLAELAGYWISGAAVDWDLLWPSGTRPAKLALPAYPFEKVRCWYPGFDDAPS
              **:** :.. **** :* .**:********:*:****  :******:*******  *****

              ID2
DORR-5|PKUN   VLRPL||AFARRAHPWVGVNASDLGGVRYTLRLRGDELLDYVYTVGRKRRYATVALLDAAL
LACT-4|PKUN   VLRPL||AFTRRGHPWVGVNRSDLHGVRFALELTGDELLDYVHTVGRTRRFTSVALLDGAL
              ***** **:**.******* *** ***::*.* ********:****.**:::*****.**

DORR-5|PKUN   AFARLAGLEGPLRLRNAQWAALPSPADTPETFTWRLGTSGD---------------GVHR
LACT-4|PKUN   AFARLAGLDGALRLRDARWAELPSPGDATEVFEWRLALSGEGASGDAASGGGASGAGGHR
              ********:*.****:*:** ****.*:.*.* ***. **:                * **

DORR-5|PKUN   VELWHADEATLRFAADVVPS-------APAEDASMPQMSSAPATLDRDDFYAALGTAGLD
LACT-4|PKUN   VELWQAERGTLHFSAEVVPATAVAAGAVDARPADAAALLAAPVTLDGDAFYSALGEAGVD
              ****:*:..**:*:*:***:           . *. *. . : :**.*** * **:*** **:*

DORR-5|PKUN   ARPYARSVEGVTELDAHRLLVRVAEPAMCQDPHKQHVHLPAWALVGLTQGVQHAWGRADA
LACT-4|PKUN   ARPYARAVTGVTEAGGRRLLVRVAEPAMCQDPHKQHVSIPAWVLAGLAQGVQHATGRPRT
              ******:* ****  ..:******************** :***.*.**:****** **. :

DORR-5|PKUN   AVVRVGSVQGEQWERTRAIVLARTSDAVFHAAFLDEDGRVLGRVEDAEFTAGDLEPALPG
LACT-4|PKUN   TALRAAALYGADLTDTRALLLEPVAEATFRITFLDGDGRALGAVEDAEFTAGTLPPSLEG
              :.:*..:: * :    ***::*  .::*.*: :*** ***.** ********* * *:* *

DORR-5|PKUN   EAGRALVALPQASRP--------VLETPVGTGEWQQSEAVRPEAEPSVTVAAVADGPAA||
LACT-4|PKUN   GAVPVRAGLPGAARPSATASAPASASVPVPALAAAPVAPAVPVEPVEPAAEADADAGDA||
               *  . ..** *:**             ..** :           .. *     . :. * **.  *

              ACP2
DORR-5|PKUN   LVASLRETVADLLKFDLADIDLDTHFHAYGFESIALAKLASELNGVLGTDLTPAVFFECS
LACT-4|PKUN   LVAVLRETVADLLKFELDEIDLDTHFHAYGFESIALARLASELNGLLGTDLSPVVFFECP
              *** ***********:* :*****************:*******:*****:*.*****.
```

# Figure 9D

```
                    ACP2
DORR-5|PKUN    DIRSLAEYLLDR||YGPELS----------------LPTSADAPAP---VAATRPSPVPM
LACT-4|PKUN    DIRSLAAHLRER||YDAETAARAVRGTGGGTGTDAARAPTPAPAPAVGAASAATAPAPLSS
               ****** :* :* *..* :                **.* ***      *** *:*:.

                             KS3
DORR-5|PKUN    PAPGPDDD-----||AVAIVGAAGRFPGADDLDTFWQQLRAGEDLIADYPGDRFDGGPYAE
LACT-4|PKUN    AEPVSDHEADYPG||AVAVVGVAGRFPGAPDADTFWQRLRAGDDLIGEYPGDRFDER-YTG
               . * .*.:       ***:**.******* * *****:****:***.:*******    *:

DORR-5|PKUN    VVARADFPKFAGRIEGVDRFDADFFHLSRLEAELMDPQHRLALETVWAALENGGYAPARL
LACT-4|PKUN    VVARSDFPKFAGVLDDVDRFDAGFFNLSRLEAELMDPQHRLALETVWAALEDGGYAPGRL
               ****:*******  ::.******.**:************************:*****.**

DORR-5|PKUN    PENTGVYFGVSGSDYHHLLNASGVAPDGFTATGNAHSMLANRISYVLDVHGPSEPVDTAC
LACT-4|PKUN    PENTGVYVGVSGSDYHHLLNASGVAPDGFTATGNAHSMLANRISFVLDVHGPSEPVDTAC
               *******.************************************:***************

DORR-5|PKUN    SSSLVALHRAVEHIRSGRCEMAIAGGVNLLLSVDTFAATHMAGMLSPDGRCKTFSAGADG
LACT-4|PKUN    SSSLVALHRAVESIRSGRCDMALAGGVNLLLSIDTFAATQMAGMLSPDGRCKTFSADADG
               ************ ******:**:*********:******:****************.***

DORR-5|PKUN    YVRSEGVAAVLLKPLAQAQRDGDAIWGVVRGSAENHGGRAGSLTAPNGKAQAALIQDAMR
LACT-4|PKUN    YVRAEGVAAVLLKPLERALADGDPVWGVVRGSAENHGGRAGSLTAPNAVAQTALIREAMR
               ***:***********  :*    ***.:*********************.  **:***::***

DORR-5|PKUN    GIDPDSIGYVEAHGTGTGLGDPVEVNALDSAYRALRTAEGGPPHAARPCALGSVKTNIGH
LACT-4|PKUN    GTDPDSVGYVEAHGTGTGLGDPVEVGALDSAYRALRSDRGRVESGTAPVALGSVKTNIGH
               * ****:******************.**********:  .*      .: * ***********

DORR-5|PKUN    AESAAGLAGVLKVLLAMRHRELPPALHCDRLNPHLPLDGG-FEVVRELRRWEPCTDATGR
LACT-4|PKUN    AESAAGLAGVLKVLLAMRHGELPPTLHCDRLNPHLPLSGGGFEVVREVRRWEPRLDADGR
               ******************* ****:************.** ******:*****    ** **

                                      KS3                              ID3
DORR-5|PKUN    PWPLRAGVSSFGFGGANAHVVLEAPP||VPP------------------------A||PAEP
LACT-4|PKUN    PWPLRAGVSSFGFGGANAHVVLEAAP||AAARERAVRETASRSASVRSAHGTQGAP||QAVA
               ************************.* ...                          .  * .

DORR-5|PKUN    ARPTAPQAIVLSARDDDRLRATAGRLRDFLDRARRDGHAPDLADLAFTLQVGREAMERRL
LACT-4|PKUN    PQAVGPQIVAVSARDGERLRIVAERLRDFLRREHGAGRAPATADLARTLQTGREAMEARL
               .:...** :.:****.:*** .* ****** * :  *:**  **** ***.****** **

DORR-5|PKUN    GFVVGSMDDVLGTLDRFFAGDEPSGWHTGGIRRSRGAGVRREAEQAPEVTRALHDGRLDR
LACT-4|PKUN    AFVAEETGDVLDVLDRFLKGEEPDGWHTGALRRSRGAGVRRDRAQDPRVTRALRDGDLDA
               .**.  .***..****: *:**.*****.:**********:  * *.*****:** **

                                                               ID3
DORR-5|PKUN    VTALWCDGAPVDWQAMHPTGERRAVRLPAYPFACDRYWVPAVGTAPVPPPAAP||------
LACT-4|PKUN    AAALWCEGALVDWQSLHPPGERRTVRLPSYPFARERYWVPTDGAAPPPETGGP||GGVEDG
               .:****:** ****::**.****:****:**** :*****: *:** *  ...*

DORR-5|PKUN    ----------VPPPAAEPAFETDARAALLDAVLDGRAGPDALSRT
LACT-4|PKUN    GVEYGTGSGAAQLGDSGSAFDAGALAAVLDAVLDGRADPDDLART
                         .     : .**::.* **:*********.** *:**
```

# Figure 10A

```
DORR-6|PKUN    VTWNGMNVSRNILRVPEWRDEPARGRTAPPGNRRLVVLCDTPDADVTDLRRHLPGVSVAR
LACT-5|PKUN    -------VSRNILRVPAWRDEPSRGQAAPAGVRRLAVLCDVPDAEAALLRQHSPRLPVVL
                      ********* *****:**::**.* ***.****.***:.: **:* * :.*.

DORR-6|PKUN    VDSGDDGPAAAYEHAATLLLGELQRLLNQPAGGPRSVQVVCREGTPYGYAGLIGMLRTAA
LACT-5|PKUN    VESRDDGPAAAYEHAATRLLAELQRLLGRPAAGPCRVQVVCRESTPQGWAGLLGMLRTAA
               *:* ************* **.******.:**.** *******.** *:***:*******

DORR-6|PKUN    QEDPALHGQLIECTQRPSGEELAGVLRAEYGGQAADHVR----------YTGGRRQVRAWA
LACT-5|PKUN    QEDPRLRGQLIEFDRLPGGAELARVLDEEAAEEADHVRRAAGAAGTGTGTGAVRQVRHWS
               **** *:*****  : *.* *** **  *  .: *****            **. **** *:

                              KR3
DORR-6|PKUN    AAP------RAAAPPPV||WKADGVYLISGGAGGVGRLVAADIARHAPGARVVLCGRSPA-
LACT-5|PKUN    AARSAGRASSAGNPAPV||WRPGGVYLVSGGAGGLGRLLAADVRRHAPGAVTVVCGRGPAP
               **           *. *.** *:..****:******:***:***: ****** .*:***.**

DORR-6|PKUN    VPGPGQPGPGTEYRRVDVADADAVAELVNSLVRTYGRLDGVVHAAGLISDDYVIRKSHQD
LACT-5|PKUN    WQGAEPPADGVEYHSVDVTDRAAVAALVDRVLSAHGRLDGVVHAAGLLADDYVVRASHRE
                *.  *. *.**: ***:*  *** **: :: ::************::****:* **::

DORR-6|PKUN    AQQVLAPKAAGLVNLDEATRRLPLDFLAAFSSGAGTLGNPGQADYAAANGFLDAYLTHRA
LACT-5|PKUN    TQRVLAPKVAGLVHLDEATRELPLDFLAAFSSAAGTLGNAGQAGYAAANGFLDAYQTHRA
               :*:*****.****:******.***********.******.***.*********** ****

                                                                          KR3
DORR-6|PKUN    GLAAAGERHGASVSIGWPLWQDGGMSVPAEDVPALTARFGRPLGTDTALRALHGALALGT
LACT-5|PKUN    ALAEAGERHGRSLSVGWPLWRDGGMTVPDEQLPELTERFGRPLTTGTALTALHAALALGT
               .** ****** *:*:*****:****:** *::* ** ****** *.*** ***.******

DORR-6|PKUN    PH||LLVMD----EESGVDEESGVD-------------------------EEGPQEAETQ
LACT-5|PKUN    PH||VLVRDGAEADETGAVNATGAGTATGIATEVEVPAVNEAVGTAVDDALEDDAPEGDRK
               ** :** *     :*:*. : :*..                          *:.. *.: :

                        ACP3
DORR-6|PKUN    QTGPAELRAH||VLPLLKELIAETVRLDPARLDAAAPLDGFGIDSLAVTRLNRRFAQWFGA
LACT-5|PKUN    GTPAVEPRLR||VLPALKQLVAETVRLDPAALDAAAPLDGFGIDSLAVTRLNRRFAQWFGA
                *  ..* *  : *** **:*:********* ******************************

                                    ACP3
DORR-6|PKUN    LPKTVLYQYPTLNDLAGHLAEQH||ADGCRRWLGDVPDVAAAPAG---------------
LACT-5|PKUN    LPKTLLYQYPTLNELAGYLAEHH||PEGCRRWLADTASPSLSPSASASASPSPSPATSTSV
               ****:********:***:***:*  .:******.*...  : :*:.

                                                                   KS4
DORR-6|PKUN    -----------TPATAAAPRKARP------------------RPADADE||PIALIGLSGR
LACT-5|PKUN    SAPSAQERRPSTPVAAGAVRTAGTNGTSGAAAPVSAEAPVPARTSPVDE||PIAVIGLHGR
                          **.:*.* *.* .                   *.: .** ***:*** **

DORR-6|PKUN    YPDAPTLEAFWENLRAGRESVREVPAERWPLDAFYEPDPQRAVQQGASYSKWGAFLDDFA
LACT-5|PKUN    YPGAPTLDAFWENLRSGRDGVTEIPAERWPLEGFWEPDVERAVREGASYSKWGGFLDGFA
               **.****:*******:**:.* *:*******:.*:*** :***::********.***.**

DORR-6|PKUN    RFDAAFFGIAPRDAADMDPQERLFVESAWSVLEDAGYTRQRLAEQHASSVGVFAGITKTG
LACT-5|PKUN    QFDALFFGIAPREAADMDPQERLFVESAWSVLEDAGYTRRRLAEQHRSRVGVFAGITKTG
               :*** *******:**************************:****** * ***********

DORR-6|PKUN    FDRHRP---------PATDGLPPA-PRTSFGSLANRVSYLLDLHGPSMPIDTMCSSSLTA
LACT-5|PKUN    FDRHRPAAPAETDASSATGGVPPASPRTSFGSLANRVSYLLDLRGPSMPVDTMCSASLTA
               ******         .**.*:*** ******************:*****:*****:****

DORR-6|PKUN    IHEACEHLRHGACELAIAGGVNLYLHPSSYVELCRSRMLATDGHCRSFGAGGDGFLPGEG
LACT-5|PKUN    VHEACEHLRHGACELAVAGGVNLYLHPSTYVELCRSRMLARGGECRSFGTGGDGFVPGEG
               :**************:***********:*********** .*.*****:*****:****
```

# Figure 10B

```
DORR-6|PKUN      VGAVLLKPLSAAEADGDPIHAVIVGSAINHGGRTNGYTVPNPRAQAALIRDALDRAGVSA
LACT-5|PKUN      VGTVLLKPLSKAEADGDPVHAVILGSAINHGGRTNGYTVPNPRAQAELIREAMDRAGVSA
                 **:******* *******:****:********************** ***:*:*******

DORR-6|PKUN      AGIGYIEAHGTGTRLGDPVEIDGLTQAFAPDAGGSGACALGSVKSNIGHLEAAAGIAGLT
LACT-5|PKUN      DEVGCVEAHGTGTALGDPVEIEGLAQAFADRTDTAAPCALSSVKSNIGHLEAAAGIAGLT
                   :*  :******* *******:**:****   :.  :..***.*******************

DORR-6|PKUN      KAVLQLQHGEFAPTLHAEQTNPDIDFAATPFTLQTGGAPWPRP-ADGGPRRAGISSFGAG
LACT-5|PKUN      KLVLQLRHGELAPTLHAEVPNPDIDFGSVPFALQTAAAPWPRTGGNSGRRIAGLSSFGAG
                 * ****:***:*******  .******.:.**:***..*****.  .:.* * **:******

                           KS4 <-|                 |-> ID4
DORR-6|PKUN      GANAHVIVAEYRSA||TPAP----------ATPAPSA||RPVLLPLSARTTEDLHARAGQLS
LACT-5|PKUN      GANAHVVVAEYTGA||PAARTSAPAVADGSAATAGSG||RPVLLPLSARTPEDLRARAVQLA
                 ******:**** .*  ..*               *:.* *. ***********.***:*** **:

DORR-6|PKUN      DLLRNGAPVDLPAVAATLQTGREEMAERVCFVASTPGEWLDQLGAFLADSDSDSDSDSDS
LACT-5|PKUN      DWLDSRDAVDLTSVAATLQTGREAMDERLCCVASTPGEWREQLRAFADDP----------
                 * *  .   .***.:********** * **:* ******** :** **   *.

DORR-6|PKUN      DSDSDSDSDSGSGSEAEAEVPWSRGRVRATRETLAALAEKDELRALVTRWINRGDWHDLA
LACT-5|PKUN      ----------------EREGPWHRGRVRATGEALAALAEKDELRALVGRWTARGEWAELA
                                 * * ** ******* *:************** **  **:* :**

                                                 ID4 <-|
DORR-6|PKUN      AFWAKGMPLDWTRLHAGADTPARVHLPAYPFAGRQFWFGPA||-----------------G
LACT-5|PKUN      AFWAKGMPLDWSRLYADGRVPARLHLPAYPFAGRRYWPGPA||DVRNTADAQAPRTSTPSP
                 ***********:**:*.. .***:**********::* ***

                                              |-> ACP4
DORR-6|PKUN      SEHPATTP------VAAPSCSTAAGAAD||VERILLDALAAALQMPVAEIERRRPFADYGL
LACT-5|PKUN      STLSTSTPGASRPVAVAPVAAAPSAESY||IERVLLDALGEALQMTPAEIDPRRPFADYGL
                 *   .::**       ..** .::.:. :  :**:*****. ****. ***: *********

                                                   ACP4 <-|
DORR-6|PKUN      DSILGVNLVHTLNTALGTALETTDLFDHGTVERLHAFLVGT||YGDALHAPASP-------
LACT-5|PKUN      DSILGVHLVNVLNETLGTGLETTDLFDHGTAERLRAFLTET||YGGTVTVPDGTGAAAEFV
                 ******:**:.** :***.***********.***:***. * **.:: .* ..

                             |-> KS5
DORR-6|PKUN      --AAVAPAPDDD||AIAVVGMAARYADAEDPRALWDHLMAGHDLVEPVTRWPLGQDVSCRS
LACT-5|PKUN      PDAPVPAREADD||PVAVVGMAARYGDAEDPRALWDRLLAGDDLVEPVTRWDLGPEVTCRA
                   *.*..    ** .:*********.**********:*:**.********* ** :*:**:

DORR-6|PKUN      GSFVRGIDQFDPVFFAISGVEATTMDPQQRIFLEQCWNALEDAGYTGERLTNRNCGVYAG
LACT-5|PKUN      GSFVRGMDRFDPVFFAISGVEAAHMDPQQRIFLEQCWNALEDAGYTGERLRERNCGVYVG
                 ******:*:*************: *************************** :******.*

DORR-6|PKUN      CYAGDYHDQLDARPPAQALWGTMGSVVASRIAYHLDLKGPALTTDTSCSSSLVSLHLACR
LACT-5|PKUN      CYAGDYYDSIGDRAPAQALWGTMGSVVASRIAYQLDLKGPALTTDTSCSSSLVSLHLACR
                 ******:*.:. *.*******************:**************************

DORR-6|PKUN      DLLSGDADMAIAGGVFIQTTSRLYESASRAGMLSPSGRCHSFDARADGFVPGEGAGAVVL
LACT-5|PKUN      DLRTGAADMAIAGGVFLQTTPRLYEAATRAGMLSPTGRCHSFDSRADGFVPGEGAGAVVL
                 ** :* *********:***.****:*:*******:*******:****************

DORR-6|PKUN      KRLADARRDGDHIYGVVRGSGINQDGTTNGITAPSAASQEQLLRDVHARSGIEPGGIQLV
LACT-5|PKUN      KRLSDALRDGDHVYGLVRATGVNQDGTTNGITAPSAASQEALLREVHA--GVAPGGVQLV
                 ***:** *****:**:**.:*:****************** ***:***  *: ***:***

DORR-6|PKUN      EAHGTGTQLGDPIEFRALTRAFEDAPAGSAVLGSIKTNIGHTQFAAGIAGVIKALLALEH
LACT-5|PKUN      EAHGTGTQLGDPIEFRALSRVFGDAPAGSVVLGSVKTNLGHTQFAAGIAGVLKALLALQE
                 ******************:*.* ******.****:***:************:******:.
```

# Figure 10C

```
                                                                                   KS5
DORR-6|PKUN     RQIPPSLHFQEANRAVVLDGGPFTVTTAPQPWTAPARGPRRAAVSSFGASGTNAHVVLEE
LACT-5|PKUN     QRVPPSLHFAEANARVPLDGSPFTVATTAQPWPEPAEGPRRAAVSSFGASGTNAHVVLEE
                :::****** ***   * ***.****:*:.***. **.**********************

                  <-|           |-> ID5
DORR-6|PKUN     HP||------VPRTTGAG||GEHAFLLSARTPAALRAVAERLLAHLDREPGLPADAVAFSLA
LACT-5|PKUN     HP||PVRATTGPESAGGD||GEAAFLLSARTPAALRAVAERLLARIEREPGLPARQVAYSLA
                **          *.::*.. ** *********************:::*******  **:***

DORR-6|PKUN     AGRSHFAHRLAVVAAGLPDLAARLRSWLSG--TAGDTVLQGETAADPRPVGGVRAPAPAA
LACT-5|PKUN     AGRRHFPHRLAVVATGLPALAARLRAWLADEQPGGEGTLLHGVAHAGTRQAALGGLAPAE
                *** **.*******:*** ******:**:.   ..*: .*   .*      ..: . ***

                                                     ID5 <-|         |-> DH5
DORR-6|PKUN     LAAAYVRGEADRFADSFASASRRQVPLPTYPFERQRYWTDTTD||-------TGESQ||GLK
LACT-5|PKUN     LAAAYVGGAEGPFAESFPAGARRQVPLPTYPFERQRYWAEGTD||GHAVPAAAGTSA||VEP
                ****** *   . **:**.:.:*****************:: **          :* *

DORR-6|PKUN     DTDGAAYRLRLGGEEFFLADHHVGGRAVLPGVLSLEFARRAVTGG--------------
LACT-5|PKUN     GGRRTAYRTRLTGEEFFLADHRVGGRTVLPGVLTLEAVRRAVTAGDGGDGTGIGTGGGTG
                .    :*** ** *********:****:******:** .*****.*

DORR-6|PKUN     SFAPVGLRDVVWPEPFPVGDGGAELRVDRDGDAFRVLRDGSAVHAQGRIATPGSPVP-TP
LACT-5|PKUN     VPTPLRLRDVVWPAPFPVGADGAELRVDLDGDAFAVRQDGSSVHAQGRWTMVPAPAASTS
                  :*: ******* ***** .******* ***** * :***:****** :    :*.. *.

DORR-6|PKUN     LDALRARCGRRTLSRSQCRAALDAVGIRHGDRLRAIDTLAVGDGEVLARLVLPDGARDG-
LACT-5|PKUN     LETLRERCARRTLTREQCRAALEAVGIRHGERLRAIEQLSVGDGELLARLVLPATVETGT
                *::** **.****:*.******:*******:*****: *:*****:*******   .. *

DORR-6|PKUN     ----AFALHPAMLDSAVQAVVGLYGDATGTLDEQRGAPALPFALDAADFFAPTTERMWAH
LACT-5|PKUN     QATETFGLHPAMLDSAIQAVVGLYGDETGALGERPDAPALPFALDTADVLAPTTDRMWAH
                    :*.*********:********* **:*.*: .*********:**.:****:*****

                                                       DH5 <-|
DORR-6|PKUN     LRHTEGYTPSADRDVTKVDIDVYDDDGQLSASLRGYAFRRMTAP||SG------------
LACT-5|PKUN     LRWADGYAPGEAGEVTKTDIDLYDDAGRLCVRLRGYASRRVTPA||ATGSATAAPAGTDDA
                ** ::**:*..  :***.***:*** *:*.. ***** **:*..  :

DORR-6|PKUN     AAPRATLLAPVWDALPVVPAEPWPHPRTRVVLLGGTPEERDGLRRRYPDATVLDPHADEP
LACT-5|PKUN     DAPRAQLLAPVWDAQPHADGPRSPEPGAHVVLLGGTPEERDGLRRLVADVTVVEPERHAS
                 **** ******** * . .   *.* ::****************  .*.**::*. . .

DORR-6|PKUN     VDRLAARLPADAEHVFWLAPAGPTGAPAAAR-YDGTIAVFRLVKALLADGADARELGLTL
LACT-5|PKUN     AGELAALLPTGAEHVVWLAPRDASPAASSAEGPDGALAVFRLVKALLADGADARELSFTA
                ...*** **:.****.****  ..: *.::*.  **::*******************.:*

DORR-6|PKUN     VTRQARLLPGDTGADPAHAGVHGLAGTLAKEYPHWRIRVADVEADAAVPWPALLALPTDP
LACT-5|PKUN     VTRQARLLPGDADCDPAHAGVHGLLGTLAKEYPHWRVRGADIERDVSVPWPELLSLPADP
                ***********:..********** ***********:* **:* *.:**** **:**:**

                                                                   |-> KR5
DORR-6|PKUN     RGETLAHRHGEWYRLRLLET-----------------------DGTGVAAAP||REPGGV
LACT-5|PKUN     RGEVSARRHGEWYRQRLLEVALDASGAASFSAGSQAPNPQAPNSQASGARSAL||REPGGV
                ***. *:******* ****.                       :.:*. :*  ******

DORR-6|PKUN     IVAIGGAGGIGTVWTEHMMRRHGARVVWIGRRPLDAAIAAQQEALAAHGPKPDYVQADAT
LACT-5|PKUN     VVAIGGAGGIGTVWTEHMMRRHGARVVWIGRRPYDEEIAARQDRLAACGPRPEYVRADAT
                :****************************** *  ***:*: *** **:*:**:****

DORR-6|PKUN     DRDALRRACDEIVRRHGPVRGVLHTAIVLGDQTLARMDEDRFRTTYAAKADIAVNLADAF
LACT-5|PKUN     DARALRRAVAEIERRHGPVRGVLHTAIVLGDQSLARMDEAAFRTTYEAKAAVSVNMADAF
                *  *****. ** ******************:******  ***** ***  ::**:****
```

# Figure 10D

```
DORR-6|PKUN     AGQPLEFVAFFSSMQAFFKAPGQANYAAGCTFADAYAEHLSTRLDCPVKVMNWGYWAGVG
LACT-5|PKUN     AGQPLEFVAFFSSMQAFFKAPGQANYAAGCTFSDAWAERLSTALDCPVKVMSWGYWAGVG
                ********************************:**:**:*** ********.********

                                                KR5
DORR-6|PKUN     VVTADGYRQRMAQLGLGSIEPDEGMAAFDTLLASPY|PQLALLKATDTRSIDGLHDDDAL
LACT-5|PKUN     IVTADGYRQRMAQLGLGSIEPDEGMAAFDALLASPY|RQLALLKATDSRSIDGIYGDDEL
                :****************************:******  *********:*****::.** *

DORR-6|PKUN     THPVVTTPSLIGALGEDCPDRRAEIAQLREKAGGHAGAMQDALVRITWALLQSLGLFRDG
LACT-5|PKUN     RQLPPAAPALADTLRTDRPDRNAEIRRLREQADGHAGVMYDALVRVTWALLTSLGLFRDG
                 :   ::*:* .:*  * ***.*** :***:*.****.* *****:***** ********

DORR-6|PKUN     RAATAAEWRALGGIEDRYERWTEHTLAVLADAGLLRREGEDTYVALDTRTGSLDDAWADW
LACT-5|PKUN     RAATAAEWRAVGGIEERYQRWTEHTLEVLTAAGRLRRAGEDRYAAVAPGAVPAEDAWAEW
                **********:****:**:******* **: ** *** *** *.*: . : . :****:*

                                                MT5
DORR-6|PKUN     DRARQQWLADDAKRPQAVLVDTTLRAMTG|ILTGRRPATDVMFPNAWLELVEAVYKNNPV
LACT-5|PKUN     DRAREVWLADEAKQAQAVLVDTTLRELTA|ILTGRRAATDVMFPGSSLRLVEAVYKNNPV
                ****: ****:**:.********** :*. ******.*******.: *.***********

DORR-6|PKUN     ADYFNDVLADTLVGYLERRLADDPSARLRILEIGAGTGGTSATVLRRLRPWARHIEKYTY
LACT-5|PKUN     ADYFNEVLADTLVAYLEHRLRQDPSARLRILEIGAGTGGTSSVVFRRLRPLAGHIETYTY
                *****:*******.***:** :*******************:.*:***** * ***.***

DORR-6|PKUN     TDISKAFLLYGQREYGEIAPYLDARLFNAEKPLAGQEVDPGAYDVVIATNVLHATRNIRR
LACT-5|PKUN     TDISKAFLLHARRAYGEIAPYLDGQLFDAEKPLAGQPVAVGGHDVVIATNVLHATGNIRN
                *********:.:* *********.:**:******** *  *.:************ ***.

DORR-6|PKUN     TLRNAKAAARPNALLLLNELSDNILFSHLTFGLLDGWWLYDDPAPRIPGSPGLAPESWRR
LACT-5|PKUN     TLRNAKAAVRANGLLLLNELSDNILFSHLTFGLLDGWWLYDDPAPRIPGSPGLTPQSWRR
                ********.*.*.****************************************:*:****

                  MT5
DORR-6|PKUN     VLGEVGFR|AAFVAAGGADDLGQQVIVAESDGAIRQPRPDGESAFRGTLPEAGPR-----
LACT-5|PKUN     VLDEVGFR|GSFVAAEGADDLGQQVIVAESDGAVRQPRPGGVSAFRGSLPEARPAQPTGG
                **.***** .:**** ****************:*****.* *****:**** *

                                                                  ACP5
DORR-6|PKUN     ----------------------AAEPQLPAPTPDPVAADGVRDD----ELLADL|ARDHF
LACT-5|PKUN     AGHLAVPAEHGSAPAVTVPVTAASASSAPGSAPAAVPSGDPSGDGSMAARVAGP|ARDLF
                                      *:  ..  *..:*  .*.:..   .*      :*.  *** *

                                                                  ACP5
DORR-6|PKUN     RTLVADTLQLPVADIRADVPFDRYGIDSILVVQLTEAVRKGLCNVGSTLFFEVRTVDGLV
LACT-5|PKUN     RGLVADVLQLPVGDIRADVPFERYGIDSILVVQLTDAVRKVLDGVGSTLFFEVSTVDGLV
                * ****.*****.********:*************:**** * .********* ******

DORR-6|PKUN     QHFL|RTQPDALAALVGLSGAR-----------AARTDEQLAPAAGPEPVPVIAAEPPRA
LACT-5|PKUN     EHFL|RTRPDELAALVGVSAAEHPEPAAEAAAPEAVTEEPAASVPAPAPVAAPVSVPVPA
                :*** **:** ******:*.*.           * *:*  *....* **.. .: *  *

                       KS6
DORR-6|PKUN     EQG--M|AIAIVGMAGRYPGAPDLDTFWENLLAGRDSITEIPAGRWDHSRYYDARRGVPG
LACT-5|PKUN     APGEDV|PVAVVGMAGRYPGAADLDAFWENLLAGRDCVTEIPDGRWDHGRYYDERRGVPG
                  *  :  .:*:**********.***:**********.:**** *****.**** ******

DORR-6|PKUN     RTYSKWGGFLDGIDEFDPLFFGISPKAASTMDPQERLFLQCAHTTLEDAGYSRGALRAAA
LACT-5|PKUN     RTYSKWGGFLDGVDEFDSLFFGISPKAASTMDPQERLFLQCAWTALEDAGHTRASLRSAS
                ************:****.************************ *:*****::*.:**:*:
```

# Figure 10E

```
DORR-6|PKUN   RARVAEDAGDIGVFAGAMYSEYQLYGAEYSVRGEPVVVPGSLASIANRVSYFLDASGPSV
LACT-5|PKUN   RARLPEDAGDIGVFVGAMYSEYQLYGAEQGVRGEPVVVPGSLASIANRLSYFLDASGPSV
              ***:.*********.*************  .*****************:***********

DORR-6|PKUN   TVDTMCASALSAIHLACAALQRGECGVALAGGVNLSVHPGKYLMIGEGQFASSDGRCRSF
LACT-5|PKUN   AVDTMCASALSAVHLACAAIRRGECASAVAGGVNLSLHPSKYLMIGEGQFASSDGRCRSF
              :***********:******::****. *:*******:**.********************

DORR-6|PKUN   GEGGDGYVPGEGVGAVLLRPLADAVADGDRILGVIRGTAVNHGGHTHGFTVPNPLAQAAV
LACT-5|PKUN   GADGDGYVPGEGVGAVLLRPLADAVADGDRVLGVIRGSAVNHGGHTHGFTVPNPLAQASV
              * .****************************:******:*******************:*

DORR-6|PKUN   IRSAWRRAGVDPRDIGCIEAHGTGTSLGDPIEIAGLNAAFAEFTDARNFCAIGSAKSNIG
LACT-5|PKUN   IRGAWRRSGVDPRDIGCIEAHGTGTALGDPVEIAGLNAAFGEFTSERTFCSLGSAKSNIG
              **.****:****************:*****:*********.***.  *.**::********

DORR-6|PKUN   HLESAAGIAGLAKLLLQMRHGTLVPSLHAERVNPDIDFADSPFVLQREAAPWPRTGTRPR
LACT-5|PKUN   HLESAAGVAGLAKMLLQMRHGTLVPSLHAERTNPEIDFAATPFVLQREAAPWPRREGRPR
              *******:*****:*****************.**:****  :*************    ***

                                   KS6                          ID6
DORR-6|PKUN   LGGLSSFGAGGSNAHVVVEDYV||EEHAGKDLAPEAHRGET||VVVVLSAFDEERLRESAGR
LACT-5|PKUN   LGGISAFGAGGSNAHLLVEEYV||PTAAPPRRAAPGP----||VLAVLSARDGERLREYAGK
              ***:*:*********::**:**    *    *. .        *:.**** * ***** **:

DORR-6|PKUN   LRDALRKERWSSADLPDIAYTLQVGREAMTARFAVAVSTLPALVDALDACALGSGLPAGA
LACT-5|PKUN   LRDALRSGQWTDEDLPDIAYTLQVGREAMSARFAAEVSTLAGLMDALDACARGAALPPGA
              ******.  :*:. ****************:****. ****..*:******* *:.**.**

DORR-6|PKUN   YFNPGGDRGGAVKDFLTDEDFQETAVRWARRGKPAPLAEAWTSGLAVDWARLHTEGP-KP
LACT-5|PKUN   RLRTDGGRGGPVQDLADDEDFRETVVRWLRRGKLAPLAEAWTGGLDVDWARGHGTGEDRP
               :...*.***.*:*:  ****:**.*** **** ********.** ***** *  *  :*

                               ID6
DORR-6|PKUN   RKVALPGYPFARERYWYTDGLPE||LQEIPATFGNAARQPAAPPPAVEAAPATTSAVPAPP
LACT-5|PKUN   RKVGLPGYPFARERYWWNDGLAE||AGGEGADG--LGDEGAAGGTAGSGNGSGPRSARTDG
              ***.************:.***.*     *     . : **  .* ..  : . :. :

DORR-6|PKUN   ARPANSYELPAGDLTLHPVWEPVRLLRG---SPYPSAASRVVAIGLAPDALAELTARRPQ
LACT-5|PKUN   TRPG---ELPPGDLTLHPVWEPVHAAGGGADAPFFPQPADRVVAVGLAPEARAALEAYGTR
              :**.    ***.************:   *   :*:*..*.****:****:* * * *  .:

DORR-6|PKUN   TVVLDTAAS---SAEEVRDELAVLGDFDHVVMRFPTAAAAHGAEAQISTQRAAIRSMFRV
LACT-5|PKUN   VVTLPAPRDGGRSVADVRRELETAGPFDHVVVECPTP-AAQGARQRVEAQRASVRGLFRL
              .*.* :. .    *. :** **  . * *****:. **. **:**. ::.:***::*.:**:

DORR-6|PKUN   LKAL-ALTRDEQRLGLTLLTSGAFDAGGSGTADPAQASLHGLLGGLAKEQPHWRIRAVDL
LACT-5|PKUN   LQALSALRADEPRTGLTLVTRDAFDPDRTGGADPAQAALHGLVGGLAKEQPYWRVRAVDL
              *:** **  ** * ****:* .***.. :* ******:****:********:**:*****

DORR-6|PKUN   ADGEPFVADEVFALPADRRAHPLVRRGGQWLRRQLLPVDATEP-----------------
LACT-5|PKUN   AEGEPFVPEEICALPADRRAHPLVRRGGQWLSRRLLPVGDVRPGTPDDGPRTAVDGTDAA
              *:*****.:*: ******************* *:****. ..*

                                   KR6
DORR-6|PKUN   ---------------PAEPV||LRRDGVYVLIGGAGDLGVLLSEYLVRQHDAHVVWVGRRA
LACT-5|PKUN   PQAVSVPSGSVSSVSVPSGG||FRGDGVYVLIGGAGDLGTVLTEHLLRRYDARVVWVGRRA
                            ..   :* **************.:*:*:*:*::**:*******

DORR-6|PKUN   EDEDIRARADRAAAG--GRTPVYLSADASDPDALARMRDEVVRRYGRIDGVVHLAMVFSH
LACT-5|PKUN   EDDAVRAAAARVAAATGGEAPVYLSADARDPGALARVRDEVLRRYGRIDGLVHLAMVFSH
              **: :** * *.**.   *.:******** **.****:****:*********:*********
```

# Figure 10F

```
DORR-6|PKUN     TPLARMTERELEATLAAKVDPCAHFADVFAGHGLDFVLLISSLVSFIRNSHQAHYSAACA
LACT-5|PKUN     TLLAELPEEDLNATLAAKADPTEHFADVFAGQRLDFVLLVSSLVSFIRNSHQAHYAAACA
                * **.:.*.:*:******.**  ********: ******:***************:****

                                                                         KR6
DORR-6|PKUN     FEDAHAAALREALDCRVKVVNWGYWGNVPDELLRDVTSMGLAPIAPATAMGALERLLAGP
LACT-5|PKUN     YEDARAPGLGRALGCPVKVVNWGYWGNVSDEVLRGVTEMGLAPIEPASAMAAVEELLTGP
                :***:*..* .**.* ************.**:**.**.****** **:**.*:*.**:**

DORR-6|PKUN     L||HQIGFMRLGRPLPVEGVLTAETLTPQTHGAAARDG--AAALALPTGLAAYHESPVPGE
LACT-5|PKUN     L||DQIGFMRLGRPLPVEGVLAGETLSGHPYAAVSRTAAEPAPVPVPAALAEHHAGPVPGE
                *  .*****************:.***: :.:.*.:* .   .*.:.:*:.** :* .*****

DORR-6|PKUN     IDAFLLRRLAAELRRAGLEEPRHGLAEWKERQG--------VDARFDGWLSATLHALAEH
LACT-5|PKUN     IDALLCRCVAATLRRAGLRRPADGFASGSGSGSGAGSAGVRVDERFDGWFAATVRTLREY
                ***:* * :** ******..* .*:*. .    .          ** *****::**:::* *:

                                                                      MT6
DORR-6|PKUN     AMIDDRGRWTTSSPAATDADACRADWAAQTPRWAAANPDLRAPLNLLDRTLPALP||DVLC
LACT-5|PKUN     GLVDSRGDWSERAPGAGDAAACLAEWERAAERWASAHADLRAPTGLLGRTLPALA||DILR
                .::*.** *:  :*.* ** ** *:*  .: ***:*:.***** .**.******.  *:*

DORR-6|PKUN     GRVRATDVLFPQGKFSLVEGVYRDNRVAAHFNAVLAEHVAAFLRARRDADPGARLRVLEI
LACT-5|PKUN     GRIPATDVLFPEGSFSLVEGVYRDNAVAAHFNAVLAAQVTAFLHGRRAADPAARLRVLEI
                **: *******:*.*********** ********** :*:***:.** ***.********

DORR-6|PKUN     GAGTGGTTGPVLDRLAHEGLDLAEYCFTDLSQAFLQNAQDTFGPGRDHLTYRIFDAARPP
LACT-5|PKUN     GAGTGGTTAPVLEQLECAGLELAEYCFTDLSLAFLQRAEDAFGPGRAHFACRTLDVSRAP
                ********.***::*   **:********** ****.*:*:***** *:: * :*.:*.*

DORR-6|PKUN     HTQGLDTGAFDVVIAANVLHATDTIRPALRHAKALLRGNGLLALNEISGFYLVNHLTFGL
LACT-5|PKUN     RTQGFDAGAYDVVIAANVLHATDDVRTALRHAKSLLRGGGMLALNEISGFYLVNHLTFGL
                :***:*:**:************* :*.******:****.*:*******************

                                                       MT6
DORR-6|PKUN     LDGWWLYDDAELRVPGSPALSPAAWQLVLEQEGFTG||IRHPARDALALGQQVVVAHSDGL
LACT-5|PKUN     LDGWWLYGDAELRAPGSPALPPESWRRVLTQEGFTG||VADPARDARALGQQVVIAHSDGL
                *******.*****.******.* :*: ** ******  : .***** *******:******

DORR-6|PKUN     ARSPRLLSG-----TPEMSSPPSQPPAETAAP-----AAASAS----------ARAVT||D
LACT-5|PKUN     ARGPVTDAAPAAPAAPAAVARPETNTAVSAAPNMAVSAASSAAGGPQTSGGPDVRVVA||D
                **.*   :.     :*   : *.  .* :***      **:**:          .*.*: *

                ACP6
DORR-6|PKUN     VVLAALADALRMPADRIGPDRAFADYGLDSIVGVRFVQRLNEELGTDLPTTVVFDYRSVA
LACT-5|PKUN     VVETELADALRLPAERIDRAGAFADVGLDSIVGARFVRRLNEELGLDLPTTVIFDYRSVD
                ** : ******:**:**.    **** *******.***:******* ******:******

                     ACP6
DORR-6|PKUN     QLAAHIAESHRP||QP---------------------------APAAAAPVPAPDAAGAP
LACT-5|PKUN     ELAAHIVEDHRP||TSPAPGGTGAATAQEPPAERESGRAPEREHGPVVAPDVTVPDATEPG
                :*****.*.***  .                          .*..*. *..***: .

                       KS7
DORR-6|PKUN     NRPEGRE||PIAIVGISGRFAQSDDTDALWQHLAAGRDLVGPVERWDLSGYSQDQLSCRAG
LACT-5|PKUN     SAPYGRE||PIAVVGVSGRFAGSDDLDALWRHLAAGDDLVGPIDRWDLSAYGEDELTCRSG
                . * ***  ***:**:***** *** ****:***** *****::*****.*.:*:*:**:*

DORR-6|PKUN     SFLDGIDRFDARFFHLTGLEATYTDPQQRLFLEQAWTAIEDAGYAGSALDGRRCGVYAGC
LACT-5|PKUN     SFLDGIDRFDARFFKLSGREAAYTDPQQRLFLEQAWTALEDAGHGGASTDGMRCGVYVGC
                **************:*:* **:****************:****:.*:: ** *****.**
```

# Figure 10G

```
DORR-6|PKUN    TGGDYPQWFEDAPPAQAAWGNAPSVVPARIAYHLNLQGPALAVDTACSSSLVAVHLACQG
LACT-5|PKUN    TGGDYKDHFEDAPPAQAVWGNAPSIVPARIAYHLNLQGPAIAVDTACSSSLVAVHLACQG
               ***** : *********.******:***************:*******************

DORR-6|PKUN    LWSGETDMALAGGVSVQTTPDTYLAAGRGGMLSPTGKCHTFDAAADGFVPGEGVGVVVLR
LACT-5|PKUN    LWSGETEMAVAGGVSVQTTPATYLSASRAGMLSPTGRCHTFDAAADGFVPGEGVGVVVLR
               ******:**:********** ***:*.*.*******:***********************

DORR-6|PKUN    RLSDALADGDHIHAVIRGSAVNQDGATNGITAPSALSQERLIRQVHTEFGIDPAEIGMVE
LACT-5|PKUN    RLSDALRDGDHVHAVIRGSGVNQDGATNGITAPSALSQERLLRQVYEDFAIDPSEIGMVE
               ****** ****:*******.*********************:***: :*.***:******

DORR-6|PKUN    AHGTGTQLGDPIECQALVGAFGTAGGSDTCALGSIKTNLGHTTSAAGVAGLLKVVLSLRH
LACT-5|PKUN    AHGTGTQLGDPIECHALRRVFEGSDVPGGCALGSIKTNLGHTTSAAGVAGLLKIVLSLRH
               **************:**  .*  :. .. ************************:******

                                                                    KS7
DORR-6|PKUN    GQIPPSLHHYETNPAIRLTESPFHVNTTLRPWQPNGQGKRVAALSAFGFSGTNGHMVVEN
LACT-5|PKUN    RQIPPSLHYRDRNPEIRLEGGPLYVNTSLRPWEPNAGGSRAAALSSFGFSGTNSHLVVEE
                *******: : ** ***  .*::***:****:**. *.*.****:*******.*:***:

                                      ID7
DORR-6|PKUN    AP‖DRDER---------QQAADEL‖LFVLSAQQPEALRHRAEDLLAYLRRAPDAALGDVS
LACT-5|PKUN    AP‖ARPGRSPLSGAAAVEEPGLPR‖VFPLSAPQPAALRERVRDLAVHLRSTPDAVLVDVS
               ** *  *         ::..    :* *** ** ***.*..** .:** :***.* ***

DORR-6|PKUN    YTLAAGRDHFTHRAAFVAADRDTLAHRLEAWLADGRSDT--------------VGRRGDT
LACT-5|PKUN    HTLATGRAHFAHRAAFVARTREELIGQLDDWLDGEAGDAGKAAKTGEAAKTGDVGEAGGA
               :***:** **:*******  *: *  :*: ** .  .*:              **. *.:

                                                     ID7
DORR-6|PKUN    AP-ERARARYLNGEEVDFAPLFSGLDVRRTPLPTYPFQRKSYW‖PTATAPSRRHQAPQAA
LACT-5|PKUN    GPEELARDRYLAGEPADFAALFAGSGARRTPLPTYPFQRRSHW‖VRGGAPG---SAPDAA
               .* * ** *** ** .***.**:* ..************:*:*  . **.   .**:**

                                                ACP7
DORR-6|PKUN    NGPAAAPSPEPARPAPAQPAPDTDEATVRY‖LAGELLLAELSRVLMMEPEEIDPQASFSD
LACT-5|PKUN    GSGTSTTS-------------------------------------------------------
               .. :::.*

                                                        ACP7
DORR-6|PKUN    YGVDSILTVRLVAAVNNALAVDLPSTALFEHSSLDRLTDHLVTR‖YGAQLRSSGALRGPA
LACT-5|PKUN    ---------------------------------------------------GTPALRTDA
                                                                .: ***  *

                                                                  KS8
DORR-6|PKUN    AEAGGAPAQDDHGPAAEAPSAAPAAPVASAGTAAVPAHAPAAAAGDPADDG‖VAVVGIAA
LACT-5|PKUN    REKG-------------------------------------RGAARAEDDA‖VAVVGLSA
                * *                                      .*. . **. *****::*

DORR-6|PKUN    RFAQSPDAAALWAHLAAGDDLVGEVTRWDMDEELGAGAPRQYGSFVDDIERFDAWFFRMS
LACT-5|PKUN    RFAQSPDAEALWAHLAAGDDLVGEVTRWDLSQIS--GGRTEHGSFVEDIARFDALYFGVS
               ******** ********************:.:    *.  ::****:** **** :* :*

DORR-6|PKUN    GKEATYTDPQQRIFLEECWHALEDAGYAGERLDGRGCGVYVGGSPSDYQQLIGDDAPPQT
LACT-5|PKUN    GNEATYADPQQRIYLEECWHALEDAGYAGERLDGRGCGVYVGAYPGDYHELIGADRPPQT
               *:****:******:****************************. *.**::*** * ****

DORR-6|PKUN    LWGNISSVIASRISYFLDLQGAALAVDTACSSSLVAIHQACQDLRLGNTSMALAGGVFVQ
LACT-5|PKUN    MWGNMASVIASRISYFLDLDGPAMSVDSACSSSLVAIHTACQDLRLGTTSMALAGGVFIQ
               :***::*************:*.*::**:********** ********.**********:*

DORR-6|PKUN    STPIFYRSAVRANMLSARGRCHTFDERADGFVPGEGAGVVVLKRLADALRDGDQVYGVIR
LACT-5|PKUN    ATPRLYQYSGKARMLSATGRCHAFDAAADGFVPGEGAGVVVLKRLSDALRDGDRVYGVIR
               :** :*: : :*.**** ****:**  ******************:*******:******
```

# Figure 10H

```
DORR-6|PKUN    GSGMNQDGTTNGLTAPSAGSQERLLRSVHERAGVDPAGIQLIEAHGTGTPLGDPIEFEAL
LACT-5|PKUN    SSGVNQDGTTNGITAPSGAAQENLVRDVYERAGVAPSGIQLIEAHGTGTPLGDPIEFEAL
               .**:********:****..:**.*:*.*:***** *:***********************

DORR-6|PKUN    RAAFGDAPEAGCALGSVKTSLGHTQFAAGVAGVIKVLLALRNEQLPPSLHFRRANPAITL
LACT-5|PKUN    RAVFADAPTGGCALGTIKSNVGHTQFTAGVAGVLKVLLALDHEQLPPSLHFTRPNPAIDL
               **.*.*** .*****::*:.:*****:******:****** :********* *.**** *

                                                      KS8
DORR-6|PKUN    EGSPFFYVNTELRPWPAPADGPRRAGVSSFGAAGTNAHALIEQAP||---AVRTAGHGPRHA
LACT-5|PKUN    ANSPFHVNTELLPWRAPADGPRRAGVSSFGAAGTNAHVLIEQAP||SDAAARARRHG-RAQ||
               .***:***** ** **********************.******    *.*:  ** *

                 ID8
DORR-6|PKUN    ||WLIVLSAQDDAGRRAQAERLLDHALAHEDLDLGDVAYTLATGRRHCSHRWAGVATDREQ
LACT-5|PKUN    ||WLLVLSGQDGTALRAQAERMLDHVERHPDLDLGDTAWTLATGRRHSAHRLACVAADREQ
                 **:***.**.:. ******:***.  * ******.*:********.:** * **:****

DORR-6|PKUN    LVAALRTWLCDGRAEGVVTGEAPDGHRR-------QDPAEDARAGRLMAEPDRHDSLTEL
LACT-5|PKUN    WTAALRGWLRDGRAEGVWTGEADESPRSGHSGESGEGSGEPARAEALMAEHDRPGNLAAL
                .**** ** ******* ****:. *        :...* ***  **** ** ..*: *

                                                          ID8
DORR-6|PKUN    AGLFAQGQDLGFAPLFGDGGFRIVSLPAYPFFAGERYWVGSRP||-AAPAATPASAPVRAPV
LACT-5|PKUN    AELYVRGEVARFAPLYADGDFRIVSLPGYPFGGERYWTGPLP||GDTPDGTDGTDGTYGTD
               * *:.:*:    ****:.**.*******.***.*****.*. *   :* .* .:  . ..

                            DH8
DORR-6|PKUN    PVAAPSPLEGR||RLTGDPGSPSFAVELAGREFFLDDHRVRNVPVLPGVAYLELAYAAARA
LACT-5|PKUN    GISESG--GES||RPSAEPRPYAGALALTGEEFFLDDHRVGGVPVLPGVAYLELAHAAATA
               :: ..         * :.:* . : *: *:*.*********  .*************:*** *

DORR-6|PKUN    EG-VDPAHARLRNVVWSRPARITGPTAVEIALRP-CEDDAFTYEITTAADGEQPVIHGQG
LACT-5|PKUN    QGGLAPGGVLLRNVVWSRPARVTEPLSVETVLEPRAADGTFGYEIATVRDGARRLVHGRG
               :* : *. .  ***********:* * :** .*.* . *.:* ***:*. ** : ::**:*

DORR-6|PKUN    RIERCGTPSPARLDIAALRAQCEVRTLEHDDCYRLFDRMGIGYGPAMRGIRRIHVGAGLA
LACT-5|PKUN    RIEPRPGGAPARLGLAALRERCDVRSLDHAECYALLGATGMSYGAAMRGLEELHVGRGLA
               ***       :****.:**** :*:**:*:* :** *:.  *:.**.****:..:*** ***

DORR-6|PKUN    VARLSLPQAARDGAGWDLHPSMLDAAVQATLGLSLAEDTDTVAPALPFVLEEVQLLAPSP
LACT-5|PKUN    LGRLRVPREARDGRPWTLHPALLDAAVQATVGLALDGESDGLTAALPFAVEQVQVLAASP
               :.** :*: ****  * ***::****:***:**:*  :* ::.****.:*:**:**.**

                                                          DH8
DORR-6|PKUN    AGGWAVVRPAAGDGGGAVRRIDIELCDDDGEVCVRLLGFTARVL||A-------AGDDPAG
LACT-5|PKUN    ESGWAVARPADGAAEGPVRRMDVEICDDEGTVCVRLLGFSTREL||PGATASVTTGATTGA
               .****.*** * . *.***:*:*:***:* ********::* * .        :*  ...

DORR-6|PKUN    GENTGGATLTLMRAGWRPAEPTR-----------------------ASRPLVHHEVLLGG
LACT-5|PKUN    GSGAGSAAASPAPAAADPGAPADGSLVFARPVWRAVPSADVREERPAPSPAPYREILLAG
               *..:*.*: :   *.  *. *:                        *. *  ::*:**.*

DORR-6|PKUN    LAGTDPAAVRDGLGVPCTALPDDGDPARCFTRQAETVLARLQQFVPRTRDGEVLLQVVVP
LACT-5|PKUN    PESVDAAEVRKRSGVPCSALPGGADLPERYTRQAQALLAKVQQLLPRVREERVLLQVAVP
                ..*.* **.  ****:***...* .. :****::**::**::**:**.*: .*****.**

DORR-6|PKUN    ADGENRVLAGLGGLLRTARMEHPKLLTQLVEVETPVDAATLCERLRRDAASPDDVAVRYS
LACT-5|PKUN    AHGEGRLFAGLAGLLRTACAEHPGLAAQLVETDA-ADAATLCAHLDAEAAQPGVATVRRT
               *.**.*::***.******  *** * :****.:: .****** :*  :**.*. .:** :

                                    KR8
DORR-6|PKUN    GGQRRVPQWTAVEDAPPAR||PWKAGGVYLLTGGVGGLGAHFARREIARQAPGAALVLCGRS
LACT-5|PKUN    GGERLVRQWHGFRPERGDQ||PWKPGGVHLVTGGAGGLGALFARRIARTAPGSVLVLCGRS
               **:* * **  ...     : ***.***:*:***.*****  ***.*** ***:.*******
```

# Figure 10I

```
DORR-6|PKUN     PEGPAQRELLCELGDLGAS-AVYRVLDVARRDAVTACVNTVVAEHGRLDGVVHTAGVVRD
LACT-5|PKUN     PEGAAQRELLGELRESGAAHAEYHSLDVGRRADVVRLVRQVVDRHGRLDGVIHSAGVLRD
                ***.****** ** : **: * *: ***.** *. *. ** .*******:*:***:**

DORR-6|PKUN     GYLARKSAEELREVLAAKVAGFVHLDGATAALDLDCFIGFSSLSAYGNQGQGDYAAANAF
LACT-5|PKUN     GFVAHKTPEYLGEVFAPKAGGVVHLDEATAALELDFFLVFSSMSVLGNPGQADYAAANAF
                *::*:*:.* * **:*.*..*.**** *****:** *: ***:*. ** **.********

DORR-6|PKUN     MDAYAGLRHERVARGERRGRTLVVGWPLWADGGMTVDAATERRLHDSVGMVPIRAPHGVE
LACT-5|PKUN     LDAYVAHRAGLADRGERHGRSLSVGWPLWADGGMHVDAATERRIHQSSGMRPLRAREGFE
                :***.. * . ****:**:* *********** ********:*:* ** *:** .*.*

                     KR8
DORR-6|PKUN     ALLRAYGTGDPH||VLAVFGDRARIDATLLAAPAATGAAPAVTAP---------------
LACT-5|PKUN     ALERLYGSGLPH||ALTAFGDRERIASVLLDGSEGSDGSARPDGPDAERETDERRRTPADA
                ** * **:* ** .*:.**** ** :.** .. .:..:. .*

                           ACP8-1
DORR-6|PKUN     ----DRAAL||HARVLGRAISHACAVLGVPAAELDGAVELSEYGFDPVSLTGFAARLTTEF
LACT-5|PKUN     NDERNEAMS||HTALVGRLAAHLSELLDVPAEEIEGGVELSEYGFDSISLTEFVTLLNGAY
                :.* *: ::** :* . :*.*** *::*.*********.:*** *.: *. :

                       ACP8-1
DORR-6|PKUN     GLPPVPKPFSEHLTLGEVVDHLLD||THPHHFGTVP---------------------PAPA
LACT-5|PKUN     GLSLVPTVLFEHSTLDGVAGHLLE||EYADRFAPEPEPEPEPQPVQAQMPEPVPVPEPEPA
                **. **. : ** **. *..***: :..:*.. * * **

                                       ACP8-2
DORR-6|PKUN     PEPSAGPES-AAAPVATAGREQQ||HKALLKKLIARVSDLLDVPAERITGTAEMTRYGFDS
LACT-5|PKUN     PVPARGPVAPSTAPVAADDDDAL||RRALVKRLRELTSRILRVPAEKISATQEMSKYGVDS
                * *: ** : ::****: . : ::**:*:* .* :* ****:*:.* **::**.**

                                            ACP8-2
DORR-6|PKUN     LSFIGFANDLNAEFGLSLAPTLFFENPTLDGVVDHLLDHHADR||VA-----ATAAPQQEP
LACT-5|PKUN     LSLAELAAAVNAEFSLMLDPTLFFEHPTLEAVARYLLDRHADR||LTGLVTEETPEPAMTE
                **: :* :****.* * ******:***:.*. :***:**** :: *. *

DORR-6|PKUN     RAAAAPAAPEPATADTPASRTDAPGN------------------------------E||P
LACT-5|PKUN     QAVAEPVVAEPPVVESPATTSPAAETSVTETSVREPAAPAAAPAPAFAAAPGPGAAEE||P
                :*.* *...**...::**: : *. . * *

                KS9
DORR-6|PKUN     IAVIGISGRFPMADDLDAFWENLSEGRDCTREVPTDRWDWRAHYGDPVKEPNTSNVTSGG
LACT-5|PKUN     VAVIGISARFPMADDLAEFWENLREGRDCIREVPSDRWDWREYYGDPVKEPNKTNVTSGG
                :******.******** ***** ***** ****:****** :*********.:******

DORR-6|PKUN     FMDGVGDFDPLFFDISPKEAELMDPQQRLLLMYVWKALEDAGYSAEALAGTNTALIAGTT
LACT-5|PKUN     FMDGVGDFDPLFFDISPKEAELMDPQQRLLMLHTWKALEDAGYAPDSLAGTGTALFVGTT
                ******************************:::.*********:.::****.***:.***

DORR-6|PKUN     STGYSTLVTRYSPMIEGYDITGAAPSMGPNRMSYFLDLHGPSEPVDTACSSALVALHRAV
LACT-5|PKUN     NTGYGSMVSRYSPVIEGYDATGAAPCMGPNRMSHFLDLHGPSEPVDTACSSSLIAMHRAI
                .***.::*:****:***** *****.*******:****************:*:*:***:

DORR-6|PKUN     QAIRDGQSDLAIAGGVNTMVSVDGHISISKAGMLSPEGRCKTFSDRADGYARGEGVGMLV
LACT-5|PKUN     QAIHDGHSDMAIAGGVNTMVSIDGHISISRAGMLSVDGRCKTFSVGADGYGRGEGVGILV
                ***:**:**:***********:*******:***** :******* ****.******:**

DORR-6|PKUN     LKSLSAAERDGDHIYGVIRSTAENHGGRGSSLTAPNPKAQAALLREAYGKAGIDPRTVGY
LACT-5|PKUN     LKRLSAAVRDGDHVYGVVRGSAVNHGGRANSLTAPNPRAQADLVVGAWSRAGVDPRSVGY
                ** **** *****:***:*.:* *****..*******:*** *: *:.:**:***:***
```

# Figure 10J

```
DORR-6|PKUN    IEAHGTGTKLGDPVEINGLKAAFRDMYEEHGAV-VEEAHCGIGSVKTNIGHLELAAGAAG
LACT-5|PKUN    VEAHGTGTGLGDPVEVNGLKAAFAELYERWGVSGAGEAHCGLGSVKTNIGHLELASGVAG
               :******* ******:******* ::**. *.   . *****:*************:*.**

DORR-6|PKUN    VIKVLLQMRHRTLVKSLHCDTVNPYIDLDGSPFHLVRERQPWPALRDAEGRELPRRAGVS
LACT-5|PKUN    VIKVLLQMRHRTLVGSLHCGSVNPYVRLEGSPFRLVREREPWRAVRDENGRELPRRAGVS
               ************** ****.:****: *:****:*****:** *:** :***********

                         KS9                        ID9
DORR-6|PKUN    SFGFGGVNAHVVLEEYRP||RTAPEPDRAP---------T||APVPVVLSASHPDVLCELAE
LACT-5|PKUN    SFGFGGANAHIVLEEYQP||PAGTQTDAHTRTGPSTTVHS||GPVAVLLSAHRPDVLRESAT
               ******.***:*****:*  :..:.*   .          : .**.*:***  :**** * *

DORR-6|PKUN    RWVDALRRGDYDDTDMASIAYTTQTGRTPMTERLACLARTAGELREALESWLRGEPAADV
LACT-5|PKUN    RWVEVLRRGDYRDADLPALSYTSQTGRTAMAERLAVVAGTLEELRAGLESWLRGEPTPAV
               ***:.****** *:*:.:::**:*****.*:**** :* *  *** .*********:. *

DORR-6|PKUN    FRGKVARGVDLPDAPAGFGP-HDDHDSAGRHDWARLLQAWVNGAPFDWDRLHTGRRPRRI
LACT-5|PKUN    FTGRAPRDGDAPAAPAALTDGFASGGRTEARHWAPVLQAWTTGAECDWRTLWGERHPQRI
               * *:..*. * * ***.:     . . . :  :.** :****..**  **  *    *:*:**

                     ID9                       DH9
DORR-6|PKUN    ALPTYPFRLRRYWVD||TSRPANG-TQTEALHPLVHT||NTSDLNEHRYTSHFTGREFFLAD
LACT-5|PKUN    SMPTYPFQLRRYWLD||MTTPAHGPHVSRGLHPLVHR||NTSDLSEQRYTSHFTGREFYIAD
               ::*****:*****:*  : **:*    :..******  *****.*:***********::**

DORR-6|PKUN    HRVRAQVMETVSGWRPGRRPTAYDVRADAVPVLEAVAYLEMARAAAVQAAGGDERAWSLK
LACT-5|PKUN    HRVQGE-----------------------QVVPGAALLEMARAAAVLAAGGAETDWALR
               ***:.:                        *:*..* ********* **** *  *:*:

DORR-6|PKUN    LASWLRPLTVEKATDVHTTLTTRAGGGLSYEVYAVDEDGERVTFGRGQLRRATA------
LACT-5|PKUN    QVVWSRPLTAGRPVDVHTAVSVRADGEPAFEIYTEGPGGERVVHSTGRLHRRTAGNAAEL
                . * ****. :..****:::.**.*  ::*:*: . .****... *:*:* **

DORR-6|PKUN    -----VP--AERLDLAALRAQCDGPVLDAETCYARFTGIGMAYGPALRGIERLHTGSRQS
LACT-5|PKUN    LDGPELPGGAGHLDVAALRAQCDGTVLDAEECYARFSGVGLEYGPTLRAVETLSGGTRQA
                     :*  * :**:*********.***** *****:*:*: ***:**.:* *  *:**:

DORR-6|PKUN    VARLKLPAAASRERGWVLNPGMLDAALQATVGLFVDDPGTPRTALPFALGELEVLRAVPG
LACT-5|PKUN    VARLRLSAAASARTGFALHPSLLDAALQSTAGLFTG-SGTSSAALPFALDRLEVLRATPS
               ****:*.**** . *:.*:*.:******:*.***.. .**. :******..******.*.

                                                   DH9
DORR-6|PKUN    TGWVVVRFAEDDHVGAVRRLDLDLCDDDGEVCVRLRGFSVRTLGG||SEP----------
LACT-5|PKUN    SGWAVARFAADDRPGGVRRLDIDVCDDDGEVCVRIRGFQVRTYGG||DAAPSASGAANGTH
               :**.*.*** **: *.*****:*:**********:***.*** **  . .

DORR-6|PKUN    ------TGDSEPTRPAEQAPEPPSGSDDAYLLDLIEAIGRREMSADEFKRSLA
LACT-5|PKUN    AVTTDGTGNGTDTGNGNSTGTGSEADADARLLHLIHAIGEGALSADEFQRSLI
                     **:.  *  .:.:   .... ** **.**.***.  :*****:***
```

# Figure 11A

```
DORR-7|PKUN    -------------------VGQDEALRLIRDWKQEQEQEQEQDQNQEQARARTQTARPAD
LACT-6|PKUN    MTTHATSLTELHEQIRTGRIGQDEALRLIRAWQQGRQTGSEGGPAERQATGDDAAAR---
                                  :********** *:* ::  .* .  :.** .   :**.

                                  ACP9
DORR-7|PKUN    VADTEALTERVCAVVVEKVC||ELLKVTTDDLDVHVDLSEYGLDSLVITQLVNMVNDALGL
LACT-6|PKUN    ---GEALRERVCDIVTHAVS||ELLKVGPDDLDADVELSEYGLDSIVMSQLVNAVNDELGL
                  *** **** :*.. *. ***** .****..*:*********:*::**** *** ***

                         ACP9
DORR-7|PKUN    ELVPTVLFEHATIQAFGAHLTDEY||GPALAARLG-------LRSPGAATEPP-----AVE
LACT-6|PKUN    ELAPTVLFEHPNLRAFSAHLADTY||ADSLSVRLLGTPGTGPAPAPSTATSPAPSTATSAE
               **.*******..::**.***:* * . :*:.**        :*.:**.*.      :.*

DORR-7|PKUN    PVGTPVP----------------------------------------AAAVPARAVPVP
LACT-6|PKUN    PAAVAAPSTSPSEARTESRVPTPPATGGRFFPAAVPSAPSAETEPVTAPAPAPASEQASP
               *.....*                                        .*..**   . *

                        KS10
DORR-7|PKUN    LPADRHDD||PIAVVGMSGRFPQAEDLDAFWRNLRDGRDCIAEVPADRWDWRALFGDPLQE
LACT-6|PKUN    AQASAAEE||PVAVVGMSGRFPMADDLAEFWENLREGRDCIREVPSDRWDWREYYGDPVEE
                *.  :: *:********** *:**  **.***:***** ***:******   :***::*

DORR-7|PKUN    PGRTNVKWGGFMEGVADFDPLFFGIAPKDAVHMDPQQRLLMLYVWKALEDAGYAADALAG
LACT-6|PKUN    PGRTDVKWGGFIDGVADFDPLFFGIAPKEALHMDPQQRLLMLYVWKALEDAGHSADSLAG
               ****:******::**************:*:*********************::**:***

DORR-7|PKUN    SSFGLFVGTSDTGYGLLSDRSSGRGESVTPTGSVPSVGPNRMSYFLDVHGPSEPIETACS
LACT-6|PKUN    SDLAMFVGTNDTGYGTLAER-CGKRDSVSPTGGVPSLGPNRMSFFLDVHGPSEPVETACS
               *.:.:****.***** *::* .*: :**:***.***.******:**********:*****

DORR-7|PKUN    SSLVAMHRGVISIERGECDMAVVGGINTMVIPDGHVSFSKSGMLSAEGRCKTFSDRADGY
LACT-6|PKUN    SSLVAMHRGVTAIARAECETAVVGGINTIVVPDGHVSFSRAGMLSVDGRCKTFSVGADGY
               **********  :* *.**: ********:*:********::****.:*******   ****

DORR-7|PKUN    ARGEGVGMLVLKSLSAAERDGDHVYGIIRSTAENHGGRSNSLTAPNPKAQAALIRRAYST
LACT-6|PKUN    GRGEGVGILVLKRLSAAVRDGDHVYGVVRGSAVNHGGRANSLTAPNPRAQADLVVGAWSR
               .******:**** **** ********::*.:* *****:********:*** *:  *:*

DORR-7|PKUN    AGIDPRTVGYIEAHGTGTKLGDPVEINGLKAAFRELYEEHGAV-VDDAHCGIGTVKTNIG
LACT-6|PKUN    AGVDPRSVGYVEAHGTGTGLGDPVEVNGLKAAFAELYERWGVSGAGEAHCGLGSVKTNIG
               **:***:***:******* ******:******* ****. *.  ..:****:*:******

DORR-7|PKUN    HLELAAGVAGVIKVLLQMRHRTLAKSLHCDTVNPYIDLDGSPFHLVREQQPWPALRDAEG
LACT-6|PKUN    HLELASGVAGVIKVLLQMRHRTLVGSLHCGSVNPYVRLEGSPFRLVREREPWRAVRDENG
               *****:*****************. ****.:****: *:****:****::** *:** :*

                                  KS10                   ID10
DORR-7|PKUN    RELPRRAGVSSFGFGGVNAHVVLEEYVP||----------RPVPPVSTP-||DPVAVVLSAP
LACT-6|PKUN    RELPRRAGVSSFGFGGANAHIVLEEYQP||PAGTQTDAHTRTGPSTTVHS||GPVAVLLSAR
               ****************.***:***** *            *. *..:.   .****:***

DORR-7|PKUN    EPEMLRARARQLADRIDSGGLGEADLPDLAHTLQVGRVAMDERLAFLTSSLADLRERLGA
LACT-6|PKUN    EPETLRARARQLVDWLDKGEATEADLPRISYTLQVGRVAMPERLACVTESLAELRAQLQE
               *** ********.* :*.*    *****  :::********* **** :*.***:** :*

DORR-7|PKUN    FLDGGTVQGLHTGRAQRPGPWNELAGDDDIALALDSWIAKGKLGRLLKLWVTGFDVDWRR
LACT-6|PKUN    FLDGERPRGVRTGRAERRGIWNDLADDEDITAAVDNWMAKGKLDRLLKLWVAGAEFDWRR
               ****   :*::****:* * **:**.*:**: *:*.*:*****.*******.* :.****

                                  ID10                   DH10
DORR-7|PKUN    LYAGRPMRRIPLPVYPFQLKRYWITD||---AKSTTRPPAPVAAAP---||DAQPSPYRRDL
LACT-6|PKUN    LWGEHPPRRIPLPAYPFRLQRYWIAD||GTSGRSTRRPSTAREGTPYGG||TPRDAEYRELL
               *:. :* ******.***:*:****:*   .:** **.:.  .:*     .: : **. *
```

# Figure 11B

```
DORR-7|PKUN     TGHEFYVSDHRVGDTPVLPGTAYLEFVRDALVRATSAGTATGVRLRDVTWLRPLEVTALR
LACT-6|PKUN     RGDEYFLRDHRVGGVPTLPGAACLELVRAAWTHADRAPDTAPLRLRDVLWLRPLQVTAPR
                 *.*::: *****..*.***:* **:** * .:*  *   ::  :***** *****:*** *

DORR-7|PKUN     TLAVDVDPAGGTFEVYDHGSGDRVLHANGTAHVD-------PALLAAD------------
LACT-6|PKUN     TVAVALDPADGTYEVRAADGDEREVYARGTVTADGPHTVDGPHSAGGDRPGGTAEPAEPV
                *:** :***.**:**     ...:* ::*.**. .*        *    ..*

DORR-7|PKUN     DTHDIDALRANLPFRRDGAECYALFARRGMGYGPAFRAVQELYHGADTALARLLLPEAAA
LACT-6|PKUN     PAHDIAALRDRCPHRLDADGCYDRFADLGLAYGPALRAVETLHYGADLALARLVLPEAAA
                 :*** ***  . *.* *.   **   **   *:.****:***: *::*** *****:******

DORR-7|PKUN     SSLTLNPVMLDAALQATLGLALGEHVDAPQGT-----------ALPFTVREVQVLAPTPA
LACT-6|PKUN     GERTLNPSMLDAAFQTTLGVLLGEQAQAADAERAAAGGSEDVAALPFAVREVRILAPTPA
                ..  **** *****:*:***: ***:.:*.:.               ****:****::******

                                                          DH10
DORR-7|PKUN     EGWALVRRAADDRHDTGIRRLDIDLCDTQGNVCVRLLGFSTRMKPSPA||PRAAEPTTTPA
LACT-6|PKUN     EGWAVARAAEGDRPGGTVRTLDIDLCDTSGRVCVRLTGFSTRTVPEDG||-APQLPGEPPV
                ****:.* * .**  .   :* ********.*.***** *****  *. .    .  *  .*.

DORR-7|PKUN     LLIQADWRESAAREHHGDDVKRHVVLCELPAADATALGAALGGATCETWQARGETGTRYT
LACT-6|PKUN     LMIEPAWREAEAASVAAMPEDHRVVLCELPGVDAAELAGSLGG-DCETWQAEGDVAARYT
                *:*:. ***: *  .   .  .::*******..**: *..:***   ******.*:..:***

DORR-7|PKUN     EYAERLLKLLRDKAPEAARQPC--------------LIQVVTPAHAPWLGGLSGMLRTAR
LACT-6|PKUN     EYARRLLELLQEEARSPAPAPAPAPGGTSGGVPGGRLVQLVTPSSAPWLGGLSGMVRTAR
                ***.***:**:::*  ..*  *.                 *:*:***: **********:****

DORR-7|PKUN     MEHPKLLTQWIALDGDGALAPAELAGRLRCDGADTAEEAVRYRGGRRQVSQWHEVAPAAP
LACT-6|PKUN     QEHPKLLVQWIEAEDD--CSADELAVLLRGDGADPAEVAVRHGDGRRRVSRWRETPPPAP
                 ******.***   :.*     :. ***   ** ****.** ***: .***:**:*:*..*.**

                   KR10
DORR-7|PKUN     ER||PWRDGGVYLLTGGAGGLGALFAQDIARRVETPALVLCGRSPVGPAQQELLTALRALG
LACT-6|PKUN     RV||PWRDGGVYLVTGGSGGLAALFAKDMARRVRRPSLVLCGRGAAGPEQRELVAELEALG
                .   *********:***:***.****:*:****. *:******...** *:**:: *.***

DORR-7|PKUN     ARADYRVLDVADRADVTRVVREVQAEYGALHGIVHAAGVLRDGFVAKKTADDLREVFAAK
LACT-6|PKUN     ARAEYRVLDVSDAGAVSAAVREVVAAHGALHGVVHAAGVLRDGFLARKSAEELRQVFAGK
                ***:******:* . *: .**** * :*****:***********:*:*:*::**:***.*

DORR-7|PKUN     VAGLCHLDEATASVPLDCFIGFSSMAAFGNVGQADYAAANAFMDGYAAHRDSLVDQGSRS
LACT-6|PKUN     VAGLRHLDEATADVELDFLIAFSSMAAFGNAGQADYAAANAFLDGYAQHREALRARGERH
                **** *******.* ** :*.*********.***********:**** **::*   :*.*

                                                                    KR10
DORR-7|PKUN     GRTLMVNWPLWEKGGMGADPSTVQLLESVGMRPMRASVGIDALDRVWATGLPS||AIALDG
LACT-6|PKUN     GRTLSVNWPLWEKGGMRGGAGTEAVLQGVGMRPMRAETGLDALYRAWACGLTS||VLVLEG
                **** ***********  ....*  :*:.********..*:*** *.** **.* .:.*:*

DORR-7|PKUN     DHARMRERFLPAHPEPEAPAEPAPAAATLPATAPVAEPAEP-----------------S
LACT-6|PKUN     DHERMRSRLLPEQPPLPEPPHRPDAQKPLDAQKPLDAPELPDGPEATRTGGGVPVRSGSA
                ** ***.*:** :*     *.. .*  .* *  *:  *  *                  :.

                   ACP10
DORR-7|PKUN     S||VGTVVADLMATLLEVDVETLRWDKSLGDYGFDSIFMMQFLAQAQTHLDASLTLDVIAD
LACT-6|PKUN     D||VARRVTAVLADLLEIDAESLRPDVPLREYGLDSIFLTQFLGTARKEFDPALTLDVIAG
                . *.  *: ::* ***:*.*:** * .* :**:****: ***. *:..:*.:*******.

                  ACP10
DORR-7|PKUN     CETLQDVVDAI||TGTASDTGTAAPKPASVAP------VEEAP---AAAAPAKAPVRRAAA
LACT-6|PKUN     CETLTDFIDAI||ERAVAPPAATPPAPASASAPSPSSGTEGEPSTRPAPEPDGVPVVRPVA
                **** *.:***    :.: ..::.* ***.:.       .*  *   .*. *  .** *..*
```

# Figure 11C

```
                                           Te
DORR-7|PKUN     ASPNDFPELVRMNGVTS||GRPVFWVHHGNGGVESYAPLAARCPRPFYGIQPKGWIDSTDI
LACT-6|PKUN     KAPEEFPELIPMNAVRE||GRPVFWVHHGNGGVESYAAVAECCGRPFYGIQPRGWTGSEDI
                 :*::****: **.* . ******************.:*  * ********:** .* **

DORR-7|PKUN     LTGQYAMAEHYASLILAVQPEGPYDIGGFSLGGLFAYETVRQLQLTGADVRTLVMLDTLD
LACT-6|PKUN     LTGQEAMAAYYVDIIRAVQPEGPYDVGGFSLGGLFAYEVVRQLQLQDATVDTLVMLDTLD
                **** *** :*..:* *********:************.****** .* * *********

DORR-7|PKUN     AESTNKANALIVGG-NFDADVVTKVSDFRAVNLILGNNRFDSHGGATPILRRDEVDTTLE
LACT-6|PKUN     AASTNLANSLMTGGRQDDADVVAKVSAFRAVNLMLGNDSLDAREGTSSVLHRDEVDTALD
                * *** **:*:.** : *****:*** ******:***: :*:: *::.:*:******:*:

DORR-7|PKUN     PKEFLGSLIDAALARGVSKTETQLRSRVRQLSRYFEATQGETYTVDPLPRRDGLRCYYLR
LACT-6|PKUN     PDAFLDSLVEAAVARGIHKTPAQLRTRVRQLARYFDAVHGERHVVHPLPRREEVRCYYLR
                *. **.**::**:***: ** :***:*****:***:*.:** :.*.*****: :******

DORR-7|PKUN     NRGGKFFGAFEEHMVLFPNPELPTVDGVAYWQEWADQIDDFFTIDVDTSMHAEVMTAPQS
LACT-6|PKUN     NAGGQFFGPFEEYMVLFPEPDLPAVDGTPYWREWADAVDDFFVIDVDTETHAQVMTEPAA
                * **:***.***:*****:*:**:***..**:**** :****.*****. **:*** * :

                        Te
DORR-7|PKUN     LDKLMRLCDRLY||AAEDAAAPATSAQGGR
LACT-6|PKUN     LKKVLRLCDRLY||------APEQHAQGGR
                *.*::*******        **   *****
```

# Figure 12

```
                   AT
DORR-8|AYOA       ‖MKAVVFPGQGAQRRGMGRELFDAYPELADEASEVLGYSLRTLCLDDPHQQLGRTEYTQP
LACT-7|AYOA       ‖MEAVVFPGQGAQRRGMGRELFDAFPSLTEQASDVLGYSVRELCVADPERRLRSTEYTQP
                    *:*********************:*.*:::**:*****:* **: **.::*  ******

                                       active site
                                         ------
DORR-8|AYOA       ALFVVGALAHRQWRESTGDEPAFLAGHSLGEYCALHAAGAFDFATGLRLVQRRGALMAQA
LACT-7|AYOA       ALFVVGTLAHLKWQEETGRSAAYFAGHSVGEYTALHAAGAFGFETGLRLVQRRGLLMSQA
                  ******:*** :*:*.** ..*::****:*** ********.* ********** **:**

DORR-8|AYOA       RGGGMAAVVGVDAARLRELLDEGGFCRLTVANDNAPQQKVVSGDTATVDALVAYLEARDV
LACT-7|AYOA       EGGGMAAVLGLGAGELTELLREGGFVSLALANDNTPDQQVVSGAAHEIDVLEAYLSERGV
                  .*******:*:.*..* *** ****  *::****:*:*:**** :  :*.* ***. *.*

DORR-8|AYOA       RCVRLNVSGAFHSPLMRQAQQDFARFADGFALGDPATPVIANATARPYVPGRTARTLVDQ
LACT-7|AYOA       RGVRLNVSGAFHSPLMLPAQEAFAAYVRDFTLGDPETPVISNVTARPHPPGGTAELLVRQ
                  * **************  **: ** :. .*:**** ****:*.****: ** **. ** *

                                                        AT
DORR-8|AYOA       IVQPVRWTESVHHLLDQGVTDFVELGGRVLVRLIDQIR‖SAPRPVAQHDAPA-ARPDTPA
LACT-7|AYOA       ISSPVRWTESVRHLLDLGVEEFTELGGSVVAKLVRQIR‖EAHRRDADASGPAPAAPAAPV
                  * .********:**** ** :*.**** *:.:*: *** .* *  *:  ..** * * :*.

                            Ox
DORR-8|AYOA       -------AALG‖SPLFRRRMGVRHAYAVGGMYRGIASAQMVVRLGRHRILGFLGTGGLPL
LACT-7|AYOA       RSEPAARSALG‖SAVFRERLGLRHSYAAGGMYRGIASPAMVVRLARAGMLGFLGTGGLTP
                         :*** *.:**.*:*:**:**.*********. *****.*  :*********.

DORR-8|AYOA       PEIEQGVKEVQHGLADGQPYGVNVLADHDDPAAERALVDLLMRHGVPVIEASAFMQMTPA
LACT-7|AYOA       EAVEERILQVRRELREGEPFGVNFLADHDDPAAERRVAEMLMRRGVTVVEAAAFIGMTPA
                    :*: : :*:: * :*:*:***.*********** :.::***:**.*:**:**: ****

DORR-8|AYOA       LVLYRARGLRRGADGRTVCDHRIVAKVSRPEVAEQFMAPAPGPVLDRLRREHALTDEQAE
LACT-7|AYOA       SSSTARAGMHRGPDGAPRCAHRIVAKVSRPEVGRRLHGTAPGKVVDGLLREGAITGEQAE
                         *::**.**  . * ************..:: ..*** *:* * ** *:*.****

DORR-8|AYOA       LARTVPMSHDITVEADSGGHTDGGVATVMMPAMLKLRQQAQDRYGYDEPICMGLAGGLGT
LACT-7|AYOA       LVRQVPMSHDITVEADSGGHTDGGIATVMLPAMLGLRRQAQRSHDYAEPLCMGLAGGLGT
                  *.* ********************:****:**** **:***  :.* **:**********

DORR-8|AYOA       PAAVAAAFMLGADYVLTGSINQCTVESGMSTEVKDMLQDVGIADTAYAPAGDMFEFGAKV
LACT-7|AYOA       PEAVAAAFMLGADYVLTGSVNQCTVEADTSDAVKDMLQTIDIQDTGYAPAGDMFEMGARV
                  * *****************:******:. *  ****** :.* **.*********:**:*

                                                                 Ox
DORR-8|AYOA       QVLRKGVFFPTRANRLFSLYSHYDGLDDIPQKTRSLLERTYFGKSIEE‖VWDEVRAYLRS
LACT-7|AYOA       QVLRKGVFFPTRANKLYALYQHHDGLDDLPAKTRALLERSYFHRTFDE‖IWTEVREHYRA
                  **************:*::**.*:*****:* ***:****:** ::::*  :* *** : *:

DORR-8|AYOA       QGRDADIDRADAEPKQKMALVFRWYFFHTTRLAMDGDGSGKVNYQVQTGPALGAFNQWVE
LACT-7|AYOA       KGQPEVTDKAERQPKVKMALVFRWYFAYSARLALAGQDGDKVNFQIHTGPALGAFNQWVK
                  :*:    *:*: :** ********** :::***: *:...***:*::************:

DORR-8|AYOA       GTELASWRHRHVDRIGLMLLDGAAEHIATACRHWRDTLGVPSA---------
LACT-7|AYOA       GTACESWRARHADAIGLMLMEGAAEHVAAACESWGGTSRFAPAEERALAART
                  **   *** **.* *****::*****:*:**. * .*  ...*
```

# Figure 13

```
DORR-11|OXRC        ------------------------------------MLVELARSDKDSTLSLAG------
LACT-8|OXRC         MRGHAVTTHLTTDIDEIVTDVFQSTEGKKNPYPLYRRLQELGQVHRSEQLGWVATGYEVC
                                                         * **.: .:.. *. ..

DORR-11|OXRC        --------------------------------------MDAPEHTRARRAVVGEFTFRRM
LACT-8|OXRC         SAALRDPRVIKGPEQIQPGRPDPAEHSAEALLRGTMHRLDPPDHTRLRRLVNGAFTPRSV
                                                          :*.*:*** ** * * ** * :

DORR-11|OXRC        EALRPRVQEIVDECVDAMLAGPNPADLVKTMS---FPVPSLVICELLGVSVADRDFFEDR
LACT-8|OXRC         AALEPDIQELIDDLITPAVKKAEAGEPVDMMSGFAFPLSVAVIGRMLGVPASDWHRFHDV
                     **.* :**::*: : . :  .:..: *. **   **:.  ** .:***..:* . *.*

DORR-11|OXRC        TSRMLSMTLPPLT------RQQAFFDLQTYLDELVTAKEKVPGDDLLSRQIAKGRKDSAY
LACT-8|OXRC         VLDLSSMVELGFTGDELPKADAAADELIAYFRKLGAERMRNPADDLTSTLANATEAGDRL
                    .  : **.   :*       : *  :* :*: :* : : : *.*** *      . ..

DORR-11|OXRC        DHDALVDLAFLLLTAGHDTTGNMISLGMLALMETPELRARITDDPGTTPQVVEELLRYFT
LACT-8|OXRC         TEQELVTMLILLFMAGFETTTHSMGNGMFALLENPEQTQWLRRNMDAMPAAVEELIRYDS
                     .: ** : :**: **.:** : :. **:**:*.**    :  : .: * .****:** :
                                                                              --
DORR-11|OXRC        ITDIITTRVAKEDVEIGGQTIRAGEGVFALGGSANHDPDVFENPGKLDVDRGARQHLAFG
LACT-8|OXRC         PVQFIAGYTKEPVELADGTAVPADEYLFLMIGAANRDPRVFSDPELLRLDRGEAAPMSFG
                     .::*:  . :     .* :: *.* :* : *:**:** **.:*  * :***    ::**

                    ---heme------
DORR-11|OXRC        HGPHQCLGQSLARMELEIVYDTLLRRIPGLRPAGPAEDLPLKNDAAIFGLHELPVIW---
LACT-8|OXRC         GGIHYCLGAGLARLEIRKIFTSLLTRFSAIELAEPEPER--RSGLALRGYARIPMWLTPA
                     * *.*** .***:*:. :: :** *:..:. * *  :   :.. *: *  .:*:
```

SEQUENCE LISTING

<110> ECOPIA BIOSCIENCES INC.
Farnet, Chris
Yang, Xianshu
Staffa, Alfredo
Zazopoulos, Emmanuel

<120> POLYKETIDE SYNTHASE ENZYMES

<130> 3012-3PCT

<160> 43

<170> PatentIn version 3.0

<210> 1
<211> 52101
<212> DNA
<213> Streptomyces platensis subsp. rosaceus

<400> 1

```
ggcgcatctg atcacctctc agctcggtcc tgctccccgg cacgtgcacc ggaaccgtcg      60
ccgccctcac ccggccgcca ccgcctccgc ggccccacgg cccaggaggt cgtggaggag     120
gcgggtggcg gggagttccg gatgaaggta gacgggttcg tcgctctggc cgacggctgc     180
gaggagggcg tggaaggtgg tcttcgcgtc ctccgtccgt ccggtccggt gctgggcctg     240
gccgaggagg cccaggtgga gggggcggcg caagtgggtt ctggagttgc ggagttcggc     300
gagggaggag tgcatcaggg tgaggccgtc ctcctgtccg gagtgggtgc ggccccagcc     360
ctccagcacg ccgagcatgg ccttccagta gagcagtccg tgttcgtccg ccaggcggac     420
tccctcggcg ccggaggagc gggccgtgcg ggcgtcgcct tcccaggccg ccacgaccgc     480
gtccacgtag agagcgaagg agcggtcgga gggcctgctg tcgtactcgg tgaggcgcag     540
taattcgcgg cggcgtgccg tggcggtctt gcggtcgccc atgagccagt gggtgaaggt     600
gtcgtaggag cggcaggaga cgcgtgggtc gtgctggaac gtgcgggcca gggagtggcc     660
ctcgccggcg tactcgtccg ccatggcgac gccgtgctcc agttcggtga gtgccggccg     720
caactggccg cggatgtgga ggacgatgcc ctcgccgtag gccgcgccga gcaccgccac     780
cgggtggccg gtccggtcgg cgaggttccg gagcaggccc gagaactggc gtgaggcgtc     840
gtagcggccg gtgacgatgt acgccgcgca cagggcccag agcaccgacg ggtcttcggg     900
ggagtgggtg gccgcgctca gggcccgtcc gcgggcgagt gccgtctggg cctcggcgtg     960
gccgtagccc cgggtgatgg ccagcacctg gccgagttga atgtgcagcc gctggttcag    1020
ggacacggcc gaggggtcgc cgggcggcag caggccgacc aggtgcaccg cgcggcgcag    1080
ccaggtctcc acctgttcgt aggcgagttg ctgctcggcc tgctcggcgg cgcgcagcaa    1140
cagaggaagc gtttcttcag tcggcagtgc gctcttggcg tgccaggcgt ggtgggcgat    1200
```

ccgctcgatc tcctcgtccg ccacctgacc gagcgtgcgg gtcgatatcc cctcggcgac 1260
tctcgcgtgc agccgctgcc ggtcctcgcg ggggagctcg tcgatgagcg tctcctggac 1320
cagggcgtgg gtgaagtgga gccgttccgg gtggtggcga tcctcgccga gcagcccggc 1380
ccggatcgcc aactccagtg ccgcggtgac cggttcgtct tcggtggcgg tgcgttccag 1440
cagatcggtg tcgacctcgg tgccgatgac ggcgcagagc ctcagcacgc gcagcaccgt 1500
ctcggggagt gcggcgaacc gctggtgcag ggcctcgcgg acgcccgtcg ggacgcgcgt 1560
caggagcacg tccaccgcgt cgggccgccg caggctgcga gcgtcaccca gaagggagag 1620
gagctgcatg acgaagtacg ggttgccctc gctgcgccgg tgcagcacct cgacgactct 1680
cgcgtcgacg ccgggtccga cctgggcgac gacgagggcg gcgaccgcgc ggcggggcag 1740
gccgccgagc cgcagggtct cggtcctggg gccgcgcagc acctcggaga gcatgcggcg 1800
cagcgtcgca tcggactcga tctcgtgctc acgggcggtc aggacgatgc cgagcgggtg 1860
gccctggctg cgggtgctca gcagtctgag gaggtccagg gaggcggggt cggaccaatg 1920
caggtcctcc atgagcagga cgagtggccg ctgggcggcg agggcgagca ggacttcgca 1980
gaccgcgtcg tgggtgagga accgggcttg gccccagtcg gattcggagg cgagtccgcc 2040
gggccgggcc gcgcgctcgg gcatcagcgg ggcgagcagg gtgccgaacg gtttcgccgc 2100
ctcgcggaag gcgtccgggc gggtcgtgga cagtcgccgg aggatctggg tccacaccca 2160
gtagggcggc acgccctcgc ccaggaagca gtggctccag atcacctcca ggccggcgcg 2220
ctcctgaccg gtgccatcct cgccggcgcg ggcggcttcc agctggggga cgagttcgag 2280
gagcagacgg gtcttgccga cgcccgcggg gccgaggacg cccgcaacgt gcccgtgacc 2340
ggcgagcgcg ctcgacgccg cggctgtcag gcgctgtaac tcctcgctgc ggccggtgaa 2400
cggcgactgg gcgcccggac cgccgcacac cggccctgag gcctcggtgc tcgtgtcgca 2460
ctcctgggct ttcgcatcct gcgtcggacg cgcgctctcc gctgaatccg cggtggtcga 2520
ccgcgatgtc gcggtgatcg tcaccggccg gcgcgcgggt cccggtgtgg cggtgggcgc 2580
gtccgcctgc gccccgtccg cggcagcgct tccggcccct cccgatcccg cggacagcgt 2640
gccgtcctgg ggagcgtcat caccgcagtc tgctgcgaag ggctcgtccg gagccgctgc 2700
cgcccggccg atcccaccgg acgcctcggc cgctgtgggc cgctcgggct cctgcgccgc 2760
gcgtggcggg tgcgcatccg gccctcgtgc accgtcgccc tcgtgcgccg tggccacccc 2820
gatcacagga gcccccagcg cgggcccggc aggttcgggg cgaccgggtc gtggtcccgc 2880
ggggggaccg cccgcacccg gctcctggcg caggatcgcg gtcctgaccc ggcggagttc 2940
ggcggccgtg cccacgccga actcctcgtt caggtggacg cgggtccgtt cgtacacttc 3000
gagcgcctcg gcctgccgcc cgagctggga cagggccgtc atcaagtggc cgaccaggcg 3060

ttcccgcgcc gggtggcggc gcacctcgcg gtcgaggccc gcggccactt cctgggcctc 3120
gccgagggcg agtcgggcct gcgcgcagga ctccacggcg gtgagccgga cgtgttcgag 3180
ccgcgcactc tcgtcgctga gcggaggatg gccgtcgaac tccgcatagg gcgagccccg 3240
ccacagtgtc agggctgcgg cgaaccggtc ccgcgcggcg agcgggtcgc gttgttccag 3300
cagccgccgc ccttcggcga ccaggcgctc gaagcggcag gcgtcgagct gttcgggggc 3360
gagttccagg acgtagccgg gcgcgcggtg gcgcagcacg gtggggcggt cgagcccgga 3420
cgccggctgc agtgcgcggc gcaggtggga tacgtggctc tggagcgtgg cgacggcctg 3480
acgcgggggc tcctcgcccc acagctcctc gatgagcagt tccgtgggca ccacacgccc 3540
caggcgtatg aggagcaggg cgagcagggc cctgcggcgt ggcggaccga gtgggaggtc 3600
gcggccgtcg tggcgtgccg acatggggcc cagaacgctc agttggaggg gagtcccgtg 3660
cggcccgtcc tcggaggccg gagtgagggg cggctgattc ggcgtcaggg atgaaggcat 3720
atcggtccca atcgttctcg cacgtgcgat cgtgctgacg ggcgggtggc gcagtgcggc 3780
cggtgcccgt ccaaaccgga gctttgcata gagaacgggg acgttccaag atcaccctgg 3840
ctgtcggcaa gcgaatggca aggtccctgc agggagttcg gcaaggttgg cggaaaaccg 3900
ctacagcgaa cggcaggaat tcaccagtgt gaagaccagg ccgctgcaag gggggtggtg 3960
cacgatgccc gtcatcggaa accaccgctg aagggggagg tcatgaaccc gccggacgac 4020
tgccctgccg tcacccgaga acactccgct gcgggcccgg catgagggct tccacagcgg 4080
gagtgcccgc cgtgctcttc cccggccagg ggtcccaggc ccgtggtatg ggagccggcc 4140
tcttcgaccg gtaccccgaa ctgaccgcgt tggccagcga tattctcggc tacgacctcc 4200
cgcggctgtg cctggaggac ccggacgggc ggctcgacga cacgcgctgc acccagcccg 4260
cgctctacgt cgtcaacgcc ctctcctacc aagactcgct ggagcgcggc gaacccgagg 4320
gcgggtacct gctgggccac agcctcgggg agtacaacgc cctgcacgcc gccggggcct 4380
tcgacttcga gaccggcctg aagctcgtcc tcaagcgggg cgagctcatg gcccgggctc 4440
cggacggggc catgctcgcc gtcgtggggc cggacgcggg tgaggtgcgg gccttcctct 4500
cggaggaggg cctgtcgcgg ctcgacgtcg ccaacatcaa cacccccgtc cagaccgtgc 4560
tgtccggggc ccgggacgag atcgagcgcg cgcacaagac gctcgacgcg cacgggaccc 4620
gggtcgcccg gctgaaggtc tcggccgcct tccactcgcg gttcatggcc gctgcccgcg 4680
acgagttcgc cgccttcctc aaaggcttcc ggttcgcgcc cctgcgggcc acggtgatcg 4740
ccaacctcac cgcacggccg tacacggacc aggacgtcgc ggcgacgctg agcgagcaga 4800
tctgcggatc ggtgcagtgg ctggacagcg tccgctacct gctggagcgc acgaccgccg 4860

gccactgccg tgaggtgggc gggggaggag tcctgacccg catgatccgg cagatcgacg 4920
cggcccccgc ccgcgggatt ccacagccga agccgaagcc gaagccgaag ccgaagccga 4980
agccgcagtc acggccacgc ctgttctgca tcgcctacgc cggaggcgac gagcgcgcct 5040
acgcaggact cgccgaacac tgcccggatg tcgacgtcgt gacgctggaa cgccccggac 5100
gcggccggcg cgcctccgag ccgctgctgc gcgaacccgc cgcgatcgtc gacgatctgc 5160
tccggcagct gcggggccgg ctcgacgccc cgtacgcgct ctacggccac agcctcggag 5220
cccggctggc attcctgctc tgtcgggcgc tgcgcgccga gcggctgccc gcaccggccc 5280
acctgttcgt ctccggggag agcggaccgg ccctcccgag ccgggaacgc cacacctggg 5340
agctgccggc cgacgccttc tgggaccacc tcaaggagct cggcggaatc ccggcggagc 5400
tgtgggagca ccccgacctg atggcgtatt acgagccggt catacgggcc gacttcaccg 5460
cgctgggcgc ctaccggcac gaggacgctc cgccgctgga cgtgccggtc accgccatgg 5520
ccggcgagga cgagtggttc acccgggccg acctggaggc ctggcaacgc gagagcaccc 5580
ggcccctgac cacacaccgg ttccccggtg atcacttctt catccgggcc cagtggcccg 5640
cgctggcccg gatcgtcgct gccgggctcg cggccccctg acgccgcgcc ggaacagcga 5700
agagctcacg cgcgccggcc accggcacgc gccccgaatc gaccatcccg agggtgacgg 5760
acagccatga agcaggaact caagaagcac atggaagagc ggttcatgtt cgagttcgac 5820
tcggacatca ccgaggacac cgacctgttc aaggcgggca tcctcgactc gttcggttat 5880
atctcgctga tgacgcacat cgaggaggag tacggcgtgc cgctcggcga cgagatcctc 5940
ggcaacgtcg cggtctcgct gtccggcatc gtcgcgttcg tcgacgccgc ccgcctgcgg 6000
gccgccggga gccggtgacc cgatgtgcgg catcgccggc ttctacggaa gccccctgcc 6060
accgcaggaa tacgagaccc tgatccacgg catgctcgcc cagatcgagc accgcggccc 6120
ggacgaggcg ggctgcttcc tcgacgaccg cctggccatg ggcacggtac gactgagcat 6180
catcgacctg tccaccggct cgcagccggt cggcagcgcc gacggccgct actggctctg 6240
ctacaacggc gagctgtaca actaccggga gctgcgtgag cagctgaccg cccgcggctt 6300
cgtcttccgc accgagtccg ataccgaggt cgtgctggcc gcctgggtcg cctggggcct 6360
ggactgcctg ccccgcttca acggtgcctt cgcctttgcc ctttacgaca gtgccaccgg 6420
cgaactgcac ctggtgcgcg accggttcgg caagcggccg ctgtacgtgg cgcggcaccg 6480
cggcgcgtgg ctgttcgcct ccgagatgaa ggcgttcctg gcctaccccg acttcaggtt 6540
cgccttcgac gaggcacagc tggcgtcggt cttcgccacc tggaccccgc tgcccggcca 6600
gagcggatac caagggatcg agcagatccc catgggcgag tatctgtccg tacgcggcga 6660
cgaggtccgg cgcggccgct gggccacgct cgacctggcc caaggcccgg ctccggagag 6720

cgagcaggag gccgccgagc ttgtccgcgc ggacctcgaa gccgcggtcg acgtgcgcct 6780
gcgcagcgat gtcgaggtcg gcgtctacgc ctccggcggc ctggactcct cgatcatcgc 6840
gcacatcgcc gcgcagcgga cgagccgccc gctgcggacg ttctcgatcg agttcgagga 6900
cgcagagttc gacgaatcgg ccgaacaggc cgagctggcc gcacacctgg gcacccgcca 6960
ctccaccgtg cgcgtgaccg acgaggacgt cgccgacgcc ttccccgaag ccgtccggca 7020
cgccgaggtg cccgtcttcc gcaccgcctt cgtccccatg tacctgctgg caggccacgt 7080
ccgcagcgaa gggatcaagg tcgtgctcag cggcgagggc gccgacgagg ccttcctcgg 7140
ctacggcatc ttcaaggaca cgctgctgct ctcgacctgg cacgagctgg acgacgacac 7200
ccgtctgcgc cgcatgagcc agctctaccc gtacctgagc cacttcagcg gcgaggacgg 7260
ccaccgccgg atgctcggcc tctaccggca gttcaccgag gagaccctgc ccggcctctt 7320
ctcccaccag atgcggttcc agaacggccg cttcgccgca cgcctgctca agaacccggg 7380
cgaccccttc gcggccctcg gggaactcgt ggccggtgag cccggctacg cacagctcac 7440
ccccgtacag aaggcccagt ggctggagtt ccgcacgctc ctgagcggct acctgctctc 7500
gacccagggc gagcgcatgg cgctggccca cggcgtggag aaccgctgcc ccttcctcga 7560
tcccgccgtg gtccgccgcg ccgcatcggt gaacctgcgg ttcggcgacc cctacgacga 7620
gaagtacctg ctcaagtgcg cctatgccga tgtgctgccg gaacggatcg tcaagaaggg 7680
gaagttcccc taccgcgccc cggacagcgc cgcgttcgtc cgctcccgcc cggactaccg 7740
cgagctgctg accgaccccg gcaccctcga cgagatcggc gtcctcgatg cgcgcttcgt 7800
gaagcggttc accgaccgcg tcttcgacag cccgcctgag cagatcggca cgaaggagaa 7860
ccaggccttc gtctctttgg cgtcgacggt ctggctgcac cactggtacg tgcgcggcaa 7920
cgcccgccgc cgggctccgc tcggggtccc cctgtacgtc gtcgaccggc gcagtggcgc 7980
cctgtcggcc taggacggag aacgcgccat gaagaagcag aacggcgtcc tcgccgacga 8040
ccgggacatc gccgtcatcg gcctgtccct gcggttgccc ggctcgcgca cgcccgagga 8100
gttctggagc cacctggccg agggccgctc gctcatcagc gaagtcccgg agcgccgatg 8160
gcgaaaggag gaccacctcg gtcacccgcg ccgcgaattc aacaagacca acagcgtctg 8220
ggcggcttc gtcgacgacg ccgactgctt cgacgccgat ttcttccaga tctccccgcg 8280
cgaggcgcag tccatggacc cgcagcagcg gatggccctg gagctgagct ggcacgccct 8340
ggaggacgcc ggctaccggg ccgaccgcgt ggcgggctcc cgcaccggtg tcttcatggg 8400
cgtctgccac tgggactacg ccgagctgat ggagcaggaa gtcgaggaga tcgacgccta 8460
ctacccgacc ggcgccgcgt acgcgatcat cgccaaccgg gtctcccacc acttcgactt 8520

ccgcggaccg agcgtcgtca acgacacggc ctgcgcgggc tcgctcgtgg ccgtgcagca 8580
ggcggtgcag gccctccagg ccggcgactg cgacctcgcg ctcgccggcg gcgtcaatct 8640
gacctggtcg ccgcggcact tcatcgcctt cgccaaggcg ggcatgctct cgcccgacgg 8700
cctgtgccgg gcgttcgacg cgaatgccaa cggctatgtg cgcggcgagg gcggcggcat 8760
cgtgctgctg aagcgggccg cggacgcccg ccgcgacggc gacgccgtgc acgccgtgat 8820
caagggcatc ggcagcaacc acggcgggcg caccagttcg ctgaccgtta ccaacccggc 8880
cgcacaggcc gaactgatcg cgggtgtcta ccgcaaggcc ggcatcgcac ccgagaccgt 8940
cacctacgtg gagacccacg gccccggcac accggtcggg gaccccatcg aggtccgcgg 9000
cctcaagcag gccttcgtcg acctgggcgc agaccggccc ggggaggctc cggcccaccg 9060
gtgcggcatc ggctccgtga agaccaacat cggccacctg gaaggggccg ccggcatcgc 9120
gggcatgctc aaggtcatcc tcgccatgcg ccaccgcaag ctgcccgcga cggtcaactt 9180
ccgcaagctc aacccccctca tcgacctgga cggcagcccg ctgtacgtcc tcgaccgcct 9240
caccgactgg accgccgaag ggtccgcacc gctgcgcgcc ggcgtcagct ccttcggatt 9300
cggcgggacc aacgcccacg tcctgctgga agccgcggag ccggtggccg ccaccgagga 9360
cgccggcgaa cagtggctgc cggtgtccgc catggacgag gaccggcttc gcgagacgtg 9420
cgcccggctc gcccgctggg tccggacccg gatcgagcag aacgatgctc cgtccctgac 9480
cgatgccgcc cgcacgctgc gcgaaggccg ggtgtccatg cgcgagcgcg tggtgttccg 9540
cgcaagcggc atcgaggagt gggcggcaca gctggagagc gtcgccgcgg gggacggccc 9600
gcccgcggac tgcccgcgcg gccgggccgg aaccgaagcc cccgacggcc tggacgcgga 9660
cgacctgacg gccctggccg agcgctggct ggagaagggc cggtgggaca agttcgcggc 9720
cgcctgggcc cagggcctgg ccgtggactg ggcaccgtgg cccgagcgcg gccgccgcgt 9780
gcacgtgccc ggctacgcgt tcgcccgcac gccgcactgg ttccggacgg accggaacga 9840
gacgaccgga aggccggagc gcggcgcgac gaacaccgct cccgccccgc tcggcgaagg 9900
caagccggaa ggcggcagct ggaccttccc cctgcacttc gacgccaccc agggattcgt 9960
ccgcgaccac cgcgtcaacg gcgcacggat cgtcccgggc gtggtggccc tggaactcgt 10020
caccgtggca gccgaacggg ccgccgccgc aggtgcccgg gccgggctga cgcccccgcat 10080
ccgcaacgcg gtgtggatcc gtccgctgct cgtcggcgac accgtgctct ccccgcagct 10140
ccgcctgacc cccgccgccg acggctacga ctacgcgatc accgacgagc acggcacgca 10200
gtacaccagc ggccgggtcg agtacggcga ggctgccgcg gccgagaaga cggacccggg 10260
cgcgctgcgc gagcgcttcc cccagcgcgt cgacaccgcc gagggttacg ccgcgctgcg 10320
gtccagcggc atcgagcacg gccccgccct gcgcggcctc aacgccctgc accgcggtcc 10380

ggacggcgtg ctggccgagc tgcggctccc cgcaggtgcc ccggagggca tggcgctgca 10440
gcccgcgatc ctcgacagcg ccctcctcgc ggccctggct ctcggctcgg ccgacggcgg 10500
ctggcgcagg cccgccgccc ccgtggtgcc gttcgcgctg gaccggctca ccgtgcacgc 10560
ggcgacgacc tcgacgatgt gggcgtggct gcggccggcc ggcagcggca cggcaggtga 10620
catggccaga tccgacatcg acctgttcga cgacaacggc cggctgtgcg tgcgcctggc 10680
cggctacacg tcgagggaac tgcccaccgc agaaccggca gcagtccagg ccccggaagg 10740
ggagctcctg gaggtcaccg gtgtgtggga ggaggcccct gccccggcgc ccgcagccgg 10800
tcaggccacc ccggtcggcc cggtgacggt gctcaacgcc gcactggacg gcgacctcgc 10860
agcggcgagc gccgcgcggc tgggcatgga catccggcag ctggccggtc ccgcggaagc 10920
caccgatgcc accgatgccg tcgccatgaa ggcggcgttc gaggcctgct acccgcaggt 10980
ccggcaactg ctcggtcagg gacggcaggt gctcgtcgtc gccccgggcg ccccggactc 11040
gccggtctac gccccactgg cggcgctgct gaagaccgca caacaggaga atccttcctt 11100
ccgggggagg ctggtgctcc tcgacggcta cgacccccgc gacgccgacc gcttcgagcg 11160
tgtcgtcagc gcggaggcgg gcgccggaga cgacaccgaa gtcgcctacg acgcccagga 11220
ccgccgactg cggcacggct tcgtggaact tccccggggc gaggcggggg agagcctgct 11280
gcgcgacggt ggcgtctact ggatcaccgg gggcgccggc ggactcggcc tgctgctcgc 11340
cgagcggctc tgcgagcgcc gccgcgccac ggtcgtcgtc agcggccgct cggcggacag 11400
ccgggccatc gaggcactgc gggcccgcct gttccacggc gaggtggcgt accgccgcac 11460
ggacgtcacg gacgcggacg ccgtacggga cgcggtcgcg gacatccgcg cgcggtacgg 11520
acggctcgac ggcgtgttcc acgcggccgg cgtcctcgac gacggctacc tcgcgagcaa 11580
gcccctcgcg ggcaccgccg ccgtactcgc gcccaaggtg gacggcgcca cgtccatcga 11640
tgacgcgacg cgcgcccacg gcctggactt cctcctgctg ttcggctccg tggcgggcgc 11700
cttcggcaac gccgcgcagg ccgactacgc cgccgccaac gccttcctcg acgcgttcgc 11760
cgcacgacgg caggccgccg gcagcgtgac ccgctccgtc gactggccgc tgtgggccga 11820
cggcggcatg cgcgtggacg acgccagcct cgcctacctg cgcaagcgca ccgggaccgt 11880
gccactgccg agcgagaccg gcctggacgc actggagcgc gcactgcact ccgccgcgcc 11940
ggtccgccgc gtggtgctct tcggggagcg gtccaagctg cgcgggtacg cgggcctgga 12000
ccgcgtcgcg aagccggagc cgcgcacgtc cggggcgcag cggaacacgg ccgcgccggc 12060
cgtcctggag gagagcgaac tcgtagcccg tacacaggac ctgctgcgga acctgttcgc 12120
cgaggtgacc ctgcaggacg cggagcacat cctggccgag gagaagctgg agacctacgg 12180

```
tatcgaatcg atctccatcg tcgagctgac cagcaagctg gaggacacct tcgggtcgct 12240
gcccaagacg ctcttcttcg agtacgtcga tctgcagggc gtggccggct acttcgtcgc 12300
cgagcaccgc gaccggctcc tcgaactctt cgcccccgaa gcacccgccc ccgaagcacc 12360
cgcccccgaa gcacccgccc ccgaagcacc cgcgcccgag gagcccgccc cggaggggcc 12420
tgccgtcgag gagccgcccg cggccgcgcc caccccggcc gtccggccgt ccgtggaggc 12480
cgccgccggg cgcgcccgcc cggcctgggc cgatccggag cgccacgaca tcgcggtcat 12540
cggtatggcg ggccggtacc cgggcgccga caccctggag gagttctggg agctgctcag 12600
cgagggccgg cacagtttcg agcccgtccc ggaatcgcgg tggcggcacg gcgacatcta 12660
cttcgacgag cgtgacgtcg acggcaagac cgtcgtcaag accggcacct tcctgcggga 12720
cgtcgaggcg ttcgatccgc gctacttcaa catctcccag cgcgacgccg agctgctgtc 12780
gccggaggtc cggctgttcc tgcaggcggg cgtggaggcc ctggaggacg cgggctactc 12840
acgtgagacg ctgcggcgcc gctacgacgg cgacgtcggt gtgctcgtcg gctcgatgaa 12900
caacagctac tcgctctacg gcttccagaa catgctgatg cgcggcaccg cgaccagcgg 12960
cagcgagctc ggtgtgatgg cgaacatgct gtcgtaccac tacggcttca ccgggccgtc 13020
cgtgttcctc gacaccatgt gctcctcggc gtcggcgtgt gtgcaccagg cggtgcgtat 13080
gctgcgcagc ggcgagtgcc gcatgaccgt cgtcggcggc atcaatctga tgctgcaccc 13140
gttcgacctc atcgcgacct cgcaggcgca cttcaccacc aagtcggccg aagtcgtgcg 13200
cagttacggc ctcggcgccg acggcacgat cctgggcgaa ggcgtgggca cgctcgtgct 13260
caagccgctg gccgaagccg tcgccgacgg cgaccacgtc tacggcgtga tcaagggcag 13320
cggcatgacc aacgccgggg tccgcaacgg cttcacggtg ccgagcccac agcagcaggc 13380
gcgcgccatc gagaaggcgc tcgacgacgc cgccgtggac gcgcgcacga tcagctacct 13440
ggagggtcac ggctcggcga cctccctcgg cgaccccatc gagatcaagg gcgccgccct 13500
cgcgttcggc cgggacaccc aggacctggg gttctgcgcg ctgggctcgg tcaagtccaa 13560
cgtggcgcac ctgctgtccg gatccggcat ggccggcctg accaaggtgc tgctgcagct 13620
caagcaccgc acgctggcgc cctcgctgca cgccgggacg ctcagctcag cgatcgactt 13680
cgaggagacc ccgttcgtgg tgcagcgcca ccgcgacacg tggcggcgcc ccgtggtcgg 13740
cggcgaggag gcgccgcgcc gggcaggcgt cacgtccatc ggcgcgggcg gcatcaacgt 13800
gcacatcgtc gtcgaggagt acgacggcca ggtcgtcgcc gcaccggagc gcggtcgccc 13860
gcggctgctg gtgttctccg ccatgacacc ccaggccctg cagtcggtgc tgcgcgccat 13920
gcacgagcac gtacgggaga ccgcaccggg cctggacgcc ctcgcgtaca ccctgcagac 13980
cggcaagaac gaactgccgt gccggctggc cttcgtcgcg gacgacatcg cggacgccca 14040
```

ggcccgtctg gcccggctgt ccgcggtgga ctggacggcg gagtcacccg gcgtgcccgc 14100
aggcgtgcac ttcacggcga gcacgctgcg gcgccggcgc accgccgacg cggcgaccgt 14160
cgaacaggcc ctgcgcgacg ggaagcaggc ggagctggcg cagcactggg cggacggcgc 14220
gagcgtcgac tgggacctgc tgtggccggc gggaagccgt ccggccaagc cgtcgctgcc 14280
cgcctacccc ttcgacaagg tgcgctgctg gtaccccgag gacgacgacg cgcccagcgt 14340
gctgcggccg ctcgccttcg cccggcgcgc gcacccctgg gtcggcgtca acgcctcgga 14400
cctgggcggg gtgcgctaca ccctccggct gcgcggcgac gaactcctcg actacgtcta 14460
caccgtagga cgcaagcgcc gttacgccac cgtggcgctg ctggacgcgg cactggcgtt 14520
cgcgcggctc gccgggctgg aagggccgct gcggttgcgg aacgcgcagt gggccgcact 14580
cccgtcgccc gcggacaccc ccgagacgtt cacctggcgg ctcggcacgt ccggcgacgg 14640
cgtgcatcgc gtcgagctgt ggcacgccga tgaggccacg ctccggttcg ccgccgacgt 14700
cgtaccgtcc gcgcctgccg aagacgcatc gatgccgcag atgagcagcg cgcccgcgac 14760
cctcgaccgg gacgacttct acgccgcgct cggcaccgcg ggcctcgacg cccggccgta 14820
cgcgcgcagc gtcgaagggg tcaccgaact cgacgcccac cggctgctcg tacgggtcgc 14880
cgaaccggcc atgtgccagg acccgcacaa gcagcacgtg catctcccgg cctgggcgct 14940
cgtcgggctg acccagggtg ttcagcacgc gtggggccgg gccgacgccg ccgtggtgcg 15000
ggtcggatcc gtgcagggcg agcagtggga gcgcacccgg gcgatcgtgc tggcgcggac 15060
gtccgacgcc gtcttccatg cggctttcct cgacgaggac ggccgcgtgc tgggccgggt 15120
cgaggacgcc gagttcaccg cgggcgacct ggagccggca ctccccggtg aggccggacg 15180
cgcactcgtg gcactgccgc aggcgtcgcg tccggtgctg gagacgccgg ttggtacggg 15240
ggagtggcag cagtcggagg ccgtgcggcc ggaggccgag ccgtccgtga ccgttgcggc 15300
ggtcgcggac gggccggcgg cgctcgtcgc gtcgctgcgc gagaccgtcg ccgacctgct 15360
caagttcgac ctggcggaca tcgacctcga cacgcacttc cacgcgtacg gcttcgagtc 15420
catcgcgctg gccaaactgg cctcggaact caacggcgtc ctcggcacgg acctcacccc 15480
cgccgtcttc ttcgagtgct ccgacatccg cagcctcgcc gagtacctgc tcgaccgcta 15540
cggccccgag ctgagcctcc ccacgagcgc cgacgccccc gcgccggtcg ccgccacccg 15600
gccgtcccca gtgccgatgc cggcacccgg gccggacgac gacgcggtgg ccatcgtcgg 15660
cgctgccgga cggttccccg gcgcggacga cctggacacc ttctggcagc agctgcgcgc 15720
gggcgaggac ctgatcgccg actaccccgg cgaccgcttc gacgggggcc cctacgcgga 15780
ggtcgtcgcg cgggcggact tcccgaagtt tgccggccgg atcgagggcg tggaccgctt 15840

cgacgcggac ttcttccacc tgtcgcggct ggaggcggag ctgatggacc cgcagcaccg 15900
gctggccctg gagaccgtgt gggccgcgct ggagaacggc ggctacgccc cggcgcgcct 15960
ccccgagaac accggggtct acttcggcgt ctccggcagc gactaccacc acctgctcaa 16020
cgccagtggc gtggcacccg acggcttcac cgccaccggc aacgcccact cgatgctggc 16080
caaccggatc tcctacgtcc tggacgtgca cgggccgagc gaacccgtcg acacggcctg 16140
ctccagctcg ctcgtcgcgc tgcaccgcgc cgtcgagcac atccggtcgg gccgatgcga 16200
gatggccatc gcgggcggtg tcaacctgct gctgagcgtg gacaccttcg ccgcgacgca 16260
catggcgggc atgctcagcc ccgacggccg ctgcaagacc ttctccgccg gcgcggacgg 16320
ctacgtccgc tccgagggcg tcgccgcggt gctgctcaag ccgctcgccc aggcgcagcg 16380
ggacggcgac gccatctggg gcgtcgtccg gggcagcgcc gagaaccacg gcggccgcgc 16440
cggttcgctg accgccccca acggcaaggc gcaggccgcc ctgatccagg acgccatgcg 16500
cggcatcgac ccggacagca tcggctacgt cgaggcgcac ggcacgggca ccggcctggg 16560
cgacccggtc gaggtcaacg ccctcgacag cgcctaccgc gccctgcgca ccgccgaggg 16620
cgggccgccg cacgcggccc ggccgtgcgc gctcggctcg gtgaagacca acatcggcca 16680
cgcggagtcg gccgcgggcc tggccggagt gctgaaggtg ctgctcgcca tgcgtcaccg 16740
cgagctgccg ccggccttgc actgcgaccg gctcaacccg cacctgccgc tcgacggcgg 16800
attcgaggtc gtacgcgaac tgcgccgctg ggaaccgtgc accgacgcca ccgggcggcc 16860
gtggcccctg cgggccggag tgagcagctt cggcttcggc ggcgccaacg cccatgtcgt 16920
cctcgaagca ccgccccgtac cgcccgcacc ggcggagccg gcccgcccga ccgcccccca 16980
ggccatcgtg ctgtccgccc gcgacgacga ccggctgcgt gccacggccg gacgactgcg 17040
ggacttcctc gaccgggcgc gccgcgacgg acacgccccg gacctggcgg acctggcgtt 17100
caccctccag gtaggccggg aggccatgga acggcgcctg ggcttcgtcg tcggaagcat 17160
ggacgacgtg ctcggtacgc tggaccggtt cttcgcgggc gacgagccct ccggctggca 17220
caccggcggc atcaggcggt cgcgtggcgc cggagtgcgg cgcgaggcgg agcaggcccc 17280
cgaggtgacc cgggccctcc acgacggacg gctcgaccgg gtgacggccc tgtggtgcga 17340
cggcgccccg gtcgactggc aggcgatgca tcccacaggc gagcgccgcg ccgtgcggct 17400
gcccgcgtac cccttcgcct gcgaccgcta ctgggtgccc gcggtcggca cagcccccgt 17460
cccgccgccc gcggcacccg tcccgccccc cgcggccgag cccgcgttcg agaccgatgc 17520
ccgtgcggcg ctgctcgacg cggtcctcga cggccgtgcc ggcccggacg ccctgagcag 17580
gacctgacgc cttccccctg cctcctcccc ggaccgggcg gctcggccgc cccgtgacct 17640
ggaacggaat gaacgtgagc agaaacatcc ttcgtgtgcc ggaatggcgc gacgaaccgg 17700

cgcgagggcg caccgcgccc cccggaaacc ggcggctggt cgtgctgtgc gacaccccccg 17760
acgcggacgt gaccgacctg cgccggcacc tgcccggcgt gtccgtcgcc cgcgtggaca 17820
gcggtgacga cgggcccgct gccgcctacg agcacgcggc gaccctgctg ctcggcgagc 17880
tccagcggct gctgaaccag ccggccggcg gcccccgttc cgtgcaggtc gtgtgccggg 17940
aggggactcc gtacggctac gccggtctga tcggcatgct gcgcaccgcc gcgcaggagg 18000
acccggcgct gcacggccag ctgatcgagt gcacgcagcg gccgtcgggc gaggaactcg 18060
ccggcgtgct gcgggcggag tacgggcagg cggcggatca cgtgcgctac accggcggcc 18120
gccgccaggt ccgcgcctgg gcagcggccc cgcgtgcggc ggcaccccccg ccggtgtgga 18180
aggccgacgg cgtctacctg atcagcgggg gagcgggcgg cgtcggccgg ctggtcgccg 18240
ccgacatcgc acggcacgcc cccggcgccc gggtcgtcct gtgcggacgc tcgccggcgg 18300
tccccgggcc cggtcagccg ggcccgggga ccgagtaccg ccgggtggac gtcgccgacg 18360
ccgacgccgt ggcggagctc gtcaactccc ttgtgcgcac gtacggcagg ctcgacgggg 18420
tcgtgcacgc ggcgggcctg atcagcgacg actacgtgat ccgcaagtcc caccaggacg 18480
cccagcaggt cctggcgccg aaagccgctg ggctggtgaa cctcgacgag gccacccgcc 18540
gcctgccgtt ggacttcctc gcggcgttct cctccggcgc ggggacgctg ggcaaccccg 18600
ggcaggccga ctacgccgcc gcgaacgggt tcctcgacgc ctacctgacc caccgcgccg 18660
gcctggccgc cgcgggcgag cgccacggcg cgagcgtctc gatcggctgg ccgctgtggc 18720
aggacggcgg gatgagcgtc ccggccgagg acgtgcccgc gctcaccgcc cgcttcgggc 18780
gccccctggg aacggacacg gcactgcggg ccctgcacgg cgcactggcg ctcggcacac 18840
cacacctact ggtcatggac gaggagagcg gagtggacga agagagcgga gtggacgagg 18900
aaggtccgca ggaggcggag acgcagcaga cggggccggc ggaactgcgg gcacatgtgc 18960
tgcccctgct gaaggagttg atcgccgaga cggtgcggct cgaccccgcc cggctggacg 19020
ccgccgctcc gctcgacggc ttcggcatcg actcgctggc cgtgacccgg ctcaaccgcc 19080
ggttcgcgca gtggttcggc gcgctgccca agacggtgct ctaccagtac ccgacgctga 19140
acgacctggc cgggcacctg gcggagcagc acgcggacgg ctgccgccgc tggctcggcg 19200
acgtcccgga cgtggccgcc gccccggccg ggactccggc gacggcggcc gcgccgcgga 19260
aggcgcggcc ccgtccggcc gacgcggacg agccgatcgc cctcatcggc ctgagcgggc 19320
gctatccgga cgcccccgacc ctggaggcgt tctgggagaa cctgcgcgcg ggccgcgaga 19380
gcgtccgcga ggtccccgcc gagcgctggc cgctggacgc cttctacgaa ccggacccgc 19440
agcgggccgt gcagcagggc gccagctaca gcaagtgggg cgcgttcctc gacgacttcg 19500

cccgcttcga cgccgcgttc ttcgggatcg cgccgcgcga cgccgccgac atggacccgc 19560
aggaacggct gttcgtcgag agcgcgtggt cggtgctgga ggacgcgggc tatacgcggc 19620
agcgcctcgc cgagcagcac gcatcgtcgg tcggcgtctt cgccgggatc accaagaccg 19680
gcttcgaccg ccaccgcccg ccggcgaccg acggactgcc gcccgcgccg cgcacgtcct 19740
tcggatcgct ggccaaccgg gtgtcgtacc tgctggacct gcacggcccg agcatgccga 19800
tcgacaccat gtgctcgtcg tcgctgaccg cgattcacga ggcatgcgag cacctgcgcc 19860
acggcgcgtg cgagctggcc atcgccggcg gtgtcaacct ctacctgcac ccctcctcgt 19920
acgtcgagtt gtgccgttcc cggatgctcg ccaccgacgg gcactgccgc agcttcggcg 19980
cgggcggcga cgggttcctg cccggcgagg gcgtcggcgc ggtgctgttg aagccgctgt 20040
ccgcggccga ggccgacggc gaccccatcc acgcggtgat cgtcggctcc gcgatcaacc 20100
atggtgggcg caccaacggt tacaccgtgc ccaacccgcg cgcacaggcc gcgctgatcc 20160
gcgacgcgct ggaccgcgcc ggtgtgtccg cggccggcat cggctacatc gaggcgcacg 20220
gcaccggcac ccggctcggc gaccccgtcg agatcgacgg cctgacccag gccttcgctc 20280
ctgacgccgg cgggagcggt gcgtgcgccc tcggctcggt caagtcgaac atcgggcacc 20340
tggaggccgc tgcgggtatc gcgggcctga ccaaggccgt actgcaactg cagcacggcg 20400
agttcgcgcc caccctgcat gccgagcaga ccaacccgga catcgacttc gcggccaccc 20460
cgttcaccct gcagaccggc ggggcccctt ggccgcggcc cgcggacggc ggcccgcgga 20520
gggcaggcat ctcctcgttc ggcgcgggcg gcgccaacgc ccatgtcatc gtcgccgagt 20580
accggagcgc gacgcccgca cccgccacgc ccgccccgtc cgcgcggccg gtgctgctgc 20640
cgctgtccgc ccggaccacc gaggacctgc acgcacgggc cggccaactg tccgacctgc 20700
tccgcaacgg cgcccccgtg gacctgcccg ccgtcgcggc caccctccag accggccgcg 20760
aggagatggc ggagcgggtg tgcttcgtcg cgagcacacc cggggaatgg ctcgaccagc 20820
tcggcgcctt cctcgccgac tccgactccg actccgactc cgactccgac tccgactccg 20880
actccgactc cgactccgac tccggctccg gctccgaggc cgaggccgag gtcccgtggt 20940
cccgcggccg ggtcagggcc acccgcgaga ccctggcagc cctggcggag aaggacgaac 21000
tgcgcgcact cgtcacccgc tggatcaacc gcggcgactg gcacgacctg gccgccttct 21060
gggccaaggg catgccgctc gactggaccc gcctgcacgc cggtgcggac acgcccgcac 21120
gggtccacct gcccgcctac cccttcgccg gacggcagtt ctggttcggc ccggccggca 21180
gcgagcaccc ggcaacgacg ccggtggccg ccccgtcctg ctcgacggca gccggtgccg 21240
ccgacgtcga gcgcatcctg ctcgacgcac tggcagcggc cctgcagatg ccggtcgccg 21300
agatcgagcg ccgccgcccc ttcgccgact acggcctgga ctccatcctc ggcgtgaacc 21360

tggtccacac gctcaacacg gccctcggca ccgcgctgga gaccaccgat ctgttcgacc 21420
acggcaccgt cgagcgcctg cacgcgttcc tcgtcggtac ctacggtgac gcactgcacg 21480
caccggcctc cccggcagcc gtcgccccgg cgccagacga cgacgccatc gccgtcgtcg 21540
ggatggccgc ccgctacgcc gacgccgagg acccgcgcgc gctctgggac cacttgatgg 21600
ccggccacga cctcgtcgaa ccggtgaccc gctggccgct cggccaggac gtgagctgcc 21660
gctccggcag cttcgtccgc ggcatcgacc agttcgaccc ggtgttcttc gcgatctccg 21720
gtgtcgaggc caccaccatg gaccctcagc agcgcatctt cctcgaacag tgctggaacg 21780
ccctggagga cgccggctac accggcgaac gcctgaccaa ccgcaactgt ggcgtctacg 21840
ccggctgcta cgccggcgac taccacgacc agctggacgc ccggccgccg gcgcaggcgc 21900
tgtggggcac catgggctcg gtcgtcgcct cccggatcgc ctaccacctc gacctcaagg 21960
gccctgccct caccaccgac acctcctgct ccagctcact cgtctccctg cacctggcct 22020
gccgcgacct gctctccggg gacgccgaca tggcgatcgc gggcggggtg ttcatccaga 22080
ccacgtcgcg gctgtacgag tcggcgtcgc gcgcgggcat gctctcgccc agcggccgct 22140
gccacagctt cgacgcccgc gccgacggct tcgtcccggg cgagggcgcg ggcgcagtcg 22200
tcctcaaacg gctcgccgac gcccggcgcg acggcgacca catctacggc gtcgtccgcg 22260
gctccggcat caaccaggac ggcaccacca acggcatcac cgccccgagc gcggcctcgc 22320
aggaacagct cctgcgcgac gtccacgccc gcagcggcat cgagccgggc ggcatccagc 22380
tcgtcgaggc gcacggcacg ggtacccagc tcggcgaccc gatcgaattc cgcgcgctca 22440
cccgcgcgtt cgaggacgcc ccggccggga gcgccgtgct gggatcgatc aagaccaaca 22500
tcgggcacac gcagttcgcc gccggcatcg cgggcgtcat caaggcgctg ctggccctgg 22560
agcaccggca gatcccgccg tcgctccact tccaagaggc caaccgggcc gtcgtgctcg 22620
acggcggccc gttcaccgtc accaccgccc cgcagccctg gacggcgcct gcccgcggcc 22680
cgcgccgggc ggccgtgagt tccttcgggg ccagcggcac caacgcgcat gtcgtgctgg 22740
aggagcaccc ggtcccccgg acgaccggcg cgggcgggga acacgccttt ctgctgtcgg 22800
cccgcacacc ggccgctctc cgtgccgtcg ccgaacggct gctcgcccac ctcgaccgcg 22860
aacccgggct gcccgccgac gccgtcgcct tcagcctggc cgcgggacgc agccacttcg 22920
cgcaccggct ggccgtcgtc gccgccggcc tgcccgacct ggcggcacgc ctgcgctcct 22980
ggctgtccgg caccgccggt gacacggtgc tgcagggggga gaccgccgcg gacccccgcc 23040
ccgtcggcgg tgtgcgcgcg ccggccccgg ccgcgctggc cgcagcgtac gtacggggcg 23100
aggccgaccg gttcgccgac agcttcgcgt ccgcctcgcg ccgccaggtg ccgctgccga 23160

cctacccgtt cgagcggcag cgctactgga ccgacacgac cgacaccggg gaaagccagg 23220
ggctcaagga cacggacggg gccgcgtacc gcctccggct cggcggcgag gagttcttcc 23280
tggccgacca ccacgtgggc ggccgggccg tgctgcccgg cgtgctctcg ctggagttcg 23340
cacgccgtgc cgtgaccggc ggttccttcg cgccggtcgg cctgcgcgat gtcgtatggc 23400
cggagccgtt ccccgtcggg gacggcggcg ccgaactacg agtcgatcgg gacggcgacg 23460
ccttccgcgt cctgcgcgac ggctcggccg tacacgccca gggccggatc gccacgcccg 23520
gctcgcccgt ccccacgccg ttggacgccc tgcgggcccg ctgcggccgc cgcaccctgt 23580
cgcggagcca gtgccgtgcg gccctcgacg ccgtcggcat ccgccacgga gaccgcctgc 23640
gcgccatcga caccctggcc gtcggtgacg gcgaggtcct ggcccggctc gtcctgcccg 23700
acggcgcccg cgacggcgcg ttcgcgctgc accccgcgat gctcgacagc gccgtgcagg 23760
ccgtcgtcgg cctctacggc gacgccaccg gcacgctcga cgagcaacgc ggcgcgcccg 23820
cactgccctt cgccctggac gccgccgact tcttcgcccc caccaccgaa cgcatgtggg 23880
cccacctgcg ccacaccgag ggctacaccc cctcggccga ccgggacgtg acgaaagtgg 23940
acatcgacgt gtacgacgac gacggacagc tctccgcgag cctgcgcggc tacgcgttcc 24000
gccgcatgac cgccccgtcc ggcgcggccc cgcgtgccac gctgctggca ccggtgtggg 24060
acgccctgcc cgtcgtgccc gccgagccgt ggccccaccc gcggacccgc gtcgtgctgc 24120
tgggcggcac ccccgaggaa cgggacgggc tccgccgccg ctaccccgac gccaccgtcc 24180
tggaccccca cgccgacgaa ccggtcgacc ggctggccgc gcggctgccc gccgacgccg 24240
agcacgtctt ctggctcgcc ccggccggcc ccaccggcgc cccggccgcc gcgcggtacg 24300
acggcacgat cgccgtattc cgactggtca aggcgctcct ggccgacggc gcggacgccc 24360
gtgaactggg cctgaccctg gtcacccggc aggcgcgcct gctaccgggc gacaccggtg 24420
ccgaccccgc ccacgccggt gtgcacggcc tcgccggcac cctggccaag gagtacccgc 24480
actggcggat ccgcgtcgcc gacgtcgagg cggacgccgc cgtgccctgg ccggctctgc 24540
tggctctgcc caccgacccc cgcggcgaga ccctggccca ccggcacggc gagtggtacc 24600
gcctgcgcct gctggagacg gacgggaccg gcgtcgcggc cgccccgcgc gagcccggcg 24660
gcgtgatcgt ggccatcggc ggcgccggcg gcatcggcac cgtgtggacc gagcacatga 24720
tgcgccgtca cggcgcccgg gtcgtctgga tcggacgccg cccgctggac gccgccatcg 24780
ccgctcagca ggaagccctg gcagcccacg gccccaagcc ggactacgtg caggccgacg 24840
cgaccgaccg cgacgccctg cgccgcgcct gcgacgagat cgtgcggcgg cacggccccg 24900
tgcgcggcgt cctgcacacc gcgatcgtcc tcggcgacca gaccctcgcc cggatggacg 24960
aggaccggtt ccgcacgacc tacgccgcca aggccgacat cgccgtgaac ctcgccgacg 25020

ccttcgccgg ccagccgctg gaattcgtcg cgttcttctc ctccatgcag gccttcttca 25080
aggcccccgg ccaggccaac tacgcggcgg gctgcacctt cgccgacgcc tacgccgagc 25140
acctgtccac ccggctcgac tgcccggtca aggtcatgaa ctggggttac tgggccggcg 25200
tcggcgtcgt caccgccgac ggctaccggc agcggatggc acagctgggc ctgggctcga 25260
tcgaaccgga cgagggcatg gccgccttcg acaccctgct ggcctccccg tacccgcagc 25320
tcgcactcct caaggccacg gacacccgca gcatcgacgg cctccacgac gacgacgccc 25380
tcacgcaccc ggtcgtcacc accccctccc tgatcggcgc cctgggcgag gactgccccg 25440
accgccgcgc cgagatcgcg cagctgcgtg agaaggcggg cgggcacgcc ggagccatgc 25500
aggacgcgct cgtccgcatc acctgggcgc tgctgcagtc cctgggcctg ttccgcgacg 25560
gccgcgcggc caccgccgcc gagtggcgcg ccctcggcgg catcgaggac cgctacgagc 25620
gctggaccga gcacaccctc gccgtactcg ccgacgcagg cctcctgcgc cgcgagggcg 25680
aggacacgta cgtggccctc gacacccgta ccggatccct cgacgacgcc tgggccgact 25740
gggaccgggc gcggcagcag tggctggccg acgacgccaa gcgtccccag gcggtcctcg 25800
tcgacacgac gctgcgcgcc atgaccggca tcctcaccgg ccgccgcccg gccaccgacg 25860
tgatgttccc gaacgcctgg ctcgaactcg tcgaggccgt gtacaagaac aaccccgtcg 25920
ccgactactt caacgacgtg ctcgccgaca ccctcgtcgg ctacctcgaa cggcggctgg 25980
cggacgaccc gtccgcccgc ctgcgcatcc tggagatcgg cgccggcacc ggcggtacca 26040
gcgccacggt cctgcgcagg ctgcggccgt gggcccggca catcgagaag tacacctaca 26100
ccgacatctc caaggcgttc ttgctgtacg ggcagcggga gtacggcgag atcgccccgt 26160
acctggacgc acggctcttc aatgccgaga agccgctggc aggccaggag gtggaccccg 26220
gcgcgtacga cgtcgtgatc gccaccaacg tgctgcacgc gacccgcaac atccgcagga 26280
cgctgcgcaa cgccaaggcc gccgcgcgcc cgaacgccct gctgctgctc aacgagctca 26340
gcgacaacat cctcttcagc cacctcacgt tcggcctcct ggacggctgg tggctctacg 26400
acgacccggc gccgcgtatc cccggttctc cgggcctggc gccggagagc tggcggcggg 26460
tcctcggcga ggtcggcttc cgcgcggcgt tcgtcgccgc cgggggcgcc gacgacctcg 26520
gccagcaggt gatcgtcgcc gagagcgacg gcgcgatccg ccagccgcgc ccggacgggg 26580
agtccgcttt ccgcggcacc ctcccggagg ccgggccgcg ggccgccgag cctcaactgc 26640
ccgccccgac accggatccg gtcgccgccg acggcgtacg tgacgacgag ctcctggcgg 26700
acctggcccg cgaccacttc cgcaccctgg tcgcggacac cttgcaactg ccggtcgccg 26760
acatccgcgc cgatgtgccc ttcgaccgct acggcatcga ctcgatcctg gtcgtccagc 26820

tgacggaagc ggtccgcaag gggctctgca acgtcggcag cacgctgttc ttcgaagtac 26880
ggacggtcga cgggctcgtc cagcacttcc tgcgcaccca gcccgacgcg ctcgcggcac 26940
tggtcggcct gagcggcgcg cgggcagcgc gcacggacga gcagctcgcg ccggccgccg 27000
ggccggagcc ggtccccgtc atcgccgccg aaccgccccg cgccgagcag ggcatggcca 27060
tcgcgatcgt cggcatggca ggccgctacc ccggcgcacc cgacctggac accttctggg 27120
agaacctgct cgccggccgg gacagcatca ccgagatccc ggccgggcgc tgggaccaca 27180
gccgctacta cgacgcgcgt cgcggcgtgc ccggcaggac gtacagcaag tggggcggct 27240
tcctcgacgg gatcgacgag ttcgacccgc tgttcttcgg gatctcgccg aaggcggcgt 27300
ccacgatgga cccgcaggag cggctgttcc tgcagtgcgc ccacaccacg ctggaggacg 27360
ccggctactc gcgcggcgcc ctgcgcgccg ccgcccgcgc ccgggtggcg gaggacgccg 27420
gcgacatcgg ggtgttcgcc ggcgcgatgt actccgagta ccagctctac ggcgccgagt 27480
acagcgtgcg cggtgagccg gtcgtggtgc cggggagcct ggcgtccatc gccaaccgcg 27540
tctcgtactt cctggacgcg agcggcccca gcgtcaccgt cgacaccatg tgcgcctcgg 27600
cgctgtccgc gatccacctc gcctgcgccg ccctccagcg aggggagtgc ggtgtcgccc 27660
tggccggcgg ggtcaacctg tcggtgcacc cgggcaagta cctgatgatc ggggagggcc 27720
agttcgcctc cagcgacggc cgctgccgca gcttcggcga gggcggcgac ggctacgtgc 27780
ccggcgaggg cgtcggcgcg gtgctgctgc gcccgctcgc cgacgccgtc gccgacggcg 27840
accgcatcct cggcgtgatc cgcggcaccg ccgtgaacca cggcggccac acgcacggat 27900
tcaccgtgcc gaacccgctc gcgcaggcgg cggtcatccg cagcgcctgg cgccgggccg 27960
gagtggaccc ccgggacatc ggctgcatcg aggcgcacgg taccggcacc tcgctgggcg 28020
acccgatcga aatcgccggg ctgaacgcgg ccttcgccga gttcaccgac gcacggaact 28080
tctgcgccat cggctcggcg aagtcgaaca tcggccacct ggagtccgcg gcgggtatcg 28140
cgggcctcgc caagctgctg ctgcagatgc ggcacggcac gctcgtgccc tccctgcacg 28200
ccgaacgcgt caacccggac atcgacttcg ccgacagccc cttcgtcctg cagcgcgaag 28260
ccgcgccctg gccgaggacc ggcacccgcc cgcgcctcgg cggcctctcc tcgttcggcg 28320
cgggcggctc caacgcccac gtcgtggtcg aggactacgt cgaggagcac gccgggaagg 28380
acctcgcgcc cgaggcgcac cgtggcgaaa ccgtcgtcgt ggtgctgtcc gccttcgacg 28440
aggagcgcct gcgcgagtcg gccgggcggc tgcgcgacgc gctgcggaag gagcggtgga 28500
gcagcgcgga cctgcccgac atcgcctaca cgctgcaggt cggccgcgag gcgatgaccg 28560
cacggttcgc cgtggccgtc agcacgcttc ccgccctggt cgacgcgctg gacgcctgcg 28620
cgctcggcag cgggctgccc gcgggcgcgt atttcaaccc cggcggcgac cggggcggcg 28680

cggtcaagga cttcctcacc gacgaggact tccaggagac ggccgtgcgc tgggcacggc 28740
gcggaaagcc ggcgccgctg gccgaggcct ggaccagcgg cctggccgtc gactgggccc 28800
gcctccacac cgagggaccg aagccgcgca aggtcgcact gcccggctac ccgttcgccc 28860
gggagcgcta ctggtacacc gacggacttc cggaactcca ggaaatcccc gccacgttcg 28920
ggaacgccgc acggcagccc gccgccccgc cccctgccgt ggaggccgcg cctgcgacga 28980
cgtccgccgt gcccgccccg cccgcgcggc cggccaactc ctacgagctt cccgcgggcg 29040
acctcaccct gcaccccgtc tgggagcctg tccggctgct gcgcggcagc ccttacccgt 29100
ccgcggcctc ccgtgtggtg gcgatcggcc tcgcaccgga cgcgctcgcg gagctgaccg 29160
cccgccgccc gcagaccgtg gtgctggaca ccgccgcgtc atccgccgaa gaggtgcgtg 29220
acgaactcgc cgtcctgggc gacttcgacc acgtcgtcat gcggttcccg accgcagccg 29280
ccgcccacgg cgccgaggcg cagatcagca cgcagcgcgc ggcgatccgg agcatgttcc 29340
gggtcctcaa ggcactggcc ctcacccggg acgagcagcg gctcggactc accctcctga 29400
ccagcggcgc gttcgacgca ggcggctcgg ggaccgccga cccggcgcag gcgagcctgc 29460
acggtctgct cggcggcctg gccaaggagc agccgcactg gcgcatccgc gcggtcgacc 29520
tggccgacgg cgaaccgttc gtcgccgacg aggtcttcgc cctgcccgcc gaccgccgcg 29580
cgcacccgct cgtccgccgc ggcggccagt ggctgcgccg tcagctcctg ccggtggacg 29640
ccaccgagcc gcccgcggag cccgtgctgc gccgcgacgg cgtctatgtg ctcatcggcg 29700
gcgcgggcga cctcggcgtg ctgctcagcg agtacctcgt acggcaacac gacgcacacg 29760
tcgtatgggt cggccgccgc gccgaggacg aggacatccg ggccagggcg gaccgggccg 29820
cagcgggcgg gcggaccccc gtctacctgt ccgccgacgc ctccgacccc gacgcgctcg 29880
cccgcatgcg ggacgaggtc gtccgccgct acggccgcat cgacggcgtg gtgcacctgg 29940
cgatggtgtt cagtcacacg ccgctcgccc ggatgaccga gcgcgaactg gaggccaccc 30000
tcgcggccaa ggtcgacccg tgcgcgcact tcgccgacgt cttcgccggg cacggcctgg 30060
acttcgtcct gctgatctcc tcgctggtga gcttcatccg caactcccac caggcgcact 30120
actcggcggc ctgcgccttc gaggacgcgc acgccgccgc cctgcgcgag gcgctggact 30180
gccgggtcaa ggtcgtcaac tggggctact ggggcaacgt ccccgacgag ctcctgcgcg 30240
acgtgacgtc catgggactg gccccgatcg ccccggccac ggcgatgggc gcactggagc 30300
gcctcctggc cggcccgctc caccagatcg gcttcatgcg cctcggccgc ccgctgcccg 30360
tcgaaggggt gctcaccgcg gagacgctga ccccgcagac gcacggtgcg gcggcccgcg 30420
acggcgccgc ggccctcgct ctgcccaccg gcctggccgc gtaccacgag agcccggtcc 30480

cgggcgagat cgacgcgttc ctgctccgcc gcctcgccgc cgagctgcgc cgagcgggtc 30540

tggaggagcc gcgccacggc ctggccgagt ggaaggagcg gcagggcgtc gacgcacggt 30600

tcgacggctg gctctcggcc accctgcacg cgctcgccga gcacgcgatg atcgacgacc 30660

ggggccgctg gaccaccagc agtccggccg ccacggacgc cgacgcctgc cgcgccgact 30720

gggccgcgca gacaccccgg tgggccgccg ccaaccccga tctgcgcgca ccgctgaacc 30780

tgctggaccg gaccctgccc gcgctccccg acgtcctgtg cggccgggtg cgcgccaccg 30840

acgtgctctt cccccagggg aagttctccc tggtcgaagg cgtctaccgc gacaaccgcg 30900

tggccgcgca cttcaacgcc gtcctcgccg aacacgtggc ggccttcctg cgcgcacgcc 30960

gggacgccga tcccggcgcc cgcctgcgcg tgctggagat cggcgcaggc accggcggta 31020

ccaccggccc cgtgctcgac cgcctcgccc acgaagggct ggacctggcc gagtactgct 31080

tcaccgacct gtcccaggca ttcctgcaga acgcccagga caccttcggg ccgggccgcg 31140

accacctcac ctaccgcatc ttcgacgcgg ccaggccccc gcacacccaa gggctcgaca 31200

ccggcgcctt cgacgtcgtc atcgcggcca acgtgctgca cgccaccgac accatccgcc 31260

cggccctgcg gcacgccaag gcgctcctgc gcggcaacgg cctgctggct ctcaacgaga 31320

tcagcggctt ctacctcgtc aaccacctca ccttcggcct gctcgacggc tggtggctct 31380

acgacgacgc cgaactgcgc gtgcccggca gccccgcgct gtcgccggcg gcctggcagc 31440

tcgtactgga acaggaaggc ttcaccggca tccgccatcc ggcgcgggac gccctggcac 31500

tcgggcagca ggtcgtcgtg gcccacagcg acggtctcgc ccgcagcccg cgcctgctct 31560

ccggaacgcc cgagatgagc agcccgccct cccagccgcc ggcggaaacc gcggctccgg 31620

ccgccgcctc cgcttcggcc cgggccgtca cggacgtggt gctggccgcg ctcgccgacg 31680

cgctgcgcat gcccgccgac cggatcggcc cggaccgggc gttcgccgac tacggcctcg 31740

actccatcgt cggcgtccgg ttcgtccagc gcctcaacga ggagctgggc accgacctgc 31800

cgaccacggt cgtcttcgac taccgcagcg tggcgcagct cgccgcccac atcgccgaga 31860

gccaccggcc gcaaccggcc cccgccgcgg cggcaccggt gcccgcaccg gacgccgccg 31920

gggcaccgaa ccgtcccgaa ggacgcgagc ccatcgccat cgtcgggatc agcggccgct 31980

tcgcgcagtc ggacgacacc gacgccctct ggcagcacct cgccgccggc cgcgacctcg 32040

tgggcccggt cgaacggtgg gacctctccg gctacagcca ggaccaactg tcctgccgcg 32100

cgggcagctt cctcgacggc atcgaccggt tcgacgcacg cttcttccac ctgaccggcc 32160

tcgaagccac ctacaccgac ccccagcagc ggctgttcct ggaacaggcg tggacggcca 32220

tcgaggatgc cggctacgcg ggctccgcgc tggacggccg ccggtgcggc gtctacgccg 32280

gctgcaccgg cggcgactac ccccagtggt tcgaggacgc gccgcccgcc caggcggcat 32340

ggggcaacgc gccctcggtc gtaccggcgc gcatcgccta ccacctgaac ctccagggtc 32400
ccgccctcgc ggtcgacacg gcctgctcca gctcactggt cgccgtccac ctcgcctgcc 32460
agggcctgtg gagcggcgaa accgacatgg ccctcgcagg aggcgtcagc gtccagacca 32520
ccccggacac ctacctggcg gccggccgcg gcgggatgct ctcgcccacc ggcaagtgcc 32580
acaccttcga cgccgccgcc gacggattcg tccccggcga gggcgtgggc gtcgtggtgc 32640
tccgccgcct gtccgacgca ctggccgacg gcgaccacat ccacgccgtg atccgcggct 32700
ccgccgtcaa ccaggacggg gcgaccaacg gcatcaccgc acccagcgcc ctgtcgcagg 32760
aacgcctcat ccgccaggtg cacaccgaat tcggcatcga cccggccgag atcggcatgg 32820
tcgaggcgca cggcaccggc acccagctcg gcgaccccat cgaatgccag gccctggtcg 32880
gcgcgttcgg cacggccggc ggcagcgaca cctgcgcact cggctcgatc aagacgaacc 32940
tcggtcacac cacctccgcc gcgggcgtgg ccggcctgct caaggtcgtg ctctcgctgc 33000
gccacggtca gatcccgccg tccctccacc actacgagac caaccccgcg atccgactca 33060
ccgaaagtcc cttccacgtg aacaccacgc tgcggccgtg gcagcccaac ggccagggca 33120
agcgcgtcgc cgccctgagc gcgttcggct tcagcggcac caacggccat atggtcgtgg 33180
agaacgcccc ggaccgtgac gagcgccaac aggccgccga cgagctgctg ttcgtcctgt 33240
ccgcccagca gcccgaggcg ctgcgccacc gcgccgagga cctcttggcg tacctgcgcc 33300
gcgcacccga cgccgcgctg ggcgacgtca gctacacgct ggcggcaggc cgggaccact 33360
tcacccaccg cgcggccttt gtcgccgccg accgcgacac gctcgcccac cggctggagg 33420
cctggctggc cgacggacgg agcgacaccg tcggccggcg cggcgacacc gcgccggagc 33480
gcgcccgggc ccggtacctg aacggcgagg aggtcgactt cgcgccgctg ttctccggcc 33540
tcgacgtccg tcgcacgccg ctgcccacct acccgttcca gcgcaagagc tactggccga 33600
cggccactgc tccgagccgg cgccaccaag ccccgcaggc cgcgaacggc cctgccgccg 33660
ccccgtcgcc cgagcccgcc cggccggcac ccgcgcagcc ggcaccggac acggacgagg 33720
cgaccgtgcg gtacctggcc ggcgaactcc tgctggccga gctctcccgc gtgctcatga 33780
tggagcccga ggagatcgac ccgcaggcgt ccttctccga ctacggcgtg gactcgatcc 33840
tcaccgtcag gctcgtcgca gcggtgaaca acgccctcgc cgtcgacctg ccgagcaccg 33900
cactgttcga acacagctcg ctcgaccggc tgacggacca cctggtcacc cggtacgggg 33960
cgcagttgcg gtcctccggt gcgctgcgcg ggccggcagc cgaggccgga ggggctccgg 34020
cgcaggacga ccacgggccc gccgccgagg ctccgtccgc tgctcctgct gctcctgtcg 34080
cctccgccgg aactgccgcc gtccccgctc acgcccccgc ggccgctgcg ggcgacccgg 34140

ccgacgacgg cgtcgccgtg gtgggcatcg ccgcccggtt cgcgcagtcg cccgacgccg 34200
ccgccctgtg ggcacacctc gccgcgggcg acgacctggt cggcgaggtc acccgctggg 34260
acatggacga ggagctgggc gcgggcgccc cgcgccagta cggaagcttc gtcgacgaca 34320
tcgagcgctt cgacgcctgg ttcttccgga tgtccggtaa ggaggccacc tacaccgacc 34380
cgcaacagcg catcttcctg gaggagtgct ggcacgccct ggaggatgcg ggctacgccg 34440
gtgaacggct cgacggccgc gggtgcggcg tctacgtcgg cggctcaccc agcgactacc 34500
agcagttgat cggcgacgac gcgccaccgc agacactgtg gggcaacatc tcctcggtca 34560
tcgcgtcgcg gatctcctac ttcctcgacc tgcagggtgc cgcgctggcg gtcgacacgg 34620
cctgctccag ttcgctggtg gccattcacc aggcctgcca ggacctccgc ctgggcaaca 34680
cgtccatggc gctggcgggc ggagtcttcg tccagtccac gccgatcttc taccggtccg 34740
ccgtgcgggc gaacatgctg tccgcccgcg ggcgctgcca caccttcgac gagcgcgccg 34800
acggcttcgt gccgggggag ggcgccggcg tggtcgtgct caagaggctc gccgacgcgc 34860
tgcgcgacgg cgaccaggtc tacggcgtga tccgcggctc cggcatgaac caggacggca 34920
ccaccaacgg gctcaccgcg cccagcgccg gatcgcagga acgcctcctg cgcagcgtcc 34980
acgagcgcgc cggcgtcgac cccgccggca tccagctgat cgaggcgcac gggaccggca 35040
cgccgctggg cgaccccatc gagttcgagg ccctgcgcgc cgcgttcggc gacgcgcccg 35100
aggcaggctg cgccctgggg tccgtcaaga ccagcctcgg gcacacccag ttcgccgcgg 35160
gcgtggccgg cgtcatcaag gtgctgctgg cgctcaggaa cgagcaactg cccccgtcgc 35220
tgcacttccg ccgggccaac ccggcgatca cgctggaggg cagccccttc tacgtcaaca 35280
cggaactgcg cccgtggccc gcacccgccg acggtccgcg ccgcgccggc gtcagctcgt 35340
tcggcgccgc aggcaccaac gcgcacgcgc tgatcgaaca ggcccccgcc gtgcggaccg 35400
ccgggcacgg cccccggcat gcctggctga tcgtcctgtc ggcacaggac gacgccggcc 35460
gccgagccca ggccgagcgc ctgctggacc acgccctcgc ccacgaggac ctggacctgg 35520
gcgacgtggc gtacaccctg gccaccggac gccgccactg cagccaccgc tgggcgggcg 35580
tggccacgga ccgcgagcag ctcgtcgccg ccctgcggac ctggctgtgc gacggccggg 35640
cggagggcgt ggtcaccggt gaggcgcccg acgggcaccg ccgtcaggac cccgccgagg 35700
acgcccgcgc cggccgcctg atggccgagc ccgaccgtca cgacagcctc accgagctgg 35760
ccgggctctt cgcccagggg caagatctgg gcttcgcccc gctcttcggc gacggcggct 35820
tccgtatcgt ctccctcccg gcctatccgt tcgcgggcga gcgctactgg gtcggatcac 35880
gtccggcggc ccccgctgcg accccggcct ccgctccggt acgcgccccg gtccccgttg 35940
cggccccgtc gccgctggaa ggccgccggc tgaccggtga tcccggctcg ccgtccttcg 36000

ccgtcgagct ggccggccgc gagttcttcc tcgacgacca ccgggtgcgc aacgtgccgg 36060
tgctccccgg cgtggcctat ctggagctgg cgtacgcggc ggcccgggcc gagggcgtcg 36120
accccgccca cgcccgcctg cgcaacgtcg tctggtcgcg ccccgcacgg atcaccgggc 36180
cgaccgcggt cgagatcgcg ctgcggccgt gcgaggacga cgccttcacc tacgagatca 36240
ccacggcggc cgacggcgaa cagccggtga tccacgggca aggacgcatc gagcggtgcg 36300
ggacgccgtc acccgcgcgc ctggacatcg ccgcgctgcg cgcccagtgc gaggtgcgca 36360
ctctggaaca cgacgactgc taccggctct tcgaccgcat gggcatcggc tacggcccgg 36420
ccatgcgggg catccggcgg atccacgtcg gcgccgggct cgccgtcgca cgcctgagcc 36480
tgccgcaggc cgcccgggac ggcgccggct gggacctgca cccgtcgatg ctcgacgccg 36540
ccgtacaggc caccttgggc ctgtcactgg ccgaggacac cgacaccgtg gcgccggcac 36600
tgcccttcgt cctggaggag gtgcagctgc tcgcgcccag cccggccggc gggtgggccg 36660
tggtgcgacc cgcagcgggc gacggcggcg gagccgtacg ccgcatcgac atcgaactgt 36720
gcgacgacga cggcgaggtg tgcgtacgcc tgctcgggtt caccgcacgc gtcctggccg 36780
ccggtgacga ccccgccggc ggagagaaca ccgggggcgc gacgctcacc ctcatgcgtg 36840
ccggctggcg cccggccgag cccacccggg cctcgcgccc gctggtgcac cacgaggtgc 36900
tgctcggcgg actcgcaggg accgaccccg cggcggtccg ggacgggctc ggtgtgccct 36960
gcaccgcatt gcccgacgac ggcgatccgg cccggtgttt cacccgccag gccgagacgg 37020
tgctggcccg cctgcagcag ttcgtcccac gcacccgcga cggcgaggtc ctgctgcagg 37080
tggtcgtgcc cgccgacggt gagaaccggg tcctcgcggg cctgggcggc ctgctgcgca 37140
cggcccgcat ggagcacccc aagctgctga cccagctcgt cgaggtggag acgcccgtcg 37200
acgccgcgac gctgtgcgag cgcctgcgcc gggacgcggc gagccccgac gacgtggccg 37260
tgcggtactc cggcgggcag cgccgggtgc cgcagtggac cgccgtcgag gacgccccgc 37320
cggcccgccc ctggaaagcc ggcggcgtct acctcctcac cgggggagtc ggcgggctcg 37380
gcgcacactt cgcccgcgag atcgcccggc aggcgcccgg cgccgccctc gtgctctgcg 37440
ggcgctcgcc ggagggcccg gcccagcgtg aactcctgtg tgagctgggc gacttgggtg 37500
cctccgccgt ctaccgggtg ctggacgtcg cccggcgcga cgccgtgacc gcctgcgtga 37560
acaccgtcgt cgccgagcac ggccgcctgg acggcgtcgt ccacaccgcc ggtgtggtgc 37620
gcgacggcta cctggcccgt aagagcgccg aagagctgcg ggaggtcctc gccgccaagg 37680
tcgccggctt cgtccacctc gacggagcga ccgccgcgct cgacctggac tgcttcatcg 37740
gattctcctc actgtcggcg tacggcaacc agggccaggg cgactacgcg gcggccaacg 37800

ccttcatgga cgcctacgcc ggcctccgcc acgagcgggt ggccaggggc gagcgccgcg 37860
gccgcacact ggtggtcggc tggcccctgt gggccgacgg cggcatgacg gtggacgccg 37920
ccaccgaacg ccgcctgcac gacagcgtcg gcatggtgcc gatccgcgcc ccgcacggtg 37980
tggaggcgct gctacgcgcc tacggcaccg gcgacccgca cgtcctggcc gtcttcggcg 38040
accgcgcccg catcgacgcc accctcctgg ccgcccccggc ggccacgggc gccgcaccgg 38100
cggtgaccgc acccgaccgc gccgccctgc acgcgagggt cctcggccgc gccatcagcc 38160
acgcctgcgc cgtgctgggc gttccggcgg cggagctcga cggtgcggtg gagctgagcg 38220
agtacggctt cgaccccgtc tcgctcaccg ggttcgccgc ccgcctcacc acggagttcg 38280
ggcttccgcc cgtgcccaag cccttctccg aacacctcac cctgggagag gtcgtggacc 38340
acctgctcga cacccacccc catcacttcg ggacggtccc gccggccccc gcgcccgagc 38400
cctccgccgg gcccgaaagc gccgccgcgc ccgtcgcgac ggccggccgg gagcagcagc 38460
acaaggcgct gctgaagaag ctgatcgccc gcgtgtccga cctgctggac gtgcccgccg 38520
agcggatcac cggcacggcc gagatgaccc gctacggctt cgactccctc tcgttcatcg 38580
gcttcgccaa cgacctcaac gccgagttcg ggctctcgct ggcaccgacc ctgttcttcg 38640
agaaccccac cctggacggg gtcgtcgacc acctcctcga ccaccacgcc gaccgcgtcg 38700
ccgccaccgc ggcaccgcag caggaaccgc gcgcggcggc ggcccccgcc gccccagagc 38760
ccgccacagc cgacaccccc gcgtccccgta cggatgcgcc cgggaacgag ccgatcgccg 38820
tcatcggcat cagcggccgc ttcccgatgg ccgacgatct cgacgcgttc tgggagaacc 38880
tcagcgaagg ccgcgactgc acccgtgagg tccccacgga ccgctgggac tggcgcgccc 38940
actacggcga ccccgtcaaa gagcccaaca cgtcgaacgt gacgtccggc ggcttcatgg 39000
acggcgtcgg cgacttcgac ccgcttttct tcgacatctc ccccaaggaa gcggagttga 39060
tggatccgca gcagcggctc ctgctgatgt acgtatggaa ggcgctggag gacgccgggt 39120
actcggcgga ggccctcgcg ggcacgaaca cggccctcat cgccggcacc accagcaccg 39180
gctacagcac cctcgtcacc cggtactcgc cgatgatcga gggatacgac atcaccggcg 39240
cggccccctc catgggcccg aaccggatga gctacttcct tgacctgcac ggtccgagcg 39300
agcccgtcga cacggcctgt tcgagcgcgc tcgtcgccct gcaccgggcc gtccaggcca 39360
tccgcgacgg tcagtcggac ctggccatcg ccggcggcgt caacaccatg gtcagcgtcg 39420
acgggcacat cagcatctcc aaggcgggca tgctcagccc cgaaggccgc tgcaagacct 39480
tctccgaccg cgcggacggt tatgcccgtg gtgagggcgt gggcatgctg gtgctcaaga 39540
gcctgtcggc ggccgagcgc gacggcgacc acatctacgg ggtcatccgc tcgacggccg 39600
agaaccacgg cggccggggc agctccctga ccgcgcccaa ccccaaggcc caggccgccc 39660

tcctgcggga ggcctacggg aaggccggga tcgatcctcg gacggtgggc tacatcgagg 39720
cccacggcac cggcaccaaa ctcggcgacc cggtcgagat caacgggctc aaggccgcgt 39780
tccgggacat gtacgaggag cacggcgcgg tggtcgagga ggcccactgc ggtatcggct 39840
cggtgaagac caatatcggt catctcgaac tggccgcggg cgccgccggc gtgatcaagg 39900
tgttgctcca gatgcggcac cgcaccctgg tcaagagcct gcactgcgac accgtcaacc 39960
cctacatcga cctcgacggc agcccgttcc acctcgtacg cgaacggcag ccctggcccg 40020
ccctgcgcga tgccgaaggc cgtgagctgc cgcgccgggc cggagtcagc tccttcggct 40080
tcggcggcgt caacgcccat gtggttcttg aggagtaccg gccgcgcacc gcacccgagc 40140
cggaccgggc gcccaccgca ccggtccccg tcgtcctgtc ggcgagccac cccgacgtgc 40200
tgtgcgaact cgccgagcgc tgggtggacg cactgcgccg cggcgactac gacgacaccg 40260
acatggcgtc gatcgcctac accacgcaga ccggacgcac gcccatgacc gagcgcctcg 40320
cctgcctggc ccgcacggcc ggcgaactgc gggaggcact ggagtcctgg ctgcgcggcg 40380
agcccgcggc cgacgtcttc cgcggcaagg tcgcgcgcgg cgtcgacctg ccggacgcac 40440
cagccgggtt cggcccgcac gacgaccacg acagcgcggg ccggcacgac tgggcccgcc 40500
tgctccaggc atgggtgaac ggcgcccccct tcgactggga ccgcctccac accgggcgcc 40560
gcccgcgccg gatcgccctg ccgacctacc cgttccgcct ccggcgctac tgggtcgaca 40620
cctcgcgccc cgcgaacggc acacaaacgg aggcactgca cccgctggtg cacacgaaca 40680
cctcggacct gaacgagcac cgctacacct cgcacttcac cggccgcgag ttcttcctcg 40740
ccgaccaccg cgtacgcgcc caggtgatgg agacggtctc cggctggcgg cccggccgcc 40800
ggcccaccgc ctacgacgtc cgcgcggacg ccgtgccggt gctgccggcg gtggcctacc 40860
tggagatggc gcgcgccgcc gcggtccagg cggccggcgg cgacgagcgc gcctggtcac 40920
tgaagttggc ctcctggctg cgcccgctca ccgtcgagaa ggcgaccgac gtgcacacca 40980
cgctgaccac ccgggccggc ggcggactga gctacgaggt gtacgcggtg gacgaggacg 41040
gcgaacgcgt caccttcggc cgcggccagc tgcggcgcgc aacagcggtg cccgccgagc 41100
ggctcgacct cgcggccctg cgcgcgcagt gcgacggccc cgtgctcgac gccgagacct 41160
gctacgcacg cttcaccggc atcggcatgg cctacggccc ggcactgcgc ggcatcgagc 41220
gcctgcacac cggctcgcgg cagtcggtgg cgcggctgaa gctgcccgcc gccgcgtccc 41280
gcgagcgcgg ctgggtactc aacccgggca tgctcgacgc cgccctccaa gccacggtcg 41340
gcctcttcgt cgacgacccc ggcacgccgc gcacggcact gccgttcgcc ctgggcgagc 41400
tggaggtgct gcgggcggtc ccgggcaccg gctgggtcgt ggtccgcttc gccgaggacg 41460

accacgtggg cgccgtgcgc cgcctcgacc tcgacctctg cgacgacgac ggcgaggtgt 41520
gcgtacgcct gcgcggcttc agcgtccgca cgctcggcgg cagcgagccc accggtgaca 41580
gcgagcccac ccggcccgcc gaacaggcac ccgagccgcc gtccgggtcc gacgacgcct 41640
acctgctgga cctgatcgaa gccattggcc gacgcgagat gagcgcggac gaattcaaga 41700
ggagcctggc atgagcacca cccgcatcgc atccctggac gacctgcacc gggctcattc 41760
gcgagggaca ggtgggacag gacgaagcgc tccgcctgat ccgcgactgg aagcaggagc 41820
aggagcagga gcaggagcag gatcagaatc aggagcaggc gcgagcacgg acgcagaccg 41880
cacggccggc tgacgtcgcc gacaccgagg ccctgacgga gcgggtctgc gccgtcgtgg 41940
tggagaaggt ctgcgagctg ctcaaggtca ccacggacga cctggacgtg catgtcgacc 42000
tcagcgaata cgggctcgac tccctcgtca tcactcagct ggtgaacatg gtgaacgacg 42060
ctctgggtct ggaactcgtg cccaccgtgc tgttcgagca cgcgacgatc caggccttcg 42120
gcgcccacct gaccgacgag tacggccctg cgctggccgc ccgcctgggg ctgcggtcgc 42180
ccggcgccgc cacggagccc cctgccgtcg agcccgtcgg tacgcctgtg ccggccgcag 42240
ccgtccccgc acgggccgta cccgtcccgc tgcccgccga ccggcacgac gacccgatcg 42300
cggtggtcgg catgagcggc cggttccccc aggccgagga cctcgacgcc ttctggcgca 42360
acctgcgcga cggccgcgac tgcatcgcgg aagtccccgc cgaccggtgg gactggcgcg 42420
ccctcttcgg cgaccccctt caggaaccgg gccgcaccaa cgtgaagtgg ggcgggttca 42480
tggagggcgt cgccgacttc gatccgctgt tcttcggcat cgctccgaag gacgccgtcc 42540
acatggaccc gcagcagcgc ctgctgatgc tgtacgtgtg gaaggcgctg gaggacgccg 42600
gctacgccgc cgacgccctg gccgggagca gcttcggcct gttcgtcggc accagcgaca 42660
ccggctacgg cctgctctcc gaccgcagca gcggcagggg cgagagcgtc acgcccacgg 42720
gcagcgtccc ctccgtcggc ccgaaccgga tgagctactt cctggacgta cacgggccga 42780
gcgagccgat cgagacggcc tgttcgagtt ccctggtcgc catgcaccgc ggcgtcatct 42840
cgatcgaacg cggcgagtgc gacatggccg tcgtcggcgg tatcaacacc atggtgatcc 42900
ccgatggcca cgtcagcttc agcaagtccg ggatgctcag cgccgagggg cgctgcaaga 42960
ccttctccga ccgcgcggac ggttatgccc gtggtgaggg cgtgggcatg ctggtgctca 43020
agagcctgtc ggcggccgag cgcgacggcg accacgtcta cggcatcatc cgctcgacgg 43080
ccgagaacca cggcggccgc tccaactccc tgaccgcgcc caaccccaag gcccaggccg 43140
ccctgatccg gcgcgcctac agcaccgcgg gcatcgaccc tcggacggtg ggctacatcg 43200
aggcccacgg caccggcacc aagctcggcg acccggtcga gatcaacggg ctcaaggccg 43260
cgttccggga actgtacgag gagcacggcg cggtggtcga cgacgcccac tgcggtatcg 43320

gcacggtgaa gaccaacatc ggccacctcg aactcgcggc gggcgtcgcc ggcgtgatca 43380
aggtgctgct gcagatgcgg caccgcacgc tcgccaagag cctgcactgc gacaccgtca 43440
acccctacat cgacctcgac ggcagcccgt tccacctcgt acgcgagcag cagccctggc 43500
ccgccctgcg cgatgcggag ggccgtgagc tgccgcgccg ggccggagtg agctccttcg 43560
gcttcggcgg cgtcaacgcc catgtggtgc ttgaggagta cgtgccgcgc cccgtaccgc 43620
cggtgagcac accggacccc gtggccgtcg tcctgtcggc cccgagccc gagatgctgc 43680
gcgcccgggc ccggcagctg gccgaccgga tcgactcggg cgggctcggc gaggccgacc 43740
tgccggacct cgcccacacg ctgcaggtgg gccgcgtcgc gatggacgag cgcctcgcct 43800
tcctgacctc ctcgctcgcc gacctgcgcg agcggctggg cgccttcctc gacggcggca 43860
ccgtacaggg cctccacacc ggacgggcac agcgcccggg gccgtggaac gagctcgccg 43920
gagacgacga catcgccctc gccctcgaca gctggatagc caagggcaag ctcggacgcc 43980
tgctcaaact ctgggtcacc ggcttcgacg tggactggcg gcgcctgtac gccggccggc 44040
cgatgcggcg catcccgctg cccgtctacc cgttccagct gaagcgctac tggatcaccg 44100
acgcgaagag cacgacccgg cccccggcac cggtggccgc ggcgccggac gcacaaccgt 44160
cgccctaccg ccgcgacctg accgggcacg agttctacgt gagcgaccac cgcgtggggg 44220
acacgcccgt cctgcccggc accgcctacc tcgagttcgt gcgcgacgcg ctcgtccggg 44280
ccacgtccgc aggcaccgcc acgggcgtgc gcctgcgcga cgtgacctgg ctgcgtcccc 44340
tggaggtgac ggcactgcgc accctcgccg tcgacgtgga cccggccggt gggacattcg 44400
aggtgtacga ccacggctcc ggcgaccgcg tcctgcacgc gaacggcacc gcacacgtcg 44460
accccgcact cctcgccgcc gacgacaccc acgacatcga cgcactgcgc gcgaacctcc 44520
cgttccggcg tgacggcgcc gagtgctacg cgctgttcgc gcgcaggggc atgggatacg 44580
gccccgcgtt ccgggccgtg caggagctgt accacggtgc ggacaccgcc cttgcccgcc 44640
tcctcctccc cgaggcggcg gcatcctcgc tgacgctcaa cccggtcatg ctcgacgccg 44700
ccctgcaggc gaccctggga ctggcgctcg gcgagcacgt cgacgccccg caggggacgg 44760
cacttccgtt caccgtgcgc gaggtacagg tcctggcccc caccccggcc gagggctggg 44820
cgctcgtgcg ccgtgccgcg gacgaccgcc acgacaccgg catacgccgc ctggacatcg 44880
acctctgtga cacgcagggc aacgtctgcg tgcgtctgct gggcttttcc acccgcatga 44940
agccgagccc ggcgccccgc gccgccgagc cgaccaccac gcccgcactg ctgatccagg 45000
cggactggcg cgagagcgcg gcacgggagc accacggcga cgacgtcaag cggcacgtcg 45060
tcctgtgcga actcccggcg gcggacgcca ccgcgctcgg cgcagcgctg ggcggcgcca 45120

cctgcgaaac ctggcaggcc cgcggcgaga ccggcacccg ctacaccgag tacgccgagc 45180
ggttgctgaa gctgctgcgc gacaaggcac cggaggccgc ccggcagccg tgcctgatcc 45240
aggtcgtcac ccccgcgcac gcgccctggc tgggcgggtt gagcggcatg ctccgcacgg 45300
cgcgcatgga gcaccccaag ctgctgacgc agtggatcgc tctggacggt gacggcgccc 45360
tggccccggc cgagctggcc ggacggctgc ggtgcgacgg cgccgacacg gccgaggagg 45420
ccgtgcgcta ccgcggagga cgccggcagg tgtcccagtg gcacgaagtc gcaccggccg 45480
cacccgaacg gccctggcgc gacggcggcg tctacctgct gaccggcggc gccggaggac 45540
tcggcgccct gttcgcgcag gacatcgccc ggcgcgtcga gacgcccgcc ctggtcctgt 45600
gcggtcgcag cccggtcggc ccggcacagc aggaactgct taccgccctg cgcgcgctgg 45660
gtgcccgtgc cgactaccgc gtgctcgatg tcgccgaccg cgccgacgtg acccgggtcg 45720
tgcgcgaggt ccaggccgag tacggcgcgc tgcatggcat cgtgcacgcc gccggagtgc 45780
tgcgcgacgg cttcgtggcc aagaagaccg cggacgacct ccgcgaggtg ttcgcggcca 45840
aggtggccgg gctgtgccac ctcgacgagg cgactgcctc cgtcccgctc gactgcttca 45900
tcggcttctc ctccatggcc gccttcggca acgtcggaca ggccgactac gccgccgcca 45960
acgccttcat ggacggatac gccgcccacc gcgactccct ggtggaccag ggcagccggt 46020
cgggccgcac cctgatggtg aactggccgc tgtgggagaa gggcggcatg ggcgccgacc 46080
cgtcgaccgt ccagctcctg gagtccgtgg gcatgcggcc gatgcgcgca tccgtgggca 46140
tcgacgccct cgaccgcgtc tgggcgaccg gcctgcccag cgccatcgcc ctcgacggcg 46200
accacgcccg gatgcgggag cgcttcctgc cggcgcaccc cgagccggag gcccctgccg 46260
aacccgcgcc ggccgccgcg acgttgccgg ccaccgcacc ggtcgccgag ccggccgagc 46320
cgtcgagcgt gggaaccgtc gtcgcggacc tcatggcgac actgctggag gtcgacgtcg 46380
agaccctgcg gtgggacaag tccctgggtg actacggctt cgactccatc ttcatgatgc 46440
agttcctcgc ccaggcgcag acgcacctcg acgcgtccct caccctcgac gtcatcgccg 46500
actgcgaaac gctgcaggac gtcgtcgacg cgatcaccgg caccgcctct gacaccggca 46560
cggccgcccc aaagcccgct tcggtggctc ccgttgagga ggccccggcg gccgccgcac 46620
cggcaaaggc cccggtacgg cgcgccgcgg ccgcatcgcc gaacgacttc cccgaactgg 46680
tccgcatgaa cggtgtcacc tccggccggc ccgtgttctg ggtccaccac ggcaacggcg 46740
gcgtggagtc ctacgccccg ctggccgcac gctgcccgcg tcccttctac ggcatccagc 46800
cgaagggctg gatcgactcc accgacatcc tcaccggtca gtacgccatg gccgagcact 46860
acgcgtccct catcctcgcc gtacagccgg aaggcccgta cgacatcggc gggttctccc 46920
tgggcggcct gttcgcgtac gagaccgtgc ggcagctcca gctgacgggc gccgacgtgc 46980

gcacgctggt catgctggac acgctggacg ccgaatccac caacaaggcc aacgccctca 47040
tcgtcggcgg gaacttcgac gccgacgtgg tcaccaaggt gagcgacttc cgcgccgtca 47100
acctgatcct cggcaacaac cgcttcgact cgcacggcgg cgccaccccg atcctgcgcc 47160
gcgacgaggt cgacaccacc ctggaaccca aggagttcct cggctccctg atcgacgccg 47220
ccctcgcccg cggcgtcagc aagaccgaga cgcagctgcg ctcccgcgtc cggcaactgt 47280
cccgctactt cgaggccacg cagggcgaga cgtacacggt ggacccgctg ccgcggcgcg 47340
acggactgcg ctgctactac ctgcgcaacc ggggcggcaa gttcttcggc gccttcgagg 47400
agcacatggt gctcttcccg aaccccgagc tccccaccgt ggacggcgtc gcgtactggc 47460
aggagtgggc cgaccagatc gacgacttct tcaccatcga cgtcgacacc tcgatgcatg 47520
ccgaggtcat gacggccccg cagtcgctcg acaagctgat gcgcctctgc gaccggctct 47580
acgccgccga ggacgccgcc gccccggcga cctccgcgca gggaggccgc tgacatgaag 47640
gcagtcgtct ttcccggcca gggcgcccag cggcgcggca tgggacgcga gctgttcgac 47700
gcgtatcccg aactcgccga cgaagcctcc gaagtcctcg gctactccct ccgcacgctg 47760
tgcctggacg atccgcacca gcaactgggc cgcaccgagt acacgcagcc cgcgctgttc 47820
gtggtcgggg cgctcgccca ccggcagtgg cgcgagagca ccggcgacga gccggccttc 47880
ctcgccgggc acagcctggg ggagtactgc gccctgcacg ccgccggcgc cttcgacttc 47940
gcgaccggac tgcgcctcgt ccagcggcgc ggcgcactga tggcgcaggc acggggcggt 48000
ggcatggcgg ccgtggtcgg ggtcgacgcg gcacggctgc gcgaactcct cgacgaaggc 48060
ggcttctgcc gcctgaccgt cgccaacgac aacgcacccc agcagaaggt cgtgtccggc 48120
gacaccgcga ccgtcgacgc gctggtggca tacctcgagg cgcgcgacgt ccgctgcgtg 48180
agactgaacg tgtccggcgc cttccactcg cccctgatgc ggcaggcaca gcaggacttc 48240
gcccgcttcg ccgacggatt cgccctcggg gacccggcga cgcccgtgat cgccaacgcc 48300
acggcgcgcc cgtacgtccc cggccggacc gcgcgcacac tcgtagacca gatcgtgcag 48360
cccgtgcgct ggaccgagag cgtgcaccac ctcctcgacc agggcgtcac cgacttcgtc 48420
gaactgggag gccgggtgct cgtcaggctg atcgaccaga tccgctccgc cccgcggccg 48480
gtcgcccagc acgatgcacc ggctgcccgg cccgacacac cggccgccgc gctcggcagc 48540
cccttgttcc ggcggcgcat gggcgtgcgc cacgcctacg ccgtgggcgg catgtaccgg 48600
ggaatcgcct cggcccagat ggtcgtcagg ctcggccgtc accgcattct cggcttcctg 48660
ggaaccggcg gcctgccgct cccggagatc gagcaggggg tgaaggaggt ccagcacggc 48720
ctggccgacg ggcagcccta cggcgtcaac gtactggccg accacgacga tcccgcggcc 48780

gagcgcgcgc tggtcgactt gctgatgcgc cacggcgtcc ccgtcatcga ggcgtcagcc 48840
ttcatgcaga tgacccccgc gctcgttctc taccgggcac ggggactccg ccgcggtgcc 48900
gacggccgga cggtgtgcga ccaccgcatc gtggccaagg tctcccggcc ggaggtcgcc 48960
gagcagttca tggcacccgc ccccgggccg gtcctggaca ggctccgccg ggagcacgcc 49020
ctcaccgacg aacaggcgga actcgcccgg acggtgccga tgagccacga catcacggtc 49080
gaggcggact ccggagggca cacggacggc ggcgtcgcca cggtcatgat gcccgccatg 49140
ctcaagctcc ggcagcaggc ccaggaccgg tacggctacg acgaaccgat ctgcatgggg 49200
ctcgccggcg gcctcggcac cccgcggcg gtcgcggcgg ccttcatgct gggcgccgac 49260
tacgtactca ccggatccat caaccagtgc acggtggaat ccggcatgag caccgaggtc 49320
aaggacatgc tgcaggacgt cggcatcgcc gacaccgcct acgcgcccgc aggcgacatg 49380
ttcgaattcg gtgccaaggt gcaggtgctc cgcaaaggcg tcttcttccc cacgcgggcc 49440
aacagactgt tctccctcta ctcccactac gacggcctcg acgatattcc gcagaaaacg 49500
cgctccctcc tggagagaac ctacttcgga aagagcatcg aagaggtctg ggacgaagta 49560
cgcgcctatc tccgttcgca ggggcgcgac gccgacatcg accgggccga cgccgagccc 49620
aaacagaaga tggcgctggt attccgctgg tacttcttcc acaccacgcg cctcgccatg 49680
gacggcgacg gctccggaaa agtgaactac caggtccaga ccggtccggc gctgggtgcc 49740
ttcaaccagt gggtcgaagg caccgaactc gcctcatggc ggcaccgcca cgtagaccgg 49800
atcggcctca tgctgctcga cggtgccgcc gaacacatcg ccaccgcatg ccggcactgg 49860
cgcgacaccc tcggggtgcc cagtgcgtga cggacatccc accgcagtcg aggagaacgt 49920
gatgaccgtt ccgtccacgg gcaccgaagt ccggctcgcc acccgccccg aggggtggcc 49980
gaccactgag aacttctcgg tcgtgcaggc ggaaccgccc gcggtcagga ccggccaggt 50040
gctgatccgc aacctggtga tgagcgtcga cccgtacatg cgcgggcgga tgaacaaaac 50100
caggtcctac gttccgccgt tcgccgtcgg caaggcgctc gacgggggcg ccgtcggcga 50160
ggtcgtcgtc tcgaagtcat cgcaactcgc cgtcggtgac ctggtcctgc acggcctcgg 50220
ctggcgggag tacgccgtcg tgggcgctgc cggcgcggtc aggatcgacc cggcgctcgc 50280
gccgcccggc gcgtatctcg gagtgctcgg catgccgggg cacgccgcct acacggggtt 50340
gctcaaggcc gccgaattcc ggcccggcga caccgtgttc gtctccgggg ccgcgggcgc 50400
ggtgggctcc ctcgtcggtc agatcgcccg gctctgcggc gcggaacgcg tgatcggatc 50460
ggcgggcagc gccgagaaag tcgcctatct gaccggggag ctcggcttcg acgcggcatt 50520
cgactacaag gacgggccgg ttctcgaaca gctggcgaag gccgcgccga cgggcatcga 50580
cgtgtacttc gacaacgtgg gcggcgacca cctggacgcc gccctggtcc tggccaggat 50640

gggcgcgcgg ttcgccctct gcggcaacat ctcgcaggcc aacgagaagg acccgccggc 50700
cggcccacgg aacctgacgc aggccatcgc caagggcatc accctgcgcg gcgtcctcgt 50760
cggaggccac gccgacctcc cggacgagtt caccgcccgc atgggtggct ggctggcaga 50820
cgggagaatc tcctaccggg agaccgtcgt caggggactg gagaacgcac ccgccgcctt 50880
catcgacatg ctgcgcggcg ccaacaccgg caaaatgctc gtgagaatcg ccgaatgaaa 50940
acaccacgcc ctgcggaaga aaagggtgaa ctccagggag tttcctgtct ccacggaatt 51000
tcctgtggaa ttgttcccca ttttcttccc ggccggtatg gtgatctcca agcgcagacg 51060
ccacacagat ggcgggcttc acgcctcgcg cagagaattc cccacctgcc ccattttccc 51120
tgggcattca gcctgcggtg agtagacgct tccggcgttc cggccgaatc cacgcgccca 51180
ccgtgcacgt tccacccctt tccggttcac cagacagaaa acggaggact tccatgtcca 51240
gcacgtccac gaccgccccc gtctccgtcc ccgtctccgc ccccgtcccc gaagaggtcg 51300
gacacctcta cgaccgcctc accgcactgg acaccgaagc ggccggcggc agcctccacc 51360
tcggctactg ggacgtcgac gacaacgaca ccccgctcgt ggaagcggcc gaccggctca 51420
ccgacacgat gaccgaccgc ctgcggatcg accagggaca gcgggtcctc gacgtcggct 51480
gcggagtcgg ccagccggcc atgcggatcg cccggcgcac cggcgcccat gtcacgggca 51540
tcgcgatcag caaggaccag atcgcccgcg ccaccgccct cgccgagggc gccggcctga 51600
gcgaccgcgt ggagttccgg cacgccgacg ccatggaact gcccttcccc gacgactcct 51660
tcgacgccgc catcgccatc gagtcgatct tccacatgcc cgaccgcgga cgggtcctcg 51720
ccgagatccg ccgcgtactg cgcccccggcg gccgcctggt cctcaccgac ttcttcgagc 51780
gcggccccgt ccccgccgag aagcagcccg cggtggaccg gctcctccgc gacttcatca 51840
tgacgctggc ccggcccgag gactacgtgc ccatgctgcg cgacgcaggc ctgcgcttcg 51900
tcgagctcct cgacatcacc gagcagagcg tgcgtcagac cttcgagcag atgagccagg 51960
gctcccagga gatgcagacc gtcttcgacg acgaggcaga ggaaaagttc agccccgcct 52020
ccatgatcga cgtcgacgaa ttcggctccg ttctgctgac cgcccaaaag cccctctgac 52080
cggggacgtt cgaacgaggt g 52101

<210> 2
<211> 1217
<212> PRT
<213> Streptomyces platensis subsp. rosaceus

<400> 2

Met Pro Ser Ser Leu Thr Pro Asn Gln Pro Pro Leu Thr Pro Ala Ser
1 5 10 15

Glu Asp Gly Pro His Gly Thr Pro Leu Gln Leu Ser Val Leu Gly Pro
20 25 30

Met Ser Ala Arg His Asp Gly Arg Asp Leu Pro Leu Gly Pro Pro Arg
35 40 45

Arg Arg Ala Leu Leu Ala Leu Leu Leu Ile Arg Leu Gly Arg Val Val
50 55 60

Pro Thr Glu Leu Leu Ile Glu Glu Leu Trp Gly Glu Glu Pro Pro Arg
65 70 75 80

Gln Ala Val Ala Thr Leu Gln Ser His Val Ser His Leu Arg Arg Ala
85 90 95

Leu Gln Pro Ala Ser Gly Leu Asp Arg Pro Thr Val Leu Arg His Arg
100 105 110

Ala Pro Gly Tyr Val Leu Glu Leu Ala Pro Glu Gln Leu Asp Ala Cys
115 120 125

Arg Phe Glu Arg Leu Val Ala Glu Gly Arg Arg Leu Leu Glu Gln Arg
130 135 140

Asp Pro Leu Ala Ala Arg Asp Arg Phe Ala Ala Ala Leu Thr Leu Trp
145 150 155 160

Arg Gly Ser Pro Tyr Ala Glu Phe Asp Gly His Pro Pro Leu Ser Asp
165 170 175

Glu Ser Ala Arg Leu Glu His Val Arg Leu Thr Ala Val Glu Ser Cys
180 185 190

Ala Gln Ala Arg Leu Ala Leu Gly Glu Ala Gln Glu Val Ala Ala Gly
195 200 205

Leu Asp Arg Glu Val Arg Arg His Pro Ala Arg Glu Arg Leu Val Gly
210 215 220

His Leu Met Thr Ala Leu Ser Gln Leu Gly Arg Gln Ala Glu Ala Leu
225 230 235 240

Glu Val Tyr Glu Arg Thr Arg Val His Leu Asn Glu Glu Phe Gly Val
245 250 255

Gly Thr Ala Ala Glu Leu Arg Arg Val Arg Thr Ala Ile Leu Arg Gln
260 265 270

Glu Pro Gly Ala Gly Gly Pro Pro Ala Gly Pro Arg Pro Gly Arg Pro
275 280 285

Glu Pro Ala Gly Pro Ala Leu Gly Ala Pro Val Ile Gly Val Ala Thr
290 295 300

Ala His Glu Gly Asp Gly Ala Arg Gly Pro Asp Ala His Pro Pro Arg
305 310 315 320

Ala Ala Gln Glu Pro Glu Arg Pro Thr Ala Ala Glu Ala Ser Gly Gly
325 330 335

Ile Gly Arg Ala Ala Ala Ala Pro Asp Glu Pro Phe Ala Ala Asp Cys
340 345 350

Gly Asp Asp Ala Pro Gln Asp Gly Thr Leu Ser Ala Gly Ser Gly Gly
355 360 365

Ala Gly Ser Ala Ala Ala Asp Gly Ala Gln Ala Asp Ala Pro Thr Ala
370 375 380

Thr Pro Gly Pro Ala Arg Arg Pro Val Thr Ile Thr Ala Thr Ser Arg
385 390 395 400

Ser Thr Thr Ala Asp Ser Ala Glu Ser Ala Arg Pro Thr Gln Asp Ala
405 410 415

Lys Ala Gln Glu Cys Asp Thr Ser Thr Glu Ala Ser Gly Pro Val Cys
420 425 430

Gly Gly Pro Gly Ala Gln Ser Pro Phe Thr Gly Arg Ser Glu Glu Leu
435 440 445

Gln Arg Leu Thr Ala Ala Ala Ser Ser Ala Leu Ala Gly His Gly His
450 455 460

Val Ala Gly Val Leu Gly Pro Ala Gly Val Gly Lys Thr Arg Leu Leu
465 470 475 480

Leu Glu Leu Val Pro Gln Leu Glu Ala Ala Arg Ala Gly Glu Asp Gly
485 490 495

Thr Gly Gln Glu Arg Ala Gly Leu Glu Val Ile Trp Ser His Cys Phe
500 505 510

Leu Gly Glu Gly Val Pro Pro Tyr Trp Val Trp Thr Gln Ile Leu Arg
515 520 525

Arg Leu Ser Thr Thr Arg Pro Asp Ala Phe Arg Glu Ala Ala Lys Pro
530 535 540

Phe Gly Thr Leu Leu Ala Pro Leu Met Pro Glu Arg Ala Ala Arg Pro
545 550 555 560

Gly Gly Leu Ala Ser Glu Ser Asp Trp Gly Gln Ala Arg Phe Leu Thr
565 570 575

His Asp Ala Val Cys Glu Val Leu Leu Ala Leu Ala Ala Gln Arg Pro
580 585 590

Leu Val Leu Leu Met Glu Asp Leu His Trp Ser Asp Pro Ala Ser Leu
595 600 605

Asp Leu Leu Arg Leu Leu Ser Thr Arg Ser Gln Gly His Pro Leu Gly
610 615 620

Ile Val Leu Thr Ala Arg Glu His Glu Ile Glu Ser Asp Ala Thr Leu
625 630 635 640

Arg Arg Met Leu Ser Glu Val Leu Arg Gly Pro Arg Thr Glu Thr Leu
645 650 655

Arg Leu Gly Gly Leu Pro Arg Arg Ala Val Ala Ala Leu Val Val Ala

660 665 670

Gln Val Gly Pro Gly Val Asp Ala Arg Val Val Glu Val Leu His Arg
675 680 685

Arg Ser Glu Gly Asn Pro Tyr Phe Val Met Gln Leu Leu Ser Leu Leu
690 695 700

Gly Asp Ala Arg Ser Leu Arg Arg Pro Asp Ala Val Asp Val Leu Leu
705 710 715 720

Thr Arg Val Pro Thr Gly Val Arg Glu Ala Leu His Gln Arg Phe Ala
725 730 735

Ala Leu Pro Glu Thr Val Leu Arg Val Leu Arg Leu Cys Ala Val Ile
740 745 750

Gly Thr Glu Val Asp Thr Asp Leu Leu Glu Arg Thr Ala Thr Glu Asp
755 760 765

Glu Pro Val Thr Ala Ala Leu Glu Leu Ala Ile Arg Ala Gly Leu Leu
770 775 780

Gly Glu Asp Arg His His Pro Glu Arg Leu His Phe Thr His Ala Leu
785 790 795 800

Val Gln Glu Thr Leu Ile Asp Glu Leu Pro Arg Glu Asp Arg Gln Arg
805 810 815

Leu His Ala Arg Val Ala Glu Gly Ile Ser Thr Arg Thr Leu Gly Gln
820 825 830

Val Ala Asp Glu Glu Ile Glu Arg Ile Ala His His Ala Trp His Ala
835 840 845

Lys Ser Ala Leu Pro Thr Glu Glu Thr Leu Pro Leu Leu Leu Arg Ala
850 855 860

Ala Glu Gln Ala Glu Gln Gln Leu Ala Tyr Glu Gln Val Glu Thr Trp
865 870 875 880

Leu Arg Arg Ala Val His Leu Val Gly Leu Leu Pro Pro Gly Asp Pro
885 890 895

Ser Ala Val Ser Leu Asn Gln Arg Leu His Ile Gln Leu Gly Gln Val
900 905 910

Leu Ala Ile Thr Arg Gly Tyr Gly His Ala Glu Ala Gln Thr Ala Leu
915 920 925

Ala Arg Gly Arg Ala Leu Ser Ala Ala Thr His Ser Pro Glu Asp Pro
930 935 940

Ser Val Leu Trp Ala Leu Cys Ala Ala Tyr Ile Val Thr Gly Arg Tyr
945 950 955 960

Asp Ala Ser Arg Gln Phe Ser Gly Leu Leu Arg Asn Leu Ala Asp Arg
965 970 975

Thr Gly His Pro Val Ala Val Leu Gly Ala Ala Tyr Gly Glu Gly Ile
980 985 990

Val Leu His Ile Arg Gly Gln Leu Arg Pro Ala Leu Thr Glu Leu Glu
995 1000 1005

His Gly Val Ala Met Ala Asp Glu Tyr Ala Gly Glu Gly His Ser
1010 1015 1020

Leu Ala Arg Thr Phe Gln His Asp Pro Arg Val Ser Cys Arg Ser
1025 1030 1035

Tyr Asp Thr Phe Thr His Trp Leu Met Gly Asp Arg Lys Thr Ala
1040 1045 1050

Thr Ala Arg Arg Arg Glu Leu Leu Arg Leu Thr Glu Tyr Asp Ser
1055 1060 1065

Arg Pro Ser Asp Arg Ser Phe Ala Leu Tyr Val Asp Ala Val Val
1070 1075 1080

Ala Ala Trp Glu Gly Asp Ala Arg Thr Ala Arg Ser Ser Gly Ala
1085 1090 1095

Glu Gly Val Arg Leu Ala Asp Glu His Gly Leu Leu Tyr Trp Lys
1100 1105 1110

Ala Met Leu Gly Val Leu Glu Gly Trp Gly Arg Thr His Ser Gly
1115 1120 1125

Gln Glu Asp Gly Leu Thr Leu Met His Ser Ser Leu Ala Glu Leu
1130 1135 1140

Arg Asn Ser Arg Thr His Leu Arg Arg Pro Leu His Leu Gly Leu
1145 1150 1155

Leu Gly Gln Ala Gln His Arg Thr Gly Arg Thr Glu Asp Ala Lys
1160 1165 1170

Thr Thr Phe His Ala Leu Leu Ala Ala Val Gly Gln Ser Asp Glu
1175 1180 1185

Pro Val Tyr Leu His Pro Glu Leu Pro Ala Thr Arg Leu Leu His
1190 1195 1200

Asp Leu Leu Gly Arg Gly Ala Ala Glu Ala Val Ala Ala Gly
1205 1210 1215

<210> 3
<211> 3654
<212> DNA
<213> Streptomyces platensis subsp. rosaceus

<400> 3

atgatctgga tgagctggcg ccagttccgc tggcaggccc tggccggtgc cgtcgccctg 60
gtgccgttgg tggcctactt gatcgtcacg agcctggaca tccggcgcgc ccacgaccgc 120
tatcaggcgc agtgcgcgtc catcggcaac tgcgccgagg cgatgctcca gttccagaac 180
gacttccgca cccgcctgct gctgctcgcc atcctgctgg ccgcgatccc cggcatcctc 240
ggggtgttct ggggcgcgcc gctggtggcc cgcgagctcg agaccggcac gcaccgcctg 300

gtctggaacc agagcgtcac ccggcgccgg tggctggcgg tcaaggtgct gttcgtcggt 360
gtcgccgcga tggccgtggc cacgctcgtc agcacgctgc tgacctgggc gagcagcccg 420
gtcgacgcgg tgtcgcagga ccggttcggc gcgctggtgt tcgacgcccg caacatcgtg 480
ccggtcgcgt acgccgcctt cgccctcgtc ctcggcacgg tgatcggcct gctcgtgcgc 540
cgcaccatcc cggccatggc gctcaccatg ctcgtcttcg ccgtcgtgca gttcaccgtg 600
ccggcgctgg cccggccgca cctgatggcg ccggagaccc agacccggca gatgacgttg 660
caggagttcg gcgaggtgcg cggcttcggc gacgagccca cggtcaacgg gctgagcatc 720
cggggcgcgt gggtgaccag caccagcccg ctgctcaccg ccgacgggac ccggctcgac 780
aaggccacgt accgcaaatg cgtgaccgac cccccggccg tctcgggcgg agctcccggc 840
gtcggcggca ccgtcgcctg cctggccgac ctcgatctgc acgtcgaggt ggcctaccag 900
cccaacgacc ggtactggac cttccagtgg atcgagtcgg ccctctacct ggcgctcggt 960
ggactgctcc tcgccgtggg cctgtggcgc atccgccgcc acgtcatctg aggccgggcg 1020
gcagcggctc cggacgagcc cttcgcagca gactgcggtg atgacgctcc ccaggacggc 1080
acgctgtccg cgggatcggg aggggccgga agcgctgccg cggacggggc gcaggcggac 1140
gcgcccaccg ccacaccggg acccgcgcgc cggccggtga cgatcaccgc gacatcgcgg 1200
tcgaccaccg cggattcagc ggagagcgcg cgtccgacgc aggatgcgaa agcccaggag 1260
tgcgacacga gcaccgaggc ctcagggccg gtgtgcggcg gtccgggcgc ccagtcgccg 1320
ttcaccggcc gcagcgagga gttacagcgc ctgacagccg cggcgtcgag cgcgctcgcc 1380
ggtcacgggc acgttgcggg cgtcctcggc cccgcgggcg tcggcaagac ccgtctgctc 1440
ctcgaactcg tcccccagct ggaagccgcc cgcgccggcg aggatggcac cggtcaggag 1500
cgcgccggcc tggaggtgat ctggagccac tgcttcctgg gcgagggcgt gccgccctac 1560
tgggtgtgga cccagatcct ccggcgactg tccacgaccc gcccggacgc cttccgcgag 1620
gcggcgaaac cgttcggcac cctgctcgcc ccgctgatgc ccgagcgcgc ggcccggccc 1680
ggcggactcg cctccgaatc cgactggggc caagcccggt tcctcaccca cgacgcggtc 1740
tgcgaagtcc tgctcgccct cgccgcccag cggccactcg tcctgctcat ggaggacctg 1800
cattggtccg accccgcctc cctggacctc ctcagactgc tgagcacccg cagccagggc 1860
cacccgctcg gcatcgtcct gaccgcccgt gagcacgaga tcgagtccga tgcgacgctg 1920
cgccgcatgc tctccgaggt gctgcgcggc cccaggaccg agaccctgcg gctcggcggc 1980
ctgccccgcc gcgcggtcgc cgccctcgtc gtcgcccagg tcggacccgg cgtcgacgcg 2040
agagtcgtcg aggtgctgca ccggcgcagc gagggcaacc cgtacttcgt catgcagctc 2100

ctctcccttc tgggtgacgc tcgcagcctg cggcggcccg acgcggtgga cgtgctcctg 2160

acgcgcgtcc cgacgggcgt ccgcgaggcc ctgcaccagc ggttcgccgc actccccgag 2220

acggtgctgc gcgtgctgag gctctgcgcc gtcatcggca ccgaggtcga caccgatctg 2280

ctggaacgca ccgccaccga agacgaaccg gtcaccgcgg cactggagtt ggcgatccgg 2340

gccgggctgc tcggcgagga tcgccaccac ccggaacggc tccacttcac ccacgccctg 2400

gtccaggaga cgctcatcga cgagctcccc cgcgaggacc ggcagcggct gcacgcgaga 2460

gtcgccgagg ggatatcgac ccgcacgctc ggtcaggtgg cggacgagga gatcgagcgg 2520

atcgcccacc acgcctggca cgccaagagc gcactgccga ctgaagaaac gcttcctctg 2580

ttgctgcgcg ccgccgagca ggccgagcag caactcgcct acgaacaggt ggagacctgg 2640

ctgcgccgcg cggtgcacct ggtcggcctg ctgccgcccg gcgacccctc ggccgtgtcc 2700

ctgaaccagc ggctgcacat tcaactcggc caggtgctgg ccatcacccg gggctacggc 2760

cacgccgagg cccagacggc actcgcccgc ggacgggccc tgagcgcggc cacccactcc 2820

cccgaagacc cgtcggtgct ctgggccctg tgcgcggcgt acatcgtcac cggccgctac 2880

gacgcctcac gccagttctc gggcctgctc cggaacctcg ccgaccggac cggccacccg 2940

gtggcggtgc tcggcgcggc ctacggcgag ggcatcgtcc tccacatccg cggccagttg 3000

cggccggcac tcaccgaact ggagcacggc gtcgccatgg cggacgagta cgccggcgag 3060

ggccactccc tggcccgcac gttccagcac gacccacgcg tctcctgccg ctcctacgac 3120

accttcaccc actggctcat gggcgaccgc aagaccgcca cggcacgccg ccgcgaatta 3180

ctgcgcctca ccgagtacga cagcaggccc tccgaccgct ccttcgctct ctacgtggac 3240

gcggtcgtgg cggcctggga aggcgacgcc cgcacggccc gctcctccgg cgccgaggga 3300

gtccgcctgg cggacgaaca cggactgctc tactggaagg ccatgctcgg cgtgctggag 3360

ggctggggcc gcacccactc cggacaggag gacggcctca ccctgatgca ctcctccctc 3420

gccgaactcc gcaactccag aacccacttg cgccgccccc tccacctggg cctcctcggc 3480

caggcccagc accggaccgg acggacggag gacgcgaaga ccaccttcca cgccctcctc 3540

gcagccgtcg gccagagcga cgaacccgtc taccttcatc cggaactccc cgccacccgc 3600

ctcctccacg acctcctggg ccgtggggcc gcggaggcgg tggcggccgg gtga 3654

<210> 4
<211> 529
<212> PRT
<213> Streptomyces platensis subsp. rosaceus

<400> 4

Val Leu Phe Pro Gly Gln Gly Ser Gln Ala Arg Gly Met Gly Ala Gly

```
1               5                   10                  15
Leu Phe Asp Arg Tyr Pro Glu Leu Thr Ala Leu Ala Ser Asp Ile Leu
            20                  25                  30
Gly Tyr Asp Leu Pro Arg Leu Cys Leu Glu Asp Pro Asp Gly Arg Leu
        35                  40                  45
Asp Asp Thr Arg Cys Thr Gln Pro Ala Leu Tyr Val Val Asn Ala Leu
    50                  55                  60
Ser Tyr Gln Asp Ser Leu Glu Arg Gly Glu Pro Glu Gly Gly Tyr Leu
65                  70                  75                  80
Leu Gly His Ser Leu Gly Glu Tyr Asn Ala Leu His Ala Ala Gly Ala
                85                  90                  95
Phe Asp Phe Glu Thr Gly Leu Lys Leu Val Leu Lys Arg Gly Glu Leu
            100                 105                 110
Met Ala Arg Ala Pro Asp Gly Ala Met Leu Ala Val Val Gly Pro Asp
        115                 120                 125
Ala Gly Glu Val Arg Ala Phe Leu Ser Glu Glu Gly Leu Ser Arg Leu
    130                 135                 140
Asp Val Ala Asn Ile Asn Thr Pro Val Gln Thr Val Leu Ser Gly Ala
145                 150                 155                 160
Arg Asp Glu Ile Glu Arg Ala His Lys Thr Leu Asp Ala His Gly Thr
                165                 170                 175
Arg Val Ala Arg Leu Lys Val Ser Ala Ala Phe His Ser Arg Phe Met
            180                 185                 190
Ala Ala Ala Arg Asp Glu Phe Ala Ala Phe Leu Lys Gly Phe Arg Phe
        195                 200                 205
Ala Pro Leu Arg Ala Thr Val Ile Ala Asn Leu Thr Ala Arg Pro Tyr
    210                 215                 220
Thr Asp Gln Asp Val Ala Ala Thr Leu Ser Glu Gln Ile Cys Gly Ser
225                 230                 235                 240
Val Gln Trp Leu Asp Ser Val Arg Tyr Leu Leu Glu Arg Thr Thr Ala
                245                 250                 255
Gly His Cys Arg Glu Val Gly Gly Gly Gly Val Leu Thr Arg Met Ile
            260                 265                 270
Arg Gln Ile Asp Ala Ala Pro Ala Arg Gly Ile Pro Gln Pro Lys Pro
        275                 280                 285
Lys Pro Lys Pro Lys Pro Lys Pro Lys Pro Gln Ser Arg Pro Arg Leu
    290                 295                 300
Phe Cys Ile Ala Tyr Ala Gly Gly Asp Glu Arg Ala Tyr Ala Gly Leu
305                 310                 315                 320
Ala Glu His Cys Pro Asp Val Asp Val Val Thr Leu Glu Arg Pro Gly
                325                 330                 335
```

Arg Gly Arg Arg Ala Ser Glu Pro Leu Leu Arg Glu Pro Ala Ala Ile
340 345 350

Val Asp Asp Leu Leu Arg Gln Leu Arg Gly Arg Leu Asp Ala Pro Tyr
355 360 365

Ala Leu Tyr Gly His Ser Leu Gly Ala Arg Leu Ala Phe Leu Leu Cys
370 375 380

Arg Ala Leu Arg Ala Glu Arg Leu Pro Ala Pro Ala His Leu Phe Val
385 390 395 400

Ser Gly Glu Ser Gly Pro Ala Leu Pro Ser Arg Glu Arg His Thr Trp
405 410 415

Glu Leu Pro Ala Asp Ala Phe Trp Asp His Leu Lys Glu Leu Gly Gly
420 425 430

Ile Pro Ala Glu Leu Trp Glu His Pro Asp Leu Met Ala Tyr Tyr Glu
435 440 445

Pro Val Ile Arg Ala Asp Phe Thr Ala Leu Gly Ala Tyr Arg His Glu
450 455 460

Asp Ala Pro Pro Leu Asp Val Pro Val Thr Ala Met Ala Gly Glu Asp
465 470 475 480

Glu Trp Phe Thr Arg Ala Asp Leu Glu Ala Trp Gln Arg Glu Ser Thr
485 490 495

Arg Pro Leu Thr Thr His Arg Phe Pro Gly Asp His Phe Phe Ile Arg
500 505 510

Ala Gln Trp Pro Ala Leu Ala Arg Ile Val Ala Ala Gly Leu Ala Ala
515 520 525

Pro

<210> 5
<211> 1590
<212> DNA
<213> Streptomyces platensis subsp. rosaceus

<400> 5

gtgctcttcc ccggccaggg gtcccaggcc cgtggtatgg gagccggcct cttcgaccgg 60
taccccgaac tgaccgcgtt ggccagcgat attctcggct acgacctccc gcggctgtgc 120
ctggaggacc cggacgggcg gctcgacgac acgcgctgca cccagcccgc gctctacgtc 180
gtcaacgccc tctcctacca agactcgctg gagcgcggcg aacccgaggg cgggtacctg 240
ctgggccaca gcctcgggga gtacaacgcc ctgcacgccg ccggggcctt cgacttcgag 300
accggcctga agctcgtcct caagcggggc gagctcatgg cccgggctcc ggacggggcc 360
atgctcgccg tcgtggggcc ggacgcgggt gaggtgcggg ccttcctctc ggaggagggc 420
ctgtcgcggc tcgacgtcgc caacatcaac acccccgtcc agaccgtgct gtccggggcc 480

cgggacgaga tcgagcgcgc gcacaagacg ctcgacgcgc acgggacccg ggtcgcccgg 540
ctgaaggtct cggccgcctt ccactcgcgg ttcatggccg ctgcccgcga cgagttcgcc 600
gccttcctca aaggcttccg gttcgcgccc ctgcgggcca cggtgatcgc caacctcacc 660
gcacggccgt acacggacca ggacgtcgcg gcgacgctga gcgagcagat ctgcggatcg 720
gtgcagtggc tggacagcgt ccgctacctg ctggagcgca cgaccgccgg ccactgccgt 780
gaggtgggcg ggggaggagt cctgacccgc atgatccggc agatcgacgc ggcccccgcc 840
cgcgggattc cacagccgaa gccgaagccg aagccgaagc cgaagccgaa gccgcagtca 900
cggccacgcc tgttctgcat cgcctacgcc ggaggcgacg agcgcgccta cgcaggactc 960
gccgaacact gcccggatgt cgacgtcgtg acgctggaac gccccggacg cggccggcgc 1020
gcctccgagc cgctgctgcg cgaacccgcc gcgatcgtcg acgatctgct ccggcagctg 1080
cggggccggc tcgacgcccc gtacgcgctc tacggccaca gcctcggagc ccggctggca 1140
ttcctgctct gtcgggcgct gcgcgccgag cggctgcccg caccggccca cctgttcgtc 1200
tccggggaga gcggaccggc cctcccgagc cgggaacgcc acacctggga gctgccggcc 1260
gacgccttct gggaccacct caaggagctc ggcggaatcc cggcggagct gtgggagcac 1320
cccgacctga tggcgtatta cgagccggtc atacgggccg acttcaccgc gctgggcgcc 1380
taccggcacg aggacgctcc gccgctggac gtgccggtca ccgccatggc cggcgaggac 1440
gagtggttca cccgggccga cctggaggcc tggcaacgcg agagcacccg gcccctgacc 1500
acacaccggt tccccggtga tcacttcttc atccgggccc agtggcccgc gctggcccgg 1560
atcgtcgctg ccgggctcgc ggccccctga 1590

<210> 6
<211> 83
<212> PRT
<213> Streptomyces platensis subsp. rosaceus

<400> 6

Met Lys Gln Glu Leu Lys Lys His Met Glu Glu Arg Phe Met Phe Glu
1               5                   10                  15

Phe Asp Ser Asp Ile Thr Glu Asp Thr Asp Leu Phe Lys Ala Gly Ile
            20                  25                  30

Leu Asp Ser Phe Gly Tyr Ile Ser Leu Met Thr His Ile Glu Glu Glu
        35                  40                  45

Tyr Gly Val Pro Leu Gly Asp Glu Ile Leu Gly Asn Val Ala Val Ser
    50                  55                  60

Leu Ser Gly Ile Val Ala Phe Val Asp Ala Ala Arg Leu Arg Ala Ala
65                  70                  75                  80

Gly Ser Arg

<210> 7
<211> 252
<212> DNA
<213> Streptomyces platensis subsp. rosaceus

<400> 7

atgaagcagg aactcaagaa gcacatggaa gagcggttca tgttcgagtt cgactcggac 60

atcaccgagg acaccgacct gttcaaggcg ggcatcctcg actcgttcgg ttatatctcg 120

ctgatgacgc acatcgagga ggagtacggc gtgccgctcg gcgacgagat cctcggcaac 180

gtcgcggtct cgctgtccgg catcgtcgcg ttcgtcgacg ccgcccgcct gcgggccgcc 240

gggagccggt ga 252

<210> 8
<211> 656
<212> PRT
<213> Streptomyces platensis subsp. rosaceus

<400> 8

```
Met Cys Gly Ile Ala Gly Phe Tyr Gly Ser Pro Leu Pro Pro Gln Glu
1               5                   10                  15

Tyr Glu Thr Leu Ile His Gly Met Leu Ala Gln Ile Glu His Arg Gly
            20                  25                  30

Pro Asp Glu Ala Gly Cys Phe Leu Asp Asp Arg Leu Ala Met Gly Thr
        35                  40                  45

Val Arg Leu Ser Ile Ile Asp Leu Ser Thr Gly Ser Gln Pro Val Gly
    50                  55                  60

Ser Ala Asp Gly Arg Tyr Trp Leu Cys Tyr Asn Gly Glu Leu Tyr Asn
65                  70                  75                  80

Tyr Arg Glu Leu Arg Glu Gln Leu Thr Ala Arg Gly Phe Val Phe Arg
                85                  90                  95

Thr Glu Ser Asp Thr Glu Val Val Leu Ala Ala Trp Val Ala Trp Gly
            100                 105                 110

Leu Asp Cys Leu Pro Arg Phe Asn Gly Ala Phe Ala Phe Ala Leu Tyr
        115                 120                 125

Asp Ser Ala Thr Gly Glu Leu His Leu Val Arg Asp Arg Phe Gly Lys
    130                 135                 140

Arg Pro Leu Tyr Val Ala Arg His Arg Gly Ala Trp Leu Phe Ala Ser
145                 150                 155                 160

Glu Met Lys Ala Phe Leu Ala Tyr Pro Asp Phe Arg Phe Ala Phe Asp
                165                 170                 175
```

Glu Ala Gln Leu Ala Ser Val Phe Ala Thr Trp Thr Pro Leu Pro Gly
180 185 190

Gln Ser Gly Tyr Gln Gly Ile Glu Gln Ile Pro Met Gly Glu Tyr Leu
195 200 205

Ser Val Arg Gly Asp Glu Val Arg Arg Gly Arg Trp Ala Thr Leu Asp
210 215 220

Leu Ala Gln Gly Pro Ala Pro Glu Ser Glu Gln Glu Ala Ala Glu Leu
225 230 235 240

Val Arg Ala Asp Leu Glu Ala Ala Val Asp Val Arg Leu Arg Ser Asp
245 250 255

Val Glu Val Gly Val Tyr Ala Ser Gly Gly Leu Asp Ser Ser Ile Ile
260 265 270

Ala His Ile Ala Ala Gln Arg Thr Ser Arg Pro Leu Arg Thr Phe Ser
275 280 285

Ile Glu Phe Glu Asp Ala Glu Phe Asp Glu Ser Ala Glu Gln Ala Glu
290 295 300

Leu Ala Ala His Leu Gly Thr Arg His Ser Thr Val Arg Val Thr Asp
305 310 315 320

Glu Asp Val Ala Asp Ala Phe Pro Glu Ala Val Arg His Ala Glu Val
325 330 335

Pro Val Phe Arg Thr Ala Phe Val Pro Met Tyr Leu Leu Ala Gly His
340 345 350

Val Arg Ser Glu Gly Ile Lys Val Val Leu Ser Gly Glu Gly Ala Asp
355 360 365

Glu Ala Phe Leu Gly Tyr Gly Ile Phe Lys Asp Thr Leu Leu Leu Ser
370 375 380

Thr Trp His Glu Leu Asp Asp Asp Thr Arg Leu Arg Arg Met Ser Gln
385 390 395 400

Leu Tyr Pro Tyr Leu Ser His Phe Ser Gly Glu Asp Gly His Arg Arg
405 410 415

Met Leu Gly Leu Tyr Arg Gln Phe Thr Glu Glu Thr Leu Pro Gly Leu
420 425 430

Phe Ser His Gln Met Arg Phe Gln Asn Gly Arg Phe Ala Ala Arg Leu
435 440 445

Leu Lys Asn Pro Gly Asp Pro Phe Ala Ala Leu Gly Glu Leu Val Ala
450 455 460

Gly Glu Pro Gly Tyr Ala Gln Leu Thr Pro Val Gln Lys Ala Gln Trp
465 470 475 480

Leu Glu Phe Arg Thr Leu Leu Ser Gly Tyr Leu Leu Ser Thr Gln Gly
485 490 495

Glu Arg Met Ala Leu Ala His Gly Val Glu Asn Arg Cys Pro Phe Leu

```
            500                 505                 510

Asp Pro Ala Val Val Arg Arg Ala Ala Ser Val Asn Leu Arg Phe Gly
        515                 520                 525

Asp Pro Tyr Asp Glu Lys Tyr Leu Leu Lys Cys Ala Tyr Ala Asp Val
    530                 535                 540

Leu Pro Glu Arg Ile Val Lys Lys Gly Lys Phe Pro Tyr Arg Ala Pro
545                 550                 555                 560

Asp Ser Ala Ala Phe Val Arg Ser Arg Pro Asp Tyr Arg Glu Leu Leu
                565                 570                 575

Thr Asp Pro Gly Thr Leu Asp Glu Ile Gly Val Leu Asp Ala Arg Phe
            580                 585                 590

Val Lys Arg Phe Thr Asp Arg Val Phe Asp Ser Pro Pro Glu Gln Ile
        595                 600                 605

Gly Thr Lys Glu Asn Gln Ala Phe Val Ser Leu Ala Ser Thr Val Trp
    610                 615                 620

Leu His His Trp Tyr Val Arg Gly Asn Ala Arg Arg Arg Ala Pro Leu
625                 630                 635                 640

Gly Val Pro Leu Tyr Val Val Asp Arg Arg Ser Gly Ala Leu Ser Ala
                645                 650                 655
```

<210> 9
<211> 1971
<212> DNA
<213> Streptomyces platensis subsp. rosaceus

<400> 9

```
atgtgcggca tcgccggctt ctacggaagc cccctgccac cgcaggaata cgagaccctg     60
atccacggca tgctcgccca gatcgagcac cgcggcccgg acgaggcggg ctgcttcctc    120
gacgaccgcc tggccatggg cacggtacga ctgagcatca tcgacctgtc caccggctcg    180
cagccggtcg gcagcgccga cggccgctac tggctctgct acaacggcga gctgtacaac    240
taccgggagc tgcgtgagca gctgaccgcc cgcggcttcg tcttccgcac cgagtccgat    300
accgaggtcg tgctggccgc ctgggtcgcc tggggcctgg actgcctgcc ccgcttcaac    360
ggtgccttcg cctttgccct ttacgacagt gccaccggcg aactgcacct ggtgcgcgac    420
cggttcggca agcggccgct gtacgtggcg cggcaccgcg gcgcgtggct gttcgcctcc    480
gagatgaagg cgttcctggc ctaccccgac ttcaggttcg ccttcgacga ggcacagctg    540
gcgtcggtct tcgccacctg gaccccgctg cccggccaga gcggatacca agggatcgag    600
cagatcccca tgggcgagta tctgtccgta cgcggcgacg aggtccggcg cggccgctgg    660
gccacgctcg acctggccca aggcccggct ccggagagcg agcaggaggc cgccgagctt    720
gtccgcgcgg acctcgaagc cgcggtcgac gtgcgcctgc gcagcgatgt cgaggtcggc    780
```

gtctacgcct ccggcggcct ggactcctcg atcatcgcgc acatcgccgc gcagcggacg 840
agccgcccgc tgcggacgtt ctcgatcgag ttcgaggacg cagagttcga cgaatcggcc 900
gaacaggccg agctggccgc acacctgggc acccgccact ccaccgtgcg cgtgaccgac 960
gaggacgtcg ccgacgcctt ccccgaagcc gtccggcacg ccgaggtgcc cgtcttccgc 1020
accgccttcg tccccatgta cctgctggca ggccacgtcc gcagcgaagg gatcaaggtc 1080
gtgctcagcg gcgagggcgc cgacgaggcc ttcctcggct acggcatctt caaggacacg 1140
ctgctgctct cgacctggca cgagctggac gacgacaccc gtctgcgccg catgagccag 1200
ctctacccgt acctgagcca cttcagcggc gaggacggcc accgccggat gctcggcctc 1260
taccggcagt tcaccgagga gaccctgccc ggcctcttct cccaccagat gcggttccag 1320
aacggccgct tcgccgcacg cctgctcaag aacccgggcg acccccttcgc ggccctcggg 1380
gaactcgtgg ccggtgagcc cggctacgca cagctcaccc ccgtacagaa ggcccagtgg 1440
ctggagttcc gcacgctcct gagcggctac ctgctctcga cccagggcga gcgcatggcg 1500
ctggcccacg gcgtggagaa ccgctgcccc ttcctcgatc ccgccgtggt ccgccgcgcc 1560
gcatcggtga acctgcggtt cggcgacccc tacgacgaga agtacctgct caagtgcgcc 1620
tatgccgatg tgctgccgga acggatcgtc aagaagggga agttccccta ccgcgccccg 1680
gacagcgccg cgttcgtccg ctcccgcccg gactaccgcg agctgctgac cgaccccggc 1740
accctcgacg agatcggcgt cctcgatgcg cgcttcgtga agcggttcac cgaccgcgtc 1800
ttcgacagcc cgcctgagca gatcggcacg aaggagaacc aggccttcgt ctctttggcg 1860
tcgacggtct ggctgcacca ctggtacgtg cgcggcaacg cccgccgccg ggctccgctc 1920
ggggtccccc tgtacgtcgt cgaccggcgc agtggcgccc tgtcggccta g 1971

<210> 10
<211> 3192
<212> PRT
<213> Streptomyces platensis subsp. rosaceus

<400> 10

Met Lys Lys Gln Asn Gly Val Leu Ala Asp Asp Arg Asp Ile Ala Val
1 5 10 15

Ile Gly Leu Ser Leu Arg Leu Pro Gly Ser Arg Thr Pro Glu Glu Phe
20 25 30

Trp Ser His Leu Ala Glu Gly Arg Ser Leu Ile Ser Glu Val Pro Glu
35 40 45

Arg Arg Trp Arg Lys Glu Asp His Leu Gly His Pro Arg Arg Glu Phe
50 55 60

Asn Lys Thr Asn Ser Val Trp Gly Gly Phe Val Asp Asp Ala Asp Cys

65 70 75 80

Phe Asp Ala Asp Phe Phe Gln Ile Ser Pro Arg Glu Ala Gln Ser Met
85 90 95

Asp Pro Gln Gln Arg Met Ala Leu Glu Leu Ser Trp His Ala Leu Glu
100 105 110

Asp Ala Gly Tyr Arg Ala Asp Arg Val Ala Gly Ser Arg Thr Gly Val
115 120 125

Phe Met Gly Val Cys His Trp Asp Tyr Ala Glu Leu Met Glu Gln Glu
130 135 140

Val Glu Glu Ile Asp Ala Tyr Tyr Pro Thr Gly Ala Ala Tyr Ala Ile
145 150 155 160

Ile Ala Asn Arg Val Ser His His Phe Asp Phe Arg Gly Pro Ser Val
165 170 175

Val Asn Asp Thr Ala Cys Ala Gly Ser Leu Val Ala Val Gln Gln Ala
180 185 190

Val Gln Ala Leu Gln Ala Gly Asp Cys Asp Leu Ala Leu Ala Gly Gly
195 200 205

Val Asn Leu Thr Trp Ser Pro Arg His Phe Ile Ala Phe Ala Lys Ala
210 215 220

Gly Met Leu Ser Pro Asp Gly Leu Cys Arg Ala Phe Asp Ala Asn Ala
225 230 235 240

Asn Gly Tyr Val Arg Gly Glu Gly Gly Gly Ile Val Leu Leu Lys Arg
245 250 255

Ala Ala Asp Ala Arg Arg Asp Gly Asp Ala Val His Ala Val Ile Lys
260 265 270

Gly Ile Gly Ser Asn His Gly Gly Arg Thr Ser Ser Leu Thr Val Thr
275 280 285

Asn Pro Ala Ala Gln Ala Glu Leu Ile Ala Gly Val Tyr Arg Lys Ala
290 295 300

Gly Ile Ala Pro Glu Thr Val Thr Tyr Val Glu Thr His Gly Pro Gly
305 310 315 320

Thr Pro Val Gly Asp Pro Ile Glu Val Arg Gly Leu Lys Gln Ala Phe
325 330 335

Val Asp Leu Gly Ala Asp Arg Pro Gly Glu Ala Pro Ala His Arg Cys
340 345 350

Gly Ile Gly Ser Val Lys Thr Asn Ile Gly His Leu Glu Gly Ala Ala
355 360 365

Gly Ile Ala Gly Met Leu Lys Val Ile Leu Ala Met Arg His Arg Lys
370 375 380

Leu Pro Ala Thr Val Asn Phe Arg Lys Leu Asn Pro Leu Ile Asp Leu
385 390 395 400

Asp Gly Ser Pro Leu Tyr Val Leu Asp Arg Leu Thr Asp Trp Thr Ala
405 410 415

Glu Gly Ser Ala Pro Leu Arg Ala Gly Val Ser Ser Phe Gly Phe Gly
420 425 430

Gly Thr Asn Ala His Val Leu Leu Glu Ala Ala Glu Pro Val Ala Ala
435 440 445

Thr Glu Asp Ala Gly Glu Gln Trp Leu Pro Val Ser Ala Met Asp Glu
450 455 460

Asp Arg Leu Arg Glu Thr Cys Ala Arg Leu Ala Arg Trp Val Arg Thr
465 470 475 480

Arg Ile Glu Gln Asn Asp Ala Pro Ser Leu Thr Asp Ala Ala Arg Thr
485 490 495

Leu Arg Glu Gly Arg Val Ser Met Arg Glu Arg Val Val Phe Arg Ala
500 505 510

Ser Gly Ile Glu Glu Trp Ala Ala Gln Leu Glu Ser Val Ala Ala Gly
515 520 525

Asp Gly Pro Pro Ala Asp Cys Pro Arg Gly Arg Ala Gly Thr Glu Ala
530 535 540

Pro Asp Gly Leu Asp Ala Asp Asp Leu Thr Ala Leu Ala Glu Arg Trp
545 550 555 560

Leu Glu Lys Gly Arg Trp Asp Lys Phe Ala Ala Ala Trp Ala Gln Gly
565 570 575

Leu Ala Val Asp Trp Ala Pro Trp Pro Glu Arg Gly Arg Arg Val His
580 585 590

Val Pro Gly Tyr Ala Phe Ala Arg Thr Pro His Trp Phe Arg Thr Asp
595 600 605

Arg Asn Glu Thr Thr Gly Arg Pro Glu Arg Gly Ala Thr Asn Thr Ala
610 615 620

Pro Ala Pro Leu Gly Glu Gly Lys Pro Glu Gly Gly Ser Trp Thr Phe
625 630 635 640

Pro Leu His Phe Asp Ala Thr Gln Gly Phe Val Arg Asp His Arg Val
645 650 655

Asn Gly Ala Arg Ile Val Pro Gly Val Val Ala Leu Glu Leu Val Thr
660 665 670

Val Ala Ala Glu Arg Ala Ala Ala Ala Gly Ala Arg Ala Gly Leu Thr
675 680 685

Pro Arg Ile Arg Asn Ala Val Trp Ile Arg Pro Leu Leu Val Gly Asp
690 695 700

Thr Val Leu Ser Pro Gln Leu Arg Leu Thr Pro Ala Ala Asp Gly Tyr
705 710 715 720

Asp Tyr Ala Ile Thr Asp Glu His Gly Thr Gln Tyr Thr Ser Gly Arg
725 730 735

Val Glu Tyr Gly Glu Ala Ala Ala Ala Glu Lys Thr Asp Pro Gly Ala
740 745 750

Leu Arg Glu Arg Phe Pro Gln Arg Val Asp Thr Ala Glu Gly Tyr Ala
755 760 765

Ala Leu Arg Ser Ser Gly Ile Glu His Gly Pro Ala Leu Arg Gly Leu
770 775 780

Asn Ala Leu His Arg Gly Pro Asp Gly Val Leu Ala Glu Leu Arg Leu
785 790 795 800

Pro Ala Gly Ala Pro Glu Gly Met Ala Leu Gln Pro Ala Ile Leu Asp
805 810 815

Ser Ala Leu Leu Ala Ala Leu Ala Leu Gly Ser Ala Asp Gly Gly Trp
820 825 830

Arg Arg Pro Ala Ala Pro Val Val Pro Phe Ala Leu Asp Arg Leu Thr
835 840 845

Val His Ala Ala Thr Thr Ser Thr Met Trp Ala Trp Leu Arg Pro Ala
850 855 860

Gly Ser Gly Thr Ala Gly Asp Met Ala Arg Ser Asp Ile Asp Leu Phe
865 870 875 880

Asp Asp Asn Gly Arg Leu Cys Val Arg Leu Ala Gly Tyr Thr Ser Arg
885 890 895

Glu Leu Pro Thr Ala Glu Pro Ala Ala Val Gln Ala Pro Glu Gly Glu
900 905 910

Leu Leu Glu Val Thr Gly Val Trp Glu Glu Ala Pro Ala Pro Ala Pro
915 920 925

Ala Ala Gly Gln Ala Thr Pro Val Gly Pro Val Thr Val Leu Asn Ala
930 935 940

Ala Leu Asp Gly Asp Leu Ala Ala Ala Ser Ala Ala Arg Leu Gly Met
945 950 955 960

Asp Ile Arg Gln Leu Ala Gly Pro Ala Glu Ala Thr Asp Ala Thr Asp
965 970 975

Ala Val Ala Met Lys Ala Ala Phe Glu Ala Cys Tyr Pro Gln Val Arg
980 985 990

Gln Leu Leu Gly Gln Gly Arg Gln Val Leu Val Val Ala Pro Gly Ala
995 1000 1005

Pro Asp Ser Pro Val Tyr Ala Pro Leu Ala Ala Leu Leu Lys Thr
1010 1015 1020

Ala Gln Gln Glu Asn Pro Ser Phe Arg Gly Arg Leu Val Leu Leu
1025 1030 1035

Asp Gly Tyr Asp Pro Arg Asp Ala Asp Arg Phe Glu Arg Val Val

1040 1045 1050

Ser Ala Glu Ala Gly Ala Gly Asp Asp Thr Glu Val Ala Tyr Asp
1055 1060 1065

Ala Gln Asp Arg Arg Leu Arg His Gly Phe Val Glu Leu Pro Arg
1070 1075 1080

Gly Glu Ala Gly Glu Ser Leu Leu Arg Asp Gly Gly Val Tyr Trp
1085 1090 1095

Ile Thr Gly Gly Ala Gly Gly Leu Gly Leu Leu Leu Ala Glu Arg
1100 1105 1110

Leu Cys Glu Arg Arg Arg Ala Thr Val Val Val Ser Gly Arg Ser
1115 1120 1125

Ala Asp Ser Arg Ala Ile Glu Ala Leu Arg Ala Arg Leu Phe His
1130 1135 1140

Gly Glu Val Ala Tyr Arg Arg Thr Asp Val Thr Asp Ala Asp Ala
1145 1150 1155

Val Arg Asp Ala Val Ala Asp Ile Arg Ala Arg Tyr Gly Arg Leu
1160 1165 1170

Asp Gly Val Phe His Ala Ala Gly Val Leu Asp Asp Gly Tyr Leu
1175 1180 1185

Ala Ser Lys Pro Leu Ala Gly Thr Ala Ala Val Leu Ala Pro Lys
1190 1195 1200

Val Asp Gly Ala Thr Ser Ile Asp Asp Ala Thr Arg Ala His Gly
1205 1210 1215

Leu Asp Phe Leu Leu Leu Phe Gly Ser Val Ala Gly Ala Phe Gly
1220 1225 1230

Asn Ala Ala Gln Ala Asp Tyr Ala Ala Ala Asn Ala Phe Leu Asp
1235 1240 1245

Ala Phe Ala Ala Arg Arg Gln Ala Ala Gly Ser Val Thr Arg Ser
1250 1255 1260

Val Asp Trp Pro Leu Trp Ala Asp Gly Gly Met Arg Val Asp Asp
1265 1270 1275

Ala Ser Leu Ala Tyr Leu Arg Lys Arg Thr Gly Thr Val Pro Leu
1280 1285 1290

Pro Ser Glu Thr Gly Leu Asp Ala Leu Glu Arg Ala Leu His Ser
1295 1300 1305

Ala Ala Pro Val Arg Arg Val Val Leu Phe Gly Glu Arg Ser Lys
1310 1315 1320

Leu Arg Gly Tyr Ala Gly Leu Asp Arg Val Ala Lys Pro Glu Pro
1325 1330 1335

Arg Thr Ser Gly Ala Gln Arg Asn Thr Ala Ala Pro Ala Val Leu
1340 1345 1350

Glu Glu Ser Glu Leu Val Ala Arg Thr Gln Asp Leu Leu Arg Asn
1355 1360 1365

Leu Phe Ala Glu Val Thr Leu Gln Asp Ala Glu His Ile Leu Ala
1370 1375 1380

Glu Glu Lys Leu Glu Thr Tyr Gly Ile Glu Ser Ile Ser Ile Val
1385 1390 1395

Glu Leu Thr Ser Lys Leu Glu Asp Thr Phe Gly Ser Leu Pro Lys
1400 1405 1410

Thr Leu Phe Phe Glu Tyr Val Asp Leu Gln Gly Val Ala Gly Tyr
1415 1420 1425

Phe Val Ala Glu His Arg Asp Arg Leu Leu Glu Leu Phe Ala Pro
1430 1435 1440

Glu Ala Pro Ala Pro Glu Ala Pro Ala Pro Glu Ala Pro Ala Pro
1445 1450 1455

Glu Ala Pro Ala Pro Glu Glu Pro Ala Pro Glu Gly Pro Ala Val
1460 1465 1470

Glu Glu Pro Pro Ala Ala Ala Pro Thr Pro Ala Val Arg Pro Ser
1475 1480 1485

Val Glu Ala Ala Ala Gly Arg Ala Arg Pro Ala Trp Ala Asp Pro
1490 1495 1500

Glu Arg His Asp Ile Ala Val Ile Gly Met Ala Gly Arg Tyr Pro
1505 1510 1515

Gly Ala Asp Thr Leu Glu Glu Phe Trp Glu Leu Leu Ser Glu Gly
1520 1525 1530

Arg His Ser Phe Glu Pro Val Pro Glu Ser Arg Trp Arg His Gly
1535 1540 1545

Asp Ile Tyr Phe Asp Glu Arg Asp Val Asp Gly Lys Thr Val Val
1550 1555 1560

Lys Thr Gly Thr Phe Leu Arg Asp Val Glu Ala Phe Asp Pro Arg
1565 1570 1575

Tyr Phe Asn Ile Ser Gln Arg Asp Ala Glu Leu Leu Ser Pro Glu
1580 1585 1590

Val Arg Leu Phe Leu Gln Ala Gly Val Glu Ala Leu Glu Asp Ala
1595 1600 1605

Gly Tyr Ser Arg Glu Thr Leu Arg Arg Arg Tyr Asp Gly Asp Val
1610 1615 1620

Gly Val Leu Val Gly Ser Met Asn Asn Ser Tyr Ser Leu Tyr Gly
1625 1630 1635

Phe Gln Asn Met Leu Met Arg Gly Thr Ala Thr Ser Gly Ser Glu
1640 1645 1650

Leu Gly Val Met Ala Asn Met Leu Ser Tyr His Tyr Gly Phe Thr
1655 1660 1665

Gly Pro Ser Val Phe Leu Asp Thr Met Cys Ser Ser Ala Ser Ala
1670 1675 1680

Cys Val His Gln Ala Val Arg Met Leu Arg Ser Gly Glu Cys Arg
1685 1690 1695

Met Thr Val Val Gly Gly Ile Asn Leu Met Leu His Pro Phe Asp
1700 1705 1710

Leu Ile Ala Thr Ser Gln Ala His Phe Thr Thr Lys Ser Ala Glu
1715 1720 1725

Val Val Arg Ser Tyr Gly Leu Gly Ala Asp Gly Thr Ile Leu Gly
1730 1735 1740

Glu Gly Val Gly Thr Leu Val Leu Lys Pro Leu Ala Glu Ala Val
1745 1750 1755

Ala Asp Gly Asp His Val Tyr Gly Val Ile Lys Gly Ser Gly Met
1760 1765 1770

Thr Asn Ala Gly Val Arg Asn Gly Phe Thr Val Pro Ser Pro Gln
1775 1780 1785

Gln Gln Ala Arg Ala Ile Glu Lys Ala Leu Asp Asp Ala Ala Val
1790 1795 1800

Asp Ala Arg Thr Ile Ser Tyr Leu Glu Gly His Gly Ser Ala Thr
1805 1810 1815

Ser Leu Gly Asp Pro Ile Glu Ile Lys Gly Ala Ala Leu Ala Phe
1820 1825 1830

Gly Arg Asp Thr Gln Asp Leu Gly Phe Cys Ala Leu Gly Ser Val
1835 1840 1845

Lys Ser Asn Val Ala His Leu Leu Ser Gly Ser Gly Met Ala Gly
1850 1855 1860

Leu Thr Lys Val Leu Leu Gln Leu Lys His Arg Thr Leu Ala Pro
1865 1870 1875

Ser Leu His Ala Gly Thr Leu Ser Ser Ala Ile Asp Phe Glu Glu
1880 1885 1890

Thr Pro Phe Val Val Gln Arg His Arg Asp Thr Trp Arg Arg Pro
1895 1900 1905

Val Val Gly Gly Glu Glu Ala Pro Arg Arg Ala Gly Val Thr Ser
1910 1915 1920

Ile Gly Ala Gly Gly Ile Asn Val His Ile Val Val Glu Glu Tyr
1925 1930 1935

Asp Gly Gln Val Val Ala Ala Pro Glu Arg Gly Arg Pro Arg Leu
1940 1945 1950

Leu Val Phe Ser Ala Met Thr Pro Gln Ala Leu Gln Ser Val Leu

1955 1960 1965

Arg Ala Met His Glu His Val Arg Glu Thr Ala Pro Gly Leu Asp
1970 1975 1980

Ala Leu Ala Tyr Thr Leu Gln Thr Gly Lys Asn Glu Leu Pro Cys
1985 1990 1995

Arg Leu Ala Phe Val Ala Asp Asp Ile Ala Asp Ala Gln Ala Arg
2000 2005 2010

Leu Ala Arg Leu Ser Ala Val Asp Trp Thr Ala Glu Ser Pro Gly
2015 2020 2025

Val Pro Ala Gly Val His Phe Thr Ala Ser Thr Leu Arg Arg Arg
2030 2035 2040

Arg Thr Ala Asp Ala Ala Thr Val Glu Gln Ala Leu Arg Asp Gly
2045 2050 2055

Lys Gln Ala Glu Leu Ala Gln His Trp Ala Asp Gly Ala Ser Val
2060 2065 2070

Asp Trp Asp Leu Leu Trp Pro Ala Gly Ser Arg Pro Ala Lys Pro
2075 2080 2085

Ser Leu Pro Ala Tyr Pro Phe Asp Lys Val Arg Cys Trp Tyr Pro
2090 2095 2100

Glu Asp Asp Asp Ala Pro Ser Val Leu Arg Pro Leu Ala Phe Ala
2105 2110 2115

Arg Arg Ala His Pro Trp Val Gly Val Asn Ala Ser Asp Leu Gly
2120 2125 2130

Gly Val Arg Tyr Thr Leu Arg Leu Arg Gly Asp Glu Leu Leu Asp
2135 2140 2145

Tyr Val Tyr Thr Val Gly Arg Lys Arg Arg Tyr Ala Thr Val Ala
2150 2155 2160

Leu Leu Asp Ala Ala Leu Ala Phe Ala Arg Leu Ala Gly Leu Glu
2165 2170 2175

Gly Pro Leu Arg Leu Arg Asn Ala Gln Trp Ala Ala Leu Pro Ser
2180 2185 2190

Pro Ala Asp Thr Pro Glu Thr Phe Thr Trp Arg Leu Gly Thr Ser
2195 2200 2205

Gly Asp Gly Val His Arg Val Glu Leu Trp His Ala Asp Glu Ala
2210 2215 2220

Thr Leu Arg Phe Ala Ala Asp Val Val Pro Ser Ala Pro Ala Glu
2225 2230 2235

Asp Ala Ser Met Pro Gln Met Ser Ser Ala Pro Ala Thr Leu Asp
2240 2245 2250

Arg Asp Asp Phe Tyr Ala Ala Leu Gly Thr Ala Gly Leu Asp Ala
2255 2260 2265

Arg Pro Tyr Ala Arg Ser Val Glu Gly Val Thr Glu Leu Asp Ala
2270 2275 2280

His Arg Leu Leu Val Arg Val Ala Glu Pro Ala Met Cys Gln Asp
2285 2290 2295

Pro His Lys Gln His Val His Leu Pro Ala Trp Ala Leu Val Gly
2300 2305 2310

Leu Thr Gln Gly Val Gln His Ala Trp Gly Arg Ala Asp Ala Ala
2315 2320 2325

Val Val Arg Val Gly Ser Val Gln Gly Glu Gln Trp Glu Arg Thr
2330 2335 2340

Arg Ala Ile Val Leu Ala Arg Thr Ser Asp Ala Val Phe His Ala
2345 2350 2355

Ala Phe Leu Asp Glu Asp Gly Arg Val Leu Gly Arg Val Glu Asp
2360 2365 2370

Ala Glu Phe Thr Ala Gly Asp Leu Glu Pro Ala Leu Pro Gly Glu
2375 2380 2385

Ala Gly Arg Ala Leu Val Ala Leu Pro Gln Ala Ser Arg Pro Val
2390 2395 2400

Leu Glu Thr Pro Val Gly Thr Gly Glu Trp Gln Gln Ser Glu Ala
2405 2410 2415

Val Arg Pro Glu Ala Glu Pro Ser Val Thr Val Ala Ala Val Ala
2420 2425 2430

Asp Gly Pro Ala Ala Leu Val Ala Ser Leu Arg Glu Thr Val Ala
2435 2440 2445

Asp Leu Leu Lys Phe Asp Leu Ala Asp Ile Asp Leu Asp Thr His
2450 2455 2460

Phe His Ala Tyr Gly Phe Glu Ser Ile Ala Leu Ala Lys Leu Ala
2465 2470 2475

Ser Glu Leu Asn Gly Val Leu Gly Thr Asp Leu Thr Pro Ala Val
2480 2485 2490

Phe Phe Glu Cys Ser Asp Ile Arg Ser Leu Ala Glu Tyr Leu Leu
2495 2500 2505

Asp Arg Tyr Gly Pro Glu Leu Ser Leu Pro Thr Ser Ala Asp Ala
2510 2515 2520

Pro Ala Pro Val Ala Ala Thr Arg Pro Ser Pro Val Pro Met Pro
2525 2530 2535

Ala Pro Gly Pro Asp Asp Asp Ala Val Ala Ile Val Gly Ala Ala
2540 2545 2550

Gly Arg Phe Pro Gly Ala Asp Asp Leu Asp Thr Phe Trp Gln Gln
2555 2560 2565

Leu Arg Ala Gly Glu Asp Leu Ile Ala Asp Tyr Pro Gly Asp Arg
2570 2575 2580

Phe Asp Gly Gly Pro Tyr Ala Glu Val Val Ala Arg Ala Asp Phe
2585 2590 2595

Pro Lys Phe Ala Gly Arg Ile Glu Gly Val Asp Arg Phe Asp Ala
2600 2605 2610

Asp Phe Phe His Leu Ser Arg Leu Glu Ala Glu Leu Met Asp Pro
2615 2620 2625

Gln His Arg Leu Ala Leu Glu Thr Val Trp Ala Ala Leu Glu Asn
2630 2635 2640

Gly Gly Tyr Ala Pro Ala Arg Leu Pro Glu Asn Thr Gly Val Tyr
2645 2650 2655

Phe Gly Val Ser Gly Ser Asp Tyr His His Leu Leu Asn Ala Ser
2660 2665 2670

Gly Val Ala Pro Asp Gly Phe Thr Ala Thr Gly Asn Ala His Ser
2675 2680 2685

Met Leu Ala Asn Arg Ile Ser Tyr Val Leu Asp Val His Gly Pro
2690 2695 2700

Ser Glu Pro Val Asp Thr Ala Cys Ser Ser Ser Leu Val Ala Leu
2705 2710 2715

His Arg Ala Val Glu His Ile Arg Ser Gly Arg Cys Glu Met Ala
2720 2725 2730

Ile Ala Gly Gly Val Asn Leu Leu Leu Ser Val Asp Thr Phe Ala
2735 2740 2745

Ala Thr His Met Ala Gly Met Leu Ser Pro Asp Gly Arg Cys Lys
2750 2755 2760

Thr Phe Ser Ala Gly Ala Asp Gly Tyr Val Arg Ser Glu Gly Val
2765 2770 2775

Ala Ala Val Leu Leu Lys Pro Leu Ala Gln Ala Gln Arg Asp Gly
2780 2785 2790

Asp Ala Ile Trp Gly Val Val Arg Gly Ser Ala Glu Asn His Gly
2795 2800 2805

Gly Arg Ala Gly Ser Leu Thr Ala Pro Asn Gly Lys Ala Gln Ala
2810 2815 2820

Ala Leu Ile Gln Asp Ala Met Arg Gly Ile Asp Pro Asp Ser Ile
2825 2830 2835

Gly Tyr Val Glu Ala His Gly Thr Gly Thr Gly Leu Gly Asp Pro
2840 2845 2850

Val Glu Val Asn Ala Leu Asp Ser Ala Tyr Arg Ala Leu Arg Thr
2855 2860 2865

Ala Glu Gly Gly Pro Pro His Ala Ala Arg Pro Cys Ala Leu Gly

2870 2875 2880

Ser Val Lys Thr Asn Ile Gly His Ala Glu Ser Ala Ala Gly Leu
2885 2890 2895

Ala Gly Val Leu Lys Val Leu Leu Ala Met Arg His Arg Glu Leu
2900 2905 2910

Pro Pro Ala Leu His Cys Asp Arg Leu Asn Pro His Leu Pro Leu
2915 2920 2925

Asp Gly Gly Phe Glu Val Val Arg Glu Leu Arg Arg Trp Glu Pro
2930 2935 2940

Cys Thr Asp Ala Thr Gly Arg Pro Trp Pro Leu Arg Ala Gly Val
2945 2950 2955

Ser Ser Phe Gly Phe Gly Gly Ala Asn Ala His Val Val Leu Glu
2960 2965 2970

Ala Pro Pro Val Pro Pro Ala Pro Ala Glu Pro Ala Arg Pro Thr
2975 2980 2985

Ala Pro Gln Ala Ile Val Leu Ser Ala Arg Asp Asp Asp Arg Leu
2990 2995 3000

Arg Ala Thr Ala Gly Arg Leu Arg Asp Phe Leu Asp Arg Ala Arg
3005 3010 3015

Arg Asp Gly His Ala Pro Asp Leu Ala Asp Leu Ala Phe Thr Leu
3020 3025 3030

Gln Val Gly Arg Glu Ala Met Glu Arg Arg Leu Gly Phe Val Val
3035 3040 3045

Gly Ser Met Asp Asp Val Leu Gly Thr Leu Asp Arg Phe Phe Ala
3050 3055 3060

Gly Asp Glu Pro Ser Gly Trp His Thr Gly Gly Ile Arg Arg Ser
3065 3070 3075

Arg Gly Ala Gly Val Arg Arg Glu Ala Glu Gln Ala Pro Glu Val
3080 3085 3090

Thr Arg Ala Leu His Asp Gly Arg Leu Asp Arg Val Thr Ala Leu
3095 3100 3105

Trp Cys Asp Gly Ala Pro Val Asp Trp Gln Ala Met His Pro Thr
3110 3115 3120

Gly Glu Arg Arg Ala Val Arg Leu Pro Ala Tyr Pro Phe Ala Cys
3125 3130 3135

Asp Arg Tyr Trp Val Pro Ala Val Gly Thr Ala Pro Val Pro Pro
3140 3145 3150

Pro Ala Ala Pro Val Pro Pro Pro Ala Ala Glu Pro Ala Phe Glu
3155 3160 3165

Thr Asp Ala Arg Ala Ala Leu Leu Asp Ala Val Leu Asp Gly Arg
3170 3175 3180

Ala Gly Pro Asp Ala Leu Ser Arg Thr
3185 3190

<210> 11
<211> 9579
<212> DNA
<213> Streptomyces platensis subsp. rosaceus

<400> 11

atgaagaagc agaacggcgt cctcgccgac gaccgggaca tcgccgtcat cggcctgtcc 60
ctgcggttgc ccggctcgcg cacgcccgag gagttctgga gccacctggc cgagggccgc 120
tcgctcatca gcgaagtccc ggagcgccga tggcgaaagg aggaccacct. cggtcacccg 180
cgccgcgaat tcaacaagac caacagcgtc tggggcggct tcgtcgacga cgccgactgc 240
ttcgacgccg atttcttcca gatctccccg cgcgaggcgc agtccatgga cccgcagcag 300
cggatggccc tggagctgag ctggcacgcc ctggaggacg ccggctaccg ggccgaccgc 360
gtggcgggct cccgcaccgg tgtcttcatg ggcgtctgcc actgggacta cgccgagctg 420
atggagcagg aagtcgagga gatcgacgcc tactacccga ccggcgccgc gtacgcgatc 480
atcgccaacc gggtctccca ccacttcgac ttccgcggac cgagcgtcgt caacgacacg 540
gcctgcgcgg gctcgctcgt ggccgtgcag caggcggtgc aggccctcca ggccggcgac 600
tgcgacctcg cgctcgccgg cggcgtcaat ctgacctggt cgccgcggca cttcatcgcc 660
ttcgccaagg cgggcatgct ctcgcccgac ggcctgtgcc gggcgttcga cgcgaatgcc 720
aacggctatg tgcgcggcga gggcggcggc atcgtgctgc tgaagcgggc cgcggacgcc 780
cgccgcgacg gcgacgccgt gcacgccgtg atcaagggca tcggcagcaa ccacggcggg 840
cgcaccagtt cgctgaccgt taccaacccg gccgcacagg ccgaactgat cgcgggtgtc 900
taccgcaagg ccggcatcgc acccgagacc gtcacctacg tggagaccca cggcccggc 960
acaccggtcg gggaccccat cgaggtccgc ggcctcaagc aggccttcgt cgacctgggc 1020
gcagaccggc ccggggaggc tccggcccac cggtgcggca tcggctccgt gaagaccaac 1080
atcggccacc tggaaggggc cgccggcatc gcgggcatgc tcaaggtcat cctcgccatg 1140
cgccaccgca agctgcccgc gacggtcaac ttccgcaagc tcaacccccct catcgacctg 1200
gacggcagcc cgctgtacgt cctcgaccgc ctcaccgact ggaccgccga agggtccgca 1260
ccgctgcgcg ccggcgtcag ctccttcgga ttcggcggga ccaacgccca cgtcctgctg 1320
gaagccgcgg agccggtggc cgccaccgag gacgccggcg aacagtggct gccggtgtcc 1380
gccatggacg aggaccggct tcgcgagacg tgcgcccggc tcgcccgctg ggtccggacc 1440
cggatcgagc agaacgatgc tccgtccctg accgatgccg cccgcacgct gcgcgaaggc 1500
cgggtgtcca tgcgcgagcg cgtggtgttc cgcgcaagcg gcatcgagga gtgggcggca 1560

cagctggaga gcgtcgccgc gggggacggc ccgcccgcgg actgcccgcg cggccgggcc 1620
ggaaccgaag cccccgacgg cctggacgcg gacgacctga cggccctggc cgagcgctgg 1680
ctggagaagg gccggtggga caagttcgcg gccgcctggg cccagggcct ggccgtggac 1740
tgggcaccgt ggcccgagcg cggccgccgc gtgcacgtgc ccggctacgc gttcgcccgc 1800
acgccgcact ggttccggac ggaccggaac gagacgaccg gaaggccgga gcgcggcgcg 1860
acgaacaccg ctcccgcccc gctcggcgaa ggcaagccgg aaggcggcag ctggaccttc 1920
cccctgcact tcgacgccac ccagggattc gtccgcgacc accgcgtcaa cggcgcacgg 1980
atcgtcccgg gcgtggtggc cctggaactc gtcaccgtgg cagccgaacg ggccgccgcc 2040
gcaggtgccc gggccgggct gacgccccgc atccgcaacg cggtgtggat ccgtccgctg 2100
ctcgtcggcg acaccgtgct ctccccgcag ctccgcctga cccccgccgc cgacggctac 2160
gactacgcga tcaccgacga gcacggcacg cagtacacca gcggccgggt cgagtacggc 2220
gaggctgccg cggccgagaa gacggacccg ggcgcgctgc gcgagcgctt cccccagcgc 2280
gtcgacaccg ccgagggtta cgccgcgctg cggtccagcg gcatcgagca cggccccgcc 2340
ctgcgcggcc tcaacgccct gcaccgcggt ccggacggcg tgctggccga gctgcggctc 2400
cccgcaggtg ccccggaggg catggcgctg cagcccgcga tcctcgacag cgccctcctc 2460
gcggccctgg ctctcggctc ggccgacggc ggctggcgca ggcccgccgc ccccgtggtg 2520
ccgttcgcgc tggaccggct caccgtgcac gcggcgacga cctcgacgat gtgggcgtgg 2580
ctgcggccgg ccggcagcgg cacggcaggt gacatggcca gatccgacat cgacctgttc 2640
gacgacaacg gccggctgtg cgtgcgcctg gccggctaca cgtcgaggga actgcccacc 2700
gcagaaccgg cagcagtcca ggccccggaa ggggagctcc tggaggtcac cggtgtgtgg 2760
gaggaggccc ctgccccggc gcccgcagcc ggtcaggcca ccccggtcgg cccggtgacg 2820
gtgctcaacg ccgcactgga cggcgacctc gcagcggcga gcgccgcgcg gctgggcatg 2880
gacatccggc agctggccgg tcccgcggaa gccaccgatg ccaccgatgc cgtcgccatg 2940
aaggcggcgt tcgaggcctg ctacccgcag gtccggcaac tgctcggtca gggacggcag 3000
gtgctcgtcg tcgccccggg cgccccggac tcgccggtct acgccccact ggcggcgctg 3060
ctgaagaccg cacaacagga gaatccttcc ttccgggggga ggctggtgct cctcgacggc 3120
tacgaccccc gcgacgccga ccgcttcgag cgtgtcgtca gcgcggaggc gggcgccgga 3180
gacgacaccg aagtcgccta cgacgcccag gaccgccgac tgcggcacgg cttcgtggaa 3240
cttccccggg gcgaggcggg ggagagcctg ctgcgcgacg gtggcgtcta ctggatcacc 3300
gggggcgccg gcggactcgg cctgctgctc gccgagcggc tctgcgagcg ccgccgcgcc 3360

acggtcgtcg tcagcggccg ctcggcggac agccgggcca tcgaggcact gcgggcccgc 3420
ctgttccacg gcgaggtggc gtaccgccgc acggacgtca cggacgcgga cgccgtacgg 3480
gacgcggtcg cggacatccg cgcgcggtac ggacggctcg acggcgtgtt ccacgcggcc 3540
ggcgtcctcg acgacggcta cctcgcgagc aagcccctcg cgggcaccgc cgccgtactc 3600
gcgcccaagg tggacggcgc cacgtccatc gatgacgcga cgcgcgccca cggcctggac 3660
ttcctcctgc tgttcggctc cgtggcgggc gccttcggca acgccgcgca ggccgactac 3720
gccgccgcca acgccttcct cgacgcgttc gccgcacgac ggcaggccgc cggcagcgtg 3780
acccgctccg tcgactggcc gctgtgggcc gacggcggca tgcgcgtgga cgacgccagc 3840
ctcgcctacc tgcgcaagcg caccgggacc gtgccactgc cgagcgagac cggcctggac 3900
gcactggagc gcgcactgca ctccgccgcg ccggtccgcc gcgtggtgct cttcggggag 3960
cggtccaagc tgcgcgggta cgcgggcctg gaccgcgtcg cgaagccgga gccgcgcacg 4020
tccggggcgc agcggaacac ggccgcgccg gccgtcctgg aggagagcga actcgtagcc 4080
cgtacacagg acctgctgcg gaacctgttc gccgaggtga ccctgcagga cgcggagcac 4140
atcctggccg aggagaagct ggagacctac ggtatcgaat cgatctccat cgtcgagctg 4200
accagcaagc tggaggacac cttcgggtcg ctgcccaaga cgctcttctt cgagtacgtc 4260
gatctgcagg gcgtggccgg ctacttcgtc gccgagcacc gcgaccggct cctcgaactc 4320
ttcgcccccg aagcacccgc ccccgaagca cccgcccccg aagcacccgc ccccgaagca 4380
cccgcgcccg aggagcccgc cccggagggg cctgccgtcg aggagccgcc cgcggccgcg 4440
cccaccccgg ccgtccggcc gtccgtggag gccgccgccg ggcgcgcccg cccggcctgg 4500
gccgatccgg agcgccacga catcgcggtc atcggtatgg cgggccggta cccgggcgcc 4560
gacaccctgg aggagttctg ggagctgctc agcgagggcc ggcacagttt cgagcccgtc 4620
ccggaatcgc ggtggcggca cggcgacatc tacttcgacg agcgtgacgt cgacggcaag 4680
accgtcgtca agaccggcac cttcctgcgg gacgtcgagg cgttcgatcc gcgctacttc 4740
aacatctccc agcgcgacgc cgagctgctg tcgccggagg tccggctgtt cctgcaggcg 4800
ggcgtggagg ccctggagga cgcgggctac tcacgtgaga cgctgcggcg ccgctacgac 4860
ggcgacgtcg gtgtgctcgt cggctcgatg aacaacagct actcgctcta cggcttccag 4920
aacatgctga tgcgcggcac cgcgaccagc ggcagcgagc tcggtgtgat ggcgaacatg 4980
ctgtcgtacc actacggctt caccgggccg tccgtgttcc tcgacaccat gtgctcctcg 5040
gcgtcggcgt gtgtgcacca ggcggtgcgt atgctgcgca gcggcgagtg ccgcatgacc 5100
gtcgtcggcg gcatcaatct gatgctgcac ccgttcgacc tcatcgcgac ctcgcaggcg 5160
cacttcacca ccaagtcggc cgaagtcgtg cgcagttacg gcctcggcgc cgacggcacg 5220

atcctgggcg aaggcgtggg cacgctcgtg ctcaagccgc tggccgaagc cgtcgccgac 5280
ggcgaccacg tctacggcgt gatcaagggc agcggcatga ccaacgccgg ggtccgcaac 5340
ggcttcacgg tgccgagccc acagcagcag gcgcgcgcca tcgagaaggc gctcgacgac 5400
gccgccgtgg acgcgcgcac gatcagctac ctggagggtc acggctcggc gacctccctc 5460
ggcgacccca tcgagatcaa gggcgccgcc ctcgcgttcg gccgggacac ccaggacctg 5520
gggttctgcg cgctgggctc ggtcaagtcc aacgtggcgc acctgctgtc cggatccggc 5580
atggccggcc tgaccaaggt gctgctgcag ctcaagcacc gcacgctggc gccctcgctg 5640
cacgccggga cgctcagctc agcgatcgac ttcgaggaga ccccgttcgt ggtgcagcgc 5700
caccgcgaca cgtggcggcg ccccgtggtc ggcggcgagg aggcgccgcg ccgggcaggc 5760
gtcacgtcca tcggcgcggg cggcatcaac gtgcacatcg tcgtcgagga gtacgacggc 5820
caggtcgtcg ccgcaccgga gcgcggtcgc ccgcggctgc tggtgttctc cgccatgaca 5880
ccccaggccc tgcagtcggt gctgcgcgcc atgcacgagc acgtacggga gaccgcaccg 5940
ggcctggacg ccctcgcgta caccctgcag accggcaaga acgaactgcc gtgccggctg 6000
gccttcgtcg cggacgacat cgcggacgcc caggcccgtc tggcccggct gtccgcggtg 6060
gactggacgg cggagtcacc cggcgtgccc gcaggcgtgc acttcacggc gagcacgctg 6120
cggcgccggc gcaccgccga cgcggcgacc gtcgaacagg ccctgcgcga cgggaagcag 6180
gcggagctgg cgcagcactg ggcggacggc gcgagcgtcg actgggacct gctgtggccg 6240
gcgggaagcc gtccggccaa gccgtcgctg cccgcctacc ccttcgacaa ggtgcgctgc 6300
tggtaccccg aggacgacga cgcgcccagc gtgctgcggc cgctcgcctt cgcccggcgc 6360
gcgcacccct gggtcggcgt caacgcctcg gacctgggcg gggtgcgcta caccctccgg 6420
ctgcgcggcg acgaactcct cgactacgtc tacaccgtag gacgcaagcg ccgttacgcc 6480
accgtggcgc tgctggacgc ggcactggcg ttcgcgcggc tcgccgggct ggaagggccg 6540
ctgcggttgc ggaacgcgca gtgggccgca ctcccgtcgc ccgcggacac ccccgagacg 6600
ttcacctggc ggctcggcac gtccggcgac ggcgtgcatc gcgtcgagct gtggcacgcc 6660
gatgaggcca cgctccggtt cgccgccgac gtcgtaccgt ccgcgcctgc cgaagacgca 6720
tcgatgccgc agatgagcag cgcgcccgcg accctcgacc gggacgactt ctacgccgcg 6780
ctcggcaccg cgggcctcga cgcccggccg tacgcgcgca gcgtcgaagg ggtcaccgaa 6840
ctcgacgccc accggctgct cgtacgggtc gccgaaccgg ccatgtgcca ggacccgcac 6900
aagcagcacg tgcatctccc ggcctgggcg ctcgtcgggc tgacccaggg tgttcagcac 6960
gcgtggggcc gggccgacgc cgccgtggtg cgggtcggat ccgtgcaggg cgagcagtgg 7020

gagcgcaccc gggcgatcgt gctggcgcgg acgtccgacg ccgtcttcca tgcggctttc 7080
ctcgacgagg acggccgcgt gctgggccgg gtcgaggacg ccgagttcac cgcgggcgac 7140
ctggagccgg cactccccgg tgaggccgga cgcgcactcg tggcactgcc gcaggcgtcg 7200
cgtccggtgc tggagacgcc ggttggtacg gggagtggc agcagtcgga ggccgtgcgg 7260
ccggaggccg agccgtccgt gaccgttgcg gcggtcgcgg acgggccggc ggcgctcgtc 7320
gcgtcgctgc gcgagaccgt cgccgacctg ctcaagttcg acctggcgga catcgacctc 7380
gacacgcact tccacgcgta cggcttcgag tccatcgcgc tggccaaact ggcctcggaa 7440
ctcaacggcg tcctcggcac ggacctcacc cccgccgtct tcttcgagtg ctccgacatc 7500
cgcagcctcg ccgagtacct gctcgaccgc tacggccccg agctgagcct ccccacgagc 7560
gccgacgccc ccgcgccggt cgccgccacc cggccgtccc cagtgccgat gccggcaccc 7620
gggccggacg acgacgcggt ggccatcgtc ggcgctgccg gacggttccc cggcgcggac 7680
gacctggaca ccttctggca gcagctgcgc gcgggcgagg acctgatcgc cgactacccc 7740
ggcgaccgct tcgacggggg cccctacgcg gaggtcgtcg cgcgggcgga cttcccgaag 7800
tttgccggcc ggatcgaggg cgtggaccgc ttcgacgcgg acttcttcca cctgtcgcgg 7860
ctggaggcgg agctgatgga cccgcagcac cggctggccc tggagaccgt gtgggccgcg 7920
ctggagaacg gcggctacgc cccggcgcgc ctccccgaga acaccggggt ctacttcggc 7980
gtctccggca gcgactacca ccacctgctc aacgccagtg gcgtggcacc cgacggcttc 8040
accgccaccg gcaacgccca ctcgatgctg gccaaccgga tctcctacgt cctggacgtg 8100
cacgggccga gcgaacccgt cgacacggcc tgctccagct cgctcgtcgc gctgcaccgc 8160
gccgtcgagc acatccggtc gggccgatgc gagatggcca tcgcgggcgg tgtcaacctg 8220
ctgctgagcg tggacacctt cgccgcgacg cacatggcgg gcatgctcag ccccgacggc 8280
cgctgcaaga ccttctccgc cggcgcggac ggctacgtcc gctccgaggg cgtcgccgcg 8340
gtgctgctca agccgctcgc ccaggcgcag cgggacggcg acgccatctg ggcgtcgtc 8400
cggggcagcg ccgagaacca cggcggccgc gccggttcgc tgaccgcccc caacggcaag 8460
gcgcaggccg ccctgatcca ggacgccatg cgcggcatcg acccggacag catcggctac 8520
gtcgaggcgc acggcacggg caccggcctg ggcgacccgg tcgaggtcaa cgccctcgac 8580
agcgcctacc gcgccctgcg caccgccgag ggcgggccgc cgcacgcggc ccggccgtgc 8640
gcgctcggct cggtgaagac caacatcggc cacgcggagt cggccgcggg cctggccgga 8700
gtgctgaagg tgctgctcgc catgcgtcac cgcgagctgc cgccggcctt gcactgcgac 8760
cggctcaacc cgcacctgcc gctcgacggc ggattcgagg tcgtacgcga actgcgccgc 8820
tgggaaccgt gcaccgacgc caccgggcgg ccgtggcccc tgcgggccgg agtgagcagc 8880

ttcggcttcg gcggcgccaa cgcccatgtc gtcctcgaag caccgcccgt accgcccgca 8940
ccggcggagc cggcccgccc gaccgccccc caggccatcg tgctgtccgc ccgcgacgac 9000
gaccggctgc gtgccacggc cggacgactg cgggacttcc tcgaccgggc gcgccgcgac 9060
ggacacgccc cggacctggc ggacctggcg ttcaccctcc aggtaggccg ggaggccatg 9120
gaacggcgcc tgggcttcgt cgtcggaagc atggacgacg tgctcggtac gctggaccgg 9180
ttcttcgcgg gcgacgagcc ctccggctgg cacaccggcg gcatcaggcg gtcgcgtggc 9240
gccggagtgc ggcgcgaggc ggagcaggcc cccgaggtga cccgggccct ccacgacgga 9300
cggctcgacc gggtgacggc cctgtggtgc gacggcgccc cggtcgactg gcaggcgatg 9360
catcccacag gcgagcgccg cgccgtgcgg ctgcccgcgt acccccttcgc ctgcgaccgc 9420
tactgggtgc ccgcggtcgg cacagccccc gtcccgccgc ccgcggcacc cgtcccgccc 9480
cccgcggccg agcccgcgtt cgagaccgat gcccgtgcgg cgctgctcga cgcggtcctc 9540
gacggccgtg ccggcccgga cgccctgagc aggacctga 9579

<210> 12
<211> 8026
<212> PRT
<213> Streptomyces platensis subsp. rosaceus

<400> 12

Val Thr Trp Asn Gly Met Asn Val Ser Arg Asn Ile Leu Arg Val Pro
1 5 10 15

Glu Trp Arg Asp Glu Pro Ala Arg Gly Arg Thr Ala Pro Pro Gly Asn
20 25 30

Arg Arg Leu Val Val Leu Cys Asp Thr Pro Asp Ala Asp Val Thr Asp
35 40 45

Leu Arg Arg His Leu Pro Gly Val Ser Val Ala Arg Val Asp Ser Gly
50 55 60

Asp Asp Gly Pro Ala Ala Ala Tyr Glu His Ala Ala Thr Leu Leu Leu
65 70 75 80

Gly Glu Leu Gln Arg Leu Leu Asn Gln Pro Ala Gly Gly Pro Arg Ser
85 90 95

Val Gln Val Val Cys Arg Glu Gly Thr Pro Tyr Gly Tyr Ala Gly Leu
100 105 110

Ile Gly Met Leu Arg Thr Ala Ala Gln Glu Asp Pro Ala Leu His Gly
115 120 125

Gln Leu Ile Glu Cys Thr Gln Arg Pro Ser Gly Glu Glu Leu Ala Gly
130 135 140

Val Leu Arg Ala Glu Tyr Gly Gln Ala Ala Asp His Val Arg Tyr Thr

145 150 155 160

Gly Gly Arg Arg Gln Val Arg Ala Trp Ala Ala Ala Pro Arg Ala Ala
165 170 175

Ala Pro Pro Pro Val Trp Lys Ala Asp Gly Val Tyr Leu Ile Ser Gly
180 185 190

Gly Ala Gly Gly Val Gly Arg Leu Val Ala Ala Asp Ile Ala Arg His
195 200 205

Ala Pro Gly Ala Arg Val Val Leu Cys Gly Arg Ser Pro Ala Val Pro
210 215 220

Gly Pro Gly Gln Pro Gly Pro Gly Thr Glu Tyr Arg Arg Val Asp Val
225 230 235 240

Ala Asp Ala Asp Ala Val Ala Glu Leu Val Asn Ser Leu Val Arg Thr
245 250 255

Tyr Gly Arg Leu Asp Gly Val Val His Ala Ala Gly Leu Ile Ser Asp
260 265 270

Asp Tyr Val Ile Arg Lys Ser His Gln Asp Ala Gln Gln Val Leu Ala
275 280 285

Pro Lys Ala Ala Gly Leu Val Asn Leu Asp Glu Ala Thr Arg Arg Leu
290 295 300

Pro Leu Asp Phe Leu Ala Ala Phe Ser Ser Gly Ala Gly Thr Leu Gly
305 310 315 320

Asn Pro Gly Gln Ala Asp Tyr Ala Ala Ala Asn Gly Phe Leu Asp Ala
325 330 335

Tyr Leu Thr His Arg Ala Gly Leu Ala Ala Ala Gly Glu Arg His Gly
340 345 350

Ala Ser Val Ser Ile Gly Trp Pro Leu Trp Gln Asp Gly Gly Met Ser
355 360 365

Val Pro Ala Glu Asp Val Pro Ala Leu Thr Ala Arg Phe Gly Arg Pro
370 375 380

Leu Gly Thr Asp Thr Ala Leu Arg Ala Leu His Gly Ala Leu Ala Leu
385 390 395 400

Gly Thr Pro His Leu Leu Val Met Asp Glu Glu Ser Gly Val Asp Glu
405 410 415

Glu Ser Gly Val Asp Glu Glu Gly Pro Gln Glu Ala Glu Thr Gln Gln
420 425 430

Thr Gly Pro Ala Glu Leu Arg Ala His Val Leu Pro Leu Leu Lys Glu
435 440 445

Leu Ile Ala Glu Thr Val Arg Leu Asp Pro Ala Arg Leu Asp Ala Ala
450 455 460

Ala Pro Leu Asp Gly Phe Gly Ile Asp Ser Leu Ala Val Thr Arg Leu
465 470 475 480

Asn Arg Arg Phe Ala Gln Trp Phe Gly Ala Leu Pro Lys Thr Val Leu
485 490 495

Tyr Gln Tyr Pro Thr Leu Asn Asp Leu Ala Gly His Leu Ala Glu Gln
500 505 510

His Ala Asp Gly Cys Arg Arg Trp Leu Gly Asp Val Pro Asp Val Ala
515 520 525

Ala Ala Pro Ala Gly Thr Pro Ala Thr Ala Ala Ala Pro Arg Lys Ala
530 535 540

Arg Pro Arg Pro Ala Asp Ala Asp Glu Pro Ile Ala Leu Ile Gly Leu
545 550 555 560

Ser Gly Arg Tyr Pro Asp Ala Pro Thr Leu Glu Ala Phe Trp Glu Asn
565 570 575

Leu Arg Ala Gly Arg Glu Ser Val Arg Glu Val Pro Ala Glu Arg Trp
580 585 590

Pro Leu Asp Ala Phe Tyr Glu Pro Asp Pro Gln Arg Ala Val Gln Gln
595 600 605

Gly Ala Ser Tyr Ser Lys Trp Gly Ala Phe Leu Asp Asp Phe Ala Arg
610 615 620

Phe Asp Ala Ala Phe Phe Gly Ile Ala Pro Arg Asp Ala Ala Asp Met
625 630 635 640

Asp Pro Gln Glu Arg Leu Phe Val Glu Ser Ala Trp Ser Val Leu Glu
645 650 655

Asp Ala Gly Tyr Thr Arg Gln Arg Leu Ala Glu Gln His Ala Ser Ser
660 665 670

Val Gly Val Phe Ala Gly Ile Thr Lys Thr Gly Phe Asp Arg His Arg
675 680 685

Pro Pro Ala Thr Asp Gly Leu Pro Pro Ala Pro Arg Thr Ser Phe Gly
690 695 700

Ser Leu Ala Asn Arg Val Ser Tyr Leu Leu Asp Leu His Gly Pro Ser
705 710 715 720

Met Pro Ile Asp Thr Met Cys Ser Ser Ser Leu Thr Ala Ile His Glu
725 730 735

Ala Cys Glu His Leu Arg His Gly Ala Cys Glu Leu Ala Ile Ala Gly
740 745 750

Gly Val Asn Leu Tyr Leu His Pro Ser Ser Tyr Val Glu Leu Cys Arg
755 760 765

Ser Arg Met Leu Ala Thr Asp Gly His Cys Arg Ser Phe Gly Ala Gly
770 775 780

Gly Asp Gly Phe Leu Pro Gly Glu Gly Val Gly Ala Val Leu Leu Lys
785 790 795 800

Pro Leu Ser Ala Ala Glu Ala Asp Gly Asp Pro Ile His Ala Val Ile
805 810 815

Val Gly Ser Ala Ile Asn His Gly Gly Arg Thr Asn Gly Tyr Thr Val
820 825 830

Pro Asn Pro Arg Ala Gln Ala Ala Leu Ile Arg Asp Ala Leu Asp Arg
835 840 845

Ala Gly Val Ser Ala Ala Gly Ile Gly Tyr Ile Glu Ala His Gly Thr
850 855 860

Gly Thr Arg Leu Gly Asp Pro Val Glu Ile Asp Gly Leu Thr Gln Ala
865 870 875 880

Phe Ala Pro Asp Ala Gly Gly Ser Gly Ala Cys Ala Leu Gly Ser Val
885 890 895

Lys Ser Asn Ile Gly His Leu Glu Ala Ala Ala Gly Ile Ala Gly Leu
900 905 910

Thr Lys Ala Val Leu Gln Leu Gln His Gly Glu Phe Ala Pro Thr Leu
915 920 925

His Ala Glu Gln Thr Asn Pro Asp Ile Asp Phe Ala Ala Thr Pro Phe
930 935 940

Thr Leu Gln Thr Gly Gly Ala Pro Trp Pro Arg Pro Ala Asp Gly Gly
945 950 955 960

Pro Arg Arg Ala Gly Ile Ser Ser Phe Gly Ala Gly Gly Ala Asn Ala
965 970 975

His Val Ile Val Ala Glu Tyr Arg Ser Ala Thr Pro Ala Pro Ala Thr
980 985 990

Pro Ala Pro Ser Ala Arg Pro Val Leu Leu Pro Leu Ser Ala Arg Thr
995 1000 1005

Thr Glu Asp Leu His Ala Arg Ala Gly Gln Leu Ser Asp Leu Leu
1010 1015 1020

Arg Asn Gly Ala Pro Val Asp Leu Pro Ala Val Ala Ala Thr Leu
1025 1030 1035

Gln Thr Gly Arg Glu Glu Met Ala Glu Arg Val Cys Phe Val Ala
1040 1045 1050

Ser Thr Pro Gly Glu Trp Leu Asp Gln Leu Gly Ala Phe Leu Ala
1055 1060 1065

Asp Ser Asp Ser Asp Ser Asp Ser Asp Ser Asp Ser Asp Ser Asp
1070 1075 1080

Ser Asp Ser Asp Ser Asp Ser Gly Ser Gly Ser Glu Ala Glu Ala
1085 1090 1095

Glu Val Pro Trp Ser Arg Gly Arg Val Arg Ala Thr Arg Glu Thr
1100 1105 1110

Leu Ala Ala Leu Ala Glu Lys Asp Glu Leu Arg Ala Leu Val Thr

1115 1120 1125

Arg Trp Ile Asn Arg Gly Asp Trp His Asp Leu Ala Ala Phe Trp
1130 1135 1140

Ala Lys Gly Met Pro Leu Asp Trp Thr Arg Leu His Ala Gly Ala
1145 1150 1155

Asp Thr Pro Ala Arg Val His Leu Pro Ala Tyr Pro Phe Ala Gly
1160 1165 1170

Arg Gln Phe Trp Phe Gly Pro Ala Gly Ser Glu His Pro Ala Thr
1175 1180 1185

Thr Pro Val Ala Ala Pro Ser Cys Ser Thr Ala Ala Gly Ala Ala
1190 1195 1200

Asp Val Glu Arg Ile Leu Leu Asp Ala Leu Ala Ala Ala Leu Gln
1205 1210 1215

Met Pro Val Ala Glu Ile Glu Arg Arg Arg Pro Phe Ala Asp Tyr
1220 1225 1230

Gly Leu Asp Ser Ile Leu Gly Val Asn Leu Val His Thr Leu Asn
1235 1240 1245

Thr Ala Leu Gly Thr Ala Leu Glu Thr Thr Asp Leu Phe Asp His
1250 1255 1260

Gly Thr Val Glu Arg Leu His Ala Phe Leu Val Gly Thr Tyr Gly
1265 1270 1275

Asp Ala Leu His Ala Pro Ala Ser Pro Ala Ala Val Ala Pro Ala
1280 1285 1290

Pro Asp Asp Asp Ala Ile Ala Val Val Gly Met Ala Ala Arg Tyr
1295 1300 1305

Ala Asp Ala Glu Asp Pro Arg Ala Leu Trp Asp His Leu Met Ala
1310 1315 1320

Gly His Asp Leu Val Glu Pro Val Thr Arg Trp Pro Leu Gly Gln
1325 1330 1335

Asp Val Ser Cys Arg Ser Gly Ser Phe Val Arg Gly Ile Asp Gln
1340 1345 1350

Phe Asp Pro Val Phe Phe Ala Ile Ser Gly Val Glu Ala Thr Thr
1355 1360 1365

Met Asp Pro Gln Gln Arg Ile Phe Leu Glu Gln Cys Trp Asn Ala
1370 1375 1380

Leu Glu Asp Ala Gly Tyr Thr Gly Glu Arg Leu Thr Asn Arg Asn
1385 1390 1395

Cys Gly Val Tyr Ala Gly Cys Tyr Ala Gly Asp Tyr His Asp Gln
1400 1405 1410

Leu Asp Ala Arg Pro Pro Ala Gln Ala Leu Trp Gly Thr Met Gly
1415 1420 1425

Ser Val Val Ala Ser Arg Ile Ala Tyr His Leu Asp Leu Lys Gly
1430 1435 1440

Pro Ala Leu Thr Thr Asp Thr Ser Cys Ser Ser Ser Leu Val Ser
1445 1450 1455

Leu His Leu Ala Cys Arg Asp Leu Leu Ser Gly Asp Ala Asp Met
1460 1465 1470

Ala Ile Ala Gly Gly Val Phe Ile Gln Thr Thr Ser Arg Leu Tyr
1475 1480 1485

Glu Ser Ala Ser Arg Ala Gly Met Leu Ser Pro Ser Gly Arg Cys
1490 1495 1500

His Ser Phe Asp Ala Arg Ala Asp Gly Phe Val Pro Gly Glu Gly
1505 1510 1515

Ala Gly Ala Val Val Leu Lys Arg Leu Ala Asp Ala Arg Arg Asp
1520 1525 1530

Gly Asp His Ile Tyr Gly Val Val Arg Gly Ser Gly Ile Asn Gln
1535 1540 1545

Asp Gly Thr Thr Asn Gly Ile Thr Ala Pro Ser Ala Ala Ser Gln
1550 1555 1560

Glu Gln Leu Leu Arg Asp Val His Ala Arg Ser Gly Ile Glu Pro
1565 1570 1575

Gly Gly Ile Gln Leu Val Glu Ala His Gly Thr Gly Thr Gln Leu
1580 1585 1590

Gly Asp Pro Ile Glu Phe Arg Ala Leu Thr Arg Ala Phe Glu Asp
1595 1600 1605

Ala Pro Ala Gly Ser Ala Val Leu Gly Ser Ile Lys Thr Asn Ile
1610 1615 1620

Gly His Thr Gln Phe Ala Ala Gly Ile Ala Gly Val Ile Lys Ala
1625 1630 1635

Leu Leu Ala Leu Glu His Arg Gln Ile Pro Pro Ser Leu His Phe
1640 1645 1650

Gln Glu Ala Asn Arg Ala Val Val Leu Asp Gly Gly Pro Phe Thr
1655 1660 1665

Val Thr Thr Ala Pro Gln Pro Trp Thr Ala Pro Ala Arg Gly Pro
1670 1675 1680

Arg Arg Ala Ala Val Ser Ser Phe Gly Ala Ser Gly Thr Asn Ala
1685 1690 1695

His Val Val Leu Glu Glu His Pro Val Pro Arg Thr Thr Gly Ala
1700 1705 1710

Gly Gly Glu His Ala Phe Leu Leu Ser Ala Arg Thr Pro Ala Ala
1715 1720 1725

Leu Arg Ala Val Ala Glu Arg Leu Leu Ala His Leu Asp Arg Glu
1730 1735 1740

Pro Gly Leu Pro Ala Asp Ala Val Ala Phe Ser Leu Ala Ala Gly
1745 1750 1755

Arg Ser His Phe Ala His Arg Leu Ala Val Val Ala Ala Gly Leu
1760 1765 1770

Pro Asp Leu Ala Ala Arg Leu Arg Ser Trp Leu Ser Gly Thr Ala
1775 1780 1785

Gly Asp Thr Val Leu Gln Gly Glu Thr Ala Ala Asp Pro Arg Pro
1790 1795 1800

Val Gly Gly Val Arg Ala Pro Ala Pro Ala Ala Leu Ala Ala Ala
1805 1810 1815

Tyr Val Arg Gly Glu Ala Asp Arg Phe Ala Asp Ser Phe Ala Ser
1820 1825 1830

Ala Ser Arg Arg Gln Val Pro Leu Pro Thr Tyr Pro Phe Glu Arg
1835 1840 1845

Gln Arg Tyr Trp Thr Asp Thr Thr Asp Thr Gly Glu Ser Gln Gly
1850 1855 1860

Leu Lys Asp Thr Asp Gly Ala Ala Tyr Arg Leu Arg Leu Gly Gly
1865 1870 1875

Glu Glu Phe Phe Leu Ala Asp His His Val Gly Gly Arg Ala Val
1880 1885 1890

Leu Pro Gly Val Leu Ser Leu Glu Phe Ala Arg Arg Ala Val Thr
1895 1900 1905

Gly Gly Ser Phe Ala Pro Val Gly Leu Arg Asp Val Val Trp Pro
1910 1915 1920

Glu Pro Phe Pro Val Gly Asp Gly Gly Ala Glu Leu Arg Val Asp
1925 1930 1935

Arg Asp Gly Asp Ala Phe Arg Val Leu Arg Asp Gly Ser Ala Val
1940 1945 1950

His Ala Gln Gly Arg Ile Ala Thr Pro Gly Ser Pro Val Pro Thr
1955 1960 1965

Pro Leu Asp Ala Leu Arg Ala Arg Cys Gly Arg Arg Thr Leu Ser
1970 1975 1980

Arg Ser Gln Cys Arg Ala Ala Leu Asp Ala Val Gly Ile Arg His
1985 1990 1995

Gly Asp Arg Leu Arg Ala Ile Asp Thr Leu Ala Val Gly Asp Gly
2000 2005 2010

Glu Val Leu Ala Arg Leu Val Leu Pro Asp Gly Ala Arg Asp Gly
2015 2020 2025

Ala Phe Ala Leu His Pro Ala Met Leu Asp Ser Ala Val Gln Ala

2030 2035 2040

Val Val Gly Leu Tyr Gly Asp Ala Thr Gly Thr Leu Asp Glu Gln
2045 2050 2055

Arg Gly Ala Pro Ala Leu Pro Phe Ala Leu Asp Ala Ala Asp Phe
2060 2065 2070

Phe Ala Pro Thr Thr Glu Arg Met Trp Ala His Leu Arg His Thr
2075 2080 2085

Glu Gly Tyr Thr Pro Ser Ala Asp Arg Asp Val Thr Lys Val Asp
2090 2095 2100

Ile Asp Val Tyr Asp Asp Asp Gly Gln Leu Ser Ala Ser Leu Arg
2105 2110 2115

Gly Tyr Ala Phe Arg Arg Met Thr Ala Pro Ser Gly Ala Ala Pro
2120 2125 2130

Arg Ala Thr Leu Leu Ala Pro Val Trp Asp Ala Leu Pro Val Val
2135 2140 2145

Pro Ala Glu Pro Trp Pro His Pro Arg Thr Arg Val Val Leu Leu
2150 2155 2160

Gly Gly Thr Pro Glu Glu Arg Asp Gly Leu Arg Arg Arg Tyr Pro
2165 2170 2175

Asp Ala Thr Val Leu Asp Pro His Ala Asp Glu Pro Val Asp Arg
2180 2185 2190

Leu Ala Ala Arg Leu Pro Ala Asp Ala Glu His Val Phe Trp Leu
2195 2200 2205

Ala Pro Ala Gly Pro Thr Gly Ala Pro Ala Ala Ala Arg Tyr Asp
2210 2215 2220

Gly Thr Ile Ala Val Phe Arg Leu Val Lys Ala Leu Leu Ala Asp
2225 2230 2235

Gly Ala Asp Ala Arg Glu Leu Gly Leu Thr Leu Val Thr Arg Gln
2240 2245 2250

Ala Arg Leu Leu Pro Gly Asp Thr Gly Ala Asp Pro Ala His Ala
2255 2260 2265

Gly Val His Gly Leu Ala Gly Thr Leu Ala Lys Glu Tyr Pro His
2270 2275 2280

Trp Arg Ile Arg Val Ala Asp Val Glu Ala Asp Ala Ala Val Pro
2285 2290 2295

Trp Pro Ala Leu Leu Ala Leu Pro Thr Asp Pro Arg Gly Glu Thr
2300 2305 2310

Leu Ala His Arg His Gly Glu Trp Tyr Arg Leu Arg Leu Leu Glu
2315 2320 2325

Thr Asp Gly Thr Gly Val Ala Ala Ala Pro Arg Glu Pro Gly Gly
2330 2335 2340

Val Ile Val Ala Ile Gly Gly Ala Gly Gly Ile Gly Thr Val Trp
2345 2350 2355

Thr Glu His Met Met Arg Arg His Gly Ala Arg Val Val Trp Ile
2360 2365 2370

Gly Arg Arg Pro Leu Asp Ala Ala Ile Ala Ala Gln Gln Glu Ala
2375 2380 2385

Leu Ala Ala His Gly Pro Lys Pro Asp Tyr Val Gln Ala Asp Ala
2390 2395 2400

Thr Asp Arg Asp Ala Leu Arg Arg Ala Cys Asp Glu Ile Val Arg
2405 2410 2415

Arg His Gly Pro Val Arg Gly Val Leu His Thr Ala Ile Val Leu
2420 2425 2430

Gly Asp Gln Thr Leu Ala Arg Met Asp Glu Asp Arg Phe Arg Thr
2435 2440 2445

Thr Tyr Ala Ala Lys Ala Asp Ile Ala Val Asn Leu Ala Asp Ala
2450 2455 2460

Phe Ala Gly Gln Pro Leu Glu Phe Val Ala Phe Phe Ser Ser Met
2465 2470 2475

Gln Ala Phe Phe Lys Ala Pro Gly Gln Ala Asn Tyr Ala Ala Gly
2480 2485 2490

Cys Thr Phe Ala Asp Ala Tyr Ala Glu His Leu Ser Thr Arg Leu
2495 2500 2505

Asp Cys Pro Val Lys Val Met Asn Trp Gly Tyr Trp Ala Gly Val
2510 2515 2520

Gly Val Val Thr Ala Asp Gly Tyr Arg Gln Arg Met Ala Gln Leu
2525 2530 2535

Gly Leu Gly Ser Ile Glu Pro Asp Glu Gly Met Ala Ala Phe Asp
2540 2545 2550

Thr Leu Leu Ala Ser Pro Tyr Pro Gln Leu Ala Leu Leu Lys Ala
2555 2560 2565

Thr Asp Thr Arg Ser Ile Asp Gly Leu His Asp Asp Asp Ala Leu
2570 2575 2580

Thr His Pro Val Val Thr Thr Pro Ser Leu Ile Gly Ala Leu Gly
2585 2590 2595

Glu Asp Cys Pro Asp Arg Arg Ala Glu Ile Ala Gln Leu Arg Glu
2600 2605 2610

Lys Ala Gly Gly His Ala Gly Ala Met Gln Asp Ala Leu Val Arg
2615 2620 2625

Ile Thr Trp Ala Leu Leu Gln Ser Leu Gly Leu Phe Arg Asp Gly
2630 2635 2640

Arg Ala Ala Thr Ala Ala Glu Trp Arg Ala Leu Gly Gly Ile Glu
2645 2650 2655

Asp Arg Tyr Glu Arg Trp Thr Glu His Thr Leu Ala Val Leu Ala
2660 2665 2670

Asp Ala Gly Leu Leu Arg Arg Glu Gly Glu Asp Thr Tyr Val Ala
2675 2680 2685

Leu Asp Thr Arg Thr Gly Ser Leu Asp Asp Ala Trp Ala Asp Trp
2690 2695 2700

Asp Arg Ala Arg Gln Gln Trp Leu Ala Asp Asp Ala Lys Arg Pro
2705 2710 2715

Gln Ala Val Leu Val Asp Thr Thr Leu Arg Ala Met Thr Gly Ile
2720 2725 2730

Leu Thr Gly Arg Arg Pro Ala Thr Asp Val Met Phe Pro Asn Ala
2735 2740 2745

Trp Leu Glu Leu Val Glu Ala Val Tyr Lys Asn Asn Pro Val Ala
2750 2755 2760

Asp Tyr Phe Asn Asp Val Leu Ala Asp Thr Leu Val Gly Tyr Leu
2765 2770 2775

Glu Arg Arg Leu Ala Asp Asp Pro Ser Ala Arg Leu Arg Ile Leu
2780 2785 2790

Glu Ile Gly Ala Gly Thr Gly Gly Thr Ser Ala Thr Val Leu Arg
2795 2800 2805

Arg Leu Arg Pro Trp Ala Arg His Ile Glu Lys Tyr Thr Tyr Thr
2810 2815 2820

Asp Ile Ser Lys Ala Phe Leu Leu Tyr Gly Gln Arg Glu Tyr Gly
2825 2830 2835

Glu Ile Ala Pro Tyr Leu Asp Ala Arg Leu Phe Asn Ala Glu Lys
2840 2845 2850

Pro Leu Ala Gly Gln Glu Val Asp Pro Gly Ala Tyr Asp Val Val
2855 2860 2865

Ile Ala Thr Asn Val Leu His Ala Thr Arg Asn Ile Arg Arg Thr
2870 2875 2880

Leu Arg Asn Ala Lys Ala Ala Ala Arg Pro Asn Ala Leu Leu Leu
2885 2890 2895

Leu Asn Glu Leu Ser Asp Asn Ile Leu Phe Ser His Leu Thr Phe
2900 2905 2910

Gly Leu Leu Asp Gly Trp Trp Leu Tyr Asp Asp Pro Ala Pro Arg
2915 2920 2925

Ile Pro Gly Ser Pro Gly Leu Ala Pro Glu Ser Trp Arg Arg Val
2930 2935 2940

Leu Gly Glu Val Gly Phe Arg Ala Ala Phe Val Ala Ala Gly Gly

2945 2950 2955

Ala Asp Asp Leu Gly Gln Gln Val Ile Val Ala Glu Ser Asp Gly
2960 2965 2970

Ala Ile Arg Gln Pro Arg Pro Asp Gly Glu Ser Ala Phe Arg Gly
2975 2980 2985

Thr Leu Pro Glu Ala Gly Pro Arg Ala Ala Glu Pro Gln Leu Pro
2990 2995 3000

Ala Pro Thr Pro Asp Pro Val Ala Ala Asp Gly Val Arg Asp Asp
3005 3010 3015

Glu Leu Leu Ala Asp Leu Ala Arg Asp His Phe Arg Thr Leu Val
3020 3025 3030

Ala Asp Thr Leu Gln Leu Pro Val Ala Asp Ile Arg Ala Asp Val
3035 3040 3045

Pro Phe Asp Arg Tyr Gly Ile Asp Ser Ile Leu Val Val Gln Leu
3050 3055 3060

Thr Glu Ala Val Arg Lys Gly Leu Cys Asn Val Gly Ser Thr Leu
3065 3070 3075

Phe Phe Glu Val Arg Thr Val Asp Gly Leu Val Gln His Phe Leu
3080 3085 3090

Arg Thr Gln Pro Asp Ala Leu Ala Ala Leu Val Gly Leu Ser Gly
3095 3100 3105

Ala Arg Ala Ala Arg Thr Asp Glu Gln Leu Ala Pro Ala Ala Gly
3110 3115 3120

Pro Glu Pro Val Pro Val Ile Ala Ala Glu Pro Pro Arg Ala Glu
3125 3130 3135

Gln Gly Met Ala Ile Ala Ile Val Gly Met Ala Gly Arg Tyr Pro
3140 3145 3150

Gly Ala Pro Asp Leu Asp Thr Phe Trp Glu Asn Leu Leu Ala Gly
3155 3160 3165

Arg Asp Ser Ile Thr Glu Ile Pro Ala Gly Arg Trp Asp His Ser
3170 3175 3180

Arg Tyr Tyr Asp Ala Arg Arg Gly Val Pro Gly Arg Thr Tyr Ser
3185 3190 3195

Lys Trp Gly Gly Phe Leu Asp Gly Ile Asp Glu Phe Asp Pro Leu
3200 3205 3210

Phe Phe Gly Ile Ser Pro Lys Ala Ala Ser Thr Met Asp Pro Gln
3215 3220 3225

Glu Arg Leu Phe Leu Gln Cys Ala His Thr Thr Leu Glu Asp Ala
3230 3235 3240

Gly Tyr Ser Arg Gly Ala Leu Arg Ala Ala Ala Arg Ala Arg Val
3245 3250 3255

Ala Glu Asp Ala Gly Asp Ile Gly Val Phe Ala Gly Ala Met Tyr
3260 3265 3270

Ser Glu Tyr Gln Leu Tyr Gly Ala Glu Tyr Ser Val Arg Gly Glu
3275 3280 3285

Pro Val Val Val Pro Gly Ser Leu Ala Ser Ile Ala Asn Arg Val
3290 3295 3300

Ser Tyr Phe Leu Asp Ala Ser Gly Pro Ser Val Thr Val Asp Thr
3305 3310 3315

Met Cys Ala Ser Ala Leu Ser Ala Ile His Leu Ala Cys Ala Ala
3320 3325 3330

Leu Gln Arg Gly Glu Cys Gly Val Ala Leu Ala Gly Gly Val Asn
3335 3340 3345

Leu Ser Val His Pro Gly Lys Tyr Leu Met Ile Gly Glu Gly Gln
3350 3355 3360

Phe Ala Ser Ser Asp Gly Arg Cys Arg Ser Phe Gly Glu Gly Gly
3365 3370 3375

Asp Gly Tyr Val Pro Gly Glu Gly Val Gly Ala Val Leu Leu Arg
3380 3385 3390

Pro Leu Ala Asp Ala Val Ala Asp Gly Asp Arg Ile Leu Gly Val
3395 3400 3405

Ile Arg Gly Thr Ala Val Asn His Gly Gly His Thr His Gly Phe
3410 3415 3420

Thr Val Pro Asn Pro Leu Ala Gln Ala Ala Val Ile Arg Ser Ala
3425 3430 3435

Trp Arg Arg Ala Gly Val Asp Pro Arg Asp Ile Gly Cys Ile Glu
3440 3445 3450

Ala His Gly Thr Gly Thr Ser Leu Gly Asp Pro Ile Glu Ile Ala
3455 3460 3465

Gly Leu Asn Ala Ala Phe Ala Glu Phe Thr Asp Ala Arg Asn Phe
3470 3475 3480

Cys Ala Ile Gly Ser Ala Lys Ser Asn Ile Gly His Leu Glu Ser
3485 3490 3495

Ala Ala Gly Ile Ala Gly Leu Ala Lys Leu Leu Leu Gln Met Arg
3500 3505 3510

His Gly Thr Leu Val Pro Ser Leu His Ala Glu Arg Val Asn Pro
3515 3520 3525

Asp Ile Asp Phe Ala Asp Ser Pro Phe Val Leu Gln Arg Glu Ala
3530 3535 3540

Ala Pro Trp Pro Arg Thr Gly Thr Arg Pro Arg Leu Gly Gly Leu
3545 3550 3555

Ser Ser Phe Gly Ala Gly Gly Ser Asn Ala His Val Val Val Glu
3560 3565 3570

Asp Tyr Val Glu Glu His Ala Gly Lys Asp Leu Ala Pro Glu Ala
3575 3580 3585

His Arg Gly Glu Thr Val Val Val Val Leu Ser Ala Phe Asp Glu
3590 3595 3600

Glu Arg Leu Arg Glu Ser Ala Gly Arg Leu Arg Asp Ala Leu Arg
3605 3610 3615

Lys Glu Arg Trp Ser Ser Ala Asp Leu Pro Asp Ile Ala Tyr Thr
3620 3625 3630

Leu Gln Val Gly Arg Glu Ala Met Thr Ala Arg Phe Ala Val Ala
3635 3640 3645

Val Ser Thr Leu Pro Ala Leu Val Asp Ala Leu Asp Ala Cys Ala
3650 3655 3660

Leu Gly Ser Gly Leu Pro Ala Gly Ala Tyr Phe Asn Pro Gly Gly
3665 3670 3675

Asp Arg Gly Gly Ala Val Lys Asp Phe Leu Thr Asp Glu Asp Phe
3680 3685 3690

Gln Glu Thr Ala Val Arg Trp Ala Arg Arg Gly Lys Pro Ala Pro
3695 3700 3705

Leu Ala Glu Ala Trp Thr Ser Gly Leu Ala Val Asp Trp Ala Arg
3710 3715 3720

Leu His Thr Glu Gly Pro Lys Pro Arg Lys Val Ala Leu Pro Gly
3725 3730 3735

Tyr Pro Phe Ala Arg Glu Arg Tyr Trp Tyr Thr Asp Gly Leu Pro
3740 3745 3750

Glu Leu Gln Glu Ile Pro Ala Thr Phe Gly Asn Ala Ala Arg Gln
3755 3760 3765

Pro Ala Ala Pro Pro Pro Ala Val Glu Ala Ala Pro Ala Thr Thr
3770 3775 3780

Ser Ala Val Pro Ala Pro Pro Ala Arg Pro Ala Asn Ser Tyr Glu
3785 3790 3795

Leu Pro Ala Gly Asp Leu Thr Leu His Pro Val Trp Glu Pro Val
3800 3805 3810

Arg Leu Leu Arg Gly Ser Pro Tyr Pro Ser Ala Ala Ser Arg Val
3815 3820 3825

Val Ala Ile Gly Leu Ala Pro Asp Ala Leu Ala Glu Leu Thr Ala
3830 3835 3840

Arg Arg Pro Gln Thr Val Val Leu Asp Thr Ala Ala Ser Ser Ala
3845 3850 3855

Glu Glu Val Arg Asp Glu Leu Ala Val Leu Gly Asp Phe Asp His

3860 3865 3870

Val Val Met Arg Phe Pro Thr Ala Ala Ala Ala His Gly Ala Glu
3875 3880 3885

Ala Gln Ile Ser Thr Gln Arg Ala Ala Ile Arg Ser Met Phe Arg
3890 3895 3900

Val Leu Lys Ala Leu Ala Leu Thr Arg Asp Glu Gln Arg Leu Gly
3905 3910 3915

Leu Thr Leu Leu Thr Ser Gly Ala Phe Asp Ala Gly Gly Ser Gly
3920 3925 3930

Thr Ala Asp Pro Ala Gln Ala Ser Leu His Gly Leu Leu Gly Gly
3935 3940 3945

Leu Ala Lys Glu Gln Pro His Trp Arg Ile Arg Ala Val Asp Leu
3950 3955 3960

Ala Asp Gly Glu Pro Phe Val Ala Asp Glu Val Phe Ala Leu Pro
3965 3970 3975

Ala Asp Arg Arg Ala His Pro Leu Val Arg Arg Gly Gly Gln Trp
3980 3985 3990

Leu Arg Arg Gln Leu Leu Pro Val Asp Ala Thr Glu Pro Pro Ala
3995 4000 4005

Glu Pro Val Leu Arg Arg Asp Gly Val Tyr Val Leu Ile Gly Gly
4010 4015 4020

Ala Gly Asp Leu Gly Val Leu Leu Ser Glu Tyr Leu Val Arg Gln
4025 4030 4035

His Asp Ala His Val Val Trp Val Gly Arg Arg Ala Glu Asp Glu
4040 4045 4050

Asp Ile Arg Ala Arg Ala Asp Arg Ala Ala Ala Gly Gly Arg Thr
4055 4060 4065

Pro Val Tyr Leu Ser Ala Asp Ala Ser Asp Pro Asp Ala Leu Ala
4070 4075 4080

Arg Met Arg Asp Glu Val Val Arg Arg Tyr Gly Arg Ile Asp Gly
4085 4090 4095

Val Val His Leu Ala Met Val Phe Ser His Thr Pro Leu Ala Arg
4100 4105 4110

Met Thr Glu Arg Glu Leu Glu Ala Thr Leu Ala Ala Lys Val Asp
4115 4120 4125

Pro Cys Ala His Phe Ala Asp Val Phe Ala Gly His Gly Leu Asp
4130 4135 4140

Phe Val Leu Leu Ile Ser Ser Leu Val Ser Phe Ile Arg Asn Ser
4145 4150 4155

His Gln Ala His Tyr Ser Ala Ala Cys Ala Phe Glu Asp Ala His
4160 4165 4170

Ala Ala Ala Leu Arg Glu Ala Leu Asp Cys Arg Val Lys Val Val
4175 4180 4185

Asn Trp Gly Tyr Trp Gly Asn Val Pro Asp Glu Leu Leu Arg Asp
4190 4195 4200

Val Thr Ser Met Gly Leu Ala Pro Ile Ala Pro Ala Thr Ala Met
4205 4210 4215

Gly Ala Leu Glu Arg Leu Leu Ala Gly Pro Leu His Gln Ile Gly
4220 4225 4230

Phe Met Arg Leu Gly Arg Pro Leu Pro Val Glu Gly Val Leu Thr
4235 4240 4245

Ala Glu Thr Leu Thr Pro Gln Thr His Gly Ala Ala Ala Arg Asp
4250 4255 4260

Gly Ala Ala Ala Leu Ala Leu Pro Thr Gly Leu Ala Ala Tyr His
4265 4270 4275

Glu Ser Pro Val Pro Gly Glu Ile Asp Ala Phe Leu Leu Arg Arg
4280 4285 4290

Leu Ala Ala Glu Leu Arg Arg Ala Gly Leu Glu Glu Pro Arg His
4295 4300 4305

Gly Leu Ala Glu Trp Lys Glu Arg Gln Gly Val Asp Ala Arg Phe
4310 4315 4320

Asp Gly Trp Leu Ser Ala Thr Leu His Ala Leu Ala Glu His Ala
4325 4330 4335

Met Ile Asp Asp Arg Gly Arg Trp Thr Thr Ser Ser Pro Ala Ala
4340 4345 4350

Thr Asp Ala Asp Ala Cys Arg Ala Asp Trp Ala Ala Gln Thr Pro
4355 4360 4365

Arg Trp Ala Ala Ala Asn Pro Asp Leu Arg Ala Pro Leu Asn Leu
4370 4375 4380

Leu Asp Arg Thr Leu Pro Ala Leu Pro Asp Val Leu Cys Gly Arg
4385 4390 4395

Val Arg Ala Thr Asp Val Leu Phe Pro Gln Gly Lys Phe Ser Leu
4400 4405 4410

Val Glu Gly Val Tyr Arg Asp Asn Arg Val Ala Ala His Phe Asn
4415 4420 4425

Ala Val Leu Ala Glu His Val Ala Ala Phe Leu Arg Ala Arg Arg
4430 4435 4440

Asp Ala Asp Pro Gly Ala Arg Leu Arg Val Leu Glu Ile Gly Ala
4445 4450 4455

Gly Thr Gly Gly Thr Thr Gly Pro Val Leu Asp Arg Leu Ala His
4460 4465 4470

Glu Gly Leu Asp Leu Ala Glu Tyr Cys Phe Thr Asp Leu Ser Gln
4475 4480 4485

Ala Phe Leu Gln Asn Ala Gln Asp Thr Phe Gly Pro Gly Arg Asp
4490 4495 4500

His Leu Thr Tyr Arg Ile Phe Asp Ala Ala Arg Pro Pro His Thr
4505 4510 4515

Gln Gly Leu Asp Thr Gly Ala Phe Asp Val Val Ile Ala Ala Asn
4520 4525 4530

Val Leu His Ala Thr Asp Thr Ile Arg Pro Ala Leu Arg His Ala
4535 4540 4545

Lys Ala Leu Leu Arg Gly Asn Gly Leu Leu Ala Leu Asn Glu Ile
4550 4555 4560

Ser Gly Phe Tyr Leu Val Asn His Leu Thr Phe Gly Leu Leu Asp
4565 4570 4575

Gly Trp Trp Leu Tyr Asp Asp Ala Glu Leu Arg Val Pro Gly Ser
4580 4585 4590

Pro Ala Leu Ser Pro Ala Ala Trp Gln Leu Val Leu Glu Gln Glu
4595 4600 4605

Gly Phe Thr Gly Ile Arg His Pro Ala Arg Asp Ala Leu Ala Leu
4610 4615 4620

Gly Gln Gln Val Val Val Ala His Ser Asp Gly Leu Ala Arg Ser
4625 4630 4635

Pro Arg Leu Leu Ser Gly Thr Pro Glu Met Ser Ser Pro Pro Ser
4640 4645 4650

Gln Pro Pro Ala Glu Thr Ala Ala Pro Ala Ala Ala Ser Ala Ser
4655 4660 4665

Ala Arg Ala Val Thr Asp Val Val Leu Ala Ala Leu Ala Asp Ala
4670 4675 4680

Leu Arg Met Pro Ala Asp Arg Ile Gly Pro Asp Arg Ala Phe Ala
4685 4690 4695

Asp Tyr Gly Leu Asp Ser Ile Val Gly Val Arg Phe Val Gln Arg
4700 4705 4710

Leu Asn Glu Glu Leu Gly Thr Asp Leu Pro Thr Thr Val Val Phe
4715 4720 4725

Asp Tyr Arg Ser Val Ala Gln Leu Ala Ala His Ile Ala Glu Ser
4730 4735 4740

His Arg Pro Gln Pro Ala Pro Ala Ala Ala Ala Pro Val Pro Ala
4745 4750 4755

Pro Asp Ala Ala Gly Ala Pro Asn Arg Pro Glu Gly Arg Glu Pro
4760 4765 4770

Ile Ala Ile Val Gly Ile Ser Gly Arg Phe Ala Gln Ser Asp Asp

4775 4780 4785

Thr Asp Ala Leu Trp Gln His Leu Ala Ala Gly Arg Asp Leu Val
4790 4795 4800

Gly Pro Val Glu Arg Trp Asp Leu Ser Gly Tyr Ser Gln Asp Gln
4805 4810 4815

Leu Ser Cys Arg Ala Gly Ser Phe Leu Asp Gly Ile Asp Arg Phe
4820 4825 4830

Asp Ala Arg Phe Phe His Leu Thr Gly Leu Glu Ala Thr Tyr Thr
4835 4840 4845

Asp Pro Gln Gln Arg Leu Phe Leu Glu Gln Ala Trp Thr Ala Ile
4850 4855 4860

Glu Asp Ala Gly Tyr Ala Gly Ser Ala Leu Asp Gly Arg Arg Cys
4865 4870 4875

Gly Val Tyr Ala Gly Cys Thr Gly Gly Asp Tyr Pro Gln Trp Phe
4880 4885 4890

Glu Asp Ala Pro Pro Ala Gln Ala Ala Trp Gly Asn Ala Pro Ser
4895 4900 4905

Val Val Pro Ala Arg Ile Ala Tyr His Leu Asn Leu Gln Gly Pro
4910 4915 4920

Ala Leu Ala Val Asp Thr Ala Cys Ser Ser Ser Leu Val Ala Val
4925 4930 4935

His Leu Ala Cys Gln Gly Leu Trp Ser Gly Glu Thr Asp Met Ala
4940 4945 4950

Leu Ala Gly Gly Val Ser Val Gln Thr Thr Pro Asp Thr Tyr Leu
4955 4960 4965

Ala Ala Gly Arg Gly Gly Met Leu Ser Pro Thr Gly Lys Cys His
4970 4975 4980

Thr Phe Asp Ala Ala Ala Asp Gly Phe Val Pro Gly Glu Gly Val
4985 4990 4995

Gly Val Val Val Leu Arg Arg Leu Ser Asp Ala Leu Ala Asp Gly
5000 5005 5010

Asp His Ile His Ala Val Ile Arg Gly Ser Ala Val Asn Gln Asp
5015 5020 5025

Gly Ala Thr Asn Gly Ile Thr Ala Pro Ser Ala Leu Ser Gln Glu
5030 5035 5040

Arg Leu Ile Arg Gln Val His Thr Glu Phe Gly Ile Asp Pro Ala
5045 5050 5055

Glu Ile Gly Met Val Glu Ala His Gly Thr Gly Thr Gln Leu Gly
5060 5065 5070

Asp Pro Ile Glu Cys Gln Ala Leu Val Gly Ala Phe Gly Thr Ala
5075 5080 5085

Gly Gly Ser Asp Thr Cys Ala Leu Gly Ser Ile Lys Thr Asn Leu
5090 5095 5100

Gly His Thr Thr Ser Ala Ala Gly Val Ala Gly Leu Leu Lys Val
5105 5110 5115

Val Leu Ser Leu Arg His Gly Gln Ile Pro Pro Ser Leu His His
5120 5125 5130

Tyr Glu Thr Asn Pro Ala Ile Arg Leu Thr Glu Ser Pro Phe His
5135 5140 5145

Val Asn Thr Thr Leu Arg Pro Trp Gln Pro Asn Gly Gln Gly Lys
5150 5155 5160

Arg Val Ala Ala Leu Ser Ala Phe Gly Phe Ser Gly Thr Asn Gly
5165 5170 5175

His Met Val Val Glu Asn Ala Pro Asp Arg Asp Glu Arg Gln Gln
5180 5185 5190

Ala Ala Asp Glu Leu Leu Phe Val Leu Ser Ala Gln Gln Pro Glu
5195 5200 5205

Ala Leu Arg His Arg Ala Glu Asp Leu Leu Ala Tyr Leu Arg Arg
5210 5215 5220

Ala Pro Asp Ala Ala Leu Gly Asp Val Ser Tyr Thr Leu Ala Ala
5225 5230 5235

Gly Arg Asp His Phe Thr His Arg Ala Ala Phe Val Ala Ala Asp
5240 5245 5250

Arg Asp Thr Leu Ala His Arg Leu Glu Ala Trp Leu Ala Asp Gly
5255 5260 5265

Arg Ser Asp Thr Val Gly Arg Arg Gly Asp Thr Ala Pro Glu Arg
5270 5275 5280

Ala Arg Ala Arg Tyr Leu Asn Gly Glu Glu Val Asp Phe Ala Pro
5285 5290 5295

Leu Phe Ser Gly Leu Asp Val Arg Arg Thr Pro Leu Pro Thr Tyr
5300 5305 5310

Pro Phe Gln Arg Lys Ser Tyr Trp Pro Thr Ala Thr Ala Pro Ser
5315 5320 5325

Arg Arg His Gln Ala Pro Gln Ala Ala Asn Gly Pro Ala Ala Ala
5330 5335 5340

Pro Ser Pro Glu Pro Ala Arg Pro Ala Pro Ala Gln Pro Ala Pro
5345 5350 5355

Asp Thr Asp Glu Ala Thr Val Arg Tyr Leu Ala Gly Glu Leu Leu
5360 5365 5370

Leu Ala Glu Leu Ser Arg Val Leu Met Met Glu Pro Glu Glu Ile
5375 5380 5385

Asp Pro Gln Ala Ser Phe Ser Asp Tyr Gly Val Asp Ser Ile Leu
5390 5395 5400

Thr Val Arg Leu Val Ala Ala Val Asn Asn Ala Leu Ala Val Asp
5405 5410 5415

Leu Pro Ser Thr Ala Leu Phe Glu His Ser Ser Leu Asp Arg Leu
5420 5425 5430

Thr Asp His Leu Val Thr Arg Tyr Gly Ala Gln Leu Arg Ser Ser
5435 5440 5445

Gly Ala Leu Arg Gly Pro Ala Ala Glu Ala Gly Gly Ala Pro Ala
5450 5455 5460

Gln Asp Asp His Gly Pro Ala Ala Glu Ala Pro Ser Ala Ala Pro
5465 5470 5475

Ala Ala Pro Val Ala Ser Ala Gly Thr Ala Ala Val Pro Ala His
5480 5485 5490

Ala Pro Ala Ala Ala Ala Gly Asp Pro Ala Asp Asp Gly Val Ala
5495 5500 5505

Val Val Gly Ile Ala Ala Arg Phe Ala Gln Ser Pro Asp Ala Ala
5510 5515 5520

Ala Leu Trp Ala His Leu Ala Ala Gly Asp Asp Leu Val Gly Glu
5525 5530 5535

Val Thr Arg Trp Asp Met Asp Glu Glu Leu Gly Ala Gly Ala Pro
5540 5545 5550

Arg Gln Tyr Gly Ser Phe Val Asp Asp Ile Glu Arg Phe Asp Ala
5555 5560 5565

Trp Phe Phe Arg Met Ser Gly Lys Glu Ala Thr Tyr Thr Asp Pro
5570 5575 5580

Gln Gln Arg Ile Phe Leu Glu Glu Cys Trp His Ala Leu Glu Asp
5585 5590 5595

Ala Gly Tyr Ala Gly Glu Arg Leu Asp Gly Arg Gly Cys Gly Val
5600 5605 5610

Tyr Val Gly Gly Ser Pro Ser Asp Tyr Gln Gln Leu Ile Gly Asp
5615 5620 5625

Asp Ala Pro Pro Gln Thr Leu Trp Gly Asn Ile Ser Ser Val Ile
5630 5635 5640

Ala Ser Arg Ile Ser Tyr Phe Leu Asp Leu Gln Gly Ala Ala Leu
5645 5650 5655

Ala Val Asp Thr Ala Cys Ser Ser Ser Leu Val Ala Ile His Gln
5660 5665 5670

Ala Cys Gln Asp Leu Arg Leu Gly Asn Thr Ser Met Ala Leu Ala
5675 5680 5685

Gly Gly Val Phe Val Gln Ser Thr Pro Ile Phe Tyr Arg Ser Ala

5690 5695 5700

Val Arg Ala Asn Met Leu Ser Ala Arg Gly Arg Cys His Thr Phe
5705 5710 5715

Asp Glu Arg Ala Asp Gly Phe Val Pro Gly Glu Gly Ala Gly Val
5720 5725 5730

Val Val Leu Lys Arg Leu Ala Asp Ala Leu Arg Asp Gly Asp Gln
5735 5740 5745

Val Tyr Gly Val Ile Arg Gly Ser Gly Met Asn Gln Asp Gly Thr
5750 5755 5760

Thr Asn Gly Leu Thr Ala Pro Ser Ala Gly Ser Gln Glu Arg Leu
5765 5770 5775

Leu Arg Ser Val His Glu Arg Ala Gly Val Asp Pro Ala Gly Ile
5780 5785 5790

Gln Leu Ile Glu Ala His Gly Thr Gly Thr Pro Leu Gly Asp Pro
5795 5800 5805

Ile Glu Phe Glu Ala Leu Arg Ala Ala Phe Gly Asp Ala Pro Glu
5810 5815 5820

Ala Gly Cys Ala Leu Gly Ser Val Lys Thr Ser Leu Gly His Thr
5825 5830 5835

Gln Phe Ala Ala Gly Val Ala Gly Val Ile Lys Val Leu Leu Ala
5840 5845 5850

Leu Arg Asn Glu Gln Leu Pro Pro Ser Leu His Phe Arg Arg Ala
5855 5860 5865

Asn Pro Ala Ile Thr Leu Glu Gly Ser Pro Phe Tyr Val Asn Thr
5870 5875 5880

Glu Leu Arg Pro Trp Pro Ala Pro Ala Asp Gly Pro Arg Arg Ala
5885 5890 5895

Gly Val Ser Ser Phe Gly Ala Ala Gly Thr Asn Ala His Ala Leu
5900 5905 5910

Ile Glu Gln Ala Pro Ala Val Arg Thr Ala Gly His Gly Pro Arg
5915 5920 5925

His Ala Trp Leu Ile Val Leu Ser Ala Gln Asp Asp Ala Gly Arg
5930 5935 5940

Arg Ala Gln Ala Glu Arg Leu Leu Asp His Ala Leu Ala His Glu
5945 5950 5955

Asp Leu Asp Leu Gly Asp Val Ala Tyr Thr Leu Ala Thr Gly Arg
5960 5965 5970

Arg His Cys Ser His Arg Trp Ala Gly Val Ala Thr Asp Arg Glu
5975 5980 5985

Gln Leu Val Ala Ala Leu Arg Thr Trp Leu Cys Asp Gly Arg Ala
5990 5995 6000

Glu Gly Val Val Thr Gly Glu Ala Pro Asp Gly His Arg Arg Gln
6005 6010 6015

Asp Pro Ala Glu Asp Ala Arg Ala Gly Arg Leu Met Ala Glu Pro
6020 6025 6030

Asp Arg His Asp Ser Leu Thr Glu Leu Ala Gly Leu Phe Ala Gln
6035 6040 6045

Gly Gln Asp Leu Gly Phe Ala Pro Leu Phe Gly Asp Gly Gly Phe
6050 6055 6060

Arg Ile Val Ser Leu Pro Ala Tyr Pro Phe Ala Gly Glu Arg Tyr
6065 6070 6075

Trp Val Gly Ser Arg Pro Ala Ala Pro Ala Ala Thr Pro Ala Ser
6080 6085 6090

Ala Pro Val Arg Ala Pro Val Pro Val Ala Ala Pro Ser Pro Leu
6095 6100 6105

Glu Gly Arg Arg Leu Thr Gly Asp Pro Gly Ser Pro Ser Phe Ala
6110 6115 6120

Val Glu Leu Ala Gly Arg Glu Phe Phe Leu Asp Asp His Arg Val
6125 6130 6135

Arg Asn Val Pro Val Leu Pro Gly Val Ala Tyr Leu Glu Leu Ala
6140 6145 6150

Tyr Ala Ala Ala Arg Ala Glu Gly Val Asp Pro Ala His Ala Arg
6155 6160 6165

Leu Arg Asn Val Val Trp Ser Arg Pro Ala Arg Ile Thr Gly Pro
6170 6175 6180

Thr Ala Val Glu Ile Ala Leu Arg Pro Cys Glu Asp Asp Ala Phe
6185 6190 6195

Thr Tyr Glu Ile Thr Thr Ala Ala Asp Gly Glu Gln Pro Val Ile
6200 6205 6210

His Gly Gln Gly Arg Ile Glu Arg Cys Gly Thr Pro Ser Pro Ala
6215 6220 6225

Arg Leu Asp Ile Ala Ala Leu Arg Ala Gln Cys Glu Val Arg Thr
6230 6235 6240

Leu Glu His Asp Asp Cys Tyr Arg Leu Phe Asp Arg Met Gly Ile
6245 6250 6255

Gly Tyr Gly Pro Ala Met Arg Gly Ile Arg Arg Ile His Val Gly
6260 6265 6270

Ala Gly Leu Ala Val Ala Arg Leu Ser Leu Pro Gln Ala Ala Arg
6275 6280 6285

Asp Gly Ala Gly Trp Asp Leu His Pro Ser Met Leu Asp Ala Ala
6290 6295 6300

Val Gln Ala Thr Leu Gly Leu Ser Leu Ala Glu Asp Thr Asp Thr
6305 6310 6315

Val Ala Pro Ala Leu Pro Phe Val Leu Glu Glu Val Gln Leu Leu
6320 6325 6330

Ala Pro Ser Pro Ala Gly Gly Trp Ala Val Val Arg Pro Ala Ala
6335 6340 6345

Gly Asp Gly Gly Gly Ala Val Arg Arg Ile Asp Ile Glu Leu Cys
6350 6355 6360

Asp Asp Asp Gly Glu Val Cys Val Arg Leu Leu Gly Phe Thr Ala
6365 6370 6375

Arg Val Leu Ala Ala Gly Asp Asp Pro Ala Gly Gly Glu Asn Thr
6380 6385 6390

Gly Gly Ala Thr Leu Thr Leu Met Arg Ala Gly Trp Arg Pro Ala
6395 6400 6405

Glu Pro Thr Arg Ala Ser Arg Pro Leu Val His His Glu Val Leu
6410 6415 6420

Leu Gly Gly Leu Ala Gly Thr Asp Pro Ala Ala Val Arg Asp Gly
6425 6430 6435

Leu Gly Val Pro Cys Thr Ala Leu Pro Asp Asp Gly Asp Pro Ala
6440 6445 6450

Arg Cys Phe Thr Arg Gln Ala Glu Thr Val Leu Ala Arg Leu Gln
6455 6460 6465

Gln Phe Val Pro Arg Thr Arg Asp Gly Glu Val Leu Leu Gln Val
6470 6475 6480

Val Val Pro Ala Asp Gly Glu Asn Arg Val Leu Ala Gly Leu Gly
6485 6490 6495

Gly Leu Leu Arg Thr Ala Arg Met Glu His Pro Lys Leu Leu Thr
6500 6505 6510

Gln Leu Val Glu Val Glu Thr Pro Val Asp Ala Ala Thr Leu Cys
6515 6520 6525

Glu Arg Leu Arg Arg Asp Ala Ala Ser Pro Asp Asp Val Ala Val
6530 6535 6540

Arg Tyr Ser Gly Gly Gln Arg Arg Val Pro Gln Trp Thr Ala Val
6545 6550 6555

Glu Asp Ala Pro Pro Ala Arg Pro Trp Lys Ala Gly Gly Val Tyr
6560 6565 6570

Leu Leu Thr Gly Gly Val Gly Gly Leu Gly Ala His Phe Ala Arg
6575 6580 6585

Glu Ile Ala Arg Gln Ala Pro Gly Ala Ala Leu Val Leu Cys Gly
6590 6595 6600

Arg Ser Pro Glu Gly Pro Ala Gln Arg Glu Leu Leu Cys Glu Leu

6605 6610 6615

Gly Asp Leu Gly Ala Ser Ala Val Tyr Arg Val Leu Asp Val Ala
6620 6625 6630

Arg Arg Asp Ala Val Thr Ala Cys Val Asn Thr Val Val Ala Glu
6635 6640 6645

His Gly Arg Leu Asp Gly Val Val His Thr Ala Gly Val Val Arg
6650 6655 6660

Asp Gly Tyr Leu Ala Arg Lys Ser Ala Glu Glu Leu Arg Glu Val
6665 6670 6675

Leu Ala Ala Lys Val Ala Gly Phe Val His Leu Asp Gly Ala Thr
6680 6685 6690

Ala Ala Leu Asp Leu Asp Cys Phe Ile Gly Phe Ser Ser Leu Ser
6695 6700 6705

Ala Tyr Gly Asn Gln Gly Gln Gly Asp Tyr Ala Ala Ala Asn Ala
6710 6715 6720

Phe Met Asp Ala Tyr Ala Gly Leu Arg His Glu Arg Val Ala Arg
6725 6730 6735

Gly Glu Arg Arg Gly Arg Thr Leu Val Val Gly Trp Pro Leu Trp
6740 6745 6750

Ala Asp Gly Gly Met Thr Val Asp Ala Ala Thr Glu Arg Arg Leu
6755 6760 6765

His Asp Ser Val Gly Met Val Pro Ile Arg Ala Pro His Gly Val
6770 6775 6780

Glu Ala Leu Leu Arg Ala Tyr Gly Thr Gly Asp Pro His Val Leu
6785 6790 6795

Ala Val Phe Gly Asp Arg Ala Arg Ile Asp Ala Thr Leu Leu Ala
6800 6805 6810

Ala Pro Ala Ala Thr Gly Ala Ala Pro Ala Val Thr Ala Pro Asp
6815 6820 6825

Arg Ala Ala Leu His Ala Arg Val Leu Gly Arg Ala Ile Ser His
6830 6835 6840

Ala Cys Ala Val Leu Gly Val Pro Ala Ala Glu Leu Asp Gly Ala
6845 6850 6855

Val Glu Leu Ser Glu Tyr Gly Phe Asp Pro Val Ser Leu Thr Gly
6860 6865 6870

Phe Ala Ala Arg Leu Thr Thr Glu Phe Gly Leu Pro Pro Val Pro
6875 6880 6885

Lys Pro Phe Ser Glu His Leu Thr Leu Gly Glu Val Val Asp His
6890 6895 6900

Leu Leu Asp Thr His Pro His His Phe Gly Thr Val Pro Pro Ala
6905 6910 6915

Pro Ala Pro Glu Pro Ser Ala Gly Pro Glu Ser Ala Ala Ala Pro
6920 6925 6930

Val Ala Thr Ala Gly Arg Glu Gln Gln His Lys Ala Leu Leu Lys
6935 6940 6945

Lys Leu Ile Ala Arg Val Ser Asp Leu Leu Asp Val Pro Ala Glu
6950 6955 6960

Arg Ile Thr Gly Thr Ala Glu Met Thr Arg Tyr Gly Phe Asp Ser
6965 6970 6975

Leu Ser Phe Ile Gly Phe Ala Asn Asp Leu Asn Ala Glu Phe Gly
6980 6985 6990

Leu Ser Leu Ala Pro Thr Leu Phe Phe Glu Asn Pro Thr Leu Asp
6995 7000 7005

Gly Val Val Asp His Leu Leu Asp His His Ala Asp Arg Val Ala
7010 7015 7020

Ala Thr Ala Ala Pro Gln Gln Glu Pro Arg Ala Ala Ala Ala Pro
7025 7030 7035

Ala Ala Pro Glu Pro Ala Thr Ala Asp Thr Pro Ala Ser Arg Thr
7040 7045 7050

Asp Ala Pro Gly Asn Glu Pro Ile Ala Val Ile Gly Ile Ser Gly
7055 7060 7065

Arg Phe Pro Met Ala Asp Asp Leu Asp Ala Phe Trp Glu Asn Leu
7070 7075 7080

Ser Glu Gly Arg Asp Cys Thr Arg Glu Val Pro Thr Asp Arg Trp
7085 7090 7095

Asp Trp Arg Ala His Tyr Gly Asp Pro Val Lys Glu Pro Asn Thr
7100 7105 7110

Ser Asn Val Thr Ser Gly Gly Phe Met Asp Gly Val Gly Asp Phe
7115 7120 7125

Asp Pro Leu Phe Phe Asp Ile Ser Pro Lys Glu Ala Glu Leu Met
7130 7135 7140

Asp Pro Gln Gln Arg Leu Leu Leu Met Tyr Val Trp Lys Ala Leu
7145 7150 7155

Glu Asp Ala Gly Tyr Ser Ala Glu Ala Leu Ala Gly Thr Asn Thr
7160 7165 7170

Ala Leu Ile Ala Gly Thr Thr Ser Thr Gly Tyr Ser Thr Leu Val
7175 7180 7185

Thr Arg Tyr Ser Pro Met Ile Glu Gly Tyr Asp Ile Thr Gly Ala
7190 7195 7200

Ala Pro Ser Met Gly Pro Asn Arg Met Ser Tyr Phe Leu Asp Leu
7205 7210 7215

His Gly Pro Ser Glu Pro Val Asp Thr Ala Cys Ser Ser Ala Leu
7220 7225 7230

Val Ala Leu His Arg Ala Val Gln Ala Ile Arg Asp Gly Gln Ser
7235 7240 7245

Asp Leu Ala Ile Ala Gly Gly Val Asn Thr Met Val Ser Val Asp
7250 7255 7260

Gly His Ile Ser Ile Ser Lys Ala Gly Met Leu Ser Pro Glu Gly
7265 7270 7275

Arg Cys Lys Thr Phe Ser Asp Arg Ala Asp Gly Tyr Ala Arg Gly
7280 7285 7290

Glu Gly Val Gly Met Leu Val Leu Lys Ser Leu Ser Ala Ala Glu
7295 7300 7305

Arg Asp Gly Asp His Ile Tyr Gly Val Ile Arg Ser Thr Ala Glu
7310 7315 7320

Asn His Gly Gly Arg Gly Ser Ser Leu Thr Ala Pro Asn Pro Lys
7325 7330 7335

Ala Gln Ala Ala Leu Leu Arg Glu Ala Tyr Gly Lys Ala Gly Ile
7340 7345 7350

Asp Pro Arg Thr Val Gly Tyr Ile Glu Ala His Gly Thr Gly Thr
7355 7360 7365

Lys Leu Gly Asp Pro Val Glu Ile Asn Gly Leu Lys Ala Ala Phe
7370 7375 7380

Arg Asp Met Tyr Glu Glu His Gly Ala Val Val Glu Glu Ala His
7385 7390 7395

Cys Gly Ile Gly Ser Val Lys Thr Asn Ile Gly His Leu Glu Leu
7400 7405 7410

Ala Ala Gly Ala Ala Gly Val Ile Lys Val Leu Leu Gln Met Arg
7415 7420 7425

His Arg Thr Leu Val Lys Ser Leu His Cys Asp Thr Val Asn Pro
7430 7435 7440

Tyr Ile Asp Leu Asp Gly Ser Pro Phe His Leu Val Arg Glu Arg
7445 7450 7455

Gln Pro Trp Pro Ala Leu Arg Asp Ala Glu Gly Arg Glu Leu Pro
7460 7465 7470

Arg Arg Ala Gly Val Ser Ser Phe Gly Phe Gly Gly Val Asn Ala
7475 7480 7485

His Val Val Leu Glu Glu Tyr Arg Pro Arg Thr Ala Pro Glu Pro
7490 7495 7500

Asp Arg Ala Pro Thr Ala Pro Val Pro Val Val Leu Ser Ala Ser
7505 7510 7515

His Pro Asp Val Leu Cys Glu Leu Ala Glu Arg Trp Val Asp Ala

7520 7525 7530

Leu Arg Arg Gly Asp Tyr Asp Asp Thr Asp Met Ala Ser Ile Ala
7535 7540 7545

Tyr Thr Thr Gln Thr Gly Arg Thr Pro Met Thr Glu Arg Leu Ala
7550 7555 7560

Cys Leu Ala Arg Thr Ala Gly Glu Leu Arg Glu Ala Leu Glu Ser
7565 7570 7575

Trp Leu Arg Gly Glu Pro Ala Ala Asp Val Phe Arg Gly Lys Val
7580 7585 7590

Ala Arg Gly Val Asp Leu Pro Asp Ala Pro Ala Gly Phe Gly Pro
7595 7600 7605

His Asp Asp His Asp Ser Ala Gly Arg His Asp Trp Ala Arg Leu
7610 7615 7620

Leu Gln Ala Trp Val Asn Gly Ala Pro Phe Asp Trp Asp Arg Leu
7625 7630 7635

His Thr Gly Arg Arg Pro Arg Arg Ile Ala Leu Pro Thr Tyr Pro
7640 7645 7650

Phe Arg Leu Arg Arg Tyr Trp Val Asp Thr Ser Arg Pro Ala Asn
7655 7660 7665

Gly Thr Gln Thr Glu Ala Leu His Pro Leu Val His Thr Asn Thr
7670 7675 7680

Ser Asp Leu Asn Glu His Arg Tyr Thr Ser His Phe Thr Gly Arg
7685 7690 7695

Glu Phe Phe Leu Ala Asp His Arg Val Arg Ala Gln Val Met Glu
7700 7705 7710

Thr Val Ser Gly Trp Arg Pro Gly Arg Arg Pro Thr Ala Tyr Asp
7715 7720 7725

Val Arg Ala Asp Ala Val Pro Val Leu Pro Ala Val Ala Tyr Leu
7730 7735 7740

Glu Met Ala Arg Ala Ala Ala Val Gln Ala Ala Gly Gly Asp Glu
7745 7750 7755

Arg Ala Trp Ser Leu Lys Leu Ala Ser Trp Leu Arg Pro Leu Thr
7760 7765 7770

Val Glu Lys Ala Thr Asp Val His Thr Thr Leu Thr Thr Arg Ala
7775 7780 7785

Gly Gly Gly Leu Ser Tyr Glu Val Tyr Ala Val Asp Glu Asp Gly
7790 7795 7800

Glu Arg Val Thr Phe Gly Arg Gly Gln Leu Arg Arg Ala Thr Ala
7805 7810 7815

Val Pro Ala Glu Arg Leu Asp Leu Ala Ala Leu Arg Ala Gln Cys
7820 7825 7830

Asp Gly Pro Val Leu Asp Ala Glu Thr Cys Tyr Ala Arg Phe Thr
7835 7840 7845

Gly Ile Gly Met Ala Tyr Gly Pro Ala Leu Arg Gly Ile Glu Arg
7850 7855 7860

Leu His Thr Gly Ser Arg Gln Ser Val Ala Arg Leu Lys Leu Pro
7865 7870 7875

Ala Ala Ala Ser Arg Glu Arg Gly Trp Val Leu Asn Pro Gly Met
7880 7885 7890

Leu Asp Ala Ala Leu Gln Ala Thr Val Gly Leu Phe Val Asp Asp
7895 7900 7905

Pro Gly Thr Pro Arg Thr Ala Leu Pro Phe Ala Leu Gly Glu Leu
7910 7915 7920

Glu Val Leu Arg Ala Val Pro Gly Thr Gly Trp Val Val Val Arg
7925 7930 7935

Phe Ala Glu Asp Asp His Val Gly Ala Val Arg Arg Leu Asp Leu
7940 7945 7950

Asp Leu Cys Asp Asp Asp Gly Glu Val Cys Val Arg Leu Arg Gly
7955 7960 7965

Phe Ser Val Arg Thr Leu Gly Gly Ser Glu Pro Thr Gly Asp Ser
7970 7975 7980

Glu Pro Thr Arg Pro Ala Glu Gln Ala Pro Glu Pro Pro Ser Gly
7985 7990 7995

Ser Asp Asp Ala Tyr Leu Leu Asp Leu Ile Glu Ala Ile Gly Arg
8000 8005 8010

Arg Glu Met Ser Ala Asp Glu Phe Lys Arg Ser Leu Ala
8015 8020 8025

<210> 13
<211> 24081
<212> DNA
<213> Streptomyces platensis subsp. rosaceus

<400> 13

```
gtgacctgga acggaatgaa cgtgagcaga aacatccttc gtgtgccgga atggcgcgac      60
gaaccggcgc gagggcgcac cgcgcccccc ggaaaccggc ggctggtcgt gctgtgcgac     120
acccccgacg cggacgtgac cgacctgcgc cggcacctgc ccggcgtgtc cgtcgcccgc     180
gtggacagcg gtgacgacgg gcccgctgcc gcctacgagc acgcggcgac cctgctgctc     240
ggcgagctcc agcggctgct gaaccagccg gccggcggcc cccgttccgt gcaggtcgtg     300
tgccgggagg ggactccgta cggctacgcc ggtctgatcg gcatgctgcg caccgccgcg     360
caggaggacc cggcgctgca cggccagctg atcgagtgca cgcagcggcc gtcgggcgag     420
gaactcgccg gcgtgctgcg ggcggagtac gggcaggcgg cggatcacgt gcgctacacc     480
```

ggcggccgcc gccaggtccg cgcctgggca gcggccccgc gtgcggcggc acccccgccg 540
gtgtggaagg ccgacggcgt ctacctgatc agcgggggag cgggcggcgt cggccggctg 600
gtcgccgccg acatcgcacg gcacgccccc ggcgcccggg tcgtcctgtg cggacgctcg 660
ccggcggtcc ccgggcccgg tcagccgggc ccggggaccg agtaccgccg ggtggacgtc 720
gccgacgccg acgccgtggc ggagctcgtc aactcccttg tgcgcacgta cggcaggctc 780
gacggggtcg tgcacgcggc gggcctgatc agcgacgact acgtgatccg caagtcccac 840
caggacgccc agcaggtcct ggcgccgaaa gccgctgggc tggtgaacct cgacgaggcc 900
acccgccgcc tgccgttgga cttcctcgcg gcgttctcct ccggcgcggg gacgctgggc 960
aaccccgggc aggccgacta cgccgccgcg aacgggttcc tcgacgccta cctgacccac 1020
cgcgccggcc tggccgccgc gggcgagcgc cacggcgcga gcgtctcgat cggctggccg 1080
ctgtggcagg acggcgggat gagcgtcccg gccgaggacg tgcccgcgct caccgcccgc 1140
ttcgggcgcc ccctgggaac ggacacggca ctgcgggccc tgcacggcgc actggcgctc 1200
ggcacaccac acctactggt catggacgag gagagcggag tggacgaaga gagcggagtg 1260
gacgaggaag gtccgcagga ggcggagacg cagcagacgg ggccggcgga actgcgggca 1320
catgtgctgc ccctgctgaa ggagttgatc gccgagacgg tgcggctcga ccccgcccgg 1380
ctggacgccg ccgctccgct cgacggcttc ggcatcgact cgctggccgt gacccggctc 1440
aaccgccggt tcgcgcagtg gttcggcgcg ctgcccaaga cggtgctcta ccagtacccg 1500
acgctgaacg acctggccgg gcacctggcg gagcagcacg cggacggctg ccgccgctgg 1560
ctcggcgacg tcccggacgt ggccgccgcc ccggccggga ctccggcgac ggcggccgcg 1620
ccgcggaagg cgcggccccg tccggccgac gcggacgagc cgatcgccct catcggcctg 1680
agcgggcgct atccggacgc cccgaccctg gaggcgttct gggagaacct gcgcgcgggc 1740
cgcgagagcg tccgcgaggt ccccgccgag cgctggccgc tggacgcctt ctacgaaccg 1800
gacccgcagc gggccgtgca gcagggcgcc agctacagca agtggggcgc gttcctcgac 1860
gacttcgccc gcttcgacgc cgcgttcttc gggatcgcgc cgcgcgacgc cgccgacatg 1920
gacccgcagg aacggctgtt cgtcgagagc gcgtggtcgg tgctggagga cgcgggctat 1980
acgcggcagc gcctcgccga gcagcacgca tcgtcggtcg gcgtcttcgc cgggatcacc 2040
aagaccggct tcgaccgcca ccgcccgccg gcgaccgacg gactgccgcc cgcgccgcgc 2100
acgtccttcg gatcgctggc caaccgggtg tcgtacctgc tggacctgca cggcccgagc 2160
atgccgatcg acaccatgtg ctcgtcgtcg ctgaccgcga ttcacgaggc atgcgagcac 2220
ctgcgccacg gcgcgtgcga gctggccatc gccggcggtg tcaacctcta cctgcacccc 2280

tcctcgtacg tcgagttgtg ccgttcccgg atgctcgcca ccgacgggca ctgccgcagc 2340
ttcggcgcgg gcggcgacgg gttcctgccc ggcgagggcg tcggcgcggt gctgttgaag 2400
ccgctgtccg cggccgaggc cgacggcgac cccatccacg cggtgatcgt cggctccgcg 2460
atcaaccatg gtgggcgcac caacggttac accgtgccca acccgcgcgc acaggccgcg 2520
ctgatccgcg acgcgctgga ccgcgccggt gtgtccgcgg ccggcatcgg ctacatcgag 2580
gcgcacggca ccggcacccg gctcggcgac cccgtcgaga tcgacggcct gacccaggcc 2640
ttcgctcctg acgccggcgg gagcggtgcg tgcgccctcg gctcggtcaa gtcgaacatc 2700
gggcacctgg aggccgctgc gggtatcgcg ggcctgacca aggccgtact gcaactgcag 2760
cacggcgagt tcgcgcccac cctgcatgcc gagcagacca acccggacat cgacttcgcg 2820
gccaccccgt tcaccctgca gaccggcggg gccccttggc cgcggcccgc ggacggcggc 2880
ccgcggaggg caggcatctc ctcgttcggc gcgggcggcg ccaacgccca tgtcatcgtc 2940
gccgagtacc ggagcgcgac gcccgcaccc gccacgcccg ccccgtccgc gcggccggtg 3000
ctgctgccgc tgtccgcccg gaccaccgag gacctgcacg cacgggccgg ccaactgtcc 3060
gacctgctcc gcaacggcgc ccccgtggac ctgcccgccg tcgcggccac cctccagacc 3120
ggccgcgagg agatggcgga gcgggtgtgc ttcgtcgcga gcacacccgg ggaatggctc 3180
gaccagctcg gcgccttcct cgccgactcc gactccgact ccgactccga ctccgactcc 3240
gactccgact ccgactccga ctccgactcc ggctccggct ccgaggccga ggccgaggtc 3300
ccgtggtccc gcggccgggt cagggccacc cgcgagaccc tggcagccct ggcggagaag 3360
gacgaactgc gcgcactcgt cacccgctgg atcaaccgcg gcgactggca cgacctggcc 3420
gccttctggg ccaagggcat gccgctcgac tggacccgcc tgcacgccgg tgcggacacg 3480
cccgcacggg tccacctgcc cgcctacccc ttcgccggac ggcagttctg gttcggcccg 3540
gccggcagcg agcacccggc aacgacgccg gtggccgccc cgtcctgctc gacggcagcc 3600
ggtgccgccg acgtcgagcg catcctgctc gacgcactgg cagcggccct gcagatgccg 3660
gtcgccgaga tcgagcgccg ccgcccccttc gccgactacg gcctggactc catcctcggc 3720
gtgaacctgg tccacacgct caacacggcc ctcggcaccg cgctggagac caccgatctg 3780
ttcgaccacg gcaccgtcga gcgcctgcac gcgttcctcg tcggtaccta cggtgacgca 3840
ctgcacgcac cggcctcccc ggcagccgtc gccccggcgc cagacgacga cgccatcgcc 3900
gtcgtcggga tggccgcccg ctacgccgac gccgaggacc cgcgcgcgct ctgggaccac 3960
ttgatggccg gccacgacct cgtcgaaccg gtgacccgct ggccgctcgg ccaggacgtg 4020
agctgccgct ccggcagctt cgtccgcggc atcgaccagt tcgacccggt gttcttcgcg 4080
atctccggtg tcgaggccac caccatggac cctcagcagc gcatcttcct cgaacagtgc 4140

tggaacgccc tggaggacgc cggctacacc ggcgaacgcc tgaccaaccg caactgtggc 4200
gtctacgccg gctgctacgc cggcgactac cacgaccagc tggacgcccg gccgccggcg 4260
caggcgctgt ggggcaccat gggctcggtc gtcgcctccc ggatcgccta ccacctcgac 4320
ctcaagggcc ctgccctcac caccgacacc tcctgctcca gctcactcgt ctccctgcac 4380
ctggcctgcc gcgacctgct ctccggggac gccgacatgg cgatcgcggg cggggtgttc 4440
atccagacca cgtcgcggct gtacgagtcg gcgtcgcgcg cgggcatgct ctcgcccagc 4500
ggccgctgcc acagcttcga cgcccgcgcc gacggcttcg tcccgggcga gggcgcgggc 4560
gcagtcgtcc tcaaacggct cgccgacgcc cggcgcgacg gcgaccacat ctacggcgtc 4620
gtccgcggct ccggcatcaa ccaggacggc accaccaacg gcatcaccgc cccgagcgcg 4680
gcctcgcagg aacagctcct gcgcgacgtc cacgcccgca gcggcatcga gccgggcggc 4740
atccagctcg tcgaggcgca cggcacgggt acccagctcg gcgacccgat cgaattccgc 4800
gcgctcaccc gcgcgttcga ggacgccccg gccgggagcg ccgtgctggg atcgatcaag 4860
accaacatcg ggcacacgca gttcgccgcc ggcatcgcgg gcgtcatcaa ggcgctgctg 4920
gccctggagc accggcagat cccgccgtcg ctccacttcc aagaggccaa ccgggccgtc 4980
gtgctcgacg gcggcccgtt caccgtcacc accgccccgc agccctggac ggcgcctgcc 5040
cgcggcccgc gccgggcggc cgtgagttcc ttcggggcca gcggcaccaa cgcgcatgtc 5100
gtgctggagg agcacccggt cccccggacg accggcgcgg gcggggaaca cgcctttctg 5160
ctgtcggccc gcacaccggc cgctctccgt gccgtcgccg aacggctgct cgcccacctc 5220
gaccgcgaac ccgggctgcc cgccgacgcc gtcgccttca gcctggccgc gggacgcagc 5280
cacttcgcgc accggctggc cgtcgtcgcc gccggcctgc ccgacctggc ggcacgcctg 5340
cgctcctggc tgtccggcac cgccggtgac acggtgctgc agggggagac cgccgcggac 5400
ccccgccccg tcggcggtgt gcgcgcgccg gccccggccg cgctggccgc agcgtacgta 5460
cggggcgagg ccgaccggtt cgccgacagc ttcgcgtccg cctcgcgccg ccaggtgccg 5520
ctgccgacct acccgttcga gcggcagcgc tactggaccg acacgaccga caccggggaa 5580
agccaggggc tcaaggacac ggacggggcc gcgtaccgcc tccggctcgg cggcgaggag 5640
ttcttcctgg ccgaccacca cgtgggcggc cgggccgtgc tgcccggcgt gctctcgctg 5700
gagttcgcac gccgtgccgt gaccggcggt tccttcgcgc cggtcggcct gcgcgatgtc 5760
gtatggccgg agccgttccc cgtcggggac ggcggcgccg aactacgagt cgatcgggac 5820
ggcgacgcct tccgcgtcct gcgcgacggc tcggccgtac acgcccaggg ccggatcgcc 5880
acgcccggct cgcccgtccc cacgccgttg gacgccctgc gggcccgctg cggccgccgc 5940

acccctgtcgc ggagccagtg ccgtgcggcc ctcgacgccg tcggcatccg ccacggagac 6000
cgcctgcgcg ccatcgacac cctggccgtc ggtgacggcg aggtcctggc ccggctcgtc 6060
ctgcccgacg gcgcccgcga cggcgcgttc gcgctgcacc ccgcgatgct cgacagcgcc 6120
gtgcaggccg tcgtcggcct ctacggcgac gccaccggca cgctcgacga gcaacgcggc 6180
gcgcccgcac tgcccttcgc cctggacgcc gccgacttct tcgcccccac caccgaacgc 6240
atgtgggccc acctgcgcca caccgagggc tacaccccct cggccgaccg ggacgtgacg 6300
aaagtggaca tcgacgtgta cgacgacgac ggacagctct ccgcgagcct gcgcggctac 6360
gcgttccgcc gcatgaccgc cccgtccggc gcggccccgc gtgccacgct gctggcaccg 6420
gtgtgggacg ccctgcccgt cgtgcccgcc gagccgtggc cccacccgcg gacccgcgtc 6480
gtgctgctgg gcggcacccc cgaggaacgg gacgggctcc gccgccgcta ccccgacgcc 6540
accgtcctgg acccccacgc cgacgaaccg gtcgaccggc tggccgcgcg gctgcccgcc 6600
gacgccgagc acgtcttctg gctcgccccg gccggcccca ccggcgcccc ggccgccgcg 6660
cggtacgacg gcacgatcgc cgtattccga ctggtcaagg cgctcctggc cgacggcgcg 6720
gacgcccgtg aactgggcct gaccctggtc acccggcagg cgcgcctgct accgggcgac 6780
accggtgccg accccgccca cgccggtgtg cacggcctcg ccggcaccct ggccaaggag 6840
tacccgcact ggcggatccg cgtcgccgac gtcgaggcgg acgccgccgt gccctggccg 6900
gctctgctgg ctctgcccac cgacccccgc ggcgagaccc tggcccaccg gcacggcgag 6960
tggtaccgcc tgcgcctgct ggagacggac gggaccggcg tcgcggccgc cccgcgcgag 7020
cccggcggcg tgatcgtggc catcggcggc gccggcggca tcggcaccgt gtggaccgag 7080
cacatgatgc gccgtcacgg cgcccgggtc gtctggatcg gacgccgccc gctggacgcc 7140
gccatcgccg ctcagcagga agccctggca gcccacggcc ccaagccgga ctacgtgcag 7200
gccgacgcga ccgaccgcga cgccctgcgc cgcgcctgcg acgagatcgt gcggcggcac 7260
ggccccgtgc gcggcgtcct gcacaccgcg atcgtcctcg gcgaccagac cctcgcccgg 7320
atggacgagg accggttccg cacgacctac gccgccaagg ccgacatcgc cgtgaacctc 7380
gccgacgcct tcgccggcca gccgctggaa ttcgtcgcgt tcttctcctc catgcaggcc 7440
ttcttcaagg cccccggcca ggccaactac gcggcgggct gcaccttcgc cgacgcctac 7500
gccgagcacc tgtccacccg gctcgactgc ccggtcaagg tcatgaactg ggttactgg 7560
gccggcgtcg gcgtcgtcac cgccgacggc taccggcagc ggatggcaca gctgggcctg 7620
ggctcgatcg aaccggacga gggcatggcc gccttcgaca ccctgctggc ctcccgtac 7680
ccgcagctcg cactcctcaa ggccacggac acccgcagca tcgacggcct ccacgacgac 7740
gacgccctca cgcacccggt cgtcaccacc ccctccctga tcggcgccct gggcgaggac 7800

tgccccgacc gccgcgccga gatcgcgcag ctgcgtgaga aggcgggcgg gcacgccgga 7860
gccatgcagg acgcgctcgt ccgcatcacc tgggcgctgc tgcagtccct gggcctgttc 7920
cgcgacggcc gcgcggccac cgccgccgag tggcgcgccc tcggcggcat cgaggaccgc 7980
tacgagcgct ggaccgagca caccctcgcc gtactcgccg acgcaggcct cctgcgccgc 8040
gagggcgagg acacgtacgt ggccctcgac acccgtaccg gatccctcga cgacgcctgg 8100
gccgactggg accgggcgcg gcagcagtgg ctggccgacg acgccaagcg tccccaggcg 8160
gtcctcgtcg acacgacgct gcgcgccatg accggcatcc tcaccggccg ccgcccggcc 8220
accgacgtga tgttcccgaa cgcctggctc gaactcgtcg aggccgtgta caagaacaac 8280
cccgtcgccg actacttcaa cgacgtgctc gccgacaccc tcgtcggcta cctcgaacgg 8340
cggctggcgg acgacccgtc cgcccgcctg cgcatcctgg agatcggcgc cggcaccggc 8400
ggtaccagcg ccacggtcct gcgcaggctg cggccgtggg cccggcacat cgagaagtac 8460
acctacaccg acatctccaa ggcgttcttg ctgtacgggc agcgggagta cggcgagatc 8520
gccccgtacc tggacgcacg gctcttcaat gccgagaagc cgctggcagg ccaggaggtg 8580
gaccccggcg cgtacgacgt cgtgatcgcc accaacgtgc tgcacgcgac ccgcaacatc 8640
cgcaggacgc tgcgcaacgc caaggccgcc gcgcgcccga acgccctgct gctgctcaac 8700
gagctcagcg acaacatcct cttcagccac ctcacgttcg gcctcctgga cggctggtgg 8760
ctctacgacg acccggcgcc gcgtatcccc ggttctccgg gcctggcgcc ggagagctgg 8820
cggcgggtcc tcggcgaggt cggcttccgc gcggcgttcg tcgccgccgg gggcgccgac 8880
gacctcggcc agcaggtgat cgtcgccgag agcgacggcg cgatccgcca gccgcgcccg 8940
gacggggagt ccgctttccg cggcaccctc ccggaggccg ggccgcgggc cgccgagcct 9000
caactgcccg ccccgacacc ggatccggtc gccgccgacg gcgtacgtga cgacgagctc 9060
ctggcggacc tggcccgcga ccacttccgc accctggtcg cggacacctt gcaactgccg 9120
gtcgccgaca tccgcgccga tgtgcccttc gaccgctacg gcatcgactc gatcctggtc 9180
gtccagctga cggaagcggt ccgcaagggg ctctgcaacg tcggcagcac gctgttcttc 9240
gaagtacgga cggtcgacgg gctcgtccag cacttcctgc gcacccagcc cgacgcgctc 9300
gcggcactgg tcggcctgag cggcgcgcgg gcagcgcgca cggacgagca gctcgcgccg 9360
gccgccgggc cggagccggt ccccgtcatc gccgccgaac cgccccgcgc cgagcagggc 9420
atggccatcg cgatcgtcgg catggcaggc cgctaccccg gcgcacccga cctggacacc 9480
ttctgggaga acctgctcgc cggccgggac agcatcaccg agatcccggc cgggcgctgg 9540
gaccacagcc gctactacga cgcgcgtcgc ggcgtgcccg gcaggacgta cagcaagtgg 9600

ggcggcttcc tcgacgggat cgacgagttc gacccgctgt tcttcgggat ctcgccgaag 9660
gcggcgtcca cgatggaccc gcaggagcgg ctgttcctgc agtgcgccca caccacgctg 9720
gaggacgccg gctactcgcg cggcgccctg cgcgccgccg cccgcgcccg ggtggcggag 9780
gacgccggcg acatcggggt gttcgccggc gcgatgtact ccgagtacca gctctacggc 9840
gccgagtaca gcgtgcgcgg tgagccggtc gtggtgccgg ggagcctggc gtccatcgcc 9900
aaccgcgtct cgtacttcct ggacgcgagc ggccccagcg tcaccgtcga caccatgtgc 9960
gcctcggcgc tgtccgcgat ccacctcgcc tgcgccgccc tccagcgagg ggagtgcggt 10020
gtcgccctgg ccggcggggt caacctgtcg gtgcacccgg gcaagtacct gatgatcggg 10080
gagggccagt tcgcctccag cgacggccgc tgccgcagct tcggcgaggg cggcgacggc 10140
tacgtgcccg gcgagggcgt cggcgcggtg ctgctgcgcc cgctcgccga cgccgtcgcc 10200
gacggcgacc gcatcctcgg cgtgatccgc ggcaccgccg tgaaccacgg cggccacacg 10260
cacggattca ccgtgccgaa cccgctcgcg caggcggcgg tcatccgcag cgcctggcgc 10320
cgggccggag tggacccccg ggacatcggc tgcatcgagg cgcacggtac cggcacctcg 10380
ctgggcgacc cgatcgaaat cgccgggctg aacgcggcct tcgccgagtt caccgacgca 10440
cggaacttct gcgccatcgg ctcggcgaag tcgaacatcg gccacctgga gtccgcggcg 10500
ggtatcgcgg gcctcgccaa gctgctgctg cagatgcggc acggcacgct cgtgccctcc 10560
ctgcacgccg aacgcgtcaa cccggacatc gacttcgccg acagcccctt cgtcctgcag 10620
cgcgaagccg cgccctggcc gaggaccggc acccgcccgc gcctcggcgg cctctcctcg 10680
ttcggcgcgg gcggctccaa cgcccacgtc gtggtcgagg actacgtcga ggagcacgcc 10740
gggaaggacc tcgcgcccga ggcgcaccgt ggcgaaaccg tcgtcgtggt gctgtccgcc 10800
ttcgacgagg agcgcctgcg cgagtcggcc gggcggctgc gcgacgcgct gcggaaggag 10860
cggtggagca gcgcggacct gcccgacatc gcctacacgc tgcaggtcgg ccgcgaggcg 10920
atgaccgcac ggttcgccgt ggccgtcagc acgcttcccg ccctggtcga cgcgctggac 10980
gcctgcgcgc tcggcagcgg gctgcccgcg ggcgcgtatt tcaaccccgg cggcgaccgg 11040
ggcggcgcgg tcaaggactt cctcaccgac gaggacttcc aggagacggc cgtgcgctgg 11100
gcacggcgcg gaaagccggc gccgctggcc gaggcctgga ccagcggcct ggccgtcgac 11160
tgggcccgcc tccacaccga gggaccgaag ccgcgcaagg tcgcactgcc cggctacccg 11220
ttcgcccggg agcgctactg gtacaccgac ggacttccgg aactccagga aatccccgcc 11280
acgttcggga acgccgcacg gcagcccgcc gccccgcccc ctgccgtgga ggccgcgcct 11340
gcgacgacgt ccgccgtgcc cgccccgccc gcgcggccgg ccaactccta cgagcttccc 11400
gcgggcgacc tcaccctgca ccccgtctgg gagcctgtcc ggctgctgcg cggcagccct 11460

tacccgtccg cggcctcccg tgtggtggcg atcggcctcg caccggacgc gctcgcggag 11520
ctgaccgccc gccgcccgca gaccgtggtg ctggacaccg ccgcgtcatc cgccgaagag 11580
gtgcgtgacg aactcgccgt cctgggcgac ttcgaccacg tcgtcatgcg gttcccgacc 11640
gcagccgccg cccacggcgc cgaggcgcag atcagcacgc agcgcgcggc gatccggagc 11700
atgttccggg tcctcaaggc actggccctc acccgggacg agcagcggct cggactcacc 11760
ctcctgacca gcggcgcgtt cgacgcaggc ggctcgggga ccgccgaccc ggcgcaggcg 11820
agcctgcacg gtctgctcgg cggcctggcc aaggagcagc cgcactggcg catccgcgcg 11880
gtcgacctgg ccgacggcga accgttcgtc gccgacgagg tcttcgccct gcccgccgac 11940
cgccgcgcgc acccgctcgt ccgccgcggc ggccagtggc tgcgccgtca gctcctgccg 12000
gtggacgcca ccgagccgcc cgcggagccc gtgctgcgcc gcgacggcgt ctatgtgctc 12060
atcggcggcg cgggcgacct cggcgtgctg ctcagcgagt acctcgtacg gcaacacgac 12120
gcacacgtcg tatgggtcgg ccgccgcgcc gaggacgagg acatccgggc cagggcggac 12180
cgggccgcag cgggcgggcg gacccccgtc tacctgtccg ccgacgcctc cgaccccgac 12240
gcgctcgccc gcatgcggga cgaggtcgtc cgccgctacg gccgcatcga cggcgtggtg 12300
cacctggcga tggtgttcag tcacacgccg ctcgcccgga tgaccgagcg cgaactggag 12360
gccaccctcg cggccaaggt cgacccgtgc gcgcacttcg ccgacgtctt cgccgggcac 12420
ggcctggact tcgtcctgct gatctcctcg ctggtgagct tcatccgcaa ctcccaccag 12480
gcgcactact cggcggcctg cgccttcgag gacgcgcacg ccgccgccct gcgcgaggcg 12540
ctggactgcc gggtcaaggt cgtcaactgg ggctactggg gcaacgtccc cgacgagctc 12600
ctgcgcgacg tgacgtccat gggactggcc ccgatcgccc cggccacggc gatgggcgca 12660
ctggagcgcc tcctggccgg cccgctccac cagatcggct tcatgcgcct cggccgcccg 12720
ctgcccgtcg aaggggtgct caccgcggag acgctgaccc cgcagacgca cggtgcggcg 12780
gcccgcgacg gcgccgcggc cctcgctctg cccaccggcc tggccgcgta ccacgagagc 12840
ccggtcccgg gcgagatcga cgcgttcctg ctccgccgcc tcgccgccga gctgcgccga 12900
gcgggtctgg aggagccgcg ccacggcctg gccgagtgga aggagcggca gggcgtcgac 12960
gcacggttcg acggctggct ctcggccacc ctgcacgcgc tcgccgagca cgcgatgatc 13020
gacgaccggg gccgctggac caccagcagt ccggccgcca cggacgccga cgcctgccgc 13080
gccgactggg ccgcgcagac accccggtgg gccgccgcca accccgatct gcgcgcaccg 13140
ctgaacctgc tggaccggac cctgcccgcg ctccccgacg tcctgtgcgg ccgggtgcgc 13200
gccaccgacg tgctcttccc ccaggggaag ttctccctgg tcgaaggcgt ctaccgcgac 13260

aaccgcgtgg ccgcgcactt caacgccgtc ctcgccgaac acgtggcggc cttcctgcgc 13320
gcacgccggg acgccgatcc cggcgcccgc ctgcgcgtgc tggagatcgg cgcaggcacc 13380
ggcggtacca ccggccccgt gctcgaccgc ctcgcccacg aagggctgga cctggccgag 13440
tactgcttca ccgacctgtc ccaggcattc ctgcagaacg cccaggacac cttcgggccg 13500
ggccgcgacc acctcaccta ccgcatcttc gacgcggcca ggcccccgca cacccaaggg 13560
ctcgacaccg gcgccttcga cgtcgtcatc gcggccaacg tgctgcacgc caccgacacc 13620
atccgcccgg ccctgcggca cgccaaggcg ctcctgcgcg gcaacggcct gctggctctc 13680
aacgagatca gcggcttcta cctcgtcaac cacctcacct tcggcctgct cgacggctgg 13740
tggctctacg acgacgccga actgcgcgtg cccggcagcc ccgcgctgtc gccggcggcc 13800
tggcagctcg tactggaaca ggaaggcttc accggcatcc gccatccggc gcgggacgcc 13860
ctggcactcg ggcagcaggt cgtcgtggcc cacagcgacg gtctcgcccg cagcccgcgc 13920
ctgctctccg gaacgcccga gatgagcagc ccgccctccc agccgccggc ggaaaccgcg 13980
gctccggccg ccgcctccgc ttcggcccgg gccgtcacgg acgtggtgct ggccgcgctc 14040
gccgacgcgc tgcgcatgcc cgccgaccgg atcggcccgg accgggcgtt cgccgactac 14100
ggcctcgact ccatcgtcgg cgtccggttc gtccagcgcc tcaacgagga gctgggcacc 14160
gacctgccga ccacggtcgt cttcgactac cgcagcgtgg cgcagctcgc cgcccacatc 14220
gccgagagcc accggccgca accggccccc gccgcggcgg caccggtgcc cgcaccggac 14280
gccgccgggg caccgaaccg tcccgaagga cgcgagccca tcgccatcgt cgggatcagc 14340
ggccgcttcg cgcagtcgga cgacaccgac gccctctggc agcacctcgc cgccggccgc 14400
gacctcgtgg gcccggtcga acggtgggac ctctccggct acagccagga ccaactgtcc 14460
tgccgcgcgg gcagcttcct cgacggcatc gaccggttcg acgcacgctt cttccacctg 14520
accggcctcg aagccaccta caccgacccc cagcagcggc tgttcctgga acaggcgtgg 14580
acggccatcg aggatgccgg ctacgcgggc tccgcgctgg acggccgccg gtgcggcgtc 14640
tacgccggct gcaccggcgg cgactacccc cagtggttcg aggacgcgcc gcccgcccag 14700
gcggcatggg gcaacgcgcc ctcggtcgta ccggcgcgca tcgcctacca cctgaacctc 14760
cagggtcccg ccctcgcggt cgacacggcc tgctccagct cactggtcgc cgtccacctc 14820
gcctgccagg gcctgtggag cggcgaaacc gacatggccc tcgcaggagg cgtcagcgtc 14880
cagaccaccc cggacaccta cctggcggcc ggccgcggcg ggatgctctc gcccaccggc 14940
aagtgccaca ccttcgacgc cgccgccgac ggattcgtcc ccggcgaggg cgtgggcgtc 15000
gtggtgctcc gccgcctgtc cgacgcactg gccgacggcg accacatcca cgccgtgatc 15060
cgcggctccg ccgtcaacca ggacggggcg accaacggca tcaccgcacc cagcgccctg 15120

tcgcaggaac gcctcatccg ccaggtgcac accgaattcg gcatcgaccc ggccgagatc 15180
ggcatggtcg aggcgcacgg caccggcacc cagctcggcg accccatcga atgccaggcc 15240
ctggtcggcg cgttcggcac ggccggcggc agcgacacct gcgcactcgg ctcgatcaag 15300
acgaacctcg gtcacaccac ctccgccgcg ggcgtggccg gcctgctcaa ggtcgtgctc 15360
tcgctgcgcc acggtcagat cccgccgtcc ctccaccact acgagaccaa ccccgcgatc 15420
cgactcaccg aaagtccctt ccacgtgaac accacgctgc ggccgtggca gcccaacggc 15480
cagggcaagc gcgtcgccgc cctgagcgcg ttcggcttca gcggcaccaa cggccatatg 15540
gtcgtggaga acgccccgga ccgtgacgag cgccaacagg ccgccgacga gctgctgttc 15600
gtcctgtccg cccagcagcc cgaggcgctg cgccaccgcg ccgaggacct cttggcgtac 15660
ctgcgccgcg cacccgacgc cgcgctgggc gacgtcagct acacgctggc ggcaggccgg 15720
gaccacttca cccaccgcgc ggcctttgtc gccgccgacc gcgacacgct cgcccaccgg 15780
ctggaggcct ggctggccga cggacggagc gacaccgtcg gccggcgcgg cgacaccgcg 15840
ccggagcgcg cccgggcccg gtacctgaac ggcgaggagg tcgacttcgc gccgctgttc 15900
tccggcctcg acgtccgtcg cacgccgctg cccacctacc cgttccagcg caagagctac 15960
tggccgacgg ccactgctcc gagccggcgc caccaagccc cgcaggccgc gaacggccct 16020
gccgccgccc cgtcgcccga gcccgcccgg ccggcacccg cgcagccggc accggacacg 16080
gacgaggcga ccgtgcggta cctggccggc gaactcctgc tggccgagct ctcccgcgtg 16140
ctcatgatgg agcccgagga gatcgacccg caggcgtcct tctccgacta cggcgtggac 16200
tcgatcctca ccgtcaggct cgtcgcagcg gtgaacaacg ccctcgccgt cgacctgccg 16260
agcaccgcac tgttcgaaca cagctcgctc gaccggctga cggaccacct ggtcacccgg 16320
tacggggcgc agttgcggtc ctccggtgcg ctgcgcgggc cggcagccga ggccggaggg 16380
gctccggcgc aggacgacca cgggcccgcc gccgaggctc cgtccgctgc tcctgctgct 16440
cctgtcgcct ccgccggaac tgccgccgtc cccgctcacg cccccgcggc cgctgcgggc 16500
gacccggccg acgacggcgt cgccgtggtg ggcatcgccg cccggttcgc gcagtcgccc 16560
gacgccgccg ccctgtgggc acacctcgcc gcgggcgacg acctggtcgg cgaggtcacc 16620
cgctgggaca tggacgagga gctgggcgcg ggcgccccgc gccagtacgg aagcttcgtc 16680
gacgacatcg agcgcttcga cgcctggttc ttccggatgt ccggtaagga ggccacctac 16740
accgacccgc aacagcgcat cttcctggag gagtgctggc acgccctgga ggatgcgggc 16800
tacgccggtg aacggctcga cggccgcggg tgcggcgtct acgtcggcgg ctcacccagc 16860
gactaccagc agttgatcgg cgacgacgcg ccaccgcaga cactgtgggg caacatctcc 16920

tcggtcatcg cgtcgcggat ctcctacttc ctcgacctgc agggtgccgc gctggcggtc 16980
gacacggcct gctccagttc gctggtggcc attcaccagg cctgccagga cctccgcctg 17040
ggcaacacgt ccatggcgct ggcgggcgga gtcttcgtcc agtccacgcc gatcttctac 17100
cggtccgccg tgcgggcgaa catgctgtcc gcccgcgggc gctgccacac cttcgacgag 17160
cgcgccgacg gcttcgtgcc gggggagggc gccggcgtgg tcgtgctcaa gaggctcgcc 17220
gacgcgctgc gcgacggcga ccaggtctac ggcgtgatcc gcggctccgg catgaaccag 17280
gacggcacca ccaacgggct caccgcgccc agcgccggat cgcaggaacg cctcctgcgc 17340
agcgtccacg agcgcgccgg cgtcgacccc gccggcatcc agctgatcga ggcgcacggg 17400
accggcacgc cgctgggcga ccccatcgag ttcgaggccc tgcgcgccgc gttcggcgac 17460
gcgcccgagg caggctgcgc cctggggtcc gtcaagacca gcctcgggca cacccagttc 17520
gccgcgggcg tggccggcgt catcaaggtg ctgctggcgc tcaggaacga gcaactgccc 17580
ccgtcgctgc acttccgccg ggccaacccg gcgatcacgc tggagggcag cccccttctac 17640
gtcaacacgg aactgcgccc gtggcccgca cccgccgacg gtccgcgccg cgccggcgtc 17700
agctcgttcg gcgccgcagg caccaacgcg cacgcgctga tcgaacaggc cccccgccgtg 17760
cggaccgccg ggcacggccc ccggcatgcc tggctgatcg tcctgtcggc acaggacgac 17820
gccggccgcc gagcccaggc cgagcgcctg ctggaccacg ccctcgccca cgaggacctg 17880
gacctgggcg acgtggcgta caccctggcc accggacgcc gccactgcag ccaccgctgg 17940
gcgggcgtgg ccacggaccg cgagcagctc gtcgccgccc tgcggacctg gctgtgcgac 18000
ggccgggcgg agggcgtggt caccggtgag gcgcccgacg ggcaccgccg tcaggacccc 18060
gccgaggacg ccccgcgccgg ccgcctgatg gccgagcccg accgtcacga cagcctcacc 18120
gagctggccg ggctcttcgc ccaggggcaa gatctgggct tcgccccgct cttcggcgac 18180
ggcggcttcc gtatcgtctc cctcccggcc tatccgttcg cgggcgagcg ctactgggtc 18240
ggatcacgtc cggcggcccc cgctgcgacc ccggcctccg ctccggtacg cgccccggtc 18300
cccgttgcgg ccccgtcgcc gctggaaggc cgccggctga ccggtgatcc cggctcgccg 18360
tccttcgccg tcgagctggc cggccgcgag ttcttcctcg acgaccaccg ggtgcgcaac 18420
gtgccggtgc tccccggcgt ggcctatctg gagctggcgt acgcggcggc ccgggccgag 18480
ggcgtcgacc ccgcccacgc ccgcctgcgc aacgtcgtct ggtcgcgccc cgcacggatc 18540
accgggccga ccgcggtcga gatcgcgctg cggccgtgcg aggacgacgc cttcacctac 18600
gagatcacca cggcggccga cggcgaacag ccggtgatcc acgggcaagg acgcatcgag 18660
cggtgcggga cgccgtcacc cgcgcgcctg gacatcgccg cgctgcgcgc ccagtgcgag 18720
gtgcgcactc tggaacacga cgactgctac cggctcttcg accgcatggg catcggctac 18780

ggcccggcca tgcggggcat ccggcggatc cacgtcggcg ccgggctcgc cgtcgcacgc 18840
ctgagcctgc cgcaggccgc ccgggacggc gccggctggg acctgcaccc gtcgatgctc 18900
gacgccgccg tacaggccac cttgggcctg tcactggccg aggacaccga caccgtggcg 18960
ccggcactgc ccttcgtcct ggaggaggtg cagctgctcg cgcccagccc ggccggcggg 19020
tgggccgtgg tgcgacccgc agcgggcgac ggcggcggag ccgtacgccg catcgacatc 19080
gaactgtgcg acgacgacgg cgaggtgtgc gtacgcctgc tcgggttcac cgcacgcgtc 19140
ctggccgccg gtgacgaccc cgccggcgga gagaacaccg gggcgcgac gctcaccctc 19200
atgcgtgccg gctggcgccc ggccgagccc acccgggcct cgcgcccgct ggtgcaccac 19260
gaggtgctgc tcggcggact cgcagggacc gaccccgcgg cggtccggga cgggctcggt 19320
gtgccctgca ccgcattgcc cgacgacggc gatccggccc ggtgtttcac ccgccaggcc 19380
gagacggtgc tggcccgcct gcagcagttc gtcccacgca cccgcgacgg cgaggtcctg 19440
ctgcaggtgg tcgtgcccgc cgacggtgag aaccgggtcc tcgcgggcct gggcggcctg 19500
ctgcgcacgg cccgcatgga gcaccccaag ctgctgaccc agctcgtcga ggtggagacg 19560
cccgtcgacg ccgcgacgct gtgcgagcgc ctgcgccggg acgcggcgag ccccgacgac 19620
gtggccgtgc ggtactccgg cgggcagcgc cgggtgccgc agtggaccgc cgtcgaggac 19680
gccccgccgg cccgcccctg gaaagccggc ggcgtctacc tcctcaccgg gggagtcggc 19740
gggctcggcg cacacttcgc ccgcgagatc gcccggcagg cgcccggcgc cgccctcgtg 19800
ctctgcgggc gctcgccgga gggcccggcc cagcgtgaac tcctgtgtga gctgggcgac 19860
ttgggtgcct ccgccgtcta ccgggtgctg gacgtcgccc ggcgcgacgc cgtgaccgcc 19920
tgcgtgaaca ccgtcgtcgc cgagcacggc cgcctggacg gcgtcgtcca caccgccggt 19980
gtggtgcgcg acggctacct ggcccgtaag agcgccgaag agctgcggga ggtcctcgcc 20040
gccaaggtcg ccggcttcgt ccacctcgac ggagcgaccg ccgcgctcga cctggactgc 20100
ttcatcggat tctcctcact gtcggcgtac ggcaaccagg gccagggcga ctacgcggcg 20160
gccaacgcct tcatggacgc ctacgccggc ctccgccacg agcgggtggc caggggcgag 20220
cgccgcggcc gcacactggt ggtcggctgg cccctgtggg ccgacggcgg catgacggtg 20280
gacgccgcca ccgaacgccg cctgcacgac agcgtcggca tggtgccgat ccgcgccccg 20340
cacggtgtgg aggcgctgct acgcgcctac ggcaccggcg acccgcacgt cctggccgtc 20400
ttcggcgacc gcgcccgcat cgacgccacc ctcctggccg ccccggcggc cacgggcgcc 20460
gcaccggcgg tgaccgcacc cgaccgcgcc gccctgcacg cgagggtcct cggccgcgcc 20520
atcagccacg cctgcgccgt gctgggcgtt ccggcggcgg agctcgacgg tgcggtggag 20580

ctgagcgagt acggcttcga ccccgtctcg ctcaccgggt tcgccgcccg cctcaccacg 20640
gagttcgggc ttccgcccgt gcccaagccc ttctccgaac acctcaccct gggagaggtc 20700
gtggaccacc tgctcgacac ccaccccat cacttcggga cggtcccgcc ggcccccgcg 20760
cccgagccct ccgccgggcc cgaaagcgcc gccgcgcccg tcgcgacggc cggccgggag 20820
cagcagcaca aggcgctgct gaagaagctg atcgcccgcg tgtccgacct gctggacgtg 20880
cccgccgagc ggatcaccgg cacggccgag atgacccgct acggcttcga ctccctctcg 20940
ttcatcggct tcgccaacga cctcaacgcc gagttcgggc tctcgctggc accgaccctg 21000
ttcttcgaga accccaccct ggacggggtc gtcgaccacc tcctcgacca ccacgccgac 21060
cgcgtcgccg ccaccgcggc accgcagcag gaaccgcgcg cggcggcggc cccgccgcc 21120
ccagagcccg ccacagccga cacccccgcg tcccgtacgg atgcgcccgg gaacgagccg 21180
atcgccgtca tcggcatcag cggccgcttc ccgatggccg acgatctcga cgcgttctgg 21240
gagaacctca gcgaaggccg cgactgcacc cgtgaggtcc ccacggaccg ctgggactgg 21300
cgcgcccact acggcgaccc cgtcaaagag cccaacacgt cgaacgtgac gtccggcggc 21360
ttcatggacg gcgtcggcga cttcgacccg cttttcttcg acatctcccc caaggaagcg 21420
gagttgatgg atccgcagca gcggctcctg ctgatgtacg tatggaaggc gctggaggac 21480
gccgggtact cggcggaggc cctcgcgggc acgaacacgg ccctcatcgc cggcaccacc 21540
agcaccggct acagcaccct cgtcacccgg tactcgccga tgatcgaggg atacgacatc 21600
accggcgcgg ccccctccat gggcccgaac cggatgagct acttccttga cctgcacggt 21660
ccgagcgagc ccgtcgacac ggcctgttcg agcgcgctcg tcgccctgca ccgggccgtc 21720
caggccatcc gcgacggtca gtcggacctg gccatcgccg gcggcgtcaa caccatggtc 21780
agcgtcgacg ggcacatcag catctccaag gcgggcatgc tcagccccga aggccgctgc 21840
aagaccttct ccgaccgcgc ggacggttat gcccgtggtg agggcgtggg catgctggtg 21900
ctcaagagcc tgtcggcggc cgagcgcgac ggcgaccaca tctacggggt catccgctcg 21960
acggccgaga accacggcgg ccggggcagc tccctgaccg cgcccaaccc caaggcccag 22020
gccgccctcc tgcgggaggc ctacgggaag gccgggatcg atcctcggac ggtgggctac 22080
atcgaggccc acggcaccgg caccaaactc ggcgacccgg tcgagatcaa cgggctcaag 22140
gccgcgttcc gggacatgta cgaggagcac ggcgcggtgg tcgaggaggc ccactgcggt 22200
atcggctcgg tgaagaccaa tatcggtcat ctcgaactgg ccgcgggcgc cgccggcgtg 22260
atcaaggtgt tgctccagat gcggcaccgc accctggtca agagcctgca ctgcgacacc 22320
gtcaacccct acatcgacct cgacggcagc ccgttccacc tcgtacgcga acggcagccc 22380
tggcccgccc tgcgcgatgc cgaaggccgt gagctgccgc gccgggccgg agtcagctcc 22440

ttcggcttcg gcggcgtcaa cgcccatgtg gttcttgagg agtaccggcc gcgcaccgca 22500
cccgagccgg accgggcgcc caccgcaccg gtccccgtcg tcctgtcggc gagccacccc 22560
gacgtgctgt gcgaactcgc cgagcgctgg gtggacgcac tgcgccgcgg cgactacgac 22620
gacaccgaca tggcgtcgat cgcctacacc acgcagaccg gacgcacgcc catgaccgag 22680
cgcctcgcct gcctggcccg cacggccggc gaactgcggg aggcactgga gtcctggctg 22740
cgcggcgagc ccgcggccga cgtcttccgc ggcaaggtcg cgcgcggcgt cgacctgccg 22800
gacgcaccag ccgggttcgg cccgcacgac gaccacgaca gcgcgggccg gcacgactgg 22860
gcccgcctgc tccaggcatg ggtgaacggc gccccctcg actgggaccg cctccacacc 22920
gggcgccgcc cgcgccggat cgccctgccg acctacccgt tccgcctccg gcgctactgg 22980
gtcgacacct cgcgccccgc gaacggcaca caaacggagg cactgcaccc gctggtgcac 23040
acgaacacct cggacctgaa cgagcaccgc tacacctcgc acttcaccgg ccgcgagttc 23100
ttcctcgccg accaccgcgt acgcgcccag gtgatggaga cggtctccgg ctggcggccc 23160
ggccgccggc ccaccgccta cgacgtccgc gcggacgccg tgccggtgct gccggcggtg 23220
gcctacctgg agatggcgcg cgccgccgcg gtccaggcgg ccggcggcga cgagcgcgcc 23280
tggtcactga agttggcctc ctggctgcgc ccgctcaccg tcgagaaggc gaccgacgtg 23340
cacaccacgc tgaccacccg ggccggcggc ggactgagct acgaggtgta cgcggtggac 23400
gaggacggcg aacgcgtcac cttcggccgc ggccagctgc ggcgcgcaac agcggtgccc 23460
gccgagcggc tcgacctcgc ggccctgcgc gcgcagtgcg acggccccgt gctcgacgcc 23520
gagacctgct acgcacgctt caccggcatc ggcatggcct acggcccggc actgcgcggc 23580
atcgagcgcc tgcacaccgg ctcgcggcag tcggtggcgc ggctgaagct gcccgccgcc 23640
gcgtcccgcg agcgcggctg ggtactcaac ccgggcatgc tcgacgccgc cctccaagcc 23700
acggtcggcc tcttcgtcga cgaccccggc acgccgcgca cggcactgcc gttcgccctg 23760
ggcgagctgg aggtgctgcg ggcggtcccg ggcaccggct gggtcgtggt ccgcttcgcc 23820
gaggacgacc acgtgggcgc cgtgcgccgc ctcgacctcg acctctgcga cgacgacggc 23880
gaggtgtgcg tacgcctgcg cggcttcagc gtccgcacgc tcggcggcag cgagcccacc 23940
ggtgacagcg agcccacccg gcccgccgaa caggcacccg agccgccgtc cgggtccgac 24000
gacgcctacc tgctggacct gatcgaagcc attggccgac gcgagatgag cgcggacgaa 24060
ttcaagagga gcctggcatg a 24081

<210> 14
<211> 1953
<212> PRT
<213> Streptomyces platensis subsp. rosaceus

<400> 14

Val Gly Gln Asp Glu Ala Leu Arg Leu Ile Arg Asp Trp Lys Gln Glu
1 5 10 15

Gln Glu Gln Glu Gln Glu Gln Asp Gln Asn Gln Glu Gln Ala Arg Ala
20 25 30

Arg Thr Gln Thr Ala Arg Pro Ala Asp Val Ala Asp Thr Glu Ala Leu
35 40 45

Thr Glu Arg Val Cys Ala Val Val Val Glu Lys Val Cys Glu Leu Leu
50 55 60

Lys Val Thr Thr Asp Asp Leu Asp Val His Val Asp Leu Ser Glu Tyr
65 70 75 80

Gly Leu Asp Ser Leu Val Ile Thr Gln Leu Val Asn Met Val Asn Asp
85 90 95

Ala Leu Gly Leu Glu Leu Val Pro Thr Val Leu Phe Glu His Ala Thr
100 105 110

Ile Gln Ala Phe Gly Ala His Leu Thr Asp Glu Tyr Gly Pro Ala Leu
115 120 125

Ala Ala Arg Leu Gly Leu Arg Ser Pro Gly Ala Ala Thr Glu Pro Pro
130 135 140

Ala Val Glu Pro Val Gly Thr Pro Val Pro Ala Ala Ala Val Pro Ala
145 150 155 160

Arg Ala Val Pro Val Pro Leu Pro Ala Asp Arg His Asp Asp Pro Ile
165 170 175

Ala Val Val Gly Met Ser Gly Arg Phe Pro Gln Ala Glu Asp Leu Asp
180 185 190

Ala Phe Trp Arg Asn Leu Arg Asp Gly Arg Asp Cys Ile Ala Glu Val
195 200 205

Pro Ala Asp Arg Trp Asp Trp Arg Ala Leu Phe Gly Asp Pro Leu Gln
210 215 220

Glu Pro Gly Arg Thr Asn Val Lys Trp Gly Gly Phe Met Glu Gly Val
225 230 235 240

Ala Asp Phe Asp Pro Leu Phe Phe Gly Ile Ala Pro Lys Asp Ala Val
245 250 255

His Met Asp Pro Gln Gln Arg Leu Leu Met Leu Tyr Val Trp Lys Ala
260 265 270

Leu Glu Asp Ala Gly Tyr Ala Ala Asp Ala Leu Ala Gly Ser Ser Phe
275 280 285

Gly Leu Phe Val Gly Thr Ser Asp Thr Gly Tyr Gly Leu Leu Ser Asp
290 295 300

Arg Ser Ser Gly Arg Gly Glu Ser Val Thr Pro Thr Gly Ser Val Pro
305 310 315 320

Ser Val Gly Pro Asn Arg Met Ser Tyr Phe Leu Asp Val His Gly Pro
325 330 335

Ser Glu Pro Ile Glu Thr Ala Cys Ser Ser Ser Leu Val Ala Met His
340 345 350

Arg Gly Val Ile Ser Ile Glu Arg Gly Glu Cys Asp Met Ala Val Val
355 360 365

Gly Gly Ile Asn Thr Met Val Ile Pro Asp Gly His Val Ser Phe Ser
370 375 380

Lys Ser Gly Met Leu Ser Ala Glu Gly Arg Cys Lys Thr Phe Ser Asp
385 390 395 400

Arg Ala Asp Gly Tyr Ala Arg Gly Glu Gly Val Gly Met Leu Val Leu
405 410 415

Lys Ser Leu Ser Ala Ala Glu Arg Asp Gly Asp His Val Tyr Gly Ile
420 425 430

Ile Arg Ser Thr Ala Glu Asn His Gly Gly Arg Ser Asn Ser Leu Thr
435 440 445

Ala Pro Asn Pro Lys Ala Gln Ala Ala Leu Ile Arg Arg Ala Tyr Ser
450 455 460

Thr Ala Gly Ile Asp Pro Arg Thr Val Gly Tyr Ile Glu Ala His Gly
465 470 475 480

Thr Gly Thr Lys Leu Gly Asp Pro Val Glu Ile Asn Gly Leu Lys Ala
485 490 495

Ala Phe Arg Glu Leu Tyr Glu Glu His Gly Ala Val Val Asp Asp Ala
500 505 510

His Cys Gly Ile Gly Thr Val Lys Thr Asn Ile Gly His Leu Glu Leu
515 520 525

Ala Ala Gly Val Ala Gly Val Ile Lys Val Leu Leu Gln Met Arg His
530 535 540

Arg Thr Leu Ala Lys Ser Leu His Cys Asp Thr Val Asn Pro Tyr Ile
545 550 555 560

Asp Leu Asp Gly Ser Pro Phe His Leu Val Arg Glu Gln Gln Pro Trp
565 570 575

Pro Ala Leu Arg Asp Ala Glu Gly Arg Glu Leu Pro Arg Arg Ala Gly
580 585 590

Val Ser Ser Phe Gly Phe Gly Gly Val Asn Ala His Val Val Leu Glu
595 600 605

Glu Tyr Val Pro Arg Pro Val Pro Pro Val Ser Thr Pro Asp Pro Val
610 615 620

Ala Val Val Leu Ser Ala Pro Glu Pro Glu Met Leu Arg Ala Arg Ala

625 630 635 640

Arg Gln Leu Ala Asp Arg Ile Asp Ser Gly Gly Leu Gly Glu Ala Asp
645 650 655

Leu Pro Asp Leu Ala His Thr Leu Gln Val Gly Arg Val Ala Met Asp
660 665 670

Glu Arg Leu Ala Phe Leu Thr Ser Ser Leu Ala Asp Leu Arg Glu Arg
675 680 685

Leu Gly Ala Phe Leu Asp Gly Gly Thr Val Gln Gly Leu His Thr Gly
690 695 700

Arg Ala Gln Arg Pro Gly Pro Trp Asn Glu Leu Ala Gly Asp Asp Asp
705 710 715 720

Ile Ala Leu Ala Leu Asp Ser Trp Ile Ala Lys Gly Lys Leu Gly Arg
725 730 735

Leu Leu Lys Leu Trp Val Thr Gly Phe Asp Val Asp Trp Arg Arg Leu
740 745 750

Tyr Ala Gly Arg Pro Met Arg Arg Ile Pro Leu Pro Val Tyr Pro Phe
755 760 765

Gln Leu Lys Arg Tyr Trp Ile Thr Asp Ala Lys Ser Thr Thr Arg Pro
770 775 780

Pro Ala Pro Val Ala Ala Ala Pro Asp Ala Gln Pro Ser Pro Tyr Arg
785 790 795 800

Arg Asp Leu Thr Gly His Glu Phe Tyr Val Ser Asp His Arg Val Gly
805 810 815

Asp Thr Pro Val Leu Pro Gly Thr Ala Tyr Leu Glu Phe Val Arg Asp
820 825 830

Ala Leu Val Arg Ala Thr Ser Ala Gly Thr Ala Thr Gly Val Arg Leu
835 840 845

Arg Asp Val Thr Trp Leu Arg Pro Leu Glu Val Thr Ala Leu Arg Thr
850 855 860

Leu Ala Val Asp Val Asp Pro Ala Gly Gly Thr Phe Glu Val Tyr Asp
865 870 875 880

His Gly Ser Gly Asp Arg Val Leu His Ala Asn Gly Thr Ala His Val
885 890 895

Asp Pro Ala Leu Leu Ala Ala Asp Asp Thr His Asp Ile Asp Ala Leu
900 905 910

Arg Ala Asn Leu Pro Phe Arg Arg Asp Gly Ala Glu Cys Tyr Ala Leu
915 920 925

Phe Ala Arg Arg Gly Met Gly Tyr Gly Pro Ala Phe Arg Ala Val Gln
930 935 940

Glu Leu Tyr His Gly Ala Asp Thr Ala Leu Ala Arg Leu Leu Leu Pro
945 950 955 960

Glu Ala Ala Ala Ser Ser Leu Thr Leu Asn Pro Val Met Leu Asp Ala
965 970 975

Ala Leu Gln Ala Thr Leu Gly Leu Ala Leu Gly Glu His Val Asp Ala
980 985 990

Pro Gln Gly Thr Ala Leu Pro Phe Thr Val Arg Glu Val Gln Val Leu
995 1000 1005

Ala Pro Thr Pro Ala Glu Gly Trp Ala Leu Val Arg Arg Ala Ala
1010 1015 1020

Asp Asp Arg His Asp Thr Gly Ile Arg Arg Leu Asp Ile Asp Leu
1025 1030 1035

Cys Asp Thr Gln Gly Asn Val Cys Val Arg Leu Leu Gly Phe Ser
1040 1045 1050

Thr Arg Met Lys Pro Ser Pro Ala Pro Arg Ala Ala Glu Pro Thr
1055 1060 1065

Thr Thr Pro Ala Leu Leu Ile Gln Ala Asp Trp Arg Glu Ser Ala
1070 1075 1080

Ala Arg Glu His His Gly Asp Asp Val Lys Arg His Val Val Leu
1085 1090 1095

Cys Glu Leu Pro Ala Ala Asp Ala Thr Ala Leu Gly Ala Ala Leu
1100 1105 1110

Gly Gly Ala Thr Cys Glu Thr Trp Gln Ala Arg Gly Glu Thr Gly
1115 1120 1125

Thr Arg Tyr Thr Glu Tyr Ala Glu Arg Leu Leu Lys Leu Leu Arg
1130 1135 1140

Asp Lys Ala Pro Glu Ala Ala Arg Gln Pro Cys Leu Ile Gln Val
1145 1150 1155

Val Thr Pro Ala His Ala Pro Trp Leu Gly Gly Leu Ser Gly Met
1160 1165 1170

Leu Arg Thr Ala Arg Met Glu His Pro Lys Leu Leu Thr Gln Trp
1175 1180 1185

Ile Ala Leu Asp Gly Asp Gly Ala Leu Ala Pro Ala Glu Leu Ala
1190 1195 1200

Gly Arg Leu Arg Cys Asp Gly Ala Asp Thr Ala Glu Glu Ala Val
1205 1210 1215

Arg Tyr Arg Gly Gly Arg Arg Gln Val Ser Gln Trp His Glu Val
1220 1225 1230

Ala Pro Ala Ala Pro Glu Arg Pro Trp Arg Asp Gly Gly Val Tyr
1235 1240 1245

Leu Leu Thr Gly Gly Ala Gly Gly Leu Gly Ala Leu Phe Ala Gln
1250 1255 1260

Asp Ile Ala Arg Arg Val Glu Thr Pro Ala Leu Val Leu Cys Gly
1265 1270 1275

Arg Ser Pro Val Gly Pro Ala Gln Gln Glu Leu Leu Thr Ala Leu
1280 1285 1290

Arg Ala Leu Gly Ala Arg Ala Asp Tyr Arg Val Leu Asp Val Ala
1295 1300 1305

Asp Arg Ala Asp Val Thr Arg Val Val Arg Glu Val Gln Ala Glu
1310 1315 1320

Tyr Gly Ala Leu His Gly Ile Val His Ala Ala Gly Val Leu Arg
1325 1330 1335

Asp Gly Phe Val Ala Lys Lys Thr Ala Asp Asp Leu Arg Glu Val
1340 1345 1350

Phe Ala Ala Lys Val Ala Gly Leu Cys His Leu Asp Glu Ala Thr
1355 1360 1365

Ala Ser Val Pro Leu Asp Cys Phe Ile Gly Phe Ser Ser Met Ala
1370 1375 1380

Ala Phe Gly Asn Val Gly Gln Ala Asp Tyr Ala Ala Ala Asn Ala
1385 1390 1395

Phe Met Asp Gly Tyr Ala Ala His Arg Asp Ser Leu Val Asp Gln
1400 1405 1410

Gly Ser Arg Ser Gly Arg Thr Leu Met Val Asn Trp Pro Leu Trp
1415 1420 1425

Glu Lys Gly Gly Met Gly Ala Asp Pro Ser Thr Val Gln Leu Leu
1430 1435 1440

Glu Ser Val Gly Met Arg Pro Met Arg Ala Ser Val Gly Ile Asp
1445 1450 1455

Ala Leu Asp Arg Val Trp Ala Thr Gly Leu Pro Ser Ala Ile Ala
1460 1465 1470

Leu Asp Gly Asp His Ala Arg Met Arg Glu Arg Phe Leu Pro Ala
1475 1480 1485

His Pro Glu Pro Glu Ala Pro Ala Glu Pro Ala Pro Ala Ala Ala
1490 1495 1500

Thr Leu Pro Ala Thr Ala Pro Val Ala Glu Pro Ala Glu Pro Ser
1505 1510 1515

Ser Val Gly Thr Val Val Ala Asp Leu Met Ala Thr Leu Leu Glu
1520 1525 1530

Val Asp Val Glu Thr Leu Arg Trp Asp Lys Ser Leu Gly Asp Tyr
1535 1540 1545

Gly Phe Asp Ser Ile Phe Met Met Gln Phe Leu Ala Gln Ala Gln
1550 1555 1560

Thr His Leu Asp Ala Ser Leu Thr Leu Asp Val Ile Ala Asp Cys

1565 1570 1575

Glu Thr Leu Gln Asp Val Val Asp Ala Ile Thr Gly Thr Ala Ser
1580 1585 1590

Asp Thr Gly Thr Ala Ala Pro Lys Pro Ala Ser Val Ala Pro Val
1595 1600 1605

Glu Glu Ala Pro Ala Ala Ala Ala Pro Ala Lys Ala Pro Val Arg
1610 1615 1620

Arg Ala Ala Ala Ala Ser Pro Asn Asp Phe Pro Glu Leu Val Arg
1625 1630 1635

Met Asn Gly Val Thr Ser Gly Arg Pro Val Phe Trp Val His His
1640 1645 1650

Gly Asn Gly Gly Val Glu Ser Tyr Ala Pro Leu Ala Ala Arg Cys
1655 1660 1665

Pro Arg Pro Phe Tyr Gly Ile Gln Pro Lys Gly Trp Ile Asp Ser
1670 1675 1680

Thr Asp Ile Leu Thr Gly Gln Tyr Ala Met Ala Glu His Tyr Ala
1685 1690 1695

Ser Leu Ile Leu Ala Val Gln Pro Glu Gly Pro Tyr Asp Ile Gly
1700 1705 1710

Gly Phe Ser Leu Gly Gly Leu Phe Ala Tyr Glu Thr Val Arg Gln
1715 1720 1725

Leu Gln Leu Thr Gly Ala Asp Val Arg Thr Leu Val Met Leu Asp
1730 1735 1740

Thr Leu Asp Ala Glu Ser Thr Asn Lys Ala Asn Ala Leu Ile Val
1745 1750 1755

Gly Gly Asn Phe Asp Ala Asp Val Val Thr Lys Val Ser Asp Phe
1760 1765 1770

Arg Ala Val Asn Leu Ile Leu Gly Asn Asn Arg Phe Asp Ser His
1775 1780 1785

Gly Gly Ala Thr Pro Ile Leu Arg Arg Asp Glu Val Asp Thr Thr
1790 1795 1800

Leu Glu Pro Lys Glu Phe Leu Gly Ser Leu Ile Asp Ala Ala Leu
1805 1810 1815

Ala Arg Gly Val Ser Lys Thr Glu Thr Gln Leu Arg Ser Arg Val
1820 1825 1830

Arg Gln Leu Ser Arg Tyr Phe Glu Ala Thr Gln Gly Glu Thr Tyr
1835 1840 1845

Thr Val Asp Pro Leu Pro Arg Arg Asp Gly Leu Arg Cys Tyr Tyr
1850 1855 1860

Leu Arg Asn Arg Gly Gly Lys Phe Phe Gly Ala Phe Glu Glu His
1865 1870 1875

Met Val Leu Phe Pro Asn Pro Glu Leu Pro Thr Val Asp Gly Val
1880 1885 1890

Ala Tyr Trp Gln Glu Trp Ala Asp Gln Ile Asp Asp Phe Phe Thr
1895 1900 1905

Ile Asp Val Asp Thr Ser Met His Ala Glu Val Met Thr Ala Pro
1910 1915 1920

Gln Ser Leu Asp Lys Leu Met Arg Leu Cys Asp Arg Leu Tyr Ala
1925 1930 1935

Ala Glu Asp Ala Ala Ala Pro Ala Thr Ser Ala Gln Gly Gly Arg
1940 1945 1950

<210> 15
<211> 5862
<212> DNA
<213> Streptomyces platensis subsp. rosaceus

<400> 15

```
gtgggacagg acgaagcgct ccgcctgatc cgcgactgga agcaggagca ggagcaggag      60
caggagcagg atcagaatca ggagcaggcg cgagcacgga cgcagaccgc acggccggct     120
gacgtcgccg acaccgaggc cctgacggag cgggtctgcg ccgtcgtggt ggagaaggtc     180
tgcgagctgc tcaaggtcac cacggacgac ctggacgtgc atgtcgacct cagcgaatac     240
gggctcgact ccctcgtcat cactcagctg gtgaacatgg tgaacgacgc tctgggtctg     300
gaactcgtgc ccaccgtgct gttcgagcac gcgacgatcc aggccttcgg cgcccacctg     360
accgacgagt acggccctgc gctggccgcc cgcctggggc tgcggtcgcc cggcgccgcc     420
acggagcccc ctgccgtcga gcccgtcggt acgcctgtgc cggccgcagc cgtccccgca     480
cgggccgtac ccgtcccgct gcccgccgac cggcacgacg acccgatcgc ggtggtcggc     540
atgagcggcc ggttccccca ggccgaggac ctcgacgcct tctggcgcaa cctgcgcgac     600
ggccgcgact gcatcgcgga agtccccgcc gaccggtggg actggcgcgc cctcttcggc     660
gacccccttc aggaaccggg ccgcaccaac gtgaagtggg gcgggttcat ggagggcgtc     720
gccgacttcg atccgctgtt cttcggcatc gctccgaagg acgccgtcca catggacccg     780
cagcagcgcc tgctgatgct gtacgtgtgg aaggcgctgg aggacgccgg ctacgccgcc     840
gacgccctgg ccgggagcag cttcggcctg ttcgtcggca ccagcgacac cggctacggc     900
ctgctctccg accgcagcag cggcaggggc gagagcgtca cgcccacggg cagcgtcccc     960
tccgtcggcc cgaaccggat gagctacttc ctggacgtac acgggccgag cgagccgatc    1020
gagacggcct gttcgagttc cctggtcgcc atgcaccgcg gcgtcatctc gatcgaacgc    1080
ggcgagtgcg acatggccgt cgtcggcggt atcaacacca tggtgatccc cgatggccac    1140
gtcagcttca gcaagtccgg gatgctcagc gccgaggggc gctgcaagac cttctccgac    1200
```

cgcgcggacg gttatgcccg tggtgagggc gtgggcatgc tggtgctcaa gagcctgtcg 1260
gcggccgagc gcgacggcga ccacgtctac ggcatcatcc gctcgacggc cgagaaccac 1320
ggcggccgct ccaactccct gaccgcgccc aaccccaagg cccaggccgc cctgatccgg 1380
cgcgcctaca gcaccgcggg catcgaccct cggacggtgg gctacatcga ggcccacggc 1440
accggcacca agctcggcga cccggtcgag atcaacgggc tcaaggccgc gttccgggaa 1500
ctgtacgagg agcacggcgc ggtggtcgac gacgcccact gcggtatcgg cacggtgaag 1560
accaacatcg gccacctcga actcgcggcg ggcgtcgccg gcgtgatcaa ggtgctgctg 1620
cagatgcggc accgcacgct cgccaagagc ctgcactgcg acaccgtcaa cccctacatc 1680
gacctcgacg gcagcccgtt ccacctcgta cgcgagcagc agccctggcc cgccctgcgc 1740
gatgcggagg gccgtgagct gccgcgccgg gccggagtga gctccttcgg cttcggcggc 1800
gtcaacgccc atgtggtgct tgaggagtac gtgccgcgcc ccgtaccgcc ggtgagcaca 1860
ccggaccccg tggccgtcgt cctgtcggcc cccgagcccg agatgctgcg cgcccgggcc 1920
cggcagctgg ccgaccggat cgactcgggc gggctcggcg aggccgacct gccggacctc 1980
gcccacacgc tgcaggtggg ccgcgtcgcg atggacgagc gcctcgcctt cctgacctcc 2040
tcgctcgccg acctgcgcga gcggctgggc gccttcctcg acggcggcac cgtacagggc 2100
ctccacaccg gacgggcaca gcgcccgggg ccgtggaacg agctcgccgg agacgacgac 2160
atcgccctcg ccctcgacag ctggatagcc aagggcaagc tcggacgcct gctcaaactc 2220
tgggtcaccg gcttcgacgt ggactggcgg cgcctgtacg ccggccggcc gatgcggcgc 2280
atcccgctgc ccgtctaccc gttccagctg aagcgctact ggatcaccga cgcgaagagc 2340
acgacccggc ccccggcacc ggtggccgcg gcgccggacg cacaaccgtc gccctaccgc 2400
cgcgacctga ccgggcacga gttctacgtg agcgaccacc gcgtggggga cacgcccgtc 2460
ctgcccggca ccgcctacct cgagttcgtg cgcgacgcgc tcgtccgggc cacgtccgca 2520
ggcaccgcca cgggcgtgcg cctgcgcgac gtgacctggc tgcgtcccct ggaggtgacg 2580
gcactgcgca ccctcgccgt cgacgtggac ccggccggtg ggacattcga ggtgtacgac 2640
cacggctccg gcgaccgcgt cctgcacgcg aacggcaccg cacacgtcga ccccgcactc 2700
ctcgccgccg acgacaccca cgacatcgac gcactgcgcg cgaacctccc gttccggcgt 2760
gacggcgccg agtgctacgc gctgttcgcg cgcaggggca tgggatacgg ccccgcgttc 2820
cgggccgtgc aggagctgta ccacggtgcg gacaccgccc ttgcccgcct cctcctcccc 2880
gaggcggcgg catcctcgct gacgctcaac ccggtcatgc tcgacgccgc cctgcaggcg 2940
accctgggac tggcgctcgg cgagcacgtc gacgccccgc aggggacggc acttccgttc 3000

accgtgcgcg aggtacaggt cctggccccc accccggccg agggctgggc gctcgtgcgc 3060
cgtgccgcgg acgaccgcca cgacaccggc atacgccgcc tggacatcga cctctgtgac 3120
acgcagggca acgtctgcgt gcgtctgctg ggcttttcca cccgcatgaa gccgagcccg 3180
gcgccccgcg ccgccgagcc gaccaccacg cccgcactgc tgatccaggc ggactggcgc 3240
gagagcgcgg cacgggagca ccacggcgac gacgtcaagc ggcacgtcgt cctgtgcgaa 3300
ctcccggcgg cggacgccac cgcgctcggc gcagcgctgg gcggcgccac ctgcgaaacc 3360
tggcaggccc gcggcgagac cggcacccgc tacaccgagt acgccgagcg gttgctgaag 3420
ctgctgcgcg acaaggcacc ggaggccgcc cggcagccgt gcctgatcca ggtcgtcacc 3480
cccgcgcacg cgccctggct gggcgggttg agcggcatgc tccgcacggc gcgcatggag 3540
caccccaagc tgctgacgca gtggatcgct ctggacggtg acggcgccct ggccccggcc 3600
gagctggccg gacggctgcg gtgcgacggc gccgacacgg ccgaggaggc cgtgcgctac 3660
cgcggaggac gccggcaggt gtcccagtgg cacgaagtcg caccggccgc acccgaacgg 3720
cgctggcgcg acggcggcgt ctacctgctg accggcggcg ccggaggact cggcgccctg 3780
ttcgcgcagg acatcgcccg gcgcgtcgag acgcccgccc tggtcctgtg cggtcgcagc 3840
ccggtcggcc cggcacagca ggaactgctt accgccctgc gcgcgctggg tgcccgtgcc 3900
gactaccgcg tgctcgatgt cgccgaccgc gccgacgtga cccgggtcgt gcgcgaggtc 3960
caggccgagt acggcgcgct gcatggcatc gtgcacgccg ccggagtgct gcgcgacggc 4020
ttcgtggcca agaagaccgc ggacgacctc cgcgaggtgt tcgcggccaa ggtggccggg 4080
ctgtgccacc tcgacgaggc gactgcctcc gtcccgctcg actgcttcat cggcttctcc 4140
tccatggccg ccttcggcaa cgtcggacag gccgactacg ccgccgccaa cgccttcatg 4200
gacggatacg ccgcccaccg cgactccctg gtggaccagg gcagccggtc gggccgcacc 4260
ctgatggtga actggccgct gtgggagaag ggcggcatgg gcgccgaccc gtcgaccgtc 4320
cagctcctgg agtccgtggg catgcggccg atgcgcgcat ccgtgggcat cgacgccctc 4380
gaccgcgtct gggcgaccgg cctgcccagc gccatcgccc tcgacggcga ccacgcccgg 4440
atgcgggagc gcttcctgcc ggcgcacccc gagccggagg cccctgccga acccgcgccg 4500
gccgccgcga cgttgccggc caccgcaccg gtcgccgagc cggccgagcc gtcgagcgtg 4560
ggaaccgtcg tcgcggacct catggcgaca ctgctggagg tcgacgtcga gaccctgcgg 4620
tgggacaagt ccctgggtga ctacggcttc gactccatct tcatgatgca gttcctcgcc 4680
caggcgcaga cgcacctcga cgcgtccctc accctcgacg tcatcgccga ctgcgaaacg 4740
ctgcaggacg tcgtcgacgc gatcaccggc accgcctctg acaccggcac ggccgcccca 4800
aagcccgctt cggtggctcc cgttgaggag gccccggcgg ccgccgcacc ggcaaaggcc 4860

ccggtacggc gcgccgcggc cgcatcgccg aacgacttcc ccgaactggt ccgcatgaac 4920
ggtgtcacct ccggccggcc cgtgttctgg gtccaccacg gcaacggcgg cgtggagtcc 4980
tacgccccgc tggccgcacg ctgcccgcgt cccttctacg gcatccagcc gaagggctgg 5040
atcgactcca ccgacatcct caccggtcag tacgccatgg ccgagcacta cgcgtccctc 5100
atcctcgccg tacagccgga aggcccgtac gacatcggcg ggttctccct gggcggcctg 5160
ttcgcgtacg agaccgtgcg gcagctccag ctgacgggcg ccgacgtgcg cacgctggtc 5220
atgctggaca cgctggacgc cgaatccacc aacaaggcca acgccctcat cgtcggcggg 5280
aacttcgacg ccgacgtggt caccaaggtg agcgacttcc gcgccgtcaa cctgatcctc 5340
ggcaacaacc gcttcgactc gcacggcggc gccaccccga tcctgcgccg cgacgaggtc 5400
gacaccaccc tggaacccaa ggagttcctc ggctccctga tcgacgccgc cctcgcccgc 5460
ggcgtcagca agaccgagac gcagctgcgc tcccgcgtcc ggcaactgtc ccgctacttc 5520
gaggccacgc agggcgagac gtacacggtg gacccgctgc cgcggcgcga cggactgcgc 5580
tgctactacc tgcgcaaccg gggcggcaag ttcttcggcg ccttcgagga gcacatggtg 5640
ctcttcccga accccgagct ccccaccgtg gacggcgtcg cgtactggca ggagtgggcc 5700
gaccagatcg acgacttctt caccatcgac gtcgacacct cgatgcatgc cgaggtcatg 5760
acggccccgc agtcgctcga caagctgatg cgcctctgcg accggctcta cgccgccgag 5820
gacgccgccg ccccggcgac ctccgcgcag ggaggccgct ga 5862

<210> 16
<211> 751
<212> PRT
<213> Streptomyces platensis subsp. rosaceus

<400> 16

Met Lys Ala Val Val Phe Pro Gly Gln Gly Ala Gln Arg Arg Gly Met
1 5 10 15

Gly Arg Glu Leu Phe Asp Ala Tyr Pro Glu Leu Ala Asp Glu Ala Ser
20 25 30

Glu Val Leu Gly Tyr Ser Leu Arg Thr Leu Cys Leu Asp Asp Pro His
35 40 45

Gln Gln Leu Gly Arg Thr Glu Tyr Thr Gln Pro Ala Leu Phe Val Val
50 55 60

Gly Ala Leu Ala His Arg Gln Trp Arg Glu Ser Thr Gly Asp Glu Pro
65 70 75 80

Ala Phe Leu Ala Gly His Ser Leu Gly Glu Tyr Cys Ala Leu His Ala
85 90 95

Ala Gly Ala Phe Asp Phe Ala Thr Gly Leu Arg Leu Val Gln Arg Arg
100 105 110

Gly Ala Leu Met Ala Gln Ala Arg Gly Gly Gly Met Ala Ala Val Val
115 120 125

Gly Val Asp Ala Ala Arg Leu Arg Glu Leu Leu Asp Glu Gly Gly Phe
130 135 140

Cys Arg Leu Thr Val Ala Asn Asp Asn Ala Pro Gln Gln Lys Val Val
145 150 155 160

Ser Gly Asp Thr Ala Thr Val Asp Ala Leu Val Ala Tyr Leu Glu Ala
165 170 175

Arg Asp Val Arg Cys Val Arg Leu Asn Val Ser Gly Ala Phe His Ser
180 185 190

Pro Leu Met Arg Gln Ala Gln Gln Asp Phe Ala Arg Phe Ala Asp Gly
195 200 205

Phe Ala Leu Gly Asp Pro Ala Thr Pro Val Ile Ala Asn Ala Thr Ala
210 215 220

Arg Pro Tyr Val Pro Gly Arg Thr Ala Arg Thr Leu Val Asp Gln Ile
225 230 235 240

Val Gln Pro Val Arg Trp Thr Glu Ser Val His His Leu Leu Asp Gln
245 250 255

Gly Val Thr Asp Phe Val Glu Leu Gly Gly Arg Val Leu Val Arg Leu
260 265 270

Ile Asp Gln Ile Arg Ser Ala Pro Arg Pro Val Ala Gln His Asp Ala
275 280 285

Pro Ala Ala Arg Pro Asp Thr Pro Ala Ala Ala Leu Gly Ser Pro Leu
290 295 300

Phe Arg Arg Arg Met Gly Val Arg His Ala Tyr Ala Val Gly Gly Met
305 310 315 320

Tyr Arg Gly Ile Ala Ser Ala Gln Met Val Val Arg Leu Gly Arg His
325 330 335

Arg Ile Leu Gly Phe Leu Gly Thr Gly Gly Leu Pro Leu Pro Glu Ile
340 345 350

Glu Gln Gly Val Lys Glu Val Gln His Gly Leu Ala Asp Gly Gln Pro
355 360 365

Tyr Gly Val Asn Val Leu Ala Asp His Asp Asp Pro Ala Ala Glu Arg
370 375 380

Ala Leu Val Asp Leu Leu Met Arg His Gly Val Pro Val Ile Glu Ala
385 390 395 400

Ser Ala Phe Met Gln Met Thr Pro Ala Leu Val Leu Tyr Arg Ala Arg
405 410 415

Gly Leu Arg Arg Gly Ala Asp Gly Arg Thr Val Cys Asp His Arg Ile

420 425 430

Val Ala Lys Val Ser Arg Pro Glu Val Ala Glu Gln Phe Met Ala Pro
435 440 445

Ala Pro Gly Pro Val Leu Asp Arg Leu Arg Arg Glu His Ala Leu Thr
450 455 460

Asp Glu Gln Ala Glu Leu Ala Arg Thr Val Pro Met Ser His Asp Ile
465 470 475 480

Thr Val Glu Ala Asp Ser Gly Gly His Thr Asp Gly Gly Val Ala Thr
485 490 495

Val Met Met Pro Ala Met Leu Lys Leu Arg Gln Gln Ala Gln Asp Arg
500 505 510

Tyr Gly Tyr Asp Glu Pro Ile Cys Met Gly Leu Ala Gly Gly Leu Gly
515 520 525

Thr Pro Ala Ala Val Ala Ala Ala Phe Met Leu Gly Ala Asp Tyr Val
530 535 540

Leu Thr Gly Ser Ile Asn Gln Cys Thr Val Glu Ser Gly Met Ser Thr
545 550 555 560

Glu Val Lys Asp Met Leu Gln Asp Val Gly Ile Ala Asp Thr Ala Tyr
565 570 575

Ala Pro Ala Gly Asp Met Phe Glu Phe Gly Ala Lys Val Gln Val Leu
580 585 590

Arg Lys Gly Val Phe Phe Pro Thr Arg Ala Asn Arg Leu Phe Ser Leu
595 600 605

Tyr Ser His Tyr Asp Gly Leu Asp Asp Ile Pro Gln Lys Thr Arg Ser
610 615 620

Leu Leu Glu Arg Thr Tyr Phe Gly Lys Ser Ile Glu Glu Val Trp Asp
625 630 635 640

Glu Val Arg Ala Tyr Leu Arg Ser Gln Gly Arg Asp Ala Asp Ile Asp
645 650 655

Arg Ala Asp Ala Glu Pro Lys Gln Lys Met Ala Leu Val Phe Arg Trp
660 665 670

Tyr Phe Phe His Thr Thr Arg Leu Ala Met Asp Gly Asp Gly Ser Gly
675 680 685

Lys Val Asn Tyr Gln Val Gln Thr Gly Pro Ala Leu Gly Ala Phe Asn
690 695 700

Gln Trp Val Glu Gly Thr Glu Leu Ala Ser Trp Arg His Arg His Val
705 710 715 720

Asp Arg Ile Gly Leu Met Leu Leu Asp Gly Ala Ala Glu His Ile Ala
725 730 735

Thr Ala Cys Arg His Trp Arg Asp Thr Leu Gly Val Pro Ser Ala
740 745 750

<210> 17
<211> 2256
<212> DNA
<213> Streptomyces platensis subsp. rosaceus

<400> 17

```
atgaaggcag tcgtctttcc cggccagggc gcccagcggc gcggcatggg acgcgagctg      60
ttcgacgcgt atcccgaact cgccgacgaa gcctccgaag tcctcggcta ctccctccgc     120
acgctgtgcc tggacgatcc gcaccagcaa ctgggccgca ccgagtacac gcagcccgcg     180
ctgttcgtgg tcggggcgct cgcccaccgg cagtggcgcg agagcaccgg cgacgagccg     240
gccttcctcg ccgggcacag cctgggggag tactgcgccc tgcacgccgc cggcgccttc     300
gacttcgcga ccggactgcg cctcgtccag cggcgcggcg cactgatggc gcaggcacgg     360
ggcggtggca tggcggccgt ggtcggggtc gacgcggcac ggctgcgcga actcctcgac     420
gaaggcggct tctgccgcct gaccgtcgcc aacgacaacg caccccagca gaaggtcgtg     480
tccggcgaca ccgcgaccgt cgacgcgctg gtggcatacc tcgaggcgcg cgacgtccgc     540
tgcgtgagac tgaacgtgtc cggcgccttc cactcgcccc tgatgcggca ggcacagcag     600
gacttcgccc gcttcgccga cggattcgcc ctcggggacc cggcgacgcc cgtgatcgcc     660
aacgccacgg cgcgcccgta cgtccccggc cggaccgcgc gcacactcgt agaccagatc     720
gtgcagcccg tgcgctggac cgagagcgtg caccacctcc tcgaccaggg cgtcaccgac     780
ttcgtcgaac tgggaggccg ggtgctcgtc aggctgatcg accagatccg ctccgccccg     840
cggccggtcg cccagcacga tgcaccggct gcccggcccg acacaccggc cgccgcgctc     900
ggcagcccct tgttccggcg gcgcatgggc gtgcgccacg cctacgccgt gggcggcatg     960
taccggggaa tcgcctcggc ccagatggtc gtcaggctcg gccgtcaccg cattctcggc    1020
ttcctgggaa ccggcggcct gccgctcccg gagatcgagc agggggtgaa ggaggtccag    1080
cacggcctgg ccgacgggca gccctacggc gtcaacgtac tggccgacca cgacgatccc    1140
gcggccgagc gcgcgctggt cgacttgctg atgcgccacg gcgtccccgt catcgaggcg    1200
tcagccttca tgcagatgac ccccgcgctc gttctctacc gggcacgggg actccgccgc    1260
ggtgccgacg gccggacggt gtgcgaccac cgcatcgtgg ccaaggtctc ccggccggag    1320
gtcgccgagc agttcatggc acccgccccc gggccggtcc tggacaggct ccgccgggag    1380
cacgccctca ccgacgaaca ggcggaactc gcccggacgg tgccgatgag ccacgacatc    1440
acggtcgagg cggactccgg agggcacacg gacggcggcg tcgccacggt catgatgccc    1500
gccatgctca agctccggca gcaggcccag gaccggtacg gctacgacga accgatctgc    1560
atggggctcg ccggcggcct cggcaccccc gcggcggtcg cggcggcctt catgctgggc    1620
```

gccgactacg tactcaccgg atccatcaac cagtgcacgg tggaatccgg catgagcacc 1680
gaggtcaagg acatgctgca ggacgtcggc atcgccgaca ccgcctacgc gcccgcaggc 1740
gacatgttcg aattcggtgc caaggtgcag gtgctccgca aaggcgtctt cttccccacg 1800
cgggccaaca gactgttctc cctctactcc cactacgacg gcctcgacga tattccgcag 1860
aaaacgcgct ccctcctgga gagaacctac ttcggaaaga gcatcgaaga ggtctgggac 1920
gaagtacgcg cctatctccg ttcgcagggg cgcgacgccg acatcgaccg ggccgacgcc 1980
gagcccaaac agaagatggc gctggtattc cgctggtact tcttccacac cacgcgcctc 2040
gccatggacg gcgacggctc cggaaaagtg aactaccagg tccagaccgg tccggcgctg 2100
ggtgccttca accagtgggt cgaaggcacc gaactcgcct catggcggca ccgccacgta 2160
gaccggatcg gcctcatgct gctcgacggt gccgccgaac acatcgccac cgcatgccgg 2220
cactggcgcg acaccctcgg ggtgcccagt gcgtga 2256

<210> 18
<211> 338
<212> PRT
<213> Streptomyces platensis subsp. rosaceus

<400> 18

Met Thr Val Pro Ser Thr Gly Thr Glu Val Arg Leu Ala Thr Arg Pro
1               5                   10                  15

Glu Gly Trp Pro Thr Thr Glu Asn Phe Ser Val Val Gln Ala Glu Pro
            20                  25                  30

Pro Ala Val Arg Thr Gly Gln Val Leu Ile Arg Asn Leu Val Met Ser
        35                  40                  45

Val Asp Pro Tyr Met Arg Gly Arg Met Asn Lys Thr Arg Ser Tyr Val
    50                  55                  60

Pro Pro Phe Ala Val Gly Lys Ala Leu Asp Gly Gly Ala Val Gly Glu
65                  70                  75                  80

Val Val Val Ser Lys Ser Ser Gln Leu Ala Val Gly Asp Leu Val Leu
                85                  90                  95

His Gly Leu Gly Trp Arg Glu Tyr Ala Val Val Gly Ala Ala Gly Ala
            100                 105                 110

Val Arg Ile Asp Pro Ala Leu Ala Pro Pro Gly Ala Tyr Leu Gly Val
        115                 120                 125

Leu Gly Met Pro Gly His Ala Ala Tyr Thr Gly Leu Leu Lys Ala Ala
    130                 135                 140

Glu Phe Arg Pro Gly Asp Thr Val Phe Val Ser Gly Ala Ala Gly Ala
145                 150                 155                 160

Val Gly Ser Leu Val Gly Gln Ile Ala Arg Leu Cys Gly Ala Glu Arg

165 170 175

Val Ile Gly Ser Ala Gly Ser Ala Glu Lys Val Ala Tyr Leu Thr Gly
180 185 190

Glu Leu Gly Phe Asp Ala Ala Phe Asp Tyr Lys Asp Gly Pro Val Leu
195 200 205

Glu Gln Leu Ala Lys Ala Ala Pro Thr Gly Ile Asp Val Tyr Phe Asp
210 215 220

Asn Val Gly Gly Asp His Leu Asp Ala Ala Leu Val Leu Ala Arg Met
225 230 235 240

Gly Ala Arg Phe Ala Leu Cys Gly Asn Ile Ser Gln Ala Asn Glu Lys
245 250 255

Asp Pro Pro Ala Gly Pro Arg Asn Leu Thr Gln Ala Ile Ala Lys Gly
260 265 270

Ile Thr Leu Arg Gly Val Leu Val Gly Gly His Ala Asp Leu Pro Asp
275 280 285

Glu Phe Thr Ala Arg Met Gly Gly Trp Leu Ala Asp Gly Arg Ile Ser
290 295 300

Tyr Arg Glu Thr Val Val Arg Gly Leu Glu Asn Ala Pro Ala Ala Phe
305 310 315 320

Ile Asp Met Leu Arg Gly Ala Asn Thr Gly Lys Met Leu Val Arg Ile
325 330 335

Ala Glu

<210> 19
<211> 1017
<212> DNA
<213> Streptomyces platensis subsp. rosaceus

<400> 19

atgaccgttc cgtccacggg caccgaagtc cggctcgcca cccgccccga ggggtggccg 60

accactgaga acttctcggt cgtgcaggcg gaaccgcccg cggtcaggac cggccaggtg 120

ctgatccgca acctggtgat gagcgtcgac ccgtacatgc gcgggcggat gaacaaaacc 180

aggtcctacg ttccgccgtt cgccgtcggc aaggcgctcg acgggggcgc cgtcggcgag 240

gtcgtcgtct cgaagtcatc gcaactcgcc gtcggtgacc tggtcctgca cggcctcggc 300

tggcgggagt acgccgtcgt gggcgctgcc ggcgcggtca ggatcgaccc ggcgctcgcg 360

ccgcccggcg cgtatctcgg agtgctcggc atgccggggc acgccgccta cacggggttg 420

ctcaaggccg ccgaattccg gcccggcgac accgtgttcg tctccggggc cgcgggcgcg 480

gtgggctccc tcgtcggtca gatcgcccgg ctctgcggcg cggaacgcgt gatcggatcg 540

gcgggcagcg ccgagaaagt cgcctatctg accggggagc tcggcttcga cgcggcattc 600

gactacaagg acgggccggt tctcgaacag ctggcgaagg ccgcgccgac gggcatcgac 660

gtgtacttcg acaacgtggg cggcgaccac ctggacgccg ccctggtcct ggccaggatg 720

ggcgcgcggt tcgccctctg cggcaacatc tcgcaggcca acgagaagga cccgccggcc 780

ggcccacgga acctgacgca ggccatcgcc aagggcatca ccctgcgcgg cgtcctcgtc 840

ggaggccacg ccgacctccc ggacgagttc accgcccgca tgggtggctg gctggcagac 900

gggagaatct cctaccggga gaccgtcgtc aggggactgg agaacgcacc cgccgccttc 960

atcgacatgc tgcgcggcgc caacaccggc aaaatgctcg tgagaatcgc cgaatga 1017

<210> 20
<211> 281
<212> PRT
<213> Streptomyces platensis subsp. rosaceus

<400> 20

Met Ser Ser Thr Ser Thr Thr Ala Pro Val Ser Val Pro Val Ser Ala
1 5 10 15

Pro Val Pro Glu Glu Val Gly His Leu Tyr Asp Arg Leu Thr Ala Leu
20 25 30

Asp Thr Glu Ala Ala Gly Gly Ser Leu His Leu Gly Tyr Trp Asp Val
35 40 45

Asp Asp Asn Asp Thr Pro Leu Val Glu Ala Ala Asp Arg Leu Thr Asp
50 55 60

Thr Met Thr Asp Arg Leu Arg Ile Asp Gln Gly Gln Arg Val Leu Asp
65 70 75 80

Val Gly Cys Gly Val Gly Gln Pro Ala Met Arg Ile Ala Arg Arg Thr
85 90 95

Gly Ala His Val Thr Gly Ile Ala Ile Ser Lys Asp Gln Ile Ala Arg
100 105 110

Ala Thr Ala Leu Ala Glu Gly Ala Gly Leu Ser Asp Arg Val Glu Phe
115 120 125

Arg His Ala Asp Ala Met Glu Leu Pro Phe Pro Asp Asp Ser Phe Asp
130 135 140

Ala Ala Ile Ala Ile Glu Ser Ile Phe His Met Pro Asp Arg Gly Arg
145 150 155 160

Val Leu Ala Glu Ile Arg Arg Val Leu Arg Pro Gly Gly Arg Leu Val
165 170 175

Leu Thr Asp Phe Phe Glu Arg Gly Pro Val Pro Ala Glu Lys Gln Pro
180 185 190

Ala Val Asp Arg Leu Leu Arg Asp Phe Ile Met Thr Leu Ala Arg Pro
195 200 205

Glu Asp Tyr Val Pro Met Leu Arg Asp Ala Gly Leu Arg Phe Val Glu
210 215 220

Leu Leu Asp Ile Thr Glu Gln Ser Val Arg Gln Thr Phe Glu Gln Met
225 230 235 240

Ser Gln Gly Ser Gln Glu Met Gln Thr Val Phe Asp Asp Glu Ala Glu
245 250 255

Glu Lys Phe Ser Pro Ala Ser Met Ile Asp Val Asp Glu Phe Gly Ser
260 265 270

Val Leu Leu Thr Ala Gln Lys Pro Leu
275 280

<210> 21
<211> 846
<212> DNA
<213> Streptomyces platensis subsp. rosaceus

<400> 21

atgtccagca cgtccacgac cgccccgtc tccgtccccg tctccgcccc cgtccccgaa 60
gaggtcggac acctctacga ccgcctcacc gcactggaca ccgaagcggc cggcggcagc 120
ctccacctcg gctactggga cgtcgacgac aacgacaccc cgctcgtgga agcggccgac 180
cggctcaccg acacgatgac cgaccgcctg cggatcgacc agggacagcg ggtcctcgac 240
gtcggctgcg gagtcggcca gccggccatg cggatcgccc ggcgcaccgg cgcccatgtc 300
acgggcatcg cgatcagcaa ggaccagatc gcccgcgcca ccgccctcgc cgagggcgcc 360
ggcctgagcg accgcgtgga gttccggcac gccgacgcca tggaactgcc cttccccgac 420
gactccttcg acgccgccat cgccatcgag tcgatcttcc acatgcccga ccgcggacgg 480
gtcctcgccg agatccgccg cgtactgcgc cccggcggcc gcctggtcct caccgacttc 540
ttcgagcgcg gccccgtccc cgccgagaag cagcccgcgg tggaccggct cctccgcgac 600
ttcatcatga cgctggcccg gcccgaggac tacgtgccca tgctgcgcga cgcaggcctg 660
cgcttcgtcg agctcctcga catcaccgag cagagcgtgc gtcagacctt cgagcagatg 720
agccagggct cccaggagat gcagaccgtc ttcgacgacg aggcagagga aaagttcagc 780
cccgcctcca tgatcgacgt cgacgaattc ggctccgttc tgctgaccgc ccaaaagccc 840
ctctga 846

<210> 22
<211> 1700
<212> DNA
<213> Streptomyces platensis subsp. rosaceus

<400> 22

tgccggcaga agtacaacgg cctccccaaa aagcgggccc cgtttagaag gtgatcctgc 60
cgaccggtaa gccgggcgtg gatcctgacc cgctacgacg acgttcgcaa ggcgctgctc 120

gacccgcatc tcagctccga ccgggccgat ccgaacttcc cgatgctggt cgagctcgcg 180
cggagcgaca aggactccac actgtccctg gcgggcatgg acgcgcccga acacacccgc 240
gcgcggcgcg ccgtggtcgg cgagttcacc ttccgccgga tggaagccct gcgcccgcgc 300
gtccaggaga tcgtcgacga gtgcgtcgac gcgatgctcg ccggccccaa cccggccgac 360
ctggtgaaga cgatgtcctt cccggtgccc tccctggtca tctgcgagct gctcggcgtg 420
tccgtcgccg accgcgactt cttcgaggac cgcacctccc gcatgctcag catgaccctc 480
ccgccgctga cccggcagca ggcgttcttc gacctgcaga cctacttgga cgagctcgtg 540
acggccaagg agaaagtccc gggggacgac ctgctcagcc gccagatcgc caagggccga 600
aaggacagcg cgtacgacca cgacgcactc gtcgacctgg cgttcctgct gctgaccgcc 660
gggcacgaca cgaccggaaa catgatctcg ctgggcatgc tcgccctgat ggagacgccc 720
gagctgcggg cgcgcatcac cgacgacccc ggcaccacgc cgcaggtcgt cgaggaactg 780
ctgcgctact tcaccatcac cgacatcatc accacgcgcg tggccaagga ggacgtcgag 840
atcggcgggc agaccatccg cgccggcgaa ggcgtcttcg cgctgggcgg ctcggccaac 900
cacgacccgg acgtcttcga gaaccccgga aagctggacg tcgaccgcgg cgcacgccag 960
cacctcgcct tcggccacgg accgcaccag tgcctcgggc agagcctcgc ccgcatggaa 1020
ctggagatcg tctacgacac cctgctccgc cgcatccccg gactccggcc ggccggcccc 1080
gctgaagacc tgccgctgaa gaacgacgcg gccatcttcg gcctgcacga actcccggtc 1140
atctggtagg cggagcctgt gcgcgccagg cggtcatgac gtggctgtcc gtgcggtgac 1200
gcgggccggg gcgtcgtgca tcggtcgtac tcaggggctt ggtggtgatg ccgatcagct 1260
gtaggacccg cggccgccgg tgtggagact gatcaccatg cgtactgact ttgatcccgc 1320
tgccatgtcc cgtaacgact tctaccggct cctgaccgcg acggtggtgc cccggccgat 1380
cgcctgggtg tcgaccacgt cggcagacgg aacgcacaac ctcgcacccg gacaccccga 1440
tggcgaggtt gtcatcggtc ccggaggcca gggtcgcggc aacgccttcg ggtgagcggc 1500
ggtgctgctc agcccagtct tcggcaacgg atcgggacca cctgctgcgc ccgttgacag 1560
tggattgtgg agggggaacg tcgccttcac cgcgggagat gtaagcgcgc agggtggaag 1620
cggcaatggc gccgatcttc gccatgtccg gcaccggctg ctggtgggac ggcaggggag 1680
ttgcgggccc caccagttgg 1700

<210> 23
<211> 328

<212> PRT
<213> Streptomyces platensis subsp. rosaceus

<400> 23

Met Leu Val Glu Leu Ala Arg Ser Asp Lys Asp Ser Thr Leu Ser Leu
1               5                   10                  15

Ala Gly Met Asp Ala Pro Glu His Thr Arg Ala Arg Arg Ala Val Val
            20                  25                  30

Gly Glu Phe Thr Phe Arg Arg Met Glu Ala Leu Arg Pro Arg Val Gln
        35                  40                  45

Glu Ile Val Asp Glu Cys Val Asp Ala Met Leu Ala Gly Pro Asn Pro
    50                  55                  60

Ala Asp Leu Val Lys Thr Met Ser Phe Pro Val Pro Ser Leu Val Ile
65                  70                  75                  80

Cys Glu Leu Leu Gly Val Ser Val Ala Asp Arg Asp Phe Phe Glu Asp
                85                  90                  95

Arg Thr Ser Arg Met Leu Ser Met Thr Leu Pro Pro Leu Thr Arg Gln
            100                 105                 110

Gln Ala Phe Phe Asp Leu Gln Thr Tyr Leu Asp Glu Leu Val Thr Ala
        115                 120                 125

Lys Glu Lys Val Pro Gly Asp Asp Leu Leu Ser Arg Gln Ile Ala Lys
    130                 135                 140

Gly Arg Lys Asp Ser Ala Tyr Asp His Asp Ala Leu Val Asp Leu Ala
145                 150                 155                 160

Phe Leu Leu Leu Thr Ala Gly His Asp Thr Thr Gly Asn Met Ile Ser
                165                 170                 175

Leu Gly Met Leu Ala Leu Met Glu Thr Pro Glu Leu Arg Ala Arg Ile
            180                 185                 190

Thr Asp Asp Pro Gly Thr Thr Pro Gln Val Val Glu Glu Leu Leu Arg
        195                 200                 205

Tyr Phe Thr Ile Thr Asp Ile Ile Thr Thr Arg Val Ala Lys Glu Asp
    210                 215                 220

Val Glu Ile Gly Gly Gln Thr Ile Arg Ala Gly Glu Gly Val Phe Ala
225                 230                 235                 240

Leu Gly Gly Ser Ala Asn His Asp Pro Asp Val Phe Glu Asn Pro Gly
                245                 250                 255

Lys Leu Asp Val Asp Arg Gly Ala Arg Gln His Leu Ala Phe Gly His
            260                 265                 270

Gly Pro His Gln Cys Leu Gly Gln Ser Leu Ala Arg Met Glu Leu Glu
        275                 280                 285

Ile Val Tyr Asp Thr Leu Leu Arg Arg Ile Pro Gly Leu Arg Pro Ala
    290                 295                 300

Gly Pro Ala Glu Asp Leu Pro Leu Lys Asn Asp Ala Ala Ile Phe Gly
305                 310                 315                 320

Leu His Glu Leu Pro Val Ile Trp
325

<210> 24
<211> 987
<212> DNA
<213> Streptomyces platensis subsp. rosaceus

<400> 24

```
atgctggtcg agctcgcgcg gagcgacaag gactccacac tgtccctggc gggcatggac     60
gcgcccgaac acacccgcgc gcggcgcgcc gtggtcggcg agttcacctt ccgccggatg    120
gaagccctgc gcccgcgcgt ccaggagatc gtcgacgagt gcgtcgacgc gatgctcgcc    180
ggccccaacc cggccgacct ggtgaagacg atgtccttcc cggtgccctc cctggtcatc    240
tgcgagctgc tcggcgtgtc cgtcgccgac cgcgacttct tcgaggaccg cacctcccgc    300
atgctcagca tgaccctccc gccgctgacc cggcagcagg cgttcttcga cctgcagacc    360
tacttggacg agctcgtgac ggccaaggag aaagtcccgg gggacgacct gctcagccgc    420
cagatcgcca agggccgaaa ggacagcgcg tacgaccacg acgcactcgt cgacctggcg    480
ttcctgctgc tgaccgccgg gcacgacacg accggaaaca tgatctcgct gggcatgctc    540
gccctgatgg agacgcccga gctgcgggcg cgcatcaccg acgaccccgg caccacgccg    600
caggtcgtcg aggaactgct gcgctacttc accatcaccg acatcatcac cacgcgcgtg    660
gccaaggagg acgtcgagat cggcgggcag accatccgcg ccggcgaagg cgtcttcgcg    720
ctgggcggct cggccaacca cgacccggac gtcttcgaga accccggaaa gctggacgtc    780
gaccgcggcg cacgccagca cctcgccttc ggccacggac cgcaccagtg cctcgggcag    840
agcctcgccc gcatggaact ggagatcgtc tacgacaccc tgctccgccg catccccgga    900
ctccggccgg ccggccccgc tgaagacctg ccgctgaaga acgacgcggc catcttcggc    960
ctgcacgaac tcccggtcat ctggtag                                         987
```

<210> 25
<211> 50543
<212> DNA
<213> Streptomyces amphibiosporus

<400> 25

```
gtgctcttcc cgggacaggg ctctcagtcg aagggaatgg gaagagaact cttcgaccgt     60
tttcccgaga ccaccgcgtc ggcctgcgac gtcctcggat acgacctgcg cgagctgtgc    120
ctggagaacc cggagggccg gctcgacgac acccggtaca cccagtccgc cctctacacc    180
gtcaacgccc ttgagtatct ggggtcgttg gaggacgggg cgccggaggg cgactacctg    240
ctggggcaca gcctcggcga gtacaacgcg ctgctcgcgg cgggcgtctt cgacttcgag    300
```

```
accgggctgc ggctcgtcct caagcgcggt gagctgatgg cgcgggcgcg ccacggcggg    360
atgctggccg tactggggcc cggcgaggag gagttgcgcc ggacgctggc cgaggagggc    420
atggagcgtc tcgacgtcgc caacgtcaac acccccgcgc agaccgtgct ttcggggccg    480
gtggaggaga tcgagcgcgc ccagcggcac ttcgacgagc gccgggtgcg tacggcgcgg    540
ctgaaggtct ccgccgcctt ccactcacgg ctgatgagac ccgcgcggga ggaattccgt    600
gcgtttctcc ggggattccg cttcgcgtcg ccgcgtgcca cggtgatcgc caatgtgtcg    660
gcacggccct acggcgatgt cgcggagatg ctgagcgagc agatcgccgg gccggtgcgg    720
tggctggaga gcgtccggta cgtgctggag cggacctccg ccgagcgcgg cagggaggcg    780
ggacccggga ccgtactgac gcggatgctg cggcagatcg acggtgtgtc cggggcgggg    840
aattcccccct ccgtctctgc gtccggctcc gtgcccgctg cctccgcccc ggccgagtct    900
ccgtccgctc ccgcagcgtc aacgtccggc agcgcgcggc ccgttgggcg cgccaccgcc    960
gccaccgccg atgccgtacg ggagcccacc cggcccctgc tgatctgcgc gccctacgcg   1020
ggcggcgacg agcgctccta cgcggggctc gccgagcaac tgcccgaggc ggacgtcgtc   1080
accctggagc ggccgggccg cgggcggcgg gtctccgagc cgctgctgac cgaaccgggg   1140
cccgtcgtcg aggacatgct gtcccggata cgggaccggg tgagccggcc gtacgcgctc   1200
tacgggcaca gcctcggcgc gcggctcgtc catctcctcg cccgccggct gcgcgaggag   1260
ggcctgcccg gcccccgcca tctgttcgtc tccggcgagt gcggcccctc gcggcccagc   1320
cgggagcgct acaccagcga tctgccgacc gacgccttct ggaagcacct gagagaactc   1380
ggcggcgtgc cggacgagtt gttcgagtac gaggacctca ccacgttcta cgagcgcgtt   1440
ctgcgcgccg acttcaccgt cctcggggcc tgcgcgtaca cccccgccgc acccctggac   1500
tgccccgtca ccgccatgac cggcgacgag gagggcctga ccgaggccga cgtcggggcc   1560
tggcagcggg agaccaccgc gccgctcacg gcccgggtct tcaccggaga ccacttcttc   1620
atccgggcgc actggcccgg cgtcgcacgc gtcgtcgccg ccgggctggg cgcccgccga   1680
cccgcaggga cccgctgacc cggaggaacg cgacaggacc gtacgaggac ccaccgcggc   1740
agcgcgtcga cggcgacgcg cggcgagtcg gccccggcac gaccggttcc gtccgcaccg   1800
ccgtgccggc tcccgggtac cgcccgggcc ccggctccgc cccaacccgc ccgcatcccg   1860
ggcccgacca cacccggaac cagatccgga acgagatgag gtacgccatg gagcaggaac   1920
tcaagcagta catggaagag cagttcatgt tcgagttcga ttcggagatc accgaggaca   1980
ccgacctgtt caaggcgggc gtgctcgact cgttcggcta catctcgctc atcgggcaca   2040
tcgaggggga gtacggcgtc aagttcggcg aggaggcgct gctcggcaac gtcgccgtca   2100
ccttcgccgg cctcgtcgag tccgtggcgt ccgcccgtcg gcagaccgcc gagagcaagt   2160
```

aaccggtgtg cggcatagcg ggcttccacg cgagccccct gcacccggag agctaccggg 2220
acatcgccgg tgccatgctc gcgcagatcg agcaccgggg ccccgacgag gcgggctgct 2280
tcctggacga ccgtacggcc atgggcacgg tgcggctgag catcatcgac ctcgcctccg 2340
gctcgcagcc cgtcggcagc cccgacggcc ggtactggct ctgctacaac ggcgagctgt 2400
acaactaccg ggaactgcgc gccgagttgg cgggccgcgg ggtgtccttc cgtacggagt 2460
ccgacaccga ggtcgtcctg atggcctggg cgcactgggg gcgctcgtgc ctcgaacgct 2520
tcaacggtgc cttcgcgttc gccctgaagg acaccgtcac cggtgaactg cacctggccc 2580
gcgaccggtt cggcaagcgg ccgctgtatg tggcgcggca cggcgacgca tggctgttcg 2640
cctccgagat gaaggccttc ctggcctacc ccggcttcga gttcgccttc gacgaagagc 2700
atctcgcctc gacgttcgcc acctggacgc cgctgcccgc gcagagcgga taccgcggcg 2760
tcgaacagct ccccatgggc gagtatctga cggtccgcgg gacggagacc gaacgcggcc 2820
gctgggcgtc gctcgacctg accggcggcg agccgcccgc caccgaggac gaggccgtcg 2880
acctcgtgcg cgccgatctg gaggccgccg tcgacctgcg gctgcgcagc gacgtcgagg 2940
tcggcgtcta cgcctccggc ggtctggact cctcgatcct cgcccatctc accaaggagc 3000
gggccgggct gccgccgcgt acgttctcca tccagttcga ggacgccgag ttcgacgaga 3060
ccgccgagca ggaggagctg accaagcacc tcgggacgca ccactccacc gtccgcgtct 3120
ccgactccga cgtcgtggag accttccccg aggccgtacg ccacgccgaa gtccccgtct 3180
tccgcacggc gttcgtgccg atgtacctgc tggcgcagca tgtgcgcagc gaaggcgtca 3240
aggtcgtgct cagcggcgag ggcgccgacg aggcattcct cggctacggc atcttcaagg 3300
acgcccggct gctctccgag tggcacgagc tggacgaggc gacccgcatg cggcgcatgg 3360
cgcagctcta cccgtatctg cgccacttca gcggcgagga cgggcaccgc cggatgctgg 3420
gcctctaccg gcagttcacg gaggagacca tgcccggtct cttctcccac cagatgcggt 3480
tccagaacgg ccggttcgcc gtgcggctgc tcaaggacgc gggcgacccc ttcgccgcgg 3540
tacggcggct cgtcgcggag gagcccggat acgcggagct gtccgcggtg cagaaggcac 3600
agtggctgga gttccgtacg ctgctcagcg gctatctgct cgccacccag ggcgagcgga 3660
tggcgctcgc gcacggcgtg gagaaccgct gcccgttcct cgacccggcg gtggtgcgcc 3720
gcgcggcgtc cgtcaacggc cgcttcggcg acccgtacga cgagaagtac ctgctcaagc 3780
gcgcgtacgg ggacgtgctg cccgaacgca tcgtcagcaa gggcaagttc ccctaccggg 3840
cgccggacag cgccgcgttc gtacggtccc gtcccgacta ccgcgacctg ctggccgacc 3900
ccggcaccct cggcgacatc ggcgtgctcg acgagcgctt cgtgcggcgc ttcaccgacc 3960

gggtcttcga caggccgccc gagcggatcg gcaccaagga gaaccaggcg ttcgtcctgc 4020
tggcgtccac ggtctggctg caccactggt acgtgcgcgg caacgcccgc cgcgacaccc 4080
ccctcgctgt ccccctgtac gtcgtcgacc ggcgcagcag cgcgctgccg gcctaggacg 4140
gagatcctgc gatgaaggaa gaatccggcg ccctccccga ggaaggcccc gtcggcaccg 4200
ctgtcggcac cgccgccgac ggcgcggccg gcggcccggt ggacgggcag gacatcgctg 4260
tcgtgggcct gtccctgcgg ctgccgggcg cacggaaccc ggaggagttc tgggagcacc 4320
tggccgcggg ccgctcgctg atcagcgagg tgcccgagcg gcgctggcgc aaggaggacc 4380
atctcggcaa cccgcgccgg gagttcaaca agaccaacag cgtgtggggc ggcttcgtcg 4440
acgacgccga ctgcttcgac gccgagttct tccatgtctc gccgcgcgag gcccgctcca 4500
tggacccgca gcagcggatg gcgctggaga tgagctggca ggcgctggag gacgccggat 4560
accgggccga ccgggtcgcc ggctcccgta cgggcgtctt catgggggtg tgccactggg 4620
actacgccga gctcatcgag aaggaggtct ccgaggtcga cgcctactac ccgacgggcg 4680
ccgcgtacgc gatcatcgcc aaccgcgtat cgcaccactt cgatttccgc gggcccagcg 4740
tcgtcaacga caccgcgtgc gccagttcgc tggtggcggt gcagcaggcg gtgcaggcgc 4800
tccagtccgg ggactgcgac cacgcgctgg ccggaggcgt caacctgacc tggtcgccac 4860
ggcacttcat cgccttcgcc aaggcgggca tgctctcgcc ggacgggctc tgccgcgcct 4920
tcgacgcgga cgccaacggc tacgtacggg gtgagggcgg cggcgtcgtc ctgctgaagc 4980
gggcggcgga cgcccgccgg gacggcgacc ccgtacacgc ggtgatcaag ggcatcggca 5040
gcaaccacgg cgggcgcacc agttcgctca ccgtcaccaa ccccgccgcg caggccgagc 5100
tgatcgccgg gatctaccgg cgggcgggga tcgccccgga gtccgtctcc tacatcgaga 5160
cccacgggcc gggcaccccg gtcggcgacc ccatcgaagt cagcggcctc aagcgggcgt 5220
tcgcgcagct cggcgaggga cgggaggccg agccgtccgg gcaccgctgc ggcatcggct 5280
cggtgaagac caacatcggg catctggagg gcgccgcggg catcgccggg atgctcaagg 5340
tgatcctggc gatgcgtcac cgcaagctgc ccgcgacggt gaacttccgc cgtctcaacc 5400
cgctgatcac gctggacggc agcccgctgt acgtcgtgga ccggctcacc gactgggaga 5460
cggacggcga cgggacgctg cgggcgggtg tcagctcgtt cggcttcggc ggcaccaacg 5520
cgcatgtggt gctggaggcg cccggcggtc acgcggcgga ggtcacggac gcggaggcgg 5580
tcacggacgc ggaggcggac gccgacgtgg acggcgggcc ggacgagggg cccgacgagg 5640
gcgccgaacc gcgtgctctg cggctccccg tctccgccga cgacgaggag cggctgcgtg 5700
agctgtgccg ctcgctcgcc gagtgggccc gtgcccgcga agccgaaggc acggcgccgc 5760
cgctggccga catcgcccgc accctgcgcg aagggcgggt gccgatgcgg gagcgtgcgg 5820

tcttccgcgc gcggagcgtc gccgagtggg cggaacagct cacggccctc gccgagggga 5880
ccggcgggga gccgcccgct ggctgtctgc gcggacgcgc ggaggacggc gccggggacg 5940
gcctggacgc cgacgatgtc gcggccctga ccgcgcgctg gcgggagcgg gacgaggagg 6000
agaagttcgc cgcggcctgg acgaggggcc tgcccgtgga ctgggcgcag tggcccgccg 6060
agggccgccg cgtccatctg cccggacagg tcttccagcg gacgccgcac tggttccgtc 6120
cggacgaaca gccgcgcggc gaggccgagt cggcgggcgg tgcggcggct cagcgggaca 6180
ccgctccaga gcgggacgcg gcgtccgggt cggaacgcgg gcccggcgca ccggagccgg 6240
cgggaccggt gggagggccg gggctgccgg gcgagggcgt ccaggacggg cggggctggc 6300
acttcccgct gcgcttcgcc gccaccgacc ccttcgtacg cgaccatctg gtcttgggcg 6360
cccgtatcgt ccccggcgtc gtggcgctgg aggccgtgac cgccgccgcg gcacgtcccg 6420
ctgtggccgg tgcccgtgcc ggtgcggcgc cgcacatccg caacgcggtg tgggtgcggc 6480
cgctgcgcgt ggacggccaa gtcctcgaaa cgagcctgcg gttgacgccc gccggcccgg 6540
agtccggcgg cggctacgac tgggccgtca ccgacgccgc gggcacgccg tacagcagcg 6600
gtcgcgtcga gtacgccgac gggcccgcgc ccgccgccac ggatctggac gcgctgcacc 6660
ggcggcacac ccgtcccgtg gaggtggccg ggggatacgc ggcgctgtac gccagcggca 6720
tcgagcacgg cccggcgctg cgcgccctgc acacgctgcg cgccgggccc gaagggctgc 6780
tggccgagct gcggttgccc gccgagccgg ctgcgggcgc ggctctccag cccgccgttc 6840
tggacagcgc cctgctggcc gtgctcgcgc tcggtacggg cggtggcgac ggcacagagg 6900
gcaccggcgg cacagacggc gcgggctggc gccgaccgga cgcgcccgcc gtgccgttcg 6960
cgctggacgg cctgaccgcg tacgccccga ccacggccac gacatgggcc tggctgcggc 7020
ccgcgggcgg acgccgtccc ggcgccgccg acatcgatct gttcgacgag cgggggcggt 7080
tgtgcgcccg tctgacgggc tacacctcgc gtgaactgtc caccgggagc ccggcgttga 7140
gggaagcgcg cgtttctgca ccggcaccgg gcgaaggggc ggcgggcgag gaggctccgg 7200
ggaaggacgc tccgggcgag ctgctggagg tcaccgggcg ctggacgccc gcgcccctcg 7260
gcctccccgc cgccgaggcg ggaccggccg cggcacagtc gggcgccgcc gctcccgtca 7320
cggtgctgaa cgccgccctg gacgccgacc tcgtcgccgc gagcgccgcc cgtctcggca 7380
tggacgtcga gcacctggcc gtaccgcgcg acgcgggcga cgcggacgcc atgaaggcgg 7440
cgttcgcggc ctgttacccc catgtccggc ggctcgtcgg acagtcgcgg cgcgttctgc 7500
tcgtggcccc cggcgcaccc gactccccgg tcttcgcgcc gctggcggcc ctgctgaaga 7560
cggcacacca ggagaacccg tccttccacg gcaccaccgt gctcctggag ggcttcgacc 7620

cgcgtgactc cgcacgcttc gagcaggtcg tccgtacgga ggcggcagcg acggcggacg 7680
ccgcgggcgg cgcggcggac gaggaggtcg cccacaccgc cgacggccgc cggctgcgcc 7740
atgagacggc cgaactgccg cacggcacaa cgggcgagag cctgctggcg gagggcggcg 7800
tctactggat caccggcggc gcgggcggca tcggcctgct gctggccgag cggctgtgcc 7860
tgcggtacgg ggcgacggtc gtcctcagcg gacggtcgcc cgctgcgccc gcggccgacg 7920
cgctcgcctc ccggctcacc cgcggcacgc tggcctaccg cggcgcggac gtcaccgacc 7980
aggacagcgt ggacgcgctg gtggccgccg tgctggccga acacggccgg atcgacggtg 8040
tgttccacgc cgccggggtg ctcgacgacg gctatctgac ggccaagccg ctcgccggga 8100
ccgaggccgt gctcgcgccg aaggtggacg gcgtcacctg cgtcgaccgc gccacgcgcg 8160
cgggcgctcc gggcttcctg ctggtcttcg ggtcggtcgc gggtgccttc ggcaacgcgg 8220
cgcaggccgg ctacgccgcc gcgaacgcct acctcgacgc gttcgccgcc cggcggcagg 8280
ccgccgggct gaccacccgg gccgtcgact ggccgctgtg ggccgagggc ggcatgcgcg 8340
tggacgacgc gagcctgaag tatctgcgca agcgcaccgg caccgtgccg ctgccctccg 8400
gcaccggcct cgacgcgctg gagcgtgcgc tgcacaccgg ttcgccggtg cggcgcgtgg 8460
tcctctacgg cgaccgtccg gcgctgcggg tgtacgcggg tctggaccgt ccgcaggtca 8520
cgggcgcacg gtccggctcc gcgtccgcgt ccgcgccggg ctcctcgtcc gggtccgtgt 8580
ccgccgtgcg cggcgagggg accgggacgg caccggccgc gctcaccgac gccgaactcc 8640
tcgtccgcac acaggacttc ctgcgggagc agttcgccga ggtcaccctc caggacgccg 8700
agcagatcca ccccgaggag aagctggaga cgtacgggat cgagtcgatc tcgatcgtcg 8760
atctgacgag caggctggag gacgtcttcg gctcgctgcc caagaccctc ttcttcgagt 8820
acgtcgatct gaagggcgtg gccgagtact tcgtggccga gcaccgtgcg cggctgaccg 8880
aactcttcgc gccggaggag ccgcaggcgt ccgaggcggc ggagcccgca ccggaagaac 8940
ccgtggcgcc cgcccccgta ccggtggagc cggccgccgc ggcacccgca cccgcacccg 9000
cacctgtacc ggcgcctccg gcgccaaccg ccgcaccggg tacgtccgtt gaggccgtcc 9060
ccgcgcccgt tcccgcttcc gtacccacgc cgcgccccgc cccgccgggg aacggcgaca 9120
tcgctgtcgt cggcatggcg ggccgctacc cgggcgccga caccctggag gagttctggg 9180
agctgctcag cgagggacgg cacagcttcg agcccgtgcc gtcctcgcgg tggccgcacg 9240
gcgacctgta cttcgacgag cgggacgtgc tgggcaagac cacggtgcgc accggcacct 9300
tcctgcgtga cgtcgacgcc ttcgaccccc gctacttcag catctcccag cgcgacgccg 9360
aactcctctc gcccgaggtg cgcctcttcc tccaggcggg cgtgacggct ctggaggacg 9420
ccgggtactc caaggagacg ctgcgccgcc gctacgacgg cgacgtgggc gtgctcgtcg 9480

gctcgatgaa caacagctac gcctactacg gcttcgagaa catgctgatg cgcggcaccg 9540
cgatgagcgg cagcgaggtc ggcgtgatgg ccaacatgct ctcgtactac tacgggttca 9600
cgggcccgtc gatgttcgtc gacaccatgt gctcgtcgtc ctcggcctgt gtgcaccagg 9660
cgctgagcat gctgcgcggc ggcgagtgcc gcatggtcgt cgtcggcggc atcaacctga 9720
tgctccaccc gtacgacctg atcgccacct cgcaggcgca cttcaccacc aagtcggcgg 9780
aggtcgtgcg cagttacgga ctcggcgcgg acggcacgat cctcggcgag ggcgtgggga 9840
ccctggtgct caagccgctc gccgaggccg tcgcggacgg cgaccacgtc tacggcgtca 9900
tcaagggcag cggcatgacc aacgccggtg tgcgcaacgg cttcacggtg cccagcccgc 9960
agcagcaggc gagggcgatc gagagggcgc tcgacgacgc cgccgtggac gcgcgcaccg 10020
tcagctacct ggagggccac ggctcggcga cctcgctggg cgacccgatc gagatcaagg 10080
gcgcgagcct cgccttcggc cgggacaccc gggacgtggg ctactgcgcg atcgggtcgg 10140
tcaagtccaa cgtggcgcat ctgctgtccg gttcgggcct cgtcggcctg acgaaggtgc 10200
tgcttcagct acggcaccgg acgctggcgc cgtcgctgca ctccgaaacc ctcagccccg 10260
ccatcgactt cggctcgacg ccgttcgtgg tgcagcgtga gcgcgccgag tggcggcgtc 10320
ccgtcgtaca cggcgcggag gtgccgcgcc gcgcgggcgt cacctcgatc ggcgcgggcg 10380
gcatcaacgt gcacctgatc gtcgaggagt tcgacggcac ggtgaactcc gcgcccgacg 10440
acggcggttc acagcttctg gtgttctccg cgatgacgcc gcaggccctg ggcaccgtgc 10500
tgcgcgacgc gcaccgccat gtcgccgacg aggcgcccgc gctcaacgcc ctcgcgtaca 10560
ccctccagac cggcaagaac gaactcccct gccggctggc cttcgtcgcg cacggcacgg 10620
ccgacgccga ggcccggctc gccgcgctgg cggcggtgga ctggacgtcc ggcgcacccg 10680
cgctgcccga cgctgtgcgc ttcaccgaga gcacgctgcg gaagcggcgc agcgtggcgg 10740
ccgccgacgt cgaacgggcc ctggcgcagg ccgacttggc ggagctggcc ggatactgga 10800
tctccggcgc ggccgtggac tgggacctgc tgtggccctc gggtacccgt ccggcgaagc 10860
tggcgctgcc cgcgtacccg ttcgagaagg tgcgctgctg gtacccgggt ttcgacgacg 10920
cccccagcgt gctgcggccc ctggccttca cccggcgcgg gcacccctgg gtcggcgtca 10980
accgctccga tctgcacggc gtgcgcttcg cgctggagct gacgggcgac gaactcctcg 11040
actacgtcca cacggtgggc cgcacccgcc gcttcacgag cgtcgccctg ctggacgggg 11100
cgctggcgtt cgcgcggctc gcgggcctgg acggcgcgct gcggctgcgc gacgcgcggt 11160
gggcggagct gccctcgccc ggcgacgcca cggaggtctt cgagtggcgg ctcgcgctct 11220
ccggcgaggg agcgtccggt gacgcggcgt ccggcggagg agcgtccggt gcgggcggcc 11280

atcgtgtcga gctgtggcag gccgagcgcg gcacgctgca cttctcggcg gaggtcgtac 11340
cggcaactgc cgtggcggcg ggggccgttg acgcgcggcc tgccgacgcc gcggcgctgc 11400
tcgccgcacc cgtgacgctg gacggcgacg cgttctactc cgcgctcggc gaggcgggcg 11460
tggacgcccg cccgtacgcg cgcgccgtca ccggcgtcac cgaggccggc ggacggcggc 11520
tgctcgtccg tgtcgccgaa ccggcgatgt gccaggaccc gcacaagcag cacgtcagca 11580
ttcccgcgtg ggtgctggcg gggctggcac agggcgtgca gcacgccacg ggccggccgc 11640
gtacgacggc actgcgcgcc gccgcgctgt acggcgccga cctgacggac acccgcgccc 11700
tgctgctgga gcccgtcgcg gaggccacct tccggatcac cttcctggac ggggacgggc 11760
gggcgctggg cgcggtggag gacgcggagt tcaccgccgg gacgttgccg ccgtcgctgg 11820
agggcggcgc cgtaccggta cgggccggac tgccgggcgc ggcacgcccg tcggcgacgg 11880
cctcggcacc ggcctcggcc tcggtaccgg taccggcgct cgcggccgca cccgtcgcac 11940
ctgccgtgcc cgtcgagccc gtcgagcccg ccgcggaggc ggacgccgac gcgggggacg 12000
cgctcgtcgc cgtgctgcgg gagacggtcg ccgacctgct caagttcgag ctggacgaga 12060
tcgacctcga cacccacttc cacgcgtacg gcttcgagtc catcgccctc gcgcggctgg 12120
cctccgaact caacggcctg ctcggaacgg acctcagccc tgtcgtgttc ttcgagtgcc 12180
ccgacatccg cagcctcgcc gcccatctgc gcgagcgcta cgacgcggag acggcggccc 12240
gcgccgtccg cggcaccggc ggcggcaccg ggacggacgc ggcccgggcg ccgactcccg 12300
ctccggcacc ggcagtcggg gcggcgtccg cggccaccgc gcccgctccc ctctcgtccg 12360
ccgagcccgt gtccgaccac gaggccgact acccgggcgc cgtcgccgtc gtcggtgtgg 12420
cgggccgctt ccccggcgcg ccggacgccg acaccttctg gcagcggctg cgcgcggggg 12480
acgacctgat cggggagtac ccgggcgacc ggttcgacga gcgctacacc ggcgtcgtcg 12540
cacggtcgga cttcccgaag ttcgcgggcg tcctcgacga cgtcgaccgc ttcgacgccg 12600
gcttcttcaa cctctcccgg ctcgaagccg aactgatgga cccccagcac cggttggccc 12660
tggagacggt gtgggcggcg ctggaggacg gcggctacgc gcccggccgg ctgccggaga 12720
acaccggcgt ctacgtcggc gtctccggca gcgactacca ccacctgctg aacgccagcg 12780
gcgtggcgcc ggacggcttc accgccaccg gcaacgccca ctcgatgctg gccaaccgga 12840
tctccttcgt cctggacgtg cacggcccga gcgagccggt ggacaccgcg tgctccagct 12900
cgctcgtcgc cctgcaccgc gcggtggaga gcatcaggtc gggccgctgc gacatggccc 12960
tggcgggagg cgtcaacctg ctgctgagca tcgacacgtt cgccgcgacg cagatggcgg 13020
gcatgctcag cccggacggc cgctgcaaga ccttctccgc ggacgccgac ggctacgtac 13080
gtgccgaggg cgtcgccgcg gtgctactca agccgctgga gcgggcgttg gcggacggcg 13140

acccggtctg gggcgtcgta cgcggcagcg ccgagaacca cggcggccgt gccggttcgc 13200
tcaccgcacc caacgccgtc gcgcagaccg cgctcatccg cgaggccatg cgcggcaccg 13260
accccgacag cgtcggctat gtcgaggcgc acggcacggg caccggtctc ggcgaccccg 13320
tcgaggtcgg cgccctcgac agcgcctacc gcgcgctgcg ttcggaccgc gggcgtgtcg 13380
agagcggcac cgcaccggtg gcgctgggct cggtgaagac caacatcggg cacgccgagt 13440
cggcggccgg actcgccggg gtgctgaagg tgctgctggc catgcgccac ggcgaactgc 13500
cgccgaccct ccactgcgac cggctcaatc cccatctgcc gctgtccggc ggcgggttcg 13560
aggtggtgcg cgaggtacgc cgctgggagc cgcggctcga cgcggacggg cggccgtggc 13620
cgctgcgcgc cggggtgagc agcttcggct tcggcggcgc caacgcgcac gtcgtgctgg 13680
aggccgcacc cgcggcggca cgggaacggg ccgtacggga aacggcttcg cggagtgcgt 13740
ccgtacggtc cgcgcacggg acgcagggcg ctccgcaggc cgtcgctccg caggccgtcg 13800
gtccgcagat cgtcgccgtc tcggcgcgtg acggcgagcg gctgcggatc gtggccgagc 13860
ggctgcggga cttcctgcgg cgggagcacg gcgcgggccg ggcgcccgcg acggccgacc 13920
tcgcccgcac gttgcagacg ggacgggagg cgatggaggc gcgtctcgcc ttcgtcgccg 13980
aggagaccgg ggacgtgctc gacgtactgg accggttcct caagggcgag gagcccgacg 14040
gctggcacac cggcgcactg cggcgctcgc gcggcgcggg agtgcggcgc gaccgggcgc 14100
aggacccgcg ggtgacccgc gcgctgcggg acggggacct cgatgcggcc gccgcgctgt 14160
ggtgcgaggg ggccctcgtc gactggcagt cgctgcaccc gccgggggag cgccgcaccg 14220
tgcggctgcc ctcgtacccg ttcgcccgcg aacgctactg ggtgcccacg gacggggcgg 14280
caccgccacc ggagaccggc gggcccggcg gcgtcgagga cggcggcgtc gagtacggca 14340
ccgggtccgg cgccgcccaa ctcggcgaca gcgggagcgc gttcgacgcc ggagcccttg 14400
ccgcggtgct cgacgcggtc ctcgacggac gggccgatcc cgacgacctc gcccgtacct 14460
gacgccgtac gcgcccgccc cgccccctct tctcttcgga cgagcggccc cgcgccgcac 14520
cgtgacctga cctggaacgg atcccacagt gagtcgaaac atcctgcgtg tgccggcatg 14580
gcgagacgag ccgtcgcgcg ggcaggcggc accggccgga gtccgccggc tggccgtcct 14640
gtgcgacgtc ccggacgcgg aggcggcgct gctgcggcag cactcgcccc gcctgcccgt 14700
cgtcctggtg gagagccggg acgacggccc cgccgccgcg tacgagcacg cagccacccg 14760
gctgctcgcc gagctccaga ggctgctggg ccgcccggcg gcgggtccgt gccgggtgca 14820
ggtggtgtgc cgggagagca cgccgcaggg ctgggcggga ctgctcggca tgctgcgtac 14880
ggcggcgcag gaagaccccc gactccgggg ccagctcatc gagttcgacc gactgccggg 14940

```
cggcgccgag ctggcgcgcg tgctggacga ggaggccgcc gaggaggcgg atcatgtgcg 15000
gcgggccgcc ggtgcagccg gtacgggtac cggaaccgga gccgtacggc aggtgcgcca 15060
ctggagcgcg gcccggtcgg cgggtcgcgc gtcgtccgcc gggaacccgg cgccggtgtg 15120
gcggcccggc ggcgtctatc tcgtcagcgg cggtgccggc ggcctcgggc ggctgctcgc 15180
cgccgacgtg cggcggcacg cgcccggcgc ggtcacggtc gtgtgcggtc gtggcccggc 15240
gccgtggcag gggcggaac cgcccgccga cggcgtcgag taccacagcg tggacgtcac 15300
cgaccgggcc gcggtggccg ccctggtcga tcgtgtgctg agtgcacacg gcaggctcga 15360
cggtgtcgtg cacgcggcgg ggctgctcgc cgacgactac gtggtccgcg cgtcgcaccg 15420
cgagacccag cgcgtactgg cgcccaaggt cgccggtctg gtccacctcg acgaggccac 15480
ccgcgaactg ccgctggact tcctggcggc cttctcctcc gccgccggga cgctcggcaa 15540
cgcgggccag gccggttacg ccgcggccaa cggcttcctc gacgcctacc agacccaccg 15600
cgccgcgctg gccgaggcgg gcgagcggca cggccgttcg ctctcggtcg gctggccgct 15660
gtggcgggac ggcgggatga ccgtgccgga cgagcaactg cccgaactca ccgagcggtt 15720
cgggcgtccg ctgacgaccg gcacggcgct gacggcactg cacgccgcgc tcgccctcgg 15780
cacaccgcac gtcctggtac gggacggcgc ggaggcggac gaaaccggag ccgtcaacgc 15840
aaccggggcc gggaccgcga ccgggatcgc gaccgaggtc gaggtcccgg ctgtgaacga 15900
agccgtcggc acggccgtcg acgacgccct ggaggacgac gccccggagg gggacaggaa 15960
gggaactccg gctgtggaac cgcgcctccg cgtactgccc gcgctgaagc aactcgtcgc 16020
cgagaccgtg cggttggacc cggccgcgct ggacgccgcc gcgccgctgg acggcttcgg 16080
catcgactcg ctggccgtca cccggctcaa ccgccgcttc gcgcagtggt tcggggcgct 16140
ccccaagacg ctgctgtacc agtacccgac gctgaacgag ctggccggat atctcgccga 16200
gcaccatccg gagggctgcc gccgctggct cgccgacacg gcgtccccgt ccctgtcccc 16260
ttccgcgtcc gcgtccgctt ccccgtcccc gtccccggca acgtccacgt ccgtgtccgc 16320
tccctccgct caggagcggc ggccgtcaac tcccgtcgcc gccggggccg ttcgcacggc 16380
cgggacgaac ggcacgagcg gtgctgccgc cccggtttcc gccgaggccc ccgttcccgc 16440
ccgtacgtca cctgtcgacg agccgatcgc cgtcatcggt ctgcacgggc gctaccccgg 16500
tgcccccacc ctggacgcct tctgggagaa cctgcgctcc ggccgggacg gcgtcaccga 16560
gatccccgcc gaacgctggc cgctggaggg cttctgggag cccgacgtcg agcgcgcggt 16620
gcgcgagggc gcgagctaca gcaagtgggg cggattcctc gacgggttcg cgcagttcga 16680
cgcgctgttt ttcgggatcg cgccgcgcga ggccgccgac atggacccgc aggagcggct 16740
gttcgtggag agcgcgtggt ccgtgctgga ggacgcgggc tacacccggc ggcgcctcgc 16800
```

cgagcaacac cgctcccgcg tcggggtgtt cgcgggcatc accaagaccg gcttcgaccg 16860
gcaccgcccg gccgcccccg cggagacgga cgcttcctcc gccacgggcg gcgtgccgcc 16920
cgcctccccg cgtacgtcct tcggctcgct cgccaaccgc gtctcgtacc tgctcgatct 16980
gcgcgggccc agcatgcccg tcgacaccat gtgctcggcg tccctcacgg ccgtgcacga 17040
ggcgtgtgag catctgcggc acggcgcctg cgagttggcc gtcgccggcg gcgtcaacct 17100
gtatctgcac ccctcgacgt acgtggagct gtgccgttcg cggatgctcg cccgcggcgg 17160
cgagtgccgc agcttcggca ccggcggcga cggcttcgtg cccggcgagg gcgtcggcac 17220
ggtgctgctg aagccgctgt cgaaggcgga ggccgacggc gaccccgtac acgcggtgat 17280
cctcggctcg gccatcaacc acggcggccg caccaacggt tacaccgtgc ccaatccgcg 17340
cgcgcaggcg gagctgatcc gcgaggcgat ggaccgcgcg ggcgtctccg ccgacgaggt 17400
cggctgtgtc gaggcgcacg gcaccggaac ggcgctcggc gaccccgtcg agatcgaggg 17460
cctggcgcag gcgttcgccg accgtacgga cacggcggcg ccgtgcgccc tcagttcggt 17520
gaagtccaac atcgggcatc tggaggccgc ggcgggcatc gcgggcctga cgaagctcgt 17580
gctccagctc cggcacggcg agctggcgcc cacgctgcac gccgaagtgc ccaaccccga 17640
catcgacttc ggctccgtac cgttcgcgct ccagaccgcc gcggcgccct ggccgcggac 17700
cggagggaac agcggacggc ggatcgcggg gctgtcgtcg ttcggcgcgg gcggggcgaa 17760
cgcgcatgtg gtcgtcgcgg agtacacggg cgccccggcc gcccgcacct ccgcacctgc 17820
cgtggccgac gggtccgccg cgaccgccgg gtccggacgc ccggtgctgc tgccgctgtc 17880
cgccaggacg cccgaggatc tgcgggcccg cgccgtacag ctcgccgact ggctcgactc 17940
ccgcgacgcg gtcgatctga cgtcggtcgc ggcgacgctc cagacgggcc gcgaggccat 18000
ggacgagcgg ctgtgctgtg tggcgtccac gcccggcgaa tggcgcgaac agctccgtgc 18060
gttcgccgac gacccggagc gcgagggccc ctggcaccgt ggccgggtgc gggcgaccgg 18120
cgaggcgctg gccgcgctgg cggagaagga cgaactccgg gcgctcgtcg gacgctggac 18180
cgcccgcggc gagtgggcgg aactggccgc gttctgggcc aagggcatgc cgctggactg 18240
gagccgcctg tacgcggacg gccgggtccc ggcccggctc catctgcccg cctatccctt 18300
cgccgggcgc cgctactggc ccggacccgc ggacgtacgg aacacggcgg acgcgcaagc 18360
gccccgcacc tccacgccca gcccgtccac gctcagcacg tcaactcccg gcgcgtccag 18420
gcccgttgcc gtcgcgcccg ttgccgccgc gccgtcggcg gagtcgtaca tcgaacgcgt 18480
gctgctcgac gcgctcggcg aggccctcca gatgacgccc gcggagatcg acccgcgccg 18540
cccgttcgcg gactacgggc tggactccat cctcggcgtc cacctggtca acgtcctcaa 18600

cgagacgctg ggcaccggcc tggagaccac cgacctcttc gaccacggca ccgccgagcg 18660
gctgcgcgcg ttcctcaccg agacctacgg cggcacggtg accgtccccg acggcacggg 18720
cgccgctgcc gagttcgtcc ccgacgctcc cgtcccggcc cgcgaggcgg acgacccggt 18780
cgccgtcgtc ggcatggccg cgcgctacgg cgacgccgag gacccccgcg ccctgtggga 18840
ccgcctgctg gccggtgacg acctcgtcga gccggtcacc cgctgggacc tggggcccga 18900
agtgacgtgc cgcgcgggca gtttcgtacg cggcatggac cggttcgacc cggtcttctt 18960
cgcgatctcc ggtgtcgagg cggcccatat ggacccgcag cagcggatct tcctggagca 19020
gtgctggaac gccctggagg acgccggata caccggcgag cggctgcgcg agcgcaactg 19080
cggcgtgtac gtgggctgtt acgcgggcga ctactacgac agcatcggcg accgcgcccc 19140
ggcccaggcg ctgtggggca ccatgggctc ggtcgtcgcc tcgcgcatcg cctatcaact 19200
cgacctgaag ggcccggcgc tcaccaccga cacttcctgc tccagctcgc tcgtctccct 19260
ccatctggcc tgccgcgatc tgcgcacggg cgccgccgac atggcgatcg cgggcggtgt 19320
cttcctccag acgacgccgc ggctgtacga ggccgcgacc cgtgctggca tgctctcgcc 19380
caccggccgc tgccacagct tcgactcccg cgccgacggc ttcgtccccg gtgagggcgc 19440
gggcgccgtc gtactgaagc ggctgtcgga cgcgttgcgc gacggcgacc acgtctatgg 19500
cctcgtccgc gccaccggcg tcaaccagga cggcaccacc aacggcatca ccgcgcccag 19560
cgcggcctcg caggaggcgc tgctgcgcga ggtgcacgcg ggcgtcgcgc ccggcggcgt 19620
ccagttggtc gaggcgcacg gcaccggtac gcagctcggc gacccgatcg aattccgcgc 19680
cctcagccgg gtgttcgggg acgcgcccgc cggcagcgtc gtcctgggct cggtgaagac 19740
caacctggga cacacccagt tcgcggcggg catcgccggt gtcctcaagg cgctgctggc 19800
gttgcaggag cagcgcgttc cgccgtcgct gcacttcgcg gaggccaacg cgcgcgtacc 19860
gctggacggc agtcccttca ccgtcgcgac gacggcacag ccgtggcccg agcccgccga 19920
gggaccgcgc cgggcagccg tcagctcctt cggggccagc ggcaccaacg cccatgtcgt 19980
actggaggag cacccgcccg tacgggcgac cacggggccg gagtccgccg gaggggacgg 20040
cgaggccgcc ttcctgctgt cggcccgcac ccccgccgcg ctgcgggccg tcgcggagcg 20100
gctgctcgcg cggatcgagc gtgaaccggg cctgcccgcc cggcaggtgg cctacagcct 20160
cgccgccggg cgtcgccact tcccgcaccg gctggccgtc gtcgccaccg ggctgcctgc 20220
tctcgccgcc cggctgcgtg cctggctggc ggacgaacag ccgggcggcg aggggacgtt 20280
gctgcacggc gtcgcccacg ccggaacgcg gcaggccgcg ctgggcgggc tcgcacccgc 20340
cgagctggcg gcggcgtatg tgggcggcgc cgaggggccg ttcgccgaga gcttcccggc 20400
cggggcgcgg cgtcaagtgc cgctgcccac ctatccgttc gagcggcagc gctactgggc 20460

ggaggggacg gacggacacg ctgtcccggc cgccgccggt acgtccgccg tggagccggg 20520
cggccggcgc accgcgtacc gcacgcggct cacgggtgag gagttcttcc tcgccgatca 20580
ccgggtgggc ggccgtacgg tgctgccggg cgtgctgacg ctggaggcgg tacggcgcgc 20640
ggtcaccgcc ggagacggcg gcgacgggac cggaatcggc accggcggcg gtacgggcgt 20700
cccgactccc ctgcggttgc gtgacgtcgt gtggcccgcg cccttccccg tcggcgcgga 20760
cggggccgaa ctgcgcgtcg atctcgacgg cgacgccttc gccgtacggc aggacggctc 20820
gtccgtgcac gcgcagggcc gctggacgat ggtgcccgcc cccgccgcct ccacgtcgct 20880
ggagaccctg cgggagcgct gcgcacgccg cacgctgacg cgcgagcagt gccgtgcggc 20940
gctggaggcc gtcggcatcc ggcacggtga gcggctccgc gcgatcgagc aactgtccgt 21000
cggggacggc gagttgctcg cccggctggt gctgcccgcg accgtggaga cggggacgca 21060
ggccacggag acgttcggac tgcacccggc gatgctcgac agcgccatcc aggccgtcgt 21120
cggactctac ggcgacgaga ccggagccct cggcgagcgt ccggacgccc ccgcactgcc 21180
cttcgccctg gacaccgccg acgtcctcgc tcccaccacc gaccggatgt gggcccatct 21240
gcgctgggcg gacggttacg cgcccggcga ggccggagag gtgacgaaga ccgacatcga 21300
tctgtacgac gacgcggggc ggctctgtgt gcgcctgcgc ggctacgcct cccgccgcgt 21360
cacgcccgcc gccaccggct ccgcgaccgc ggccccggcc ggtacggacg acgccgacgc 21420
gccacgggcg cagctcctcg caccggtgtg ggacgcacag ccgcatgcgg acggcccccg 21480
cagccccgag cccggtgcac acgtcgtcct gctcggcggc acaccggagg aacgggacgg 21540
gctgcgccgt ctcgtcgccg acgtcaccgt cgtggaaccg gaacgccacg cgtccgccgg 21600
ggagttggcc gcgctgctgc cgacgggcgc cgagcacgtc gtctggctcg cgcccgcga 21660
cgcgtcgccc gccgcctcct ccgccgaggg acccgacggg gcgctcgccg tcttccggct 21720
cgtcaaggcg ctgctcgcgg acggcgcgga cgccagggag ctgagcttca ccgcggtcac 21780
ccgccaggcc cggctgctgc ccggcgacgc ggactgcgac ccggcgcacg ccggagtgca 21840
cggtctgctg ggcacgttgg ccaaggagta cccgcactgg cgggtgcggg gcgccgacat 21900
cgagcgggac gtctccgtac cgtggccgga gctgctgtcg ctgcccgcgg atccgcgcgg 21960
cgaggtgtcg gcccgccggc acggcgagtg gtaccggcag cggctgctgg aggtcgccct 22020
cgacgcgtcc ggcgccgcct ccttctccgc cggctcccag gcccccaacc cccaggcccc 22080
caactctcag gcgagtggcg cccgttcggc actgcgcgag cccggcggcg tcgtcgtcgc 22140
catcggcggc gcgggcggca tcggcaccgt gtggaccgag cacatgatgc gacggcacgg 22200
cgcccgcgtg gtgtggatcg gccgccgccc ctacgacgag gagatcgccg cgcggcagga 22260

ccgcctcgcg gcctgcggcc cgcgcccgga gtacgtacgg gccgacgcga ccgacgcccg 22320
cgctctgcgc cgcgccgtcg cggagatcga gcgccgccac ggccccgtac gcggcgtcct 22380
gcacaccgcg atcgtcctcg gcgaccagag cctggccagg atggacgagg ccgccttccg 22440
caccacgtac gaggccaagg ccgccgtctc ggtcaacatg gccgacgcgt tcgccggtca 22500
gccgctggag ttcgtggcct tcttctcctc catgcaggcg ttcttcaagg cgccgggcca 22560
ggccaactac gcggcgggct gcacgttttc cgacgcctgg gccgagcggc tctccaccgc 22620
gctcgactgc cccgtgaagg tcatgagctg gggctactgg gccggagtcg gcatcgtgac 22680
cgccgacggc taccggcagc gcatggcgca gctcgggctc gggtccatcg aaccggacga 22740
gggcatggcc gccttcgacg cgctgctggc ctccccgtac cggcagctcg ccctgctcaa 22800
ggcgaccgac agccgcagca tcgacgggat ctacggcgac gacgagctgc ggcaactgcc 22860
gcccgccgcg cccgcgctcg cggacaccct ccgcacggac cgccccgacc ggaacgcgga 22920
gatccggcgg ctgcgggagc aggccgacgg ccacgccgga gtcatgtacg acgctcttgt 22980
ccgcgtcacc tgggcgctgt tgacgtcgct gggactcttc cgcgacggcc gcgcggccac 23040
cgccgccgag tggcgcgccg tcggcggcat cgaggagcgc taccagcgct ggacggaaca 23100
cacgctggag gtgctgaccg ccgccggacg tctgcgccgc gcgggcgagg accggtacgc 23160
cgccgtcgcc cccggagccg tacccgccga ggacgcctgg gccgagtggg accgggcgcg 23220
ggaggtgtgg ctcgcggacg aggccaagca ggcgcaggcc gtgctcgtcg acaccacgct 23280
gcgggagctg acggcgatcc tcaccggccg ccgcgccgcc accgacgtga tgttcccggg 23340
ctcctcgctg cggctcgtcg aggccgtcta caagaacaac cccgtcgcgg actacttcaa 23400
cgaggtgctc gccgacaccc tcgtcgccta cctcgaacac cggctgcgcc aggacccgtc 23460
cgcgcggctg cgcatcctgg agatcggcgc cgggaccggg ggcaccagct ccgtggtctt 23520
ccggcggctc cggccgctgg ccgggcacat cgagacctac acctacaccg acatctccaa 23580
ggcgttcctg ctgcacgccc ggcgtgcgta cggggagatc gcgccgtacc tggacgggca 23640
gctcttcgac gcggagaagc cgctcgccgg acagccggtc gccgtcggcg gacacgacgt 23700
ggtgatcgcc accaacgtgc tgcacgcgac gggcaacatc cgcaacaccc tgcgcaacgc 23760
gaaggccgcc gtacgcgcca acggcctgct gctgctcaac gagttgagcg acaacatcct 23820
cttcagccac ctcaccttcg gtctgctgga cggctggtgg ctctacgacg acccggcgcc 23880
gcgcatcccg ggatcgccgg ggctgacgcc gcagagctgg cgccgcgtcc tggacgaggt 23940
gggcttccgc gggtcgttcg tcgcagccga gggcgccgac gacctcggcc agcaggtgat 24000
cgtcgccgag agcgacggag ccgtgcggca gccgcggccc ggcggggtct ccgccttccg 24060
gggcagcctg ccggaggcgc ggccggctca acccacgggc ggggcggggc acttggcggt 24120

gccggcggag cacggctccg cgcctgccgt gaccgtgccg gtcaccgccg cgtccgcttc 24180
ctccgcaccg ggctccgcgc ccgccgccgt cccgtccggt gatccgtccg gcgacgggag 24240
catggccgca cgtgtggccg gaccggcacg ggacctcttc cgggggctcg tcgcggacgt 24300
cctgcaactg cccgtcggcg acatccgtgc cgacgtgccc ttcgagcggt acggcatcga 24360
ctcgatcctc gtcgtccaac tcaccgacgc cgtacggaag gtgctcgacg gcgtgggcag 24420
cacgctcttc ttcgaggtga gcacggtcga cggcctcgtg gagcacttcc tgcgcacccg 24480
gccggacgaa ctcgccgcgc tcgtcggcgt atccgccgcg gagcacccgg aacccgcggc 24540
ggaagccgcc gcaccggagg cggtcaccga ggagccggcg gcctctgtac ccgcacccgc 24600
acccgtagcc gctcctgtct ccgtacccgt gcccgccgcc ccgggtgagg acgtccccgt 24660
cgccgtcgtc ggcatggccg ggcgctaccc cggcgccgcc gacctggacg ccttctggga 24720
gaacctgctc gcgggccgcg actgcgtcac cgagatcccc gacggccgct gggaccacgg 24780
ccgttactac gacgagcgcc gcggcgtgcc cggcaggacg tacagcaagt ggggcggatt 24840
cctcgacggc gtcgacgagt tcgactcgct gttcttcggc atctcgccga aggccgcgtc 24900
cacgatggac ccgcaggagc ggctgttcct ccagtgcgcg tggacggcgc tggaggacgc 24960
gggccacacg cgggcctcgc tgcgctccgc ctcccgcgcc cggctgcccg aagacgccgg 25020
ggacatcggg gtgttcgtgg gcgcgatgta ctccgagtac cagctctacg gcgcggagca 25080
gggcgtacgg ggcgagcccg tcgtcgtacc cggcagcctc gcctcgatcg cgaaccggct 25140
gtcgtacttc ctcgacgcgt ccgggcccag cgtcgccgtc gacacgatgt gcgcctcggc 25200
cctgtccgcc gtgcatctgg cgtgtgccgc gatccggcgc ggtgagtgcg cctcggcggt 25260
ggccggcggc gtcaatctgt cgctgcaccc cagcaagtac ctgatgatcg gcgagggaca 25320
gttcgcctcc tcagacgggc gctgccgcag cttcggcgcg gacggcgacg gctatgtgcc 25380
cggcgagggc gtgggcgcgg tgctgctgcg gccgctcgcc gacgccgtgg ccgacggcga 25440
ccgcgtgctc ggcgtgatcc gcggcagcgc cgtgaaccac ggcgggcaca cgcacggctt 25500
caccgttccc aacccgctcg cccaggcgtc cgtgatccgc ggcgcgtggc gccgctccgg 25560
cgtggacccg cgcgacatcg gctgcatcga ggcacacggc acgggcaccg cgctgggcga 25620
ccccgtggag atcgccggac tgaacgccgc cttcggcgag ttcacctccg agcgcacctt 25680
ctgctccctc ggctccgcca agtccaacat cggacatctg gagtcggcgg cgggcgtcgc 25740
gggcctggcc aagatgctgc tccagatgcg gcacggcacg ctggtgccgt cgctgcacgc 25800
cgagcgcacc aaccccgaaa tcgacttcgc cgccacgccg ttcgtgctcc agcgggaggc 25860
cgcgccctgg ccgcgccgcg aggggcgccc acggctcggc ggcatctccg cgttcggcgc 25920

gggcggttcg aacgcgcatc tgctcgtcga ggagtacgta ccgacggcgg caccgccgcg 25980
gcgtgcggcg ccgggcccgg tcctcgcggt gctctccgcg cgggacggcg agcgcctgcg 26040
ggagtacgcc gggaagctgc gggacgcact gcgctccggg cagtggaccg acgaggacct 26100
gccggacatc gcctacaccc tccaggtcgg acgggaggcg atgagcgcac ggttcgccgc 26160
cgaggtgagc accctggccg gactcatgga cgcgctggac gcgtgcgcac ggggcgccgc 26220
cctgccgccc ggcgcccggc tgcgtaccga cggcgggcgg ggcggaccgg tccaggacct 26280
cgcggacgac gaggacttcc gggagaccgt cgtgcgctgg ctgcgccgcg ggaagctggc 26340
gccgctcgcc gaggcgtgga ccggcggcct cgacgtggac tgggcccgcg gccacggcac 26400
cggcgaggac cggccgcgca aggtcggcct gcccggctac ccgttcgcgc gggagaggta 26460
ctggtggaac gacgggctgg ccgaggccgg aggcgagggc gctgacggtc tgggagacga 26520
gggcgccgcc ggcggcaccg ccggttccgg taacggttcc gggccccgtt ccgctcgtac 26580
ggacgggacg cgccccggtg aactgccccc gggcgacctc acgttgcacc ccgtctggga 26640
gcccgtacat gcggcgggcg gcggcgcgga cgcgcccttc ccgcagcccg cggaccgcgt 26700
cgtcgcggtc ggcctggcac cggaggcccg tgccgcgctg gaggcgtacg gcacccgtgt 26760
ggtgacgctc ccggcacccc gggacggcgg ccgttccgtg gcggacgtcc gccgcgaact 26820
ggagaccgcg ggccccttcg accacgtcgt cgtggagtgc cccacccccg ccgcacaggg 26880
cgcgcggcag cgcgtcgagg ctcaacgcgc ctccgtacgc ggcctgttcc ggctgctcca 26940
ggcgctctcc gccctccgcg cggacgagcc gcggaccggt ctgaccctcg tcacccgcga 27000
cgcgttcgac ccggatcgca cgggcggcgc cgacccggcg caggccgcgc tgcacggcct 27060
cgtcggcggc ctcgccaagg aacagccgta ctggcgcgtg cgcgccgtcg acctggccga 27120
gggcgagccc ttcgtgcccg aggagatctg cgccctgccc gccgaccgcc gggcgcatcc 27180
gctcgtccgg cgcggcggcc agtggctgag ccgcaggctg ctgcccgtcg gcgacgtacg 27240
gcccggcacg ccggacgacg gcccgcgtac ggctgttgac gggacggacg ccgcgccgca 27300
ggccgtctcc gtcccgtccg gttccgtctc gtccgtctcc gtcccgtccg gcggtttccg 27360
cggtgacggc gtctatgtgc tgatcggcgg cgcgggcgac ctcggcaccg tcctcaccga 27420
gcatctgctg cgccgctacg acgcccgcgt ggtgtgggtg ggccgccgtg cggaggacga 27480
cgccgtacgc gccgcggcgg cacgcgtcgc cgcggccacc ggcggcgagg cccccgtgta 27540
tctgtccgcc gacgcccgcg acccgggcgc gctcgcccgc gtacgggacg aggtgctgcg 27600
ccggtacgga cgcatcgacg gcctggtgca cctggcgatg gtcttcagcc acacgctcct 27660
cgcggagctg ccggaggagg acctgaacgc gacgctcgcc gccaaggccg acccgaccga 27720
gcacttcgcc gacgtcttcg cgggacagcg gctcgacttc gtgctgctgg tctcctccct 27780

cgtcagcttc atccgtaact cccaccaggc gcactacgcg gcggcctgcg cctacgagga 27840
cgcccgcgcg cccggtctgg gccgggcact gggctgcccc gtcaaggtcg tcaactgggg 27900
ctactggggc aacgtcagcg acgaagtgct gcgcggcgtc acggagatgg ggctcgcgcc 27960
catcgaaccc gcctccgcca tggcggccgt cgaagagctg ctgaccggcc cgctcgacca 28020
gatcggcttc atgcggctcg gccgtccgct gcccgtcgag ggcgtgctcg cgggggagac 28080
gctgagcggg catccgtacg cggcggtctc ccgtacggcc gcggagcccg cccccgtgcc 28140
ggtgccggcc gcgctggcgg agcaccacgc ggggcctgtg ccgggtgaga tcgacgccct 28200
gctgtgccgc tgtgtcgcgg ccacgctgcg gcgtgccggg ctgcgccgcc cggccgacgg 28260
cttcgcctcc ggttccggtt ccggttccgg ggccgggagc gcgggcgtgc gcgtggacga 28320
gcggttcgac ggctggttcg cggcgaccgt acgcaccctg cgcgagtacg ggctcgtcga 28380
ctcccgcggc gactggagcg agcgcgcacc gggcgcgggg gacgccgccg cctgcctggc 28440
cgagtgggag cgcgcggccg agcggtgggc ctctgcgcac gccgatctgc gggcgccgac 28500
ggggctgttg ggccgtacgc tgcccgcgct ggccgacatc ctgcgcggcc ggatcccggc 28560
gaccgacgtg ctgttcccgg aggggtcgtt ctccctggtg gagggcgtct accgggacaa 28620
cgccgtggcc gcgcacttca acgccgtact cgccgcacag gtgacggcgt tcctgcacgg 28680
ccgccgcgcg gccgacccgg cggcgcggct gcgcgtactg gagatcggcg cgggcaccgg 28740
cggcaccacc gcgcccgtgc tggagcaact ggagtgcgcg ggcctggagt tggccgagta 28800
ctgcttcacc gacctctccc tcgccttcct ccagcgtgcc gaggacgcct tcggccccgg 28860
ccgcgcccac ttcgcctgcc gcaccctcga cgtgtcacgg gcaccgcgca cgcagggctt 28920
cgacgcgggg gcgtacgacg tggtgatcgc cgccaacgtg ctgcacgcca cggacgacgt 28980
acggaccgcg ctgcggcacg ccaagtcgct gctgcgcggc ggcggaatgc tggcgctgaa 29040
cgagatcagc ggcttctacc tcgtcaacca cctcaccttc ggcctgctgg acggctggtg 29100
gctctacggc gacgccgaac tgcgggcgcc gggcagcccc gcgctgcctc cggagagctg 29160
gcgccgggtg ctgacgcagg agggcttcac cggcgtcgcg gacccggcac gggacgcccg 29220
tgccctggga cagcaggtcg tgatcgccca cagcgacgga ctggcccgcg gcccggtgac 29280
ggacgcggca ccggcggcac cggcagcacc ggcggccgtg gcgcggccgg agacgaacac 29340
ggcggtgagc gcggcgccca acatggcggt gagcgcggcg agttcggcgg cgggcggtcc 29400
gcagacctcc ggcggtccgg acgtgcgcgt ggtcgccgac gtcgtcgaga cggagctggc 29460
cgacgcgctg cggctgccgg cggaacggat cgaccgggcg ggcgcgttcg cggacgtggg 29520
cctggactcc atcgtcggcg cgcgcttcgt acggcggctc aacgaggaac tgggcctgga 29580

cctgccgacg acggtgatct tcgattaccg gagcgtcgac gaactggccg cgcacatcgt 29640
ggaggaccac cgtccgacct cgcctgcgcc gggcggtacc ggggcggcca ccgctcagga 29700
gcccccggcc gagcgggagt cggggcgcgc cccggagcgg gagcacgggc ccgttgtggc 29760
gcccgatgtc accgtgcccg atgccaccga gcccggttcc gccccgtacg gccgggagcc 29820
catcgccgtc gtgggcgtca gcgggcgctt cgccggttcc gacgatctcg acgccctgtg 29880
gcggcatctg gccgcgggcg acgacctcgt cgggccgatc gaccgctggg atctctcggc 29940
ctacggcgag gacgaactga cctgccgcag cggcagtttc ctcgacggca tcgaccggtt 30000
cgacgcccgc ttcttcaagc tgtcgggccg cgaggccgcc tacaccgacc cgcagcagcg 30060
cctcttcctc gaacaggcat ggacggccct ggaggacgcc gggcacggcg gcgcctcgac 30120
cgacggcatg cgctgcggcg tctacgtcgg ctgcaccggc ggcgactaca aggaccactt 30180
cgaggacgcg ccgcccgcgc aggccgtctg gggcaacgcg ccctcgatcg tccccgcgcg 30240
catcgcctac cacctcaacc tccagggccc ggccatcgcg gtcgacacgg cctgctccag 30300
ctcgctggtc gccgtgcatc tggcctgcca gggactgtgg agcggcgaga ccgagatggc 30360
cgtggcgggc ggcgtcagcg tgcagaccac tccggccacc tatctctcgg ccagccgcgc 30420
cgggatgctc tcgccgacgg gacgctgcca caccttcgac gccgccgcgg acgggttcgt 30480
accgggcgag ggcgtcggcg tcgtggtgct gcgcaggctc tcggacgcgc tgcgcgacgg 30540
cgaccacgtg cacgccgtca tccgcggttc gggcgtcaac caggacggcg ccaccaacgg 30600
gatcaccgcg cccagcgccc tgtcccagga acggctgctg cgccaggtct acgaggactt 30660
cgcgatcgac ccgtccgaga tcggcatggt cgaggcccac ggcaccggca cacagctcgg 30720
agacccgatc gaatgccacg ccctgcggcg ggtgttcgag ggcagcgacg tccccggcgg 30780
ctgcgcgctc ggttcgatca agacgaacct cggccacacc acgtccgcgg cgggcgtcgc 30840
gggtctgctg aagatcgtcc tgtcgctgcg gcaccggcag atcccgccct ccctgcacta 30900
ccgcgaccgc aatcccgaga tccggctgga aggcggcccc ctgtacgtga acacctcact 30960
gcgcccctgg gagccgaacg cgggcggcag ccgtgccgcc gccctcagct cgttcggctt 31020
cagcggcacc aacagccatc tcgtcgtcga ggaggcaccg gcgcgtcccg ggcggtcccc 31080
gctctccggc gccgccgccg tggaggagcc cggactgccc cgggtcttcc cgctgtccgc 31140
gccccagccg gcggcgctgc gcgagcgcgt ccgcgatctg gccgtccatc tgcggagcac 31200
gccggacgcc gtcctcgtcg acgtcagcca caccctggcg acgggccgcg cccacttcgc 31260
gcaccgcgcc gccttcgtcg cccgcacccg cgaggagctg atcggtcaac tcgacgactg 31320
gctcgacggg gaggccggag acgccgggaa ggcggcgaag accggggagg ccgcgaagac 31380
cggagacgtc ggcgaggccg gggcgccgg gccggaggag ctggcccgcg accgctacct 31440

cgccggtgaa cccgccgact tcgccgcgct gttcgccggt tccggcgccc gtcgcacacc 31500
gctgccgacg taccccttcc agcgcaggag ccactgggtg cgcggcggcg caccggggag 31560
cgccccggac gcggccgggt ccggtacgtc caccacgtcc ggcacgcccg ccctccgtac 31620
cgacgcaagg gagaagggcc gcggagccgc ccgcgcggag gacgacgccg tcgccgtcgt 31680
cggcctctcc gcccgcttcg cgcagtcgcc ggacgccgag gccctgtggg cacatctcgc 31740
cgcgggcgac gacctggtcg gcgaggtgac ccgctgggac ctgtcgcaga tcagcggcgg 31800
acgcaccgaa cacggcagct tcgtcgagga catcgcccgt ttcgacgccc tgtacttcgg 31860
cgtctcgggc aacgaggcca cgtacgccga cccgcagcag cgcatctacc tggaggagtg 31920
ctggcacgcc ctcgaggacg ccggttacgc gggggagcgg ctggacgggc ggggctgcgg 31980
cgtctacgtg ggcgcctacc ccggcgacta ccacgagctg atcggcgccg accgcccgcc 32040
gcagacgatg tggggcaaca tggcctcggt catcgcctcg cgcatctcct acttcctcga 32100
cctggacggc ccggcgatgt ccgtcgactc ggcctgctcc agctcgctcg tcgccatcca 32160
caccgcgtgc caggacctgc gtctgggcac gacctccatg gcgctggcgg gcggtgtgtt 32220
catccaggcg acgccgcggc tctaccagta ctcgggcaag gcgcggatgc tctcggccac 32280
cggacgctgc cacgccttcg acgccgccgc ggacgggttc gtccccggcg agggcgccgg 32340
agtcgtcgtc ctcaagcggc tgtcggacgc gctgcgcgac ggcgaccgcg tctacggcgt 32400
gatccgctcc tcgggcgtca accaggacgg caccaccaac ggcatcacgg cacccagcgg 32460
cgcggctcag gagaacctcg tccgcgacgt gtacgagcgc gcgggcgtcg ccccgtccgg 32520
gatccagctc atcgaggcgc acggcaccgg cacaccgctc ggcgacccga tcgaattcga 32580
ggcgctgcgc gccgtgttcg cggacgcgcc gacgggcggc tgcgcgctgg gcacgatcaa 32640
gagcaacgtg ggccacaccc agttcaccgc cggagtcgcg ggggtcctca aggtgctgct 32700
cgcgctcgac cacgaacagc tcccgccctc cttgcacttc acccggccca acccggccat 32760
cgacctcgcg aacagcccct tccacgtcaa caccgaactg ctgccctggc gcgcgcccgc 32820
cgacgggccg cgccgcgcgg gcgtcagctc cttcggcgcc gccggcacca acgcgcacgt 32880
cctgatcgaa caggcaccgt ccgacgccgc cgcccgcgca cgccgacacg ggcgcgcaca 32940
gtggctgttg gtgctctccg gccaggacgg caccgcgctg cgcgcccagg ccgagcggat 33000
gctggaccac gtcgaacgcc acccggacct cgacctcggc gacaccgcct ggacgctcgc 33060
cacgggacgc cgccacagcg ctcaccgtct ggcgtgcgtc gccgccgacc gcgagcagtg 33120
gacggcggca ctgcggggct ggctgcgcga cggccgtgcc gagggcgtgt ggacgggcga 33180
ggccgacgag tcgccccgct ccgggcacag cggcgagagc ggcgagggca gcggcgaacc 33240

ggcccgcgcc gaggcgctga tggccgagca cgaccgtccc ggaaacctcg ccgcgctcgc 33300
ggagctgtac gtacggggcg aggtcgcgcg cttcgcaccg ctgtacgccg acggggactt 33360
ccgcatcgtc tccctgcccg gctacccctt cggcggcgag cgctactgga ccgggccgct 33420
gcccggggac acgcccgacg ggacggacgg aacggacggg acgtacggca cggacgggat 33480
cagcgaatcc ggtggcgaat cccggccgtc cgccgaaccc cggccgtacg ccggggcgtt 33540
ggcgctgacc ggggaggagt tcttcctcga cgaccaccgg gtcggcggcg tccccgtact 33600
gccgggcgtc gcgtatctgg aactcgcgca cgcggcggcg accgcacagg gcggcctcgc 33660
cccggcggt gtgctgctgc gcaacgtcgt ctggtcccgc ccggcgcgcg tcaccgagcc 33720
gctctccgtg gagacggtgc tcgaaccacg cgccgcggac ggcacgttcg gatacgagat 33780
cgccaccgtc cgggacggcg cccggcggct ggtgcacggc cggggccgga tcgagccgcg 33840
tcccggcggc gcgcccgccc ggctcggcct cgccgcgctg cgtgagcggt gcgacgtgcg 33900
gagcctggac cacgcggagt gctacgcgtt gctcggcgcc accgggatgt cgtacggcgc 33960
cgcgatgcgc ggtctggagg agctgcacgt cggccgcggg ctcgcgctcg gccggctgcg 34020
cgttccgcgc gaggcacgcg acggacgccc ctggacgctc catcccgccc tgctggacgc 34080
ggcgttgcag gcgacggtcg ggctggccct ggacggggag tccgacgggc tgacggccgc 34140
actgcccttc gcggtggagc aggtgcaggt gctcgccgcg agcccggaga gcggctgggc 34200
cgtggcccgt cccgcggacg gcgccgccga gggcccggtc cggcggatgg acgtggagat 34260
ctgcgacgac gagggcacgg tgtgcgtacg gctcctcggc ttcagcaccc gcgaactccc 34320
gggcgccacc gcgtcggtga cgaccggagc gacgaccggg gcggggtccg gggcggggtc 34380
cgccgccgcg tcccctgctc cggccgccgc cgatcccggc gcgcccgccg acggctccct 34440
ggtcttcgcc cggcccgtct ggcgcgccgt gccctccgca gacgtacggg aggagcgccc 34500
ggcgccgagt ccggcaccgt accgggagat cctgctggcc ggtccggagt ccgtcgacgc 34560
tgcggaggtg cggaagcgct cgggcgtccc gtgctcggcg ctgcccggcg gcgccgatct 34620
gcccgagcgg tacacccggc aggcccaggc gctgctggcg aaggtgcagc aactcctgcc 34680
gcgcgtacgg gaggagcgcg tcctgctcca ggtcgcggtc cccgcgcacg gagaaggccg 34740
gctcttcgcg gggctcgcgg gtctgctgcg tacggcctgc gcggagcacc ccggactggc 34800
cgcgcagctc gtcgagaccg acgccgccga cgcggcgacg ctctgcgccc acctcgacgc 34860
cgaggccgcg cagcccggcg tggcgacggt gcgccgcacg ggcggcgaac ggctggtgcg 34920
gcagtggcac ggcttccgtc cggagcgcgg cgatcagccc tggaagccgg gcggggtcca 34980
tctcgtcacc ggcggcgccg gcggcctcgg agcgctgttc gcccgccgga tcgcccgtac 35040
cgcgcccgga tccgtactgg tgctgtgcgg ccgctccccg gagggcgcgg cacagcgcga 35100

actcctgggt gagctgcggg agtcgggcgc cgcacacgcg gagtaccaca gcctggacgt 35160
cggcaggcgt gcggacgtcg tccggctcgt gcggcaggtc gtggaccggc acggacggct 35220
cgacggcgtg atccacagcg ccggagtgct gcgggacggc ttcgtcgccc acaagacccc 35280
ggagtacctg ggcgaggtct tcgccccgaa ggccggggga gtggtgcacc tcgacgaggc 35340
caccgccgca ctggagttgg acttcttcct cgtcttctcc tcgatgtcgg tgctcggcaa 35400
ccccggccag gccgactacg cggcggccaa cgcgttcctg gacgcgtacg tcgcccaccg 35460
cgccggactg gcggaccgcg gtgagcgcca cggccgctcg ctctcggtcg gctggcccct 35520
gtgggcggac ggcggcatgc acgtggacgc ggccacggag cgccgcatcc accagagctc 35580
cggaatgcgg ccgttgcgtg cccgtgaggg gttcgaggcg ctggagcgcc tgtacgggag 35640
cggactgccg cacgcgctga ccgcgttcgg cgaccgcgag cgcatcgcgt cggtgctgct 35700
cgacggttcc gagggctccg acggctcggc tcgtccggac ggtccggacg cggagcggga 35760
gacggacgag cggcgccgga ccccggcgga cgcgaacgac gaacggaacg aggccatgtc 35820
acacacggcg ctggtcggcc gactcgccgc ccatctctcg gagttgctgg acgtaccggc 35880
ggaggagatc gagggcgggg tcgagctgag cgagtacggc ttcgactcga tctcgctgac 35940
ggagttcgtc acgctgctca acggcgcgta cgggctgtcg ctcgtgccga cggtgctctt 36000
cgagcactcg acgctcgacg gggtcgcggg acatctgctg gaggagtacg cggaccgctt 36060
cgcgccggag ccggagccgg agccggagcc gcagccggtg caggcgcaga tgccggagcc 36120
ggtgccggtg ccggagccgg aacctgcacc ggtgccggcg cgcgggcccg tggcaccgtc 36180
aaccgccccc gtggcggccg acgatgacga cgcgctgcgc cgtgcgctgg tcaagcggct 36240
gcgggagctg acgtcccgca tcctccgggt gcccgcggag aagatcagcg ccacgcagga 36300
gatgagcaag tacggcgtgg actccctgtc gctggcggag ctggcggcgg ccgtgaacgc 36360
ggagttctcg ctgatgctgg acccgacgct gttcttcgag cacccgacgc tggaggccgt 36420
cgcccgctat ctcctcgacc ggcacgccga ccggctcacc ggcctggtga ccgaggagac 36480
ccccgaaccg gccatgaccg aacaggccgt ggctgaaccg gtcgtggccg agccgcccgt 36540
cgtcgaatcg cccgctacga cgtcacccgc cgcggagacg tccgtcaccg agacgtccgt 36600
acgtgaaccc gccgcccccg cggcggctcc ggctccggct ttcgccgcgg ccccggggcc 36660
gggtgctgcg gaggagcccg tcgccgtcat cgggatcagc gcgcgttttc cgatggcgga 36720
tgatctggcg gagttctggg agaacttgcg tgagggccgg gactgtatcc gtgaggtgcc 36780
ctcggatcgc tgggactggc gcgagtacta cggcgacccc gtcaaggagc ccaacaagac 36840
caacgtgacg tccggtggat tcatggacgg cgtgggcgac ttcgacccgc tcttcttcga 36900

catctcgccc aaggaagcgg agttgatgga cccgcagcag cggctgctga tgctccacac 36960
ctggaaggcc ctggaggacg cgggctatgc gccggacagt ctcgccggca ccggcacggc 37020
cctcttcgtc ggtacgacga acaccggtta cggaagcatg gtcagccgct attcaccggt 37080
gatcgaggga tacgacgcga ccggggccgc gccctgcatg ggcccgaacc ggatgagcca 37140
tttcctcgat ctgcacggcc ccagcgagcc cgtggacacc gcctgttcca gttcgctcat 37200
cgcaatgcac cgcgccattc aggcaattca cgacggccat tccgacatgg cgatcgcggg 37260
cggcgtcaac acgatggtga gcatcgacgg ccacatcagc atttcccgtg cggggatgtt 37320
gagtgtggat ggtcggtgta agacgttttc ggtgggggct gatggttatg ggcgtggtga 37380
gggggtgggg attttggtgt tgaagcgttt gtcggctgcg gtgcgtgatg gtgatcatgt 37440
gtatggggtg gtgcgtggtt cggcggtgaa tcatgggggt cgtgcgaatt ctttgacggc 37500
gccgaatcct cgtgctcagg cggatttggt ggtgggtgcg tggtcgcggg cgggtgttga 37560
tccgcggtcg gtgggttatg tggaggcgca tggtacgggg acggggctgg gtgatccggt 37620
tgaggtgaac gggttgaagg ctgcgtttgc ggagttgtat gagcggtggg gtgtttcggg 37680
ggccggtgag gcgcattgtg gtctgggttc ggtgaagacg aatatcgggc atttggagtt 37740
ggcgtcgggt gtcgcgggtg tgatcaaggt gttgttgcag atgcggcatc ggacgttggt 37800
ggggagtttg cattgtgggt cggtgaatcc gtatgtgcgg ttggagggga gtcctttccg 37860
tctggtgcgg gagcgtgagc cgtggcgggc ggtacgggat gagaacgggc gggagttgcc 37920
gcgccgtgcg ggcgtgagtt ccttcggttt cggcggcgcc aacgcccata tcgttctgga 37980
ggaataccag cccccggccg gcacgcagac cgacgcccac acccgcaccg gcccctcaac 38040
caccgtccac agcggccccg ttgccgtcct gctctccgcc caccgtcccg acgtactgcg 38100
ggagtcggcg acccgctggg tcgaggtcct gcggcgcggc gactaccgcg acgccgacct 38160
cccggcgctg tcgtacacct cgcagacggg acgcaccgcc atggccgagc ggctcgcggt 38220
cgtcgccggg acgctggagg agctgcgcgc gggactggag tcctggctcc gcggcgagcc 38280
gaccccggcc gtgttcaccg gacgcgcccc gcgcgacggc gacgcaccgg cggcaccggc 38340
cgccctcacc gacgggttcg cctccggggg acgtacggag gcgcggcact gggcgccggt 38400
gctccaggcg tggacgacgg gcgccgagtg cgactggcgg acgctgtggg gcgaacggca 38460
cccgcaacgg atctccatgc cgacgtaccc cttccaactc cggcgctact ggctcgacat 38520
gaccaccccg gcgcacggcc cgcacgtctc ccgcggactg catccgctgg tgcaccggaa 38580
cacctccgac ctgagcgagc agcgctacac ctcgcacttc accggccggg agttctacat 38640
cgccgaccac cgggtgcagg gcgaacaggt cgtccccggc gcggcgttgc tggagatggc 38700
acgcgccgcc gccgtcctcg cggcgggcgg tgcggagacc gactgggcgc tgcgccaggt 38760

ggtctggtcc cggccgctga cggccggacg gcccgtcgac gtgcacaccg ccgtgtccgt 38820
gcgggcggac ggcgaaccgg ccttcgagat ctacacggag ggccccggcg gcgaacgcgt 38880
cgtgcactcc accggacggc tgcaccgcag gaccgccggg aacgccgccg aactcctgga 38940
cggacccgaa ctccccggcg gcgccggaca cttggacgtc gccgcgctgc gtgcccagtg 39000
cgacggcacc gtcctcgacg ccgaggagtg ctacgcccgg ttctccggcg tgggcctgga 39060
gtacgggccc acgctgcgag ccgtcgagac gctgagcggc ggcacccggc aggcggtggc 39120
ccggctgcgg ctgtcggccg ccgcgtccgc gaggaccggg ttcgccctgc acccgagcct 39180
gctcgacgcc gcactccagt ccacggcggg cctcttcacc ggttccggta cgtcctcggc 39240
ggccctgccg tttgccctgg accggctgga ggtgctgcgc gcgacgccct cctcggggtg 39300
ggcggtggca cgcttcgccg ccgacgaccg cccaggcggg gtgcgccgcc tggacatcga 39360
cgtgtgcgac gacgacggcg aggtgtgcgt acggatccgg ggcttccagg tccgtacgta 39420
cggcggcgac gccgccccgt ccgcctccgg cgccgcaaac ggcacccacg ccgtgaccac 39480
ggacggcacc ggaaacggca cagacaccgg caacggaaac agcaccggca ccggcagcga 39540
ggcggacgcg gacgcccggc tgctgcacct catccacgcc atcggcgagg gcgccctgag 39600
cgctgacgaa ttccagcgga gcctcatatg accactcacg ccacgtcact taccgaactg 39660
cacgagcaga tccgtaccgg acggatcggc caggacgagg ccctccggct gatccgggcc 39720
tggcagcagg gccggcagac cgggagcgag ggcgggccgg cggagcggca ggccacgggc 39780
gacgacgccg cggcccgtgg cgaggccctg cgcgagcgcg tgtgcgacat cgtgacgcac 39840
gcggtcagcg agttgctgaa ggtcggcccg gacgacctgg acgccgacgt cgaactcagc 39900
gagtacgggc tggactcgat cgtgatgagc cagctcgtca acgcggtgaa cgacgaactc 39960
ggcctggaac tcgcccccac ggtcctcttc gagcacccga atctgcgggc cttcagcgcc 40020
cacctcgccg acacgtacgc ggactcgctc tccgtacggc tgctcggcac gcccggcacg 40080
gggcccgcgc ccgccccctc gaccgcgaca tcgcccgccc cctcgaccgc gacatcggcc 40140
gaacccgcgg ccgtcgccgc tccgtcgacg tcaccgtcgg aggcccgtac ggaatcccgg 40200
gtgcctacgc ctccggcaac cggaggccgc ttcttccccg ccgccgtccc atccgcccca 40260
tccgccgaaa ccgaacccgt caccgcaccc gcacccgcgc ccgcctctga acaggcttcc 40320
cctgcgcagg cttccgctgc ggaggagccc gtcgccgtcg tcggcatgag cggacgtttt 40380
ccgatggcgg atgatctggc ggagttctgg gagaacttgc gtgagggccg ggactgtatt 40440
cgtgaggtgc cctcggatcg ctgggactgg cgcgagtact acggcgatcc cgtcgaggag 40500
cccggccgca ccgatgtgaa gtggggcgga ttcatcgacg gcgtcgccga cttcgatccg 40560

ctcttcttcg gcatcgcgcc gaaggaagcc ctccatatgg acccgcagca gcggctgttg 40620
atgctctacg tctggaaggc cctggaggac gcgggccatt cggcggacag tctcgccggg 40680
agcgatctgg cgatgttcgt cggtacgaac gacaccggtt acggcacgct cgccgaacgg 40740
tgcggaaaac gggacagcgt ctcgcccacc ggcggcgtcc cctcactcgg cccgaaccgc 40800
atgagcttct ttctcgacgt gcacggtccc agcgagcccg tggaaacggc gtgttcgagt 40860
tcgctggtcg ccatgcaccg cggtgtcacg gcgatcgccc gcgcggaatg tgagaccgcc 40920
gtggtcggcg gcatcaacac catcgtcgtt cccgacggtc acgtcagctt ctcccgtgcg 40980
gggatgttga gtgtggatgg tcggtgtaag acgttttcgg tgggggctga tggttatggg 41040
cgtggtgagg gggtggggat tttggtgttg aagcgtttgt cggctgcggt gcgtgatggt 41100
gatcatgtgt atggggtggt gcgtggttcg gcggtgaatc atgggggtcg tgcgaattct 41160
ttgacggcgc cgaatcctcg tgctcaggcg gatttggtgg tgggtgcgtg gtcgcgggcg 41220
ggtgttgatc cgcggtcggt gggttatgtg gaggcgcatg gtacggggac ggggctgggt 41280
gatccggttg aggtgaacgg gttgaaggct gcgtttgcgg agttgtatga gcggtggggt 41340
gtttcggggg ccggtgaggc gcattgtggt ctgggttcgg tgaagacgaa tatcgggcat 41400
ttggagttgg cgtcgggtgt cgcgggtgtg atcaaggtgt tgttgcagat gcggcatcgg 41460
acgttggtgg ggagtttgca ttgtgggtcg gtgaatccgt atgtgcggtt ggaggggagt 41520
cctttccgtc tggtgcggga gcgtgagccg tggcgggcgg tacgggatga gaacgggcgg 41580
gagttgccgc gccgtgcggg cgtgagttcc ttcggtttcg gcggcgccaa cgcccatatc 41640
gttctggagg aataccagcc cccggccggc acgcagaccg acgcccacac ccgcaccggc 41700
ccctcaacca ccgtccacag cggccccgtt gccgtcctgc tctccgcgcg tgagcccgag 41760
acgctgcgcg cccgcgcacg gcagctcgtc gactggctcg acaagggcga ggccaccgag 41820
gccgatctgc cgcggatctc ctacaccctc caggtcggcc gggtcgcgat gccggaacga 41880
ctggcctgtg tgacggagtc gctggccgaa ctgcgcgccc agctccagga gttcctcgac 41940
ggcgaacggc cccgtggcgt acggaccggg cgtgccgagc ggcgcggcat ctggaacgac 42000
ctggccgacg acgaggacat caccgccgcg gtcgacaact ggatggccaa gggcaagctc 42060
gaccggctgc tcaaactctg ggtcgccggc gccgagttcg actggcggcg gctgtggggc 42120
gaacaccccc cgcggcgtat tccgctgccc gcctacccct tccggctcca gcgctactgg 42180
atcgccgacg gcacaagcgg ccggtccaca cggcggccgt cgaccgcgcg cgagggaacg 42240
ccgtacggag gcaccccgcg ggacgcggag taccgcgaac tgctgcgcgg cgacgagtac 42300
ttcctgcgtg accaccgcgt gggcggcgtg ccgacgctgc ccggtgccgc ctgtctggag 42360
ctggtccgcg cggcctggac ccacgccgac cgtgcccccg acaccgcccc gctgcggctg 42420

cgcgacgtgc tgtggctgcg tccgctccag gtcaccgcgc cccgtaccgt cgccgtcgcc 42480
ctcgacccgg ccgacggcac gtacgaggtg cgcgccgcgg acggcgacga gcgcgaggtg 42540
tacgcacgcg gcaccgtcac cgctgacggc ccccacaccg tcgacggccc ccacagcgcc 42600
ggcggcgacc gtccgggcgg gaccgccgaa ccggcggagc ccgtaccggc acacgacatc 42660
gccgccctgc gcgaccgctg cccccaccgt ctcgacgccg acggctgcta cgaccggttc 42720
gcggacctgg gcctcgccta cggcccggcc ctgcgcgccg tcgagacgct gcactacggc 42780
gcggacctgg cactggcccg tctggtgctg ccggaggcgg cggccgggga acggacgctc 42840
aacccgagca tgctggacgc cgcattccag accaccctcg gcgtgctcct cggcgagcag 42900
gcccaggcgg cggacgccga acgggccgct gccggcggtt ccgaggacgt cgcggcgctg 42960
ccgttcgccg tgcgcgaggt acggatcctc gcccccaccc ccgccgaggg ctgggccgtg 43020
gcacgcgccg cggagggcga ccggcccggc gggacggtac gcaccctgga catcgacctg 43080
tgcgacacct ccgggcgggt ctgcgtacgc ctcacggggt tcagcacccg tacggtcccc 43140
gaggacggtg cgccccaact ccccggggag ccgcccgtgt tgatgatcga gcccgcctgg 43200
cgcgaggcgg aggcggcctc cgtggcggcc atgccggagg accaccgggt cgtgctgtgc 43260
gaactgcccg gcgtggacgc ggccgagctg gccggctccc tcggcggcga ctgcgagacc 43320
tggcaggccg agggggacgt cgccgcccgc tacaccgagt acgcccggcg gctgctggaa 43380
ctcctccagg aggaggcccg cagcccggcc ccggccccgg cccccgcccc tggcgggacc 43440
tccggcgggg ttcccggcgg gcggctcgtc cagctcgtca ccccgtcctc ggcaccctgg 43500
ctcggcggtc tgagcggcat ggtgcgcacc gcccgccagg agcaccccaa gctcctcgtc 43560
cagtggatcg aggccgaaga cgactgctcc gccgatgagt tggcggtgct gctgcgcggc 43620
gacggcgccg accccgccga ggtggcggta cggcacggcg acggacggcg cagggtctct 43680
cggtggcgcg agacgccgcc gcccgcgccc cgcgtcccct ggcgcgacgg cggggtctat 43740
ctcgtcaccg gcggctcggg cggcctcgcc gcgctgttcg ccaaggacat ggcccgtcgc 43800
gtgcggcggc cgtcgctggt cctctgcgga cgcggcgcgg ccggtcccga acagcgggag 43860
ctggtcgcgg agttggaggc gctgggcgcc cgcgcggagt accgcgtact ggacgtctcc 43920
gacgccgggg ccgtcagcgc ggcggtccgg gaggtggtcg ccgcacacgg cgccctgcac 43980
ggtgtcgtcc acgcggcggg cgtgctgcgg gacggcttcc tcgcgcgcaa gagcgccgag 44040
gagctgcggc aggtcttcgc ggggaaggtc gccgggctgc gccatctcga cgaggccacg 44100
gcggacgtgg agttggactt cctgatcgcc ttctcatcga tggccgcctt cggcaacgcc 44160
gggcaggccg actacgccgc cgccaacgcg ttcctcgacg gctacgccca gcaccgcgaa 44220

gcgctccgcg cgcgcggcga gcgccacggg cggaccctct cggtgaactg gccgctgtgg 44280
gagaagggcg gtatgcgcgg cggcgcgggc accgaggccg tgctccaggg cgtgggcatg 44340
cgcccgatgc gggcggagac ggggctcgac gccctgtacc gggcgtgggc gtgcggtctg 44400
acgtccgtcc tggtgctgga gggcgaccac gagcggatgc gctcccggct gctgccggag 44460
cagccccgc tgcccgagcc cccgcaccgg ccggacgcac agaagccgct ggacgctcag 44520
aagcccctgg acgcaccgga gttgccggac ggaccggaag cgacgaggac gggcggcggc 44580
gtgccggtgc gctccggctc cgcggacgtc gcccgccggg tcaccgccgt cctggcggac 44640
ctgctggaga tcgacgcgga gtccctgcgg cccgacgtgc cgctacgcga gtacggactc 44700
gactcgatct tcctcaccca gttcctcggc acggcccgca aggagttcga cccggcgctc 44760
acgctcgacg tcatagcggg ctgcgagacg ctgacggact tcatcgacgc gatcgagcgc 44820
gccgtcgccc cgccggccgc cacgcctccg gcgcccgcat ccgcgtccgc gccctcaccc 44880
tcgtccggta cggaagggga accgtcaacg cgccccgccc cggagccgga tggcgtaccg 44940
gtggtgcgtc ccgtcgccaa ggctcccgag gagttccccg agctgatccc catgaacgcc 45000
gtacgggagg gccgcccggt cttctgggtc caccacggca acggcggagt cgagtcgtac 45060
gcggcggtgg ccgagtgctg cggacgcccc ttctacggca tccagccccg cggctggacg 45120
ggctcggagg acatcctcac cggccaggag gccatggccg cctactacgt ggacatcatc 45180
cgcgccgtcc agccggaggg cccgtacgac gtcggcggct tctccctcgg cggtctgttc 45240
gcctacgagg tcgtacgcca actccagctc caggacgcca cggtggacac cctggtgatg 45300
ctggacaccc tcgacgccgc ctcgaccaac ctggccaact ccctgatgac gggcggccgt 45360
caggacgacg ccgacgtggt ggcgaaggtc agcgccttcc gcgccgtcaa cctgatgctg 45420
ggcaacgaca gcctggacgc gcgcgagggc acctcgtccg tcctgcaccg ggacgaggtg 45480
gacaccgcgc tcgacccgga cgccttcctg gactccctcg tggaggccgc cgtcgcccgc 45540
ggcatccaca agaccccggc ccaactgcgg acgcgcgtac ggcagttggc gcggtacttc 45600
gacgccgtgc acggcgagcg gcacgtggtg cacccgctgc cgcggcgcga ggaggtgcgc 45660
tgctactacc tgcgcaacgc gggcgggcag ttcttcggcc ccttcgagga gtacatggtc 45720
ctcttccccg aaccggacct cccggcggtg gacggcaccc cgtactggcg cgaatgggcc 45780
gacgccgtcg acgacttctt cgtcatcgac gtcgacacgg agacccacgc gcaggtgatg 45840
acggagcccg cggcgctgaa gaaggtgctg cggctgtgcg accggctgta cgcgccggag 45900
cagcacgcgc agggaggccg gtgagatgga ggcggtcgtc tttcccgggc agggcgcgca 45960
gcgccgcggc atgggccgcg agctgttcga cgcctttccc tcgctcaccg agcaggcgtc 46020
cgacgtcctc gggtactccg tacgcgagtt gtgcgtggcg gaccccgagc gccggctgcg 46080

cagcaccgag tacacccagc ccgcgctgtt cgtcgtcggc accctcgcgc acctcaagtg 46140
gcaggaggag acggggcgtt cggccgcgta cttcgccggg cacagcgtgg gggagtacac 46200
cgcgctgcac gccgcgggcg ccttcggctt cgagaccggg ctgcgcctgg tgcagcggcg 46260
cggtctcctc atgtcgcagg cggagggcgg cggcatggcg gccgtactcg gcctcggcgc 46320
cggggagttg accgagctgc tccgcgaggg cggcttcgtc tccctcgccc tcgccaacga 46380
caacacgccc gatcagcagg tcgtctcggg cgccgcgcac gagatcgacg tcctggaggc 46440
gtatctgtcc gaacgcggcg tgcgcggtgt gcggctgaac gtctcgggcg ccttccactc 46500
gccgctgatg ctgcccgcac aggaggcgtt cgccgcgtac gtacgggact tcacgctcgg 46560
cgacccggag acgccggtga tctccaacgt caccgcgcgg ccccatccgc cgggcggtac 46620
ggccgagttg ctcgtacggc agatctccag ccccgtgcgg tggacggaga gcgtgcgcca 46680
tctgctcgac ctcggcgtgg aggagttcac cgaactcggc ggcagcgtgg tcgcgaagct 46740
cgtacggcag atccgcgagg cgcaccgcag ggacgcggac gcctccggcc cggcgcccgc 46800
cgcgcccgcg gctcccgtac ggtccgaacc cgccgcacgg tccgcgctcg gcagcgcggt 46860
cttccgcgag cggctggggc tgcgccactc ctacgcggcg ggcggcatgt accggggcat 46920
cgcctccccg gccatggtgg tgcgcctcgc ccgcgccggg atgctcggct tcctcggcac 46980
gggcgggctg acgcccgagg ccgtcgagga gcggatcctc caggtccggc gggagctgcg 47040
cgagggcgag cccttcggcg tcaacttcct cgccgaccac gacgatccgg ccgccgaacg 47100
ccgcgtcgcc gagatgctga tgcgccgcgg cgtgaccgtc gtcgaggcgg cggccttcat 47160
cggcatgacc ccggcctcgt cctctaccgc gcgcgcgggc atgcaccgcg gaccggacgg 47220
ggccccgcgc tgcgcccacc gcatcgtcgc caaggtctcc cgcccccgagg tgggccggcg 47280
ccttcatggc accgcgcccg ggaaggtggt cgacggcctg ctccgggagg gcgcgatcac 47340
cggcgagcag gcggagctcg tacggcaggt cccgatgagc cacgacatca ccgtcgaggc 47400
ggactccggc ggccacaccg acgggggcat cgccaccgtc atgctgcccg cgatgctcgg 47460
cctccggcgg caggcccagc gcagccacga ctacgcggag ccgctgtgca tggggctcgc 47520
cggcggcctc ggcactccgg aggccgtcgc cgcggccttc atgctgggcg ccgactacgt 47580
cctgacgggc tccgtcaacc agtgcaccgt cgaggcggac accagcgacg ccgtcaagga 47640
catgctccag accatcgaca tccaggacac gggctacgcg cccgcgggcg acatgttcga 47700
gatgggagcc cgggtccaag tgctgcgcaa gggcgtcttc ttcccgaccc gggccaacaa 47760
gctgtacgcg ctctaccagc accacgacgg cctcgacgac ctgcccgcga agacccgtgc 47820
cctgctggag aggtcgtact tccaccgcac cttcgacgag atctggacgg aggtacgcga 47880

gcactaccgc gccaagggcc agcccgaggt caccgacaag gcggagcggc agccgaaggt 47940
gaagatggcg ctggtcttcc gctggtactt cgcctactcg gcccggctgg cactggccgg 48000
gcaggacggc gacaaggtca acttccagat ccacacgggg cccgcgctcg gcgccttcaa 48060
ccagtgggtc aagggcacgg cctgcgagtc ctggcgcgcc cggcatgccg acgccatcgg 48120
gctgatgctc atggagggcg cggcagaaca cgtcgcggcg gcctgcgagt cgtggggcgg 48180
tacctcccgc ttcgcccccg cggaggaacg cgccctggcc gcccgcacct gaccccgcac 48240
ctgaccccgt accggcctcg acggcggaag ccggctgccg caccggaccg gaccgtccgg 48300
accacgtacc ccggccacgt accacggccc acggcccacg gcccacggcg tgaagtccgc 48360
gtcggcgcag gccggttcac gttcacgacg gcccccgcac acacaccacc accgcaccgg 48420
ataccgcgcc gcacggcgcc atgagaggac acgcagtgac cacccacctc accaccgaca 48480
tcgacgagat cgtcacggac gtcttccagt ccaccgaggg gaagaagaac ccctaccccc 48540
tgtaccggcg cctccaggag ctgggccagg tgcaccgctc cgagcaactg ggctgggtcg 48600
ccacgggata cgaggtgtgc agcgccgcgc tgcgcgaccc ccgcgtcatc aagggccccg 48660
agcagatcca gccgggccgc cccgaccccg ccgagcactc cgccgaggcg ttgctgcgcg 48720
gcacgatgca ccggctcgac ccgccggacc acacccggct gcgccgcctc gtcaacggcg 48780
ccttcacgcc gcgcagcgtc gccgcgctgg agccggacat ccaggaactg atcgacgacc 48840
tgatcacacc ggccgtcaag aaggcggagg cgggcgagcc cgtcgacatg atgagcggct 48900
tcgccttccc gctctccgtc gccgtgatcg gccgcatgct cggcgtgccc gcgtccgact 48960
ggcaccgctt ccacgacgtc gtgctcgacc tgtcctccat ggtcgaactc ggcttcaccg 49020
gcgacgagtt gcccaaggcc gacgccgccg cggacgaact gatcgcctac ttccgcaagt 49080
tgggcgccga gcgcatgcgc aacccgccg acgacctgac ctccacgctc gccaacgcga 49140
ccgaggccgg tgaccgcctc accgagcagg aactcgtcac catgctgatc ctgctgttca 49200
tggccggttt cgagacgacg acccactcca tgggcaacgg catgttcgcc ctgctggaga 49260
acccggagca gacgcagtgg ctgcgccgca acatggacgc catgcccgcg gccgtcgagg 49320
agctgatccg ctacgactcc ccggtgcagt tcatcgccgg ctacaccaag gagcccgtgg 49380
aactggcgga cggcaccgcc gtccccgcgg acgagtatct gttcctgatg atcggcgcgg 49440
ccaaccgcga cccgcgcgtc ttctccgacc ccgaactgct gcgtctggac cgcggtgagg 49500
ccgcgccgat gagcttcggc ggcggcatcc actactgcct cggcgcgggc ctggcccggc 49560
tggagatccg gaagatcttc acctcgctgc tcacccgctt ctccgcgatc gaactggccg 49620
agcccgaacc ggaacgccgc agcggactcg ccctgcgcgg ctacgcccgc atcccgatgt 49680
ggctcacccc ggcgtaaccc gccccgcccg tgaccgtacg aaggctcccc gccccgttcc 49740

gcctctcccg cggaacgggg cggacccgta cgggaccggc aggcggaagg tgagcgggct 49800
cagcggagcg ccgcgggccc cgtctccgtc cccgtctccg tctccgactt ggaccccgcc 49860
cctgctcccg tctcggtcct gctccccaag aggacggcgg tggccagcag cccccgccgt 49920
gccgcccagc ggccggtgaa accgcgcaac cgctcgccgc cgagcaccgg cccctccacg 49980
aggagccgcg cggagaacgt gcccgcgtcc gcgtcgatgg tgatctcggc ctcggagaag 50040
tcgagttcgc gtgaggtgag cggataccag accttgaaga cgctctcctt cgcgctgaac 50100
agcaggcggt cccaggccac ttggggatgc gccgccgcca gcgtgcgcag gtgttcccgc 50160
tcggcgggca gggagatcac gcccagaacg ccgtccggcg tcggctctgc gggttcggcg 50220
tcgatgccga tgccgaccgc gtcgctgccg cgggcgacgg ccgccgcccg gtagccgtcg 50280
cagtgcgtca tgctgccgac gacaccctcc ggccactgcg gggcgccccg gtgccccggc 50340
accagcggca cgggcgccac acccagccgc cccagtgcac tccgcgcaca cgcccgtacg 50400
tccgcgaact cccgctgccg cttgggcacc gcccgggcca cggcggcccg ctcctcgggg 50460
aagagcccac tcagcgtgtc ctcctgccca ccgacgaaga tctcctcggt gctcacccac 50520
cccggcaaca tccgcccgat cac 50543

<210> 26
<211> 565
<212> PRT
<213> Streptomyces amphibiosporus

<400> 26

Val Leu Phe Pro Gly Gln Gly Ser Gln Ser Lys Gly Met Gly Arg Glu
1               5                   10                  15

Leu Phe Asp Arg Phe Pro Glu Thr Thr Ala Ser Ala Cys Asp Val Leu
            20                  25                  30

Gly Tyr Asp Leu Arg Glu Leu Cys Leu Glu Asn Pro Glu Gly Arg Leu
        35                  40                  45

Asp Asp Thr Arg Tyr Thr Gln Ser Ala Leu Tyr Thr Val Asn Ala Leu
    50                  55                  60

Glu Tyr Leu Gly Ser Leu Glu Asp Gly Ala Pro Glu Gly Asp Tyr Leu
65                  70                  75                  80

Leu Gly His Ser Leu Gly Glu Tyr Asn Ala Leu Leu Ala Ala Gly Val
                85                  90                  95

Phe Asp Phe Glu Thr Gly Leu Arg Leu Val Leu Lys Arg Gly Glu Leu
            100                 105                 110

Met Ala Arg Ala Arg His Gly Gly Met Leu Ala Val Leu Gly Pro Gly
        115                 120                 125

Glu Glu Glu Leu Arg Arg Thr Leu Ala Glu Glu Gly Met Glu Arg Leu
130 135 140

Asp Val Ala Asn Val Asn Thr Pro Ala Gln Thr Val Leu Ser Gly Pro
145 150 155 160

Val Glu Glu Ile Glu Arg Ala Gln Arg His Phe Asp Glu Arg Arg Val
165 170 175

Arg Thr Ala Arg Leu Lys Val Ser Ala Ala Phe His Ser Arg Leu Met
180 185 190

Arg Pro Ala Arg Glu Glu Phe Arg Ala Phe Leu Arg Gly Phe Arg Phe
195 200 205

Ala Ser Pro Arg Ala Thr Val Ile Ala Asn Val Ser Ala Arg Pro Tyr
210 215 220

Gly Asp Val Ala Glu Met Leu Ser Glu Gln Ile Ala Gly Pro Val Arg
225 230 235 240

Trp Leu Glu Ser Val Arg Tyr Val Leu Glu Arg Thr Ser Ala Glu Arg
245 250 255

Gly Arg Glu Ala Gly Pro Gly Thr Val Leu Thr Arg Met Leu Arg Gln
260 265 270

Ile Asp Gly Val Ser Gly Ala Gly Asn Ser Pro Ser Val Ser Ala Ser
275 280 285

Gly Ser Val Pro Ala Ala Ser Ala Pro Ala Glu Ser Pro Ser Ala Pro
290 295 300

Ala Ala Ser Thr Ser Gly Ser Ala Arg Pro Val Gly Arg Ala Thr Ala
305 310 315 320

Ala Thr Ala Asp Ala Val Arg Glu Pro Thr Arg Pro Leu Leu Ile Cys
325 330 335

Ala Pro Tyr Ala Gly Gly Asp Glu Arg Ser Tyr Ala Gly Leu Ala Glu
340 345 350

Gln Leu Pro Glu Ala Asp Val Val Thr Leu Glu Arg Pro Gly Arg Gly
355 360 365

Arg Arg Val Ser Glu Pro Leu Leu Thr Glu Pro Gly Pro Val Val Glu
370 375 380

Asp Met Leu Ser Arg Ile Arg Asp Arg Val Ser Arg Pro Tyr Ala Leu
385 390 395 400

Tyr Gly His Ser Leu Gly Ala Arg Leu Val His Leu Leu Ala Arg Arg
405 410 415

Leu Arg Glu Glu Gly Leu Pro Gly Pro Arg His Leu Phe Val Ser Gly
420 425 430

Glu Cys Gly Pro Ser Arg Pro Ser Arg Glu Arg Tyr Thr Ser Asp Leu
435 440 445

Pro Thr Asp Ala Phe Trp Lys His Leu Arg Glu Leu Gly Gly Val Pro

450 455 460

Asp Glu Leu Phe Glu Tyr Glu Asp Leu Thr Thr Phe Tyr Glu Arg Val
465 470 475 480

Leu Arg Ala Asp Phe Thr Val Leu Gly Ala Cys Ala Tyr Thr Pro Ala
485 490 495

Ala Pro Leu Asp Cys Pro Val Thr Ala Met Thr Gly Asp Glu Glu Gly
500 505 510

Leu Thr Glu Ala Asp Val Gly Ala Trp Gln Arg Glu Thr Thr Ala Pro
515 520 525

Leu Thr Ala Arg Val Phe Thr Gly Asp His Phe Phe Ile Arg Ala His
530 535 540

Trp Pro Gly Val Ala Arg Val Val Ala Ala Gly Leu Gly Ala Arg Arg
545 550 555 560

Pro Ala Gly Thr Arg
565

<210> 27
<211> 1698
<212> DNA
<213> Streptomyces amphibiosporus

<400> 27

```
gtgctcttcc cgggacaggg ctctcagtcg aagggaatgg gaagagaact cttcgaccgt     60
tttcccgaga ccaccgcgtc ggcctgcgac gtcctcggat acgacctgcg cgagctgtgc    120
ctggagaacc cggagggccg gctcgacgac acccggtaca cccagtccgc cctctacacc    180
gtcaacgccc ttgagtatct ggggtcgttg gaggacgggg cgccggaggg cgactacctg    240
ctggggcaca gcctcggcga gtacaacgcg ctgctcgcgg cgggcgtctt cgacttcgag    300
accgggctgc ggctcgtcct caagcgcggt gagctgatgg cgcgggcgcg ccacggcggg    360
atgctggccg tactggggcc cggcgaggag gagttgcgcc ggacgctggc cgaggagggc    420
atggagcgtc tcgacgtcgc caacgtcaac acccccgcgc agaccgtgct ttcggggccg    480
gtggaggaga tcgagcgcgc ccagcggcac ttcgacgagc gccgggtgcg tacggcgcgg    540
ctgaaggtct ccgccgcctt ccactcacgg ctgatgagac ccgcgcggga ggaattccgt    600
gcgtttctcc ggggattccg cttcgcgtcg ccgcgtgcca cggtgatcgc caatgtgtcg    660
gcacggccct acggcgatgt cgcggagatg ctgagcgagc agatcgccgg gccggtgcgg    720
tggctggaga gcgtccggta cgtgctggag cggacctccg ccgagcgcgg cagggaggcg    780
ggacccggga ccgtactgac gcggatgctg cggcagatcg acggtgtgtc cggggcgggg    840
aattccccct ccgtctctgc gtccggctcc gtgcccgctg cctccgcccc ggccgagtct    900
ccgtccgctc ccgcagcgtc aacgtccggc agcgcgcggc ccgttgggcg cgccaccgcc    960

gccaccgccg atgccgtacg ggagcccacc cggcccctgc tgatctgcgc gccctacgcg   1020
ggcggcgacg agcgctccta cgcggggctc gccgagcaac tgcccgaggc ggacgtcgtc   1080
accctggagc ggccgggccg cgggcggcgg gtctccgagc cgctgctgac cgaaccgggg   1140
cccgtcgtcg aggacatgct gtcccggata cgggaccggg tgagccggcc gtacgcgctc   1200
tacgggcaca gcctcggcgc gcggctcgtc catctcctcg cccgccggct gcgcgaggag   1260
ggcctgcccg gcccccgcca tctgttcgtc tccggcgagt gcggcccctc gcggcccagc   1320
cgggagcgct acaccagcga tctgccgacc gacgccttct ggaagcacct gagagaactc   1380
ggcggcgtgc cggacgagtt gttcgagtac gaggacctca ccacgttcta cgagcgcgtt   1440
ctgcgcgccg acttcaccgt cctcggggcc tgcgcgtaca cccccgccgc accccctggac   1500
tgccccgtca ccgccatgac cggcgacgag gagggcctga ccgaggccga cgtcggggcc   1560
tggcagcggg agaccaccgc gccgctcacg gcccgggtct tcaccggaga ccacttcttc   1620
atccgggcgc actggcccgg cgtcgcacgc gtcgtcgccg ccgggctggg cgcccgccga   1680
cccgcaggga cccgctga                                                 1698
```

<210> 28
<211> 84

<212> PRT
<213> Streptomyces amphibiosporus

<400> 28

```
Met Glu Gln Glu Leu Lys Gln Tyr Met Glu Glu Gln Phe Met Phe Glu
1               5                   10                  15

Phe Asp Ser Glu Ile Thr Glu Asp Thr Asp Leu Phe Lys Ala Gly Val
            20                  25                  30

Leu Asp Ser Phe Gly Tyr Ile Ser Leu Ile Gly His Ile Glu Gly Glu
        35                  40                  45

Tyr Gly Val Lys Phe Gly Glu Glu Ala Leu Leu Gly Asn Val Ala Val
    50                  55                  60

Thr Phe Ala Gly Leu Val Glu Ser Val Ala Ser Ala Arg Arg Gln Thr
65                  70                  75                  80

Ala Glu Ser Lys
```

<210> 29
<211> 255
<212> DNA
<213> Streptomyces amphibiosporus

<400> 29

```
atggagcagg aactcaagca gtacatggaa gagcagttca tgttcgagtt cgattcggag     60

atcaccgagg acaccgacct gttcaaggcg ggcgtgctcg actcgttcgg ctacatctcg    120

ctcatcgggc acatcgaggg ggagtacggc gtcaagttcg gcgaggaggc gctgctcggc    180

aacgtcgccg tcaccttcgc cggcctcgtc gagtccgtgg cgtccgcccg tcggcagacc    240

gccgagagca agtaa                                                      255
```

<210> 30
<211> 656
<212> PRT
<213> Streptomyces amphibiosporus

<400> 30

```
Val Cys Gly Ile Ala Gly Phe His Ala Ser Pro Leu His Pro Glu Ser
1               5                   10                  15

Tyr Arg Asp Ile Ala Gly Ala Met Leu Ala Gln Ile Glu His Arg Gly
            20                  25                  30

Pro Asp Glu Ala Gly Cys Phe Leu Asp Asp Arg Thr Ala Met Gly Thr
        35                  40                  45

Val Arg Leu Ser Ile Ile Asp Leu Ala Ser Gly Ser Gln Pro Val Gly
    50                  55                  60

Ser Pro Asp Gly Arg Tyr Trp Leu Cys Tyr Asn Gly Glu Leu Tyr Asn
65                  70                  75                  80

Tyr Arg Glu Leu Arg Ala Glu Leu Ala Gly Arg Gly Val Ser Phe Arg
                85                  90                  95

Thr Glu Ser Asp Thr Glu Val Val Leu Met Ala Trp Ala His Trp Gly
            100                 105                 110

Arg Ser Cys Leu Glu Arg Phe Asn Gly Ala Phe Ala Phe Ala Leu Lys
        115                 120                 125

Asp Thr Val Thr Gly Glu Leu His Leu Ala Arg Asp Arg Phe Gly Lys
    130                 135                 140

Arg Pro Leu Tyr Val Ala Arg His Gly Asp Ala Trp Leu Phe Ala Ser
145                 150                 155                 160

Glu Met Lys Ala Phe Leu Ala Tyr Pro Gly Phe Glu Phe Ala Phe Asp
                165                 170                 175

Glu Glu His Leu Ala Ser Thr Phe Ala Thr Trp Thr Pro Leu Pro Ala
            180                 185                 190

Gln Ser Gly Tyr Arg Gly Val Glu Gln Leu Pro Met Gly Glu Tyr Leu
        195                 200                 205

Thr Val Arg Gly Thr Glu Thr Glu Arg Gly Arg Trp Ala Ser Leu Asp
    210                 215                 220

Leu Thr Gly Gly Glu Pro Pro Ala Thr Glu Asp Glu Ala Val Asp Leu
225                 230                 235                 240

Val Arg Ala Asp Leu Glu Ala Ala Val Asp Leu Arg Leu Arg Ser Asp
```

245 250 255

Val Glu Val Gly Val Tyr Ala Ser Gly Gly Leu Asp Ser Ser Ile Leu
260 265 270

Ala His Leu Thr Lys Glu Arg Ala Gly Leu Pro Pro Arg Thr Phe Ser
275 280 285

Ile Gln Phe Glu Asp Ala Glu Phe Asp Glu Thr Ala Glu Gln Glu Glu
290 295 300

Leu Thr Lys His Leu Gly Thr His His Ser Thr Val Arg Val Ser Asp
305 310 315 320

Ser Asp Val Val Glu Thr Phe Pro Glu Ala Val Arg His Ala Glu Val
325 330 335

Pro Val Phe Arg Thr Ala Phe Val Pro Met Tyr Leu Leu Ala Gln His
340 345 350

Val Arg Ser Glu Gly Val Lys Val Val Leu Ser Gly Glu Gly Ala Asp
355 360 365

Glu Ala Phe Leu Gly Tyr Gly Ile Phe Lys Asp Ala Arg Leu Leu Ser
370 375 380

Glu Trp His Glu Leu Asp Glu Ala Thr Arg Met Arg Arg Met Ala Gln
385 390 395 400

Leu Tyr Pro Tyr Leu Arg His Phe Ser Gly Glu Asp Gly His Arg Arg
405 410 415

Met Leu Gly Leu Tyr Arg Gln Phe Thr Glu Glu Thr Met Pro Gly Leu
420 425 430

Phe Ser His Gln Met Arg Phe Gln Asn Gly Arg Phe Ala Val Arg Leu
435 440 445

Leu Lys Asp Ala Gly Asp Pro Phe Ala Ala Val Arg Arg Leu Val Ala
450 455 460

Glu Glu Pro Gly Tyr Ala Glu Leu Ser Ala Val Gln Lys Ala Gln Trp
465 470 475 480

Leu Glu Phe Arg Thr Leu Leu Ser Gly Tyr Leu Leu Ala Thr Gln Gly
485 490 495

Glu Arg Met Ala Leu Ala His Gly Val Glu Asn Arg Cys Pro Phe Leu
500 505 510

Asp Pro Ala Val Val Arg Arg Ala Ala Ser Val Asn Gly Arg Phe Gly
515 520 525

Asp Pro Tyr Asp Glu Lys Tyr Leu Leu Lys Arg Ala Tyr Gly Asp Val
530 535 540

Leu Pro Glu Arg Ile Val Ser Lys Gly Lys Phe Pro Tyr Arg Ala Pro
545 550 555 560

Asp Ser Ala Ala Phe Val Arg Ser Arg Pro Asp Tyr Arg Asp Leu Leu
565 570 575

Ala Asp Pro Gly Thr Leu Gly Asp Ile Gly Val Leu Asp Glu Arg Phe
580 585 590

Val Arg Arg Phe Thr Asp Arg Val Phe Asp Arg Pro Pro Glu Arg Ile
595 600 605

Gly Thr Lys Glu Asn Gln Ala Phe Val Leu Leu Ala Ser Thr Val Trp
610 615 620

Leu His His Trp Tyr Val Arg Gly Asn Ala Arg Arg Asp Thr Pro Leu
625 630 635 640

Ala Val Pro Leu Tyr Val Val Asp Arg Arg Ser Ser Ala Leu Pro Ala
645 650 655

<210> 31
<211> 1971
<212> DNA
<213> Streptomyces amphibiosporus

<400> 31

```
gtgtgcggca tagcgggctt ccacgcgagc cccctgcacc cggagagcta ccgggacatc      60
gccggtgcca tgctcgcgca gatcgagcac cggggccccg acgaggcggg ctgcttcctg     120
gacgaccgta cggccatggg cacggtgcgg ctgagcatca tcgacctcgc ctccggctcg     180
cagcccgtcg gcagccccga cggccggtac tggctctgct acaacggcga gctgtacaac     240
taccgggaac tgcgcgccga gttggcgggc cgcggggtgt ccttccgtac ggagtccgac     300
accgaggtcg tcctgatggc ctgggcgcac tgggggcgct cgtgcctcga acgcttcaac     360
ggtgccttcg cgttcgccct gaaggacacc gtcaccggtg aactgcacct ggcccgcgac     420
cggttcggca agcggccgct gtatgtggcg cggcacggcg acgcatggct gttcgcctcc     480
gagatgaagg ccttcctggc ctaccccggc ttcgagttcg ccttcgacga agagcatctc     540
gcctcgacgt tcgccacctg gacgccgctg cccgcgcaga gcggataccg cggcgtcgaa     600
cagctcccca tgggcgagta tctgacggtc cgcgggacgg agaccgaacg cggccgctgg     660
gcgtcgctcg acctgaccgg cggcgagccg cccgccaccg aggacgaggc cgtcgacctc     720
gtgcgcgccg atctggaggc cgccgtcgac ctgcggctgc gcagcgacgt cgaggtcggc     780
gtctacgcct ccggcggtct ggactcctcg atcctcgccc atctcaccaa ggagcgggcc     840
gggctgccgc cgcgtacgtt ctccatccag ttcgaggacg ccgagttcga cgagaccgcc     900
gagcaggagg agctgaccaa gcacctcggg acgcaccact ccaccgtccg cgtctccgac     960
tccgacgtcg tggagacctt ccccgaggcc gtacgccacg ccgaagtccc cgtcttccgc    1020
acggcgttcg tgccgatgta cctgctggcg cagcatgtgc gcagcgaagg cgtcaaggtc    1080
gtgctcagcg gcgagggcgc cgacgaggca ttcctcggct acggcatctt caaggacgcc    1140
cggctgctct ccgagtggca cgagctggac gaggcgaccc gcatgcggcg catggcgcag    1200
```

ctctacccgt atctgcgcca cttcagcggc gaggacgggc accgccggat gctgggcctc 1260

taccggcagt tcacggagga gaccatgccc ggtctcttct cccaccagat gcggttccag 1320

aacggccggt tcgccgtgcg gctgctcaag gacgcgggcg acccccttcgc cgcggtacgg 1380

cggctcgtcg cggaggagcc cggatacgcg gagctgtccg cggtgcagaa ggcacagtgg 1440

ctggagttcc gtacgctgct cagcggctat ctgctcgcca cccagggcga gcggatggcg 1500

ctcgcgcacg gcgtggagaa ccgctgcccg ttcctcgacc cggcggtggt gcgccgcgcg 1560

gcgtccgtca acggccgctt cggcgacccg tacgacgaga agtacctgct caagcgcgcg 1620

tacggggacg tgctgcccga acgcatcgtc agcaagggca agttccccta ccgggcgccg 1680

gacagcgccg cgttcgtacg gtcccgtccc gactaccgcg acctgctggc cgaccccggc 1740

accctcggcg acatcggcgt gctcgacgag cgcttcgtgc ggcgcttcac cgaccgggtc 1800

ttcgacaggc cgcccgagcg gatcggcacc aaggagaacc aggcgttcgt cctgctggcg 1860

tccacggtct ggctgcacca ctggtacgtg cgcggcaacg cccgccgcga caccccccctc 1920

gctgtccccc tgtacgtcgt cgaccggcgc agcagcgcgc tgccggccta g 1971

<210> 32
<211> 3436
<212> PRT
<213> Streptomyces amphibiosporus

<400> 32

Met Lys Glu Glu Ser Gly Ala Leu Pro Glu Glu Gly Pro Val Gly Thr
1 5 10 15

Ala Val Gly Thr Ala Ala Asp Gly Ala Ala Gly Gly Pro Val Asp Gly
20 25 30

Gln Asp Ile Ala Val Val Gly Leu Ser Leu Arg Leu Pro Gly Ala Arg
35 40 45

Asn Pro Glu Glu Phe Trp Glu His Leu Ala Ala Gly Arg Ser Leu Ile
50 55 60

Ser Glu Val Pro Glu Arg Arg Trp Arg Lys Glu Asp His Leu Gly Asn
65 70 75 80

Pro Arg Arg Glu Phe Asn Lys Thr Asn Ser Val Trp Gly Gly Phe Val
85 90 95

Asp Asp Ala Asp Cys Phe Asp Ala Glu Phe Phe His Val Ser Pro Arg
100 105 110

Glu Ala Arg Ser Met Asp Pro Gln Gln Arg Met Ala Leu Glu Met Ser
115 120 125

Trp Gln Ala Leu Glu Asp Ala Gly Tyr Arg Ala Asp Arg Val Ala Gly
130 135 140

Ser Arg Thr Gly Val Phe Met Gly Val Cys His Trp Asp Tyr Ala Glu
145 150 155 160

Leu Ile Glu Lys Glu Val Ser Glu Val Asp Ala Tyr Tyr Pro Thr Gly
165 170 175

Ala Ala Tyr Ala Ile Ile Ala Asn Arg Val Ser His His Phe Asp Phe
180 185 190

Arg Gly Pro Ser Val Val Asn Asp Thr Ala Cys Ala Ser Ser Leu Val
195 200 205

Ala Val Gln Gln Ala Val Gln Ala Leu Gln Ser Gly Asp Cys Asp His
210 215 220

Ala Leu Ala Gly Gly Val Asn Leu Thr Trp Ser Pro Arg His Phe Ile
225 230 235 240

Ala Phe Ala Lys Ala Gly Met Leu Ser Pro Asp Gly Leu Cys Arg Ala
245 250 255

Phe Asp Ala Asp Ala Asn Gly Tyr Val Arg Gly Glu Gly Gly Gly Val
260 265 270

Val Leu Leu Lys Arg Ala Ala Asp Ala Arg Arg Asp Gly Asp Pro Val
275 280 285

His Ala Val Ile Lys Gly Ile Gly Ser Asn His Gly Gly Arg Thr Ser
290 295 300

Ser Leu Thr Val Thr Asn Pro Ala Ala Gln Ala Glu Leu Ile Ala Gly
305 310 315 320

Ile Tyr Arg Arg Ala Gly Ile Ala Pro Glu Ser Val Ser Tyr Ile Glu
325 330 335

Thr His Gly Pro Gly Thr Pro Val Gly Asp Pro Ile Glu Val Ser Gly
340 345 350

Leu Lys Arg Ala Phe Ala Gln Leu Gly Glu Gly Arg Glu Ala Glu Pro
355 360 365

Ser Gly His Arg Cys Gly Ile Gly Ser Val Lys Thr Asn Ile Gly His
370 375 380

Leu Glu Gly Ala Ala Gly Ile Ala Gly Met Leu Lys Val Ile Leu Ala
385 390 395 400

Met Arg His Arg Lys Leu Pro Ala Thr Val Asn Phe Arg Arg Leu Asn
405 410 415

Pro Leu Ile Thr Leu Asp Gly Ser Pro Leu Tyr Val Val Asp Arg Leu
420 425 430

Thr Asp Trp Glu Thr Asp Gly Asp Gly Thr Leu Arg Ala Gly Val Ser
435 440 445

Ser Phe Gly Phe Gly Gly Thr Asn Ala His Val Val Leu Glu Ala Pro
450 455 460

Gly Gly His Ala Ala Glu Val Thr Asp Ala Glu Ala Val Thr Asp Ala
465 470 475 480

Glu Ala Asp Ala Asp Val Asp Gly Gly Pro Asp Glu Gly Pro Asp Glu
485 490 495

Gly Ala Glu Pro Arg Ala Leu Arg Leu Pro Val Ser Ala Asp Asp Glu
500 505 510

Glu Arg Leu Arg Glu Leu Cys Arg Ser Leu Ala Glu Trp Ala Arg Ala
515 520 525

Arg Glu Ala Glu Gly Thr Ala Pro Pro Leu Ala Asp Ile Ala Arg Thr
530 535 540

Leu Arg Glu Gly Arg Val Pro Met Arg Glu Arg Ala Val Phe Arg Ala
545 550 555 560

Arg Ser Val Ala Glu Trp Ala Glu Gln Leu Thr Ala Leu Ala Glu Gly
565 570 575

Thr Gly Gly Glu Pro Pro Ala Gly Cys Leu Arg Gly Arg Ala Glu Asp
580 585 590

Gly Ala Gly Asp Gly Leu Asp Ala Asp Asp Val Ala Ala Leu Thr Ala
595 600 605

Arg Trp Arg Glu Arg Asp Glu Glu Glu Lys Phe Ala Ala Ala Trp Thr
610 615 620

Arg Gly Leu Pro Val Asp Trp Ala Gln Trp Pro Ala Glu Gly Arg Arg
625 630 635 640

Val His Leu Pro Gly Gln Val Phe Gln Arg Thr Pro His Trp Phe Arg
645 650 655

Pro Asp Glu Gln Pro Arg Gly Glu Ala Glu Ser Ala Gly Gly Ala Ala
660 665 670

Ala Gln Arg Asp Thr Ala Pro Glu Arg Asp Ala Ala Ser Gly Ser Glu
675 680 685

Arg Gly Pro Gly Ala Pro Glu Pro Ala Gly Pro Val Gly Gly Pro Gly
690 695 700

Leu Pro Gly Glu Gly Val Gln Asp Gly Arg Gly Trp His Phe Pro Leu
705 710 715 720

Arg Phe Ala Ala Thr Asp Pro Phe Val Arg Asp His Leu Val Leu Gly
725 730 735

Ala Arg Ile Val Pro Gly Val Val Ala Leu Glu Ala Val Thr Ala Ala
740 745 750

Ala Ala Arg Pro Ala Val Ala Gly Ala Arg Ala Gly Ala Ala Pro His
755 760 765

Ile Arg Asn Ala Val Trp Val Arg Pro Leu Arg Val Asp Gly Gln Val
770 775 780

Leu Glu Thr Ser Leu Arg Leu Thr Pro Ala Gly Pro Glu Ser Gly Gly

785 790 795 800

Gly Tyr Asp Trp Ala Val Thr Asp Ala Ala Gly Thr Pro Tyr Ser Ser
805 810 815

Gly Arg Val Glu Tyr Ala Asp Gly Pro Ala Pro Ala Ala Thr Asp Leu
820 825 830

Asp Ala Leu His Arg Arg His Thr Arg Pro Val Glu Val Ala Gly Gly
835 840 845

Tyr Ala Ala Leu Tyr Ala Ser Gly Ile Glu His Gly Pro Ala Leu Arg
850 855 860

Ala Leu His Thr Leu Arg Ala Gly Pro Glu Gly Leu Leu Ala Glu Leu
865 870 875 880

Arg Leu Pro Ala Glu Pro Ala Ala Gly Ala Ala Leu Gln Pro Ala Val
885 890 895

Leu Asp Ser Ala Leu Leu Ala Val Leu Ala Leu Gly Thr Gly Gly Gly
900 905 910

Asp Gly Thr Glu Gly Thr Gly Gly Thr Asp Gly Ala Gly Trp Arg Arg
915 920 925

Pro Asp Ala Pro Ala Val Pro Phe Ala Leu Asp Gly Leu Thr Ala Tyr
930 935 940

Ala Pro Thr Thr Ala Thr Thr Trp Ala Trp Leu Arg Pro Ala Gly Gly
945 950 955 960

Arg Arg Pro Gly Ala Ala Asp Ile Asp Leu Phe Asp Glu Arg Gly Arg
965 970 975

Leu Cys Ala Arg Leu Thr Gly Tyr Thr Ser Arg Glu Leu Ser Thr Gly
980 985 990

Ser Pro Ala Leu Arg Glu Ala Arg Val Ser Ala Pro Ala Pro Gly Glu
995 1000 1005

Gly Ala Ala Gly Glu Glu Ala Pro Gly Lys Asp Ala Pro Gly Glu
1010 1015 1020

Leu Leu Glu Val Thr Gly Arg Trp Thr Pro Ala Pro Leu Gly Leu
1025 1030 1035

Pro Ala Ala Glu Ala Gly Pro Ala Ala Ala Gln Ser Gly Ala Ala
1040 1045 1050

Ala Pro Val Thr Val Leu Asn Ala Ala Leu Asp Ala Asp Leu Val
1055 1060 1065

Ala Ala Ser Ala Ala Arg Leu Gly Met Asp Val Glu His Leu Ala
1070 1075 1080

Val Pro Arg Asp Ala Gly Asp Ala Asp Ala Met Lys Ala Ala Phe
1085 1090 1095

Ala Ala Cys Tyr Pro His Val Arg Arg Leu Val Gly Gln Ser Arg
1100 1105 1110

Arg Val Leu Leu Val Ala Pro Gly Ala Pro Asp Ser Pro Val Phe
1115 1120 1125

Ala Pro Leu Ala Ala Leu Leu Lys Thr Ala His Gln Glu Asn Pro
1130 1135 1140

Ser Phe His Gly Thr Thr Val Leu Leu Glu Gly Phe Asp Pro Arg
1145 1150 1155

Asp Ser Ala Arg Phe Glu Gln Val Val Arg Thr Glu Ala Ala Ala
1160 1165 1170

Thr Ala Asp Ala Ala Gly Gly Ala Ala Asp Glu Glu Val Ala His
1175 1180 1185

Thr Ala Asp Gly Arg Arg Leu Arg His Glu Thr Ala Glu Leu Pro
1190 1195 1200

His Gly Thr Thr Gly Glu Ser Leu Leu Ala Glu Gly Gly Val Tyr
1205 1210 1215

Trp Ile Thr Gly Gly Ala Gly Gly Ile Gly Leu Leu Leu Ala Glu
1220 1225 1230

Arg Leu Cys Leu Arg Tyr Gly Ala Thr Val Val Leu Ser Gly Arg
1235 1240 1245

Ser Pro Ala Ala Pro Ala Ala Asp Ala Leu Ala Ser Arg Leu Thr
1250 1255 1260

Arg Gly Thr Leu Ala Tyr Arg Gly Ala Asp Val Thr Asp Gln Asp
1265 1270 1275

Ser Val Asp Ala Leu Val Ala Ala Val Leu Ala Glu His Gly Arg
1280 1285 1290

Ile Asp Gly Val Phe His Ala Ala Gly Val Leu Asp Asp Gly Tyr
1295 1300 1305

Leu Thr Ala Lys Pro Leu Ala Gly Thr Glu Ala Val Leu Ala Pro
1310 1315 1320

Lys Val Asp Gly Val Thr Cys Val Asp Arg Ala Thr Arg Ala Gly
1325 1330 1335

Ala Pro Gly Phe Leu Leu Val Phe Gly Ser Val Ala Gly Ala Phe
1340 1345 1350

Gly Asn Ala Ala Gln Ala Gly Tyr Ala Ala Ala Asn Ala Tyr Leu
1355 1360 1365

Asp Ala Phe Ala Ala Arg Arg Gln Ala Ala Gly Leu Thr Thr Arg
1370 1375 1380

Ala Val Asp Trp Pro Leu Trp Ala Glu Gly Gly Met Arg Val Asp
1385 1390 1395

Asp Ala Ser Leu Lys Tyr Leu Arg Lys Arg Thr Gly Thr Val Pro
1400 1405 1410

Leu Pro Ser Gly Thr Gly Leu Asp Ala Leu Glu Arg Ala Leu His
1415 1420 1425

Thr Gly Ser Pro Val Arg Arg Val Val Leu Tyr Gly Asp Arg Pro
1430 1435 1440

Ala Leu Arg Val Tyr Ala Gly Leu Asp Arg Pro Gln Val Thr Gly
1445 1450 1455

Ala Arg Ser Gly Ser Ala Ser Ala Ser Ala Pro Gly Ser Ser Ser
1460 1465 1470

Gly Ser Val Ser Ala Val Arg Gly Glu Gly Thr Gly Thr Ala Pro
1475 1480 1485

Ala Ala Leu Thr Asp Ala Glu Leu Leu Val Arg Thr Gln Asp Phe
1490 1495 1500

Leu Arg Glu Gln Phe Ala Glu Val Thr Leu Gln Asp Ala Glu Gln
1505 1510 1515

Ile His Pro Glu Glu Lys Leu Glu Thr Tyr Gly Ile Glu Ser Ile
1520 1525 1530

Ser Ile Val Asp Leu Thr Ser Arg Leu Glu Asp Val Phe Gly Ser
1535 1540 1545

Leu Pro Lys Thr Leu Phe Phe Glu Tyr Val Asp Leu Lys Gly Val
1550 1555 1560

Ala Glu Tyr Phe Val Ala Glu His Arg Ala Arg Leu Thr Glu Leu
1565 1570 1575

Phe Ala Pro Glu Glu Pro Gln Ala Ser Glu Ala Ala Glu Pro Ala
1580 1585 1590

Pro Glu Glu Pro Val Ala Pro Ala Pro Val Pro Val Glu Pro Ala
1595 1600 1605

Ala Ala Ala Pro Ala Pro Ala Pro Ala Pro Val Pro Ala Pro Pro
1610 1615 1620

Ala Pro Thr Ala Ala Pro Gly Thr Ser Val Glu Ala Val Pro Ala
1625 1630 1635

Pro Val Pro Ala Ser Val Pro Thr Pro Arg Pro Ala Pro Ala Gly
1640 1645 1650

Asn Gly Asp Ile Ala Val Val Gly Met Ala Gly Arg Tyr Pro Gly
1655 1660 1665

Ala Asp Thr Leu Glu Glu Phe Trp Glu Leu Leu Ser Glu Gly Arg
1670 1675 1680

His Ser Phe Glu Pro Val Pro Ser Ser Arg Trp Pro His Gly Asp
1685 1690 1695

Leu Tyr Phe Asp Glu Arg Asp Val Leu Gly Lys Thr Thr Val Arg
1700 1705 1710

Thr Gly Thr Phe Leu Arg Asp Val Asp Ala Phe Asp Pro Arg Tyr

1715 1720 1725

Phe Ser Ile Ser Gln Arg Asp Ala Glu Leu Leu Ser Pro Glu Val
1730 1735 1740

Arg Leu Phe Leu Gln Ala Gly Val Thr Ala Leu Glu Asp Ala Gly
1745 1750 1755

Tyr Ser Lys Glu Thr Leu Arg Arg Arg Tyr Asp Gly Asp Val Gly
1760 1765 1770

Val Leu Val Gly Ser Met Asn Asn Ser Tyr Ala Tyr Tyr Gly Phe
1775 1780 1785

Glu Asn Met Leu Met Arg Gly Thr Ala Met Ser Gly Ser Glu Val
1790 1795 1800

Gly Val Met Ala Asn Met Leu Ser Tyr Tyr Tyr Gly Phe Thr Gly
1805 1810 1815

Pro Ser Met Phe Val Asp Thr Met Cys Ser Ser Ser Ser Ala Cys
1820 1825 1830

Val His Gln Ala Leu Ser Met Leu Arg Gly Gly Glu Cys Arg Met
1835 1840 1845

Val Val Val Gly Gly Ile Asn Leu Met Leu His Pro Tyr Asp Leu
1850 1855 1860

Ile Ala Thr Ser Gln Ala His Phe Thr Thr Lys Ser Ala Glu Val
1865 1870 1875

Val Arg Ser Tyr Gly Leu Gly Ala Asp Gly Thr Ile Leu Gly Glu
1880 1885 1890

Gly Val Gly Thr Leu Val Leu Lys Pro Leu Ala Glu Ala Val Ala
1895 1900 1905

Asp Gly Asp His Val Tyr Gly Val Ile Lys Gly Ser Gly Met Thr
1910 1915 1920

Asn Ala Gly Val Arg Asn Gly Phe Thr Val Pro Ser Pro Gln Gln
1925 1930 1935

Gln Ala Arg Ala Ile Glu Arg Ala Leu Asp Asp Ala Ala Val Asp
1940 1945 1950

Ala Arg Thr Val Ser Tyr Leu Glu Gly His Gly Ser Ala Thr Ser
1955 1960 1965

Leu Gly Asp Pro Ile Glu Ile Lys Gly Ala Ser Leu Ala Phe Gly
1970 1975 1980

Arg Asp Thr Arg Asp Val Gly Tyr Cys Ala Ile Gly Ser Val Lys
1985 1990 1995

Ser Asn Val Ala His Leu Leu Ser Gly Ser Gly Leu Val Gly Leu
2000 2005 2010

Thr Lys Val Leu Leu Gln Leu Arg His Arg Thr Leu Ala Pro Ser
2015 2020 2025

Leu His Ser Glu Thr Leu Ser Pro Ala Ile Asp Phe Gly Ser Thr
2030 2035 2040

Pro Phe Val Val Gln Arg Glu Arg Ala Glu Trp Arg Arg Pro Val
2045 2050 2055

Val His Gly Ala Glu Val Pro Arg Arg Ala Gly Val Thr Ser Ile
2060 2065 2070

Gly Ala Gly Gly Ile Asn Val His Leu Ile Val Glu Glu Phe Asp
2075 2080 2085

Gly Thr Val Asn Ser Ala Pro Asp Asp Gly Gly Ser Gln Leu Leu
2090 2095 2100

Val Phe Ser Ala Met Thr Pro Gln Ala Leu Gly Thr Val Leu Arg
2105 2110 2115

Asp Ala His Arg His Val Ala Asp Glu Ala Pro Ala Leu Asn Ala
2120 2125 2130

Leu Ala Tyr Thr Leu Gln Thr Gly Lys Asn Glu Leu Pro Cys Arg
2135 2140 2145

Leu Ala Phe Val Ala His Gly Thr Ala Asp Ala Glu Ala Arg Leu
2150 2155 2160

Ala Ala Leu Ala Ala Val Asp Trp Thr Ser Gly Ala Pro Ala Leu
2165 2170 2175

Pro Asp Ala Val Arg Phe Thr Glu Ser Thr Leu Arg Lys Arg Arg
2180 2185 2190

Ser Val Ala Ala Ala Asp Val Glu Arg Ala Leu Ala Gln Ala Asp
2195 2200 2205

Leu Ala Glu Leu Ala Gly Tyr Trp Ile Ser Gly Ala Ala Val Asp
2210 2215 2220

Trp Asp Leu Leu Trp Pro Ser Gly Thr Arg Pro Ala Lys Leu Ala
2225 2230 2235

Leu Pro Ala Tyr Pro Phe Glu Lys Val Arg Cys Trp Tyr Pro Gly
2240 2245 2250

Phe Asp Asp Ala Pro Ser Val Leu Arg Pro Leu Ala Phe Thr Arg
2255 2260 2265

Arg Gly His Pro Trp Val Gly Val Asn Arg Ser Asp Leu His Gly
2270 2275 2280

Val Arg Phe Ala Leu Glu Leu Thr Gly Asp Glu Leu Leu Asp Tyr
2285 2290 2295

Val His Thr Val Gly Arg Thr Arg Arg Phe Thr Ser Val Ala Leu
2300 2305 2310

Leu Asp Gly Ala Leu Ala Phe Ala Arg Leu Ala Gly Leu Asp Gly
2315 2320 2325

Ala Leu Arg Leu Arg Asp Ala Arg Trp Ala Glu Leu Pro Ser Pro
2330 2335 2340

Gly Asp Ala Thr Glu Val Phe Glu Trp Arg Leu Ala Leu Ser Gly
2345 2350 2355

Glu Gly Ala Ser Gly Asp Ala Ala Ser Gly Gly Gly Ala Ser Gly
2360 2365 2370

Ala Gly Gly His Arg Val Glu Leu Trp Gln Ala Glu Arg Gly Thr
2375 2380 2385

Leu His Phe Ser Ala Glu Val Val Pro Ala Thr Ala Val Ala Ala
2390 2395 2400

Gly Ala Val Asp Ala Arg Pro Ala Asp Ala Ala Ala Leu Leu Ala
2405 2410 2415

Ala Pro Val Thr Leu Asp Gly Asp Ala Phe Tyr Ser Ala Leu Gly
2420 2425 2430

Glu Ala Gly Val Asp Ala Arg Pro Tyr Ala Arg Ala Val Thr Gly
2435 2440 2445

Val Thr Glu Ala Gly Gly Arg Arg Leu Leu Val Arg Val Ala Glu
2450 2455 2460

Pro Ala Met Cys Gln Asp Pro His Lys Gln His Val Ser Ile Pro
2465 2470 2475

Ala Trp Val Leu Ala Gly Leu Ala Gln Gly Val Gln His Ala Thr
2480 2485 2490

Gly Arg Pro Arg Thr Thr Ala Leu Arg Ala Ala Ala Leu Tyr Gly
2495 2500 2505

Ala Asp Leu Thr Asp Thr Arg Ala Leu Leu Leu Glu Pro Val Ala
2510 2515 2520

Glu Ala Thr Phe Arg Ile Thr Phe Leu Asp Gly Asp Gly Arg Ala
2525 2530 2535

Leu Gly Ala Val Glu Asp Ala Glu Phe Thr Ala Gly Thr Leu Pro
2540 2545 2550

Pro Ser Leu Glu Gly Gly Ala Val Pro Val Arg Ala Gly Leu Pro
2555 2560 2565

Gly Ala Ala Arg Pro Ser Ala Thr Ala Ser Ala Pro Ala Ser Ala
2570 2575 2580

Ser Val Pro Val Pro Ala Leu Ala Ala Ala Pro Val Ala Pro Ala
2585 2590 2595

Val Pro Val Glu Pro Val Glu Pro Ala Ala Glu Ala Asp Ala Asp
2600 2605 2610

Ala Gly Asp Ala Leu Val Ala Val Leu Arg Glu Thr Val Ala Asp
2615 2620 2625

Leu Leu Lys Phe Glu Leu Asp Glu Ile Asp Leu Asp Thr His Phe

2630 2635 2640

His Ala Tyr Gly Phe Glu Ser Ile Ala Leu Ala Arg Leu Ala Ser
2645 2650 2655

Glu Leu Asn Gly Leu Leu Gly Thr Asp Leu Ser Pro Val Val Phe
2660 2665 2670

Phe Glu Cys Pro Asp Ile Arg Ser Leu Ala Ala His Leu Arg Glu
2675 2680 2685

Arg Tyr Asp Ala Glu Thr Ala Ala Arg Ala Val Arg Gly Thr Gly
2690 2695 2700

Gly Gly Thr Gly Thr Asp Ala Ala Arg Ala Pro Thr Pro Ala Pro
2705 2710 2715

Ala Pro Ala Val Gly Ala Ala Ser Ala Ala Thr Ala Pro Ala Pro
2720 2725 2730

Leu Ser Ser Ala Glu Pro Val Ser Asp His Glu Ala Asp Tyr Pro
2735 2740 2745

Gly Ala Val Ala Val Val Gly Val Ala Gly Arg Phe Pro Gly Ala
2750 2755 2760

Pro Asp Ala Asp Thr Phe Trp Gln Arg Leu Arg Ala Gly Asp Asp
2765 2770 2775

Leu Ile Gly Glu Tyr Pro Gly Asp Arg Phe Asp Glu Arg Tyr Thr
2780 2785 2790

Gly Val Val Ala Arg Ser Asp Phe Pro Lys Phe Ala Gly Val Leu
2795 2800 2805

Asp Asp Val Asp Arg Phe Asp Ala Gly Phe Phe Asn Leu Ser Arg
2810 2815 2820

Leu Glu Ala Glu Leu Met Asp Pro Gln His Arg Leu Ala Leu Glu
2825 2830 2835

Thr Val Trp Ala Ala Leu Glu Asp Gly Gly Tyr Ala Pro Gly Arg
2840 2845 2850

Leu Pro Glu Asn Thr Gly Val Tyr Val Gly Val Ser Gly Ser Asp
2855 2860 2865

Tyr His His Leu Leu Asn Ala Ser Gly Val Ala Pro Asp Gly Phe
2870 2875 2880

Thr Ala Thr Gly Asn Ala His Ser Met Leu Ala Asn Arg Ile Ser
2885 2890 2895

Phe Val Leu Asp Val His Gly Pro Ser Glu Pro Val Asp Thr Ala
2900 2905 2910

Cys Ser Ser Ser Leu Val Ala Leu His Arg Ala Val Glu Ser Ile
2915 2920 2925

Arg Ser Gly Arg Cys Asp Met Ala Leu Ala Gly Gly Val Asn Leu
2930 2935 2940

Leu Leu Ser Ile Asp Thr Phe Ala Ala Thr Gln Met Ala Gly Met
2945 2950 2955

Leu Ser Pro Asp Gly Arg Cys Lys Thr Phe Ser Ala Asp Ala Asp
2960 2965 2970

Gly Tyr Val Arg Ala Glu Gly Val Ala Ala Val Leu Leu Lys Pro
2975 2980 2985

Leu Glu Arg Ala Leu Ala Asp Gly Asp Pro Val Trp Gly Val Val
2990 2995 3000

Arg Gly Ser Ala Glu Asn His Gly Gly Arg Ala Gly Ser Leu Thr
3005 3010 3015

Ala Pro Asn Ala Val Ala Gln Thr Ala Leu Ile Arg Glu Ala Met
3020 3025 3030

Arg Gly Thr Asp Pro Asp Ser Val Gly Tyr Val Glu Ala His Gly
3035 3040 3045

Thr Gly Thr Gly Leu Gly Asp Pro Val Glu Val Gly Ala Leu Asp
3050 3055 3060

Ser Ala Tyr Arg Ala Leu Arg Ser Asp Arg Gly Arg Val Glu Ser
3065 3070 3075

Gly Thr Ala Pro Val Ala Leu Gly Ser Val Lys Thr Asn Ile Gly
3080 3085 3090

His Ala Glu Ser Ala Ala Gly Leu Ala Gly Val Leu Lys Val Leu
3095 3100 3105

Leu Ala Met Arg His Gly Glu Leu Pro Pro Thr Leu His Cys Asp
3110 3115 3120

Arg Leu Asn Pro His Leu Pro Leu Ser Gly Gly Gly Phe Glu Val
3125 3130 3135

Val Arg Glu Val Arg Arg Trp Glu Pro Arg Leu Asp Ala Asp Gly
3140 3145 3150

Arg Pro Trp Pro Leu Arg Ala Gly Val Ser Ser Phe Gly Phe Gly
3155 3160 3165

Gly Ala Asn Ala His Val Val Leu Glu Ala Ala Pro Ala Ala Ala
3170 3175 3180

Arg Glu Arg Ala Val Arg Glu Thr Ala Ser Arg Ser Ala Ser Val
3185 3190 3195

Arg Ser Ala His Gly Thr Gln Gly Ala Pro Gln Ala Val Ala Pro
3200 3205 3210

Gln Ala Val Gly Pro Gln Ile Val Ala Val Ser Ala Arg Asp Gly
3215 3220 3225

Glu Arg Leu Arg Ile Val Ala Glu Arg Leu Arg Asp Phe Leu Arg
3230 3235 3240

Arg Glu His Gly Ala Gly Arg Ala Pro Ala Thr Ala Asp Leu Ala
3245 3250 3255

Arg Thr Leu Gln Thr Gly Arg Glu Ala Met Glu Ala Arg Leu Ala
3260 3265 3270

Phe Val Ala Glu Glu Thr Gly Asp Val Leu Asp Val Leu Asp Arg
3275 3280 3285

Phe Leu Lys Gly Glu Glu Pro Asp Gly Trp His Thr Gly Ala Leu
3290 3295 3300

Arg Arg Ser Arg Gly Ala Gly Val Arg Arg Asp Arg Ala Gln Asp
3305 3310 3315

Pro Arg Val Thr Arg Ala Leu Arg Asp Gly Asp Leu Asp Ala Ala
3320 3325 3330

Ala Ala Leu Trp Cys Glu Gly Ala Leu Val Asp Trp Gln Ser Leu
3335 3340 3345

His Pro Pro Gly Glu Arg Arg Thr Val Arg Leu Pro Ser Tyr Pro
3350 3355 3360

Phe Ala Arg Glu Arg Tyr Trp Val Pro Thr Asp Gly Ala Ala Pro
3365 3370 3375

Pro Pro Glu Thr Gly Gly Pro Gly Gly Val Glu Asp Gly Gly Val
3380 3385 3390

Glu Tyr Gly Thr Gly Ser Gly Ala Ala Gln Leu Gly Asp Ser Gly
3395 3400 3405

Ser Ala Phe Asp Ala Gly Ala Leu Ala Ala Val Leu Asp Ala Val
3410 3415 3420

Leu Asp Gly Arg Ala Asp Pro Asp Asp Leu Ala Arg Thr
3425 3430 3435

<210> 33
<211> 10311
<212> DNA
<213> Streptomyces amphibiosporus

<400> 33

```
atgaaggaag aatccggcgc cctccccgag gaaggccccg tcggcaccgc tgtcggcacc     60
gccgccgacg gcgcggccgg cggcccggtg gacgggcagg acatcgctgt cgtgggcctg    120
tccctgcggc tgccgggcgc acggaacccg gaggagttct gggagcacct ggccgcgggc    180
cgctcgctga tcagcgaggt gcccgagcgg cgctggcgca aggaggacca tctcggcaac    240
ccgcgccggg agttcaacaa gaccaacagc gtgtggggcg gcttcgtcga cgacgccgac    300
tgcttcgacg ccgagttctt ccatgtctcg ccgcgcgagg cccgctccat ggacccgcag    360
cagcggatgg cgctggagat gagctggcag gcgctggagg acgccggata ccgggccgac    420
cgggtcgccg gctcccgtac gggcgtcttc atgggggtgt gccactggga ctacgccgag    480
```

ctcatcgaga aggaggtctc cgaggtcgac gcctactacc cgacgggcgc cgcgtacgcg 540
atcatcgcca accgcgtatc gcaccacttc gatttccgcg ggcccagcgt cgtcaacgac 600
accgcgtgcg ccagttcgct ggtggcggtg cagcaggcgg tgcaggcgct ccagtccggg 660
gactgcgacc acgcgctggc cggaggcgtc aacctgacct ggtcgccacg gcacttcatc 720
gccttcgcca aggcgggcat gctctcgccg gacgggctct gccgcgcctt cgacgcggac 780
gccaacggct acgtacgggg tgagggcggc ggcgtcgtcc tgctgaagcg ggcggcggac 840
gcccgccggg acggcgaccc cgtacacgcg gtgatcaagg gcatcggcag caaccacggc 900
gggcgcacca gttcgctcac cgtcaccaac cccgccgcgc aggccgagct gatcgccggg 960
atctaccggc gggcggggat cgcccccggag tccgtctcct acatcgagac ccacgggccg 1020
ggcaccccgg tcggcgaccc catcgaagtc agcggcctca agcgggcgtt cgcgcagctc 1080
ggcgagggac gggaggccga gccgtccggg caccgctgcg gcatcggctc ggtgaagacc 1140
aacatcgggc atctggaggg cgccgcgggc atcgccggga tgctcaaggt gatcctggcg 1200
atgcgtcacc gcaagctgcc cgcgacggtg aacttccgcc gtctcaaccc gctgatcacg 1260
ctggacggca gcccgctgta cgtcgtggac cggctcaccg actgggagac ggacggcgac 1320
gggacgctgc gggcgggtgt cagctcgttc ggcttcggcg gcaccaacgc gcatgtggtg 1380
ctggaggcgc ccggcggtca cgcggcggag gtcacggacg cggaggcggt cacggacgcg 1440
gaggcggacg ccgacgtgga cggcgggccg gacgaggggc ccgacgaggg cgccgaaccg 1500
cgtgctctgc ggctccccgt ctccgccgac gacgaggagc ggctgcgtga gctgtgccgc 1560
tcgctcgccg agtgggcccg tgcccgcgaa gccgaaggca cggcgccgcc gctggccgac 1620
atcgcccgca ccctgcgcga agggcgggtg ccgatgcggg agcgtgcggt cttccgcgcg 1680
cggagcgtcg ccgagtgggc ggaacagctc acggccctcg ccgaggggac cggcggggag 1740
ccgcccgctg gctgtctgcg cggacgcgcg gaggacggcg ccggggacgg cctggacgcc 1800
gacgatgtcg cggccctgac cgcgcgctgg cgggagcggg acgaggagga gaagttcgcc 1860
gcggcctgga cgagggggcct gcccgtggac tgggcgcagt ggcccgccga gggccgccgc 1920
gtccatctgc ccggacaggt cttccagcgg acgccgcact ggttccgtcc ggacgaacag 1980
ccgcgcggcg aggccgagtc ggcgggcggt gcggcggctc agcgggacac cgctccagag 2040
cgggacgcgg cgtccgggtc ggaacgcggg cccggcgcac cggagccggc gggaccggtg 2100
ggagggccgg ggctgccggg cgagggcgtc caggacgggc ggggctggca cttcccgctg 2160
cgcttcgccg ccaccgaccc cttcgtacgc gaccatctgg tcttgggcgc ccgtatcgtc 2220
cccggcgtcg tggcgctgga ggccgtgacc gccgccgcgg cacgtcccgc tgtggccggt 2280
gcccgtgccg gtgcggcgcc gcacatccgc aacgcggtgt gggtgcggcc gctgcgcgtg 2340

```
gacggccaag tcctcgaaac gagcctgcgg ttgacgcccg ccggcccgga gtccggcggc   2400
ggctacgact gggccgtcac cgacgccgcg ggcacgccgt acagcagcgg tcgcgtcgag   2460
tacgccgacg ggcccgcgcc cgccgccacg gatctggacg cgctgcaccg gcggcacacc   2520
cgtcccgtgg aggtggccgg gggatacgcg gcgctgtacg ccagcggcat cgagcacggc   2580
ccggcgctgc gcgccctgca cacgctgcgc gccgggcccg aagggctgct ggccgagctg   2640
cggttgcccg ccgagccggc tgcgggcgcg gctctccagc ccgccgttct ggacagcgcc   2700
ctgctggccg tgctcgcgct cggtacgggc ggtggcgacg gcacagaggg caccggcggc   2760
acagacggcg cgggctggcg ccgaccggac gcgcccgccg tgccgttcgc gctggacggc   2820
ctgaccgcgt acgccccgac cacggccacg acatgggcct ggctgcggcc cgcgggcgga   2880
cgccgtcccg gcgccgccga catcgatctg ttcgacgagc gggggcggtt gtgcgcccgt   2940
ctgacgggct acacctcgcg tgaactgtcc accgggagcc cggcgttgag ggaagcgcgc   3000
gtttctgcac cggcaccggg cgaaggggcg gcgggcgagg aggctccggg gaaggacgct   3060
ccgggcgagc tgctggaggt caccgggcgc tggacgcccg cgcccctcgg cctccccgcc   3120
gccgaggcgg gaccggccgc ggcacagtcg ggcgccgccg ctcccgtcac ggtgctgaac   3180
gccgccctgg acgccgacct cgtcgccgcg agcgccgccc gtctcggcat ggacgtcgag   3240
cacctggccg taccgcgcga cgcgggcgac gcggacgcca tgaaggcggc gttcgcggcc   3300
tgttaccccc atgtccggcg gctcgtcgga cagtcgcggc gcgttctgct cgtggccccc   3360
ggcgcacccg actccccggt cttcgcgccg ctggcggccc tgctgaagac ggcacaccag   3420
gagaacccgt ccttccacgg caccaccgtg ctcctggagg gcttcgaccc gcgtgactcc   3480
gcacgcttcg agcaggtcgt ccgtacggag gcggcagcga cggcggacgc cgcgggcggc   3540
gcggcggacg aggaggtcgc ccacaccgcc gacggccgcc ggctgcgcca tgagacggcc   3600
gaactgccgc acggcacaac gggcgagagc ctgctggcgg agggcggcgt ctactggatc   3660
accggcggcg cgggcggcat cggcctgctg ctggccgagc ggctgtgcct gcggtacggg   3720
gcgacggtcg tcctcagcgg acggtcgccc gctgcgcccg cggccgacgc gctcgcctcc   3780
cggctcaccc gcggcacgct ggcctaccgc ggcgcggacg tcaccgacca ggacagcgtg   3840
gacgcgctgg tggccgccgt gctggccgaa cacggccgga tcgacggtgt gttccacgcc   3900
gccggggtgc tcgacgacgg ctatctgacg gccaagccgc tcgccgggac cgaggccgtg   3960
ctcgcgccga aggtggacgg cgtcacctgc gtcgaccgcg ccacgcgcgc gggcgctccg   4020
ggcttcctgc tggtcttcgg gtcggtcgcg ggtgccttcg gcaacgcggc gcaggccggc   4080
tacgccgccg cgaacgccta cctcgacgcg ttcgccgccc ggcggcaggc cgccgggctg   4140
```

accacccggg ccgtcgactg gccgctgtgg gccgagggcg gcatgcgcgt ggacgacgcg 4200
agcctgaagt atctgcgcaa gcgcaccggc accgtgccgc tgccctccgg caccggcctc 4260
gacgcgctgg agcgtgcgct gcacaccggt tcgccggtgc ggcgcgtggt cctctacggc 4320
gaccgtccgg cgctgcgggt gtacgcgggt ctggaccgtc cgcaggtcac gggcgcacgg 4380
tccggctccg cgtccgcgtc cgcgccgggc tcctcgtccg ggtccgtgtc cgccgtgcgc 4440
ggcgagggga ccgggacggc accggccgcg ctcaccgacg ccgaactcct cgtccgcaca 4500
caggacttcc tgcgggagca gttcgccgag gtcaccctcc aggacgccga gcagatccac 4560
cccgaggaga agctggagac gtacgggatc gagtcgatct cgatcgtcga tctgacgagc 4620
aggctggagg acgtcttcgg ctcgctgccc aagaccctct tcttcgagta cgtcgatctg 4680
aagggcgtgg ccgagtactt cgtggccgag caccgtgcgc ggctgaccga actcttcgcg 4740
ccggaggagc cgcaggcgtc cgaggcggcg gagcccgcac cggaagaacc cgtggcgccc 4800
gcccccgtac cggtggagcc ggccgccgcg gcacccgcac ccgcacccgc acctgtaccg 4860
gcgcctccgg cgccaaccgc cgcaccgggt acgtccgttg aggccgtccc cgcgcccgtt 4920
cccgcttccg tacccacgcc gcgcccccgcc cccgccggga acggcgacat cgctgtcgtc 4980
ggcatggcgg gccgctaccc gggcgccgac accctggagg agttctggga gctgctcagc 5040
gagggacggc acagcttcga gcccgtgccg tcctcgcggt ggccgcacgg cgacctgtac 5100
ttcgacgagc gggacgtgct gggcaagacc acggtgcgca ccggcacctt cctgcgtgac 5160
gtcgacgcct tcgacccccg ctacttcagc atctcccagc gcgacgccga actcctctcg 5220
cccgaggtgc gcctcttcct ccaggcgggc gtgacggctc tggaggacgc cgggtactcc 5280
aaggagacgc tgcgccgccg ctacgacggc gacgtgggcg tgctcgtcgg ctcgatgaac 5340
aacagctacg cctactacgg cttcgagaac atgctgatgc gcggcaccgc gatgagcggc 5400
agcgaggtcg gcgtgatggc caacatgctc tcgtactact acgggttcac gggcccgtcg 5460
atgttcgtcg acaccatgtg ctcgtcgtcc tcggcctgtg tgcaccaggc gctgagcatg 5520
ctgcgcggcg gcgagtgccg catggtcgtc gtcggcggca tcaacctgat gctccacccg 5580
tacgacctga tcgccacctc gcaggcgcac ttcaccacca agtcggcgga ggtcgtgcgc 5640
agttacggac tcggcgcgga cggcacgatc ctcggcgagg gcgtggggac cctggtgctc 5700
aagccgctcg ccgaggccgt cgcggacggc gaccacgtct acggcgtcat caagggcagc 5760
ggcatgacca acgccggtgt gcgcaacggc ttcacggtgc ccagcccgca gcagcaggcg 5820
agggcgatcg agagggcgct cgacgacgcc gccgtggacg cgcgcaccgt cagctacctg 5880
gagggccacg gctcggcgac ctcgctgggc gacccgatcg agatcaaggg cgcgagcctc 5940
gccttcggcc gggacacccg ggacgtgggc tactgcgcga tcgggtcggt caagtccaac 6000

gtggcgcatc tgctgtccgg ttcgggcctc gtcggcctga cgaaggtgct gcttcagcta 6060
cggcaccgga cgctggcgcc gtcgctgcac tccgaaaccc tcagccccgc catcgacttc 6120
ggctcgacgc cgttcgtggt gcagcgtgag cgcgccgagt ggcggcgtcc cgtcgtacac 6180
ggcgcggagg tgccgcgccg cgcgggcgtc acctcgatcg gcgcgggcgg catcaacgtg 6240
cacctgatcg tcgaggagtt cgacggcacg gtgaactccg cgcccgacga cggcggttca 6300
cagcttctgg tgttctccgc gatgacgccg caggccctgg gcaccgtgct gcgcgacgcg 6360
caccgccatg tcgccgacga ggcgcccgcg ctcaacgccc tcgcgtacac cctccagacc 6420
ggcaagaacg aactcccctg ccggctggcc ttcgtcgcgc acggcacggc cgacgccgag 6480
gcccggctcg ccgcgctggc ggcggtggac tggacgtccg gcgcacccgc gctgcccgac 6540
gctgtgcgct tcaccgagag cacgctgcgg aagcggcgca gcgtggcggc cgccgacgtc 6600
gaacgggccc tggcgcaggc cgacttggcg gagctggccg gatactggat ctccggcgcg 6660
gccgtggact gggacctgct gtggccctcg ggtacccgtc cggcgaagct ggcgctgccc 6720
gcgtacccgt tcgagaaggt gcgctgctgg tacccgggtt tcgacgacgc ccccagcgtg 6780
ctgcggcccc tggccttcac ccggcgcggg cacccctggg tcggcgtcaa ccgctccgat 6840
ctgcacggcg tgcgcttcgc gctggagctg acgggcgacg aactcctcga ctacgtccac 6900
acggtgggcc gcacccgccg cttcacgagc gtcgccctgc tggacggggc gctggcgttc 6960
gcgcggctcg cgggcctgga cggcgcgctg cggctgcgcg acgcgcggtg ggcggagctg 7020
ccctcgcccg gcgacgccac ggaggtcttc gagtggcggc tcgcgctctc cggcgaggga 7080
gcgtccggtg acgcggcgtc cggcggagga gcgtccggtg cgggcggcca tcgtgtcgag 7140
ctgtggcagg ccgagcgcgg cacgctgcac ttctcggcgg aggtcgtacc ggcaactgcc 7200
gtggcggcgg gggccgttga cgcgcggcct gccgacgccg cggcgctgct cgccgcaccc 7260
gtgacgctgg acggcgacgc gttctactcc gcgctcggcg aggcgggcgt ggacgcccgc 7320
ccgtacgcgc gcgccgtcac cggcgtcacc gaggccggcg gacggcggct gctcgtccgt 7380
gtcgccgaac cggcgatgtg ccaggacccg cacaagcagc acgtcagcat tcccgcgtgg 7440
gtgctggcgg ggctggcaca gggcgtgcag cacgccacgg gccggccgcg tacgacggca 7500
ctgcgcgccg ccgcgctgta cggcgccgac ctgacggaca cccgcgccct gctgctggag 7560
cccgtcgcgg aggccacctt ccggatcacc ttcctggacg gggacgggcg ggcgctgggc 7620
gcggtggagg acgcggagtt caccgccggg acgttgccgc cgtcgctgga gggcggcgcc 7680
gtaccggtac gggccggact gccgggcgcg gcacgcccgt cggcgacggc ctcggcaccg 7740
gcctcggcct cggtaccggt accggcgctc gcggccgcac ccgtcgcacc tgccgtgccc 7800

gtcgagcccg tcgagcccgc cgcggaggcg gacgccgacg cgggggacgc gctcgtcgcc 7860
gtgctgcggg agacggtcgc cgacctgctc aagttcgagc tggacgagat cgacctcgac 7920
acccacttcc acgcgtacgg cttcgagtcc atcgccctcg cgcggctggc ctccgaactc 7980
aacggcctgc tcggaacgga cctcagccct gtcgtgttct tcgagtgccc cgacatccgc 8040
agcctcgccg cccatctgcg cgagcgctac gacgcggaga cggcggcccg cgccgtccgc 8100
ggcaccggcg gcggcaccgg gacggacgcg gcccgggcgc cgactcccgc tccggcaccg 8160
gcagtcgggg cggcgtccgc ggccaccgcg cccgctcccc tctcgtccgc cgagcccgtg 8220
tccgaccacg aggccgacta cccgggcgcc gtcgccgtcg tcggtgtggc gggccgcttc 8280
cccggcgcgc cggacgccga caccttctgg cagcggctgc gcgcggggga cgacctgatc 8340
ggggagtacc cgggcgaccg gttcgacgag cgctacaccg gcgtcgtcgc acggtcggac 8400
ttcccgaagt tcgcgggcgt cctcgacgac gtcgaccgct tcgacgccgg cttcttcaac 8460
ctctcccggc tcgaagccga actgatggac cccagcacc ggttggccct ggagacggtg 8520
tgggcggcgc tggaggacgg cggctacgcg cccggccggc tgccggagaa caccggcgtc 8580
tacgtcggcg tctccggcag cgactaccac cacctgctga acgccagcgg cgtggcgccg 8640
gacggcttca ccgccaccgg caacgcccac tcgatgctgg ccaaccggat ctccttcgtc 8700
ctggacgtgc acggcccgag cgagccggtg gacaccgcgt gctccagctc gctcgtcgcc 8760
ctgcaccgcg cggtggagag catcaggtcg ggccgctgcg acatggccct ggcgggaggc 8820
gtcaacctgc tgctgagcat cgacacgttc gccgcgacgc agatggcggg catgctcagc 8880
ccggacggcc gctgcaagac cttctccgcg gacgccgacg gctacgtacg tgccgagggc 8940
gtcgccgcgg tgctactcaa gccgctggag cgggcgttgg cggacggcga cccggtctgg 9000
ggcgtcgtac gcggcagcgc cgagaaccac ggcggccgtg ccggttcgct caccgcaccc 9060
aacgccgtcg cgcagaccgc gctcatccgc gaggccatgc gcggcaccga ccccgacagc 9120
gtcggctatg tcgaggcgca cggcacgggc accggtctcg gcgaccccgt cgaggtcggc 9180
gccctcgaca gcgcctaccg cgcgctgcgt tcggaccgcg ggcgtgtcga gagcggcacc 9240
gcaccggtgg cgctgggctc ggtgaagacc aacatcgggc acgccgagtc ggcggccgga 9300
ctcgccgggg tgctgaaggt gctgctggcc atgcgccacg gcgaactgcc gccgaccctc 9360
cactgcgacc ggctcaatcc ccatctgccg ctgtccggcg gcgggttcga ggtggtgcgc 9420
gaggtacgcc gctgggagcc gcggctcgac gcggacgggc ggccgtggcc gctgcgcgcc 9480
ggggtgagca gcttcggctt cggcggcgcc aacgcgcacg tcgtgctgga ggccgcaccc 9540
gcggcggcac gggaacgggc cgtacgggaa acggcttcgc ggagtgcgtc cgtacggtcc 9600
gcgcacggga cgcagggcgc tccgcaggcc gtcgctccgc aggccgtcgg tccgcagatc 9660

gtcgccgtct cggcgcgtga cggcgagcgg ctgcggatcg tggccgagcg gctgcgggac 9720

ttcctgcggc gggagcacgg cgcgggccgg gcgcccgcga cggccgacct cgcccgcacg 9780

ttgcagacgg gacgggaggc gatggaggcg cgtctcgcct tcgtcgccga ggagaccggg 9840

gacgtgctcg acgtactgga ccggttcctc aagggcgagg agcccgacgg ctggcacacc 9900

ggcgcactgc ggcgctcgcg cggcgcggga gtgcggcgcg accgggcgca ggacccgcgg 9960

gtgacccgcg cgctgcggga cggggacctc gatgcggccg ccgcgctgtg gtgcgagggg 10020

gccctcgtcg actggcagtc gctgcacccg ccgggggagc gccgcaccgt gcggctgccc 10080

tcgtacccgt tcgcccgcga acgctactgg gtgcccacgg acggggcggc accgccaccg 10140

gagaccggcg ggcccggcgg cgtcgaggac ggcggcgtcg agtacggcac cgggtccggc 10200

gccgcccaac tcggcgacag cgggagcgcg ttcgacgccg gagcccttgc cgcggtgctc 10260

gacgcggtcc tcgacggacg ggccgatccc gacgacctcg cccgtacctg a 10311

<210> 34
<211> 8360
<212> PRT
<213> Streptomyces amphibiosporus

<400> 34

Val Ser Arg Asn Ile Leu Arg Val Pro Ala Trp Arg Asp Glu Pro Ser
1 5 10 15

Arg Gly Gln Ala Ala Pro Ala Gly Val Arg Arg Leu Ala Val Leu Cys
20 25 30

Asp Val Pro Asp Ala Glu Ala Ala Leu Leu Arg Gln His Ser Pro Arg
35 40 45

Leu Pro Val Val Leu Val Glu Ser Arg Asp Asp Gly Pro Ala Ala Ala
50 55 60

Tyr Glu His Ala Ala Thr Arg Leu Leu Ala Glu Leu Gln Arg Leu Leu
65 70 75 80

Gly Arg Pro Ala Ala Gly Pro Cys Arg Val Gln Val Val Cys Arg Glu
85 90 95

Ser Thr Pro Gln Gly Trp Ala Gly Leu Leu Gly Met Leu Arg Thr Ala
100 105 110

Ala Gln Glu Asp Pro Arg Leu Arg Gly Gln Leu Ile Glu Phe Asp Arg
115 120 125

Leu Pro Gly Gly Ala Glu Leu Ala Arg Val Leu Asp Glu Glu Ala Ala
130 135 140

Glu Glu Ala Asp His Val Arg Arg Ala Ala Gly Ala Ala Gly Thr Gly
145 150 155 160

Thr Gly Thr Gly Ala Val Arg Gln Val Arg His Trp Ser Ala Ala Arg
165 170 175

Ser Ala Gly Arg Ala Ser Ser Ala Gly Asn Pro Ala Pro Val Trp Arg
180 185 190

Pro Gly Gly Val Tyr Leu Val Ser Gly Gly Ala Gly Gly Leu Gly Arg
195 200 205

Leu Leu Ala Ala Asp Val Arg Arg His Ala Pro Gly Ala Val Thr Val
210 215 220

Val Cys Gly Arg Gly Pro Ala Pro Trp Gln Gly Ala Glu Pro Pro Ala
225 230 235 240

Asp Gly Val Glu Tyr His Ser Val Asp Val Thr Asp Arg Ala Ala Val
245 250 255

Ala Ala Leu Val Asp Arg Val Leu Ser Ala His Gly Arg Leu Asp Gly
260 265 270

Val Val His Ala Ala Gly Leu Leu Ala Asp Asp Tyr Val Val Arg Ala
275 280 285

Ser His Arg Glu Thr Gln Arg Val Leu Ala Pro Lys Val Ala Gly Leu
290 295 300

Val His Leu Asp Glu Ala Thr Arg Glu Leu Pro Leu Asp Phe Leu Ala
305 310 315 320

Ala Phe Ser Ser Ala Ala Gly Thr Leu Gly Asn Ala Gly Gln Ala Gly
325 330 335

Tyr Ala Ala Ala Asn Gly Phe Leu Asp Ala Tyr Gln Thr His Arg Ala
340 345 350

Ala Leu Ala Glu Ala Gly Glu Arg His Gly Arg Ser Leu Ser Val Gly
355 360 365

Trp Pro Leu Trp Arg Asp Gly Gly Met Thr Val Pro Asp Glu Gln Leu
370 375 380

Pro Glu Leu Thr Glu Arg Phe Gly Arg Pro Leu Thr Thr Gly Thr Ala
385 390 395 400

Leu Thr Ala Leu His Ala Ala Leu Ala Leu Gly Thr Pro His Val Leu
405 410 415

Val Arg Asp Gly Ala Glu Ala Asp Glu Thr Gly Ala Val Asn Ala Thr
420 425 430

Gly Ala Gly Thr Ala Thr Gly Ile Ala Thr Glu Val Glu Val Pro Ala
435 440 445

Val Asn Glu Ala Val Gly Thr Ala Val Asp Asp Ala Leu Glu Asp Asp
450 455 460

Ala Pro Glu Gly Asp Arg Lys Gly Thr Pro Ala Val Glu Pro Arg Leu
465 470 475 480

Arg Val Leu Pro Ala Leu Lys Gln Leu Val Ala Glu Thr Val Arg Leu

485 490 495

Asp Pro Ala Ala Leu Asp Ala Ala Ala Pro Leu Asp Gly Phe Gly Ile
500 505 510

Asp Ser Leu Ala Val Thr Arg Leu Asn Arg Arg Phe Ala Gln Trp Phe
515 520 525

Gly Ala Leu Pro Lys Thr Leu Leu Tyr Gln Tyr Pro Thr Leu Asn Glu
530 535 540

Leu Ala Gly Tyr Leu Ala Glu His His Pro Glu Gly Cys Arg Arg Trp
545 550 555 560

Leu Ala Asp Thr Ala Ser Pro Ser Leu Ser Pro Ser Ala Ser Ala Ser
565 570 575

Ala Ser Pro Ser Pro Ser Pro Ala Thr Ser Thr Ser Val Ser Ala Pro
580 585 590

Ser Ala Gln Glu Arg Arg Pro Ser Thr Pro Val Ala Ala Gly Ala Val
595 600 605

Arg Thr Ala Gly Thr Asn Gly Thr Ser Gly Ala Ala Ala Pro Val Ser
610 615 620

Ala Glu Ala Pro Val Pro Ala Arg Thr Ser Pro Val Asp Glu Pro Ile
625 630 635 640

Ala Val Ile Gly Leu His Gly Arg Tyr Pro Gly Ala Pro Thr Leu Asp
645 650 655

Ala Phe Trp Glu Asn Leu Arg Ser Gly Arg Asp Gly Val Thr Glu Ile
660 665 670

Pro Ala Glu Arg Trp Pro Leu Glu Gly Phe Trp Glu Pro Asp Val Glu
675 680 685

Arg Ala Val Arg Glu Gly Ala Ser Tyr Ser Lys Trp Gly Gly Phe Leu
690 695 700

Asp Gly Phe Ala Gln Phe Asp Ala Leu Phe Phe Gly Ile Ala Pro Arg
705 710 715 720

Glu Ala Ala Asp Met Asp Pro Gln Glu Arg Leu Phe Val Glu Ser Ala
725 730 735

Trp Ser Val Leu Glu Asp Ala Gly Tyr Thr Arg Arg Arg Leu Ala Glu
740 745 750

Gln His Arg Ser Arg Val Gly Val Phe Ala Gly Ile Thr Lys Thr Gly
755 760 765

Phe Asp Arg His Arg Pro Ala Ala Pro Ala Glu Thr Asp Ala Ser Ser
770 775 780

Ala Thr Gly Gly Val Pro Pro Ala Ser Pro Arg Thr Ser Phe Gly Ser
785 790 795 800

Leu Ala Asn Arg Val Ser Tyr Leu Leu Asp Leu Arg Gly Pro Ser Met
805 810 815

Pro Val Asp Thr Met Cys Ser Ala Ser Leu Thr Ala Val His Glu Ala
820 825 830

Cys Glu His Leu Arg His Gly Ala Cys Glu Leu Ala Val Ala Gly Gly
835 840 845

Val Asn Leu Tyr Leu His Pro Ser Thr Tyr Val Glu Leu Cys Arg Ser
850 855 860

Arg Met Leu Ala Arg Gly Gly Glu Cys Arg Ser Phe Gly Thr Gly Gly
865 870 875 880

Asp Gly Phe Val Pro Gly Glu Gly Val Gly Thr Val Leu Leu Lys Pro
885 890 895

Leu Ser Lys Ala Glu Ala Asp Gly Asp Pro Val His Ala Val Ile Leu
900 905 910

Gly Ser Ala Ile Asn His Gly Gly Arg Thr Asn Gly Tyr Thr Val Pro
915 920 925

Asn Pro Arg Ala Gln Ala Glu Leu Ile Arg Glu Ala Met Asp Arg Ala
930 935 940

Gly Val Ser Ala Asp Glu Val Gly Cys Val Glu Ala His Gly Thr Gly
945 950 955 960

Thr Ala Leu Gly Asp Pro Val Glu Ile Glu Gly Leu Ala Gln Ala Phe
965 970 975

Ala Asp Arg Thr Asp Thr Ala Ala Pro Cys Ala Leu Ser Ser Val Lys
980 985 990

Ser Asn Ile Gly His Leu Glu Ala Ala Ala Gly Ile Ala Gly Leu Thr
995 1000 1005

Lys Leu Val Leu Gln Leu Arg His Gly Glu Leu Ala Pro Thr Leu
1010 1015 1020

His Ala Glu Val Pro Asn Pro Asp Ile Asp Phe Gly Ser Val Pro
1025 1030 1035

Phe Ala Leu Gln Thr Ala Ala Ala Pro Trp Pro Arg Thr Gly Gly
1040 1045 1050

Asn Ser Gly Arg Arg Ile Ala Gly Leu Ser Ser Phe Gly Ala Gly
1055 1060 1065

Gly Ala Asn Ala His Val Val Val Ala Glu Tyr Thr Gly Ala Pro
1070 1075 1080

Ala Ala Arg Thr Ser Ala Pro Ala Val Ala Asp Gly Ser Ala Ala
1085 1090 1095

Thr Ala Gly Ser Gly Arg Pro Val Leu Leu Pro Leu Ser Ala Arg
1100 1105 1110

Thr Pro Glu Asp Leu Arg Ala Arg Ala Val Gln Leu Ala Asp Trp
1115 1120 1125

Leu Asp Ser Arg Asp Ala Val Asp Leu Thr Ser Val Ala Ala Thr
1130 1135 1140

Leu Gln Thr Gly Arg Glu Ala Met Asp Glu Arg Leu Cys Cys Val
1145 1150 1155

Ala Ser Thr Pro Gly Glu Trp Arg Glu Gln Leu Arg Ala Phe Ala
1160 1165 1170

Asp Asp Pro Glu Arg Glu Gly Pro Trp His Arg Gly Arg Val Arg
1175 1180 1185

Ala Thr Gly Glu Ala Leu Ala Ala Leu Ala Glu Lys Asp Glu Leu
1190 1195 1200

Arg Ala Leu Val Gly Arg Trp Thr Ala Arg Gly Glu Trp Ala Glu
1205 1210 1215

Leu Ala Ala Phe Trp Ala Lys Gly Met Pro Leu Asp Trp Ser Arg
1220 1225 1230

Leu Tyr Ala Asp Gly Arg Val Pro Ala Arg Leu His Leu Pro Ala
1235 1240 1245

Tyr Pro Phe Ala Gly Arg Arg Tyr Trp Pro Gly Pro Ala Asp Val
1250 1255 1260

Arg Asn Thr Ala Asp Ala Gln Ala Pro Arg Thr Ser Thr Pro Ser
1265 1270 1275

Pro Ser Thr Leu Ser Thr Ser Thr Pro Gly Ala Ser Arg Pro Val
1280 1285 1290

Ala Val Ala Pro Val Ala Ala Ala Pro Ser Ala Glu Ser Tyr Ile
1295 1300 1305

Glu Arg Val Leu Leu Asp Ala Leu Gly Glu Ala Leu Gln Met Thr
1310 1315 1320

Pro Ala Glu Ile Asp Pro Arg Arg Pro Phe Ala Asp Tyr Gly Leu
1325 1330 1335

Asp Ser Ile Leu Gly Val His Leu Val Asn Val Leu Asn Glu Thr
1340 1345 1350

Leu Gly Thr Gly Leu Glu Thr Thr Asp Leu Phe Asp His Gly Thr
1355 1360 1365

Ala Glu Arg Leu Arg Ala Phe Leu Thr Glu Thr Tyr Gly Gly Thr
1370 1375 1380

Val Thr Val Pro Asp Gly Thr Gly Ala Ala Ala Glu Phe Val Pro
1385 1390 1395

Asp Ala Pro Val Pro Ala Arg Glu Ala Asp Asp Pro Val Ala Val
1400 1405 1410

Val Gly Met Ala Ala Arg Tyr Gly Asp Ala Glu Asp Pro Arg Ala
1415 1420 1425

Leu Trp Asp Arg Leu Leu Ala Gly Asp Asp Leu Val Glu Pro Val

1430 1435 1440

Thr Arg Trp Asp Leu Gly Pro Glu Val Thr Cys Arg Ala Gly Ser
1445 1450 1455

Phe Val Arg Gly Met Asp Arg Phe Asp Pro Val Phe Phe Ala Ile
1460 1465 1470

Ser Gly Val Glu Ala Ala His Met Asp Pro Gln Gln Arg Ile Phe
1475 1480 1485

Leu Glu Gln Cys Trp Asn Ala Leu Glu Asp Ala Gly Tyr Thr Gly
1490 1495 1500

Glu Arg Leu Arg Glu Arg Asn Cys Gly Val Tyr Val Gly Cys Tyr
1505 1510 1515

Ala Gly Asp Tyr Tyr Asp Ser Ile Gly Asp Arg Ala Pro Ala Gln
1520 1525 1530

Ala Leu Trp Gly Thr Met Gly Ser Val Val Ala Ser Arg Ile Ala
1535 1540 1545

Tyr Gln Leu Asp Leu Lys Gly Pro Ala Leu Thr Thr Asp Thr Ser
1550 1555 1560

Cys Ser Ser Ser Leu Val Ser Leu His Leu Ala Cys Arg Asp Leu
1565 1570 1575

Arg Thr Gly Ala Ala Asp Met Ala Ile Ala Gly Gly Val Phe Leu
1580 1585 1590

Gln Thr Thr Pro Arg Leu Tyr Glu Ala Ala Thr Arg Ala Gly Met
1595 1600 1605

Leu Ser Pro Thr Gly Arg Cys His Ser Phe Asp Ser Arg Ala Asp
1610 1615 1620

Gly Phe Val Pro Gly Glu Gly Ala Gly Ala Val Val Leu Lys Arg
1625 1630 1635

Leu Ser Asp Ala Leu Arg Asp Gly Asp His Val Tyr Gly Leu Val
1640 1645 1650

Arg Ala Thr Gly Val Asn Gln Asp Gly Thr Thr Asn Gly Ile Thr
1655 1660 1665

Ala Pro Ser Ala Ala Ser Gln Glu Ala Leu Leu Arg Glu Val His
1670 1675 1680

Ala Gly Val Ala Pro Gly Gly Val Gln Leu Val Glu Ala His Gly
1685 1690 1695

Thr Gly Thr Gln Leu Gly Asp Pro Ile Glu Phe Arg Ala Leu Ser
1700 1705 1710

Arg Val Phe Gly Asp Ala Pro Ala Gly Ser Val Val Leu Gly Ser
1715 1720 1725

Val Lys Thr Asn Leu Gly His Thr Gln Phe Ala Ala Gly Ile Ala
1730 1735 1740

Gly Val Leu Lys Ala Leu Leu Ala Leu Gln Glu Gln Arg Val Pro
1745 1750 1755

Pro Ser Leu His Phe Ala Glu Ala Asn Ala Arg Val Pro Leu Asp
1760 1765 1770

Gly Ser Pro Phe Thr Val Ala Thr Thr Ala Gln Pro Trp Pro Glu
1775 1780 1785

Pro Ala Glu Gly Pro Arg Arg Ala Ala Val Ser Ser Phe Gly Ala
1790 1795 1800

Ser Gly Thr Asn Ala His Val Val Leu Glu Glu His Pro Pro Val
1805 1810 1815

Arg Ala Thr Thr Gly Pro Glu Ser Ala Gly Gly Asp Gly Glu Ala
1820 1825 1830

Ala Phe Leu Leu Ser Ala Arg Thr Pro Ala Ala Leu Arg Ala Val
1835 1840 1845

Ala Glu Arg Leu Leu Ala Arg Ile Glu Arg Glu Pro Gly Leu Pro
1850 1855 1860

Ala Arg Gln Val Ala Tyr Ser Leu Ala Ala Gly Arg Arg His Phe
1865 1870 1875

Pro His Arg Leu Ala Val Val Ala Thr Gly Leu Pro Ala Leu Ala
1880 1885 1890

Ala Arg Leu Arg Ala Trp Leu Ala Asp Glu Gln Pro Gly Gly Glu
1895 1900 1905

Gly Thr Leu Leu His Gly Val Ala His Ala Gly Thr Arg Gln Ala
1910 1915 1920

Ala Leu Gly Gly Leu Ala Pro Ala Glu Leu Ala Ala Ala Tyr Val
1925 1930 1935

Gly Gly Ala Glu Gly Pro Phe Ala Glu Ser Phe Pro Ala Gly Ala
1940 1945 1950

Arg Arg Gln Val Pro Leu Pro Thr Tyr Pro Phe Glu Arg Gln Arg
1955 1960 1965

Tyr Trp Ala Glu Gly Thr Asp Gly His Ala Val Pro Ala Ala Ala
1970 1975 1980

Gly Thr Ser Ala Val Glu Pro Gly Gly Arg Arg Thr Ala Tyr Arg
1985 1990 1995

Thr Arg Leu Thr Gly Glu Glu Phe Phe Leu Ala Asp His Arg Val
2000 2005 2010

Gly Gly Arg Thr Val Leu Pro Gly Val Leu Thr Leu Glu Ala Val
2015 2020 2025

Arg Arg Ala Val Thr Ala Gly Asp Gly Gly Asp Gly Thr Gly Ile
2030 2035 2040

Gly Thr Gly Gly Gly Thr Gly Val Pro Thr Pro Leu Arg Leu Arg
2045 2050 2055

Asp Val Val Trp Pro Ala Pro Phe Pro Val Gly Ala Asp Gly Ala
2060 2065 2070

Glu Leu Arg Val Asp Leu Asp Gly Asp Ala Phe Ala Val Arg Gln
2075 2080 2085

Asp Gly Ser Ser Val His Ala Gln Gly Arg Trp Thr Met Val Pro
2090 2095 2100

Ala Pro Ala Ala Ser Thr Ser Leu Glu Thr Leu Arg Glu Arg Cys
2105 2110 2115

Ala Arg Arg Thr Leu Thr Arg Glu Gln Cys Arg Ala Ala Leu Glu
2120 2125 2130

Ala Val Gly Ile Arg His Gly Glu Arg Leu Arg Ala Ile Glu Gln
2135 2140 2145

Leu Ser Val Gly Asp Gly Glu Leu Leu Ala Arg Leu Val Leu Pro
2150 2155 2160

Ala Thr Val Glu Thr Gly Thr Gln Ala Thr Glu Thr Phe Gly Leu
2165 2170 2175

His Pro Ala Met Leu Asp Ser Ala Ile Gln Ala Val Val Gly Leu
2180 2185 2190

Tyr Gly Asp Glu Thr Gly Ala Leu Gly Glu Arg Pro Asp Ala Pro
2195 2200 2205

Ala Leu Pro Phe Ala Leu Asp Thr Ala Asp Val Leu Ala Pro Thr
2210 2215 2220

Thr Asp Arg Met Trp Ala His Leu Arg Trp Ala Asp Gly Tyr Ala
2225 2230 2235

Pro Gly Glu Ala Gly Glu Val Thr Lys Thr Asp Ile Asp Leu Tyr
2240 2245 2250

Asp Asp Ala Gly Arg Leu Cys Val Arg Leu Arg Gly Tyr Ala Ser
2255 2260 2265

Arg Arg Val Thr Pro Ala Ala Thr Gly Ser Ala Thr Ala Ala Pro
2270 2275 2280

Ala Gly Thr Asp Asp Ala Asp Ala Pro Arg Ala Gln Leu Leu Ala
2285 2290 2295

Pro Val Trp Asp Ala Gln Pro His Ala Asp Gly Pro Arg Ser Pro
2300 2305 2310

Glu Pro Gly Ala His Val Val Leu Leu Gly Gly Thr Pro Glu Glu
2315 2320 2325

Arg Asp Gly Leu Arg Arg Leu Val Ala Asp Val Thr Val Val Glu
2330 2335 2340

Pro Glu Arg His Ala Ser Ala Gly Glu Leu Ala Ala Leu Leu Pro

2345 2350 2355

Thr Gly Ala Glu His Val Val Trp Leu Ala Pro Arg Asp Ala Ser
2360 2365 2370

Pro Ala Ala Ser Ser Ala Glu Gly Pro Asp Gly Ala Leu Ala Val
2375 2380 2385

Phe Arg Leu Val Lys Ala Leu Leu Ala Asp Gly Ala Asp Ala Arg
2390 2395 2400

Glu Leu Ser Phe Thr Ala Val Thr Arg Gln Ala Arg Leu Leu Pro
2405 2410 2415

Gly Asp Ala Asp Cys Asp Pro Ala His Ala Gly Val His Gly Leu
2420 2425 2430

Leu Gly Thr Leu Ala Lys Glu Tyr Pro His Trp Arg Val Arg Gly
2435 2440 2445

Ala Asp Ile Glu Arg Asp Val Ser Val Pro Trp Pro Glu Leu Leu
2450 2455 2460

Ser Leu Pro Ala Asp Pro Arg Gly Glu Val Ser Ala Arg Arg His
2465 2470 2475

Gly Glu Trp Tyr Arg Gln Arg Leu Leu Glu Val Ala Leu Asp Ala
2480 2485 2490

Ser Gly Ala Ala Ser Phe Ser Ala Gly Ser Gln Ala Pro Asn Pro
2495 2500 2505

Gln Ala Pro Asn Ser Gln Ala Ser Gly Ala Arg Ser Ala Leu Arg
2510 2515 2520

Glu Pro Gly Gly Val Val Val Ala Ile Gly Gly Ala Gly Gly Ile
2525 2530 2535

Gly Thr Val Trp Thr Glu His Met Met Arg Arg His Gly Ala Arg
2540 2545 2550

Val Val Trp Ile Gly Arg Arg Pro Tyr Asp Glu Glu Ile Ala Ala
2555 2560 2565

Arg Gln Asp Arg Leu Ala Ala Cys Gly Pro Arg Pro Glu Tyr Val
2570 2575 2580

Arg Ala Asp Ala Thr Asp Ala Arg Ala Leu Arg Arg Ala Val Ala
2585 2590 2595

Glu Ile Glu Arg Arg His Gly Pro Val Arg Gly Val Leu His Thr
2600 2605 2610

Ala Ile Val Leu Gly Asp Gln Ser Leu Ala Arg Met Asp Glu Ala
2615 2620 2625

Ala Phe Arg Thr Thr Tyr Glu Ala Lys Ala Ala Val Ser Val Asn
2630 2635 2640

Met Ala Asp Ala Phe Ala Gly Gln Pro Leu Glu Phe Val Ala Phe
2645 2650 2655

Phe Ser Ser Met Gln Ala Phe Phe Lys Ala Pro Gly Gln Ala Asn
2660 2665 2670

Tyr Ala Ala Gly Cys Thr Phe Ser Asp Ala Trp Ala Glu Arg Leu
2675 2680 2685

Ser Thr Ala Leu Asp Cys Pro Val Lys Val Met Ser Trp Gly Tyr
2690 2695 2700

Trp Ala Gly Val Gly Ile Val Thr Ala Asp Gly Tyr Arg Gln Arg
2705 2710 2715

Met Ala Gln Leu Gly Leu Gly Ser Ile Glu Pro Asp Glu Gly Met
2720 2725 2730

Ala Ala Phe Asp Ala Leu Leu Ala Ser Pro Tyr Arg Gln Leu Ala
2735 2740 2745

Leu Leu Lys Ala Thr Asp Ser Arg Ser Ile Asp Gly Ile Tyr Gly
2750 2755 2760

Asp Asp Glu Leu Arg Gln Leu Pro Pro Ala Ala Pro Ala Leu Ala
2765 2770 2775

Asp Thr Leu Arg Thr Asp Arg Pro Asp Arg Asn Ala Glu Ile Arg
2780 2785 2790

Arg Leu Arg Glu Gln Ala Asp Gly His Ala Gly Val Met Tyr Asp
2795 2800 2805

Ala Leu Val Arg Val Thr Trp Ala Leu Leu Thr Ser Leu Gly Leu
2810 2815 2820

Phe Arg Asp Gly Arg Ala Ala Thr Ala Ala Glu Trp Arg Ala Val
2825 2830 2835

Gly Gly Ile Glu Glu Arg Tyr Gln Arg Trp Thr Glu His Thr Leu
2840 2845 2850

Glu Val Leu Thr Ala Ala Gly Arg Leu Arg Arg Ala Gly Glu Asp
2855 2860 2865

Arg Tyr Ala Ala Val Ala Pro Gly Ala Val Pro Ala Glu Asp Ala
2870 2875 2880

Trp Ala Glu Trp Asp Arg Ala Arg Glu Val Trp Leu Ala Asp Glu
2885 2890 2895

Ala Lys Gln Ala Gln Ala Val Leu Val Asp Thr Thr Leu Arg Glu
2900 2905 2910

Leu Thr Ala Ile Leu Thr Gly Arg Arg Ala Ala Thr Asp Val Met
2915 2920 2925

Phe Pro Gly Ser Ser Leu Arg Leu Val Glu Ala Val Tyr Lys Asn
2930 2935 2940

Asn Pro Val Ala Asp Tyr Phe Asn Glu Val Leu Ala Asp Thr Leu
2945 2950 2955

Val Ala Tyr Leu Glu His Arg Leu Arg Gln Asp Pro Ser Ala Arg
2960 2965 2970

Leu Arg Ile Leu Glu Ile Gly Ala Gly Thr Gly Gly Thr Ser Ser
2975 2980 2985

Val Val Phe Arg Arg Leu Arg Pro Leu Ala Gly His Ile Glu Thr
2990 2995 3000

Tyr Thr Tyr Thr Asp Ile Ser Lys Ala Phe Leu Leu His Ala Arg
3005 3010 3015

Arg Ala Tyr Gly Glu Ile Ala Pro Tyr Leu Asp Gly Gln Leu Phe
3020 3025 3030

Asp Ala Glu Lys Pro Leu Ala Gly Gln Pro Val Ala Val Gly Gly
3035 3040 3045

His Asp Val Val Ile Ala Thr Asn Val Leu His Ala Thr Gly Asn
3050 3055 3060

Ile Arg Asn Thr Leu Arg Asn Ala Lys Ala Ala Val Arg Ala Asn
3065 3070 3075

Gly Leu Leu Leu Leu Asn Glu Leu Ser Asp Asn Ile Leu Phe Ser
3080 3085 3090

His Leu Thr Phe Gly Leu Leu Asp Gly Trp Trp Leu Tyr Asp Asp
3095 3100 3105

Pro Ala Pro Arg Ile Pro Gly Ser Pro Gly Leu Thr Pro Gln Ser
3110 3115 3120

Trp Arg Arg Val Leu Asp Glu Val Gly Phe Arg Gly Ser Phe Val
3125 3130 3135

Ala Ala Glu Gly Ala Asp Asp Leu Gly Gln Gln Val Ile Val Ala
3140 3145 3150

Glu Ser Asp Gly Ala Val Arg Gln Pro Arg Pro Gly Gly Val Ser
3155 3160 3165

Ala Phe Arg Gly Ser Leu Pro Glu Ala Arg Pro Ala Gln Pro Thr
3170 3175 3180

Gly Gly Ala Gly His Leu Ala Val Pro Ala Glu His Gly Ser Ala
3185 3190 3195

Pro Ala Val Thr Val Pro Val Thr Ala Ala Ser Ala Ser Ser Ala
3200 3205 3210

Pro Gly Ser Ala Pro Ala Ala Val Pro Ser Gly Asp Pro Ser Gly
3215 3220 3225

Asp Gly Ser Met Ala Ala Arg Val Ala Gly Pro Ala Arg Asp Leu
3230 3235 3240

Phe Arg Gly Leu Val Ala Asp Val Leu Gln Leu Pro Val Gly Asp
3245 3250 3255

Ile Arg Ala Asp Val Pro Phe Glu Arg Tyr Gly Ile Asp Ser Ile

3260 3265 3270

Leu Val Val Gln Leu Thr Asp Ala Val Arg Lys Val Leu Asp Gly
3275 3280 3285

Val Gly Ser Thr Leu Phe Phe Glu Val Ser Thr Val Asp Gly Leu
3290 3295 3300

Val Glu His Phe Leu Arg Thr Arg Pro Asp Glu Leu Ala Ala Leu
3305 3310 3315

Val Gly Val Ser Ala Ala Glu His Pro Glu Pro Ala Ala Glu Ala
3320 3325 3330

Ala Ala Pro Glu Ala Val Thr Glu Glu Pro Ala Ala Ser Val Pro
3335 3340 3345

Ala Pro Ala Pro Val Ala Ala Pro Val Ser Val Pro Val Pro Ala
3350 3355 3360

Ala Pro Gly Glu Asp Val Pro Val Ala Val Val Gly Met Ala Gly
3365 3370 3375

Arg Tyr Pro Gly Ala Ala Asp Leu Asp Ala Phe Trp Glu Asn Leu
3380 3385 3390

Leu Ala Gly Arg Asp Cys Val Thr Glu Ile Pro Asp Gly Arg Trp
3395 3400 3405

Asp His Gly Arg Tyr Tyr Asp Glu Arg Arg Gly Val Pro Gly Arg
3410 3415 3420

Thr Tyr Ser Lys Trp Gly Gly Phe Leu Asp Gly Val Asp Glu Phe
3425 3430 3435

Asp Ser Leu Phe Phe Gly Ile Ser Pro Lys Ala Ala Ser Thr Met
3440 3445 3450

Asp Pro Gln Glu Arg Leu Phe Leu Gln Cys Ala Trp Thr Ala Leu
3455 3460 3465

Glu Asp Ala Gly His Thr Arg Ala Ser Leu Arg Ser Ala Ser Arg
3470 3475 3480

Ala Arg Leu Pro Glu Asp Ala Gly Asp Ile Gly Val Phe Val Gly
3485 3490 3495

Ala Met Tyr Ser Glu Tyr Gln Leu Tyr Gly Ala Glu Gln Gly Val
3500 3505 3510

Arg Gly Glu Pro Val Val Val Pro Gly Ser Leu Ala Ser Ile Ala
3515 3520 3525

Asn Arg Leu Ser Tyr Phe Leu Asp Ala Ser Gly Pro Ser Val Ala
3530 3535 3540

Val Asp Thr Met Cys Ala Ser Ala Leu Ser Ala Val His Leu Ala
3545 3550 3555

Cys Ala Ala Ile Arg Arg Gly Glu Cys Ala Ser Ala Val Ala Gly
3560 3565 3570

Gly Val Asn Leu Ser Leu His Pro Ser Lys Tyr Leu Met Ile Gly
3575 3580 3585

Glu Gly Gln Phe Ala Ser Ser Asp Gly Arg Cys Arg Ser Phe Gly
3590 3595 3600

Ala Asp Gly Asp Gly Tyr Val Pro Gly Glu Gly Val Gly Ala Val
3605 3610 3615

Leu Leu Arg Pro Leu Ala Asp Ala Val Ala Asp Gly Asp Arg Val
3620 3625 3630

Leu Gly Val Ile Arg Gly Ser Ala Val Asn His Gly Gly His Thr
3635 3640 3645

His Gly Phe Thr Val Pro Asn Pro Leu Ala Gln Ala Ser Val Ile
3650 3655 3660

Arg Gly Ala Trp Arg Arg Ser Gly Val Asp Pro Arg Asp Ile Gly
3665 3670 3675

Cys Ile Glu Ala His Gly Thr Gly Thr Ala Leu Gly Asp Pro Val
3680 3685 3690

Glu Ile Ala Gly Leu Asn Ala Ala Phe Gly Glu Phe Thr Ser Glu
3695 3700 3705

Arg Thr Phe Cys Ser Leu Gly Ser Ala Lys Ser Asn Ile Gly His
3710 3715 3720

Leu Glu Ser Ala Ala Gly Val Ala Gly Leu Ala Lys Met Leu Leu
3725 3730 3735

Gln Met Arg His Gly Thr Leu Val Pro Ser Leu His Ala Glu Arg
3740 3745 3750

Thr Asn Pro Glu Ile Asp Phe Ala Ala Thr Pro Phe Val Leu Gln
3755 3760 3765

Arg Glu Ala Ala Pro Trp Pro Arg Arg Glu Gly Arg Pro Arg Leu
3770 3775 3780

Gly Gly Ile Ser Ala Phe Gly Ala Gly Gly Ser Asn Ala His Leu
3785 3790 3795

Leu Val Glu Glu Tyr Val Pro Thr Ala Ala Pro Pro Arg Arg Ala
3800 3805 3810

Ala Pro Gly Pro Val Leu Ala Val Leu Ser Ala Arg Asp Gly Glu
3815 3820 3825

Arg Leu Arg Glu Tyr Ala Gly Lys Leu Arg Asp Ala Leu Arg Ser
3830 3835 3840

Gly Gln Trp Thr Asp Glu Asp Leu Pro Asp Ile Ala Tyr Thr Leu
3845 3850 3855

Gln Val Gly Arg Glu Ala Met Ser Ala Arg Phe Ala Ala Glu Val
3860 3865 3870

Ser Thr Leu Ala Gly Leu Met Asp Ala Leu Asp Ala Cys Ala Arg
3875 3880 3885

Gly Ala Ala Leu Pro Pro Gly Ala Arg Leu Arg Thr Asp Gly Gly
3890 3895 3900

Arg Gly Gly Pro Val Gln Asp Leu Ala Asp Asp Glu Asp Phe Arg
3905 3910 3915

Glu Thr Val Val Arg Trp Leu Arg Arg Gly Lys Leu Ala Pro Leu
3920 3925 3930

Ala Glu Ala Trp Thr Gly Gly Leu Asp Val Asp Trp Ala Arg Gly
3935 3940 3945

His Gly Thr Gly Glu Asp Arg Pro Arg Lys Val Gly Leu Pro Gly
3950 3955 3960

Tyr Pro Phe Ala Arg Glu Arg Tyr Trp Trp Asn Asp Gly Leu Ala
3965 3970 3975

Glu Ala Gly Gly Glu Gly Ala Asp Gly Leu Gly Asp Glu Gly Ala
3980 3985 3990

Ala Gly Gly Thr Ala Gly Ser Gly Asn Gly Ser Gly Pro Arg Ser
3995 4000 4005

Ala Arg Thr Asp Gly Thr Arg Pro Gly Glu Leu Pro Pro Gly Asp
4010 4015 4020

Leu Thr Leu His Pro Val Trp Glu Pro Val His Ala Ala Gly Gly
4025 4030 4035

Gly Ala Asp Ala Pro Phe Pro Gln Pro Ala Asp Arg Val Val Ala
4040 4045 4050

Val Gly Leu Ala Pro Glu Ala Arg Ala Ala Leu Glu Ala Tyr Gly
4055 4060 4065

Thr Arg Val Val Thr Leu Pro Ala Pro Arg Asp Gly Gly Arg Ser
4070 4075 4080

Val Ala Asp Val Arg Arg Glu Leu Glu Thr Ala Gly Pro Phe Asp
4085 4090 4095

His Val Val Val Glu Cys Pro Thr Pro Ala Ala Gln Gly Ala Arg
4100 4105 4110

Gln Arg Val Glu Ala Gln Arg Ala Ser Val Arg Gly Leu Phe Arg
4115 4120 4125

Leu Leu Gln Ala Leu Ser Ala Leu Arg Ala Asp Glu Pro Arg Thr
4130 4135 4140

Gly Leu Thr Leu Val Thr Arg Asp Ala Phe Asp Pro Asp Arg Thr
4145 4150 4155

Gly Gly Ala Asp Pro Ala Gln Ala Ala Leu His Gly Leu Val Gly
4160 4165 4170

Gly Leu Ala Lys Glu Gln Pro Tyr Trp Arg Val Arg Ala Val Asp

4175 4180 4185

Leu Ala Glu Gly Glu Pro Phe Val Pro Glu Glu Ile Cys Ala Leu
4190 4195 4200

Pro Ala Asp Arg Arg Ala His Pro Leu Val Arg Arg Gly Gly Gln
4205 4210 4215

Trp Leu Ser Arg Arg Leu Leu Pro Val Gly Asp Val Arg Pro Gly
4220 4225 4230

Thr Pro Asp Asp Gly Pro Arg Thr Ala Val Asp Gly Thr Asp Ala
4235 4240 4245

Ala Pro Gln Ala Val Ser Val Pro Ser Gly Ser Val Ser Ser Val
4250 4255 4260

Ser Val Pro Ser Gly Gly Phe Arg Gly Asp Gly Val Tyr Val Leu
4265 4270 4275

Ile Gly Gly Ala Gly Asp Leu Gly Thr Val Leu Thr Glu His Leu
4280 4285 4290

Leu Arg Arg Tyr Asp Ala Arg Val Val Trp Val Gly Arg Arg Ala
4295 4300 4305

Glu Asp Asp Ala Val Arg Ala Ala Ala Ala Arg Val Ala Ala Ala
4310 4315 4320

Thr Gly Gly Glu Ala Pro Val Tyr Leu Ser Ala Asp Ala Arg Asp
4325 4330 4335

Pro Gly Ala Leu Ala Arg Val Arg Asp Glu Val Leu Arg Arg Tyr
4340 4345 4350

Gly Arg Ile Asp Gly Leu Val His Leu Ala Met Val Phe Ser His
4355 4360 4365

Thr Leu Leu Ala Glu Leu Pro Glu Glu Asp Leu Asn Ala Thr Leu
4370 4375 4380

Ala Ala Lys Ala Asp Pro Thr Glu His Phe Ala Asp Val Phe Ala
4385 4390 4395

Gly Gln Arg Leu Asp Phe Val Leu Leu Val Ser Ser Leu Val Ser
4400 4405 4410

Phe Ile Arg Asn Ser His Gln Ala His Tyr Ala Ala Ala Cys Ala
4415 4420 4425

Tyr Glu Asp Ala Arg Ala Pro Gly Leu Gly Arg Ala Leu Gly Cys
4430 4435 4440

Pro Val Lys Val Val Asn Trp Gly Tyr Trp Gly Asn Val Ser Asp
4445 4450 4455

Glu Val Leu Arg Gly Val Thr Glu Met Gly Leu Ala Pro Ile Glu
4460 4465 4470

Pro Ala Ser Ala Met Ala Ala Val Glu Glu Leu Leu Thr Gly Pro
4475 4480 4485

Leu Asp Gln Ile Gly Phe Met Arg Leu Gly Arg Pro Leu Pro Val
4490 4495 4500

Glu Gly Val Leu Ala Gly Glu Thr Leu Ser Gly His Pro Tyr Ala
4505 4510 4515

Ala Val Ser Arg Thr Ala Ala Glu Pro Ala Pro Val Pro Val Pro
4520 4525 4530

Ala Ala Leu Ala Glu His His Ala Gly Pro Val Pro Gly Glu Ile
4535 4540 4545

Asp Ala Leu Leu Cys Arg Cys Val Ala Ala Thr Leu Arg Arg Ala
4550 4555 4560

Gly Leu Arg Arg Pro Ala Asp Gly Phe Ala Ser Gly Ser Gly Ser
4565 4570 4575

Gly Ser Gly Ala Gly Ser Ala Gly Val Arg Val Asp Glu Arg Phe
4580 4585 4590

Asp Gly Trp Phe Ala Ala Thr Val Arg Thr Leu Arg Glu Tyr Gly
4595 4600 4605

Leu Val Asp Ser Arg Gly Asp Trp Ser Glu Arg Ala Pro Gly Ala
4610 4615 4620

Gly Asp Ala Ala Ala Cys Leu Ala Glu Trp Glu Arg Ala Ala Glu
4625 4630 4635

Arg Trp Ala Ser Ala His Ala Asp Leu Arg Ala Pro Thr Gly Leu
4640 4645 4650

Leu Gly Arg Thr Leu Pro Ala Leu Ala Asp Ile Leu Arg Gly Arg
4655 4660 4665

Ile Pro Ala Thr Asp Val Leu Phe Pro Glu Gly Ser Phe Ser Leu
4670 4675 4680

Val Glu Gly Val Tyr Arg Asp Asn Ala Val Ala Ala His Phe Asn
4685 4690 4695

Ala Val Leu Ala Ala Gln Val Thr Ala Phe Leu His Gly Arg Arg
4700 4705 4710

Ala Ala Asp Pro Ala Ala Arg Leu Arg Val Leu Glu Ile Gly Ala
4715 4720 4725

Gly Thr Gly Gly Thr Thr Ala Pro Val Leu Glu Gln Leu Glu Cys
4730 4735 4740

Ala Gly Leu Glu Leu Ala Glu Tyr Cys Phe Thr Asp Leu Ser Leu
4745 4750 4755

Ala Phe Leu Gln Arg Ala Glu Asp Ala Phe Gly Pro Gly Arg Ala
4760 4765 4770

His Phe Ala Cys Arg Thr Leu Asp Val Ser Arg Ala Pro Arg Thr
4775 4780 4785

Gln Gly Phe Asp Ala Gly Ala Tyr Asp Val Val Ile Ala Ala Asn
4790 4795 4800

Val Leu His Ala Thr Asp Asp Val Arg Thr Ala Leu Arg His Ala
4805 4810 4815

Lys Ser Leu Leu Arg Gly Gly Gly Met Leu Ala Leu Asn Glu Ile
4820 4825 4830

Ser Gly Phe Tyr Leu Val Asn His Leu Thr Phe Gly Leu Leu Asp
4835 4840 4845

Gly Trp Trp Leu Tyr Gly Asp Ala Glu Leu Arg Ala Pro Gly Ser
4850 4855 4860

Pro Ala Leu Pro Pro Glu Ser Trp Arg Arg Val Leu Thr Gln Glu
4865 4870 4875

Gly Phe Thr Gly Val Ala Asp Pro Ala Arg Asp Ala Arg Ala Leu
4880 4885 4890

Gly Gln Gln Val Val Ile Ala His Ser Asp Gly Leu Ala Arg Gly
4895 4900 4905

Pro Val Thr Asp Ala Ala Pro Ala Ala Pro Ala Ala Pro Ala Ala
4910 4915 4920

Val Ala Arg Pro Glu Thr Asn Thr Ala Val Ser Ala Ala Pro Asn
4925 4930 4935

Met Ala Val Ser Ala Ala Ser Ser Ala Ala Gly Gly Pro Gln Thr
4940 4945 4950

Ser Gly Gly Pro Asp Val Arg Val Val Ala Asp Val Val Glu Thr
4955 4960 4965

Glu Leu Ala Asp Ala Leu Arg Leu Pro Ala Glu Arg Ile Asp Arg
4970 4975 4980

Ala Gly Ala Phe Ala Asp Val Gly Leu Asp Ser Ile Val Gly Ala
4985 4990 4995

Arg Phe Val Arg Arg Leu Asn Glu Glu Leu Gly Leu Asp Leu Pro
5000 5005 5010

Thr Thr Val Ile Phe Asp Tyr Arg Ser Val Asp Glu Leu Ala Ala
5015 5020 5025

His Ile Val Glu Asp His Arg Pro Thr Ser Pro Ala Pro Gly Gly
5030 5035 5040

Thr Gly Ala Ala Thr Ala Gln Glu Pro Pro Ala Glu Arg Glu Ser
5045 5050 5055

Gly Arg Ala Pro Glu Arg Glu His Gly Pro Val Val Ala Pro Asp
5060 5065 5070

Val Thr Val Pro Asp Ala Thr Glu Pro Gly Ser Ala Pro Tyr Gly
5075 5080 5085

Arg Glu Pro Ile Ala Val Val Gly Val Ser Gly Arg Phe Ala Gly

5090 5095 5100

Ser Asp Asp Leu Asp Ala Leu Trp Arg His Leu Ala Ala Gly Asp
5105 5110 5115

Asp Leu Val Gly Pro Ile Asp Arg Trp Asp Leu Ser Ala Tyr Gly
5120 5125 5130

Glu Asp Glu Leu Thr Cys Arg Ser Gly Ser Phe Leu Asp Gly Ile
5135 5140 5145

Asp Arg Phe Asp Ala Arg Phe Phe Lys Leu Ser Gly Arg Glu Ala
5150 5155 5160

Ala Tyr Thr Asp Pro Gln Gln Arg Leu Phe Leu Glu Gln Ala Trp
5165 5170 5175

Thr Ala Leu Glu Asp Ala Gly His Gly Gly Ala Ser Thr Asp Gly
5180 5185 5190

Met Arg Cys Gly Val Tyr Val Gly Cys Thr Gly Gly Asp Tyr Lys
5195 5200 5205

Asp His Phe Glu Asp Ala Pro Pro Ala Gln Ala Val Trp Gly Asn
5210 5215 5220

Ala Pro Ser Ile Val Pro Ala Arg Ile Ala Tyr His Leu Asn Leu
5225 5230 5235

Gln Gly Pro Ala Ile Ala Val Asp Thr Ala Cys Ser Ser Ser Leu
5240 5245 5250

Val Ala Val His Leu Ala Cys Gln Gly Leu Trp Ser Gly Glu Thr
5255 5260 5265

Glu Met Ala Val Ala Gly Gly Val Ser Val Gln Thr Thr Pro Ala
5270 5275 5280

Thr Tyr Leu Ser Ala Ser Arg Ala Gly Met Leu Ser Pro Thr Gly
5285 5290 5295

Arg Cys His Thr Phe Asp Ala Ala Ala Asp Gly Phe Val Pro Gly
5300 5305 5310

Glu Gly Val Gly Val Val Val Leu Arg Arg Leu Ser Asp Ala Leu
5315 5320 5325

Arg Asp Gly Asp His Val His Ala Val Ile Arg Gly Ser Gly Val
5330 5335 5340

Asn Gln Asp Gly Ala Thr Asn Gly Ile Thr Ala Pro Ser Ala Leu
5345 5350 5355

Ser Gln Glu Arg Leu Leu Arg Gln Val Tyr Glu Asp Phe Ala Ile
5360 5365 5370

Asp Pro Ser Glu Ile Gly Met Val Glu Ala His Gly Thr Gly Thr
5375 5380 5385

Gln Leu Gly Asp Pro Ile Glu Cys His Ala Leu Arg Arg Val Phe
5390 5395 5400

Glu Gly Ser Asp Val Pro Gly Gly Cys Ala Leu Gly Ser Ile Lys
5405 5410 5415

Thr Asn Leu Gly His Thr Thr Ser Ala Ala Gly Val Ala Gly Leu
5420 5425 5430

Leu Lys Ile Val Leu Ser Leu Arg His Arg Gln Ile Pro Pro Ser
5435 5440 5445

Leu His Tyr Arg Asp Arg Asn Pro Glu Ile Arg Leu Glu Gly Gly
5450 5455 5460

Pro Leu Tyr Val Asn Thr Ser Leu Arg Pro Trp Glu Pro Asn Ala
5465 5470 5475

Gly Gly Ser Arg Ala Ala Ala Leu Ser Ser Phe Gly Phe Ser Gly
5480 5485 5490

Thr Asn Ser His Leu Val Val Glu Glu Ala Pro Ala Arg Pro Gly
5495 5500 5505

Arg Ser Pro Leu Ser Gly Ala Ala Ala Val Glu Glu Pro Gly Leu
5510 5515 5520

Pro Arg Val Phe Pro Leu Ser Ala Pro Gln Pro Ala Ala Leu Arg
5525 5530 5535

Glu Arg Val Arg Asp Leu Ala Val His Leu Arg Ser Thr Pro Asp
5540 5545 5550

Ala Val Leu Val Asp Val Ser His Thr Leu Ala Thr Gly Arg Ala
5555 5560 5565

His Phe Ala His Arg Ala Ala Phe Val Ala Arg Thr Arg Glu Glu
5570 5575 5580

Leu Ile Gly Gln Leu Asp Asp Trp Leu Asp Gly Glu Ala Gly Asp
5585 5590 5595

Ala Gly Lys Ala Ala Lys Thr Gly Glu Ala Ala Lys Thr Gly Asp
5600 5605 5610

Val Gly Glu Ala Gly Gly Ala Gly Pro Glu Glu Leu Ala Arg Asp
5615 5620 5625

Arg Tyr Leu Ala Gly Glu Pro Ala Asp Phe Ala Ala Leu Phe Ala
5630 5635 5640

Gly Ser Gly Ala Arg Arg Thr Pro Leu Pro Thr Tyr Pro Phe Gln
5645 5650 5655

Arg Arg Ser His Trp Val Arg Gly Gly Ala Pro Gly Ser Ala Pro
5660 5665 5670

Asp Ala Ala Gly Ser Gly Thr Ser Thr Thr Ser Gly Thr Pro Ala
5675 5680 5685

Leu Arg Thr Asp Ala Arg Glu Lys Gly Arg Gly Ala Ala Arg Ala
5690 5695 5700

Glu Asp Asp Ala Val Ala Val Val Gly Leu Ser Ala Arg Phe Ala
5705 5710 5715

Gln Ser Pro Asp Ala Glu Ala Leu Trp Ala His Leu Ala Ala Gly
5720 5725 5730

Asp Asp Leu Val Gly Glu Val Thr Arg Trp Asp Leu Ser Gln Ile
5735 5740 5745

Ser Gly Gly Arg Thr Glu His Gly Ser Phe Val Glu Asp Ile Ala
5750 5755 5760

Arg Phe Asp Ala Leu Tyr Phe Gly Val Ser Gly Asn Glu Ala Thr
5765 5770 5775

Tyr Ala Asp Pro Gln Gln Arg Ile Tyr Leu Glu Glu Cys Trp His
5780 5785 5790

Ala Leu Glu Asp Ala Gly Tyr Ala Gly Glu Arg Leu Asp Gly Arg
5795 5800 5805

Gly Cys Gly Val Tyr Val Gly Ala Tyr Pro Gly Asp Tyr His Glu
5810 5815 5820

Leu Ile Gly Ala Asp Arg Pro Pro Gln Thr Met Trp Gly Asn Met
5825 5830 5835

Ala Ser Val Ile Ala Ser Arg Ile Ser Tyr Phe Leu Asp Leu Asp
5840 5845 5850

Gly Pro Ala Met Ser Val Asp Ser Ala Cys Ser Ser Ser Leu Val
5855 5860 5865

Ala Ile His Thr Ala Cys Gln Asp Leu Arg Leu Gly Thr Thr Ser
5870 5875 5880

Met Ala Leu Ala Gly Gly Val Phe Ile Gln Ala Thr Pro Arg Leu
5885 5890 5895

Tyr Gln Tyr Ser Gly Lys Ala Arg Met Leu Ser Ala Thr Gly Arg
5900 5905 5910

Cys His Ala Phe Asp Ala Ala Ala Asp Gly Phe Val Pro Gly Glu
5915 5920 5925

Gly Ala Gly Val Val Val Leu Lys Arg Leu Ser Asp Ala Leu Arg
5930 5935 5940

Asp Gly Asp Arg Val Tyr Gly Val Ile Arg Ser Ser Gly Val Asn
5945 5950 5955

Gln Asp Gly Thr Thr Asn Gly Ile Thr Ala Pro Ser Gly Ala Ala
5960 5965 5970

Gln Glu Asn Leu Val Arg Asp Val Tyr Glu Arg Ala Gly Val Ala
5975 5980 5985

Pro Ser Gly Ile Gln Leu Ile Glu Ala His Gly Thr Gly Thr Pro
5990 5995 6000

Leu Gly Asp Pro Ile Glu Phe Glu Ala Leu Arg Ala Val Phe Ala

6005 6010 6015

Asp Ala Pro Thr Gly Gly Cys Ala Leu Gly Thr Ile Lys Ser Asn
6020 6025 6030

Val Gly His Thr Gln Phe Thr Ala Gly Val Ala Gly Val Leu Lys
6035 6040 6045

Val Leu Leu Ala Leu Asp His Glu Gln Leu Pro Pro Ser Leu His
6050 6055 6060

Phe Thr Arg Pro Asn Pro Ala Ile Asp Leu Ala Asn Ser Pro Phe
6065 6070 6075

His Val Asn Thr Glu Leu Leu Pro Trp Arg Ala Pro Ala Asp Gly
6080 6085 6090

Pro Arg Arg Ala Gly Val Ser Ser Phe Gly Ala Ala Gly Thr Asn
6095 6100 6105

Ala His Val Leu Ile Glu Gln Ala Pro Ser Asp Ala Ala Ala Arg
6110 6115 6120

Ala Arg Arg His Gly Arg Ala Gln Trp Leu Leu Val Leu Ser Gly
6125 6130 6135

Gln Asp Gly Thr Ala Leu Arg Ala Gln Ala Glu Arg Met Leu Asp
6140 6145 6150

His Val Glu Arg His Pro Asp Leu Asp Leu Gly Asp Thr Ala Trp
6155 6160 6165

Thr Leu Ala Thr Gly Arg Arg His Ser Ala His Arg Leu Ala Cys
6170 6175 6180

Val Ala Ala Asp Arg Glu Gln Trp Thr Ala Ala Leu Arg Gly Trp
6185 6190 6195

Leu Arg Asp Gly Arg Ala Glu Gly Val Trp Thr Gly Glu Ala Asp
6200 6205 6210

Glu Ser Pro Arg Ser Gly His Ser Gly Glu Ser Gly Glu Gly Ser
6215 6220 6225

Gly Glu Pro Ala Arg Ala Glu Ala Leu Met Ala Glu His Asp Arg
6230 6235 6240

Pro Gly Asn Leu Ala Ala Leu Ala Glu Leu Tyr Val Arg Gly Glu
6245 6250 6255

Val Ala Arg Phe Ala Pro Leu Tyr Ala Asp Gly Asp Phe Arg Ile
6260 6265 6270

Val Ser Leu Pro Gly Tyr Pro Phe Gly Gly Glu Arg Tyr Trp Thr
6275 6280 6285

Gly Pro Leu Pro Gly Asp Thr Pro Asp Gly Thr Asp Gly Thr Asp
6290 6295 6300

Gly Thr Tyr Gly Thr Asp Gly Ile Ser Glu Ser Gly Gly Glu Ser
6305 6310 6315

Arg Pro Ser Ala Glu Pro Arg Pro Tyr Ala Gly Ala Leu Ala Leu
6320 6325 6330

Thr Gly Glu Glu Phe Phe Leu Asp Asp His Arg Val Gly Gly Val
6335 6340 6345

Pro Val Leu Pro Gly Val Ala Tyr Leu Glu Leu Ala His Ala Ala
6350 6355 6360

Ala Thr Ala Gln Gly Gly Leu Ala Pro Gly Gly Val Leu Leu Arg
6365 6370 6375

Asn Val Val Trp Ser Arg Pro Ala Arg Val Thr Glu Pro Leu Ser
6380 6385 6390

Val Glu Thr Val Leu Glu Pro Arg Ala Ala Asp Gly Thr Phe Gly
6395 6400 6405

Tyr Glu Ile Ala Thr Val Arg Asp Gly Ala Arg Arg Leu Val His
6410 6415 6420

Gly Arg Gly Arg Ile Glu Pro Arg Pro Gly Gly Ala Pro Ala Arg
6425 6430 6435

Leu Gly Leu Ala Ala Leu Arg Glu Arg Cys Asp Val Arg Ser Leu
6440 6445 6450

Asp His Ala Glu Cys Tyr Ala Leu Leu Gly Ala Thr Gly Met Ser
6455 6460 6465

Tyr Gly Ala Ala Met Arg Gly Leu Glu Glu Leu His Val Gly Arg
6470 6475 6480

Gly Leu Ala Leu Gly Arg Leu Arg Val Pro Arg Glu Ala Arg Asp
6485 6490 6495

Gly Arg Pro Trp Thr Leu His Pro Ala Leu Leu Asp Ala Ala Leu
6500 6505 6510

Gln Ala Thr Val Gly Leu Ala Leu Asp Gly Glu Ser Asp Gly Leu
6515 6520 6525

Thr Ala Ala Leu Pro Phe Ala Val Glu Gln Val Gln Val Leu Ala
6530 6535 6540

Ala Ser Pro Glu Ser Gly Trp Ala Val Ala Arg Pro Ala Asp Gly
6545 6550 6555

Ala Ala Glu Gly Pro Val Arg Arg Met Asp Val Glu Ile Cys Asp
6560 6565 6570

Asp Glu Gly Thr Val Cys Val Arg Leu Leu Gly Phe Ser Thr Arg
6575 6580 6585

Glu Leu Pro Gly Ala Thr Ala Ser Val Thr Thr Gly Ala Thr Thr
6590 6595 6600

Gly Ala Gly Ser Gly Ala Gly Ser Ala Ala Ala Ser Pro Ala Pro
6605 6610 6615

Ala Ala Ala Asp Pro Gly Ala Pro Ala Asp Gly Ser Leu Val Phe
6620 6625 6630

Ala Arg Pro Val Trp Arg Ala Val Pro Ser Ala Asp Val Arg Glu
6635 6640 6645

Glu Arg Pro Ala Pro Ser Pro Ala Pro Tyr Arg Glu Ile Leu Leu
6650 6655 6660

Ala Gly Pro Glu Ser Val Asp Ala Ala Glu Val Arg Lys Arg Ser
6665 6670 6675

Gly Val Pro Cys Ser Ala Leu Pro Gly Gly Ala Asp Leu Pro Glu
6680 6685 6690

Arg Tyr Thr Arg Gln Ala Gln Ala Leu Leu Ala Lys Val Gln Gln
6695 6700 6705

Leu Leu Pro Arg Val Arg Glu Glu Arg Val Leu Leu Gln Val Ala
6710 6715 6720

Val Pro Ala His Gly Glu Gly Arg Leu Phe Ala Gly Leu Ala Gly
6725 6730 6735

Leu Leu Arg Thr Ala Cys Ala Glu His Pro Gly Leu Ala Ala Gln
6740 6745 6750

Leu Val Glu Thr Asp Ala Ala Asp Ala Ala Thr Leu Cys Ala His
6755 6760 6765

Leu Asp Ala Glu Ala Ala Gln Pro Gly Val Ala Thr Val Arg Arg
6770 6775 6780

Thr Gly Gly Glu Arg Leu Val Arg Gln Trp His Gly Phe Arg Pro
6785 6790 6795

Glu Arg Gly Asp Gln Pro Trp Lys Pro Gly Gly Val His Leu Val
6800 6805 6810

Thr Gly Gly Ala Gly Gly Leu Gly Ala Leu Phe Ala Arg Arg Ile
6815 6820 6825

Ala Arg Thr Ala Pro Gly Ser Val Leu Val Leu Cys Gly Arg Ser
6830 6835 6840

Pro Glu Gly Ala Ala Gln Arg Glu Leu Leu Gly Glu Leu Arg Glu
6845 6850 6855

Ser Gly Ala Ala His Ala Glu Tyr His Ser Leu Asp Val Gly Arg
6860 6865 6870

Arg Ala Asp Val Val Arg Leu Val Arg Gln Val Val Asp Arg His
6875 6880 6885

Gly Arg Leu Asp Gly Val Ile His Ser Ala Gly Val Leu Arg Asp
6890 6895 6900

Gly Phe Val Ala His Lys Thr Pro Glu Tyr Leu Gly Glu Val Phe
6905 6910 6915

Ala Pro Lys Ala Gly Gly Val Val His Leu Asp Glu Ala Thr Ala

6920 6925 6930

Ala Leu Glu Leu Asp Phe Phe Leu Val Phe Ser Ser Met Ser Val
6935 6940 6945

Leu Gly Asn Pro Gly Gln Ala Asp Tyr Ala Ala Ala Asn Ala Phe
6950 6955 6960

Leu Asp Ala Tyr Val Ala His Arg Ala Gly Leu Ala Asp Arg Gly
6965 6970 6975

Glu Arg His Gly Arg Ser Leu Ser Val Gly Trp Pro Leu Trp Ala
6980 6985 6990

Asp Gly Gly Met His Val Asp Ala Ala Thr Glu Arg Arg Ile His
6995 7000 7005

Gln Ser Ser Gly Met Arg Pro Leu Arg Ala Arg Glu Gly Phe Glu
7010 7015 7020

Ala Leu Glu Arg Leu Tyr Gly Ser Gly Leu Pro His Ala Leu Thr
7025 7030 7035

Ala Phe Gly Asp Arg Glu Arg Ile Ala Ser Val Leu Leu Asp Gly
7040 7045 7050

Ser Glu Gly Ser Asp Gly Ser Ala Arg Pro Asp Gly Pro Asp Ala
7055 7060 7065

Glu Arg Glu Thr Asp Glu Arg Arg Arg Thr Pro Ala Asp Ala Asn
7070 7075 7080

Asp Glu Arg Asn Glu Ala Met Ser His Thr Ala Leu Val Gly Arg
7085 7090 7095

Leu Ala Ala His Leu Ser Glu Leu Leu Asp Val Pro Ala Glu Glu
7100 7105 7110

Ile Glu Gly Gly Val Glu Leu Ser Glu Tyr Gly Phe Asp Ser Ile
7115 7120 7125

Ser Leu Thr Glu Phe Val Thr Leu Leu Asn Gly Ala Tyr Gly Leu
7130 7135 7140

Ser Leu Val Pro Thr Val Leu Phe Glu His Ser Thr Leu Asp Gly
7145 7150 7155

Val Ala Gly His Leu Leu Glu Glu Tyr Ala Asp Arg Phe Ala Pro
7160 7165 7170

Glu Pro Glu Pro Glu Pro Glu Pro Gln Pro Val Gln Ala Gln Met
7175 7180 7185

Pro Glu Pro Val Pro Val Pro Glu Pro Glu Pro Ala Pro Val Pro
7190 7195 7200

Ala Arg Gly Pro Val Ala Pro Ser Thr Ala Pro Val Ala Ala Asp
7205 7210 7215

Asp Asp Asp Ala Leu Arg Arg Ala Leu Val Lys Arg Leu Arg Glu
7220 7225 7230

Leu Thr Ser Arg Ile Leu Arg Val Pro Ala Glu Lys Ile Ser Ala
7235 7240 7245

Thr Gln Glu Met Ser Lys Tyr Gly Val Asp Ser Leu Ser Leu Ala
7250 7255 7260

Glu Leu Ala Ala Ala Val Asn Ala Glu Phe Ser Leu Met Leu Asp
7265 7270 7275

Pro Thr Leu Phe Phe Glu His Pro Thr Leu Glu Ala Val Ala Arg
7280 7285 7290

Tyr Leu Leu Asp Arg His Ala Asp Arg Leu Thr Gly Leu Val Thr
7295 7300 7305

Glu Glu Thr Pro Glu Pro Ala Met Thr Glu Gln Ala Val Ala Glu
7310 7315 7320

Pro Val Val Ala Glu Pro Pro Val Val Glu Ser Pro Ala Thr Thr
7325 7330 7335

Ser Pro Ala Ala Glu Thr Ser Val Thr Glu Thr Ser Val Arg Glu
7340 7345 7350

Pro Ala Ala Pro Ala Ala Ala Pro Ala Pro Ala Phe Ala Ala Ala
7355 7360 7365

Pro Gly Pro Gly Ala Ala Glu Glu Pro Val Ala Val Ile Gly Ile
7370 7375 7380

Ser Ala Arg Phe Pro Met Ala Asp Asp Leu Ala Glu Phe Trp Glu
7385 7390 7395

Asn Leu Arg Glu Gly Arg Asp Cys Ile Arg Glu Val Pro Ser Asp
7400 7405 7410

Arg Trp Asp Trp Arg Glu Tyr Tyr Gly Asp Pro Val Lys Glu Pro
7415 7420 7425

Asn Lys Thr Asn Val Thr Ser Gly Gly Phe Met Asp Gly Val Gly
7430 7435 7440

Asp Phe Asp Pro Leu Phe Phe Asp Ile Ser Pro Lys Glu Ala Glu
7445 7450 7455

Leu Met Asp Pro Gln Gln Arg Leu Leu Met Leu His Thr Trp Lys
7460 7465 7470

Ala Leu Glu Asp Ala Gly Tyr Ala Pro Asp Ser Leu Ala Gly Thr
7475 7480 7485

Gly Thr Ala Leu Phe Val Gly Thr Thr Asn Thr Gly Tyr Gly Ser
7490 7495 7500

Met Val Ser Arg Tyr Ser Pro Val Ile Glu Gly Tyr Asp Ala Thr
7505 7510 7515

Gly Ala Ala Pro Cys Met Gly Pro Asn Arg Met Ser His Phe Leu
7520 7525 7530

Asp Leu His Gly Pro Ser Glu Pro Val Asp Thr Ala Cys Ser Ser
7535 7540 7545

Ser Leu Ile Ala Met His Arg Ala Ile Gln Ala Ile His Asp Gly
7550 7555 7560

His Ser Asp Met Ala Ile Ala Gly Gly Val Asn Thr Met Val Ser
7565 7570 7575

Ile Asp Gly His Ile Ser Ile Ser Arg Ala Gly Met Leu Ser Val
7580 7585 7590

Asp Gly Arg Cys Lys Thr Phe Ser Val Gly Ala Asp Gly Tyr Gly
7595 7600 7605

Arg Gly Glu Gly Val Gly Ile Leu Val Leu Lys Arg Leu Ser Ala
7610 7615 7620

Ala Val Arg Asp Gly Asp His Val Tyr Gly Val Val Arg Gly Ser
7625 7630 7635

Ala Val Asn His Gly Gly Arg Ala Asn Ser Leu Thr Ala Pro Asn
7640 7645 7650

Pro Arg Ala Gln Ala Asp Leu Val Val Gly Ala Trp Ser Arg Ala
7655 7660 7665

Gly Val Asp Pro Arg Ser Val Gly Tyr Val Glu Ala His Gly Thr
7670 7675 7680

Gly Thr Gly Leu Gly Asp Pro Val Glu Val Asn Gly Leu Lys Ala
7685 7690 7695

Ala Phe Ala Glu Leu Tyr Glu Arg Trp Gly Val Ser Gly Ala Gly
7700 7705 7710

Glu Ala His Cys Gly Leu Gly Ser Val Lys Thr Asn Ile Gly His
7715 7720 7725

Leu Glu Leu Ala Ser Gly Val Ala Gly Val Ile Lys Val Leu Leu
7730 7735 7740

Gln Met Arg His Arg Thr Leu Val Gly Ser Leu His Cys Gly Ser
7745 7750 7755

Val Asn Pro Tyr Val Arg Leu Glu Gly Ser Pro Phe Arg Leu Val
7760 7765 7770

Arg Glu Arg Glu Pro Trp Arg Ala Val Arg Asp Glu Asn Gly Arg
7775 7780 7785

Glu Leu Pro Arg Arg Ala Gly Val Ser Ser Phe Gly Phe Gly Gly
7790 7795 7800

Ala Asn Ala His Ile Val Leu Glu Glu Tyr Gln Pro Pro Ala Gly
7805 7810 7815

Thr Gln Thr Asp Ala His Thr Arg Thr Gly Pro Ser Thr Thr Val
7820 7825 7830

His Ser Gly Pro Val Ala Val Leu Leu Ser Ala His Arg Pro Asp

7835 7840 7845

Val Leu Arg Glu Ser Ala Thr Arg Trp Val Glu Val Leu Arg Arg
7850 7855 7860

Gly Asp Tyr Arg Asp Ala Asp Leu Pro Ala Leu Ser Tyr Thr Ser
7865 7870 7875

Gln Thr Gly Arg Thr Ala Met Ala Glu Arg Leu Ala Val Val Ala
7880 7885 7890

Gly Thr Leu Glu Glu Leu Arg Ala Gly Leu Glu Ser Trp Leu Arg
7895 7900 7905

Gly Glu Pro Thr Pro Ala Val Phe Thr Gly Arg Ala Pro Arg Asp
7910 7915 7920

Gly Asp Ala Pro Ala Ala Pro Ala Ala Leu Thr Asp Gly Phe Ala
7925 7930 7935

Ser Gly Gly Arg Thr Glu Ala Arg His Trp Ala Pro Val Leu Gln
7940 7945 7950

Ala Trp Thr Thr Gly Ala Glu Cys Asp Trp Arg Thr Leu Trp Gly
7955 7960 7965

Glu Arg His Pro Gln Arg Ile Ser Met Pro Thr Tyr Pro Phe Gln
7970 7975 7980

Leu Arg Arg Tyr Trp Leu Asp Met Thr Thr Pro Ala His Gly Pro
7985 7990 7995

His Val Ser Arg Gly Leu His Pro Leu Val His Arg Asn Thr Ser
8000 8005 8010

Asp Leu Ser Glu Gln Arg Tyr Thr Ser His Phe Thr Gly Arg Glu
8015 8020 8025

Phe Tyr Ile Ala Asp His Arg Val Gln Gly Glu Gln Val Val Pro
8030 8035 8040

Gly Ala Ala Leu Leu Glu Met Ala Arg Ala Ala Ala Val Leu Ala
8045 8050 8055

Ala Gly Gly Ala Glu Thr Asp Trp Ala Leu Arg Gln Val Val Trp
8060 8065 8070

Ser Arg Pro Leu Thr Ala Gly Arg Pro Val Asp Val His Thr Ala
8075 8080 8085

Val Ser Val Arg Ala Asp Gly Glu Pro Ala Phe Glu Ile Tyr Thr
8090 8095 8100

Glu Gly Pro Gly Gly Glu Arg Val Val His Ser Thr Gly Arg Leu
8105 8110 8115

His Arg Arg Thr Ala Gly Asn Ala Ala Glu Leu Leu Asp Gly Pro
8120 8125 8130

Glu Leu Pro Gly Gly Ala Gly His Leu Asp Val Ala Ala Leu Arg
8135 8140 8145

Ala Gln Cys Asp Gly Thr Val Leu Asp Ala Glu Glu Cys Tyr Ala
8150 8155 8160

Arg Phe Ser Gly Val Gly Leu Glu Tyr Gly Pro Thr Leu Arg Ala
8165 8170 8175

Val Glu Thr Leu Ser Gly Gly Thr Arg Gln Ala Val Ala Arg Leu
8180 8185 8190

Arg Leu Ser Ala Ala Ala Ser Ala Arg Thr Gly Phe Ala Leu His
8195 8200 8205

Pro Ser Leu Leu Asp Ala Ala Leu Gln Ser Thr Ala Gly Leu Phe
8210 8215 8220

Thr Gly Ser Gly Thr Ser Ser Ala Ala Leu Pro Phe Ala Leu Asp
8225 8230 8235

Arg Leu Glu Val Leu Arg Ala Thr Pro Ser Ser Gly Trp Ala Val
8240 8245 8250

Ala Arg Phe Ala Ala Asp Asp Arg Pro Gly Gly Val Arg Arg Leu
8255 8260 8265

Asp Ile Asp Val Cys Asp Asp Asp Gly Glu Val Cys Val Arg Ile
8270 8275 8280

Arg Gly Phe Gln Val Arg Thr Tyr Gly Gly Asp Ala Ala Pro Ser
8285 8290 8295

Ala Ser Gly Ala Ala Asn Gly Thr His Ala Val Thr Thr Asp Gly
8300 8305 8310

Thr Gly Asn Gly Thr Asp Thr Gly Asn Gly Asn Ser Thr Gly Thr
8315 8320 8325

Gly Ser Glu Ala Asp Ala Asp Ala Arg Leu Leu His Leu Ile His
8330 8335 8340

Ala Ile Gly Glu Gly Ala Leu Ser Ala Asp Glu Phe Gln Arg Ser
8345 8350 8355

Leu Ile
8360

<210> 35
<211> 25085
<212> DNA
<213> Streptomyces amphibiosporus

<400> 35

gtgagtcgaa acatcctgcg tgtgccggca tggcgagacg agccgtcgcg cgggcaggcg 60

gcaccggccg gagtccgccg gctggccgtc ctgtgcgacg tcccggacgc ggaggcggcg 120

ctgctgcggc agcactcgcc ccgcctgccc gtcgtcctgg tggagagccg ggacgacggg 180

cccgccgccg cgtacgagca cgcagccacc cggctgctcg ccgagctcca gaggctgctg 240

ggccgcccgg cggcgggtcc gtgccgggtg caggtggtgt gccgggagag cacgccgcag 300

ggctgggcgg gactgctcgg catgctgcgt acggcggcgc aggaagaccc ccgactccgg 360
ggccagctca tcgagttcga ccgactgccg ggcggcgccg agctggcgcg cgtgctggac 420
gaggaggccg ccgaggaggc ggatcatgtg cggcgggccg ccggtgcagc cggtacgggt 480
accggaaccg gagccgtacg gcaggtgcgc cactggagcg cggcccggtc ggcgggtcgc 540
gcgtcgtccg ccgggaaccc ggcgccggtg tggcggcccg gcggcgtcta tctcgtcagc 600
ggcggtgccg gcggcctcgg gcggctgctc gccgccgacg tgcggcggca cgcgcccggc 660
gcggtcacgg tcgtgtgcgg tcgtggcccg gcgccgtggc agggggcgga accgcccgcc 720
gacggcgtcg agtaccacag cgtggacgtc accgaccggg ccgcggtggc cgccctggtc 780
gatcgtgtgc tgagtgcaca cggcaggctc gacggtgtcg tgcacgcggc ggggctgctc 840
gccgacgact acgtggtccg cgcgtcgcac cgcgagaccc agcgcgtact ggcgcccaag 900
gtcgccggtc tggtccacct cgacgaggcc acccgcgaac tgccgctgga cttcctggcg 960
gccttctcct ccgccgccgg gacgctcggc aacgcgggcc aggccggtta cgccgcggcc 1020
aacggcttcc tcgacgccta ccagacccac cgcgccgcgc tggccgaggc gggcgagcgg 1080
cacggccgtt cgctctcggt cggctggccg ctgtggcggg acggcgggat gaccgtgccg 1140
gacgagcaac tgcccgaact caccgagcgg ttcgggcgtc cgctgacgac cggcacggcg 1200
ctgacggcac tgcacgccgc gctcgccctc ggcacaccgc acgtcctggt acgggacggc 1260
gcggaggcgg acgaaaccgg agccgtcaac gcaaccgggg ccgggaccgc gaccgggatc 1320
gcgaccgagg tcgaggtccc ggctgtgaac gaagccgtcg gcacggccgt cgacgacgcc 1380
ctggaggacg acgccccgga gggggacagg aagggaactc cggctgtgga accgcgcctc 1440
cgcgtactgc ccgcgctgaa gcaactcgtc gccgagaccg tgcggttgga cccggccgcg 1500
ctggacgccg ccgcgccgct ggacggcttc ggcatcgact cgctggccgt cacccggctc 1560
aaccgccgct tcgcgcagtg gttcggggcg ctccccaaga cgctgctgta ccagtacccg 1620
acgctgaacg agctggccgg atatctcgcc gagcaccatc cggagggctg ccgccgctgg 1680
ctcgccgaca cggcgtcccc gtccctgtcc ccttccgcgt ccgcgtccgc ttccccgtcc 1740
ccgtccccgg caacgtccac gtccgtgtcc gctccctccg ctcaggagcg gcggccgtca 1800
actcccgtcg ccgccggggc cgttcgcacg gccgggacga acggcacgag cggtgctgcc 1860
gccccggttt ccgccgaggc ccccgttccc gcccgtacgt cacctgtcga cgagccgatc 1920
gccgtcatcg gtctgcacgg gcgctacccc ggtgcccccca ccctggacgc cttctgggag 1980
aacctgcgct ccggccggga cggcgtcacc gagatccccg ccgaacgctg gccgctggag 2040
ggcttctggg agcccgacgt cgagcgcgcg gtgcgcgagg gcgcgagcta cagcaagtgg 2100

ggcggattcc tcgacgggtt cgcgcagttc gacgcgctgt ttttcgggat cgcgccgcgc 2160
gaggccgccg acatggaccc gcaggagcgg ctgttcgtgg agagcgcgtg gtccgtgctg 2220
gaggacgcgg gctacacccg gcggcgcctc gccgagcaac accgctcccg cgtcggggtg 2280
ttcgcgggca tcaccaagac cggcttcgac cggcaccgcc cggccgcccc cgcggagacg 2340
gacgcttcct ccgccacggg cggcgtgccg cccgcctccc cgcgtacgtc cttcggctcg 2400
ctcgccaacc gcgtctcgta cctgctcgat ctgcgcgggc ccagcatgcc cgtcgacacc 2460
atgtgctcgg cgtccctcac ggccgtgcac gaggcgtgtg agcatctgcg gcacggcgcc 2520
tgcgagttgg ccgtcgccgg cggcgtcaac ctgtatctgc acccctcgac gtacgtggag 2580
ctgtgccgtt cgcggatgct cgcccgcggc ggcgagtgcc gcagcttcgg caccggcggc 2640
gacggcttcg tgcccggcga gggcgtcggc acggtgctgc tgaagccgct gtcgaaggcg 2700
gaggccgacg gcgaccccgt acacgcggtg atcctcggct cggccatcaa ccacggcggc 2760
cgcaccaacg gttacaccgt gcccaatccg cgcgcgcagg cggagctgat ccgcgaggcg 2820
atggaccgcg cgggcgtctc cgccgacgag gtcggctgtg tcgaggcgca cggcaccgga 2880
acggcgctcg gcgaccccgt cgagatcgag ggcctggcgc aggcgttcgc cgaccgtacg 2940
gacacggcgg cgccgtgcgc cctcagttcg gtgaagtcca acatcgggca tctggaggcc 3000
gcggcgggca tcgcgggcct gacgaagctc gtgctccagc tccggcacgg cgagctggcg 3060
cccacgctgc acgccgaagt gcccaacccc gacatcgact tcggctccgt accgttcgcg 3120
ctccagaccg ccgcggcgcc ctggccgcgg accggaggga acagcggacg gcggatcgcg 3180
gggctgtcgt cgttcggcgc gggcggggcg aacgcgcatg tggtcgtcgc ggagtacacg 3240
ggcgccccgg ccgcccgcac ctccgcacct gccgtggccg acgggtccgc cgcgaccgcc 3300
gggtccggac gcccggtgct gctgccgctg tccgccagga cgcccgagga tctgcgggcc 3360
cgcgccgtac agctcgccga ctggctcgac tcccgcgacg cggtcgatct gacgtcggtc 3420
gcggcgacgc tccagacggg ccgcgaggcc atggacgagc ggctgtgctg tgtggcgtcc 3480
acgcccggcg aatggcgcga acagctccgt gcgttcgccg acgacccgga gcgcgagggc 3540
ccctggcacc gtggccgggt gcgggcgacc ggcgaggcgc tggccgcgct ggcggagaag 3600
gacgaactcc gggcgctcgt cggacgctgg accgcccgcg gcgagtgggc ggaactggcc 3660
gcgttctggg ccaagggcat gccgctggac tggagccgcc tgtacgcgga cggccgggtc 3720
ccggcccggc tccatctgcc cgcctatccc ttcgccgggc gccgctactg gcccggaccc 3780
gcggacgtac ggaacacggc ggacgcgcaa gcgccccgca cctccacgcc cagcccgtcc 3840
acgctcagca cgtcaactcc cggcgcgtcc aggcccgttg ccgtcgcgcc cgttgccgcc 3900
gcgccgtcgg cggagtcgta catcgaacgc gtgctgctcg acgcgctcgg cgaggccctc 3960

cagatgacgc ccgcggagat cgacccgcgc cgcccgttcg cggactacgg gctggactcc 4020
atcctcggcg tccacctggt caacgtcctc aacgagacgc tgggcaccgg cctggagacc 4080
accgacctct tcgaccacgg caccgccgag cggctgcgcg cgttcctcac cgagacctac 4140
ggcggcacgg tgaccgtccc cgacggcacg ggcgccgctg ccgagttcgt ccccgacgct 4200
cccgtcccgg cccgcgaggc ggacgacccg gtcgccgtcg tcggcatggc cgcgcgctac 4260
ggcgacgccg aggacccccg cgccctgtgg gaccgcctgc tggccggtga cgacctcgtc 4320
gagccggtca cccgctggga cctggggccc gaagtgacgt gccgcgcggg cagtttcgta 4380
cgcggcatgg accggttcga cccggtcttc ttcgcgatct ccggtgtcga ggcggcccat 4440
atggacccgc agcagcggat cttcctggag cagtgctgga acgccctgga ggacgccgga 4500
tacaccggcg agcggctgcg cgagcgcaac tgcggcgtgt acgtgggctg ttacgcgggc 4560
gactactacg acagcatcgg cgaccgcgcc ccggcccagg cgctgtgggg caccatgggc 4620
tcggtcgtcg cctcgcgcat cgcctatcaa ctcgacctga agggcccggc gctcaccacc 4680
gacacttcct gctccagctc gctcgtctcc ctccatctgg cctgccgcga tctgcgcacg 4740
ggcgccgccg acatggcgat cgcgggcggt gtcttcctcc agacgacgcc gcggctgtac 4800
gaggccgcga cccgtgctgg catgctctcg cccaccggcc gctgccacag cttcgactcc 4860
cgcgccgacg gcttcgtccc cggtgagggc gcgggcgccg tcgtactgaa gcggctgtcg 4920
gacgcgttgc gcgacggcga ccacgtctat ggcctcgtcc gcgccaccgg cgtcaaccag 4980
gacggcacca ccaacggcat caccgcgccc agcgcggcct cgcaggaggc gctgctgcgc 5040
gaggtgcacg cgggcgtcgc gcccggcggc gtccagttgg tcgaggcgca cggcaccggt 5100
acgcagctcg gcgacccgat cgaattccgc gccctcagcc gggtgttcgg ggacgcgccc 5160
gccggcagcg tcgtcctggg ctcggtgaag accaacctgg gacacaccca gttcgcggcg 5220
ggcatcgccg gtgtcctcaa ggcgctgctg gcgttgcagg agcagcgcgt tccgccgtcg 5280
ctgcacttcg cggaggccaa cgcgcgcgta ccgctggacg gcagtccctt caccgtcgcg 5340
acgacggcac agccgtggcc cgagcccgcc gagggaccgc gccgggcagc cgtcagctcc 5400
ttcggggcca gcggcaccaa cgcccatgtc gtactggagg agcacccgcc cgtacgggcg 5460
accacggggc cggagtccgc cggaggggac ggcgaggccg ccttcctgct gtcggcccgc 5520
acccccgccg cgctgcgggc cgtcgcggag cggctgctcg cgcggatcga gcgtgaaccg 5580
ggcctgcccg cccggcaggt ggcctacagc ctcgccgccg ggcgtcgcca cttcccgcac 5640
cggctggccg tcgtcgccac cgggctgcct gctctcgccg cccggctgcg tgcctggctg 5700
gcggacgaac agccgggcgg cgaggggacg ttgctgcacg gcgtcgccca cgccggaacg 5760

cggcaggccg cgctgggcgg gctcgcaccc gccgagctgg cggcggcgta tgtgggcggc 5820
gccgaggggc cgttcgccga gagcttcccg gccggggcgc ggcgtcaagt gccgctgccc 5880
acctatccgt tcgagcggca gcgctactgg gcggagggga cggacggaca cgctgtcccg 5940
gccgccgccg gtacgtccgc cgtggagccg ggcggccggc gcaccgcgta ccccgcacgc 6000
ggctcacggg tgaggagttc ttcctcgccg atcaccgggt gggcggccgt acggtgctgc 6060
cgggcgtgct gacgctggag gcggtacggc gcgcggtcac cgccggagac ggcggcgacg 6120
ggaccggaat cggcaccggc ggcggtacgg gcgtcccgac tcccctgcgg ttgcgtgacg 6180
tcgtgtggcc cgcgcccttc cccgtcggcg cggacggggc cgaactgcgc gtcgatctcg 6240
acggcgacgc cttcgccgta cggcaggacg gctcgtccgt gcacgcgcag ggccgctgga 6300
cgatggtgcc cgcccccgcc gcctccacgt cgctggagac cctgcgggag cgctgcgcac 6360
gccgcacgct gacgcgcgag cagtgccgtg cggcgctgga ggccgtcggc atccggcacg 6420
gtgagcggct ccgcgcgatc gagcaactgt ccgtcgggga cggcgagttg ctcgcccggc 6480
tggtgctgcc cgcgaccgtg gagacgggga cgcaggccac ggagacgttc ggactgcacc 6540
cggcgatgct cgacagcgcc atccaggccg tcgtcggact ctacggcgac gagaccggag 6600
ccctcggcga gcgtccggac gcccccgcac tgcccttcgc cctggacacc gccgacgtcc 6660
tcgctcccac caccgaccgg atgtgggccc atctgcgctg ggcggacggt tacgcgcccg 6720
gcgaggccgg agaggtgacg aagaccgaca tcgatctgta cgacgacgcg gggcggctct 6780
gtgtgcgcct gcgcggctac gcctcccgcc gcgtcacgcc cgccgccacc ggctccgcga 6840
ccgcggcccc ggccggtacg gacgacgccg acgcgccacg ggcgcagctc ctcgcaccgg 6900
tgtgggacgc acagccgcat gcggacggcc cccgcagccc cgagcccggt gcacacgtcg 6960
tcctgctcgg cggcacaccg gaggaacggg acgggctgcg ccgtctcgtc gccgacgtca 7020
ccgtcgtgga accggaacgc cacgcgtccg ccggggagtt ggccgcgctg ctgccgacgg 7080
gcgccgagca cgtcgtctgg ctcgcgcccc gcgacgcgtc gcccgccgcc tcctccgccg 7140
agggacccga cggggcgctc gccgtcttcc ggctcgtcaa ggcgctgctc gcggacggcg 7200
cggacgccag ggagctgagc ttcaccgcgg tcacccgcca ggcccggctg ctgcccggcg 7260
acgcggactg cgacccggcg cacgccggag tgcacggtct gctgggcacg ttggccaagg 7320
agtacccgca ctggcgggtg cggggcgccg acatcgagcg ggacgtctcc gtaccgtggc 7380
cggagctgct gtcgctgccc gcggatccgc gcggcgaggt gtcggcccgc cggcacggcg 7440
agtggtaccg gcagcggctg ctggaggtcg ccctcgacgc gtccggcgcc gcctccttct 7500
ccgccggctc ccaggccccc aacccccagg cccccaactc tcaggcgagt ggcgcccgtt 7560
cggcactgcg cgagcccggc ggcgtcgtcg tcgccatcgg cggcgcgggc ggcatcggca 7620

ccgtgtggac cgagcacatg atgcgacggc acggcgcccg cgtggtgtgg atcggccgcc 7680
gcccctacga cgaggagatc gccgcgcggc aggaccgcct cgcggcctgc ggcccgcgcc 7740
cggagtacgt acgggccgac gcgaccgacg cccgcgctct gcgccgcgcc gtcgcggaga 7800
tcgagcgccg ccacggcccc gtacgcggcg tcctgcacac cgcgatcgtc ctcggcgacc 7860
agagcctggc caggatggac gaggccgcct tccgcaccac gtacgaggcc aaggccgccg 7920
tctcggtcaa catggccgac gcgttcgccg gtcagccgct ggagttcgtg gccttcttct 7980
cctccatgca ggcgttcttc aaggcgccgg gccaggccaa ctacgcggcg ggctgcacgt 8040
tttccgacgc ctgggccgag cggctctcca ccgcgctcga ctgccccgtg aaggtcatga 8100
gctggggcta ctgggccgga gtcggcatcg tgaccgccga cggctaccgg cagcgcatgg 8160
cgcagctcgg gctcgggtcc atcgaaccgg acgagggcat ggccgccttc gacgcgctgc 8220
tggcctcccc gtaccggcag ctcgccctgc tcaaggcgac cgacagccgc agcatcgacg 8280
ggatctacgg cgacgacgag ctgcggcaac tgccgcccgc cgcgcccgcg ctcgcggaca 8340
ccctccgcac ggaccgcccc gaccggaacg cggagatccg gcggctgcgg gagcaggccg 8400
acggccacgc cggagtcatg tacgacgctc ttgtccgcgt cacctgggcg ctgttgacgt 8460
cgctgggact cttccgcgac ggccgcgcgg ccaccgccgc cgagtggcgc gccgtcggcg 8520
gcatcgagga gcgctaccag cgctggacgg aacacacgct ggaggtgctg accgccgccg 8580
gacgtctgcg ccgcgcgggc gaggaccggt acgccgccgt cgcccccgga gccgtacccg 8640
ccgaggacgc ctgggccgag tgggaccggg cgcgggaggt gtggctcgcg gacgaggcca 8700
agcaggcgca ggccgtgctc gtcgacacca cgctgcggga gctgacggcg atcctcaccg 8760
gccgccgcgc cgccaccgac gtgatgttcc cgggctcctc gctgcggctc gtcgaggccg 8820
tctacaagaa caaccccgtc gcggactact tcaacgaggt gctcgccgac accctcgtcg 8880
cctacctcga acaccggctg cgccaggacc cgtccgcgcg gctgcgcatc ctggagatcg 8940
gcgccgggac cgggggcacc agctccgtgg tcttccggcg gctccggccg ctggccgggc 9000
acatcgagac ctacacctac accgacatct ccaaggcgtt cctgctgcac gcccggcgtg 9060
cgtacgggga gatcgcgccg tacctggacg ggcagctctt cgacgcggag aagccgctcg 9120
ccggacagcc ggtcgccgtc ggcggacacg acgtggtgat cgccaccaac gtgctgcacg 9180
cgacgggcaa catccgcaac accctgcgca acgcgaaggc cgccgtacgc gccaacggcc 9240
tgctgctgct caacgagttg agcgacaaca tcctcttcag ccacctcacc ttcggtctgc 9300
tggacggctg gtggctctac gacgacccgg cgccgcgcat cccgggatcg ccggggctga 9360
cgccgcagag ctggcgccgc gtcctggacg aggtgggctt ccgcgggtcg ttcgtcgcag 9420

ccgagggcgc cgacgacctc ggccagcagg tgatcgtcgc cgagagcgac ggagccgtgc 9480
ggcagccgcg gcccggcggg gtctccgcct tccggggcag cctgccggag gcgcggccgg 9540
ctcaacccac gggcggggcg gggcacttgg cggtgccggc ggagcacggc tccgcgcctg 9600
ccgtgaccgt gccggtcacc gccgcgtccg cttcctccgc accgggctcc gcgcccgccg 9660
ccgtcccgtc cggtgatccg tccggcgacg ggagcatggc cgcacgtgtg gccggaccgg 9720
cacgggacct cttccggggg ctcgtcgcgg acgtcctgca actgcccgtc ggcgacatcc 9780
gtgccgacgt gcccttcgag cggtacggca tcgactcgat cctcgtcgtc caactcaccg 9840
acgccgtacg gaaggtgctc gacggcgtgg gcagcacgct cttcttcgag gtgagcacgg 9900
tcgacggcct cgtggagcac ttcctgcgca cccggccgga cgaactcgcc gcgctcgtcg 9960
gcgtatccgc cgcggagcac ccggaacccg cggcggaagc cgccgcaccg gaggcggtca 10020
ccgaggagcc ggcggcctct gtacccgcac ccgcacccgt agccgctcct gtctccgtac 10080
ccgtgcccgc cgcccccgggt gaggacgtcc ccgtcgccgt cgtcggcatg gccgggcgct 10140
accccggcgc cgccgacctg gacgccttct gggagaacct gctcgcgggc cgcgactgcg 10200
tcaccgagat ccccgacggc cgctgggacc acggccgtta ctacgacgag cgcccgcggcg 10260
tgcccggcag gacgtacagc aagtggggcg gattcctcga cggcgtcgac gagttcgact 10320
cgctgttctt cggcatctcg ccgaaggccg cgtccacgat ggacccgcag gagcggctgt 10380
tcctccagtg cgcgtggacg gcgctggagg acgcgggcca cacgcgggcc tcgctgcgct 10440
ccgcctcccg cgcccggctg cccgaagacg ccggggacat cggggtgttc gtgggcgcga 10500
tgtactccga gtaccagctc tacggcgcgg agcagggcgt acggggcgag cccgtcgtcg 10560
tacccggcag cctcgcctcg atcgcgaacc ggctgtcgta cttcctcgac gcgtccgggc 10620
ccagcgtcgc cgtcgacacg atgtgcgcct cggccctgtc cgccgtgcat ctggcgtgtg 10680
ccgcgatccg gcgcggtgag tgcgcctcgg cggtggccgg cggcgtcaat ctgtcgctgc 10740
accccagcaa gtacctgatg atcggcgagg gacagttcgc ctcctcagac gggcgctgcc 10800
gcagcttcgg cgcggacggc gacggctatg tgcccggcga gggcgtgggc gcggtgctgc 10860
tgcggccgct cgccgacgcc gtggccgacg gcgaccgcgt gctcggcgtg atccgcggca 10920
gcgccgtgaa ccacggcggg cacacgcacg gcttcaccgt tcccaacccg ctcgcccagg 10980
cgtccgtgat ccgcggcgcg tggcgccgct ccggcgtgga cccgcgcgac atcggctgca 11040
tcgaggcaca cggcacgggc accgcgctgg gcgaccccgt ggagatcgcc ggactgaacg 11100
ccgccttcgg cgagttcacc tccgagcgca ccttctgctc cctcggctcc gccaagtcca 11160
acatcggaca tctggagtcg gcggcgggcg tcgcgggcct ggccaagatg ctgctccaga 11220
tgcggcacgg cacgctggtg ccgtcgctgc acgccgagcg caccaacccc gaaatcgact 11280

tcgccgccac gccgttcgtg ctccagcggg aggccgcgcc ctggccgcgc cgcgaggggc 11340
gcccacggct cggcggcatc tccgcgttcg gcgcgggcgg ttcgaacgcg catctgctcg 11400
tcgaggagta cgtaccgacg gcggcaccgc cgcggcgtgc ggcgccgggc ccggtcctcg 11460
cggtgctctc cgcgcgggac ggcgagcgcc tgcgggagta cgccgggaag ctgcgggacg 11520
cactgcgctc cgggcagtgg accgacgagg acctgccgga catcgcctac accctccagg 11580
tcggacggga ggcgatgagc gcacggttcg ccgccgaggt gagcaccctg gccggactca 11640
tggacgcgct ggacgcgtgc gcacggggcg ccgccctgcc gcccggcgcc cggctgcgta 11700
ccgacggcgg gcggggcgga ccggtccagg acctcgcgga cgacgaggac ttccgggaga 11760
ccgtcgtgcg ctggctgcgc cgcgggaagc tggcgccgct cgccgaggcg tggaccggcg 11820
gcctcgacgt ggactgggcc cgcggccacg gcaccggcga ggaccggccg cgcaaggtcg 11880
gcctgcccgg ctacccgttc gcgcgggaga ggtactggtg gaacgacggg ctggccgagg 11940
ccggaggcga gggcgctgac ggtctgggag acgagggcgc cgccggcggc accgccggtt 12000
ccggtaacgg ttccgggccc cgttccgctc gtacggacgg gacgcgcccc ggtgaactgc 12060
ccccgggcga cctcacgttg caccccgtct gggagcccgt acatgcggcg ggcggcggcg 12120
cggacgcgcc cttcccgcag cccgcggacc gcgtcgtcgc ggtcggcctg gcaccggagg 12180
cccgtgccgc gctggaggcg tacggcaccc gtgtggtgac gctcccggca ccccgggacg 12240
gcggccgttc cgtggcggac gtccgccgcg aactggagac cgcgggcccc ttcgaccacg 12300
tcgtcgtgga gtgccccacc cccgccgcac agggcgcgcg gcagcgcgtc gaggctcaac 12360
gcgcctccgt acgcggcctg ttccggctgc tccaggcgct ctccgccctc cgcgcggacg 12420
agccgcggac cggtctgacc ctcgtcaccc gcgacgcgtt cgacccggat cgcacgggcg 12480
gcgccgaccc ggcgcaggcc gcgctgcacg gcctcgtcgg cggcctcgcc aaggaacagc 12540
cgtactggcg cgtgcgcgcc gtcgacctgg ccgagggcga gcccttcgtg cccgaggaga 12600
tctgcgccct gcccgccgac cgccgggcgc atccgctcgt ccggcgcggc ggccagtggc 12660
tgagccgcag gctgctgccc gtcggcgacg tacggcccgg cacgccggac gacggcccgc 12720
gtacggctgt tgacgggacg gacgccgcgc cgcaggccgt ctccgtcccg tccggttccg 12780
tctcgtccgt ctccgtcccg tccggcggtt tccgcggtga cggcgtctat gtgctgatcg 12840
gcggcgcggg cgacctcggc accgtcctca ccgagcatct gctgcgccgc tacgacgccc 12900
gcgtggtgtg ggtgggccgc cgtgcggagg acgacgccgt acgcgccgcg gcggcacgcg 12960
tcgccgcggc caccggcggc gaggcccccg tgtatctgtc cgccgacgcc cgcgacccgg 13020
gcgcgctcgc ccgcgtacgg gacgaggtgc tgcgccggta cggacgcatc gacggcctgg 13080

tgcacctggc gatggtcttc agccacacgc tcctcgcgga gctgccggag gaggacctga 13140
acgcgacgct cgccgccaag gccgacccga ccgagcactt cgccgacgtc ttcgcgggac 13200
agcggctcga cttcgtgctg ctggtctcct ccctcgtcag cttcatccgt aactcccacc 13260
aggcgcacta cgcggcggcc tgcgcctacg aggacgcccg cgcgcccggt ctgggccggg 13320
cactgggctg ccccgtcaag gtcgtcaact ggggctactg ggcaacgtc agcgacgaag 13380
tgctgcgcgg cgtcacggag atggggctcg cgcccatcga acccgcctcc gccatggcgg 13440
ccgtcgaaga gctgctgacc ggcccgctcg accagatcgg cttcatgcgg ctcggccgtc 13500
cgctgcccgt cgagggcgtg ctcgcggggg agacgctgag cgggcatccg tacgcggcgg 13560
tctcccgtac ggccgcggag cccgcccccg tgccggtgcc ggccgcgctg gcggagcacc 13620
acgcggggcc tgtgccgggt gagatcgacg ccctgctgtg ccgctgtgtc gcggccacgc 13680
tgcggcgtgc cgggctgcgc cgcccggccg acggcttcgc ctccggttcc ggttccggtt 13740
ccggggccgg gagcgcgggc gtgcgcgtgg acgagcggtt cgacggctgg ttcgcggcga 13800
ccgtacgcac cctgcgcgag tacgggctcg tcgactcccg cggcgactgg agcgagcgcg 13860
caccgggcgc gggggacgcc gccgcctgcc tggccgagtg ggagcgcgcg gccgagcggt 13920
gggcctctgc gcacgccgat ctgcgggcgc cgacggggct gttgggccgt acgctgcccg 13980
cgctggccga catcctgcgc ggccggatcc cggcgaccga cgtgctgttc ccggaggggt 14040
cgttctccct ggtggagggc gtctaccggg acaacgccgt ggccgcgcac ttcaacgccg 14100
tactcgccgc acaggtgacg gcgttcctgc acggccgccg cgcggccgac ccggcggcgc 14160
ggctgcgcgt actggagatc ggcgcgggca ccggcggcac caccgcgccc gtgctggagc 14220
aactggagtg cgcgggcctg gagttggccg agtactgctt caccgacctc tccctcgcct 14280
tcctccagcg tgccgaggac gccttcggcc ccggccgcgc ccacttcgcc tgccgcaccc 14340
tcgacgtgtc acgggcaccg cgcacgcagg gcttcgacgc gggggcgtac gacgtggtga 14400
tcgccgccaa cgtgctgcac gccacggacg acgtacggac cgcgctgcgg cacgccaagt 14460
cgctgctgcg cggcggcgga atgctggcgc tgaacgagat cagcggcttc tacctcgtca 14520
accacctcac cttcggcctg ctggacggct ggtggctcta cggcgacgcc gaactgcggg 14580
cgccgggcag ccccgcgctg cctccggaga gctggcgccg ggtgctgacg caggagggct 14640
tcaccggcgt cgcggacccg gcacgggacg cccgtgccct gggacagcag gtcgtgatcg 14700
cccacagcga cggactggcc cgcggcccgg tgacggacgc ggcaccggcg gcaccggcag 14760
caccggcggc cgtggcgcgg ccggagacga acacggcggt gagcgcggcg cccaacatgg 14820
cggtgagcgc ggcgagttcg gcggcgggcg gtccgcagac ctccggcggt ccggacgtgc 14880
gcgtggtcgc cgacgtcgtc gagacggagc tggccgacgc gctgcggctg ccggcggaac 14940

ggatcgaccg ggcgggcgcg ttcgcggacg tgggcctgga ctccatcgtc ggcgcgcgct 15000
tcgtacggcg gctcaacgag gaactgggcc tggacctgcc gacgacggtg atcttcgatt 15060
accggagcgt cgacgaactg gccgcgcaca tcgtggagga ccaccgtccg acctcgcctg 15120
cgccgggcgg taccggggcg gccaccgctc aggagccccc ggccgagcgg gagtcggggc 15180
gcgccccgga gcgggagcac gggcccgttg tggcgcccga tgtcaccgtg cccgatgcca 15240
ccgagcccgg ttccgccccg tacggccggg agcccatcgc cgtcgtgggc gtcagcgggc 15300
gcttcgccgg ttccgacgat ctcgacgccc tgtggcggca tctggccgcg ggcgacgacc 15360
tcgtcgggcc gatcgaccgc tgggatctct cggcctacgg cgaggacgaa ctgacctgcc 15420
gcagcggcag tttcctcgac ggcatcgacc ggttcgacgc ccgcttcttc aagctgtcgg 15480
gccgcgaggc cgcctacacc gacccgcagc agcgcctctt cctcgaacag gcatggacgg 15540
ccctggagga cgccgggcac ggcggcgcct cgaccgacgg catgcgctgc ggcgtctacg 15600
tcggctgcac cggcggcgac tacaaggacc acttcgagga cgcgccgccc gcgcaggccg 15660
tctggggcaa cgcgccctcg atcgtccccg cgcgcatcgc ctaccacctc aacctccagg 15720
gcccggccat cgcggtcgac acggcctgct ccagctcgct ggtcgccgtg catctggcct 15780
gccagggact gtggagcggc gagaccgaga tggccgtggc gggcggcgtc agcgtgcaga 15840
ccactccggc cacctatctc tcggccagcc gcgccgggat gctctcgccg acgggacgct 15900
gccacacctt cgacgccgcc gcggacgggt tcgtaccggg cgagggcgtc ggcgtcgtgg 15960
tgctgcgcag gctctcggac gcgctgcgcg acggcgacca cgtgcacgcc gtcatccgcg 16020
gttcgggcgt caaccaggac ggcgccacca acgggatcac cgcgcccagc gccctgtccc 16080
aggaacggct gctgcgccag gtctacgagg acttcgcgat cgacccgtcc gagatcggca 16140
tggtcgaggc ccacggcacc ggcacacagc tcggagaccc gatcgaatgc cacgccctgc 16200
ggcgggtgtt cgagggcagc gacgtccccg gcggctgcgc gctcggttcg atcaagacga 16260
acctcggcca caccacgtcc gcggcgggcg tcgcgggtct gctgaagatc gtcctgtcgc 16320
tgcggcaccg gcagatcccg ccctccctgc actaccgcga ccgcaatccc gagatccggc 16380
tggaaggcgg ccccctgtac gtgaacacct cactgcgccc ctgggagccg aacgcgggcg 16440
gcagccgtgc cgccgccctc agctcgttcg gcttcagcgg caccaacagc catctcgtcg 16500
tcgaggaggc accggcgcgt cccgggcggt ccccgctctc cggcgccgcc gccgtggagg 16560
agcccggact gccccgggtc ttcccgctgt ccgcgcccca gccggcggcg ctgcgcgagc 16620
gcgtccgcga tctggccgtc catctgcgga gcacgccgga cgccgtcctc gtcgacgtca 16680
gccacaccct ggcgacgggc cgcgcccact tcgcgcaccg cgccgccttc gtcgcccgca 16740

cccgcgagga gctgatcggt caactcgacg actggctcga cggggaggcc ggagacgccg 16800
ggaaggcggc gaagaccggg gaggccgcga agaccggaga cgtcggcgag gccgggggcg 16860
ccgggccgga ggagctggcc cgcgaccgct acctcgccgg tgaacccgcc gacttcgccg 16920
cgctgttcgc cggttccggc gcccgtcgca caccgctgcc gacgtacccc ttccagcgca 16980
ggagccactg ggtgcgcggc ggcgcaccgg ggagcgcccc ggacgcggcc gggtccggta 17040
cgtccaccac gtccggcacg cccgccctcc gtaccgacgc aagggagaag ggccgcggag 17100
ccgcccgcgc ggaggacgac gccgtcgccg tcgtcggcct ctccgcccgc ttcgcgcagt 17160
cgccggacgc cgaggccctg tgggcacatc tcgccgcggg cgacgacctg gtcggcgagg 17220
tgacccgctg ggacctgtcg cagatcagcg gcggacgcac cgaacacggc agcttcgtcg 17280
aggacatcgc ccgtttcgac gccctgtact tcggcgtctc gggcaacgag gccacgtacg 17340
ccgacccgca gcagcgcatc tacctggagg agtgctggca cgccctcgag gacgccggtt 17400
acgcggggga gcggctggac gggcggggct gcggcgtcta cgtgggcgcc taccccggcg 17460
actaccacga gctgatcggc gccgaccgcc cgccgcagac gatgtggggc aacatggcct 17520
cggtcatcgc ctcgcgcatc tcctacttcc tcgacctgga cggcccggcg atgtccgtcg 17580
actcggcctg ctccagctcg ctcgtcgcca tccacaccgc gtgccaggac ctgcgtctgg 17640
gcacgacctc catggcgctg gcgggcggtg tgttcatcca ggcgacgccg cggctctacc 17700
agtactcggg caaggcgcgg atgctctcgg ccaccggacg ctgccacgcc ttcgacgccg 17760
ccgcggacgg gttcgtcccc ggcgagggcg ccggagtcgt cgtcctcaag cggctgtcgg 17820
acgcgctgcg cgacggcgac cgcgtctacg gcgtgatccg ctcctcgggc gtcaaccagg 17880
acggcaccac caacggcatc acggcaccca gcggcgcggc tcaggagaac ctcgtccgcg 17940
acgtgtacga gcgcgcgggc gtcgccccgt ccgggatcca gctcatcgag gcgcacggca 18000
ccggcacacc gctcggcgac ccgatcgaat tcgaggcgct gcgcgccgtg ttcgcggacg 18060
cgccgacggg cggctgcgcg ctgggcacga tcaagagcaa cgtgggccac acccagttca 18120
ccgccggagt cgcgggggtc ctcaaggtgc tgctcgcgct cgaccacgaa cagctcccgc 18180
cctccttgca cttcacccgg cccaacccgg ccatcgacct cgcgaacagc cccttccacg 18240
tcaacaccga actgctgccc tggcgcgcgc ccgccgacgg gccgcgccgc gcgggcgtca 18300
gctccttcgg cgccgccggc accaacgcgc acgtcctgat cgaacaggca ccgtccgacg 18360
ccgccgcccg cgcacgccga cacgggcgcg cacagtggct gttggtgctc tccggccagg 18420
acggcaccgc gctgcgcgcc caggccgagc ggatgctgga ccacgtcgaa cgccacccgg 18480
acctcgacct cggcgacacc gcctggacgc tcgccacggg acgccgccac agcgctcacc 18540
gtctggcgtg cgtcgccgcc gaccgcgagc agtggacggc ggcactgcgg ggctggctgc 18600

gcgacggccg tgccgagggc gtgtggacgg gcgaggccga cgagtcgccc cgctccgggc 18660
acagcggcga gagcggcgag ggcagcggcg aaccggcccg cgccgaggcg ctgatggccg 18720
agcacgaccg tcccggaaac ctcgccgcgc tcgcggagct gtacgtacgg ggcgaggtcg 18780
cgcgcttcgc accgctgtac gccgacgggg acttccgcat cgtctccctg cccggctacc 18840
ccttcggcgg cgagcgctac tggaccgggc cgctgcccgg ggacacgccc gacgggacgg 18900
acggaacgga cgggacgtac ggcacggacg ggatcagcga atccggtggc gaatcccggc 18960
cgtccgccga accccggccg tacgccgggg cgttggcgct gaccggggag gagttcttcc 19020
tcgacgacca ccgggtcggc ggcgtccccg tactgccggg cgtcgcgtat ctggaactcg 19080
cgcacgcggc ggcgaccgca cagggcggcc tcgcccccgg cggtgtgctg ctgcgcaacg 19140
tcgtctggtc ccgcccggcg cgcgtcaccg agccgctctc cgtggagacg gtgctcgaac 19200
cacgcgccgc ggacggcacg ttcggatacg agatcgccac cgtccgggac ggcgcccggc 19260
ggctggtgca cggccggggc cggatcgagc cgcgtcccgg cggcgcgccc gcccggctcg 19320
gcctcgccgc gctgcgtgag cggtgcgacg tgcggagcct ggaccacgcg gagtgctacg 19380
cgttgctcgg cgccaccggg atgtcgtacg gcgccgcgat gcgcggtctg gaggagctgc 19440
acgtcggccg cgggctcgcg ctcggccggc tgcgcgttcc gcgcgaggca cgcgacggac 19500
gcccctggac gctccatccc gccctgctgg acgcggcgtt gcaggcgacg gtcgggctgg 19560
ccctggacgg ggagtccgac gggctgacgg ccgcactgcc cttcgcggtg gagcaggtgc 19620
aggtgctcgc cgcgagcccg gagagcggct gggccgtggc ccgtcccgcg gacggcgccg 19680
ccgagggccc ggtccggcgg atggacgtgg agatctgcga cgacgagggc acggtgtgcg 19740
tacggctcct cggcttcagc acccgcgaac tcccgggcgc caccgcgtcg gtgacgaccg 19800
gagcgacgac cggggcgggg tccggggcgg ggtccgccgc cgcgtcccct gctccggccg 19860
ccgccgatcc cggcgcgccc gccgacggct ccctggtctt cgcccggccc gtctggcgcg 19920
ccgtgccctc cgcagacgta cgggaggagc gcccggcgcc gagtccggca ccgtaccggg 19980
agatcctgct ggccggtccg gagtccgtcg acgctgcgga ggtgcggaag cgctcgggcg 20040
tcccgtgctc ggcgctgccc ggcggcgccg atctgcccga gcggtacacc cggcaggccc 20100
aggcgctgct ggcgaaggtg cagcaactcc tgccgcgcgt acgggaggag cgcgtcctgc 20160
tccaggtcgc ggtccccgcg cacggagaag gccggctctt cgcggggctc gcgggtctgc 20220
tgcgtacggc ctgcgcggag caccccggac tggccgcgca gctcgtcgag accgacgccg 20280
ccgacgcggc gacgctctgc gcccacctcg acgccgaggc cgcgcagccc ggcgtggcga 20340
cggtgcgccg cacgggcggc gaacggctgg tgcggcagtg gcacggcttc cgtccggagc 20400

gcggcgatca gccctggaag ccgggcgggg tccatctcgt caccggcggc gccggcggcc 20460
tcggagcgct gttcgcccgc cggatcgccc gtaccgcgcc cggatccgta ctggtgctgt 20520
gcggccgctc cccggagggc gcggcacagc gcgaactcct gggtgagctg cgggagtcgg 20580
gcgccgcaca cgcggagtac cacagcctgg acgtcggcag gcgtgcggac gtcgtccggc 20640
tcgtgcggca ggtcgtggac cggcacggac ggctcgacgg cgtgatccac agcgccggag 20700
tgctgcggga cggcttcgtc gcccacaaga ccccggagta cctgggcgag gtcttcgccc 20760
cgaaggccgg gggagtggtg cacctcgacg aggccaccgc cgcactggag ttggacttct 20820
tcctcgtctt ctcctcgatg tcggtgctcg gcaaccccgg ccaggccgac tacgcggcgg 20880
ccaacgcgtt cctggacgcg tacgtcgccc accgcgccgg actggcggac cgcggtgagc 20940
gccacggccg ctcgctctcg gtcggctggc ccctgtgggc ggacggcggc atgcacgtgg 21000
acgcggccac ggagcgccgc atccaccaga gctccggaat gcggccgttg cgtgcccgtg 21060
aggggttcga ggcgctggag cgcctgtacg ggagcggact gccgcacgcg ctgaccgcgt 21120
tcggcgaccg cgagcgcatc gcgtcggtgc tgctcgacgg ttccgagggc tccgacggct 21180
cggctcgtcc ggacggtccg gacgcggagc gggagacgga cgagcggcgc cggaccccgg 21240
cggacgcgaa cgacgaacgg aacgaggcca tgtcacacac ggcgctggtc ggccgactcg 21300
ccgcccatct ctcggagttg ctggacgtac cggcggagga gatcgagggc ggggtcgagc 21360
tgagcgagta cggcttcgac tcgatctcgc tgacggagtt cgtcacgctg ctcaacggcg 21420
cgtacgggct gtcgctcgtg ccgacggtgc tcttcgagca ctcgacgctc gacggggtcg 21480
cgggacatct gctggaggag tacgcggacc gcttcgcgcc ggagccggag ccggagccgg 21540
agccgcagcc ggtgcaggcg cagatgccgg agccggtgcc ggtgccggag ccggaacctg 21600
caccggtgcc ggcgcgcggg cccgtggcac cgtcaaccgc ccccgtggcg gccgacgatg 21660
acgacgcgct gcgccgtgcg ctggtcaagc ggctgcggga gctgacgtcc cgcatcctcc 21720
gggtgcccgc ggagaagatc agcgccacgc aggagatgag caagtacggc gtggactccc 21780
tgtcgctggc ggagctggcg gcggccgtga acgcggagtt ctcgctgatg ctggacccga 21840
cgctgttctt cgagcacccg acgctggagg ccgtcgcccg ctatctcctc gaccggcacg 21900
ccgaccggct caccggcctg gtgaccgagg agacccccga accggccatg accgaacagg 21960
ccgtggctga accggtcgtg gccgagccgc ccgtcgtcga atcgcccgct acgacgtcac 22020
ccgccgcgga gacgtccgtc accgagacgt ccgtacgtga acccgccgcc cccgcggcgg 22080
ctccggctcc ggctttcgcc gcggccccgg ggccgggtgc tgcggaggag cccgtcgccg 22140
tcatcgggat cagcgcgcgt tttccgatgg cggatgatct ggcggagttc tgggagaact 22200
tgcgtgaggg ccgggactgt atccgtgagg tgccctcgga tcgctgggac tggcgcgagt 22260

actacggcga ccccgtcaag gagcccaaca agaccaacgt gacgtccggt ggattcatgg 22320
acggcgtggg cgacttcgac ccgctcttct tcgacatctc gcccaaggaa gcggagttga 22380
tggacccgca gcagcggctg ctgatgctcc acacctggaa ggccctggag gacgcgggct 22440
atgcgccgga cagtctcgcc ggcaccggca cggccctctt cgtcggtacg acgaacaccg 22500
gttacggaag catggtcagc cgctattcac cggtgatcga gggatacgac gcgaccgggg 22560
ccgcgccctg catgggcccg aaccggatga gccatttcct cgatctgcac ggccccagcg 22620
agcccgtgga caccgcctgt tccagttcgc tcatcgcaat gcaccgcgcc attcaggcaa 22680
ttcacgacgg ccattccgac atggcgatcg cgggcggcgt caacacgatg gtgagcatcg 22740
acggccacat cagcatttcc cgtgcgggga tgttgagtgt ggatggtcgg tgtaagacgt 22800
tttcggtggg ggctgatggt tatgggcgtg gtgagggggt ggggattttg gtgttgaagc 22860
gtttgtcggc tgcggtgcgt gatggtgatc atgtgtatgg ggtggtgcgt ggttcggcgg 22920
tgaatcatgg gggtcgtgcg aattctttga cggcgccgaa tcctcgtgct caggcggatt 22980
tggtggtggg tgcgtggtcg cgggcgggtg ttgatccgcg gtcggtgggt tatgtggagg 23040
cgcatggtac ggggacgggg ctgggtgatc cggttgaggt gaacgggttg aaggctgcgt 23100
ttgcggagtt gtatgagcgg tggggtgttt cgggggccgg tgaggcgcat tgtggtctgg 23160
gttcggtgaa gacgaatatc gggcatttgg agttggcgtc gggtgtcgcg ggtgtgatca 23220
aggtgttgtt gcagatgcgg catcggacgt tggtggggag tttgcattgt gggtcggtga 23280
atccgtatgt gcggttggag gggagtcctt tccgtctggt gcgggagcgt gagccgtggc 23340
gggcggtacg ggatgagaac gggcgggagt tgccgcgccg tgcgggcgtg agttccttcg 23400
gtttcggcgg cgccaacgcc catatcgttc tggaggaata ccagcccccg gccggcacgc 23460
agaccgacgc ccacacccgc accggcccct caaccaccgt ccacagcggc cccgttgccg 23520
tcctgctctc cgcccaccgt cccgacgtac tgcgggagtc ggcgacccgc tgggtcgagg 23580
tcctgcggcg cggcgactac cgcgacgccg acctcccggc gctgtcgtac acctcgcaga 23640
cgggacgcac cgccatggcc gagcggctcg cggtcgtcgc cgggacgctg gaggagctgc 23700
gcgcgggact ggagtcctgg ctccgcggcg agccgacccc ggccgtgttc accggacgcg 23760
ccccgcgcga cggcgacgca ccggcggcac cggccgccct caccgacggg ttcgcctccg 23820
ggggacgtac ggaggcgcgg cactgggcgc cggtgctcca ggcgtggacg acgggcgccg 23880
agtgcgactg gcggacgctg tggggcgaac ggcacccgca acggatctcc atgccgacgt 23940
accccttcca actccggcgc tactggctcg acatgaccac cccggcgcac ggcccgcacg 24000
tctcccgcgg actgcatccg ctggtgcacc ggaacacctc cgacctgagc gagcagcgct 24060

```
acacctcgca cttcaccggc cgggagttct acatcgccga ccaccgggtg cagggcgaac  24120
aggtcgtccc cggcgcggcg ttgctggaga tggcacgcgc cgccgccgtc ctcgcggcgg  24180
gcggtgcgga gaccgactgg gcgctgcgcc aggtggtctg gtcccggccg ctgacggccg  24240
gacggcccgt cgacgtgcac accgccgtgt ccgtgcgggc ggacggcgaa ccggccttcg  24300
agatctacac ggagggcccc ggcggcgaac gcgtcgtgca ctccaccgga cggctgcacc  24360
gcaggaccgc cgggaacgcc gccgaactcc tggacggacc cgaactcccc ggcggcgccg  24420
gacacttgga cgtcgccgcg ctgcgtgccc agtgcgacgg caccgtcctc gacgccgagg  24480
agtgctacgc ccggttctcc ggcgtgggcc tggagtacgg gcccacgctg cgagccgtcg  24540
agacgctgag cggcggcacc cggcaggcgg tggcccggct gcggctgtcg gccgccgcgt  24600
ccgcgaggac cgggttcgcc ctgcacccga gcctgctcga cgccgcactc cagtccacgg  24660
cgggcctctt caccggttcc ggtacgtcct cggcggccct gccgtttgcc ctggaccggc  24720
tggaggtgct gcgcgcgacg ccctcctcgg ggtgggcggt ggcacgcttc gccgccgacg  24780
accgcccagg cggggtgcgc cgcctggaca tcgacgtgtg cgacgacgac ggcgaggtgt  24840
gcgtacggat ccggggcttc caggtccgta cgtacggcgg cgacgccgcc ccgtccgcct  24900
ccggcgccgc aaacggcacc cacgccgtga ccacggacgg caccggaaac ggcacagaca  24960
ccggcaacgg aaacagcacc ggcaccggca gcgaggcgga cgcggacgcc cggctgctgc  25020
acctcatcca cgccatcggc gagggcgccc tgagcgctga cgaattccag cggagcctca  25080
tatga                                                              25085
```

<210> 36
<211> 2098
<212> PRT
<213> Streptomyces amphibiosporus

<400> 36

```
Met Thr Thr His Ala Thr Ser Leu Thr Glu Leu His Glu Gln Ile Arg
1               5                   10                  15

Thr Gly Arg Ile Gly Gln Asp Glu Ala Leu Arg Leu Ile Arg Ala Trp
            20                  25                  30

Gln Gln Gly Arg Gln Thr Gly Ser Glu Gly Gly Pro Ala Glu Arg Gln
        35                  40                  45

Ala Thr Gly Asp Asp Ala Ala Ala Arg Gly Glu Ala Leu Arg Glu Arg
    50                  55                  60

Val Cys Asp Ile Val Thr His Ala Val Ser Glu Leu Leu Lys Val Gly
65                  70                  75                  80

Pro Asp Asp Leu Asp Ala Asp Val Glu Leu Ser Glu Tyr Gly Leu Asp
                85                  90                  95
```

Ser Ile Val Met Ser Gln Leu Val Asn Ala Val Asn Asp Glu Leu Gly
100 105 110

Leu Glu Leu Ala Pro Thr Val Leu Phe Glu His Pro Asn Leu Arg Ala
115 120 125

Phe Ser Ala His Leu Ala Asp Thr Tyr Ala Asp Ser Leu Ser Val Arg
130 135 140

Leu Leu Gly Thr Pro Gly Thr Gly Pro Ala Pro Ala Pro Ser Thr Ala
145 150 155 160

Thr Ser Pro Ala Pro Ser Thr Ala Thr Ser Ala Glu Pro Ala Ala Val
165 170 175

Ala Ala Pro Ser Thr Ser Pro Ser Glu Ala Arg Thr Glu Ser Arg Val
180 185 190

Pro Thr Pro Pro Ala Thr Gly Gly Arg Phe Phe Pro Ala Ala Val Pro
195 200 205

Ser Ala Pro Ser Ala Glu Thr Glu Pro Val Thr Ala Pro Ala Pro Ala
210 215 220

Pro Ala Ser Glu Gln Ala Ser Pro Ala Gln Ala Ser Ala Ala Glu Glu
225 230 235 240

Pro Val Ala Val Val Gly Met Ser Gly Arg Phe Pro Met Ala Asp Asp
245 250 255

Leu Ala Glu Phe Trp Glu Asn Leu Arg Glu Gly Arg Asp Cys Ile Arg
260 265 270

Glu Val Pro Ser Asp Arg Trp Asp Trp Arg Glu Tyr Tyr Gly Asp Pro
275 280 285

Val Glu Glu Pro Gly Arg Thr Asp Val Lys Trp Gly Gly Phe Ile Asp
290 295 300

Gly Val Ala Asp Phe Asp Pro Leu Phe Phe Gly Ile Ala Pro Lys Glu
305 310 315 320

Ala Leu His Met Asp Pro Gln Gln Arg Leu Leu Met Leu Tyr Val Trp
325 330 335

Lys Ala Leu Glu Asp Ala Gly His Ser Ala Asp Ser Leu Ala Gly Ser
340 345 350

Asp Leu Ala Met Phe Val Gly Thr Asn Asp Thr Gly Tyr Gly Thr Leu
355 360 365

Ala Glu Arg Cys Gly Lys Arg Asp Ser Val Ser Pro Thr Gly Gly Val
370 375 380

Pro Ser Leu Gly Pro Asn Arg Met Ser Phe Phe Leu Asp Val His Gly
385 390 395 400

Pro Ser Glu Pro Val Glu Thr Ala Cys Ser Ser Ser Leu Val Ala Met
405 410 415

His Arg Gly Val Thr Ala Ile Ala Arg Ala Glu Cys Glu Thr Ala Val
420 425 430

Val Gly Gly Ile Asn Thr Ile Val Val Pro Asp Gly His Val Ser Phe
435 440 445

Ser Arg Ala Gly Met Leu Ser Val Asp Gly Arg Cys Lys Thr Phe Ser
450 455 460

Val Gly Ala Asp Gly Tyr Gly Arg Gly Glu Gly Val Gly Ile Leu Val
465 470 475 480

Leu Lys Arg Leu Ser Ala Ala Val Arg Asp Gly Asp His Val Tyr Gly
485 490 495

Val Val Arg Gly Ser Ala Val Asn His Gly Gly Arg Ala Asn Ser Leu
500 505 510

Thr Ala Pro Asn Pro Arg Ala Gln Ala Asp Leu Val Val Gly Ala Trp
515 520 525

Ser Arg Ala Gly Val Asp Pro Arg Ser Val Gly Tyr Val Glu Ala His
530 535 540

Gly Thr Gly Thr Gly Leu Gly Asp Pro Val Glu Val Asn Gly Leu Lys
545 550 555 560

Ala Ala Phe Ala Glu Leu Tyr Glu Arg Trp Gly Val Ser Gly Ala Gly
565 570 575

Glu Ala His Cys Gly Leu Gly Ser Val Lys Thr Asn Ile Gly His Leu
580 585 590

Glu Leu Ala Ser Gly Val Ala Gly Val Ile Lys Val Leu Leu Gln Met
595 600 605

Arg His Arg Thr Leu Val Gly Ser Leu His Cys Gly Ser Val Asn Pro
610 615 620

Tyr Val Arg Leu Glu Gly Ser Pro Phe Arg Leu Val Arg Glu Arg Glu
625 630 635 640

Pro Trp Arg Ala Val Arg Asp Glu Asn Gly Arg Glu Leu Pro Arg Arg
645 650 655

Ala Gly Val Ser Ser Phe Gly Phe Gly Gly Ala Asn Ala His Ile Val
660 665 670

Leu Glu Glu Tyr Gln Pro Pro Ala Gly Thr Gln Thr Asp Ala His Thr
675 680 685

Arg Thr Gly Pro Ser Thr Thr Val His Ser Gly Pro Val Ala Val Leu
690 695 700

Leu Ser Ala Arg Glu Pro Glu Thr Leu Arg Ala Arg Ala Arg Gln Leu
705 710 715 720

Val Asp Trp Leu Asp Lys Gly Glu Ala Thr Glu Ala Asp Leu Pro Arg
725 730 735

Ile Ser Tyr Thr Leu Gln Val Gly Arg Val Ala Met Pro Glu Arg Leu

```
                740                 745                 750

Ala Cys Val Thr Glu Ser Leu Ala Glu Leu Arg Ala Gln Leu Gln Glu
        755                 760                 765

Phe Leu Asp Gly Glu Arg Pro Arg Gly Val Arg Thr Gly Arg Ala Glu
    770                 775                 780

Arg Arg Gly Ile Trp Asn Asp Leu Ala Asp Asp Glu Asp Ile Thr Ala
785                 790                 795                 800

Ala Val Asp Asn Trp Met Ala Lys Gly Lys Leu Asp Arg Leu Leu Lys
                805                 810                 815

Leu Trp Val Ala Gly Ala Glu Phe Asp Trp Arg Arg Leu Trp Gly Glu
            820                 825                 830

His Pro Pro Arg Arg Ile Pro Leu Pro Ala Tyr Pro Phe Arg Leu Gln
        835                 840                 845

Arg Tyr Trp Ile Ala Asp Gly Thr Ser Gly Arg Ser Thr Arg Arg Pro
    850                 855                 860

Ser Thr Ala Arg Glu Gly Thr Pro Tyr Gly Gly Thr Pro Arg Asp Ala
865                 870                 875                 880

Glu Tyr Arg Glu Leu Leu Arg Gly Asp Glu Tyr Phe Leu Arg Asp His
                885                 890                 895

Arg Val Gly Gly Val Pro Thr Leu Pro Gly Ala Ala Cys Leu Glu Leu
            900                 905                 910

Val Arg Ala Ala Trp Thr His Ala Asp Arg Ala Pro Asp Thr Ala Pro
        915                 920                 925

Leu Arg Leu Arg Asp Val Leu Trp Leu Arg Pro Leu Gln Val Thr Ala
    930                 935                 940

Pro Arg Thr Val Ala Val Ala Leu Asp Pro Ala Asp Gly Thr Tyr Glu
945                 950                 955                 960

Val Arg Ala Ala Asp Gly Asp Glu Arg Glu Val Tyr Ala Arg Gly Thr
                965                 970                 975

Val Thr Ala Asp Gly Pro His Thr Val Asp Gly Pro His Ser Ala Gly
            980                 985                 990

Gly Asp Arg Pro Gly Gly Thr Ala  Glu Pro Ala Glu Pro  Val Pro Ala
        995                 1000                1005

His Asp  Ile Ala Ala Leu Arg  Asp Arg Cys Pro His  Arg Leu Asp
    1010                1015                1020

Ala Asp  Gly Cys Tyr Asp Arg  Phe Ala Asp Leu Gly  Leu Ala Tyr
    1025                1030                1035

Gly Pro  Ala Leu Arg Ala Val  Glu Thr Leu His Tyr  Gly Ala Asp
    1040                1045                1050

Leu Ala  Leu Ala Arg Leu Val  Leu Pro Glu Ala Ala  Ala Gly Glu
    1055                1060                1065
```

Arg Thr Leu Asn Pro Ser Met Leu Asp Ala Ala Phe Gln Thr Thr
1070 1075 1080

Leu Gly Val Leu Leu Gly Glu Gln Ala Gln Ala Ala Asp Ala Glu
1085 1090 1095

Arg Ala Ala Ala Gly Gly Ser Glu Asp Val Ala Ala Leu Pro Phe
1100 1105 1110

Ala Val Arg Glu Val Arg Ile Leu Ala Pro Thr Pro Ala Glu Gly
1115 1120 1125

Trp Ala Val Ala Arg Ala Ala Glu Gly Asp Arg Pro Gly Gly Thr
1130 1135 1140

Val Arg Thr Leu Asp Ile Asp Leu Cys Asp Thr Ser Gly Arg Val
1145 1150 1155

Cys Val Arg Leu Thr Gly Phe Ser Thr Arg Thr Val Pro Glu Asp
1160 1165 1170

Gly Ala Pro Gln Leu Pro Gly Glu Pro Pro Val Leu Met Ile Glu
1175 1180 1185

Pro Ala Trp Arg Glu Ala Glu Ala Ala Ser Val Ala Ala Met Pro
1190 1195 1200

Glu Asp His Arg Val Val Leu Cys Glu Leu Pro Gly Val Asp Ala
1205 1210 1215

Ala Glu Leu Ala Gly Ser Leu Gly Gly Asp Cys Glu Thr Trp Gln
1220 1225 1230

Ala Glu Gly Asp Val Ala Ala Arg Tyr Thr Glu Tyr Ala Arg Arg
1235 1240 1245

Leu Leu Glu Leu Leu Gln Glu Glu Ala Arg Ser Pro Ala Pro Ala
1250 1255 1260

Pro Ala Pro Ala Pro Gly Gly Thr Ser Gly Gly Val Pro Gly Gly
1265 1270 1275

Arg Leu Val Gln Leu Val Thr Pro Ser Ser Ala Pro Trp Leu Gly
1280 1285 1290

Gly Leu Ser Gly Met Val Arg Thr Ala Arg Gln Glu His Pro Lys
1295 1300 1305

Leu Leu Val Gln Trp Ile Glu Ala Glu Asp Asp Cys Ser Ala Asp
1310 1315 1320

Glu Leu Ala Val Leu Leu Arg Gly Asp Gly Ala Asp Pro Ala Glu
1325 1330 1335

Val Ala Val Arg His Gly Asp Gly Arg Arg Arg Val Ser Arg Trp
1340 1345 1350

Arg Glu Thr Pro Pro Pro Ala Pro Arg Val Pro Trp Arg Asp Gly
1355 1360 1365

Gly Val Tyr Leu Val Thr Gly Gly Ser Gly Gly Leu Ala Ala Leu
1370 1375 1380

Phe Ala Lys Asp Met Ala Arg Arg Val Arg Arg Pro Ser Leu Val
1385 1390 1395

Leu Cys Gly Arg Gly Ala Ala Gly Pro Glu Gln Arg Glu Leu Val
1400 1405 1410

Ala Glu Leu Glu Ala Leu Gly Ala Arg Ala Glu Tyr Arg Val Leu
1415 1420 1425

Asp Val Ser Asp Ala Gly Ala Val Ser Ala Ala Val Arg Glu Val
1430 1435 1440

Val Ala Ala His Gly Ala Leu His Gly Val Val His Ala Ala Gly
1445 1450 1455

Val Leu Arg Asp Gly Phe Leu Ala Arg Lys Ser Ala Glu Glu Leu
1460 1465 1470

Arg Gln Val Phe Ala Gly Lys Val Ala Gly Leu Arg His Leu Asp
1475 1480 1485

Glu Ala Thr Ala Asp Val Glu Leu Asp Phe Leu Ile Ala Phe Ser
1490 1495 1500

Ser Met Ala Ala Phe Gly Asn Ala Gly Gln Ala Asp Tyr Ala Ala
1505 1510 1515

Ala Asn Ala Phe Leu Asp Gly Tyr Ala Gln His Arg Glu Ala Leu
1520 1525 1530

Arg Ala Arg Gly Glu Arg His Gly Arg Thr Leu Ser Val Asn Trp
1535 1540 1545

Pro Leu Trp Glu Lys Gly Gly Met Arg Gly Gly Ala Gly Thr Glu
1550 1555 1560

Ala Val Leu Gln Gly Val Gly Met Arg Pro Met Arg Ala Glu Thr
1565 1570 1575

Gly Leu Asp Ala Leu Tyr Arg Ala Trp Ala Cys Gly Leu Thr Ser
1580 1585 1590

Val Leu Val Leu Glu Gly Asp His Glu Arg Met Arg Ser Arg Leu
1595 1600 1605

Leu Pro Glu Gln Pro Pro Leu Pro Glu Pro Pro His Arg Pro Asp
1610 1615 1620

Ala Gln Lys Pro Leu Asp Ala Gln Lys Pro Leu Asp Ala Pro Glu
1625 1630 1635

Leu Pro Asp Gly Pro Glu Ala Thr Arg Thr Gly Gly Gly Val Pro
1640 1645 1650

Val Arg Ser Gly Ser Ala Asp Val Ala Arg Arg Val Thr Ala Val
1655 1660 1665

Leu Ala Asp Leu Leu Glu Ile Asp Ala Glu Ser Leu Arg Pro Asp

1670 1675 1680

Val Pro Leu Arg Glu Tyr Gly Leu Asp Ser Ile Phe Leu Thr Gln
1685 1690 1695

Phe Leu Gly Thr Ala Arg Lys Glu Phe Asp Pro Ala Leu Thr Leu
1700 1705 1710

Asp Val Ile Ala Gly Cys Glu Thr Leu Thr Asp Phe Ile Asp Ala
1715 1720 1725

Ile Glu Arg Ala Val Ala Pro Pro Ala Ala Thr Pro Pro Ala Pro
1730 1735 1740

Ala Ser Ala Ser Ala Pro Ser Pro Ser Ser Gly Thr Glu Gly Glu
1745 1750 1755

Pro Ser Thr Arg Pro Ala Pro Glu Pro Asp Gly Val Pro Val Val
1760 1765 1770

Arg Pro Val Ala Lys Ala Pro Glu Glu Phe Pro Glu Leu Ile Pro
1775 1780 1785

Met Asn Ala Val Arg Glu Gly Arg Pro Val Phe Trp Val His His
1790 1795 1800

Gly Asn Gly Gly Val Glu Ser Tyr Ala Ala Val Ala Glu Cys Cys
1805 1810 1815

Gly Arg Pro Phe Tyr Gly Ile Gln Pro Arg Gly Trp Thr Gly Ser
1820 1825 1830

Glu Asp Ile Leu Thr Gly Gln Glu Ala Met Ala Ala Tyr Tyr Val
1835 1840 1845

Asp Ile Ile Arg Ala Val Gln Pro Glu Gly Pro Tyr Asp Val Gly
1850 1855 1860

Gly Phe Ser Leu Gly Gly Leu Phe Ala Tyr Glu Val Val Arg Gln
1865 1870 1875

Leu Gln Leu Gln Asp Ala Thr Val Asp Thr Leu Val Met Leu Asp
1880 1885 1890

Thr Leu Asp Ala Ala Ser Thr Asn Leu Ala Asn Ser Leu Met Thr
1895 1900 1905

Gly Gly Arg Gln Asp Asp Ala Asp Val Val Ala Lys Val Ser Ala
1910 1915 1920

Phe Arg Ala Val Asn Leu Met Leu Gly Asn Asp Ser Leu Asp Ala
1925 1930 1935

Arg Glu Gly Thr Ser Ser Val Leu His Arg Asp Glu Val Asp Thr
1940 1945 1950

Ala Leu Asp Pro Asp Ala Phe Leu Asp Ser Leu Val Glu Ala Ala
1955 1960 1965

Val Ala Arg Gly Ile His Lys Thr Pro Ala Gln Leu Arg Thr Arg
1970 1975 1980

```
Val Arg Gln Leu Ala Arg Tyr Phe Asp Ala Val His Gly Glu Arg
    1985                1990                1995

His Val Val His Pro Leu Pro Arg Arg Glu Glu Val Arg Cys Tyr
    2000                2005                2010

Tyr Leu Arg Asn Ala Gly Gly Gln Phe Phe Gly Pro Phe Glu Glu
    2015                2020                2025

Tyr Met Val Leu Phe Pro Glu Pro Asp Leu Pro Ala Val Asp Gly
    2030                2035                2040

Thr Pro Tyr Trp Arg Glu Trp Ala Asp Ala Val Asp Asp Phe Phe
    2045                2050                2055

Val Ile Asp Val Asp Thr Glu Thr His Ala Gln Val Met Thr Glu
    2060                2065                2070

Pro Ala Ala Leu Lys Lys Val Leu Arg Leu Cys Asp Arg Leu Tyr
    2075                2080                2085

Ala Pro Glu Gln His Ala Gln Gly Gly Arg
    2090                2095
```

<210> 37
<211> 6297
<212> DNA
<213> Streptomyces amphibiosporus

<400> 37

```
atgaccactc acgccacgtc acttaccgaa ctgcacgagc agatccgtac cggacggatc     60
ggccaggacg aggccctccg gctgatccgg gcctggcagc agggccggca gaccgggagc    120
gagggcgggc cggcggagcg gcaggccacg ggcgacgacg ccgcggcccg tggcgaggcc    180
ctgcgcgagc gcgtgtgcga catcgtgacg cacgcggtca gcgagttgct gaaggtcggc    240
ccggacgacc tggacgccga cgtcgaactc agcgagtacg ggctggactc gatcgtgatg    300
agccagctcg tcaacgcggt gaacgacgaa ctcggcctgg aactcgcccc cacggtcctc    360
ttcgagcacc cgaatctgcg ggccttcagc gcccacctcg ccgacacgta cgcggactcg    420
ctctccgtac ggctgctcgg cacgcccggc acggggcccg cgcccgcccc ctcgaccgcg    480
acatcgcccg ccccctcgac cgcgacatcg gccgaacccg cggccgtcgc cgctccgtcg    540
acgtcaccgt cggaggcccg tacggaatcc cgggtgccta cgcctccggc aaccggaggc    600
cgcttcttcc ccgccgccgt cccatccgcc ccatccgccg aaaccgaacc cgtcaccgca    660
cccgcacccg cgcccgcctc tgaacaggct tcccctgcgc aggcttccgc tgcggaggag    720
cccgtcgccg tcgtcggcat gagcggacgt tttccgatgg cggatgatct ggcggagttc    780
tgggagaact tgcgtgaggg ccgggactgt attcgtgagg tgccctcgga tcgctgggac    840
tggcgcgagt actacggcga tcccgtcgag gagcccggcc gcaccgatgt gaagtggggc    900
```

```
ggattcatcg acggcgtcgc cgacttcgat ccgctcttct tcggcatcgc gccgaaggaa     960
gccctccata tggacccgca gcagcggctg ttgatgctct acgtctggaa ggccctggag    1020
gacgcgggcc attcggcgga cagtctcgcc gggagcgatc tggcgatgtt cgtcggtacg    1080
aacgacaccg gttacggcac gctcgccgaa cggtgcggaa aacgggacag cgtctcgccc    1140
accggcggcg tcccctcact cggcccgaac cgcatgagct tctttctcga cgtgcacggt    1200
cccagcgagc ccgtggaaac ggcgtgttcg agttcgctgg tcgccatgca ccgcggtgtc    1260
acggcgatcg cccgcgcgga atgtgagacc gccgtggtcg gcggcatcaa caccatcgtc    1320
gttcccgacg gtcacgtcag cttctcccgt gcggggatgt tgagtgtgga tggtcggtgt    1380
aagacgtttt cggtgggggc tgatggttat gggcgtggtg agggggtggg gattttggtg    1440
ttgaagcgtt tgtcggctgc ggtgcgtgat ggtgatcatg tgtatggggt ggtgcgtggt    1500
tcggcggtga atcatggggg tcgtgcgaat tctttgacgg cgccgaatcc tcgtgctcag    1560
gcggatttgg tggtgggtgc gtggtcgcgg gcgggtgttg atccgcggtc ggtgggttat    1620
gtggaggcgc atggtacggg gacggggctg ggtgatccgg ttgaggtgaa cgggttgaag    1680
gctgcgtttg cggagttgta tgagcggtgg ggtgtttcgg gggccggtga ggcgcattgt    1740
ggtctgggtt cggtgaagac gaatatcggg catttggagt tggcgtcggg tgtcgcgggt    1800
gtgatcaagg tgttgttgca gatgcggcat cggacgttgg tggggagttt gcattgtggg    1860
tcggtgaatc cgtatgtgcg gttggagggg agtcctttcc gtctggtgcg ggagcgtgag    1920
ccgtggcggg cggtacggga tgagaacggg cgggagttgc cgcgccgtgc gggcgtgagt    1980
tccttcggtt tcggcggcgc caacgcccat atcgttctgg aggaatacca gcccccggcc    2040
ggcacgcaga ccgacgccca cacccgcacc ggcccctcaa ccaccgtcca cagcggcccc    2100
gttgccgtcc tgctctccgc gcgtgagccc gagacgctgc gcgcccgcgc acggcagctc    2160
gtcgactggc tcgacaaggg cgaggccacc gaggccgatc tgccgcggat ctcctacacc    2220
ctccaggtcg gccgggtcgc gatgccggaa cgactggcct gtgtgacgga gtcgctggcc    2280
gaactgcgcg cccagctcca ggagttcctc gacggcgaac ggccccgtgg cgtacggacc    2340
gggcgtgccg agcggcgcgg catctggaac gacctggccg acgacgagga catcaccgcc    2400
gcggtcgaca actggatggc caagggcaag ctcgaccggc tgctcaaact ctgggtcgcc    2460
ggcgccgagt tcgactggcg gcggctgtgg ggcgaacacc ccccgcggcg tattccgctg    2520
cccgcctacc ccttccggct ccagcgctac tggatcgccg acggcacaag cggccggtcc    2580
acacggcggc cgtcgaccgc gcgcgaggga acgccgtacg gaggcacccc gcgggacgcg    2640
gagtaccgcg aactgctgcg cggcgacgag tacttcctgc gtgaccaccg cgtgggcggc    2700
gtgccgacgc tgcccggtgc cgcctgtctg gagctggtcc gcgcggcctg gacccacgcc    2760
```

gaccgtgccc ccgacaccgc cccgctgcgg ctgcgcgacg tgctgtggct gcgtccgctc 2820
caggtcaccg cgccccgtac cgtcgccgtc gccctcgacc cggccgacgg cacgtacgag 2880
gtgcgcgccg cggacggcga cgagcgcgag gtgtacgcac gcggcaccgt caccgctgac 2940
ggcccccaca ccgtcgacgg cccccacagc gccggcggcg accgtccggg cgggaccgcc 3000
gaaccggcgg agcccgtacc ggcacacgac atcgccgccc tgcgcgaccg ctgcccccac 3060
cgtctcgacg ccgacggctg ctacgaccgg ttcgcggacc tgggcctcgc ctacggcccg 3120
gccctgcgcg ccgtcgagac gctgcactac ggcgcggacc tggcactggc ccgtctggtg 3180
ctgccggagg cggcggccgg ggaacggacg ctcaacccga gcatgctgga cgccgcattc 3240
cagaccaccc tcggcgtgct cctcggcgag caggcccagg cggcggacgc cgaacgggcc 3300
gctgccggcg gttccgagga cgtcgcggcg ctgccgttcg ccgtgcgcga ggtacggatc 3360
ctcgccccca cccccgccga gggctgggcc gtggcacgcg ccgcggaggg cgaccggccc 3420
ggcgggacgg tacgcaccct ggacatcgac ctgtgcgaca cctccgggcg ggtctgcgta 3480
cgcctcacgg ggttcagcac ccgtacggtc cccgaggacg gtgcgcccca actccccggg 3540
gagccgcccg tgttgatgat cgagcccgcc tggcgcgagg cggaggcggc ctccgtggcg 3600
gccatgccgg aggaccaccg ggtcgtgctg tgcgaactgc ccggcgtgga cgcggccgag 3660
ctggccggct ccctcggcgg cgactgcgag acctggcagg ccgaggggga cgtcgccgcc 3720
cgctacaccg agtacgcccg gcggctgctg gaactcctcc aggaggaggc ccgcagcccg 3780
gccccggccc cggcccccgc ccctggcggg acctccggcg gggttcccgg cgggcggctc 3840
gtccagctcg tcaccccgtc ctcggcaccc tggctcggcg gtctgagcgg catggtgcgc 3900
accgcccgcc aggagcaccc caagctcctc gtccagtgga tcgaggccga agacgactgc 3960
tccgccgatg agttggcggt gctgctgcgc ggcgacggcg ccgaccccgc cgaggtggcg 4020
gtacggcacg gcgacggacg gcgcagggtc tctcggtggc gcgagacgcc gccgcccgcg 4080
ccccgcgtcc cctggcgcga cggcggggtc tatctcgtca ccggcggctc gggcggcctc 4140
gccgcgctgt tcgccaagga catggcccgt cgcgtgcggc ggccgtcgct ggtcctctgc 4200
ggacgcggcg cggccggtcc cgaacagcgg gagctggtcg cggagttgga ggcgctgggc 4260
gcccgcgcgg agtaccgcgt actggacgtc tccgacgccg gggccgtcag cgcggcggtc 4320
cgggaggtgg tcgccgcaca cggcgccctg cacggtgtcg tccacgcggc gggcgtgctg 4380
cgggacggct tcctcgcgcg caagagcgcc gaggagctgc ggcaggtctt cgcggggaag 4440
gtcgccgggc tgcgccatct cgacgaggcc acggcggacg tggagttgga cttcctgatc 4500
gccttctcat cgatggccgc cttcggcaac gccgggcagg ccgactacgc cgccgccaac 4560

```
gcgttcctcg acggctacgc ccagcaccgc gaagcgctcc gcgcgcgcgg cgagcgccac    4620
gggcggaccc tctcggtgaa ctggccgctg tgggagaagg gcggtatgcg cggcggcgcg    4680
ggcaccgagg ccgtgctcca gggcgtgggc atgcgcccga tgcgggcgga gacggggctc    4740
gacgccctgt accgggcgtg ggcgtgcggt ctgacgtccg tcctggtgct ggagggcgac    4800
cacgagcgga tgcgctcccg gctgctgccg gagcagcccc cgctgcccga gcccccgcac    4860
cggccggacg cacagaagcc gctggacgct cagaagcccc tggacgcacc ggagttgccg    4920
gacggaccgg aagcgacgag gacgggcggc ggcgtgccgg tgcgctccgg ctccgcggac    4980
gtcgcccgcc gggtcaccgc cgtcctggcg gacctgctgg agatcgacgc ggagtccctg    5040
cggcccgacg tgccgctacg cgagtacgga ctcgactcga tcttcctcac ccagttcctc    5100
ggcacggccc gcaaggagtt cgacccggcg ctcacgctcg acgtcatagc gggctgcgag    5160
acgctgacgg acttcatcga cgcgatcgag cgcgccgtcg ccccgccggc cgccacgcct    5220
ccggcgcccg catccgcgtc cgcgccctca ccctcgtccg gtacggaagg ggaaccgtca    5280
acgcgccccg ccccggagcc ggatggcgta ccggtggtgc gtcccgtcgc caaggctccc    5340
gaggagttcc ccgagctgat ccccatgaac gccgtacggg agggccgccc ggtcttctgg    5400
gtccaccacg gcaacggcgg agtcgagtcg tacgcggcgg tggccgagtg ctgcggacgc    5460
cccttctacg gcatccagcc ccgcggctgg acgggctcgg aggacatcct caccggccag    5520
gaggccatgg ccgcctacta cgtggacatc atccgcgccg tccagccgga gggcccgtac    5580
gacgtcggcg gcttctccct cggcggtctg ttcgcctacg aggtcgtacg ccaactccag    5640
ctccaggacg ccacggtgga caccctggtg atgctggaca ccctcgacgc cgcctcgacc    5700
aacctggcca actccctgat gacgggcggc cgtcaggacg acgccgacgt ggtggcgaag    5760
gtcagcgcct tccgcgccgt caacctgatg ctgggcaacg acagcctgga cgcgcgcgag    5820
ggcacctcgt ccgtcctgca ccgggacgag gtggacaccg cgctcgaccc ggacgccttc    5880
ctggactccc tcgtggaggc cgccgtcgcc cgcggcatcc acaagacccc ggcccaactg    5940
cggacgcgcg tacggcagtt ggcgcggtac ttcgacgccg tgcacggcga gcggcacgtg    6000
gtgcacccgc tgccgcggcg cgaggaggtg cgctgctact acctgcgcaa cgcgggcggg    6060
cagttcttcg gccccttcga ggagtacatg gtcctcttcc ccgaaccgga cctcccggcg    6120
gtggacggca ccccgtactg gcgcgaatgg gccgacgccg tcgacgactt cttcgtcatc    6180
gacgtcgaca cggagaccca cgcgcaggtg atgacggagc ccgcggcgct gaagaaggtg    6240
ctgcggctgt gcgaccggct gtacgcgccg gagcagcacg cgcagggagg ccggtga       6297
```

<210> 38
<211> 768
<212> PRT

<213> Streptomyces amphibiosporus

<400> 38

```
Met Glu Ala Val Val Phe Pro Gly Gln Gly Ala Gln Arg Arg Gly Met
1               5                   10                  15

Gly Arg Glu Leu Phe Asp Ala Phe Pro Ser Leu Thr Glu Gln Ala Ser
            20                  25                  30

Asp Val Leu Gly Tyr Ser Val Arg Glu Leu Cys Val Ala Asp Pro Glu
        35                  40                  45

Arg Arg Leu Arg Ser Thr Glu Tyr Thr Gln Pro Ala Leu Phe Val Val
    50                  55                  60

Gly Thr Leu Ala His Leu Lys Trp Gln Glu Glu Thr Gly Arg Ser Ala
65                  70                  75                  80

Ala Tyr Phe Ala Gly His Ser Val Gly Glu Tyr Thr Ala Leu His Ala
                85                  90                  95

Ala Gly Ala Phe Gly Phe Glu Thr Gly Leu Arg Leu Val Gln Arg Arg
            100                 105                 110

Gly Leu Leu Met Ser Gln Ala Glu Gly Gly Gly Met Ala Ala Val Leu
        115                 120                 125

Gly Leu Gly Ala Gly Glu Leu Thr Glu Leu Leu Arg Glu Gly Gly Phe
    130                 135                 140

Val Ser Leu Ala Leu Ala Asn Asp Asn Thr Pro Asp Gln Gln Val Val
145                 150                 155                 160

Ser Gly Ala Ala His Glu Ile Asp Val Leu Glu Ala Tyr Leu Ser Glu
                165                 170                 175

Arg Gly Val Arg Gly Val Arg Leu Asn Val Ser Gly Ala Phe His Ser
            180                 185                 190

Pro Leu Met Leu Pro Ala Gln Glu Ala Phe Ala Ala Tyr Val Arg Asp
        195                 200                 205

Phe Thr Leu Gly Asp Pro Glu Thr Pro Val Ile Ser Asn Val Thr Ala
    210                 215                 220

Arg Pro His Pro Pro Gly Gly Thr Ala Glu Leu Leu Val Arg Gln Ile
225                 230                 235                 240

Ser Ser Pro Val Arg Trp Thr Glu Ser Val Arg His Leu Leu Asp Leu
                245                 250                 255

Gly Val Glu Glu Phe Thr Glu Leu Gly Gly Ser Val Val Ala Lys Leu
            260                 265                 270

Val Arg Gln Ile Arg Glu Ala His Arg Arg Asp Ala Asp Ala Ser Gly
        275                 280                 285

Pro Ala Pro Ala Ala Pro Ala Ala Pro Val Arg Ser Glu Pro Ala Ala
    290                 295                 300
```

Arg Ser Ala Leu Gly Ser Ala Val Phe Arg Glu Arg Leu Gly Leu Arg
305 310 315 320

His Ser Tyr Ala Ala Gly Gly Met Tyr Arg Gly Ile Ala Ser Pro Ala
325 330 335

Met Val Val Arg Leu Ala Arg Ala Gly Met Leu Gly Phe Leu Gly Thr
340 345 350

Gly Gly Leu Thr Pro Glu Ala Val Glu Glu Arg Ile Leu Gln Val Arg
355 360 365

Arg Glu Leu Arg Glu Gly Glu Pro Phe Gly Val Asn Phe Leu Ala Asp
370 375 380

His Asp Asp Pro Ala Ala Glu Arg Arg Val Ala Glu Met Leu Met Arg
385 390 395 400

Arg Gly Val Thr Val Val Glu Ala Ala Ala Phe Ile Gly Met Thr Pro
405 410 415

Ala Ser Ser Ser Thr Ala Arg Ala Gly Met His Arg Gly Pro Asp Gly
420 425 430

Ala Pro Arg Cys Ala His Arg Ile Val Ala Lys Val Ser Arg Pro Glu
435 440 445

Val Gly Arg Arg Leu His Gly Thr Ala Pro Gly Lys Val Val Asp Gly
450 455 460

Leu Leu Arg Glu Gly Ala Ile Thr Gly Glu Gln Ala Glu Leu Val Arg
465 470 475 480

Gln Val Pro Met Ser His Asp Ile Thr Val Glu Ala Asp Ser Gly Gly
485 490 495

His Thr Asp Gly Gly Ile Ala Thr Val Met Leu Pro Ala Met Leu Gly
500 505 510

Leu Arg Arg Gln Ala Gln Arg Ser His Asp Tyr Ala Glu Pro Leu Cys
515 520 525

Met Gly Leu Ala Gly Gly Leu Gly Thr Pro Glu Ala Val Ala Ala Ala
530 535 540

Phe Met Leu Gly Ala Asp Tyr Val Leu Thr Gly Ser Val Asn Gln Cys
545 550 555 560

Thr Val Glu Ala Asp Thr Ser Asp Ala Val Lys Asp Met Leu Gln Thr
565 570 575

Ile Asp Ile Gln Asp Thr Gly Tyr Ala Pro Ala Gly Asp Met Phe Glu
580 585 590

Met Gly Ala Arg Val Gln Val Leu Arg Lys Gly Val Phe Phe Pro Thr
595 600 605

Arg Ala Asn Lys Leu Tyr Ala Leu Tyr Gln His His Asp Gly Leu Asp
610 615 620

```
Asp Leu Pro Ala Lys Thr Arg Ala Leu Leu Glu Arg Ser Tyr Phe His
625                 630                 635                 640

Arg Thr Phe Asp Glu Ile Trp Thr Glu Val Arg Glu His Tyr Arg Ala
                645                 650                 655

Lys Gly Gln Pro Glu Val Thr Asp Lys Ala Glu Arg Gln Pro Lys Val
            660                 665                 670

Lys Met Ala Leu Val Phe Arg Trp Tyr Phe Ala Tyr Ser Ala Arg Leu
        675                 680                 685

Ala Leu Ala Gly Gln Asp Gly Asp Lys Val Asn Phe Gln Ile His Thr
    690                 695                 700

Gly Pro Ala Leu Gly Ala Phe Asn Gln Trp Val Lys Gly Thr Ala Cys
705                 710                 715                 720

Glu Ser Trp Arg Ala Arg His Ala Asp Ala Ile Gly Leu Met Leu Met
                725                 730                 735

Glu Gly Ala Ala Glu His Val Ala Ala Ala Cys Glu Ser Trp Gly Gly
            740                 745                 750

Thr Ser Arg Phe Ala Pro Ala Glu Glu Arg Ala Leu Ala Ala Arg Thr
        755                 760                 765
```

<210> 39
<211> 2307
<212> DNA
<213> Streptomyces amphibiosporus

<400> 39

atggaggcgg tcgtctttcc cgggcagggc gcgcagcgcc gcggcatggg ccgcgagctg 60
ttcgacgcct ttccctcgct caccgagcag gcgtccgacg tcctcgggta ctccgtacgc 120
gagttgtgcg tggcggaccc cgagcgccgg ctgcgcagca ccgagtacac ccagcccgcg 180
ctgttcgtcg tcggcaccct cgcgcacctc aagtggcagg aggagacggg gcgttcggcc 240
gcgtacttcg ccgggcacag cgtgggggag tacaccgcgc tgcacgccgc gggcgccttc 300
ggcttcgaga ccgggctgcg cctggtgcag cggcgcggtc tcctcatgtc gcaggcggag 360
ggcggcggca tggcggccgt actcggcctc ggcgccgggg agttgaccga gctgctccgc 420
gagggcggct tcgtctccct cgccctcgcc aacgacaaca cgcccgatca gcaggtcgtc 480
tcgggcgccg cgcacgagat cgacgtcctg gaggcgtatc tgtccgaacg cggcgtgcgc 540
ggtgtgcggc tgaacgtctc gggcgccttc cactcgccgc tgatgctgcc cgcacaggag 600
gcgttcgccg cgtacgtacg ggacttcacg ctcggcgacc cggagacgcc ggtgatctcc 660
aacgtcaccg cgcggcccca tccgccgggc ggtacggccg agttgctcgt acggcagatc 720
tccagccccg tgcggtggac ggagagcgtg cgccatctgc tcgacctcgg cgtggaggag 780
ttcaccgaac tcggcggcag cgtggtcgcg aagctcgtac ggcagatccg cgaggcgcac 840

```
cgcagggacg cggacgcctc cggcccggcg cccgccgcgc ccgcggctcc cgtacggtcc    900
gaacccgccg cacggtccgc gctcggcagc gcggtcttcc gcgagcggct ggggctgcgc    960
cactcctacg cggcgggcgg catgtaccgg ggcatcgcct ccccggccat ggtggtgcgc   1020
ctcgcccgcg ccgggatgct cggcttcctc ggcacgggcg ggctgacgcc cgaggccgtc   1080
gaggagcgga tcctccaggt ccggcgggag ctgcgcgagg gcgagccctt cggcgtcaac   1140
ttcctcgccg accacgacga tccggccgcc gaacgccgcg tcgccgagat gctgatgcgc   1200
cgcggcgtga ccgtcgtcga ggcggcggcc ttcatcggca tgaccccggc ctcgtcctct   1260
accgcgcgcg cgggcatgca ccgcggaccg gacggggccc cgcgctgcgc ccaccgcatc   1320
gtcgccaagg tctcccgccc cgaggtgggc cggcgccttc atggcaccgc gcccgggaag   1380
gtggtcgacg gcctgctccg ggagggcgcg atcaccggcg agcaggcgga gctcgtacgg   1440
caggtcccga tgagccacga catcaccgtc gaggcggact ccggcggcca caccgacggg   1500
ggcatcgcca ccgtcatgct gcccgcgatg ctcggcctcc ggcggcaggc ccagcgcagc   1560
cacgactacg cggagccgct gtgcatgggg ctcgccggcg gcctcggcac tccggaggcc   1620
gtcgccgcgg ccttcatgct gggcgccgac tacgtcctga cgggctccgt caaccagtgc   1680
accgtcgagg cggacaccag cgacgccgtc aaggacatgc tccagaccat cgacatccag   1740
gacacgggct acgcgcccgc gggcgacatg ttcgagatgg gagcccgggt ccaagtgctg   1800
cgcaagggcg tcttcttccc gacccgggcc aacaagctgt acgcgctcta ccagcaccac   1860
gacggcctcg acgacctgcc cgcgaagacc cgtgccctgc tggagaggtc gtacttccac   1920
cgcaccttcg acgagatctg gacggaggta cgcgagcact accgcgccaa gggccagccc   1980
gaggtcaccg acaaggcgga gcggcagccg aaggtgaaga tggcgctggt cttccgctgg   2040
tacttcgcct actcggcccg gctggcactg gccgggcagg acggcgacaa ggtcaacttc   2100
cagatccaca cggggcccgc gctcggcgcc ttcaaccagt gggtcaaggg cacggcctgc   2160
gagtcctggc gcgcccggca tgccgacgcc atcgggctga tgctcatgga gggcgcggca   2220
gaacacgtcg cggcggcctg cgagtcgtgg ggcggtacct cccgcttcgc ccccgcggag   2280
gaacgcgccc tggccgcccg cacctga                                       2307
```

<210> 40
<211> 418
<212> PRT
<213> Streptomyces amphibiosporus

<400> 40

```
Met Arg Gly His Ala Val Thr Thr His Leu Thr Thr Asp Ile Asp Glu
1               5                   10                  15
```

Ile Val Thr Asp Val Phe Gln Ser Thr Glu Gly Lys Lys Asn Pro Tyr
20 25 30

Pro Leu Tyr Arg Arg Leu Gln Glu Leu Gly Gln Val His Arg Ser Glu
35 40 45

Gln Leu Gly Trp Val Ala Thr Gly Tyr Glu Val Cys Ser Ala Ala Leu
50 55 60

Arg Asp Pro Arg Val Ile Lys Gly Pro Glu Gln Ile Gln Pro Gly Arg
65 70 75 80

Pro Asp Pro Ala Glu His Ser Ala Glu Ala Leu Leu Arg Gly Thr Met
85 90 95

His Arg Leu Asp Pro Pro Asp His Thr Arg Leu Arg Arg Leu Val Asn
100 105 110

Gly Ala Phe Thr Pro Arg Ser Val Ala Ala Leu Glu Pro Asp Ile Gln
115 120 125

Glu Leu Ile Asp Asp Leu Ile Thr Pro Ala Val Lys Lys Ala Glu Ala
130 135 140

Gly Glu Pro Val Asp Met Met Ser Gly Phe Ala Phe Pro Leu Ser Val
145 150 155 160

Ala Val Ile Gly Arg Met Leu Gly Val Pro Ala Ser Asp Trp His Arg
165 170 175

Phe His Asp Val Val Leu Asp Leu Ser Ser Met Val Glu Leu Gly Phe
180 185 190

Thr Gly Asp Glu Leu Pro Lys Ala Asp Ala Ala Ala Asp Glu Leu Ile
195 200 205

Ala Tyr Phe Arg Lys Leu Gly Ala Glu Arg Met Arg Asn Pro Ala Asp
210 215 220

Asp Leu Thr Ser Thr Leu Ala Asn Ala Thr Glu Ala Gly Asp Arg Leu
225 230 235 240

Thr Glu Gln Glu Leu Val Thr Met Leu Ile Leu Leu Phe Met Ala Gly
245 250 255

Phe Glu Thr Thr Thr His Ser Met Gly Asn Gly Met Phe Ala Leu Leu
260 265 270

Glu Asn Pro Glu Gln Thr Gln Trp Leu Arg Arg Asn Met Asp Ala Met
275 280 285

Pro Ala Ala Val Glu Glu Leu Ile Arg Tyr Asp Ser Pro Val Gln Phe
290 295 300

Ile Ala Gly Tyr Thr Lys Glu Pro Val Glu Leu Ala Asp Gly Thr Ala
305 310 315 320

Val Pro Ala Asp Glu Tyr Leu Phe Leu Met Ile Gly Ala Ala Asn Arg
325 330 335

Asp Pro Arg Val Phe Ser Asp Pro Glu Leu Leu Arg Leu Asp Arg Gly

340 345 350

Glu Ala Ala Pro Met Ser Phe Gly Gly Gly Ile His Tyr Cys Leu Gly
355 360 365

Ala Gly Leu Ala Arg Leu Glu Ile Arg Lys Ile Phe Thr Ser Leu Leu
370 375 380

Thr Arg Phe Ser Ala Ile Glu Leu Ala Glu Pro Glu Pro Glu Arg Arg
385 390 395 400

Ser Gly Leu Ala Leu Arg Gly Tyr Ala Arg Ile Pro Met Trp Leu Thr
405 410 415

Pro Ala

<210> 41
<211> 1257
<212> DNA
<213> Streptomyces amphibiosporus

<400> 41

atgagaggac acgcagtgac cacccacctc accaccgaca tcgacgagat cgtcacggac 60
gtcttccagt ccaccgaggg gaagaagaac ccctaccccc tgtaccggcg cctccaggag 120
ctgggccagg tgcaccgctc cgagcaactg ggctgggtcg ccacgggata cgaggtgtgc 180
agcgccgcgc tgcgcgaccc ccgcgtcatc aagggccccg agcagatcca gccgggccgc 240
cccgaccccg ccgagcactc cgccgaggcg ttgctgcgcg gcacgatgca ccggctcgac 300
ccgccggacc acacccggct gcgccgcctc gtcaacggcg ccttcacgcc gcgcagcgtc 360
gccgcgctgg agccggacat ccaggaactg atcgacgacc tgatcacacc ggccgtcaag 420
aaggcggagg cgggcgagcc cgtcgacatg atgagcggct tcgccttccc gctctccgtc 480
gccgtgatcg gccgcatgct cggcgtgccc gcgtccgact ggcaccgctt ccacgacgtc 540
gtgctcgacc tgtcctccat ggtcgaactc ggcttcaccg gcgacgagtt gcccaaggcc 600
gacgccgccg cggacgaact gatcgcctac ttccgcaagt tgggcgccga gcgcatgcgc 660
aaccccgccg acgacctgac ctccacgctc gccaacgcga ccgaggccgg tgaccgcctc 720
accgagcagg aactcgtcac catgctgatc ctgctgttca tggccggttt cgagacgacg 780
acccactcca tgggcaacgg catgttcgcc ctgctggaga acccggagca gacgcagtgg 840
ctgcgccgca acatggacgc catgcccgcg gccgtcgagg agctgatccg ctacgactcc 900
ccggtgcagt tcatcgccgg ctacaccaag gagcccgtgg aactggcgga cggcaccgcc 960
gtccccgcgg acgagtatct gttcctgatg atcggcgcgg ccaaccgcga cccgcgcgtc 1020
ttctccgacc ccgaactgct gcgtctggac cgcggtgagg ccgcgccgat gagcttcggc 1080
ggcggcatcc actactgcct cggcgcgggc ctggcccggc tggagatccg gaagatcttc 1140

acctcgctgc tcacccgctt ctccgcgatc gaactggccg agcccgaacc ggaacgccgc 1200

agcggactcg ccctgcgcgg ctacgcccgc atcccgatgt ggctcacccc ggcgtaa 1257

<210> 42
<211> 247
<212> PRT
<213> Streptomyces amphibiosporus

<400> 42

Val Ile Gly Arg Met Leu Pro Gly Trp Val Ser Thr Glu Glu Ile Phe
1 5 10 15

Val Gly Gly Gln Glu Asp Thr Leu Ser Gly Leu Phe Pro Glu Glu Arg
20 25 30

Ala Ala Val Ala Arg Ala Val Pro Lys Arg Gln Arg Glu Phe Ala Asp
35 40 45

Val Arg Ala Cys Ala Arg Ser Ala Leu Gly Arg Leu Gly Val Ala Pro
50 55 60

Val Pro Leu Val Pro Gly His Arg Gly Ala Pro Gln Trp Pro Glu Gly
65 70 75 80

Val Val Gly Ser Met Thr His Cys Asp Gly Tyr Arg Ala Ala Ala Val
85 90 95

Ala Arg Gly Ser Asp Ala Val Gly Ile Gly Ile Asp Ala Glu Pro Ala
100 105 110

Glu Pro Thr Pro Asp Gly Val Leu Gly Val Ile Ser Leu Pro Ala Glu
115 120 125

Arg Glu His Leu Arg Thr Leu Ala Ala Ala His Pro Gln Val Ala Trp
130 135 140

Asp Arg Leu Leu Phe Ser Ala Lys Glu Ser Val Phe Lys Val Trp Tyr
145 150 155 160

Pro Leu Thr Ser Arg Glu Leu Asp Phe Ser Glu Ala Glu Ile Thr Ile
165 170 175

Asp Ala Asp Ala Gly Thr Phe Ser Ala Arg Leu Leu Val Glu Gly Pro
180 185 190

Val Leu Gly Gly Glu Arg Leu Arg Gly Phe Thr Gly Arg Trp Ala Ala
195 200 205

Arg Arg Gly Leu Leu Ala Thr Ala Val Leu Leu Gly Ser Arg Thr Glu
210 215 220

Thr Gly Ala Gly Ala Gly Ser Lys Ser Glu Thr Glu Thr Gly Thr Glu
225 230 235 240

Thr Gly Pro Ala Ala Leu Arg
245

<210> 43

<211> 744
<212> DNA
<213> Streptomyces amphibiosporus

<400> 43

```
gtgatcgggc ggatgttgcc ggggtgggtg agcaccgagg agatcttcgt cggtgggcag    60
gaggacacgc tgagtgggct cttccccgag gagcgggccg ccgtggcccg ggcggtgccc   120
aagcggcagc gggagttcgc ggacgtacgg gcgtgtgcgc ggagtgcact ggggcggctg   180
ggtgtggcgc ccgtgccgct ggtgccgggg caccggggcg ccccgcagtg gccggagggt   240
gtcgtcggca gcatgacgca ctgcgacggc taccgggcgg cggccgtcgc ccgcggcagc   300
gacgcggtcg gcatcggcat cgacgccgaa cccgcagagc cgacgccgga cggcgttctg   360
ggcgtgatct ccctgcccgc cgagcgggaa cacctgcgca cgctggcggc ggcgcatccc   420
caagtggcct gggaccgcct gctgttcagc gcgaaggaga gcgtcttcaa ggtctggtat   480
ccgctcacct cacgcgaact cgacttctcc gaggccgaga tcaccatcga cgcggacgcg   540
ggcacgttct ccgcgcggct cctcgtggag gggccggtgc tcggcggcga gcggttgcgc   600
ggtttcaccg gccgctgggc ggcacggcgg gggctgctgg ccaccgccgt cctcttgggg   660
agcaggaccg agacgggagc aggggcgggg tccaagtcgg agacggagac ggggacggag   720
acggggcccg cggcgctccg ctga                                          744
```